(19) Europäisches Patentamt
European Patent Office
Office européen des brevets

(11) EP 3 917 907 B1

(12) EUROPEAN PATENT SPECIFICATION

(45) Date of publication and mention of the grant of the patent:
**21.05.2025 Bulletin 2025/21**

(21) Application number: **20702775.6**

(22) Date of filing: **29.01.2020**

(51) International Patent Classification (IPC):
***C07C 233/07*** *(2006.01)* ***C07D 207/16*** *(2006.01)*
***C07D 209/40*** *(2006.01)* ***C07D 209/44*** *(2006.01)*
***C07D 213/40*** *(2006.01)* ***C07D 403/12*** *(2006.01)*
***A61K 31/404*** *(2006.01)* ***A61P 25/08*** *(2006.01)*
***C07C 233/44*** *(2006.01)* ***C07C 237/20*** *(2006.01)*
***C07C 311/14*** *(2006.01)* ***C07C 311/21*** *(2006.01)*

(52) Cooperative Patent Classification (CPC):
**C07D 209/40; A61P 25/00; C07C 233/07; C07C 233/15; C07C 233/43; C07C 233/44; C07C 233/62; C07C 237/10; C07C 237/20; C07C 311/14; C07C 311/21; C07D 207/16; C07D 207/335; C07D 209/44; C07D 213/36;**
(Cont.)

(86) International application number:
**PCT/EP2020/052156**

(87) International publication number:
**WO 2020/157126 (06.08.2020 Gazette 2020/32)**

# (54) MODULATORS OF POTASSIUM ION CHANNELS AND USES THEREOF

MODULATOREN VON KALIUMIONENKANÄLEN UND VERWENDUNGEN DAVON

MODULATEURS DE CANAUX IONIQUES POTASSIQUES ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2019 PCT/EP2019/052067**

(43) Date of publication of application:
**08.12.2021 Bulletin 2021/49**

(73) Proprietor: **Universitá Degli Studi Di Salerno**
**84084 Fisciano (SA) (IT)**

(72) Inventors:
- **AMBROSINO, Paolo**
  **Pietradefusi (AV) (IT)**
- **BERTAMINO, Alessia**
  **Napoli (NA) (IT)**
- **CAMPIGLIA, Pietro**
  **Capaccio (SA) (IT)**
- **CORRIVETTI, Giulio**
  **Salerno (SA) (IT)**
- **FERRIELLO, Anna Bella**
  **Caserta Centurano (CE) (IT)**
- **GOMEZ MONTERREY, Isabel Maria**
  **Salerno (SA) (IT)**
- **IRACI, Nunzio**
  **Barcellona Pozzo di Gotto (ME) (IT)**
- **MICELI, Francesco**
  **Sparanise (CE) (IT)**
- **NOVELLINO, Ettore**
  **Avellino (AV) (IT)**
- **OSTACOLO, Carmine**
  **Casoria (NA) (IT)**
- **SOLDOVIERI, Maria Virginia**
  **Petina (SA) (IT)**
- **TAGLIALATELA, Maurizio**
  **Campobasso (CB) (IT)**

(74) Representative: **Pace Napoleone, Maria et al**
**De Simone & Partners S.r.l.**
**Via Giulio Caccini, 1**
**00198 Roma (IT)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
**WO-A2-2011/101456** **US-A- 5 384 330**
**US-A1- 2014 336 252**

(52) Cooperative Patent Classification (CPC): (Cont.)
**C07D 213/40; C07D 295/155; C07D 403/12;**
C07C 2601/02; C07C 2601/14

## Description

### FIELD OF THE INVENTION

The present invention belongs to the technical field of pharmacy, pharmaceutical chemistry and pharmacology. In particular, the present invention relates to a class of novel compounds of general formula I, their use as medicaments and pharmaceutical compositions comprising them. Specifically, the compounds of the invention are useful as voltage-gated potassium ion channels modulators.

### BACKGROUND OF THE INVENTION

Potassium channels represent the most widely diffused and heterogeneous class of ionic channels characterized so far. Potassium channels are responsible for the stabilization of the membrane potential in neurons: they shift the membrane potential toward the $K^+$ equilibrium potential, away from the firing threshold. Potassium channels are classified both structurally and functionally in four main families: inward rectifier potassium channels (Kir); two pores potassium channels (K2p); Calcium-activated potassium channels (KCa) and voltage-gated potassium channels (Kv). (Aldrich R. et al. Potassium channels. IUPHAR/BPS Guide to PHARMACOLOGY). Among the 13 classes of Kv channels, the family identified as Kv7 (also known as KNCQ) comprises 5 different members (Kv7.1-Kv7.5), characterized by different tissue distribution and physiopathological role. The Kv7.1 channel subunit is mainly expressed in the heart, representing the molecular basis for the $I_{ks}$ ventricular repolarization current; Kv7.2 and Kv7.3 subunits are mainly expressed in the central nervous system (CNS) where they generate the M-current, a repolarizing current limiting the neuronal spike discharge rate; Kv7.4 is mainly expressed in the inner ear and in the visceral, vascular and pulmonary smooth muscles, being involved in the regulation of smooth muscle tone; finally, Kv7.5 is mainly expressed in the CNS, in vascular smooth and skeletal muscles (Soldovieri MV et al., Physiology 2011, 26(5), 365-376).

Given the prominent role played by Kv7 channels in controlling neuronal excitability and considering that neuronal hyperexcitability is a common feature of neuropsychiatric disorders such as epilepsy, neuropathic pain, ischemic stroke, amyotrophic lateral sclerosis, and several other neurodegenerative diseases, pharmacological modulation of Kv7 channels appears as a rational pharmacological approach to treat these conditions. Specific members of the Kv7 subfamily of voltage-gated potassium channels expressed in the vascular smooth muscle produce membrane hyperpolarization sufficient to reduce the likelihood of $Ca^{2+}$ channel opening and hence attenuate vasoconstrictor response; thus their pharmacological modulation reduces the frequency of spontaneous contractions and results in dose-dependent hypotension, with potentially beneficial consequences in vascular hypertension. Similar myolytic responses in the respiratory and genitourinary systems may result in Kv7 activators being potentially useful to treat asthma and bladder hyperreactivity, respectively. Further, given the described role of Kv7 channels in controlling skeletal muscle differentiation, proliferation, and function, it has been suggested that Kv7 channel activators may be useful pharmacological agents against myotonia, myokymia and other skeletal muscle hyperexcitability diseases.

In particular, the pivotal role of Kv7.2 and Kv7.3 in neuronal excitability is evidenced by the occurrence of convulsive disorders with high phenotypic variability in humans, caused by specific mutations of their corresponding genes. For instance, these mutations can be responsible of a benign neonatal epilepsy with autosomal dominant transmission, also known as "Benign familiar neonatal seizures", or of a severe sporadic neonatal epileptic encephalopathy, leading to neurocognitive impairment, anti-epileptic drugs resistance and typical neuroradiological findings (Miceli et al, GeneReviews 2016).

Current therapeutic options to treat the above-cited diseases could be improved in terms of efficacy and tolerability. Although none of the drugs currently approved for each of the mentioned indications targets Kv7 channels directly, available knowledge suggests that defects in the structure or function of at least one voltage-gated potassium channel of the Kv7 subfamily plays a relevant pathophysiological role in each of the cited conditions. The current relevance of potassium channels (possibly different from Kv7) as pharmacological targets is also highlighted by them being the therapeutic targets class III of antiarrhythmics (Amiodarone, Sotalol, Ibutilide, Dofetilide), oral hypoglycemic agents for treatment of type 2 diabetes (Sulfonylureas, Glinides), antihypertensive vasodilators (minoxidil, diazoxide and hydralazine), and for the treatment of multiple sclerosis (3,4-diaminopyridines).

Focusing on epilepsy, it is a neurologic disorder usually ascribed to impaired and paroxysmal neurons firing, responsible for an impairment in the sensitive, cognitive, mental, vegetative and motor activities. Epilepsy can be both a chronic or acute disease with an incidence rate of about 1% in the world population. Epilepsy is also associated to numerous psychiatric (such as anxiety and depression) or cognitive comorbidities, and to an increased risk of death (Sudden Death in Epilepsy or SUDEP). Seizures represent a typical clinical symptom of the epilepsy; they are involuntary and spontaneous motor manifestations caused by neuronal hyperexcitability. The hyperexcitability can be evidenced recording the brain electrical activity by specific electrodes positioned on the scalp, using a technique known as electroencephalogram (EEG). Two main electrophysiological properties characterize seizures: hyperexcitability (that

is the tendency of the cortical or subcortical neurons to overreact to a normal stimulus) and hypersincronicity (that is the tendency to simultaneous activation). Physiological neuron excitability is guaranteed by a proper balance between the main excitatory (glutamaergic) and inhibitory (GABAergic) neural transmissions.

Moreover, the mechanisms regulating seizure threshold, duration and spread, mediated by voltage-gated sodium, potassium and calcium channels, play a pivotal role.

The most important anti-epileptic drugs used nowadays in therapy can be classified on the basis of their mechanism of action, corresponding to: sodium voltage-gated channel blockade, increased GABAergic neurotransmission and voltage-gated Calcium channels blockade. In addition to these, additional molecular mechanisms appear to be exploited by newer anti-epileptic drugs. Although anticonvulsants represent important and often critical tools for epilepsy treatment, their risk/benefit ratio is suboptimal, often because of their poor target selectivity and of their wide range of side effects. Among the latest anti-epileptic drugs, retigabine (trade name in Europe Trobalt®; trade name in the US Potiga®), the prototypical Kv7 channel modulator, acts by increasing the activity of Kv7.2-5 channels and has been recommended as an adjunctive treatment for drug-resistant partial epilepsies in adults since 2011-2012 (Porter RJ et al., Neurology, 2007, 68(15), 1197-1204). Unfortunately, over the last five years, the use of this drug has been limited because of its important side effects; due to the unfavourable risk/benefit ratio, the manufacturing Company (GSK) has decided to discontinue the commercialization of retigabine, which is no longer available since June 2017 (http://www.ilae.org/Visitors/News/documents/GSK_Retigabine_market_withdrawal.pdf).

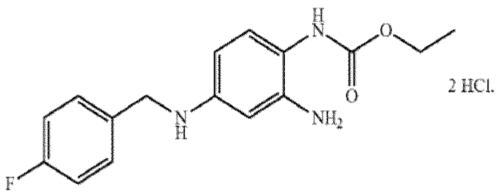

Retigabine has a peculiar pharmacokinetic profile, with a fast absorption after oral administration (1.5-2.0h after administration) but also a fast metabolization, mainly leading to the formation of glucuronic derivatives at N-2 and N-4 (Borlak et al., Metabolism: Clinical and Experimental 2006, 55(6):711-21). Moreover, retigabine is poorly stable under oxidative conditions both during synthesis and in its final form. One of the most characteristic side effects revealed during chronic (> 4 years) therapies is the occurrence of side effects represented by blue coloration of skin, lips, nails and, particularly, retina, due to the accumulation of blue-colored precipitates. These precipitates may represent degradation products of the aniline ring of the retigabine molecular structure catalyzed by oxidation triggered by visible light (Kumar et al., Mol. Pharmacol. 2016, 89(6):667-77). Another side effect is represented by urinary retention, possibly due to the interference of retigabine with Kv7 subunits located in the visceral smooth muscle and regulating bladder contraction (Kv7.4).

Different agonists of the Kv7 channels have been described so far, bearing the chemical features summarised below.

U.S. patent No 5,384,330 describes compounds with the following chemical structure, characterized by the presence of two vicinal amino group, as anticonvulsivants, antipyretics, muscle-relaxants and pheripheral analgesics:

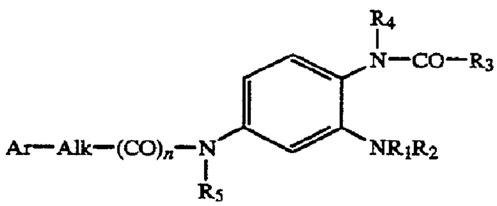

WO 2005/087754 relates to compounds with the following chemical structure, in which two amino groups are placed in para-position on the aromatic ring, one of the two groups is part of an aliphatic cycle, while the two ortho-positions to the other amino group are occupied by R1 and R2 substituents:

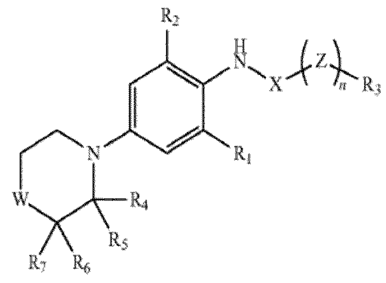

Such derivatives act as openers of the Kv family of potassium ion channels.

WO 2008/024398 refers to the following structures, characterized by a bicyclic-fused structure between aromatic and aliphatic rings, and their effect on modulation of voltage-gated potassium channels:

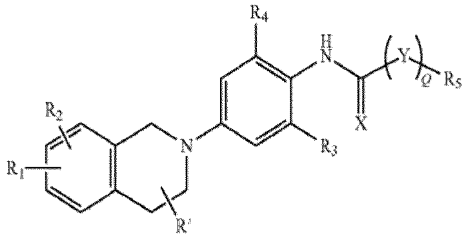

WO 2009/023677 concerns 4,6 bisubstitued-5-aminoindoline and 5-aminoindoles with the following general structure, with the N-5 derivatized as amide, carbamate, thioamide, dithiocarbamate or thiocarbamic ester:

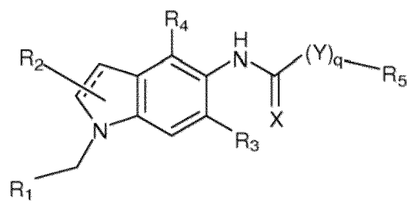

Such compounds are potassium channels modulators.

US 2014/0336252 describes compounds having the following general formula as Kv potassium channel agonists for treating nervous system diseases

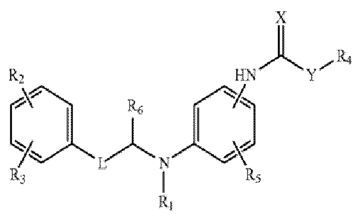

Therefore, there is still a need for the development of new Kv7 modulators characterized by increased pharmacodynamic (increased potency and selectivity), and pharmacokinetic properties (increased distribution in the CNS and stability to oxidation), as well as by reduced side effects and good tolerability compared to current therapeutic options for Kv7-mediated disorders. These novel molecules may be used for the pharmacological treatment of central nervous system diseases (such as epilepsy, neuropathic pain, ischemic stroke and neurodegenerative disorders), peripheral nervous system diseases (such as pain), respiratory system diseases (such as asthma), genitourinary system diseases (such as bladder hyper-reactivity) and cardiovascular diseases (such as hypertension).

## SUMMARY OF THE INVENTION

The present invention provides a novel class of compounds of formula I as shown below, as well as tautomers, salts, solvates, stereoisomers or isotopes thereof.

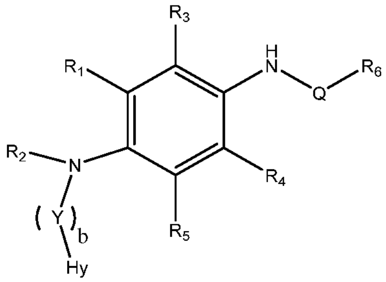

I

Compounds of the present invention were tested by patch-clamp measurements and stability assays as described in the Examples. They were found to act as voltage-gated potassium channels modulators, with $EC_{50}$ values for Kv7.2, Kv7.2/7.3, Kv7.3 and Kv7.4 in the low micromolar range, and to possess desirable stability and photostability properties.

Notably, compounds of the present invention may act as agonists or antagonists of voltage-gated potassium channels. As a matter of fact, the substituent in position $R_6$ is able to modulate the activity of the compounds of general formula I in terms of modulation (agonism/antagonism), potency and efficacy.

Furthermore, the NHQ functionality includes sulfonamide, urea, thiourea and guanidine bonds.

The inventors found an exceptional activity towards potassium channels in comparison with retigabine, herein used as reference compound, which is known to increase the activity of voltage-gated potassium channels Kv7.2-7.5 and has been used in therapy as anticonvulsant. In particular, when compared to retigabine, the compounds object of the present invention have improved pharmacological properties, both pharmacodynamic and pharmacokinetic. Moreover, they can be synthesized using easier and safer chemical procedures and show increased chemical stability than retigabine.

Therefore, compounds with general formula I are able to overcome the main problems limiting the clinical use of retigabine, in particular since they are more stable (chemically and biologically) and less prone to photoinduced oxidation.

The inventors have further observed that compounds of general formula I, in particular IA and IIA as well as IE, IIE, IIIE and IVE, possess a plethora of desiderable properties, including increased potency and stability compared to retigabine.

Another important observation made by the inventors is that the use of a sterically encumbered aliphatic and/or aromatic group in position $R_6$ is useful to switch the activity of the compounds from agonism to antagonism.

Thus, these specific substitutions represent an important feature in some embodiments of this invention to modulate the activity of some compounds, with some substitutions resulting in a blockade of the ionic currents carried by the channel (with the resulting molecules thereby acting as antagonists or blockers of the channels), whereas others enhance the ionic currents carried by the channels (with the resulting molecules thereby acting as agonists or openers). The functional electrophysiologial experiments carried out and whose results are detailed below allow a direct estimate of the pharmacological effects exerted by each drug, which could therefore be classified as a blocker or as an opener with respect to a specific channel target.

Therefore, it is an object of the present invention a compound of general formula I:

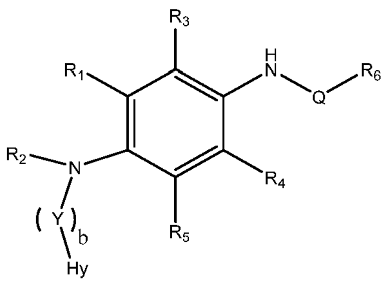

I

or a tautomer, salt, solvate, stereoisomer or isotope thereof,
wherein:

Y is selected from: C1-C10 alkyl, , C1-C10 alkylamino, C1-C10 alkoxy,

b is 1
$R_2$ is an aromatic ring bearing in one or more positions at least one substituent independently selected from the group consisting of: OH, $NO_2$, halogen, $NH_2$, C1-C3 haloalkyl, C1-C3 haloalkoxy, C1-C6 alkylcarbonyl, acetamidyl ,C1-C6 alkyloxy.
$R_1$, $R_3$, $R_4$ and $R_5$ are each independently selected from the group consisting of: H, $NH_2$, OH, halogen, C1-C6 alkyl, C3-C6 cycloalkyl, C2-C6 alkylcarbonyl, C2-C6 alkoxycarbonyl, C1-C6 alkylamino, C2-C6 dialkylamino, C2-C6 alkylaminocarbonyl, C1-C6 alkyloxy, C1-C6 alkylthio, C2-C8 alkenyl, C5-C7 cycloalkenyl, heterocycle, C2-C8 alkynyl and C5-C10 aryl, any of which being optionally substituted by one or more substituents independently selected from: hydroxyl, amine, thiol, carboxyl and halogen;
Q is selected from the group consisting of: C=O, $SO_2$,
when Q is C=O, $R_6$ is selected from: linear or branched C1-C30 alkyl, linear or branched C5-C30 arylalkyl, C3-C8 cycloalkyl, linear or branched C2-C4 alkyl substituted by C3-C8 cycloalkyl C2-C30 alkenyl, C2-C30 alkynyl any of which being optionally substituted with one or more substituents independently selected from: halogen, and cycloalkyl; and
when Q is $SO_2$ $R_6$ is C3-C8 cycloalkyl or aryl and it is optionally substituted with one or more C1-C6 alkyl

In an embodiment, the present invention discloses each one of the following compounds:

| | COMPOUND STRUCTURE | COMPOUND NAME |
|---|---|---|
| 1. | 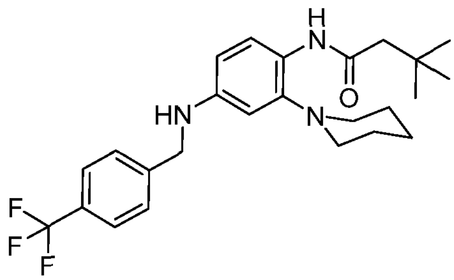 | N-(2-(piperidin-1-yl)-4-((4-(trifluoromethyl)benzyl)amino)p henyl)heptanamide |
| 2. | 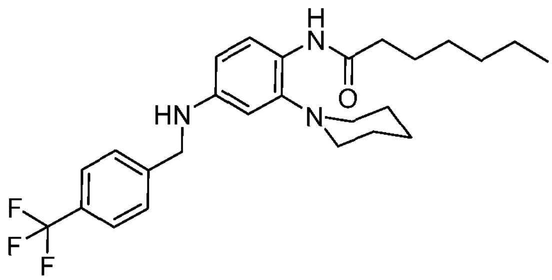 | N-(2-(piperidin-1-yl)-4-((4-(trifluoromethyl)benzyl)amino)p henyl)heptanamide |
| 3. | 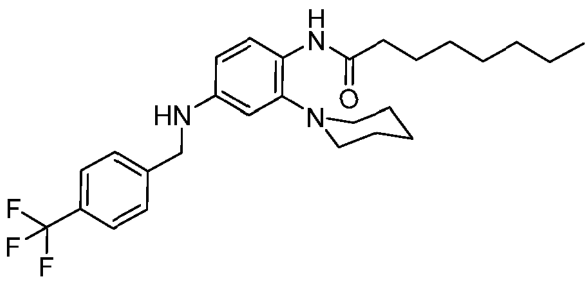 | N-(2-(piperidin-1-yl)-4-((4-(trifluoromethyl)benzyl)amino)p henyl)octanamide |

(continued)

| | COMPOUND STRUCTURE | COMPOUND NAME |
|---|---|---|
| 4. | 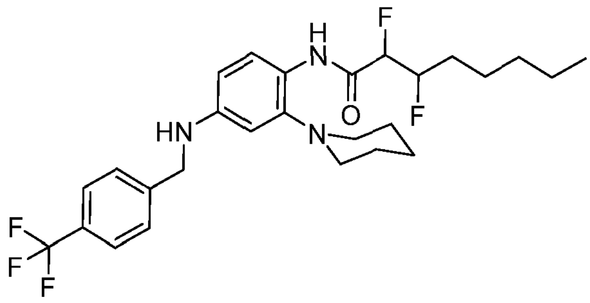 | 2,3-difluoro-N-(2-(piperidin-1-yl)-4-((4-(trifluoromethyl)benzyl)amino)p henyl)octanamide (2R,3 S)-2,3-difluoro-N-(2-(piperidin-1-yl)-4-((4-(trifluoromethyl)benzyl)amino)p henyl)octanamide (2S,3R)-2,3-difluoro-N-(2-(piperidin-1-yl)-4-((4-(trifluoromethyl)benzyl)amino)p henyl)octanamide (2R,3R)-2,3-difluoro-N-(2-(piperidin-1-yl)-4-((4-(trifluoromethyl)benzyl)amino)p henyl)octanamide (2S,3 S)-2,3-difluoro-N-(2-(piperidin-1-yl)-4-((4-(trifluoromethyl)benzyl)amino)p henyl)octanamide |
| 5. | 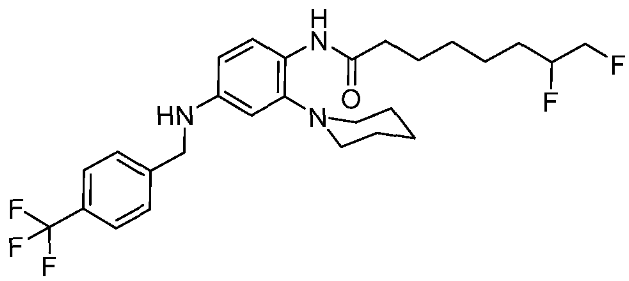 | 7,8-difluoro-N-(2-(piperidin-1-yl)-4-((4-(trifluoromethyl)benzyl)amino)p henyl)octanamide (7R)-7,8-difluoro-N-(2-(piperidin-1-yl)-4-((4-(trifluoromethyl)benzyl)amino)p henyl)octanamide (7S)-7,8-difluoro-N-(2-(piperidin-1-yl)-4-((4-(trifluoromethyl)benzyl)amino)p henyl)octanamide |
| 6. | 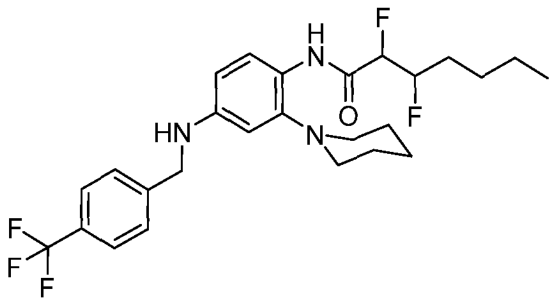 | 2,3-difluoro-N-(2-(piperidin-1-yl)-4-((4-(trifluoromethyl)benzyl)amino)p henyl)heptanamide (2R,3 S)-2,3-difluoro-N-(2-(piperidin-1-yl)-4-((4-(trifluoromethyl)benzyl)amino)p henyl)heptanamide (2S,3R)-2,3-difluoro-N-(2-(piperidin-1-yl)-4-((4-(trifluoromethyl)benzyl) amino)p henyl)heptanamide (2R,3R)-2,3-difluoro-N-(2-(piperidin-1-yl)-4-((4-(trifluoromethyl)benzyl)amino)p henyl)heptanamide (2S,3S)-2,3-difluoro-N-(2-(piperidin-1-yl)-4-((4-(trifluoromethyl)benzyl)amino)p henyl)heptanamide |
| 7. | 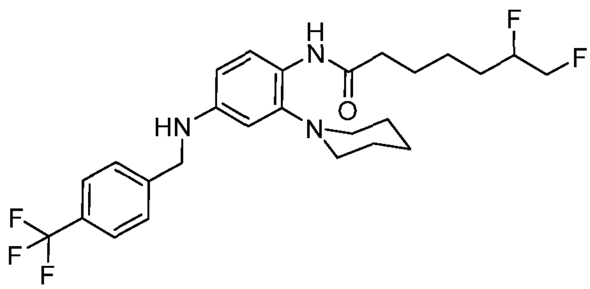 | 6,7-difluoro-N-(2-(piperidin-1-yl)-4-((4-(trifluoromethyl)benzyl)amino)p henyl)heptanamide (6R)-6,7-difluoro-N-(2-(piperidin-1-yl)-4-((4-(trifluoromethyl)benzyl)amino)p henyl)heptanamide (6S)-6,7-difluoro-N-(2-(piperidin-1-yl)-4-((4-(trifluoromethyl)benzyl)amino)p henyl)heptanamide |
| 8. | 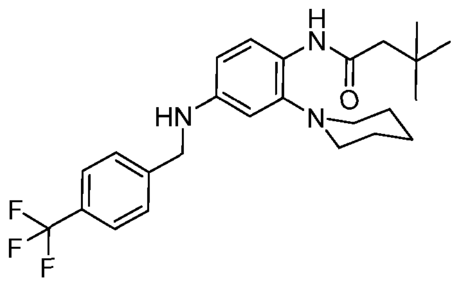 | 3,3-dimethyl-N-(2-(piperidin-1-yl)-4-((4-(trifluoromethyl)benzyl)amino)p henyl)butanamide |

(continued)

| | COMPOUND STRUCTURE | COMPOUND NAME |
|---|---|---|
| 9. | 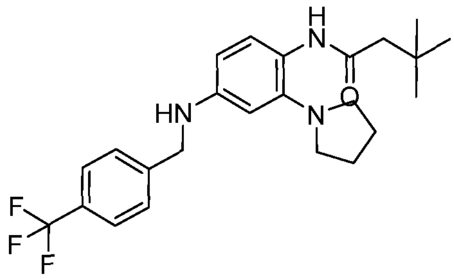 | 3,3-dimethyl-N-(2-(pyrrolidin-1-yl)-4-((4-(trifluoromethyl)benzyl)amino)p henyl)butanamide |
| 10. | 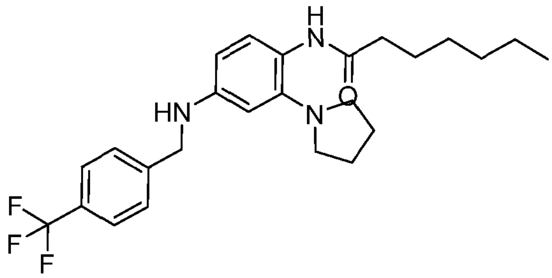 | N-(2-(pyrrolidin-1-yl)-4-((4-(trifluoromethyl)benzyl)amino)p henyl)heptanamide |
| 11. | 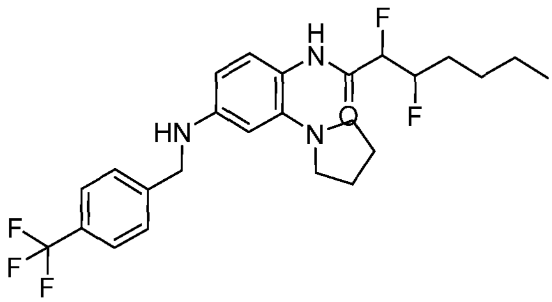 | (2R,3 S)-2,3-difluoro-N-(2-(pyrrolidin-1-yl)-4-((4-(trifluoromethyl)benzyl)amino)p henyl)heptanamide (2S,3R)- 2,3-difluoro-N-(2-(pyrrolidin-1-yl)-4-((4-(trifluoromethyl)benzyl)amino)p henyl)heptanamide (2R,3R)- 2,3-difluoro-N-(2-(pyrrolidin-1-yl)-4-((4-(trifluoromethyl)benzyl) amino)p henyl)heptanamide (2S,3S)- 2,3-difluoro-N-(2-(pyrrolidin-1-yl)-4-((4-(trifluoromethyl)benzyl)amino)p henyl)heptanamide |
| 12. | 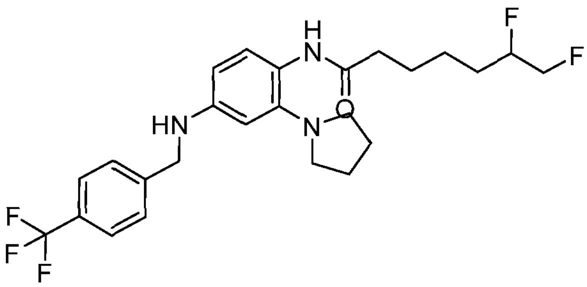 | 6,7-difluoro-N-(2-(pyrrolidin-1-yl)-4-((4-(trifluoromethyl)benzyl)amino)p henyl)heptanamide (6R)-6,7-difluoro-N-(2-(pyrrolidin-1-yl)-4-((4-(trifluoromethyl)benzyl)amino)p henyl)heptanamide (6S)-6,7-difluoro-N-(2-(pyrrolidin-1-yl)-4-((4-(trifluoromethyl)benzyl)amino)p henyl)heptanamide |
| 13. | 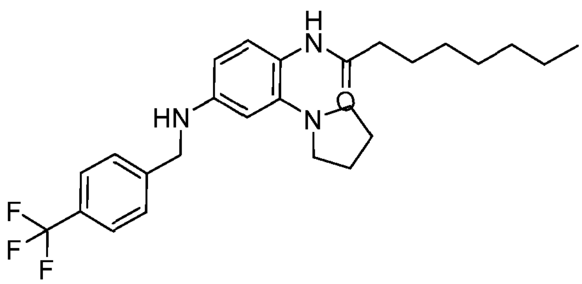 | N-(2-(pyrrolidin-1-yl)-4-((4-(trifluoromethyl)benzyl)amino)p henyl)octanamide |

(continued)

| | COMPOUND STRUCTURE | COMPOUND NAME |
|---|---|---|
| 14. | 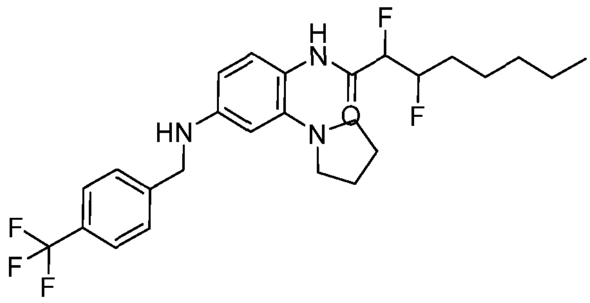 | 2,3-difluoro-N-(2-(pyrrolidin-1-yl)-4-((4-(trifluoromethyl)benzyl)amino)p henyl)octanamide (2R,3 S)-2,3-difluoro-N-(2-(pyrrolidin-1-yl)-4-((4-(trifluoromethyl)benzyl)amino)p henyl)octanamide (2S,3R)-2,3-difluoro-N-(2-(pyrrolidin-1-yl)-4-((4-(trifluoromethyl)benzyl)amino)p henyl)octanamide (2R,3R)-2,3-difluoro-N-(2-(pyrrolidin-1-yl)-4-((4-(trifluoromethyl)benzyl)amino)p henyl)octanamide (2S,3S)-2,3-difluoro-N-(2-(pyrrolidin-1-yl)-4-((4-(trifluoromethyl)benzyl)amino)p henyl)octanamide |
| 15. | 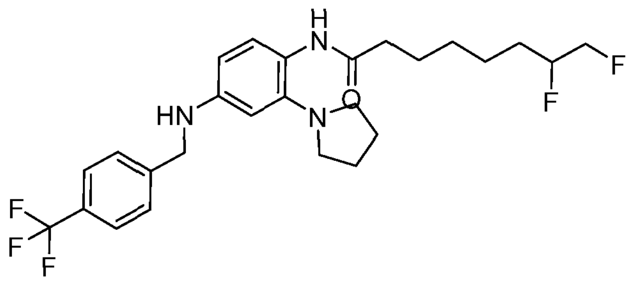 | 7,8-difluoro-N-(2-(pyrrolidin-1-yl)-4-((4-(trifluoromethyl)benzyl)amino)p henyl)octanamide (7R)-7,8-difluoro-N-(2-(pyrrolidin-1-yl)-4-((4-(trifluoromethyl)benzyl)amino)p henyl)octanamide (7S)-7,8-difluoro-N-(2-(pyrrolidin-1-yl)-4-((4-(trifluoromethyl)benzyl)amino)p henyl)octanamide |
| 16. | 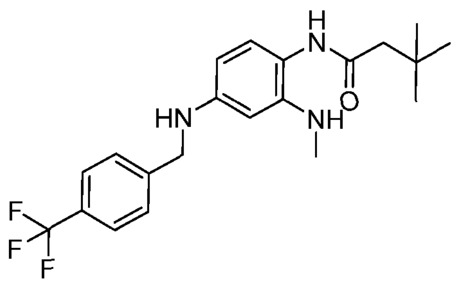 | 3,3-dimethyl-N-(2-(methylamino)-4-((4-(trifluoromethyl)benzyl)amino)p henyl)butanamide |
| 17. | 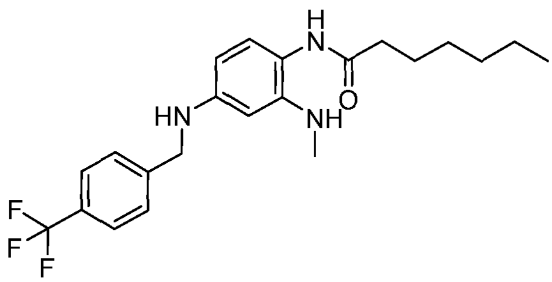 | N-(2-(methylamino)-4-((4-(trifluoromethyl)benzyl)amino)p henyl)heptanamide |
| 18. | 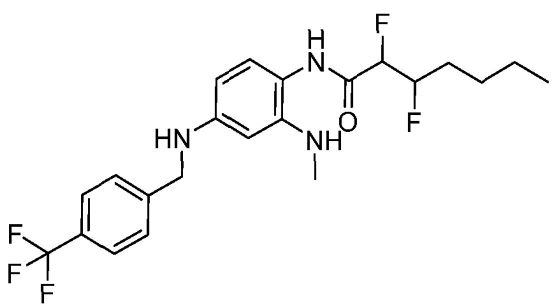 | 2,3-difluoro-N-(2-(methylamino)-4-((4-(trifluoromethyl)benzyl)amino)p henyl)heptanamide (2R, 3S)-2,3-difluoro-N-(2-(methylamino)-4-((4-(trifluoromethyl)benzyl)amino)p henyl)heptanamide (2S, 3R)-2,3-difluoro-N-(2-(methylamino)-4-((4-(trifluoromethyl)benzyl)amino)p henyl)heptanamide (2R, 3R)-2,3-difluoro-N-(2-(methylamino)-4-((4-(trifluoromethyl)benzyl)amino)p henyl)heptanamide (2S, 3S)-2,3-difluoro-N-(2-(methylamino)-4-((4-(trifluoromethyl)benzyl)amino)p henyl)heptanamide |

(continued)

| | COMPOUND STRUCTURE | COMPOUND NAME |
|---|---|---|
| 19. | 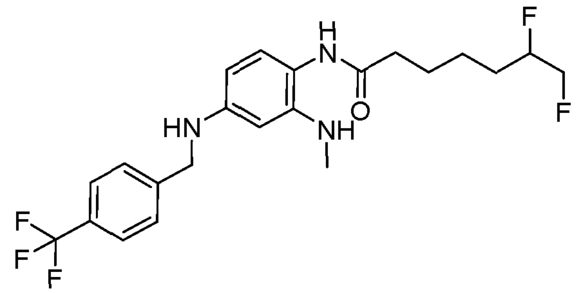 | 6,7-difluoro-N-(2-(methylamino)-4-((4-(trifluoromethyl)benzyl)amino)p henyl)heptanamide (6R)-6,7-difluoro-N-(2-(methylamino)-4-((4-(trifluoromethyl)benzyl)amino)p henyl)heptanamide (6S)-6,7-difluoro-N-(2-(methylamino)-4-((4-(trifluoromethyl)benzyl)amino)p henyl)heptanamide |
| 20. | 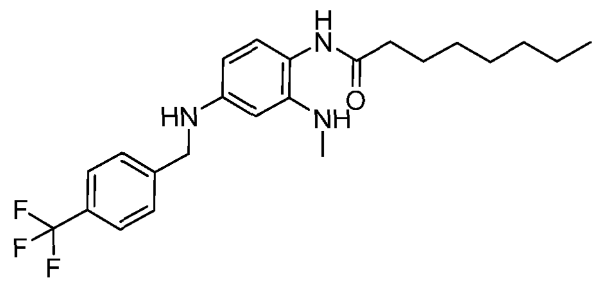 | N-(2-(methylamino)-4-((4-(trifluoromethyl)benzyl)amino)p henyl)octanamide |
| 21. | 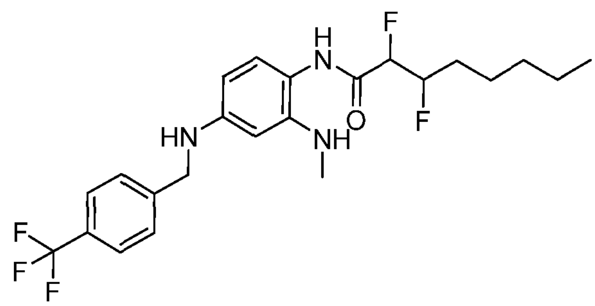 | 2,3-difluoro-N-(2-(methylamino)-4-((4-(trifluoromethyl)benzyl)amino)p henyl)octanamide (2R,3 S)-2,3-difluoro-N-(2-(methylamino)-4-((4-(trifluoromethyl)benzyl)amino)p henyl)octanamide (2S,3R)-2,3-difluoro-N-(2-(methylamino)-4-((4-(trifluoromethyl)benzyl)amino)p henyl)octanamide (2R,3R)-2,3-difluoro-N-(2-(methylamino)-4-((4-(trifluoromethyl)benzyl)amino)p henyl)octanamide (2S,3 S)-2,3-difluoro-N-(2-(methylamino)-4-((4-(trifluoromethyl)benzyl)amino)p henyl)octanamide |
| 22. | 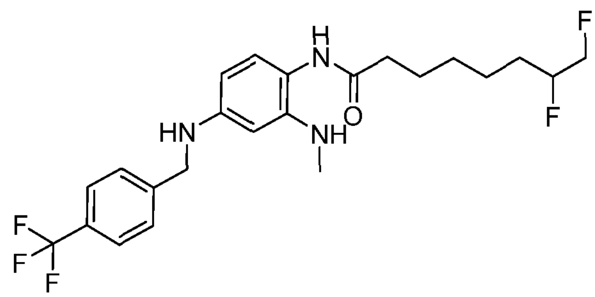 | 7,8-difluoro-N-(2-(methylamino)-4-((4-(trifluoromethyl)benzyl)amino)p henyl)octanamide (7R)-7,8-difluoro-N-(2-(methylamino)-4-((4-(trifluoromethyl)benzyl)amino)p henyl)octanamide (7S)-7,8-difluoro-N-(2-(methylamino)-4-((4-(trifluoromethyl)benzyl)amino)p henyl)octanamide |
| 23. | 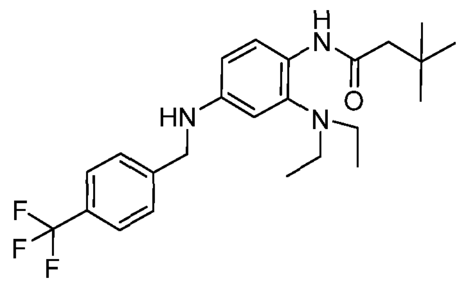 | N-(2-(diethylamino)-4-((4-(trifluoromethyl)benzyl)amino)p henyl)-3,3-dimethylbutanamide |

(continued)

| | COMPOUND STRUCTURE | COMPOUND NAME |
|---|---|---|
| 24. | 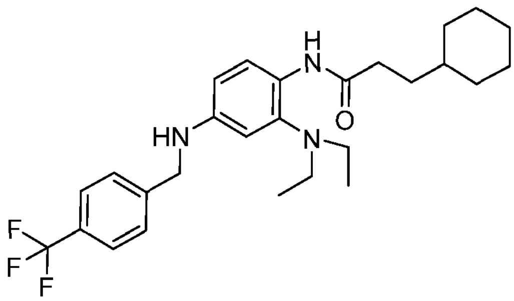 | 3-cyclohexyl-N-(2-(diethylamino)-4-((4-(trifluoromethyl)benzyl)amino)phenyl)propanamide |
| 25. | 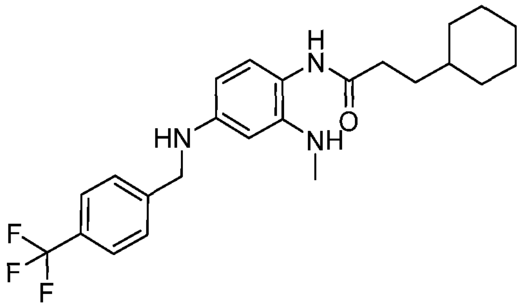 | 3-cyclohexyl-N-(2-(methylamino)-4-((4-(trifluoromethyl)benzyl)amino)phenyl)propanamide |
| 26. | 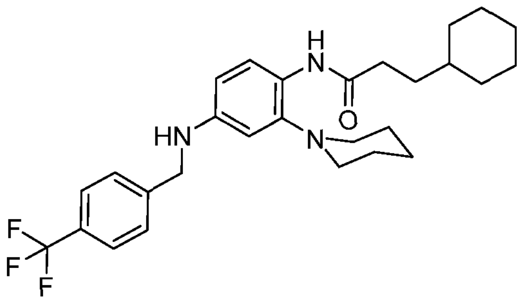 | 3-cyclohexyl-N-(2-(piperidin-1-yl)-4-((4-(trifluoromethyl)benzyl)amino)phenyl)propanamide |
| 27. | 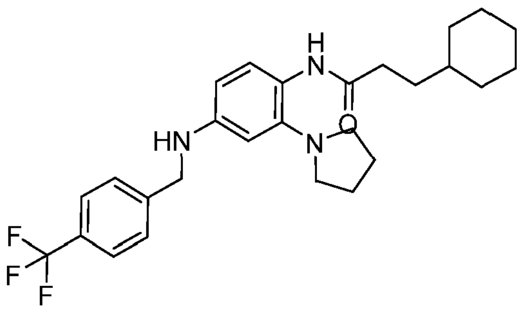 | 3-cyclohexyl-N-(2-(pyrrolidin-1-yl)-4-((4-(trifluoromethyl)benzyl)amino)phenyl)propanamide |
| 28. | 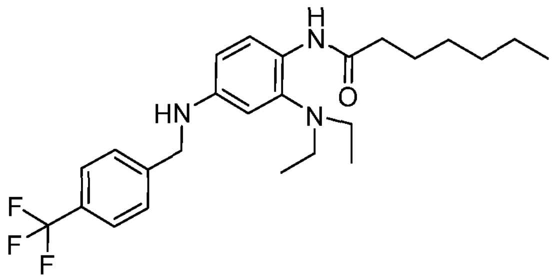 | N-(2-(diethylamino)-4-((4-(trifluoromethyl)benzyl)amino)p henyl)heptanamide |

(continued)

| | COMPOUND STRUCTURE | COMPOUND NAME |
|---|---|---|
| 29. | 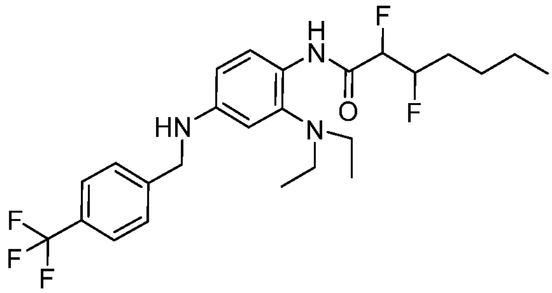 | N-(2-(diethylamino)-4-((4-(trifluoromethyl)benzyl)amino)p henyl)-2,3-difluoroheptanamide (2R,3S)- N-(2-(diethylamino)-4-((4-(trifluoromethyl)benzyl)amino)p henyl)-2,3-difluoroheptanamide (2S,3R)-N-(2-(diethylamino)-4-((4-(trifluoromethyl)benzyl)amino)p henyl)-2,3-difluoroheptanamide (2R,3R)- N-(2-(diethylamino)-4-((4-(trifluoromethyl)benzyl)amino)p henyl)-2,3-difluoroheptanamide (2S,3 S)-N-(2-(diethylamino)-4-((4-(trifluoromethyl)benzyl)amino)p henyl)-2,3-difluoroheptanamide. |
| 30. | 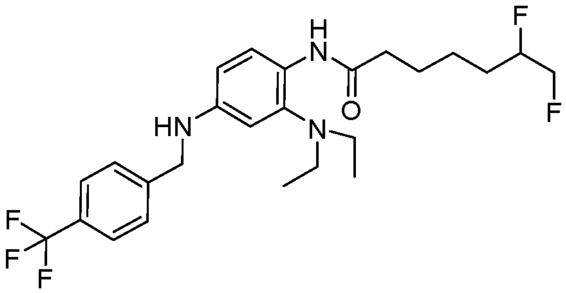 | N-(2-(diethylamino)-4-((4-(trifluoromethyl)benzyl)amino)p henyl)-6,7-difluoroheptanamide (6R)-N-(2-(diethylamino)-4-((4-(trifluoromethyl)benzyl)amino)p henyl)-6,7-difluoroheptanamide (6S)-N-(2-(diethylamino)-4-((4-(trifluoromethyl)benzyl)amino)p henyl)-6,7-difluoroheptanamide |
| 31. | 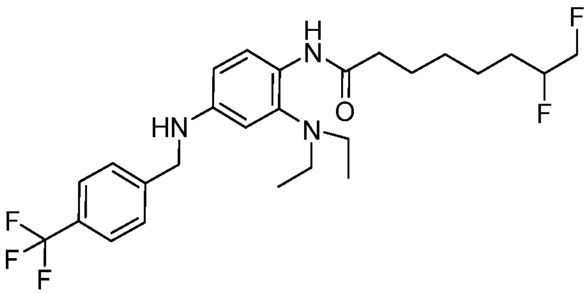 | N-(2-(diethylamino)-4-((4-(trifluoromethyl)benzyl)amino)p henyl)-7,8-difluorooctanamide (7R)-N-(2-(diethylamino)-4-((4-(trifluoromethyl)benzyl)amino)p henyl)-7,8-difluorooctanamide (7S)-N-(2-(diethylamino)-4-((4-(trifluoromethyl)benzyl)amino)p henyl)-7,8-difluorooctanamide |
| 32. | 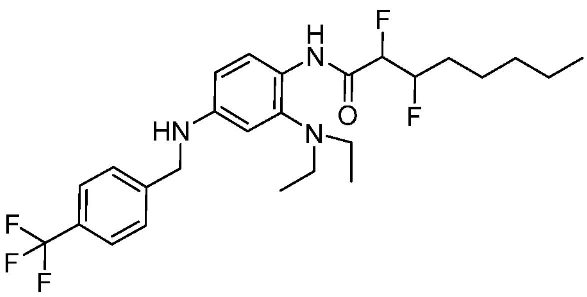 | N-(2-(diethylamino)-4-((4-(trifluoromethyl)benzyl)amino)p henyl)-2,3-difluorooctanamide (2R,3 S)-N-(2-(diethylamino)-4-((4-(trifluoromethyl)benzyl)amino)p henyl)-2,3-difluorooctanamide (2S,3R)-N-(2-(diethylamino)-4-((4-(trifluoromethyl)benzyl)amino)p henyl)-2,3-difluorooctanamide (2R,3R)-N-(2-(diethylamino)-4-((4-(trifluoromethyl)benzyl)amino)p henyl)-2,3-difluorooctanamide (2S,3S)-N-(2-(diethylamino)-4-((4-(trifluoromethyl)benzyl)amino)p henyl)-2,3-difluorooctanamide |

(continued)

| | COMPOUND STRUCTURE | COMPOUND NAME |
|---|---|---|
| 33. | 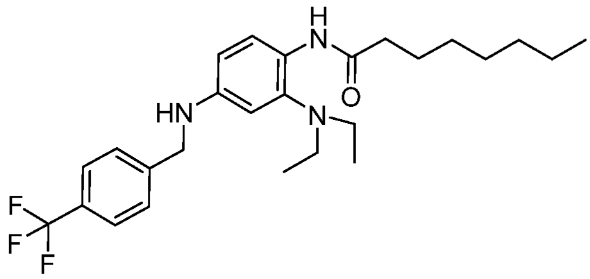 | N-(2-(diethylamino)-4-((4-(trifluoromethyl)benzyl)amino)p henyl)octanamide |
| 34. | 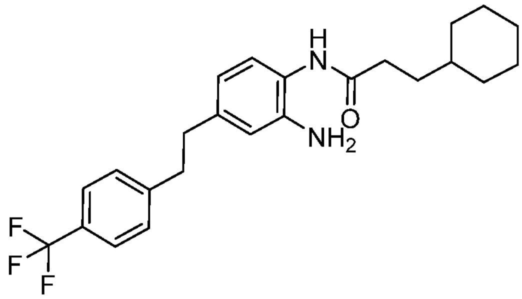 | N-(2-amino-4-(4-(trifluoromethyl)phenethyl)phen yl)-3-cyclohexylpropanamide |
| 35. | 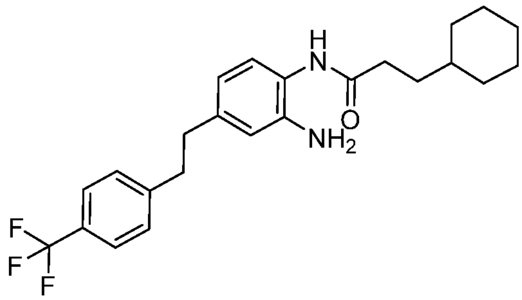 | N-(2-amino-4-(4-(trifluoromethyl)phenethyl)phen yl)-3-cyclohexylpropanamide |
| 36. | 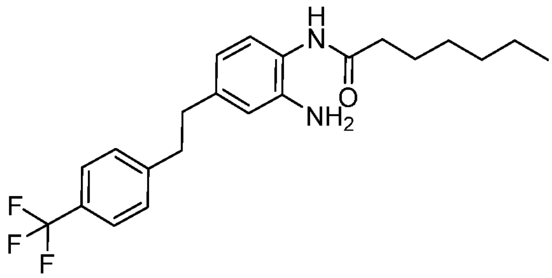 | N-(2-amino-4-(4-(trifluoromethyl)phenethyl)phen yl)heptanamide |
| 37. | 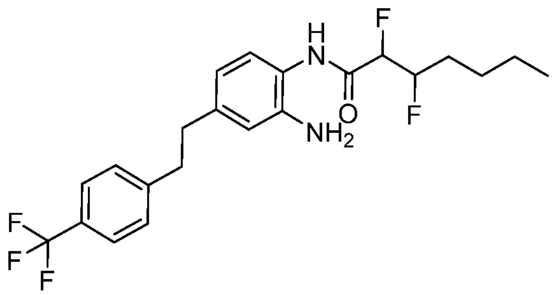 | N-(2-amino-4-(4-(trifluoromethyl)phenethyl)phen yl)-2,3-difluoroheptanamide (2R,3 S)-N-(2-amino-4-(4-(trifluoromethyl)phenethyl)phen yl)-2,3-difluoroheptanamide (2S,3R)-N-(2-amino-4-(4-(trifluoromethyl)phenethyl)phen yl)-2,3-difluoroheptanamide (2R,3R)-N-(2-amino-4-(4-(trifluoromethyl)phenethyl)phen yl)-2,3-difluoroheptanamide (2S,3 S)-N-(2-amino-4-(4-(trifluoromethyl)phenethyl)phen yl)-2,3-difluoroheptanamide |

(continued)

| | COMPOUND STRUCTURE | COMPOUND NAME |
|---|---|---|
| 38. | 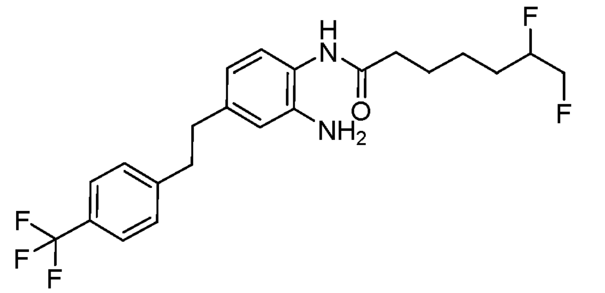 | N-(2-amino-4-(4-(trifluoromethyl)phenethyl)phen yl)-6,7-difluoroheptanamide (6R)-N-(2-amino-4-(4-(trifluoromethyl) phenethyl)phen yl)-6,7-difluoroheptanamide (6S)-N-(2-amino-4-(4-(trifluoromethyl)phenethyl)phen yl)-6,7-difluoroheptanamide |
| 39. | 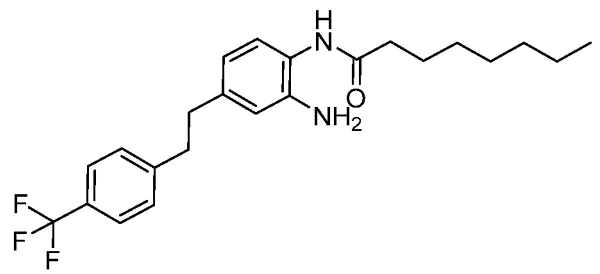 | N-(2-amino-4-(4-(trifluoromethyl)phenethyl)phen yl)octanamide |
| 40. | 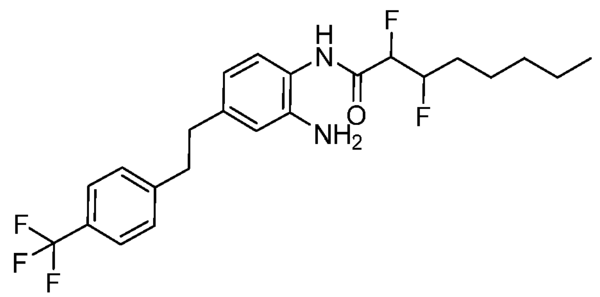 | N-(2-amino-4-(4-(trifluoromethyl)phenethyl)phen yl)-2,3-difluorooctanamide |
| 41. | 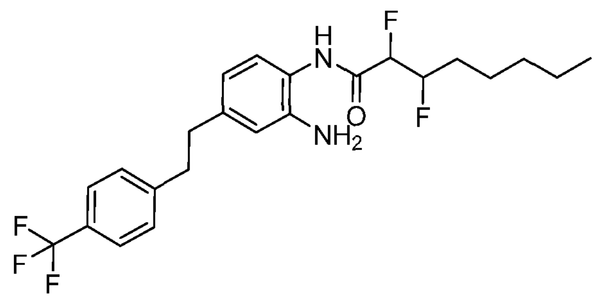 | N-(2-amino-4-(4-(trifluoromethyl)phenethyl)phen yl)-2,3-difluorooctanamide (2R,3 S)-N-(2-amino-4-(4-(trifluoromethyl)phenethyl)phen yl)-2,3-difluoroctanamide (2S,3R)-N-(2-amino-4-(4-(trifluoromethyl)phenethyl)phen yl)-2,3-difluorooctanamide (2R,3R)-N-(2-amino-4-(4-(trifluoromethyl)phenethyl) phen yl)-2,3-difluorooctanamide (2S,3 S)-N-(2-amino-4-(4-(trifluoromethyl)phenethyl)phen yl)-2,3-difluorooctanamide |
| 42. | 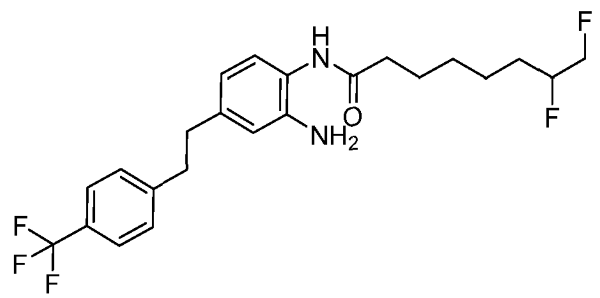 | N-(2-amino-4-(4-(trifluoromethyl)phenethyl)phen yl)-7,8-difluorooctanamide (7R)-N-(2-amino-4-(4-(trifluoromethyl) phenethyl)phen yl)-7,8-difluorooctanamide (7S)-N-(2-amino-4-(4-(trifluoromethyl)phenethyl)phen yl)-7,8-difluoroctanamide |

(continued)

| | COMPOUND STRUCTURE | COMPOUND NAME |
|---|---|---|
| 43. | 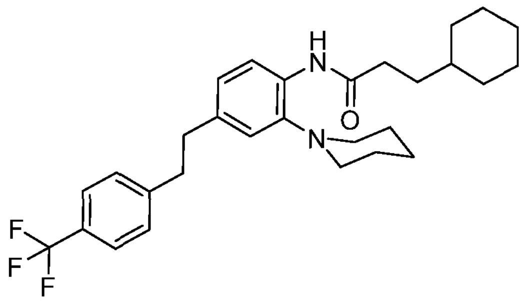 | 3-cyclohexyl-N-(2-(piperidin-1-yl)-4-(4-(trifluoromethyl)phenethyl)phenyl)propanamide |
| 44. | 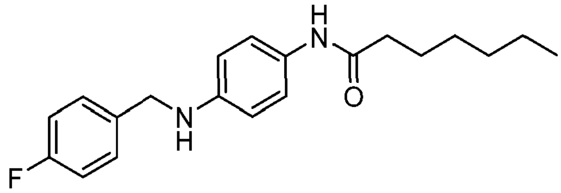 | N-(4-((4-fluorobenzyl)amino)phenyl)hept anamide |
| 45. | 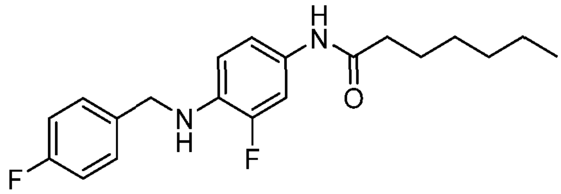 | N-(3-fluoro-4-((4-fluorobenzyl)amino) phenyl)hept anamide |
| 46. | 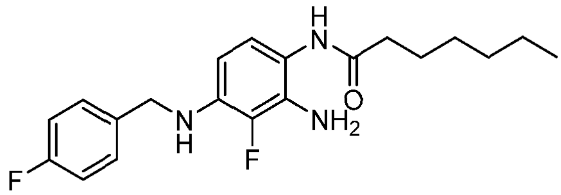 | N-(2-amino-3-fluoro-4-((4-fluorobenzyl) amino)phenyl)hept anamide |
| 47. | 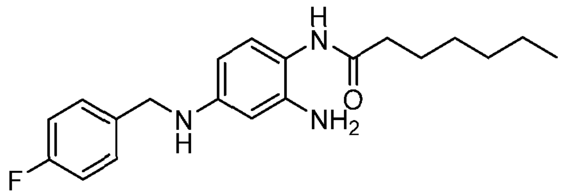 | N-(2-amino-4-((4-fluorobenzyl)amino) phenyl)hept anamide |
| 48. | 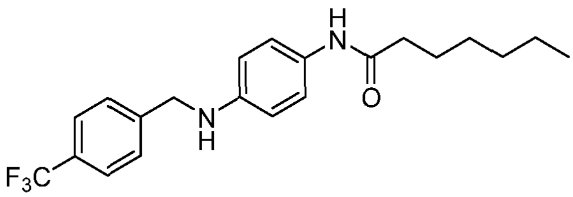 | N-(4-((4-(trifluoromethyl)benzyl)amino)p henyl)heptanamide |
| 49. | 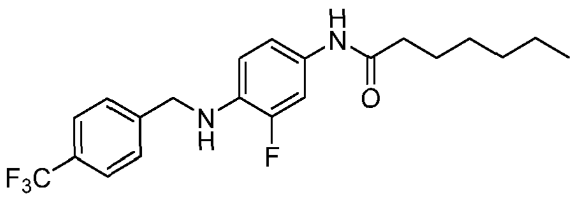 | N-(3-fluoro-4-((4-(trifluoromethyl)benzyl) amino)p henyl)heptanamide |

(continued)

| | COMPOUND STRUCTURE | COMPOUND NAME |
|---|---|---|
| 50. | 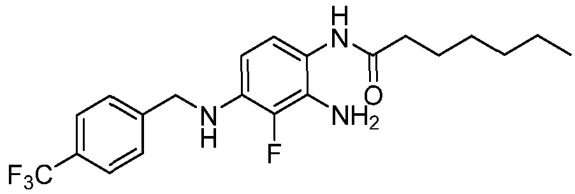 | N-(2-amino-3-fluoro-4-((4-(trifluoromethyl)benzyl)amino)p henyl)heptanamide |
| 51. | 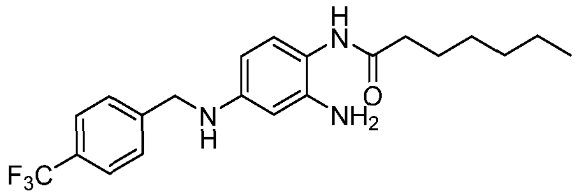 | N-(2-amino-4-((4-(trifluoromethyl)benzyl) amino)p henyl)heptanamide |
| 52. | 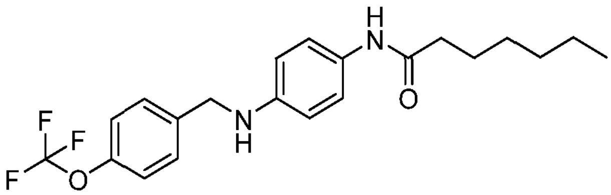 | N-(4-((4-(trifluoromethoxy)benzyl)amino ) phenyl)heptanamide |
| 53. | 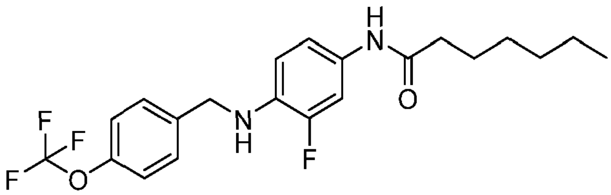 | N-(3-fluoro-4-((4-(trifluoromethoxy)benzyl)amino )phenyl)heptanamide |
| 54. | 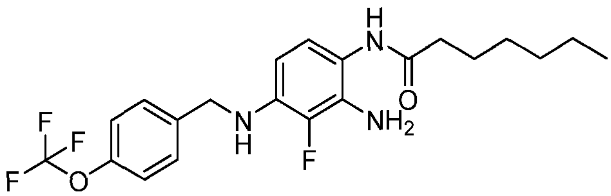 | N-(2-amino-3-fluoro-4-((4-(trifluoromethoxy)benzyl)amino )phenyl)heptanamide |
| 55. | 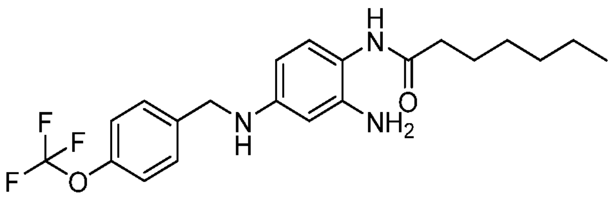 | N-(2-amino-4-((4-(trifluoromethoxy)benzyl)amino )phenyl)heptanamide |
| 56. | 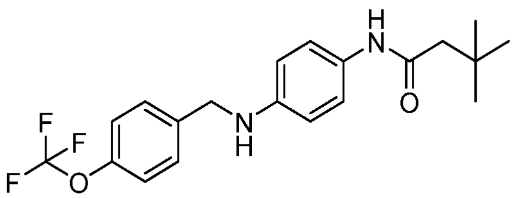 | 3,3-dimethyl-N-(4-((4-(trifluoromethoxy) benzyl)amino )phenyl)butanamide |
| 57. | 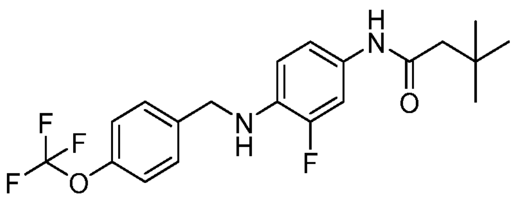 | N-(3-fluoro-4-((4-(trifluoromethoxy)benzyl)amino )phenyl)-3,3-dimethylbutanamide |

(continued)

| | COMPOUND STRUCTURE | COMPOUND NAME |
|---|---|---|
| 58. | 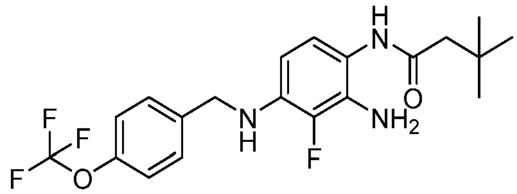 | N-(2-amino-3-fluoro-4-((4-(trifluoromethoxy)benzyl)amino )phenyl)-3,3-dimethylbutanamide |
| 59. | 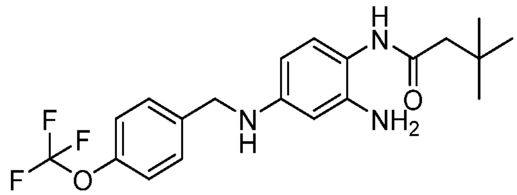 | N-(2-amino-4-((4-(trifluoromethoxy)benzyl)amino )phenyl)-3,3-dimethylbutanamide |
| 60. | 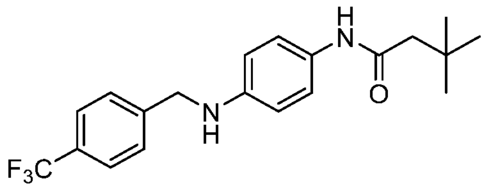 | 3,3-dimethyl-N-(4-((4-(trifluoromethyl) benzyl)amino)p henyl)butanamide |
| 61. | 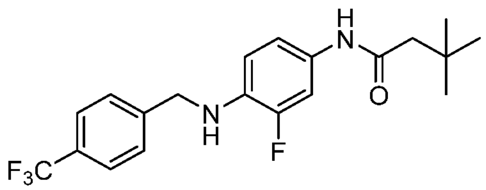 | N-(3-fluoro-4-((4-(trifluoromethyl)benzyl) amino)p henyl)-3,3-dimethylbutanamide |
| 62. | 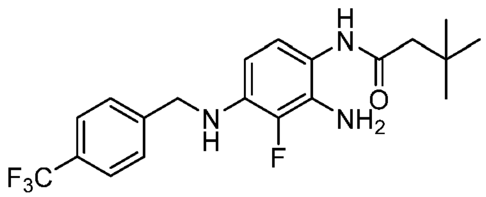 | N-(2-amino-3-fluoro-4-((4-(trifluoromethyl)benzyl)amino)p henyl)-3,3-dimethylbutanamide |
| 63. | 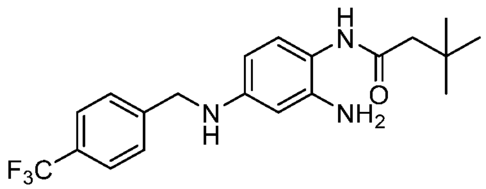 | N-(2-amino-4-((4-(trifluoromethyl)benzyl) amino)p henyl)-3,3-dimethylbutanamide |
| 64. | 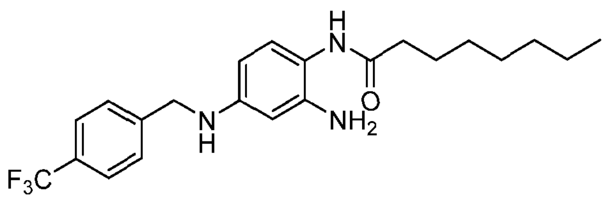 | N-(2-amino-4-((4-(trifluoromethyl)benzyl) amino)p henyl)octanamide |
| 65. | 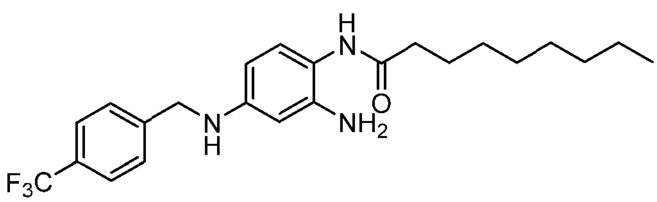 | N-(2-amino-4-((4-(trifluoromethyl)benzyl) amino)p henyl)nonanamide |

(continued)

| | COMPOUND STRUCTURE | COMPOUND NAME |
|---|---|---|
| 66. | 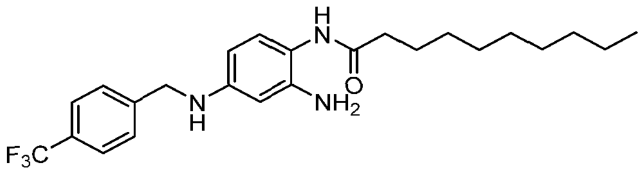 | N-(2-amino-4-((4-(trifluoromethyl)benzyl) amino)p henyl)decanamide |
| 67. | 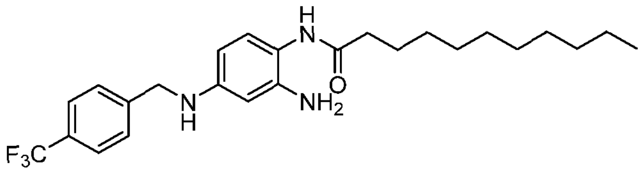 | N-(2-amino-4-((4-(trifluoromethyl)benzyl) amino)p henyl)undecanamide |
| 68. | 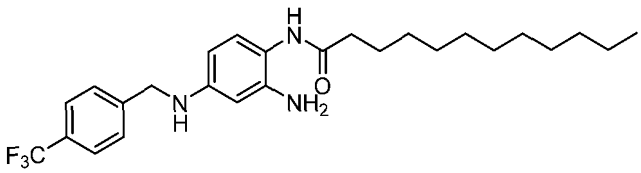 | N-(2-amino-4-((4-(trifluoromethyl)benzyl) amino)p henyl)dodecanamide |
| 69. | 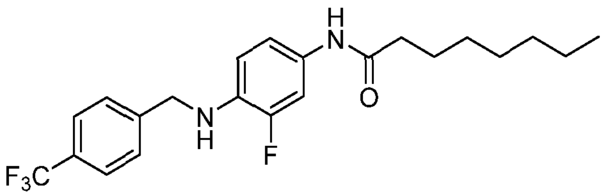 | N-(3-fluoro-4-((4-(trifluoromethyl)benzyl) amino)p henyl)octanamide |
| 70. | 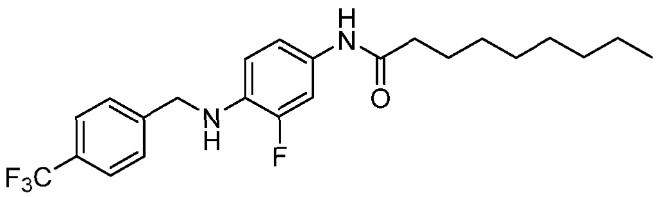 | N-(3-fluoro-4-((4-(trifluoromethyl)benzyl) amino)p henyl)nonanamide |
| 71. | 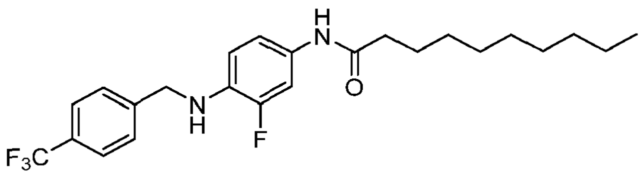 | N-(3-fluoro-4-((4-(trifluoromethyl)benzyl) amino)p henyl)decanamide |
| 72. | 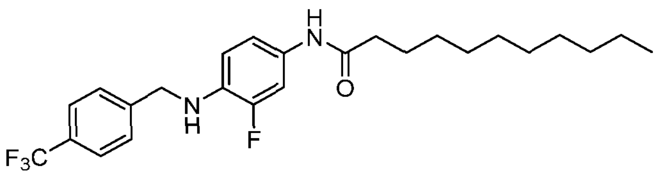 | N-(3-fluoro-4-((4-(trifluoromethyl)benzyl) amino)p henyl)undecanamide |
| 73. | 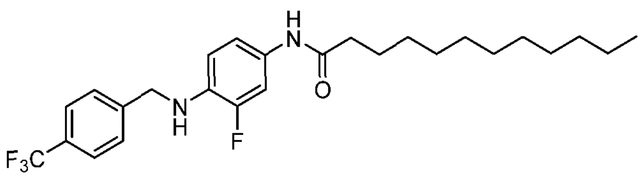 | N-(3-fluoro-4-((4-(trifluoromethyl)benzyl) amino)p henyl)dodecanamide |

(continued)

| | COMPOUND STRUCTURE | COMPOUND NAME |
|---|---|---|
| 74. | 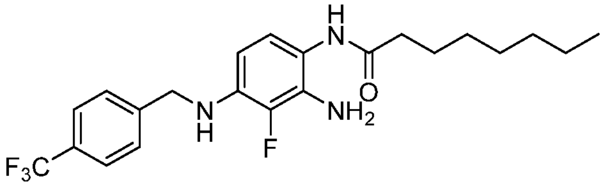 | N-(2-amino-3-fluoro-4-((4-(trifluoromethyl)benzyl)amino)p henyl)octanamide |
| 75. | 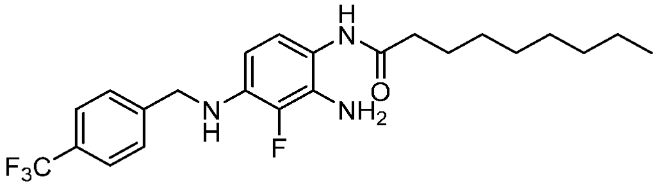 | N-(2-amino-3-fluoro-4-((4-(trifluoromethyl)benzyl)amino)p henyl)nonana-mide |
| 76. | 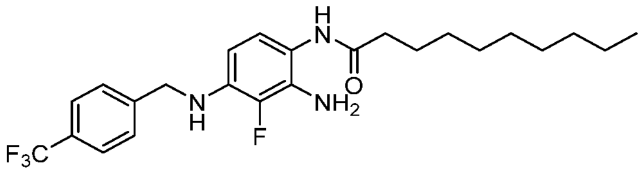 | N-(2-amino-3-fluoro-4-((4-(trifluoromethyl)benzyl)amino)p henyl)decana-mide |
| 77. | 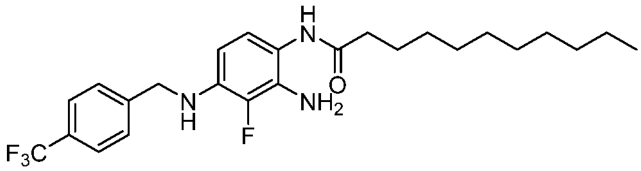 | N-(2-amino-3-fluoro-4-((4-(trifluoromethyl)benzyl)amino)p henyl)undecana-mide |
| 78. | 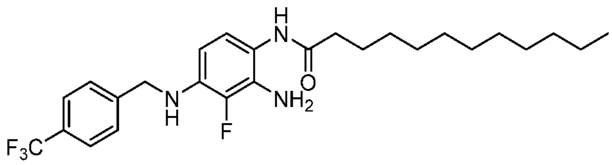 | N-(2-amino-3-fluoro-4-((4-(trifluoromethyl)benzyl)amino)p henyl)dodecana-mide |
| 79. | 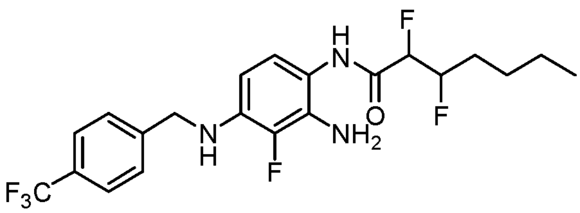 | N-(2-amino-3-fluoro-4-((4-(trifluoromethyl)benzyl)amino)p henyl)-2,3-difluoroheptanamide (2R,3 S)-N-(2-amino-3-fluoro-4-((4-(trifluoromethyl)benzyl)amino)p henyl)-2,3-difluoroheptanamide (2S,3R)-N-(2-amino-3-fluoro-4-((4-(trifluoromethyl)benzyl)amino)p henyl)-2,3-difluoroheptanamide (2R,3R)-N-(2-amino-3-fluoro-4-((4-(trifluoromethyl)benzyl) amino)p henyl)-2,3-difluoroheptanamide (2S,3S)-N-(2-amino-3-fluoro-4-((4-(trifluoromethyl)benzyl)amino)p henyl)-2,3-difluoroheptanamide racemic mixtures thereof |

(continued)

| | COMPOUND STRUCTURE | COMPOUND NAME |
|---|---|---|
| 80. | 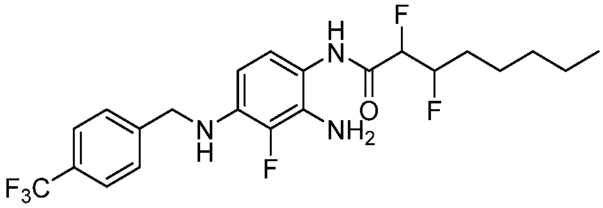 | N-(2-amino-3-fluoro-4-((4-(trifluoromethyl)benzyl)amino)p henyl)-2,3-difluorooctanamide (2R,3 S)-N-(2-amino-3-fluoro-4-((4-(trifluoromethyl)benzyl)amino)p henyl)-2,3-difluorooctanamide (2S,3R)-N-(2-amino-3-fluoro-4-((4-(trifluoromethyl)benzyl)amino)p henyl)-2,3-difluorooctanamide (2R,3R)-N-(2-amino-3-fluoro-4-((4-(trifluoromethyl)benzyl) amino)p henyl)-2,3-difluorooctanamide (2S,3S)-N-(2-amino-3-fluoro-4-((4-(trifluoromethyl)benzyl)amino)p henyl)-2,3-difluorooctanamide racemic mixtures thereof |
| 81. | 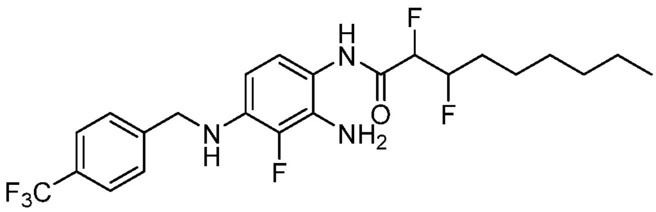 | N-(2-amino-3-fluoro-4-((4-(trifluoromethyl)benzyl)amino)p henyl)-2,3-difluorononanamide (2R,3S)-N-(2-amino-3-fluoro-4-((4-(trifluoromethyl)benzyl)amino)p henyl)-2,3-difluorononanamide (2S,3R)-N-(2-amino-3-fluoro-4-((4-(trifluoromethyl)benzyl)amino)p henyl)-2,3-difluorononanamide (2R,3R)-N-(2-amino-3-fluoro-4-((4-(trifluoromethyl)benzyl) amino)p henyl)-2,3-difluorononanamide (2S,3S)-N-(2-amino-3-fluoro-4-((4-(trifluoromethyl)benzyl)amino)p henyl)-2,3-difluorononanamide racemic mixtures thereof |
| 82. | 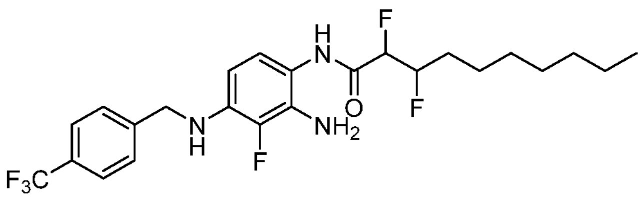 | N-(2-amino-3-fluoro-4-((4-(trifluoromethyl)benzyl)amino)p henyl)-2,3-difluorodecanamide (2R,3S)-N-(2-amino-3-fluoro-4-((4-(trifluoromethyl)benzyl)amino)p henyl)-2,3-difluorodecanamide (2S,3R)-N-(2-amino-3-fluoro-4-((4-(trifluoromethyl)benzyl)amino)p henyl)-2,3-difluorodecanamide (2R,3R)-N-(2-amino-3-fluoro-4-((4-(trifluoromethyl)benzyl) amino)p henyl)-2,3-difluorodecanamide (2S,3S)-N-(2-amino-3-fluoro-4-((4-(trifluoromethyl)benzyl)amino)p henyl)-2,3-difluorodecanamide racemic mixtures thereof |

(continued)

| | COMPOUND STRUCTURE | COMPOUND NAME |
|---|---|---|
| 83. | 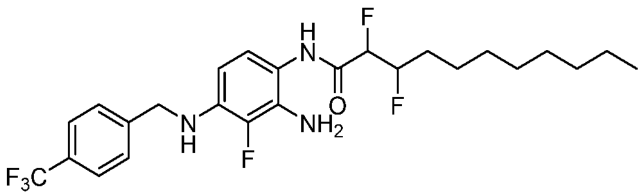 | N-(2-amino-3-fluoro-4-((4-(trifluoromethyl)benzyl)amino)p henyl)-2,3-difluoroundecanamide (2R,3 S)-N-(2-amino-3-fluoro-4-((4-(trifluoromethyl)benzyl)amino)p henyl)-2,3-difluoroundecanamide (2S,3R)-N-(2-amino-3-fluoro-4-((4-(trifluoromethyl)benzyl)amino)p henyl)-2,3-difluoroundecanamide (2R,3R)-N-(2-amino-3-fluoro-4-((4-(trifluoromethyl)benzyl) amino)p henyl)-2,3-difluoroundecanamide (2S,3S)-N-(2-amino-3-fluoro-4-((4-(trifluoromethyl)benzyl)amino)p henyl)-2,3-difluoroundecanamide racemic mixtures thereof |
| 84. | 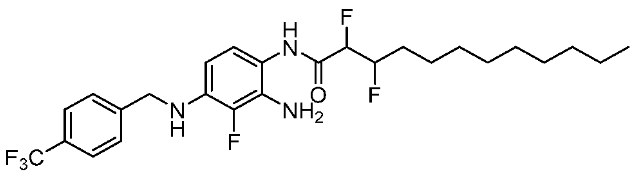 | N-(2-amino-3-fluoro-4-((4-(trifluoromethyl)benzyl)amino)p henyl)-2,3-difluorododecanamide (2R,3 S)-N-(2-amino-3-fluoro-4-((4-(trifluoromethyl)benzyl)amino)p henyl)-2,3-difluorododecanamide (2S,3R)-N-(2-amino-3-fluoro-4-((4-(trifluoromethyl)benzyl)amino)p henyl)-2,3-difluorododecanamide (2R,3R)-N-(2-amino-3-fluoro-4-((4-(trifluoromethyl)benzyl) amino)p henyl)-2,3-difluorododecanamide (2S,3S)-N-(2-amino-3-fluoro-4-((4-(trifluoromethyl)benzyl)amino)p henyl)-2,3-difluorododecanamide racemic mixtures thereof |
| 85. | 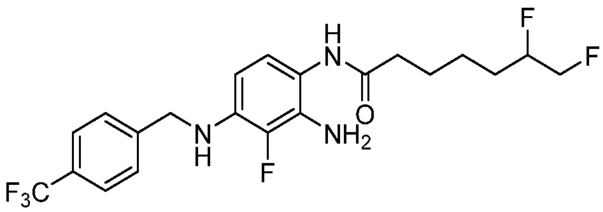 | N-(2-amino-3-fluoro-4-((4-(trifluoromethyl)benzyl)amino)p henyl)-6,7-difluoroheptanamide (6R)-N-(2-amino-3-fluoro-4-((4-(trifluoromethyl)benzyl)amino)p henyl)-6,7-difluoroheptanamide (6S)-N-(2-amino-3-fluoro-4-((4-(trifluoromethyl)benzyl)amino)p henyl)-6,7-difluoroheptanamide racemic mixtures thereof |
| 86. | 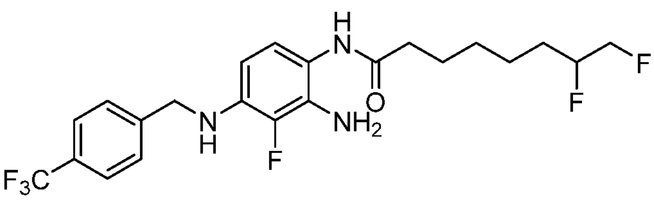 | N-(2-amino-3-fluoro-4-((4-(trifluoromethyl)benzyl)amino)p henyl)-6,7-difluorooctanamide (6R)-N-(2-amino-3-fluoro-4-((4-(trifluoromethyl)benzyl)amino)p henyl)-6,7-difluorooctanamide (6S)-N-(2-amino-3-fluoro-4-((4-(trifluoromethyl)benzyl)amino)p henyl)-6,7-difluorooctanamide racemic mixtures thereof |
| 87. | 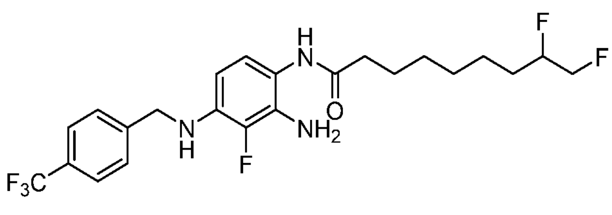 | N-(2-amino-3-fluoro-4-((4-(trifluoromethyl)benzyl)amino)p henyl)-8,9-difluorononanamide (8R)-N-(2-amino-3-fluoro-4-((4-(trifluoromethyl)benzyl)amino)p henyl)-8,9-difluorononanamide (8S)-N-(2-amino-3-fluoro-4-((4-(trifluoromethyl)benzyl)amino)p henyl)-8,9-difluorononanamide racemic mixtures thereof |

(continued)

| | COMPOUND STRUCTURE | COMPOUND NAME |
|---|---|---|
| 88. | 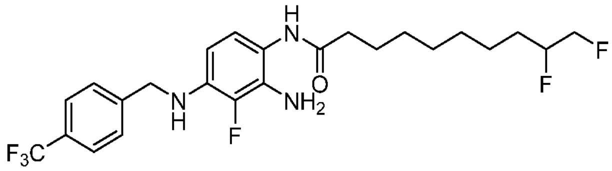 | N-(2-amino-3-fluoro-4-((4-(trifluoromethyl)benzyl)amino)p henyl)-9,10-difluorodecanamide (9R)-N-(2-amino-3-fluoro-4-((4-(trifluoromethyl)benzyl)amino)p henyl)-9,10-difluorodecanamide (9S)-N-(2-amino-3-fluoro-4-((4-(trifluoromethyl)benzyl)amino)p henyl)-9,10-difluorodecanamide racemic mixtures thereof |
| 89. | 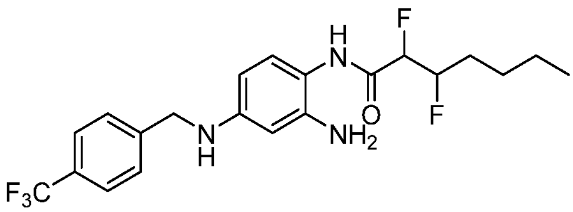 | N-(2-amino-4-((4-(trifluoromethyl)benzyl) amino)p henyl)-2,3-difluoroheptanamide (2R,3 S)-N-(2-amino-4-((4-(trifluoromethyl)benzyl)amino)p henyl)-2,3-difluoroheptanamide (2S,3R)-N-(2-amino-4-((4-(trifluoromethyl)benzyl)amino)p henyl)-2,3-difluoroheptanamide (2R,3R)-N-(2-amino-4-((4-(trifluoromethyl)benzyl)amino)p henyl)-2,3-difluoroheptanamide (2S,3 S)-N-(2-amino-4-((4-(trifluoromethyl)benzyl)amino)p henyl)-2,3-difluoroheptanamide racemic mixtures thereof |
| 90. | 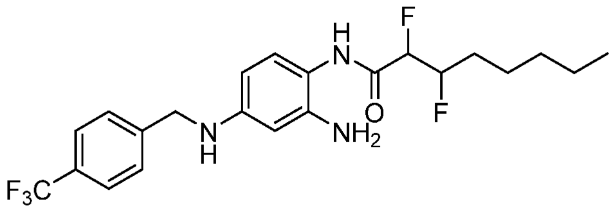 | N-(2-amino-4-((4-(trifluoromethyl)benzyl) amino)p henyl)-2,3-difluorooctanamide (2R,3 S)-N-(2-amino-4-((4-(trifluoromethyl)benzyl)amino)p henyl)-2,3-difluorooctanamide (2S,3R)-N-(2-amino-4-((4-(trifluoromethyl)benzyl)amino)p henyl)-2,3-difluorooctanamide (2R,3R)-N-(2-amino-4-((4-(trifluoromethyl)benzyl)amino)p henyl)-2,3-difluorooctanamide (2S,3 S)-N-(2-amino-4-((4-(trifluoromethyl)benzyl)amino)p henyl)-2,3-difluorooctanamide racemic mixtures thereof |
| 91. | 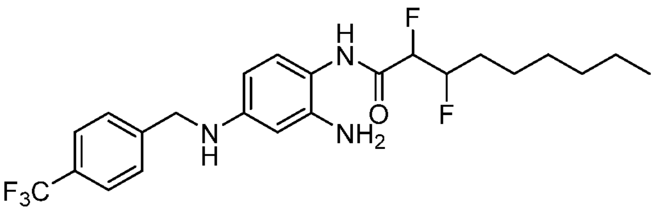 | N-(2-amino-4-((4-(trifluoromethyl)benzyl) amino)p henyl)-2,3-difluorononanamide (2R,3 S)-N-(2-amino-4-((4-(trifluoromethyl)benzyl)amino)p henyl)-2,3-difluorononanamide (2S,3R)-N-(2-amino-4-((4-(trifluoromethyl)benzyl)amino)p henyl)-2,3-difluorononanamide (2R,3R)-N-(2-amino-4-((4-(trifluoromethyl)benzyl)amino)p henyl)-2,3-difluorononanamide (2S,3 S)-N-(2-amino-4-((4-(trifluoromethyl)benzyl)amino)p henyl)-2,3-difluorononanamide racemic mixtures thereof |

(continued)

| | COMPOUND STRUCTURE | COMPOUND NAME |
|---|---|---|
| 92. | 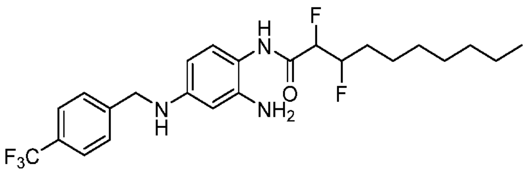 | N-(2-amino-4-((4-(trifluoromethyl)benzyl) amino)p henyl)-2,3-difluorodecanamide (2R,3 S)- N-(2-amino-4-((4-(trifluoromethyl)benzyl)amino)p henyl)-2,3-difluorodecanamide (2S,3R)- N-(2-amino-4-((4-(trifluoromethyl)benzyl)amino)p henyl)-2,3-difluorodecanamide (2R,3R)- N-(2-amino-4-((4-(trifluoromethyl)benzyl) amino)p henyl)-2,3-difluorodecanamide (2S,3 S)- N-(2-amino-4-((4-(trifluoromethyl)benzyl)amino)p henyl)-2,3-difluorodecanamide racemic mixtures thereof |
| 93. | 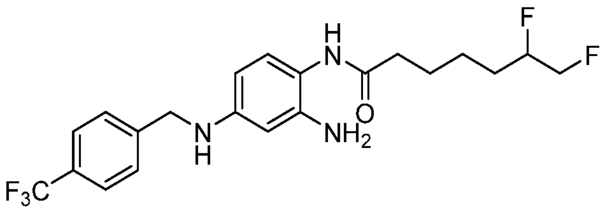 | N-(2-amino-4-((4-(trifluoromethyl)benzyl) amino)p henyl)-6,7-difluoroheptanamide (6R)-N-(2-amino-4-((4-(trifluoromethyl) benzyl)amino)p henyl)-6,7-difluoroheptanamide (6S)-N-(2-amino-4-((4-(trifluoromethyl)benzyl)amino)p henyl)-6,7-difluoroheptanamide racemic mixtures thereof |
| 94. | 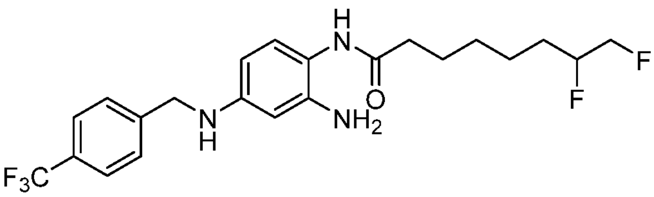 | N-(2-amino-4-((4-(trifluoromethyl)benzyl) amino)p henyl)-7,8-difluorooctanamide (7R)-N-(2-amino-4-((4-(trifluoromethyl) benzyl)amino)p henyl)-7,8-difluorooctanamide (7S)-N-(2-amino-4-((4-(trifluoromethyl)benzyl)amino)p henyl)-7,8-difluorooctanamide racemic mixtures thereof |
| 95. | 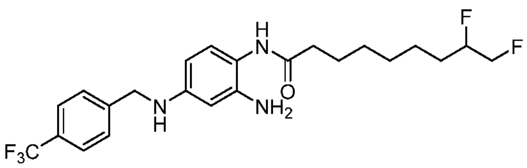 | N-(2-amino-3-fluoro-4-((4-(trifluoromethyl)benzyl)amino)p henyl)-6,7-difluorononanamide (6R)-N-(2-amino-3-fluoro-4-((4-(trifluoromethyl)benzyl)amino)p henyl)-6,7-difluorononanamide (6S)-N-(2-amino-3-fluoro-4-((4-(trifluoromethyl)benzyl)amino)p henyl)-6,7-difluorononanamide racemic mixtures thereof |
| 96. | 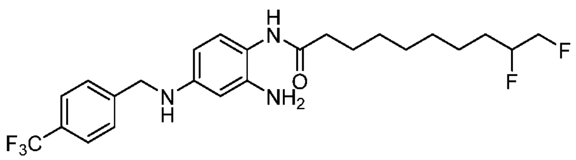 | N-(2-amino-3-fluoro-4-((4-(trifluoromethyl)benzyl)amino)p henyl)-6,7-difluorododecanamide (6R)-N-(2-amino-3-fluoro-4-((4-(trifluoromethyl)benzyl)amino)p henyl)-6,7-difluorododecanamide (6S)-N-(2-amino-3-fluoro-4-((4-(trifluoromethyl)benzyl)amino)p henyl)-6,7-difluorododecanamide racemic mixtures thereof |
| 97. | 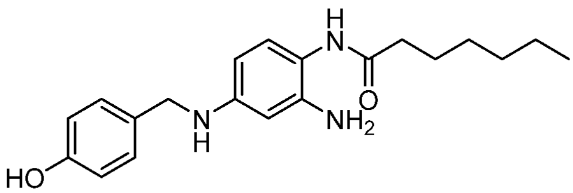 | N-(2-amino-4-((4-hydroxybenzyl)amino) phenyl)he ptanamide |

(continued)

| | COMPOUND STRUCTURE | COMPOUND NAME |
|---|---|---|
| 98. | 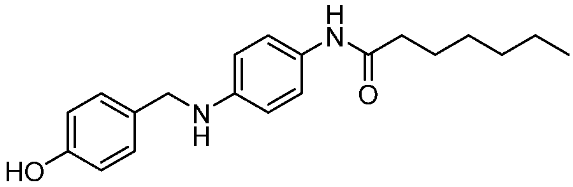 | N-(4-((4-hydroxybenzyl)amino)phenyl)he ptanamide |
| 99. | 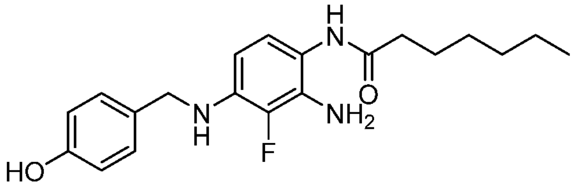 | N-(2-amino-3-fluoro-4-((4-hydroxybenzyl)amino)phenyl)he ptanamide |
| 100. | 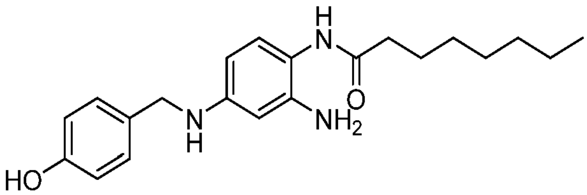 | N-(2-amino-4-((4-hydroxybenzyl)amino) phenyl)oc tanamide |
| 101. | 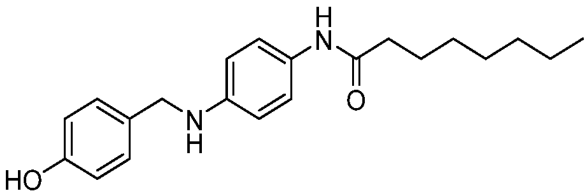 | N-(4-((4-hydroxybenzyl)amino)phenyl)oc tanamide |
| 102. | 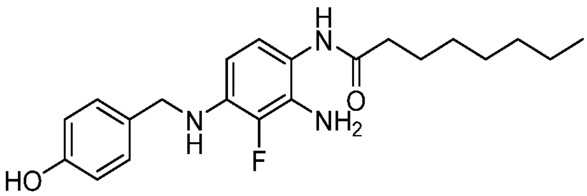 | N-(2-amino-3-fluoro-4-((4-hydroxybenzyl)amino)phenyl)oc tanamide |
| 103. | 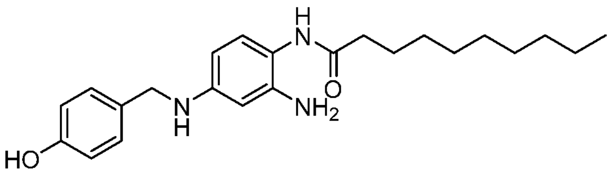 | N-(2-amino-4-((4-hydroxybenzyl)amino) phenyl)de canamide |
| 104. | 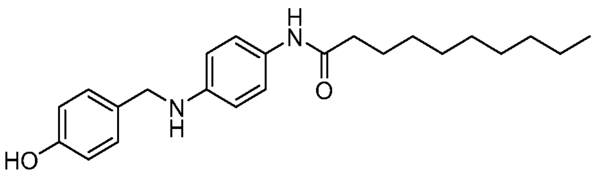 | N-(4-((4-hydroxybenzyl)amino)phenyl)de canamide |
| 105. | 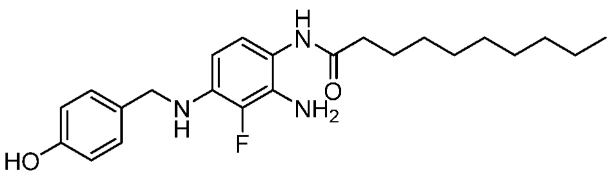 | N-(2-amino-3-fluoro-4-((4-hydroxybenzyl)amino)phenyl)de canamide |

(continued)

| | COMPOUND STRUCTURE | COMPOUND NAME |
|---|---|---|
| 106. | 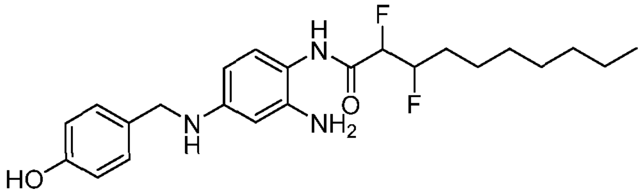 | N-(2-amino-4-((4-hydroxybenzyl)amino) phenyl)-2,3-difluorodecanamide (2R,3 S)-N-(2-amino-4-((4-hydroxybenzyl)amino)phenyl)-2,3-difluorodecanamide (2S,3R)-N-(2-amino-4-((4-hydroxybenzyl)amino)phenyl)-2,3-difluorodecanamide (2R,3R)-N-(2-amino-4-((4-hydroxybenzyl)amino)phenyl)-2,3-difluorodecanamide (2S,3 S)-N-(2-amino-4-((4-hydroxybenzyl)amino)phenyl)-2,3-difluorodecanamide racemic mixtures thereof |
| 107. | 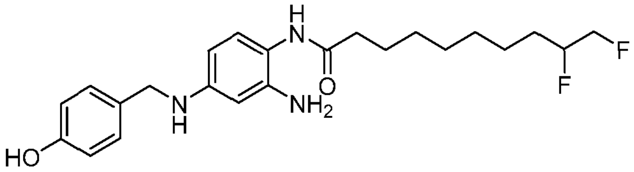 | N-(2-amino-4-((4-hydroxybenzyl)amino) phenyl)-9,10-difluorodecanamide (9R)-N-(2-amino-4-((4-hydroxybenzyl) amino)phenyl)-2,3-difluorodecanamide (9S)-N-(2-amino-4-((4-hydroxybenzyl) amino)phenyl)-2,3-difluorodecanamide racemic mixtures thereof |
| 108. | 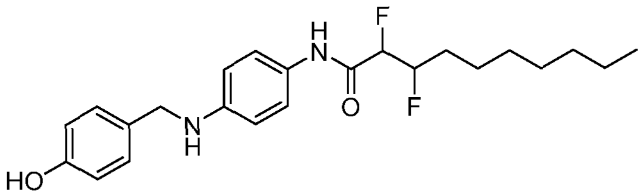 | 2,3-difluoro-N-(4-((4-hydroxybenzyl)amino)phenyl)de ca decanamide (2R,3S)-2,3-difluoro-N-(4-((4-hydroxybenzyl)amino)phenyl)de ca decanamide (2S,3R)-2,3-difluoro-N-(4-((4-hydroxybenzyl)amino)phenyl)de canamide (2R,3R)-2,3-difluoro-N-(4-((4-hydroxybenzyl)amino)phenyl)de ca decanamide (2S,3S)-2,3-difluoro-N-(4-((4-hydroxybenzyl)amino)phenyl)de canamide racemic mixtures thereof |
| 109. | 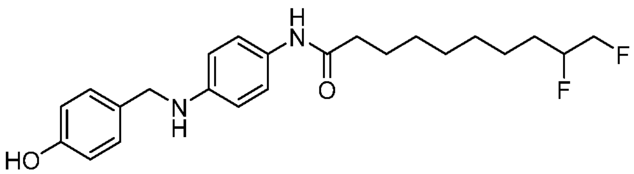 | 9,10-difluoro-N-(4-((4-hydroxybenzyl) amino)phenyl)de canamide (9R)-9,10-difluoro-N-(4-((4-hydroxybenzyl)amino) phenyl)de canamide (9S)-9,10-difluoro-N-(4-((4-hydroxybenzyl)amino)phenyl)de ca namide racemic mixtures thereof |
| 110. | 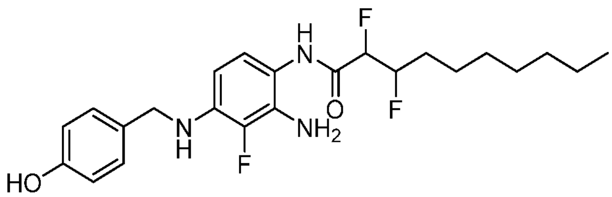 | N-(2-amino-3-fluoro-4-((4-hydroxybenzyl)amino)phenyl)-2,3-difluorodecanamide (2R,3 S)-N-(2-amino-3-fluoro-4-((4-hydroxybenzyl)amino)phenyl)-2,3-difluorodecanamide (2S,3R)-N-(2-amino-3-fluoro-4-((4-hydroxybenzyl)amino)phenyl)-2,3-difluorodecanamide (2R,3R)-N-(2-amino-3-fluoro-4-((4-hydroxybenzyl)amino)phenyl)-2,3-difluorodecanamide (2S,3S)-N-(2-amino-3-fluoro-4-((4-hydroxybenzyl)amino)phenyl)-2,3-difluorodecanamide racemic mixtures thereof |

(continued)

| | COMPOUND STRUCTURE | COMPOUND NAME |
|---|---|---|
| 111. | 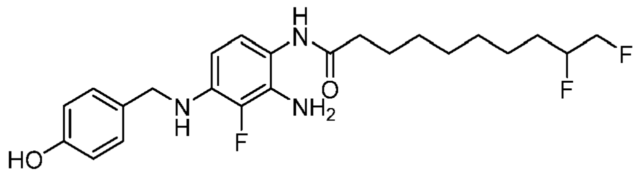 | N-(2-amino-3-fluoro-4-((4-hydroxybenzyl)amino)phenyl)-9,10-difluorodecanamide (9R)-N-(2-amino-3-fluoro-4-((4-hydroxybenzyl)amino)phenyl)-9,10-difluorodecanamide (9S)-N-(2-amino-3-fluoro-4-((4-hydroxybenzyl)amino)phenyl)-9,10-difluorodecanamide racemic mixtures thereof |
| 112. | 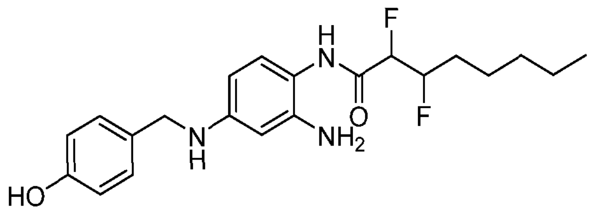 | N-(2-amino-4-((4-hydroxybenzyl)amino) phenyl)-2,3-difluorooctanamide (2R,3 S)-N-(2-amino-4-((4-hydroxybenzyl)amino)phenyl)-2,3-difluorooctanamide (2S,3R)-N-(2-amino-4-((4-hydroxybenzyl)amino)phenyl)-2,3-difluorooctanamide (2R,3R)-N-(2-amino-4-((4-hydroxybenzyl)amino)phenyl)-2,3-difluorooctanamide (2S,3 S)-N-(2-amino-4-((4-hydroxybenzyl)amino)phenyl)-2,3-difluorooctanamide racemic mixtures thereof |
| 113. | 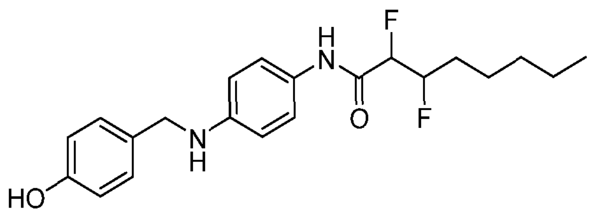 | 2,3-difluoro-N-(4-((4-hydroxybenzyl)amino)phenyl)oc tanamide (2R,3S)-2,3-difluoro-N-(4-((4-hydroxybenzyl)amino) phenyl)oc tanamide (2S,3R)-2,3-difluoro-N-(4-((4-hydroxybenzyl)amino)phenyl)oc (2R,3R)-2,3-difluoro-N-(4-((4-hydroxybenzyl)amino)phenyl)oc tanamide (2S,3S)-2,3-difluoro-N-(4-((4-hydroxybenzyl)amino)phenyl)oc tanamide racemic mixtures thereof |
| 114. | 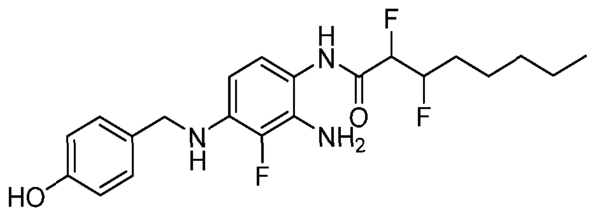 | N-(2-amino-3-fluoro-4-((4-hydroxybenzyl)amino)phenyl)-2,3-difluorooctanamide (2R,3S)-N-(2-amino-3-fluoro-4-((4-hydroxybenzyl)amino)phenyl)-2,3-difluorooctanamide (2S,3R)-N-(2-amino-3-fluoro-4-((4-hydroxybenzyl)amino)phenyl)-2,3-difluorooctanamide (2R,3R)-N-(2-amino-3-fluoro-4-((4-hydroxybenzyl)amino)phenyl)-2,3-difluorooctanamide (2S,3S)-N-(2-amino-3-fluoro-4-((4-hydroxybenzyl)amino)phenyl)-2,3-difluorooctanamide racemic mixtures thereof |
| 115. | 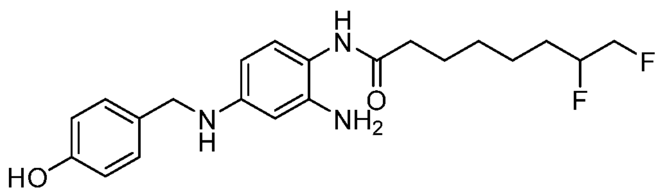 | N-(2-amino-4-((4-hydroxybenzyl)amino) phenyl)-7,8-difluorooctanamide (7S)-N-(2-amino-4-((4-hydroxybenzyl) amino)phenyl)-7,8-difluorooctanamide (7R)-N-(2-amino-4-((4-hydroxybenzyl) amino)phenyl)-7,8-difluorooctanamide racemic mixtures thereof |

(continued)

| | COMPOUND STRUCTURE | COMPOUND NAME |
|---|---|---|
| 116. | 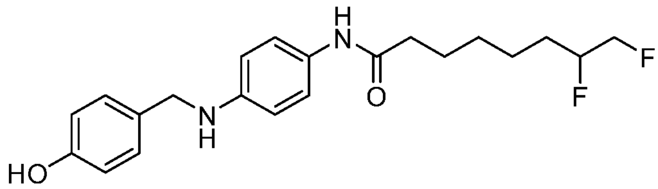 | 7,8-difluoro-N-(4-((4-hydroxybenzyl)amino)phenyl)oc tanamide (7R)-7,8-difluoro-N-(4-((4-hydroxybenzyl)amino)phenyl)oc tanamide (7S)-7,8-difluoro-N-(4-((4-hydroxybenzyl)amino)phenyl)oc tanamide racemic mixtures thereof |
| 117. | 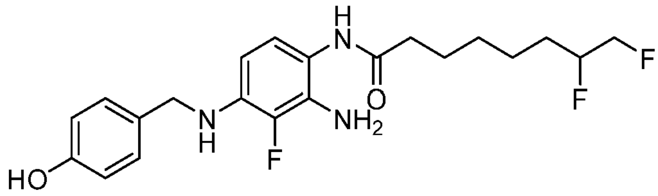 | N-(2-amino-3-fluoro-4-((4-hydroxybenzyl)amino)phenyl)-7,8-difluorooctanamide (7R)-N-(2-amino-3-fluoro-4-((4-hydroxybenzyl)amino)phenyl)-7,8-difluorooctanamide (7S)-N-(2-amino-3-fluoro-4-((4-hydroxybenzyl)amino)phenyl)-7,8-difluorooctanamide racemic mixtures thereof |
| 118. | 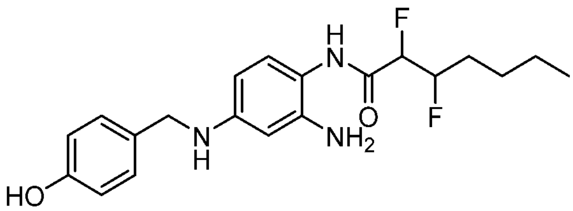 | N-(2-amino-4-((4-hydroxybenzyl)amino) phenyl)-2,3-difluoroheptanamide (2R,3 S)-N-(2-amino-4-((4-hydroxybenzyl)amino)phenyl)-2,3 -difluoroheptanamide (2S,3R)-N-(2-amino-4-((4-hydroxybenzyl)amino)phenyl)-2,3-difluoroheptanamide (2R,3R)-N-(2-amino-4-((4-hydroxybenzyl)amino)phenyl)-2,3 -difluoroheptanamide (2S,3 S)-N-(2-amino-4-((4-hydroxybenzyl)amino)phenyl)-2,3 -difluoroheptanamide racemic mixtures thereof |
| 119. | 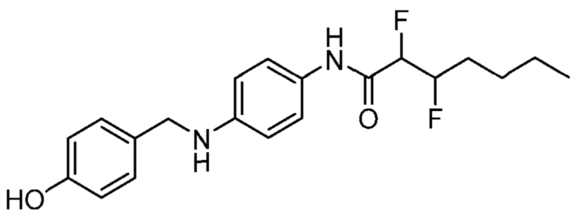 | 2,3-difluoro-N-(4-((4-hydroxybenzyl)amino)phenyl)he ptanamide (2R,3S)-2,3-difluoro-N-(4-((4-hydroxybenzyl)amino) phenyl)he ptanamide (2S,3R)-2,3-difluoro-N-(4-((4-hydroxybenzyl)amino) phenyl)he ptanamide (2R,3R)-2,3-difluoro-N-(4-((4-hydroxybenzyl)amino) phenyl)he ptanamide (2S,3S)-2,3-difluoro-N-(4-((4-hydroxybenzyl)amino) phenyl)he ptanamide racemic mixtures thereof |
| 120. | 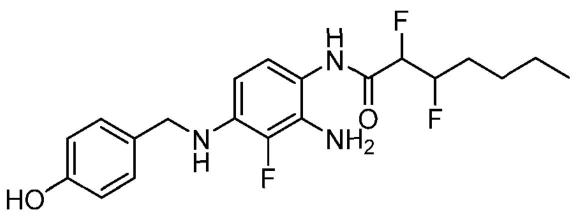 | N-(2-amino-3-fluoro-4-((4-hydroxybenzyl)amino)phenyl)-2,3 -difluoroheptanamide (2R,3 S)-N-(2-amino-3-fluoro-4-((4-hydroxybenzyl)amino)phenyl)-2,3 -difluoroheptanamide (2S,3R)-N-(2-amino-3-fluoro-4-((4-hydroxybenzyl)amino) phenyl)-2,3 -difluoroheptanamide (2R,3R)-N-(2-amino-3-fluoro-4-((4-hydroxybenzyl)amino)phenyl)-2,3 -difluoroheptanamide (2S,3S)-N-(2-amino-3-fluoro-4-((4-hydroxybenzyl)amino)phenyl)-2,3-difluoroheptanamide racemic mixtures thereof |

(continued)

| | COMPOUND STRUCTURE | COMPOUND NAME |
|---|---|---|
| 121. | 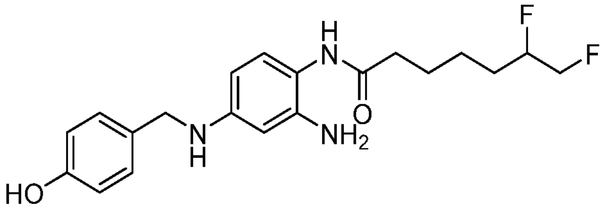 | N-(2-amino-4-((4-hydroxybenzyl)amino) phenyl)-6,7-difluoroheptanamide (6R)-N-(2-amino-4-((4-hydroxybenzyl) amino)phenyl)-6,7-difluoroheptanamide (6S)-N-(2-amino-4-((4-hydroxybenzyl) amino)phenyl)-6,7-difluoroheptanamide racemic mixtures thereof |
| 122. | 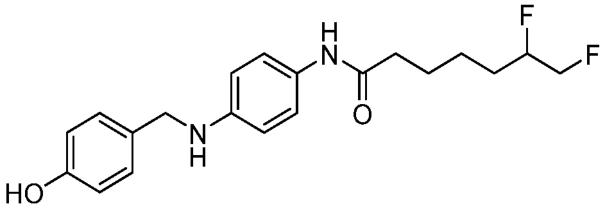 | 6,7-difluoro-N-(4-((4-hydroxybenzyl)amino)phenyl)he ptanamide (6R)-6,7-difluoro-N-(4-((4-hydroxybenzyl)amino) phenyl)he ptanamide (6S)-6,7-difluoro-N-(4-((4-hydroxybenzyl)amino)phenyl)he ptanamide racemic mixtures thereof |
| 123. | 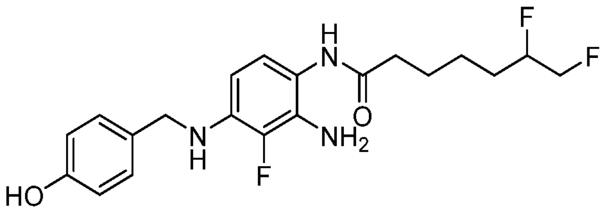 | N-(2-amino-3-fluoro-4-((4-hydroxybenzyl)amino)phenyl)-6,7-difluoroheptanamide (6R)-N-(2-amino-3-fluoro-4-((4-hydroxybenzyl)amino)phenyl)-6,7-difluoroheptanamide (6S)-N-(2-amino-3-fluoro-4-((4-hydroxybenzyl)amino)phenyl)-6,7-difluoroheptanamide racemic mixtures thereof |
| 124. | 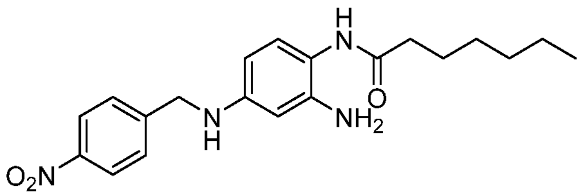 | N-(2-amino-4-((4-nitrobenzyl)amino)phenyl)hepta namide |
| 125. | 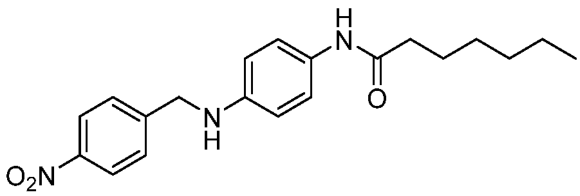 | N-(4-((4-nitrobenzyl)amino)phenyl)hepta namide |
| 126. | 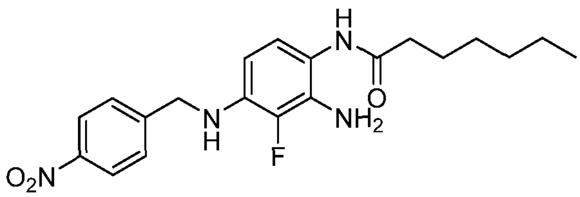 | N-(2-amino-3-fluoro-4-((4-nitrobenzyl) amino)phenyl)hepta namide |
| 127. | 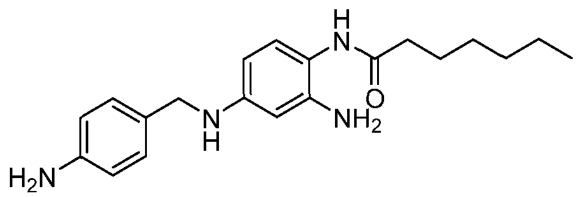 | N-(2-amino-4-((4-aminobenzyl)amino) phenyl)hept anamide |

(continued)

| | COMPOUND STRUCTURE | COMPOUND NAME |
|---|---|---|
| 128. | 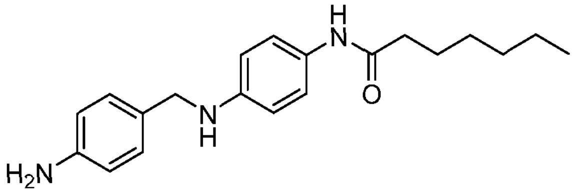 | N-(4-((4-aminobenzyl)amino)phenyl)hept anamide |
| 129. | 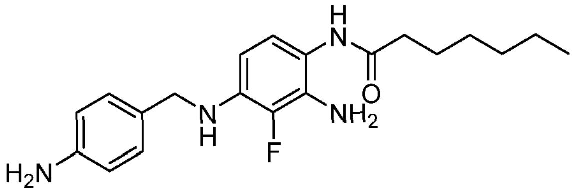 | N-(2-amino-3-fluoro-4-((4-aminobenzyl) amino)phenyl)hept anamide |
| 130. | 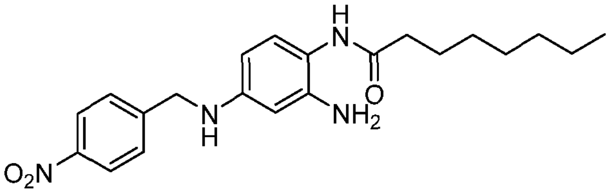 | N-(2-amino-4-((4-nitrobenzyl)amino)phe-nyl)octan amide |
| 131. | 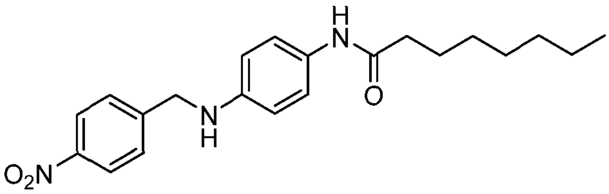 | N-(4-((4-nitrobenzyl)amino)phenyl)octan amide |
| 132. | 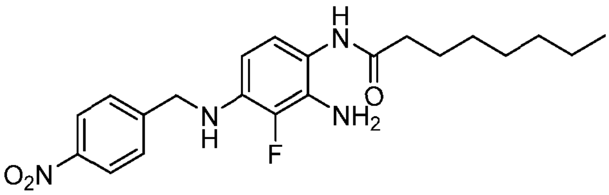 | N-(2-amino-3-fluoro-4-((4-nitrobenzyl) amino)phenyl)octan amide |
| 133. | 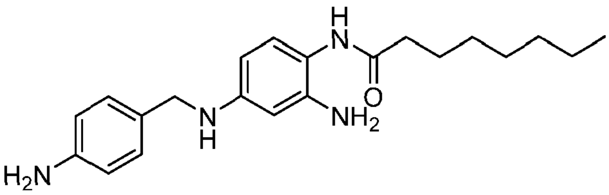 | N-(2-amino-4-((4-aminobenzyl)amino) phenyl)octa namide |
| 134. | 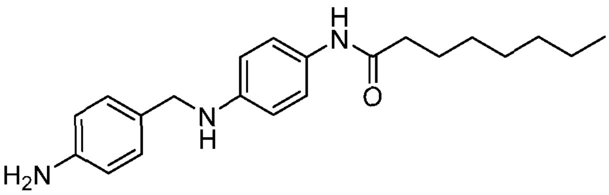 | N-(4-((4-aminobenzyl)amino)phenyl)octa namide |
| 135. | 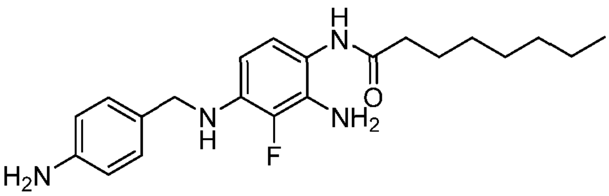 | N-(2-amino-3-fluoro-4-((4-aminobenzyl) amino)phenyl)octa namide |

(continued)

| | COMPOUND STRUCTURE | COMPOUND NAME |
|---|---|---|
| 136. | 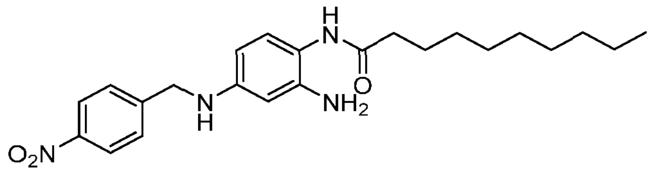 | N-(2-amino-4-((4-nitrobenzyl)amino)phe-nyl)decan amide |
| 137. | 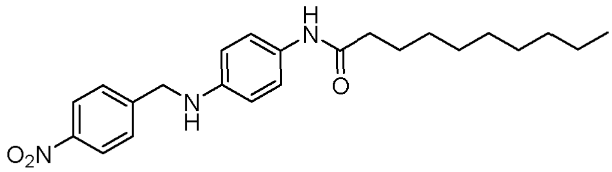 | N-(4-((4-nitrobenzyl)amino)phenyl)decan amide |
| 138. | 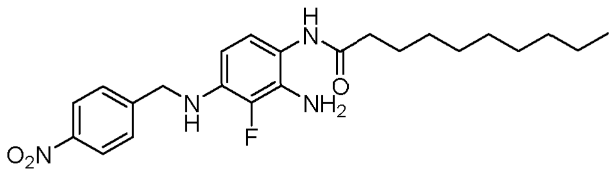 | N-(2-amino-3-fluoro-4-((4-nitrobenzyl) amino)phenyl)decan amide |
| 139. | 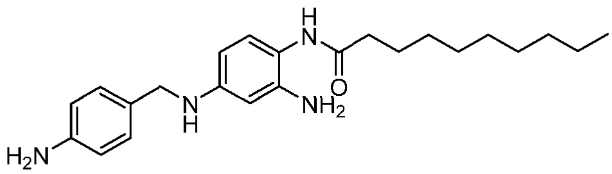 | N-(2-amino-4-((4-aminobenzyl)amino) phenyl)deca namide |
| 140. | 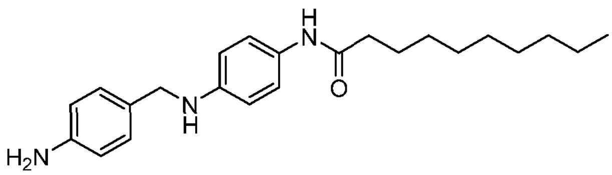 | N-(4-((4-aminobenzyl)amino)phenyl) deca namide |
| 141. | 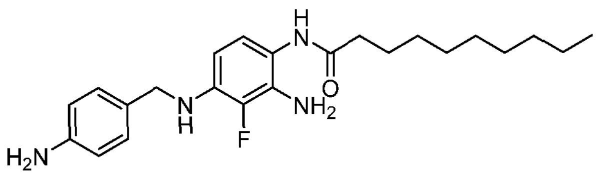 | N-(2-amino-3-fluoro-4-((4-aminobenzyl) amino)phenyl)deca namide |
| 142. | 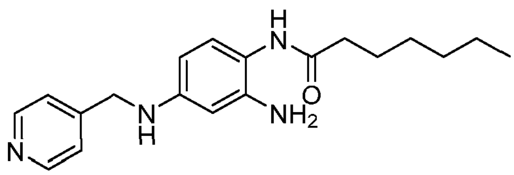 | N-(2-amino-4-((pyridin-4-ylmethyl)amino) phenyl)heptana mide |
| 143. | 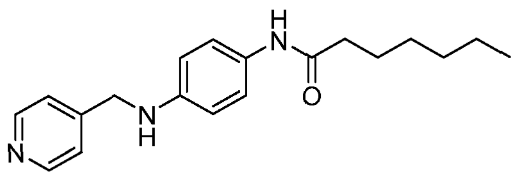 | N-(4-((pyridin-4-ylmethyl)amino)phenyl) heptana mide |
| 144. | 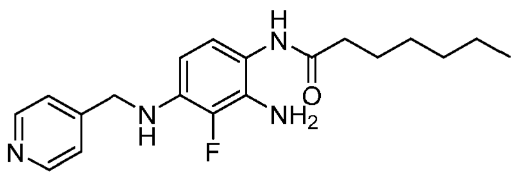 | N-(2-amino-3-fluoro-4-((pyridin-4-ylmethyl)amino)phenyl)heptana mide |

(continued)

| | COMPOUND STRUCTURE | COMPOUND NAME |
|---|---|---|
| 145. | 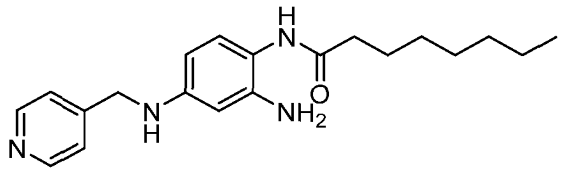 | N-(2-amino-4-((pyridin-4-ylmethyl)amino) phenyl)octanam ide |
| 146. | 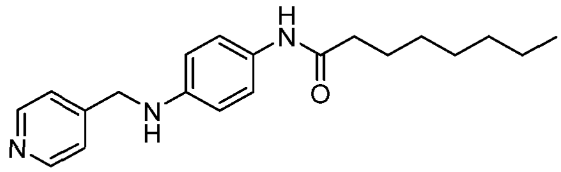 | N-(4-((pyridin-4-ylmethyl)amino)phenyl) octanam ide |
| 147. | 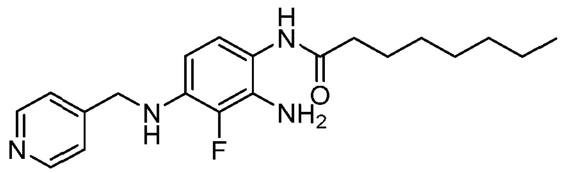 | N-(2-amino-3-fluoro-4-((pyridin-4-ylmethyl)amino)phenyl)octanam ide |
| 148. | 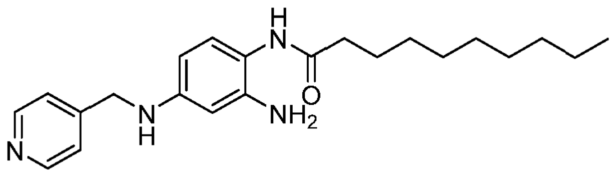 | N-(2-amino-4-((pyridin-4-ylmethyl)amino) phenyl)decana mide |
| 149. | 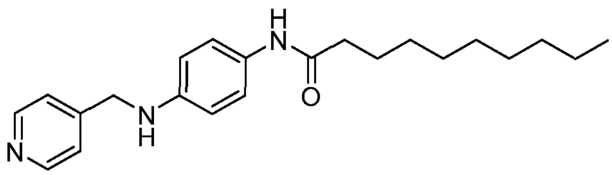 | N-(4-((pyridin-4-ylmethyl)amino)phenyl) decana mide |
| 150. | 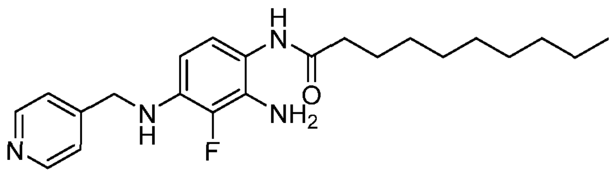 | N-(2-amino-3-fluoro-4-((pyridin-4-ylmethyl)amino)phenyl)decana mide |
| 151. | 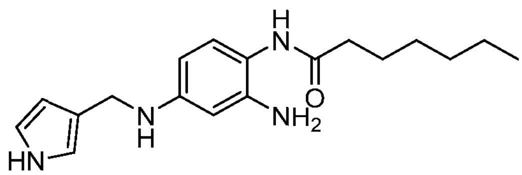 | N-(4-(((1H-pyrrol-3-yl)methyl)amino)-2-aminophenyl)heptanamide |
| 152. | 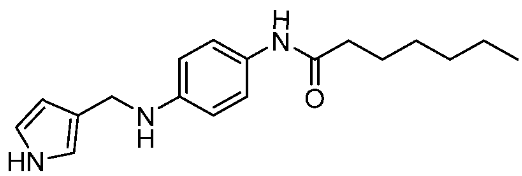 | N-(4-(((1H-pyrrol-3-yl)methyl)amino)phe-nyl)heptana mide |
| 153. | 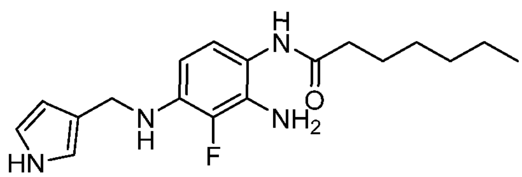 | N-(4-(((1H-pyrrol-3-yl)methyl)amino)-2-amino-3-fluorophenyl)heptanamide |

(continued)

| | COMPOUND STRUCTURE | COMPOUND NAME |
|---|---|---|
| 154. | 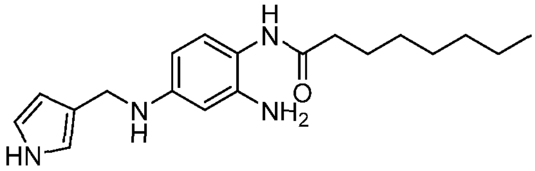 | N-(4-(((1H-pyrrol-3-yl)methyl)amino)-2-aminophenyl)octanamide |
| 155. | 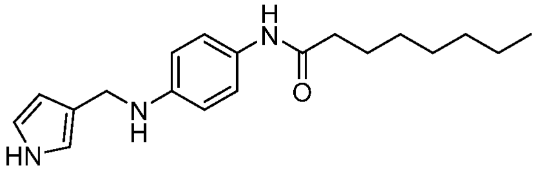 | N-(4-(((1H-pyrrol-3-yl)methyl)amino)phenyl)octana mide |
| 156. | 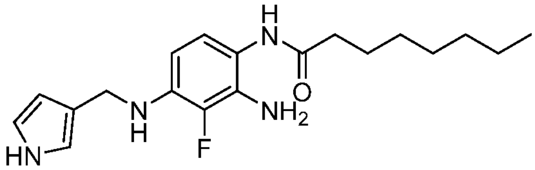 | N-(4-(((1H-pyrrol-3-yl)methyl)amino)-2-amino-3-fluorophenyl)octanamide |
| 157. | 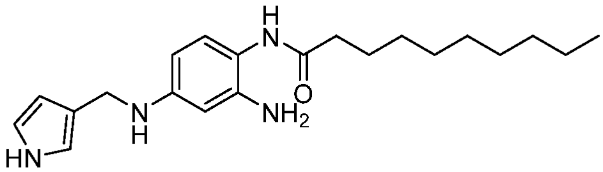 | N-(4-(((1H-pyrrol-3-yl)methyl)amino)-2-aminophenyl)decanamide |
| 158. | 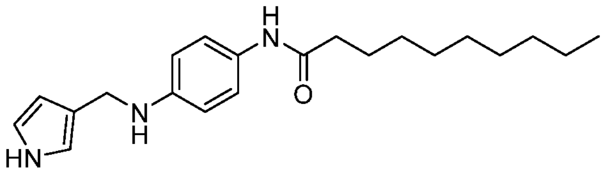 | N-(4-(((1H-pyrrol-3-yl)methyl)amino)phenyl)decana mide |
| 159. | 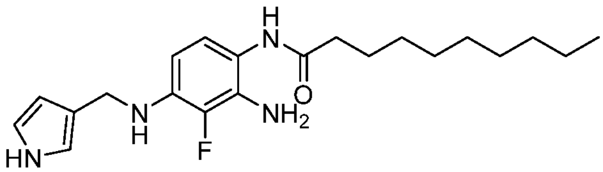 | N-(4-(((1H-pyrrol-3-yl)methyl)amino)-2-amino-3-fluorophenyl)decanamide |
| 160. | 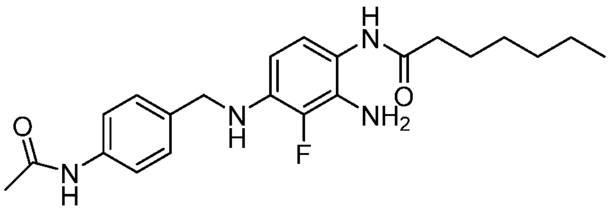 | N-(4-((4-acetamidobenzyl)amino)-2-amino-3-fluorophenyl)heptanamide |
| 161. | 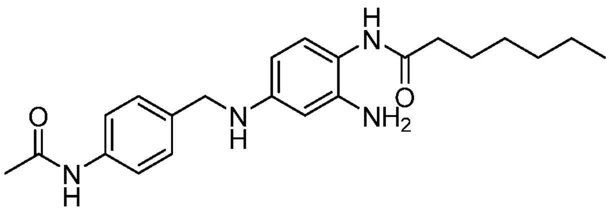 | N-(4-((4-acetamidobenzyl)amino)-2-aminophenyl)heptanamide |

(continued)

| | COMPOUND STRUCTURE | COMPOUND NAME |
|---|---|---|
| 162. | 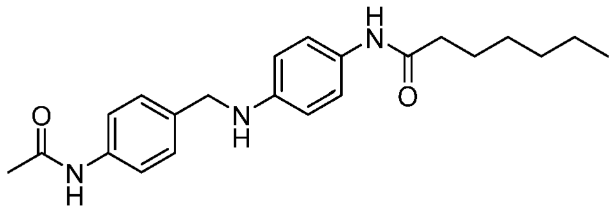 | N-(4-((4-acetamidobenzyl)amino)phenyl) heptanamide |
| 163. | 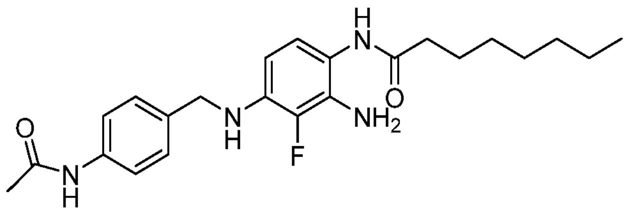 | N-(4-((4-acetamidobenzyl)amino)-2-amino-3-fluorophenyl)octanamide |
| 164. | 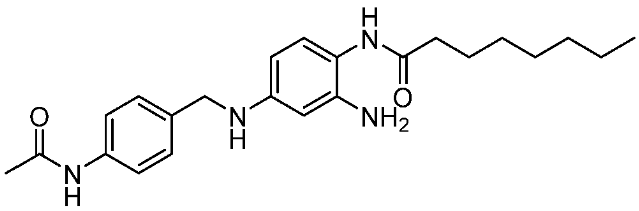 | N-(4-((4-acetamidobenzyl)amino)-2-aminophenyl)octanamide |
| 165. | 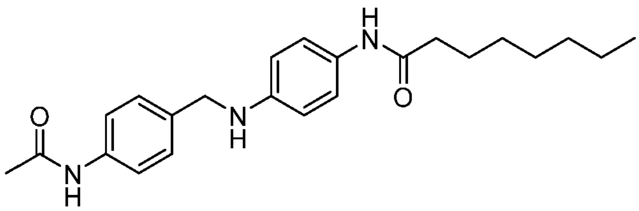 | N-(4-((4-acetamidobenzyl)amino)phenyl) octanamide |
| 166. | 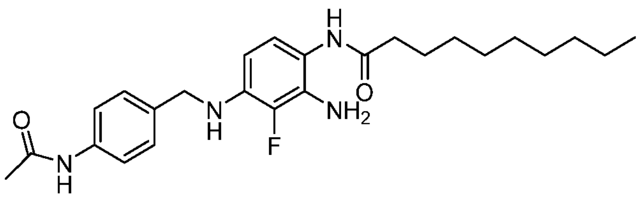 | N-(4-((4-acetamidobenzyl)amino)-2-amino-3-fluorophenyl)decanamide |
| 167. | 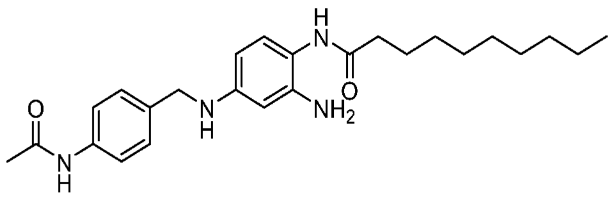 | N-(4-((4-acetamidobenzyl)amino)-2-aminophenyl)decanamide |
| 168. | 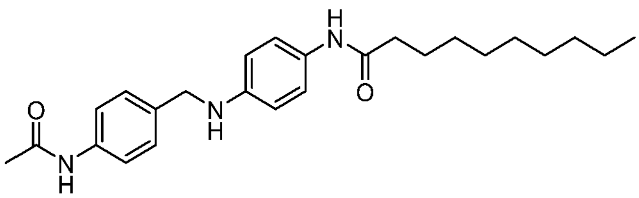 | N-(4-((4-acetamidobenzyl)amino)phenyl) decanamide |

(continued)

| | COMPOUND STRUCTURE | COMPOUND NAME |
|---|---|---|
| 169. | 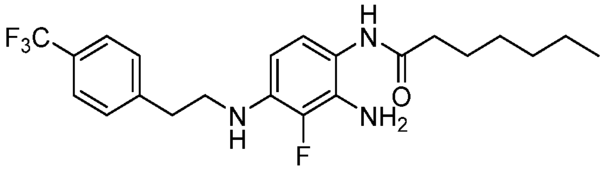 | N-(2-amino-3-fluoro-4-((4-(trifluoromethyl)phenethyl)amin o)phenyl)hepta-namide |
| 170. | 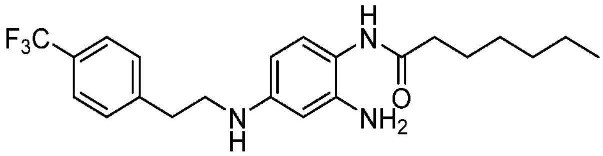 | N-(2-amino-4-((4-(trifluoromethyl)phenethyl)amin o)phenyl)heptanamide |
| 171. | 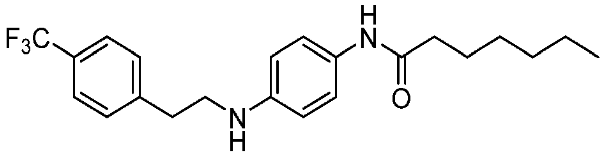 | N-(4-((4-(trifluoromethyl)phenethyl)amin o)phenyl)heptanamide |
| 172. | 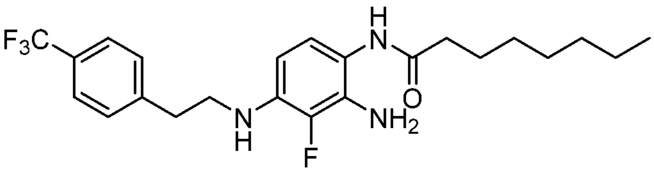 | N-(2-amino-3-fluoro-4-((4-(trifluoromethyl)phenethyl)amin o)phenyl)octana-mide |
| 173. | 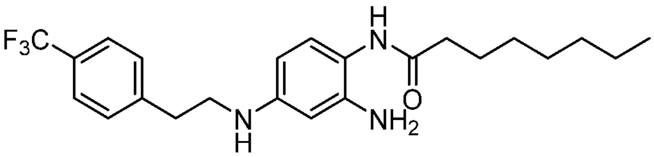 | N-(2-amino-4-((4-(trifluoromethyl)phenethyl)amin o)pheny)loctanamide |
| 174. | 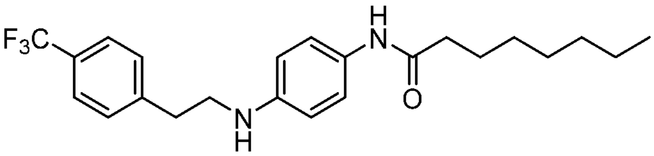 | N-(4-((4-(trifluoromethyl)phenethyl)amin o)phenyl)octanamide |
| 175. | 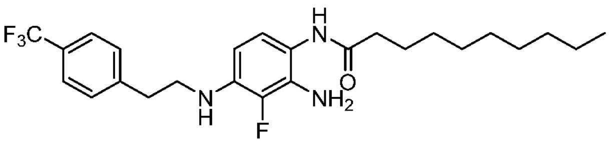 | N-(2-amino-3-fluoro-4-((4-(trifluoromethyl)phenethyl)amin o)phenyl)decana-mide |
| 176. | 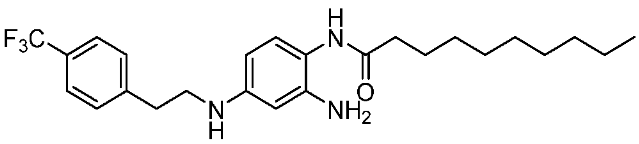 | N-(2-amino-4-((4-(trifluoromethyl)phenethyl)amin o)phenyl)decanamide |
| 177. | 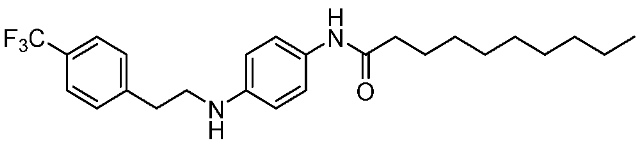 | N-(4-((4-(trifluoromethyl)phenethyl)amin o)phenyl)decanamide |

(continued)

| | COMPOUND STRUCTURE | COMPOUND NAME |
|---|---|---|
| 178. | 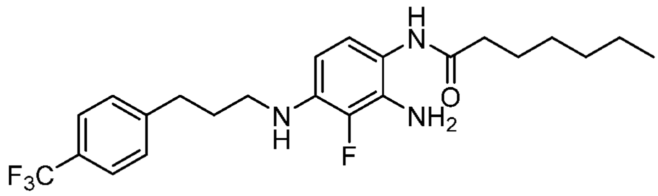 | N-(2-amino-3-fluoro-4-((3-(4-(trifluoromethyl)phenyl)propyl)a mino)phenyl)hep-tanamide |
| 179. | 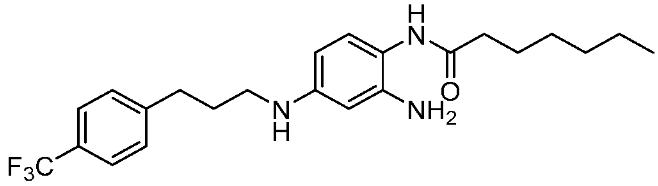 | N-(2-amino-4-((3-(4-(trifluoromethyl)phenyl)propyl)a mino)phenyl)heptanamide |
| 180. | 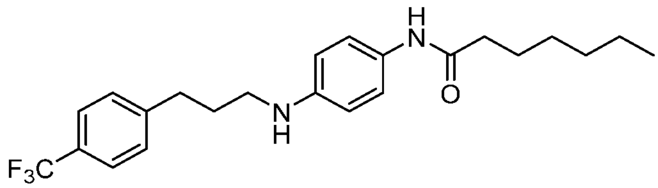 | N-(4-((3-(4-(trifluoromethyl)phenyl)propyl)a mino)phenyl)heptanamide |
| 181. | 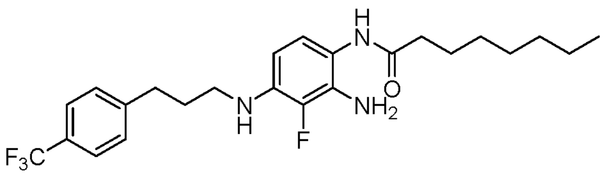 | N-(2-amino-3-fluoro-4-((3-(4-(trifluoromethyl)phenyl)propyl)a mino)phenyl)oc-tanamide |
| 182. | 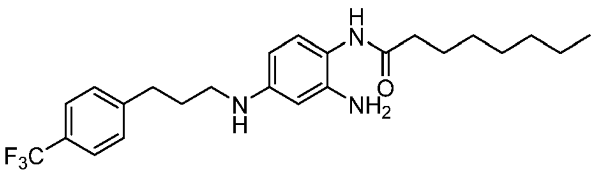 | N-(2-amino-4-((3-(4-(trifluoromethyl)phenyl)propyl)a mino)phenyl)octanamide |
| 183. | 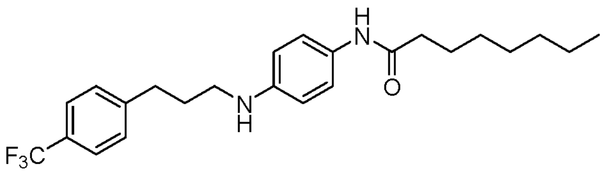 | N-(4-((3-(4-(trifluoromethyl)phenyl)propyl)a mino)phenyl)octanamide |
| 184. | 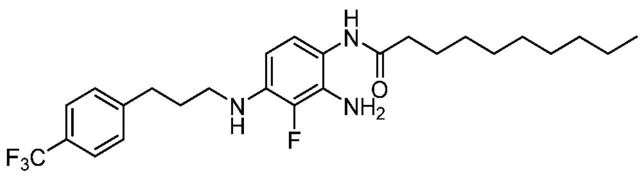 | N-(2-amino-3-fluoro-4-((3-(4-(trifluoromethyl)phenyl)propyl)a mino)phenyl)de-canamide |
| 185. | 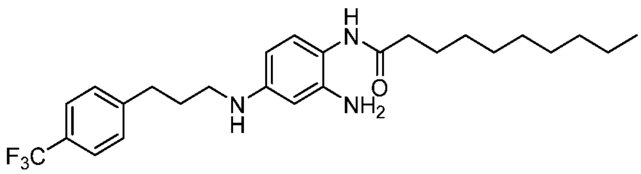 | N-(2-amino-4-((3-(4-(trifluoromethyl)phenyl)propyl)a mino)phenyl)decanamide |

(continued)

| | COMPOUND STRUCTURE | COMPOUND NAME |
|---|---|---|
| 186. | 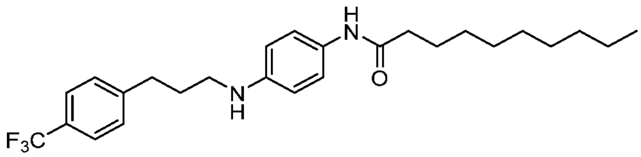 | N-(4-((3-(4-(trifluoromethyl)phenyl)propyl)a mino)phenyl)decanamide |
| 187. | 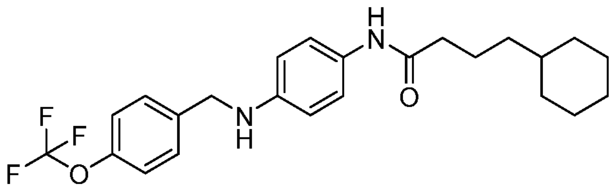 | 4-cyclohexyl-N-(4-((4-(trifluoromethoxy) benzyl)amino )phenyl)butanamide |
| 188. | 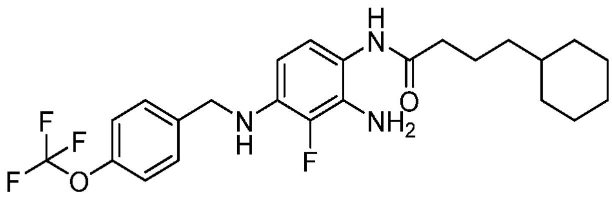 | N-(2-amino-3-fluoro-4-((4-(trifluoromethoxy)benzyl)amino )phenyl)-4-cyclohexylbutanamide |
| 189. | 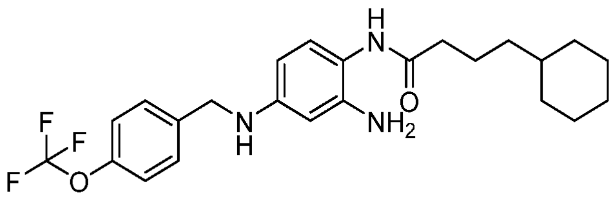 | N-(2-amino-4-((4-(trifluoromethoxy)benzyl)amino )phenyl)-4-cyclohexylbutanamide |
| 190. | 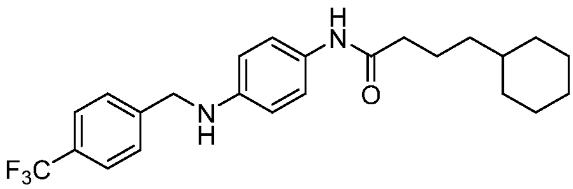 | 4-cyclohexyl-N-(4-((4-(trifluoromethyl) benzyl)amino)p henyl)butanamide |
| 191. | 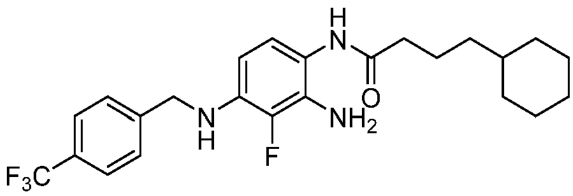 | N-(2-amino-3-fluoro-4-((4-(trifluoromethyl)benzyl)amino)p henyl)-4-cyclohexylbutanamide |
| 192. | 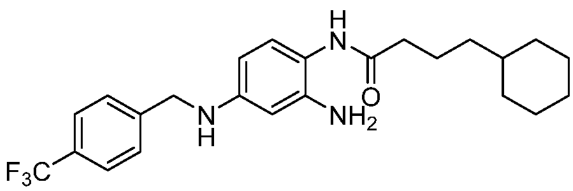 | N-(2-amino-4-((4-(trifluoromethyl)benzyl) amino)p henyl)-4-cyclohexylbutanamide |
| 193. | 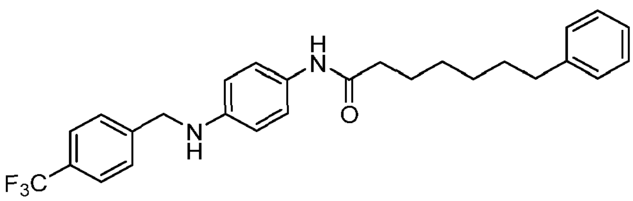 | 7-phenyl-N-(4-((4-(trifluoromethyl)benzyl) amino)p henyl)heptanamide |

(continued)

| | COMPOUND STRUCTURE | COMPOUND NAME |
|---|---|---|
| 194. | 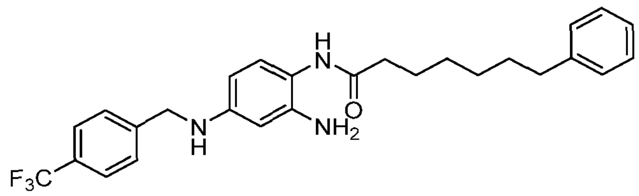 | N-(2-amino-4-((4-(trifluoromethyl)benzyl) amino)p henyl)-7-phenylheptanamide |
| 195. | 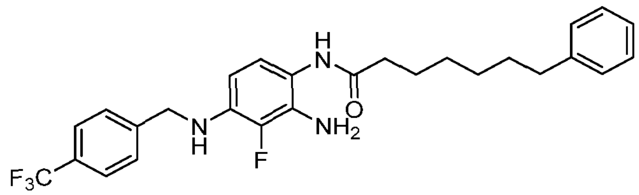 | N-(2-amino-3-fluoro-4-((4-(trifluoromethyl)benzyl)amino)p henyl)-7-phenyl-heptanamide |
| 196. | 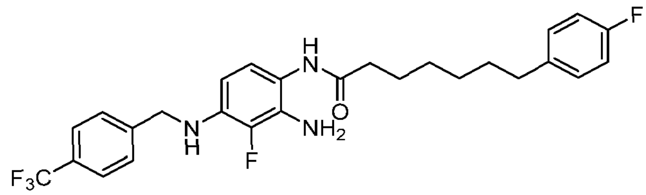 | N-(2-amino-3-fluoro-4-((4-(trifluoromethyl)benzyl)amino)p henyl)-7-(4-fluor-ophenyl)heptanamide |
| 197. | 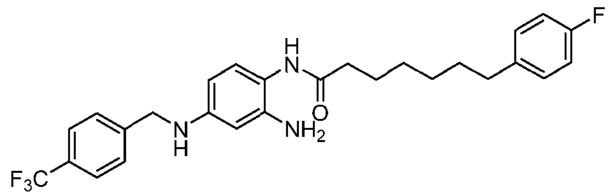 | N-(2-amino-4-((4-(trifluoromethyl)benzyl) amino)p henyl)-7-(4-fluorophenyl)hepta-namide |
| 198. | 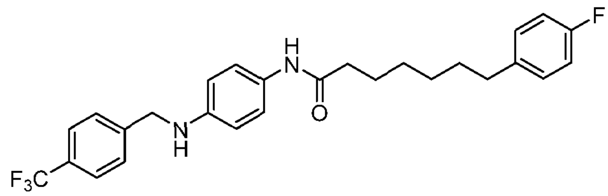 | 7-(4-fluorophenyl)-N-(4-((4-(trifluoromethyl)benzyl)amino)p henyl)heptana-mide |
| 199. | 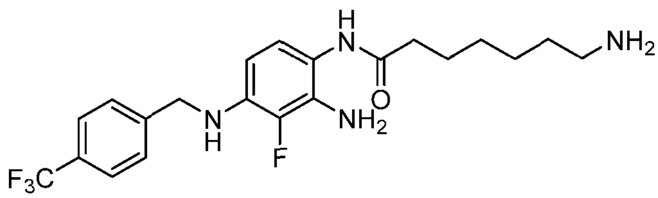 | 7-amino-N-(2-amino-3-fluoro-4-((4-(trifluoromethyl)benzyl)amino)p henyl)hep-tanamide |
| 200. | 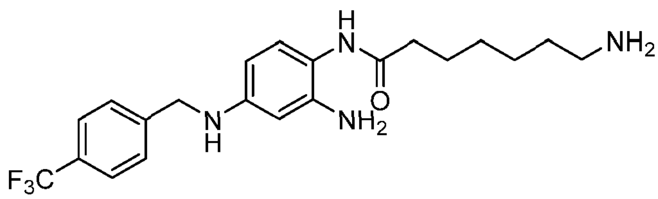 | 7-amino-N-(2-amino-4-((4-(trifluoromethyl)benzyl)amino)p henyl)heptana-mide |
| 201. | 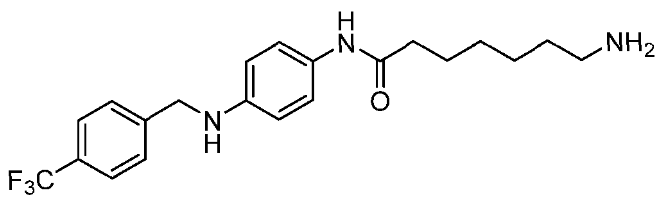 | 7-amino-N-(4-((4-(trifluoromethyl)benzyl) amino)p henyl)heptanamide |

(continued)

| | COMPOUND STRUCTURE | COMPOUND NAME |
|---|---|---|
| 202. | 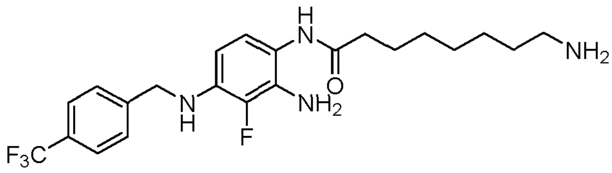 | 8-amino-N-(2-amino-3-fluoro-4-((4-(trifluoromethyl)benzyl)amino)p henyl)octanamide |
| 203. | 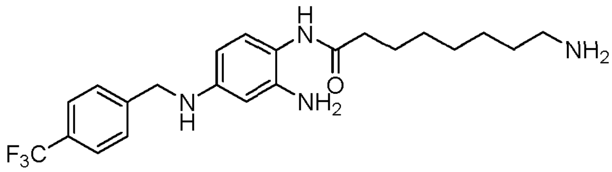 | 8-amino-N-(2-amino-4-((4-(trifluoromethyl)benzyl)amino)p henyl)octanamide |
| 204. | 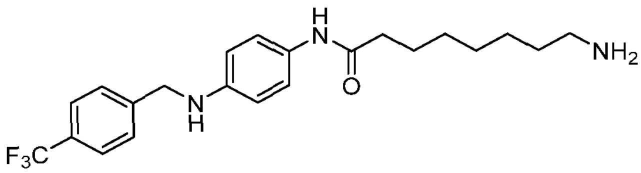 | 8-amino-N-(4-((4-(trifluoromethyl)benzyl) amino)p henyl)octanamide |
| 205. | 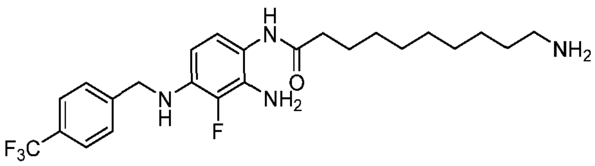 | 10-amino-N-(2-amino-3-fluoro-4-((4-(trifluoromethyl)benzyl)amino)p henyl)decanamide |
| 206. | 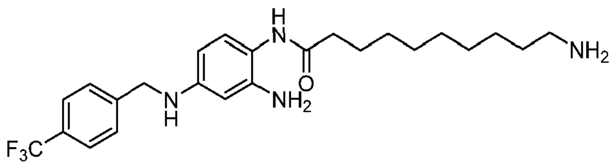 | 10-amino-N-(2-amino-4-((4-(trifluoromethyl)benzyl)amino)p henyl)decanamide |
| 207. | 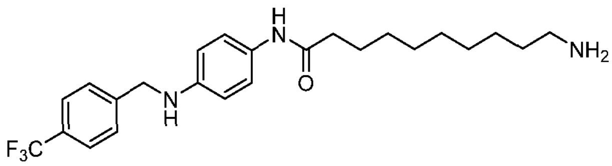 | 10-amino-N-(4-((4-(trifluoromethyl)benzyl)amino)p henyl)decanamide |
| 208. | 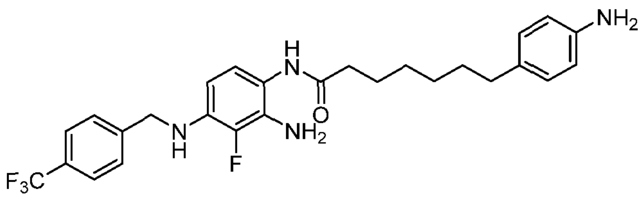 | N-(2-amino-3-fluoro-4-((4-(trifluoromethyl)benzyl)amino)p henyl)-7-(4-aminophenyl)heptanamide |
| 209. | 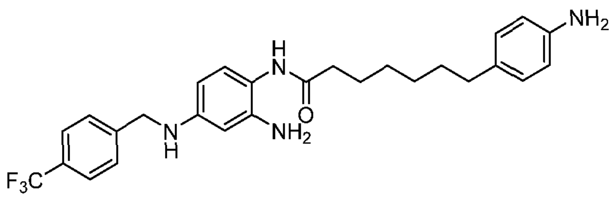 | N-(2-amino-4-((4-(trifluoromethyl)benzyl) amino)p henyl)-7-(4-aminophenyl)heptanamide |

(continued)

| | COMPOUND STRUCTURE | COMPOUND NAME |
|---|---|---|
| 210. | 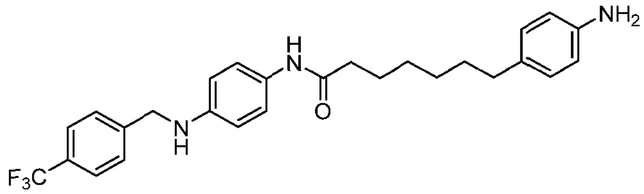 | 7-(4-aminophenyl)-N-(4-((4-(trifluoromethyl)benzyl)amino)p henyl)heptanamide |
| 211. | 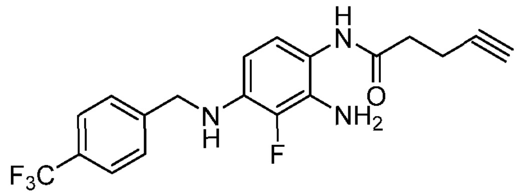 | N-(2-amino-3-fluoro-4-((4-(trifluoromethyl)benzyl)amino)p henyl)pent-4-ynamide |
| 212. | 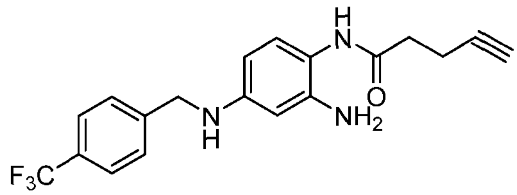 | N-(2-amino-4-((4-(trifluoromethyl)benzyl) amino)p henyl)pent-4-ynamide |
| 213. | 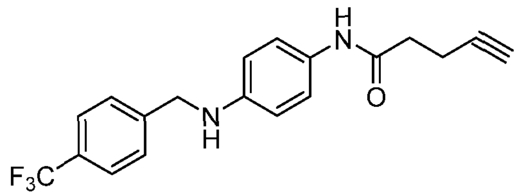 | N-(4-((4-(trifluoromethyl)benzyl)amino)p henyl)pent-4-ynamide |
| 214. | 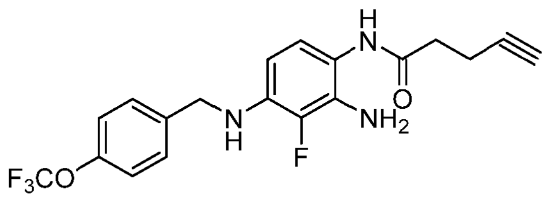 | N-(2-amino-3-fluoro-4-((4-(trifluoromethoxy)benzyl)amino )phenyl)pent-4-ynamide |
| 215. | 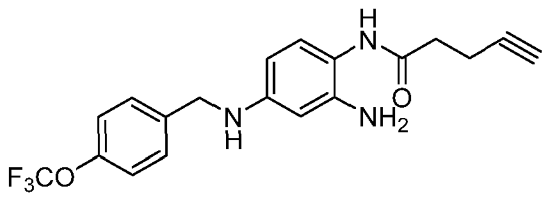 | N-(2-amino-4-((4-(trifluoromethoxy)benzyl)amino )phenyl)pent-4-ynamide |
| 216. | 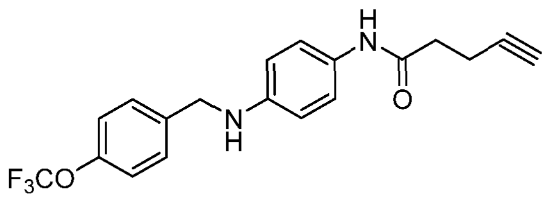 | N-(4-((4-(trifluoromethoxy)benzyl)amino ) phenyl)pent-4-ynamide |
| 217. | 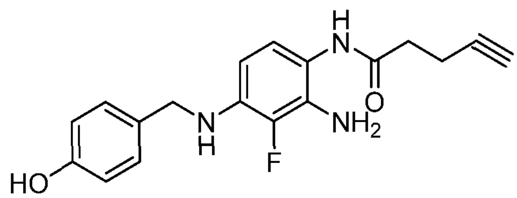 | N-(2-amino-3-fluoro-4-((4-hydroxybenzyl)amino)phenyl)pe nt-4-ynamide |

(continued)

| | COMPOUND STRUCTURE | COMPOUND NAME |
|---|---|---|
| 218. | 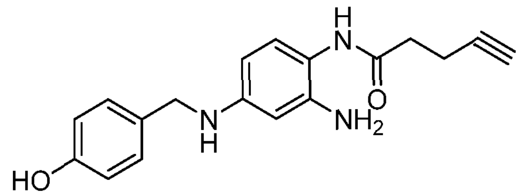 | N-(2-amino-4-((4-hydroxybenzyl)amino) phenyl)pe nt-4-ynamide |
| 219. | 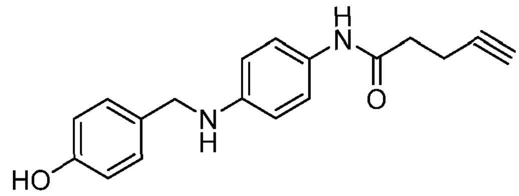 | N-(4-((4-hydroxybenzyl)amino)phenyl)pe nt-4-ynamide |
| 220. | 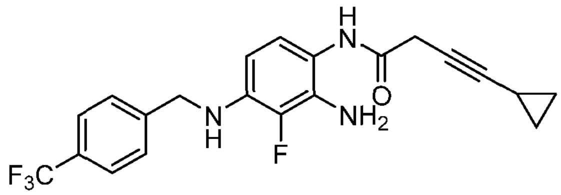 | N-(2-amino-3-fluoro-4-((4-(trifluoromethyl)benzyl)amino)p henyl)-4-cyclo-propylbut-3-ynamide |
| 221. | 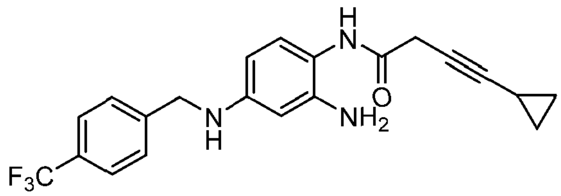 | N-(2-amino-4-((4-(trifluoromethyl)benzyl) amino)p henyl)-4-cyclopropylbut-3-yna-mide |
| 222. | 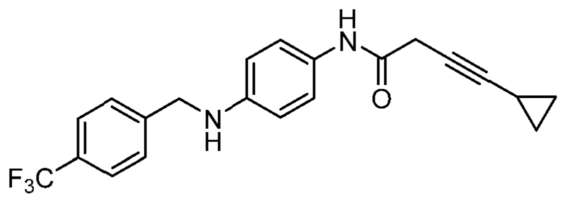 | 4-cyclopropyl-N-(4-((4-(trifluoromethyl) benzyl)amino)p henyl)but-3-ynamide |
| 223. | 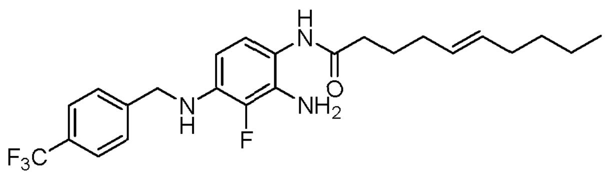 | (E)-N-(2-amino-3-fluoro-4-((4-(trifluoromethyl)benzyl)amino)p henyl)dec-5-en-amide |
| 224. | 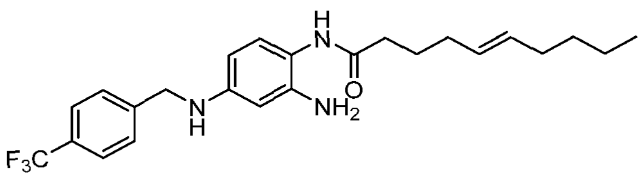 | (E)-N-(2-amino-4-((4-(trifluoromethyl) benzyl)amino)p henyl)dec-5-enamide |
| 225. | 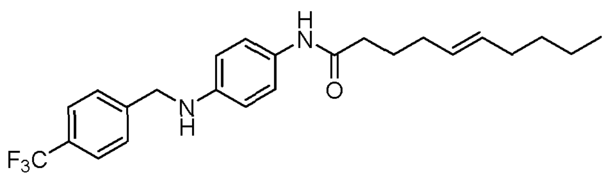 | (E)-N-(4-((4-(trifluoromethyl)benzyl)amino)p henyl)dec-5-enamide |

(continued)

| | COMPOUND STRUCTURE | COMPOUND NAME |
|---|---|---|
| 226. | 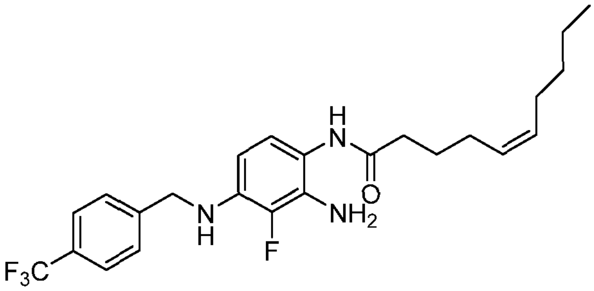 | (Z)-N-(2-amino-3-fluoro-4-((4-(trifluoromethyl)benzyl)amino)p henyl)dec-5-enamide |
| 227. | 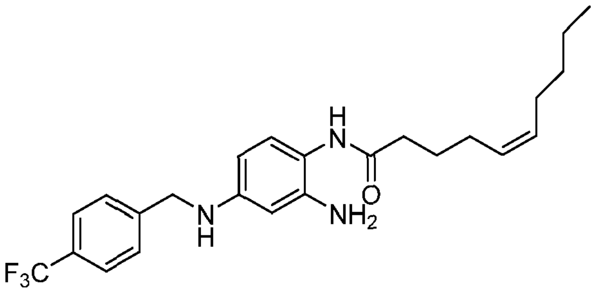 | (Z)-N-(2-amino-4-((4-(trifluoromethyl) benzyl)amino)p henyl)dec-5-enamide |
| 228. | 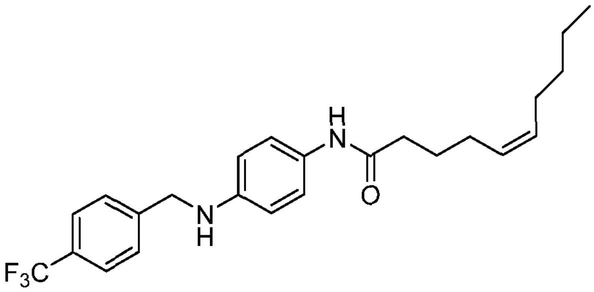 | (Z)-N-(4-((4-(trifluoromethyl)benzyl)amino)p henyl)dec-5-enamide |
| 229. | 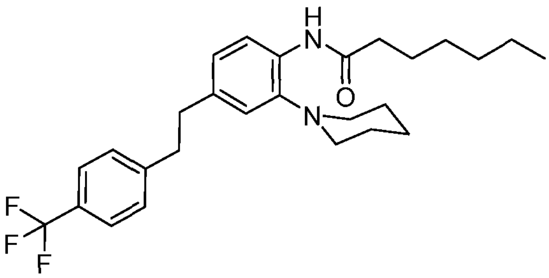 | N-(2-(piperidin-1-yl)-4-(4-(trifluoromethyl) phenethyl)phen yl)heptanamide |
| 230. | 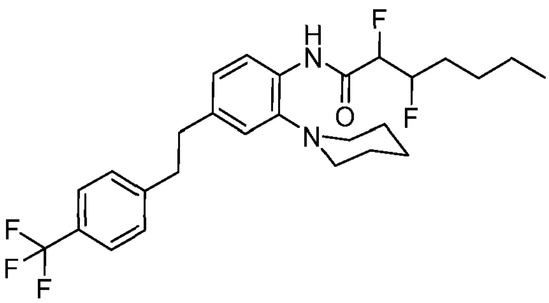 | 2,3-difluoro-N-(2-(piperidin-1-yl)-4-(4-(trifluoromethyl)phenethyl)phen yl)heptanamide (2R,3 S)-2,3-difluoro-N-(2-(piperidin-1-yl)-4-(4-(trifluoromethyl)phenethyl)phen yl)heptanamide (2S,3R)-2,3-difluoro-N-(2-(piperidin-1-yl)-4-(4-(trifluoromethyl)phenethyl)phen yl)heptanamide (2R,3R)-2,3-difluoro-N-(2-(piperidin-1-yl)-4-(4-(trifluoromethyl)phenethyl)phen yl)heptanamide (2S,3 S)-2,3-difluoro-N-(2-(piperidin-1-yl)-4-(4-(trifluoromethyl)phenethyl)phen yl)heptanamide |

(continued)

| | COMPOUND STRUCTURE | COMPOUND NAME |
|---|---|---|
| 231. | 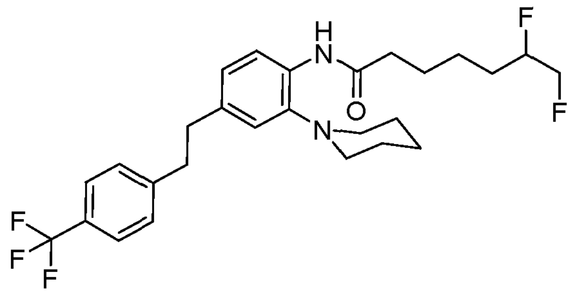 | 6,7-difluoro-N-(2-(piperidin-1-yl)-4-(4-(trifluoromethyl)phenethyl)phen yl)heptanamide<br>(6R)-6,7-difluoro-N-(2-(piperidin-1-yl)-4-(4-(trifluoromethyl)phenethyl)phen yl)heptanamide<br>(6S)-6,7-difluoro-N-(2-(piperidin-1-yl)-4-(4-(trifluoromethyl)phenethyl)phen yl)heptanamide |
| 232. | 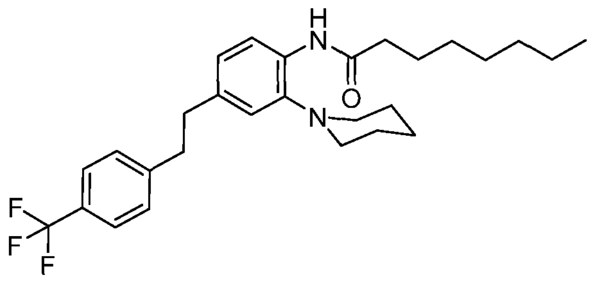 | N-(2-(piperidin-1-yl)-4-(4-(trifluoromethyl) phenethyl)phen yl)octanamide |
| 233. | 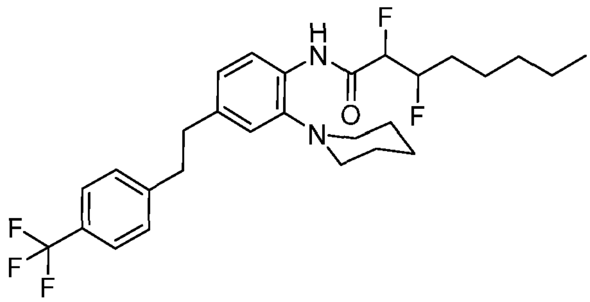 | 2,3-difluoro-N-(2-(piperidin-1-yl)-4-(4-(trifluoromethyl)phenethyl)phen yl)octanamide<br>(2R,3 S)-2,3-difluoro-N-(2-(piperidin-1-yl)-4-(4-(trifluoromethyl)phenethyl)phen yl)octanamide<br>(2S,3R)-2,3-difluoro-N-(2-(piperidin-1-yl)-4-(4-(trifluoromethyl)phenethyl)phen yl)octanamide<br>(2R,3R)-2,3-difluoro-N-(2-(piperidin-1-yl)-4-(4-(trifluoromethyl)phenethyl)phen yl)octanamide<br>(2S,3 S)-2,3-difluoro-N-(2-(piperidin-1-yl)-4-(4-(trifluoromethyl)phenethyl)phen yl)octanamide |
| 234. | 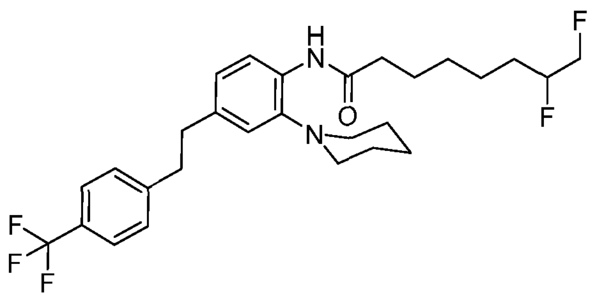 | 7,8-difluoro-N-(2-(piperidin-1-yl)-4-(4-(trifluoromethyl)phenethyl)phen yl)octanamide<br>(7R)-7,8-difluoro-N-(2-(piperidin-1-yl)-4-(4-(trifluoromethyl)phenethyl)phen yl)octanamide<br>(7S)-7,8-difluoro-N-(2-(piperidin-1-yl)-4-(4-(trifluoromethyl)phenethyl)phen yl)octanamide |
| 235. | 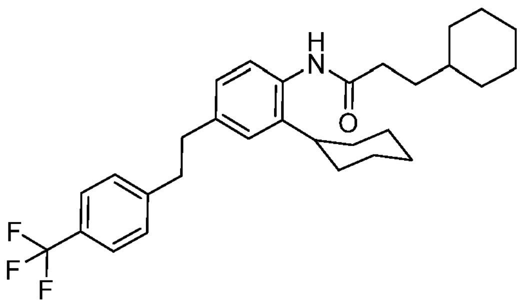 | 3-cyclohexyl-N-(2-cyclohexyl-4-(4-(trifluoromethyl)phenethyl)phen yl)propanamide |

(continued)

| | COMPOUND STRUCTURE | COMPOUND NAME |
|---|---|---|
| 236. | 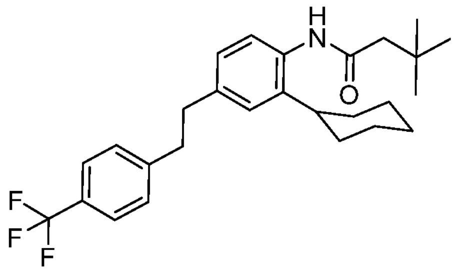 | N-(2-cyclohexyl-4-(4-(trifluoromethyl) phenethyl)phen yl)-3,3-dimethylbutanamide |
| 237. | 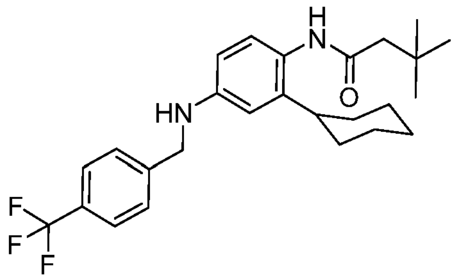 | N-(2-cyclohexyl-4-((4-(trifluoromethyl) benzyl)amino)p henyl)-3,3-dimethylbutanamide |
| 238. | 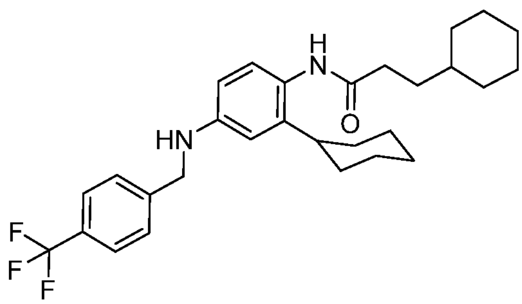 | 3-cyclohexyl-N-(2-cyclohexyl-4-((4-(trifluoromethyl)benzyl)amino)p henyl)propanamide |
| 239. | 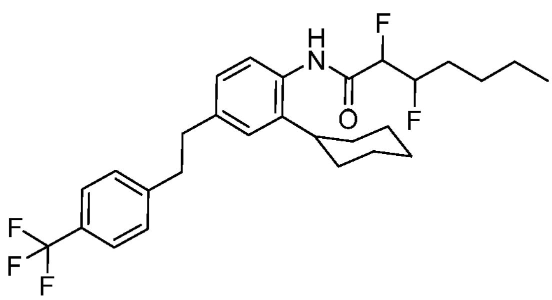 | N-(2-cyclohexyl-4-(4-(trifluoromethyl) phenethyl)phen yl)-2,3-difluoroheptanamide (2R,3 S)-N-(2-cyclohexyl-4-(4-(trifluoromethyl)phenethyl)phen yl)-2,3-difluoroheptanamide (2S,3R)-N-(2-cyclohexyl-4-(4-(trifluoromethyl)phenethyl)phen yl)-2,3-difluoroheptanamide (2R,3R)-N-(2-cyclohexyl-4-(4-(trifluoromethyl)phenethyl)phen yl)-2,3-difluoroheptanamide (2S,3 S)-N-(2-cyclohexyl-4-(4-(trifluoromethyl)phenethyl)phen yl)-2,3-difluoroheptanamide |
| 240. | 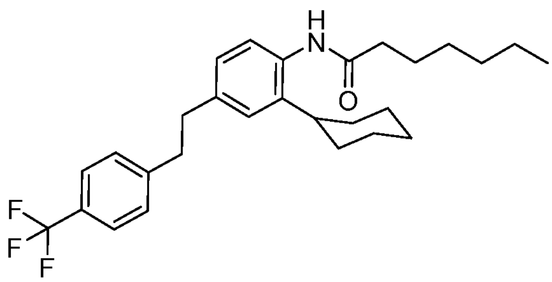 | N-(2-cyclohexyl-4-(4-(trifluoromethyl) phenethyl)phen yl)heptanamide |

(continued)

| | COMPOUND STRUCTURE | COMPOUND NAME |
|---|---|---|
| 241. | 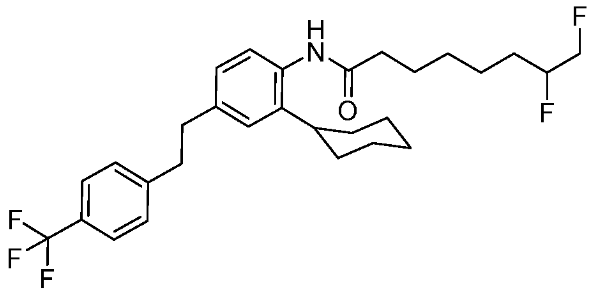 | N-(2-cyclohexyl-4-(4-(trifluoromethyl) phenethyl)phen yl)-7,8-difluorooctanamide (7R)-N-(2-cyclohexyl-4-(4-(trifluoromethyl)phenethyl)phen yl)-7,8-difluorooctanamide (7S)-N-(2-cyclohexyl-4-(4-(trifluoromethyl)phenethyl)phen yl)-7,8-difluorooctanamide |
| 242. | 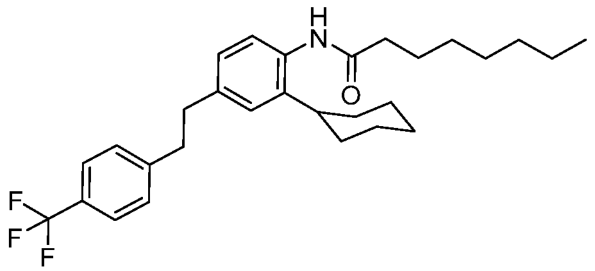 | N-(2-cyclohexyl-4-(4-(trifluoromethyl) phenethyl)phen yl)octanamide |
| 243. | 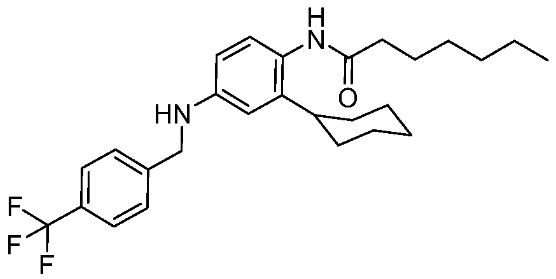 | N-(2-cyclohexyl-4-((4-(trifluoromethyl) benzyl)amino)p henyl)heptanamide |
| 244. | 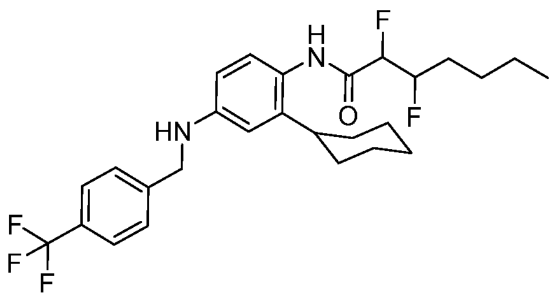 | N-(2-cyclohexyl-4-((4-(trifluoromethyl) benzyl)amino)p henyl)-2,3-difluoroheptanamide (2R,3S)-N-(2-cyclohexyl-4-((4-(trifluoromethyl)benzyl)amino)p henyl)-2,3-difluoroheptanamide (2S,3R)-N-(2-cyclohexyl-4-((4-(trifluoromethyl)benzyl)amino)p henyl)-2,3-difluoroheptanamide (2R,3R)-N-(2-cyclohexyl-4-((4-(trifluoromethyl)benzyl)amino)p henyl)-2,3-difluoroheptanamide (2S,3 S)-N-(2-cyclohexyl-4-((4-(trifluoromethyl)benzyl)amino)p henyl)-2,3-difluoroheptanamide |
| 245. | 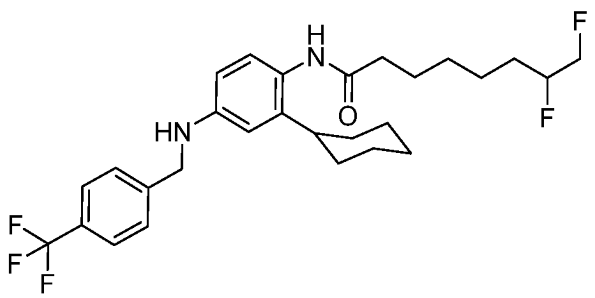 | N-(2-cyclohexyl-4-((4-(trifluoromethyl) benzyl)amino)p henyl)-7,8-difluorooctanamide (7R)-N-(2-cyclohexyl-4-((4-(trifluoromethyl)benzyl)amino)p henyl)-7,8-difluorooctanamide (7S)- N-(2-cyclohexyl-4-((4-(trifluoromethyl)benzyl)amino)p henyl)-7,8-difluorooctanamide |

(continued)

| | COMPOUND STRUCTURE | COMPOUND NAME |
|---|---|---|
| 246. | 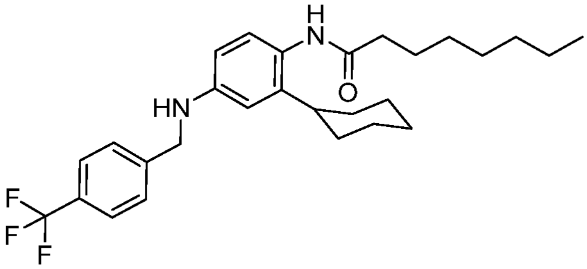 | N-(2-cyclohexyl-4-((4-(trifluoromethyl) benzyl)amino)p henyl)octanamide |
| 247. | 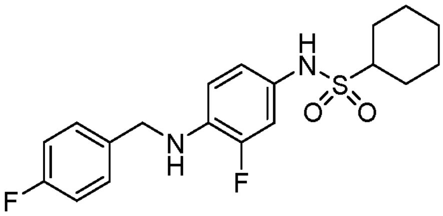 | N-(3-fluoro-4-((4-fluorobenzyl)amino) phenyl)cycl ohexanesulfonamide |
| 248. | 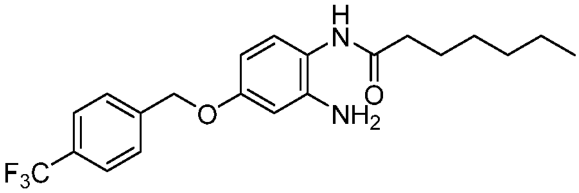 | N-(2-amino-4-((4-(trifluoromethyl)benzyl) oxy)phe nyl)heptanamide |
| 249. | 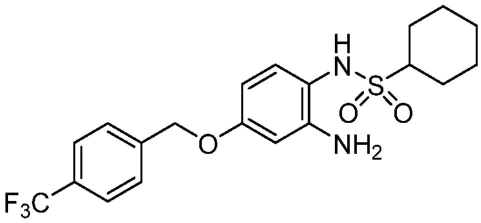 | N-(2-amino-4-((4-(trifluoromethyl)benzyl) oxy)phe nyl)cyclohexanesulfonamide |
| 250. | 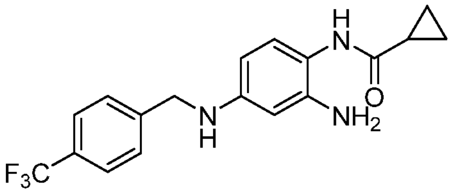 | N-(2-amino-4-((4-(trifluoromethyl)benzyl) amino)p henyl)cyclopropanecarboxamide |
| 251. | 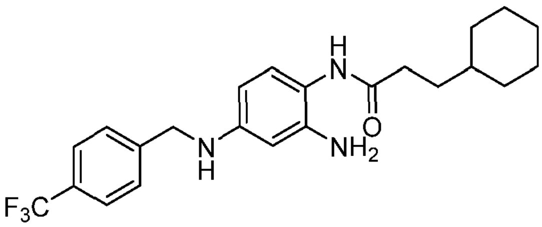 | N-(2-amino-4-((4-(trifluoromethyl)benzyl) amino)p henyl)-3-cyclohexylpropanamide |
| 252. | 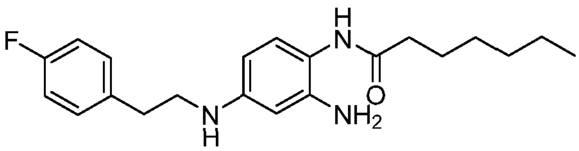 | N-(2-amino-4-((4-fluorophenethyl)amino) phenyl)h eptanamide |

(continued)

| | COMPOUND STRUCTURE | COMPOUND NAME |
|---|---|---|
| 253. | 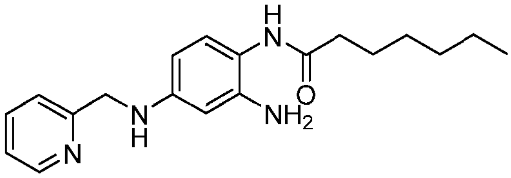 | N-(2-amino-4-((pyridin-2-ylmethyl)amino) phenyl)heptana mide |
| 254. | 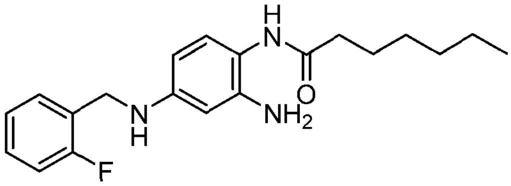 | N-(2-amino-4-((2-fluorobenzyl)amino) phenyl)hept anamide |
| 255. | 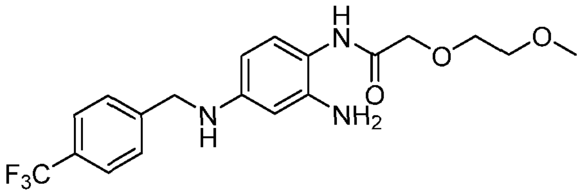 | N-(2-amino-4-((4-(trifluoromethyl)benzyl) amino)p henyl)-2-(2-methoxyethoxy) acetamide |
| 256. | 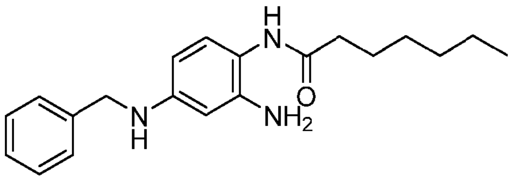 | N-(2-amino-4-(benzylamino)phenyl)hep-tanami de |
| 257. | 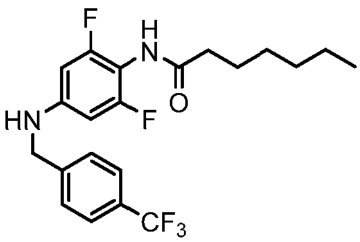 | N-(2,6-difluoro-4-((4-(trifluoromethyl)benzyl)amino)p henyl)heptanamide |
| 258. | 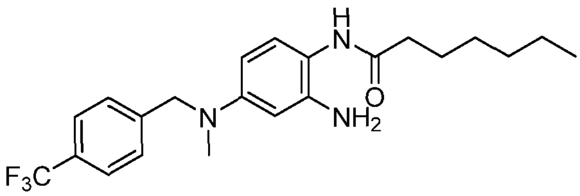 | N-(2-amino-4-(methyl(4-(trifluoromethyl) benzyl)amino)p henyl)heptanamide |
| 259. | 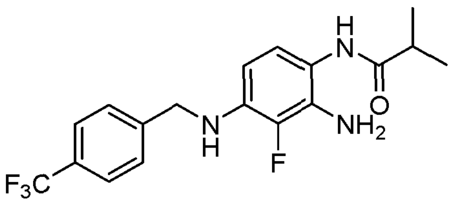 | N-(2-amino-3-fluoro-4-((4-(trifluoromethyl)benzyl)amino)p henyl)isobutyra-mide |
| 260. | 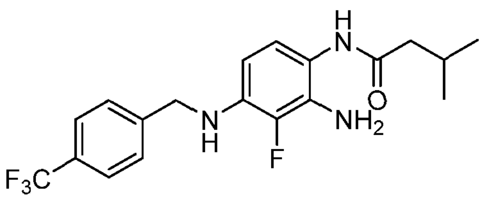 | N-(2-amino-3-fluoro-4-((4-(trifluoromethyl)benzyl)amino)p henyl)-3-methyl-butanamide |

(continued)

| | COMPOUND STRUCTURE | COMPOUND NAME |
|---|---|---|
| 261. | 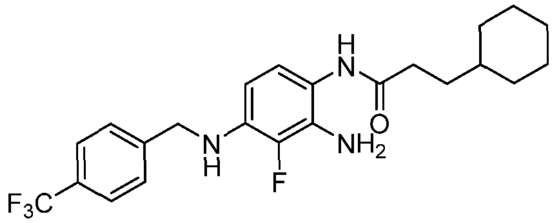 | N-(2-amino-3-fluoro-4-((4-(trifluoromethyl)benzyl)amino)p henyl)-3-cyclo-hexylpropanamide |
| 262. | 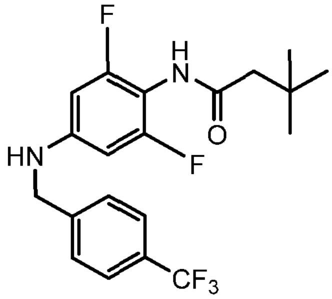 | N-(2,6-difluoro-4-((4-(trifluoromethyl)benzyl)amino)p henyl)-3,3-dimethylbutana-mide |
| 263. | 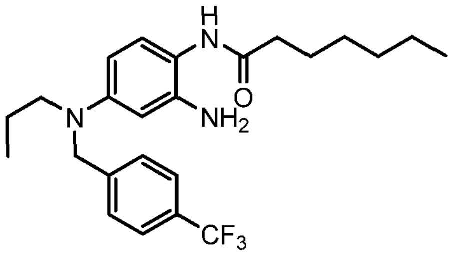 | N-(2-amino-4-(propyl(4-(trifluoromethyl) benzyl)amino)p henyl)heptanamide |
| 264. | 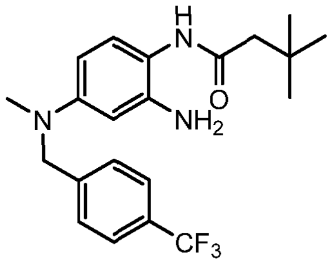 | N-(2-amino-4-(methyl(4-(trifluoromethyl) benzyl)amino)p henyl)-3,3-dimethylbuta-namide |
| 265. | 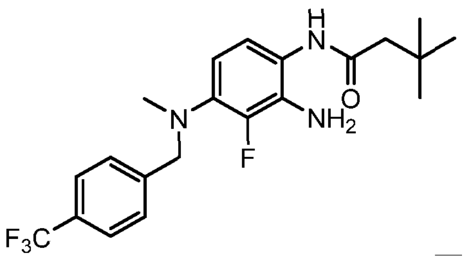 | N-(2-amino-3-fluoro-4-(methyl(4-(trifluoromethyl)benzyl)amino)p henyl)-3,3-di-methylbutanamide |
| 266. | 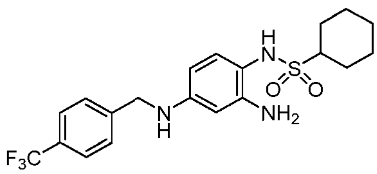 | N-(2-amino-4-((4-(trifluoromethyl)benzyl) amino)p henyl)cyclohexanesulfonamide |

(continued)

| | COMPOUND STRUCTURE | COMPOUND NAME |
|---|---|---|
| 267. | 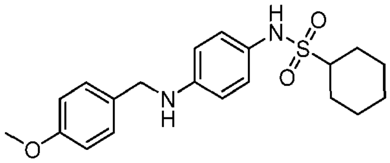 | N-(4-((4-methoxybenzyl)amino)phenyl) cyclohexanesulfonamide |
| 268. | 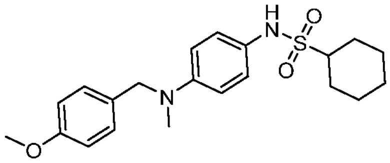 | N-(4-((4-methoxybenzyl)(methyl)amino)p henyl) cyclohexanesulfonamide |
| 269. | 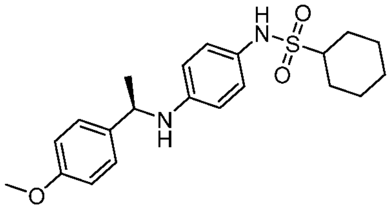 | (R)-N-(4-((1-(4-methoxyphenyl)ethyl)amino)phe nyl) cyclohexanesulfonamide |
| 270. | 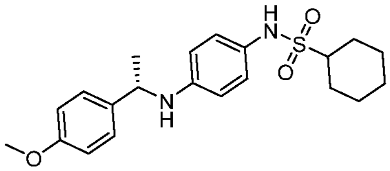 | (S)-N-(4-((1-(4-methoxyphenyl)ethyl)amino)phe nyl) cyclohexanesulfonamide |
| 271. | 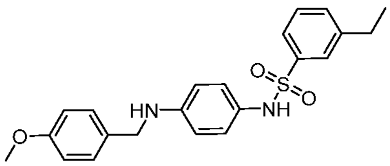 | 3-ethyl-N-(4-((4-methoxybenzyl)amino) phenyl)b enzenesulfonamide |

In a preferred embodiment, the compound or tautomer, salt, solvate, stereoisomer or isotope thereof as defined above is a voltage-gated potassium channel modulator.

Preferably the compound or tautomer, salt, solvate, stereoisomer or isotope thereof as defined above is a Kv7.1, Kv7.2, Kv7.3, Kv7.4 and/or Kv7.5 modulator.

Still more preferably, the compound or tautomer, salt, solvate, stereoisomer or isotope thereof as defined above is a Kv7.2 and Kv7.3 modulator.

In another preferred embodiment, the compound or tautomer, salt, solvate, stereoisomer or isotope thereof as defined above is for use as a medicament.

Preferably, the compound or tautomer, salt, solvate, stereoisomer or isotope thereof as defined above is for use in the treatment and/or prevention of a condition characterised by a defect in the structure and/or function of at least one voltage-gated potassium channel. Preferably, Kv7.1, Kv7.2, Kv7.3, Kv7.4 and/or Kv7.5, more preferably of both Kv7.2 and Kv7.3.

In another preferred embodiment, the compound or tautomer, salt, solvate, stereoisomer or isotope thereof as defined above is for use as an anticonvulsant, analgesic, anti-ischemic, anti-arrhythmic, neuroprotective, anti-tinnitus, antiasthmatic, gastrointestinal motility modulator, antihypertensive, antimyotonic and/or anti-skeletal muscle dystrophia medicament.

It is another object of the present invention a pharmaceutical composition comprising the compound or tautomer, salt, solvate, stereoisomer or isotope thereof as defined above and a pharmaceutically acceptable carrier.

Preferably, said pharmaceutical composition comprises a further therapeutic agent selected from: an anticonvulsivant, an analgesic and a nonsteroidal anti-inflammatory drug (NSAID).

Preferably, said anticonvulsant is selected from the group consisting of: Acetazolamide, Brivaracetam, Carbamazepine, Clobazam, Clonazepam, Diazepam, Eslicarbazepine, Ethosuximide, Fosphenytoin, Gabapentin, Lacosamide, Lamotrigine, Levetiracetam, Lorazepam, Methosuximide, Nitrazepam, Oxcarbazepine, Perampanel, Phenobarbital, Phenytoin, Pregabalin, Primidone, Rufinamide, Stiripentol, Topiramate, Valproic Acid, Vigabatrin, Felbamate, Tiagabine and Zonisamide.

Preferably, said analgesic is acetaminophen or an opioid.

Preferably, said nonsteroidal anti-inflammatory drug (NSAID) is selected from the group consisting of: aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, meloxicam, nabumetone, naproxen, nimesulide, nitroflurbiprofen, olsalazine, oxaprozin, phenylbutazone, piroxicam, sulfasalazine, sulindac, tolmetin and zomepirac. Preferably, said opioid is morphine.

In a preferred embodiment, the pharmaceutical composition as defined above is for use in the treatment and/or prevention of a condition characterised by a defect in the structure and/or function of at least one voltage-gated potassium channel. Preferably, at least one of Kv7.1, Kv7.2, Kv7.3, Kv7.4 and/or Kv7.5, more preferably of both Kv7.2 and Kv7.3.

In another preferred embodiment, the pharmaceutical composition as defined above is for use as an anticonvulsant, analgesic, anti-ischemic, anti-arrhythmic, neuroprotective, anti-tinnitus, antiasthmatic, gastrointestinal motility modulator, antihypertensive, antimyotonic and/or anti-skeletal muscle dystrophia medicament.

Preferably, said condition characterised by a defect in the structure and/or function of at least one voltage-gated potassium channel is selected from the group consisting of: a central nervous system disease, a peripheral nervous system disease, a sensory system disease, a respiratory system disease, a genitourinary system disease, a gastrointestinal system disease, a cardiovascular disease, a skeletal muscle disease and a channelopathy.

Also preferably, said condition characterised by a defect in the structure and/or function of at least one voltage-gated potassium channel is selected from the group consisting of: epilepsy, a convulsive disorder, a seizure, a seizure disorder, a disorder characterised by hyperexcitability of the nervous system, a neonatal epilepsy, spontaneous contractions, an early-onset epileptic disorder, Ohtahara syndrome, early infantile epileptic encelopathy with suppression-burst, benign familiar neonatal seizures, epileptic encelopathy, severe sporadic neonatal epileptic encelopathy, an intellectual disability, a psychiatric or cognitive comorbidity, a cognitive defect, anxiety, depression, schizophrenia, sudden death in epilepsy, drug-resistant epilepsy, neuropathic pain, an ischemic stroke, angina, migraine, a neurodegenerative disorder, a neurologic disorder, amyotrophic lateral sclerosis, Alzheimer's disease, Parkinson's disease, tremors, paroxysmal choreoathetosis, tinnitus, pain, asthma, bladder hyper-reactivity, urinary incontinence, constipation, irritable bowel syndrome, diarrhea, Krohn's disease, hypertension, vascular hypertension, muscular dystrophy, a skeletal muscle hyperexcitability disease, myokymia, myotonia, a genetic channelopathy, a transcriptional channelopathy, a Kv7.2- and/or Kv7.3-related disorder, a Kv 7.2 encelopathy, autosomal dominant type 2 deafness (DFNA2), ataxia, episodic ataxia type 1, episodic ataxia with myokymia syndrome, neuromyotonia, hypokalaemic periodic paralysis, an arrhythmia, a congenital arrhythmia, a long QT syndrome, Romano-Ward syndrome, Jervell and Lange-Nielsen syndrome, Bartter syndrome, hyperinsulinemic hypoglycaemia of infancy (HHI), attenuate vasoconstrictor response, type 2 diabetes and multiple sclerosis.

In yet another preferred embodiment, the compound for use as defined above or the pharmaceutical composition for use as defined above is administered to an animal. Preferably, said animal is a companion animal or livestock.

The present invention also provides the compound or tautomer, salt, solvate, stereoisomer or isotope thereof as defined above for use in a method of modulating a voltage-gated potassium channel. Preferably, at least one of Kv7.1, Kv7.2, Kv7.3, Kv7.4 and/or Kv7.5. More preferably, the compound or tautomer, salt, solvate, stereoisomer or isotope thereof as defined above is for use in a method of modulating Kv7.2 and Kv7.3.

It is also an object of the present invention an *in-vitro* method of modulating a voltage-gated potassium channel by using the compound or tautomer, salt, solvate, stereoisomer or isotope thereof as defined above. Preferably, Kv7.1, Kv7.2, Kv7.3, Kv7.4 and/or Kv7.5 More preferably, said *in-vitro* method is an *in-vitro* method of modulating Kv7.2 and Kv7.3 by using the compound or tautomer, salt, solvate, stereoisomer or isotope thereof as defined above.

As used herein, the term "alkylcarbonyl" preferably refers to groups such as $CH_3(CH_2)_nCO$ where n is a number comprised between 0 and 10 and to branched isomers thereof. Examples of "alkylcarbonyl" include acetamide, $CH_3(CH_2)_6CO$, $(CH_3)_3CCH_2CO$, $CH_3(CH_2)_2CH(CH_3)CO$, $(CH_3)_3C(CH_2)_6CO$, $CH_3(CH_2)_5C(CH_3)_3CO$, $CH_3(CH_2)_4C(CH_3)_3CH_2CO$.

As used herein, the term "alkoxycarbonyl" preferably refers to groups such as $CH_3(CH_2)_nOCO$ where n is a number comprised between 0 and 10 and to branched isomers thereof. Examples of "alkoxycarbonyl" include $CH_3(CH_2)_6OCO$, $(CH_3)_3CCH_2OCO$, $CH_3(CH_2)_2CH(CH_3)OCO$, $(CH_3)_3C(CH_2)_6OCO$, $CH_3(CH_2)_5C(CH_3)_3OCO$, $CH_3(CH_2)_4C(CH_3)_3CH_2OCO$.

As used herein, the term "alkylamino", preferably refers to linear or branched alkyl groups bearing a NH or $NH_2$ substitutent, preferably a terminal NH or $NH_2$ substitutent. Preferred alkylamino groups include groups such as $CH_3(CH_2)_nNH$ where n is a number comprised between 0 and 10 and their branched isomers, as well as groups such as $NH_2(CH_2)_nCH_2$ where n is a number comprised between 0 and 10 and their branched isomers. "Alkylamino" as used herein also preferably refers to C1-C10 linear or branched alkyl chains bearing a primary, secondary or tertiary amine as side

chain. Examples of "alkylamino" include NHMe, NHEt, NHiPr, $N(Me)_2$, $N(Et)_2$, $N(iPr)_2$, $CH_3(CH_2)_6NH$, $(CH_3)_3CCH_2NH$, $CH_3(CH_2)_2CH(CH_3)NH$, $(CH_3)_3C(CH_2)_6NH$, $CH_3(CH_2)_5C(CH_3)_3NH$, $CH_3(CH_2)_4C(CH_3)_3CH_2NH$, $NH_2CH_2(CH_2)_5CH_2$, $[NH_2(CH_3)_2]CCH_2NH$, $NH_2CH_2(CH_2)_2CH(CH_3)$, $NH_2(CH_3)_2C(CH_2)_6$, $NH_2CH_2(CH_2)_5C(CH_3)_2$, $NH_2CH_2(CH_2)_4C(CH_3)_3CH_2$, $CH_3CHNH_2(CH_2)_5$, $CH_3(CH_2)_2CNH_2(CH_2)_2$, $CH_3(CH_2)_2CH_2[N(CH_3)_2]$, $CH_3(CH_2)_2C[N(CH_3)_2](CH_2)_2$, $CH_3(CH_2)_2C[NH(CH_3)](CH_2)_2$.

The term "alkenylamino" as used herein refers to a linear or branched alkylamino group as defined above comprising at least one carbon-carbon double bond.

The term "alkynylamino" as used herein refers to a linear or branched alkylamino group as defined above comprising at least one carbon-carbon triple bond.

As used herein, the term "alkylaminocarbonyl" preferably refers to groups such as $NH_2(CH_2)_nCH_2CO$ where n is a number comprised between 0 and 10 and to branched isomers thereof. "Alkylaminocarbonyl" as used herein also preferably refers to C3-C10 linear or branched $CH_3X(CH_2)_nCO$ or $CH_3(CH_2)_nX(CH_2)_nCO$ where X is a primary, secondary or tertiary amine and n is comprised between 1 and 10. Examples of "alkylaminocarbonyl" include $NH_2CH_2(CH_2)_5CH_2CO$, $NH_2C(CH_3)_2CH_2CO$, $NH_2CH_2(CH_2)_2CH(CH_3)CO$, $NH_2(CH_3)_2C(CH_2)_6CO$, $NH_2CH_2(CH_2)_5C(CH_3)_3CO$, $NH_2CH_2(CH_2)_4C(CH_3)_3CH_2CO$, $CH_3CH(NH_2)(CH_2)_5CO$, $CH_3(CH_2)_2C(NH_2)(CH_2)_2CO$, $CH_3(CH_2)_2CH_2[N(CH_3)_2]CO$, $CH_3(CH_2)_2C[N(CH_3)_2](CH_2)_2CO$, $CH_3(CH_2)_2CH[NH(CH_3)](CH_2)_2CO$.

As used herein, the term "alkyloxy" or "alkoxy" refers to groups bearing at least one oxygen atom linked to an alkyl chain. Preferred alkyloxy groups include groups such as: $CH_3(CH_2)_nO$ where n is a number comprised between 0 and 10 and their branched isomers, $HO(CH_2)_nCH_2$ where n is a number comprised between 0 and 10 and their branched isomers, as well as groups such as $CH_3X(CH_2)_nCH_2$, $CH_3(CH_2)_nXCH_2$ and $CH_3(CH_2)_nX(CH_2)_n$ where X is an oxygen atom and n is comprised between 1 and 10. Examples of "alkyloxy" or "alkoxy" include methoxy, ethoxy, $HOCH_2(CH_2)_5CH_2$, $HO(CH_3)_2CCH_2$, $HOCH_2(CH_2)_2CH(CH_3)$, $HO(CH_3)_2C(CH_2)_6$, $HOCH_2(CH_2)_5C(CH_3)_3$, $HOCH_2(CH_2)_4C(CH_3)_3CH_2$, $HOCH_2CH_2O(CH_2)_5$, $CH_3(CH_2)_2C(OH)(CH_2)_2$, $CH_3(CH_2)_2CH_2OCH_3$, $CH_3(CH_2)_2C(OCH_3)(CH_2)_2$, $CH_3(CH_2)_2C(OCH_3)(CH_2)_2$, $CH_3(CH_2)_6O$, $(CH_3)_3CCH_2O$, $CH_3(CH_2)_2CH(CH_3)O$, $(CH_3)_3C(CH_2)_6O$, $CH_3(CH_2)_5C(CH_3)_3O$, $CH_3(CH_2)_4C(CH_3)_3CH_2O$, $CH_3CH_2OCH_2CH_2OH$, $CH_3CH_2OCH_2CH_2O$, $CH_2CH_2OCH_2CH_2O$.

As used herein, the term "alkylthio" refers to groups bearing at least one sulfur atom linked to an alkyl chain. Preferred alkylthio groups include groups such as: SMe, SEt, $CH_3(CH_2)_nS$ where n is a number comprised between 0 and 10 and their branched isomers, $HS(CH_2)_nCH_2$ where n is a number comprised between 0 and 10 and their branched isomers, as well as groups such as $CH_3X(CH_2)_nCH_2$, $CH_3(CH_2)_nXCH_2$ or $CH_3(CH_2)_nX(CH_2)_n$ where X is a sulfur atom and n is comprised between 1 and 10. Examples of "alkylthio" include $HSCH_2(CH_2)_5CH_2$, $HS(CH_3)_2CCH_2CO$, $HSCH_2(CH_2)_2CH(CH_3)$, $HS(CH_3)_2C(CH_2)_6$, $HSCH_2(CH_2)_5C(CH_3)_3$, $HSCH_2(CH_2)_4C(CH_3)_3CH_2$, $HSCH_2CH_2S(CH_2)_5$, $CH_3(CH_2)_2CSH(CH_2)_2$, $CH_3(CH_2)_2CH_2SCH_2$, $CH_3(CH_2)_2C(SCH_3)(CH_2)_2$, $CH_3(CH_2)_2C(SCH_3)(CH_2)_2$, $CH_3(CH_2)_6S$, $(CH_3)3CCH_2S$, $CH_3(CH_2)_2CH(CH_3)S$, $(CH_3)_3C(CH_2)_6S$, $CH_3(CH_2)_5C(CH_3)_3S$, $CH_3(CH_2)_4C(CH_3)_3CH_2S$, $CH_3CH_2S(CH_2)_5$, $CH_3CH_2SCH_2CH_2SH$, $CH_3CH_2SCH_2CH_2SCH_2CH_2SCH_2CH_2S$.

As used herein, the term "carbonyl" preferably refers to C1-C10 aliphatic aldheydhes or ketones. Examples of "carbonyl" include $HOCCH_2(CH_2)_5CH_2$, $HOC(CH_3)_2CCH_2CO$, $HOCCH_2(CH_2)_2CH(CH_3)$, $HOC(CH_3)_2C(CH_2)_6$, $HOCCH_2(CH_2)_4C(CH_3)_3$, $HOCCH_2(CH_2)_3C(CH_3)_3CH_2$, $HOCCH_2CH_2O(CH_2)_5$, and $CH_3(CH_2)_nCO(CH_2)_m$ groups where n is a number comprised between 0 and 10 and m is a number comprised between 1 and 10 and branched isomers thereof.

As used herein, the term "amide" preferably refers to groups such as $CH_3CONH$, $CH_3(CH_2)_nCONH$, $H_2NCO(CH_2)_n$ where n is a number comprised between 0 and 10 and to branched isomers thereof. In the present invention, amide functionalities are preferably substituted at the nitrogen position.

As used herein, the term "carboxyl", preferably refers to groups such as $XOOC(CH_2)_n$ where n is a number comprised between 0 and 10, X is selected between H and alkyl chain and to branched isomers thereof. "Carboxyl" also preferably refers to groups such as $CH_3(CH_2)_nCH_2(COOX)$ where X is selected between H and alkyl chain, and to branched isomers.

As used herein, the term "amine" preferably refers to groups such as $NH_2$, NHX, $NX_1X_2$ where $X_1$ and $X_2$ represents any of the groups described above, in particular linear or branched alkyl chains. As used herein, the terms "aryl" or "aromatic ring" are used interchangeably and refer to a monocyclic, bicyclic or polycyclic aromatic ring system comprising carbon atoms and hydrogen atoms, preferably comprising from 5 to 10 carbon atoms. In the present invention, aromatic rings optionally comprise as members of the ring one or more heteroatoms, preferably 1 to 3 heteroatoms, preferably selected from N, O and S, in which case they are also referred to as "heteroaryl" or "heteroaromatic rings". Aryl groups of the present invention only need to have some degree of aromatic character. Preferred aryl groups are formed by a phenyl ring and/or phenyl rings optionally substituted in one position or in more than one position, or in any possible position by any kind of possible substituents. Examples of "aryl" include benzene, naphthalene, anthracene, pyridine, quinoline, isoquinoline, pyrazine, quinoxaline, acridine, pyrimidine, quinazoline, pyridazine, cinnoline, phthalazine, 1,2,3-triazine, 1,2,4-triazine, 1,3,5 triazine, furan, benzofuran, isobenzofuran, pyrrole, indole, isoindole, thiophene, benzothiophene, benzo[c]thiophene, imidazole, benzoimidazole, purine, pyrazole, indazole, oxazole, benzoxazole, isoxazole,

benzoisoxazole, thiazole, benzothiazole. Preferably, aromatic rings are phenyl, pyridine and pyrrole. or Such aryls may optionally be substituted with one or more substituents, preferably selected from alkyloxy, nitrile, amino, hydroxyl, halogen, trifluoroalkyl, trichloroalkyl, difluoroalkyl, dichloroalkyl, alkylcarbonyl, trifluoromethoxy, alkyl alkoxycarbonyl, alkylamino, alkylaminocarbonyl, alkylthio, carbonyl, amide and/or carboxyl.

As used herein, the term "cycloalkyl" refers to a saturated hydrocarbon ring, preferably bearing from 3 to 8 cabon atoms, more preferably bearing from 3 to 6 carbon atoms, that can be unsubstituted or substituted in one, more than one or any of the possible position by any kind of possible substituents. Examples of cycloalkyl include cyclopropyl, cyclobutyl, cyclopentyl, cyclohexyl, cyclohexyl, cycloheptyl, cyclooctyl, norbornane, optionally substituted with one or more substituents, preferably selected from: alkyloxy, nitrile, amino, hydroxyl, halogen, trifluoroalkyl, trichloroalkyl, difluoroalkyl, dichloroalkyl, alkylcarbonyl, trifluoromethoxy, alkyl, alkoxycarbonyl, alkylamino, alkylaminocarbonyl, alkylthio, carbonyl, amide and carboxyl.

As used herein, the term "heterocycle", refers to a saturated or partially saturated aliphatic ring, preferably bearing from 3 to 7 carbon atoms and comprising as ring members one or more than one heteroatoms, preferably selected from N, O and S. Heterocycles can be unsubstituted or substituted in one, more than one or any of the postion possible position by any kind of possible substituents. Examples of "heterocycle" comprise piperidine, quinoline, decahydroquinoline decahydroisoquinoline, pyperazine, decahydroquinoxaline, tetradecahydroacridine, hexahydropyrimidine, decahydroquinazoline, hexahydropyridazine, decahydrocinnoline, decahydrophthalazine, tetrahydrofuran, octahydrobenzofuran, octahydroisobenzofuran, pyrrolidine, octahydro-1H-indole, octahydro-1H-isoindole, tetrahydrothiophene, octahydrobenzothiophene, octahydrobenzo[c]thiophene, imidazoline, inidazolidine, octahydrobenzoimidazole, octahydro-1H-purine, octahydro-1H-indazole, oxazolidine, octahydrobenzoxazole, isoxazolidine, octahydrobenzoisoxazole, thiazolidine, octahydrobenzothiazole optionally substituted with one or more substituents, preferably selected from: alkyloxy, nitrile, amino, hydroxyl, halogen, trifluoroalkyl, trichloroalkyl, difluoroalkyl, dichloroalkyl, alkylcarbonyl, trifluoromethoxy, alkyl, alkoxycarbonyl, alkylamino, alkylaminocarbonyl, alkylthio, carbonyl, amide and carboxyl.

As used herein, the term "alkyl" refers to a linear or branched hydrocarbon chain. Suitable examples of said alkyl include but are not limited to methyl, ethyl, n-propyl, isopropyl, butyl, sec-butyl, tert-butyl, pentyl, isopentyl, tert-pentyl, hexyl, heptyl, octyl, nonyl, decanyl, hexadecanyl, eicosanyl, $CH_2C(CH_3)_3$, $CH_2CH(CH_3)_2$ etc. Long alkyl chains, e.g. C5-30 alkyl, preferably C6-C30 alkyl, more preferably C7-C30 alkyl, still preferably C10-C30 alkyl, are particularly preferred in some embodiments of the invention.

As used herein, the term "alkenyl" refers to a linear or branched alkyl chain as defined above comprising at least one carbon-carbon double bond. Long alkenyl chains, e.g. C5-30 alkenyl, preferably C6-C30 alkenyl, more preferably C7-C30 alkenyl, still preferably C10-C30 alkenyl, are particularly preferred in some embodiments of the invention.

The term "alkynyl" as used herein refers to a linear or branched alkyl group as defined above comprising at least one carbon-carbon triple bond. Long alkynyl chains, e.g. C5-30 alkynyl, preferably C6-C30 alkynyl, more preferably C7-C30 alkynyl, still preferably C10-C30 alkynyl, are particularly preferred in some embodiments of the invention.

As used herein, the term "haloalkyl" refers to a linear or branched alkyl group, wherein at least one hydrogen atom is replaced by a halogen atom, preferably fluorine and/or chlorine. Preferred haloalkyl groups comprise 1-3 carbon atoms. Examples of "haloalkyl" include difluoroalkyl, trifluoroalkyl, trichloroalkyl, difluoroalkyl, dichloroalkyl, 2,2,2 trifluoroethyl, such as $CF_3$, $CHF_2$, $CH_2F$, $CCl_3$, $CHCl_2$, $CH_2Cl$.

As used herein, the term "haloalkoxy" refers to a linear or branched alkoxy group, wherein at least one hydrogen atom is replaced by a halogen atom, preferably fluorine and/or chlorine. Preferred haloalkoxy groups comprise 1-3 carbon atoms. Examples of "haloalkoxy" include trifluoroalkoxyl, trichloroalkoxyl, difluoroalkoxyl, dichloroalkoxyl, such as $OCF_3$, $OCHF_2$, $OCH_2F$, $OCCl_3$, $OCHCl_2$, $OCH_2Cl$.

Preferably, halogen is fluorine, chlorine or bromine.

As used herein, the term "linear or branched C6-C30 arylalkyl" refers to aryl groups, as previously defined, bearing linear or branched alkyl chain substituents in one, more or all possible positions. As used herein, the term "C3-C7 cycloalkylamino" refers to cycloalkyl groups, as previously defined, bearing one or more amines and/or alkylamines and/or dialkylamines as substituents. As used herein, the term "C7-C15 arylalkylamino" refers to arylalkyl groups, as previously defined, bearing one or more amines and/or alkylamine and/or dialkylamines as substituents.

As used herein, the term "C7-C15 arylalkenylamino" refers to to aryl groups, as previously defined, bearing linear or branched alkenyl chain, as defined above, and further substituted by one or more amines and/or alkylamine and/or dialkylamines.

As used herein, the term "C7-C15 arylalkynylamino" refers to to aryl groups, as previously defined, bearing one or more linear or branched alkynyl chain, as defined above, and further substituted by one or more amines and/or alkylamine and/or dialkylamines.

It is to be understood that in the present invention any aliphatic substituent, such as alkyl, cycloalkyl, alkylcarbonyl, alkoxycarbonyl, alkylamino, alkylaminocarbonyl, alkyloxy, alkylthio, alkenyl, cycloalkenyl, alkynyl, may be linear or branched.

In a preferred embodiment, compounds of the invention are voltage-gated potassium channel modulators.

Hence, they are useful in the treatment of a condition or disorder that is responsive to the modulation of voltage-gated potassium channels, preferably to the blocking or the opening of voltage-gated potassium channels. Compounds of the invention may also be useful in the treatment of a voltage-gated potassium channel-mediated disease or disorder. Such condition, disease or disorder may also be referred to as a condition characterised by a defect in the structure and/or function of at least one potassium channel.

In the present invention, a potassium channel is preferably a voltage-gated potassium channel, more preferably Kv7.1 (KCNQ1; NM_000218.2), Kv7.2 (KCNQ2; NM_004518.4), Kv7.2/3, Kv7.3 (KCNQ3; NM_004519.2), Kv7.4 (KCNQ4; NM_004700.4) or Kv7.5 (KCNQ5; NM_019842.3), still more preferably Kv7.2 (KCNQ2), Kv7.2/3, Kv7.3 (KCNQ3), Kv7.4 or Kv7.5. Particularly preferred potassium channels are Kv7.2 (KCNQ2) and Kv7.3 (KCNQ3).

In different human tissues, various classes of voltage-gated potassium channels are expressed, each with peculiar functional and pharmacological properties, reflecting the large genetic heterogeneity of this protein class, with over 70 human genes encoding for voltage-gated potassium channel subunits. Given the overall structural similarity among the different voltage-gated potassium channels, common also to other types of ion channels with different gating and selectivity properties (namely, non voltage-gated potassium channels and sodium and calcium channels), in a preferred embodiment, compounds of the present invention can modulate other ionic channels, such as non voltage-gated potassium channels and sodium and calcium channels.

"Modulation", as used herein in its various forms, comprises antagonism, agonism, partial antagonism and/or partial agonism of the activity associated with voltage-gated potassium channels. In particular, "modulation", as used herein in its various forms, includes opening, activating, closing, blocking, partial opening, partially-activating, partial closing or partial bocking of voltage-gated potassium channels.

As used herein, the term "voltage-gated potassium channel modulator" includes compounds able to increase or decrease the basal potassium currents, or otherwise able to increase or decrease the channel opening. Moreover, as used herein, the term "voltage-gated potassium channel modulator" refers to a compound able to modulate prevalently but not exclusively voltage-gated potassium channels.

In an embodiment, compounds of the invention are useful as voltage-gated potassium channel blockers. As used herein, the term "antagonist" or "blocker" includes a molecule that blocks the ionic current(s) carried by an ionic channel. In particular, voltage-gated potassium channel antagonists can be compounds that bind to and decrease, close, deactivate, or hamper voltage-gated potassium channel activity. Antagonism of voltage-gated potassium channels may include either or both of: (1) decreasing current through a voltage-gated potassium channel; or (2) shifting the half-activation potential of voltage-gated potassium channels to more positive voltages (i.e. a depolarizing shift of the $V_{1/2}$ for activation).

In another embodiment, compounds of the invention are useful as voltage-gated potassium channel openers. As used herein, the term "agonist" or "opener" includes a molecule that enchances the ionic current(s) carried by an ionic channel. In particular, voltage-gated potassium channel agonists can be compounds that bind to and stimulate, increase, open, activate, or facilitate voltage-gated potassium channel activity. Activation of voltage-gated potassium channels may include either or both of: (1) increasing current through a voltage-gated potassium channel; or (2) shifting the half-activation potential of voltage-gated potassium channels to less depolarized voltages (i.e. a hyperpolarizing shift of the $V_{1/2}$ for activation).

Example 7 provides an illustrative procedure to discriminate whether a compound acts as a modulator, in particular as an antagonist or as an agonist, of a voltage-gated potassium channel. In particular, a compound may be classified as a voltage-gated potassium channel antagonist when it possesses a value of $I_{drug}/I_{ctl}$, as defined in Example 7, < 1 and a compound may be classified as a voltage-gated potassium channel agonist when it possesses a value of $I_{drug}/I_{ctl}$, as defined in Example 7, > 1.

Kv7 channels are directly implicated in genetic channelopathies, namely human monogenic diseases caused by specific mutations in the primary sequence of changes Kv7 genes; these include congenital arrhythmias, such as the long QT syndrome, epileptic and non-epileptic diseases known altogether as Kv7.2- and Kv7.3-related disorders, and autosomal dominant type 2 deafness (DFNA2), a progressive form of sensorineural hearing loss. In addition to these rare monogenic diseases, and given their prominent role in controlling neuronal, smooth muscle, and skeletal muscle excitability, changes in the expression of Kv7 channels underlie diverse physiopathologial conditions (so-called transcriptional channelopathies) including, but not limited to: epilepsy, neuropathic pain, ischemic stroke, amyotrophic lateral sclerosis, and several other neurodegenerative diseases, for the neuronal component; vascular hypertension, asthma and bladder hyperreactivity for the smooth muscle component; myotonia, myokymia and other skeletal muscle hyperexcitability diseases. All such conditions may be prevented and/or treated using compounds of the present invention or pharmaceutical compositions comprising them.

As used herein, "defect in the structure and/or function of at least one voltage-gated potassium channel" includes mutations in genes coding for voltage-gated potassium channels and changes in the expression of voltage-gated potassium channels.

Preferably, a defect in the structure and/or function of at least one voltage-gated potassium channel is a mutation occurring de novo or inherited by one of the parents which produces gain-of-function or loss-of-function.

Also preferably, a defect in the structure and/or function of at least one voltage-gated potassium channel, which is responsible for changes in the function of specific ion channels, involves accessory subunits or small molecules mostly acting as cytoplasmic regulators, known to interfere with the $K^+$ channel by direct or indirect mechanisms. For example, for Kv7.2 and Kv7.3, such accessory subunits or small molecules include but are not limited to: PIP2 (Zhang H et al., Neuron 2003, 37: 963-975,), the Calmodulin (CaM) (Yus-Najera E et al., JBC 2002, 277: 28545-28553; Wen H et al., J Neurosci 2002, 22: 7991-8001), the protein kinase A anchoring (AKAP) (Hoshi N et al., Nat Neurosci 2003, 6: 564-571), the Ankyrin G (Ank-G), which regulates the subcellular distribution in level of the axonal compartment (Pan Z et al., J Neurosci 2006, 26: 2599-2613) and the ligase ubiquitin Nedd4-2 (Ekberg J et al., JBC 2007, 282: 12135-12142).

As used herein, "pain" includes disorders or conditions related to pain, such as neuropathic pain (including diabetic polyneuropathy), nociceptive pain, persistent pain, osteoarthritic pain, cancer pain, inflammatory pain, postoperative pain, fibromyalgia, chronic widespread pain, musculoskeletal pain, myofascial pain, Temporomandibular joint pain (TMJ pain).

Compounds of the invention may be used to modulate the M-current, for instance to increase or to decrease the M-current. Therefore, the present invention provides compounds of formula I or II as defined above for use in modulating the M-current, especially in a patient characterized by aberrant M-current.

Compounds of the invention may also be used in a method to open a potassium channel.

Compounds of the invention may also be used in a method to block a potassium channel.

This invention contemplates any tautomer, salt, stereoisomer of the compounds with general formula I. Moreover, it must be considered as part of the present invention any molecule having general formula I in which one or more atoms are replaced by isotopes.

In the context of the invention the salts of the compounds of the present invention are also included. On account of their potential use in medicine, the salts of the compounds of formula I are preferably pharmaceutically acceptable. Pharmaceutically acceptable salts comprise the conventional non-toxic salts obtained by salification of a compound of formula I with inorganic acids (e.g. hydrochloric, hydrobromic, sulphuric or phosphoric acid), or with organic acids (e.g. acetic, propionic, succinic, benzoic, sulfanilic, 2-acetoxy-benzoic, cinnamic, mandelic, salicylic, glycolic, lactic, oxalic, malic, maleic, malonic, fumaric, tartaric, citric, p-toluenesulfonic, methanesulfonic, ethanesulfonic, or naphthalenesulfonic acid). For a review of suitable pharmaceutical salts see SM Berge, et al., J. Pharm. Sci. 1977, 66, 1-19; PL Gould, Int. J. Pharm. 1986, 33, 201-217; LD Bighley, et al., Encyclopedia of Pharmaceutical Technology, Marcel Dekker Inc, New York 1996, Volume 13, page 453-497. Other salts which are not pharmaceutically acceptable, such as trifluoroacetate salt, can be useful in the preparation of compounds of the present invention and these form a further aspect of the invention. The invention includes within its scope all possible stoichiometric and non-stoichiometric forms of the salts of the compounds of formula I.

Furthermore, the compounds of formula I can exist in non-solvated forms as well as in forms solvated with pharmaceutically acceptable solvents such as water, EtOH and the like.

Some compounds of formula I can exist in stereoisomeric forms (e.g. they may contain one or more asymmetric carbon atoms). The individual stereoisomers (enantiomers and diastereoisomers) and any mixture, in particular racemic mixture, comprising these are included in the scope of the present invention. The present invention also covers the individual isomers of the compounds represented by formula I as well as mixtures with isomers in which one or more chiral centres are inverted.

Similarly, it is understood that the compounds of formula I can exist in tautomeric forms other than those shown in the formula and these are included in the scope of the present invention.

Though structural representations can show only one of the possible tautomeric or stereoisomeric forms, it is to be understood that the invention encompasses any tautomeric or stereoisomeric form, and mixtures thereof, and is not to be limited merely to any one tautomeric or stereoisomeric form utilized within drawings or the naming of the compounds.

The invention also includes all the suitable isotopic variations of a compound of the invention. An isotopic variation of a compound of the invention is defined as one in which at least one atom is replaced by an atom having the same atomic number but a different atomic mass from the atomic mass usually found in nature. In particular, the term "isotope" as used herein refers to any molecule having the general formula I in which one or more atoms are replaced by isotopes, preferably radioactive isotopes. Examples of isotopes which may be incorporated in the compounds of the invention include isotopes such as $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$, respectively. Certain isotopic variations of the invention, for example those in which a radioactive isotope such as $^{3}H$ or $^{14}C$ is incorporated, are useful in studies on tissue distribution of the medicament and/or substrate. Furthermore, substitution with isotopes such as deuterium $^{2}H$, may provide certain therapeutic advantages resulting from a greater metabolic stability. The isotopic variations of the compounds of the invention can generally be prepared by conventional procedures as well as with the methods illustrated or by the preparations described in the examples below using appropriate isotopic variations of suitable reagents.

The invention further comprises pharmaceutical compositions containing at least one compound of the present invention or a pharmaceutically acceptable tautomer, salt, solvate, stereoisomer or isotope thereof and one or more

pharmaceutically acceptable carriers. As used herein a pharmaceutically acceptable ingredient comprises any excipient, diluent, binder, lubricant, tablettig agent, disintegrant, preservative, buffering agent and any other ingredient tipically used in the art of formulation of pharmaceuticals and veterinary. An exemplary but not exhaustive list of examples of pharmaceutically acceptable carriers is reported in Kibbe, Handbook of Pharmaceutical Excipients, London, Pharmaceutical Press, 2000.

The pharmaceutical compositions can be chosen according to the needs of treatment. Such compositions are prepared by mixing and are suitably adapted to oral or parenteral administration, and as such can be administered in the form of tablets, capsules, oral preparations, powders, granules, pills, or injectable or infusible liquid solutions, suspensions, suppositories, preparation by inhalation.

Tablets and capsules for oral administration are usually presented in unit dosage form and contain conventional excipients such as ligands, fillers (including cellulose, mannitol, lactose), diluents, tablet agents, lubricants (including magnesium stearate), detergents, disintegrants (for example polyvinylpyrrolidone and starch derivatives such as sodium starch glycolate), colorants, flavourings and wetting agents (for example sodium lauryl sulphate).

The solid oral compositions may be prepared by conventional methods of mixing, filling or tabletting. The mixing operation can be repeated to distribute the active substance in all the compositions containing large quantities of fillers. Such operations are conventional.

The oral liquid preparations may be in the form of, for example, aqueous or oily suspensions, solutions, emulsions, syrups or elixirs, or may be presented as a dry product for reconstitution with water or with a suitable vehicle before use. Such liquid preparations may contain conventional additives such as suspending agents, for example sorbitol, syrup, methyl cellulose, gelatine, hydroxyethyl cellulose, carboxymethyl cellulose, aluminium stearate gel or hydrogenated edible fats; emulsifying agents such as lecithin, sorbitan monooleate, or acacia; non-aqueous vehicles (which may include edible oils) such as almond oil, fractionated coconut oil, oily esters such as glycerine esters, propylene glycol, or ethyl alcohol; preservatives such as methyl or propyl p-hydroxybenzoate or sorbic acid, and if desired, conventional flavourings or colourants. Oral formulations also include conventional slow-release formulations such as gastro-resistant coated tablets or granules.

The pharmaceutical preparation for administration via inhalation can be provided by an insufflator or by a pressurized nebulizer.

For parenteral administration, the unit dosage of fluid can be prepared comprising the compound and a sterile vehicle. The compound may be suspended or dissolved, depending on the vehicle and concentration. Parenteral solutions are normally prepared by dissolving the compound in a vehicle, sterilizing the latter by filtration, filling suitable vials and sealing. Advantageously, adjuvants such as local anaesthetics, preservatives and buffering agents can be dissolved in the vehicle. To increase stability, the composition can be frozen after having filled the vials and removed water under vacuum. Parenteral suspensions are prepared in substantially the same manner, except that the compound may be suspended in the vehicle instead of being dissolved and sterilized by exposure to ethylene oxide before suspending in the sterile vehicle. Advantageously, a surfactant or a wetting agent may be included in the composition to facilitate uniform distribution of the compound of the invention.

For oral or sublingual administration the compositions may be tablets, lozenges, or gel.

The compounds may be pharmaceutically formulated as suppositories or retention enemas, e.g. suppositories containing conventional bases such as cocoa butter, polyethylene glycol, or other glycerides, for rectal administration.

Another route of administration of the compounds of the invention regards topical treatment. The topical formulations may contain for example ointments, creams, lotions, gels, solutions, pastes and/or can contain liposomes, micelles and/or microspheres. Examples of ointments include oleaginous ointments such as vegetable oils, animal fats, semi-solid hydrocarbons, emulsifiable ointments such as hydroxystearic sulphate, anhydrous lanolin, hydrophilic petrolatum, cetyl alcohol, glycerol monostearate, stearic acid, water soluble ointments containing polyethylene glycols of various molecular weights. The creams, as known to persons skilled in formulation, are viscous liquids or semi-solid emulsions, and contain an oily phase, an emulsifier and an aqueous phase. The oily phase generally contains petrolatum and an alcohol such as cetyl or stearyl alcohol. The formulations suitable for topical ocular administration also include eye drops, in which the active ingredient is dissolved or suspended in a suitable vehicle, in particular in an aqueous solvent for the active ingredient.

A further method of administration of the compounds of the invention regards transdermal vehiculation. Typical transdermal formulations comprise conventional aqueous and non-aqueous vectors, such as creams, oils, lotions or pastes or may be in the form of membranes or medicated patches.

A reference for the formulations is Remington's book (Remington "The Science and Practice of Pharmacy", Lippincott Williams & Wilkins, 2000).

The compounds of the present invention can be used in the treatment and/or prevention of the conditions referred to herein as a single therapy or in combination with other therapeutic agents, either by separate administrations, or by including the two or more active substances in the same pharmaceutical formulation. The compounds can be administered simultaneously or sequentially. The other therapeutic agents may be compounds currently on the market.

Examples of suitable therapeutic agents that can be used in combination with compounds of the invention include anticonvulsants (for example, but not limited to, Acetazolamide, Brivaracetam, Carbamazepine, Clobazam, Clonazepam, Diazepam, Eslicarbazepine, Ethosuximide, Fosphenytoin, Gabapentin, Lacosamide, Lamotrigine, Levetiracetam, Lorazepam, Methosuximide, Nitrazepam, Oxcarbazepine, Perampanel, Phenobarbital, Phenytoin, Pregabalin, Primidone, Rufinamide, Stiripentol, Topiramate, Valproic Acid, Vigabatrin, Felbamate, Tiagabine, Zonisamide), analgesics (for example, acetaminophen or opioids such as, but not limited to, morphine) and nonsteroidal anti-inflammatory drugs (NSAIDs). Examples of NSAIDs include, but are not limited to, aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, meloxicam, nabumetone, naproxen, nimesulide, nitroflurbiprofen, olsalazine, oxaprozin, phenylbutazone, piroxicam, sulfasalazine, sulindac, tolmetin and zomepirac.

The combination can be administered as a separate composition (simultaneous, sequential) of the individual components of the treatment or as a single dosage form containing both agents. When the compounds of the present invention are combined with other active substances, the active substances may be formulated separately in the form of single ingredient preparations of one of the forms described above, and then given as combined preparations, administered at the same time or different times, or may be formulated together in preparations with two or more ingredients.

The compounds of general formula I may be administered to a patient in a total daily dose of, for example, 0.001 to 1000 mg/kg of body weight per day. The compositions of the dosage units may contain amounts of sub-multiples thereof to make up the daily dose. The compound can also be administered weekly or every other day. The determination of optimum dosages for a specific patient is well known to those skilled in the art. As is common practice, the compositions are normally accompanied by instructions for use in the treatment in question written or printed.

Preferably, compounds with general formula I are useful for systemic administration (oral, parenteral, or rectal), and/or topical with a variable dosing between 50 to 4000 mg die. Consequently, this invention contemplates different compositions suitable for the human or animal administration of compounds with general formula I, and/or their mixtures, and/or their isotopes, and/or any possible isomer, mixed with pharmaceutically acceptable ingredients. Hence, the present invention contemplates solutions or suspensions (sterile or not), syrups, any kind of tablets and capsules (chewable, dispersible etc.), topical ointments, controlled release pharmaceutical or veterinary formulations, nanotechnology-based pharmaceutical or veterinary formulations suitable for the administrations of compounds with general formula I, and/or their mixtures, and/or their isotopes, and/or any possible isomer.

## DETAILED DESCRIPTION OF THE INVENTION

### SYNTHETIC PROCEDURES

The following schemes are examples of synthetic procedures that may be used to prepare the compounds of the invention. In the following schemes, unless otherwise indicated, $R_1$, $R_2$, $R_3$, $R_4$, $R_5$ and $R_6$ are as defined herein above for general formula I. The substituent R as used in the following schemes refers to the one or more substituents that are optionally present on the Hy group of general formula I.

It will be understood by those skilled in the art that these synthetic procedures may be modified to still obtain compounds of the invention. For instance, by choosing the appropriate reagents, those skilled in the art will obtain compounds of general formula I wherein $(Y)_b$-Hy is different from $CH_2Ph$, as shown below for the indoline core. In addition, reactive groups can be protected with protecting groups and deprotected according to well established techniques. Further, certain compounds of the invention can be converted into other compounds of the invention according to standard chemical methods.

### Example 1 - Synthesis of compounds of general formula 4a or 4b

The synthetic procedures for compounds with general formula **4a** or **4b** is shown in Scheme 1.

Scheme 1:

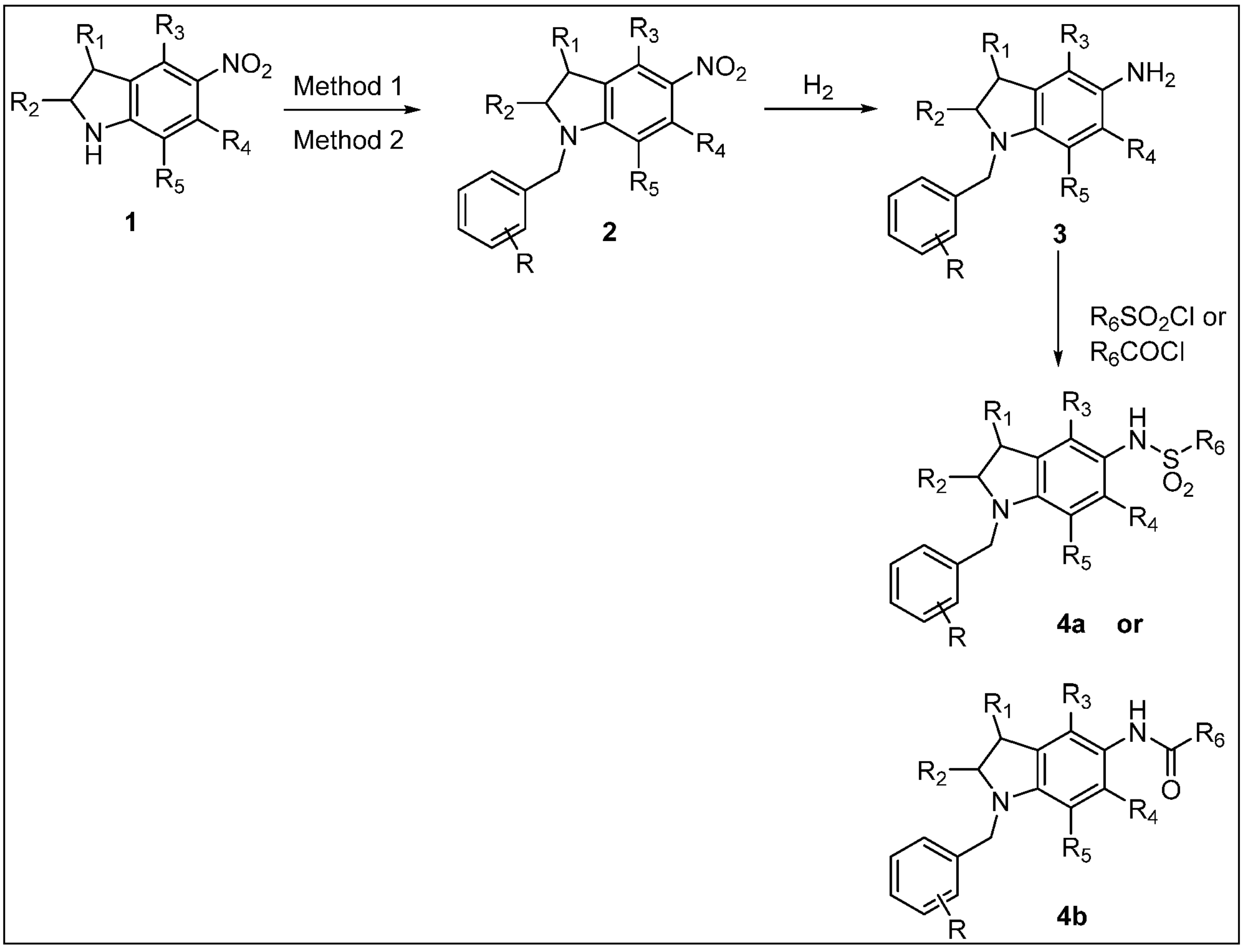


*Synthesis of intermediates of formula **2***

The differently substitued 5-nitroindoline of formula **1** used as starting compounds in Scheme 1 can be synthesized using typical and well described synthetic methods. As an example, see WO 2009/023677 A1. The compounds of formula **1** have been derivatized using one of the two methods described below (method 1 and method 2).

**Method 1:**

1.0 equivalents of **1** are dissolved in a mixture of dichloromethane and acetic acid (3:1 v/v, 16 ml any 6 mmol of **1),** added with 2.0 equivalents of the proper aldehyde of formula RHyCHO (Scheme 1 in particular refers to RPhCHO), and warmed to 65°C for 1-3h under magnetic stirring. The mixture is then added with 1.8 equivalents of sodium triacetoxyborohydride maintaining at the same temperature for further 3h. The reaction is then diluted with dichloromethane and quenched with aqueous NaOH (3N). The organic phase is dried over anhydrous sodium sulfate, filtered and concentrated *in vacuo* to obtain a compound of formula **2.** Crude products are purified by flash column chromatography using n-hexane/ethyl acetate as mobile phases.

**Method 2:**

1.0 equivalents of **1** are dissolved in 10 mL of anhydrous dichloromethane, and are added dropwise to a suspension of sodium hydride (1.2 equivalents) in 5 mL of anhydrous dichloromethane while maintaining under stirring in an ice bath at 0°C. After about 30 minutes the resulting solution is added with 1.2 equivalents of the proper Hy-alkyl halide of formula $RHy(CH_2)_bX$ or Hy-acyl halide of formula $RHy(CH_2)_nCOX$ (Scheme 1 refers in particular to $RPh(CH_2)X$ and $RPh(CH_2)COX$) and is left under stirring at ambient temperature for further 2h. Reaction is then quenched by aqueous citric acid (2N) and the organic phase is isolated, washed with brine, dried over anhydrous sodium sulfate, filtered and

concentrated *in vacuo* to obtain a compound of formula **2.** Crude products are purified by flas h column chromatography using n-hexane/ethyl acetate as mobile phases.

*Synthesis of intermediates of formula **3***

The 1-substitued-5nitroindolines of formula **2,** obtained as descrive above, are dissolved in a mixture of tetrahydrofurane/methanol (1:1.5 v/v) at a final concentration of 0.1M and subjected to catalytic hydrogenation using a continuous-flow hydrogenation reactor (H-Cube, TalesNano, Budapest, Hungary). The typical process parameters selected are: 30°C of temperature, 10 atm of pressure and 1 ml/min of flow. Prepacked Pd/C 10% cartridges (TalesNano) are used as supported catalyst. The products of formula **3** obtained at the end of the reduction cycle are used directly in the following steps without further purification.

*Synthesis of final products of formula **4a***

To a solution of an intermediate of formula **3** (1 eq) in 10 mL of anhydrous dichloromethane are added 2.0 equivalents of 1,5-diazabicyclo(5.4.0)undec-5-ene (DBU) and 2.5 equivalents of the proper sulfonyl halide of formula $R_6SO_2X$ (Scheme 1 indicates in particular a sulfonyl chloride of formula $R_6SO_2Cl$). The reaction is stirred at room temperature for 1-3h, then diluted with dichloromethane and washed with aqueous citric acid (2N) and brine. The organic phase is dried over anhydrous sodium sulfate, filtered, concentrated in vacuo and purified by flash column chromatography using n-hexane/ethyl acetate as mobile phases to afford the final compound of formula **4a.**

*Synthesis of final products of formula **4b***

To a solution of an intermediate of formula **3** (1 eq) in 15 mL of anhydrous dichloromethane are added 1.2 equivalents of N,N'-diisopropylethylamine (DIPEA) and 1.2 equivalents of the proper acyl halide of formula $R_6COX$ (Scheme 1 indicates in particular an acyl chloride of formula $R_6COCl$) or alkyl chloroformate. The reaction is stirred at room temperature for 1h, then diluted with dichloromethane and washed with aqueous citric acid (2N) and brine. The organic phase is dried over anhydrous sodium sulfate, filtered, concentrated in vacuo and purified by flash column chromatography using n-hexane/ethyl acetate as mobile phases.

The list below depicts examples of some compounds prepared by using the above procedure:

**N-(1-(4-(trifluoromethyl)benzyl)indolin-5-yl)cyclohexanesulfonamide**

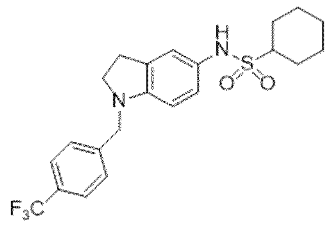

Synthesized starting from 5-nitroindoline, reacted with 4-trifluoromethylbenzhaldheyde following method 1. After reduction of the nitro group, the corresponding amine was coupled with cyclohexylsulfonyl chloride. $^1H$ NMR ($CDCl_3$, 400 MHz): δ: 1.22-1.28 (m, 2H, C*H*$_2$); 1.56-1.65 (m, 4H, 2 C*H*$_2$); 1.69-1.73 (m, 2H, C*H*$_2$); 2.17-2.22 (m, 2H, C*H*$_2$); 2.92-2.98 (m, 1H, CH); 3.02 (t, 2H, C*H*$_2$, *J* = 8.0 Hz); 3.39 (t, 2H, C*H*$_2$, *J* = 8.0 Hz); 4.30 (s, 2H, C*H*$_2$); 6.14 (s, 1H, NH); 6.37 (d, 1H, aryl, *J* = 8.0 Hz); 6.91 (d, 1H, aryl, *J* = 8.0 Hz); 7.09 (s, 1H, aryl); 7.50 (d, 2H, aryl, *J* = 8.0 Hz); 7.63 (d, 2H, aryl, *J* = 8.0 Hz). HR-MS *m*/*z:* calcd for $C_{22}H_{26}F_3N_2O_2S$, [(M+H)$^+$]: 439.1662; found 439.1677; [(M+Na)$^+$]: 461.1481; found 461.1490.

**N-(1-(4-methoxybenzyl)indolin-5-yl)cyclohexanesulfonamide**

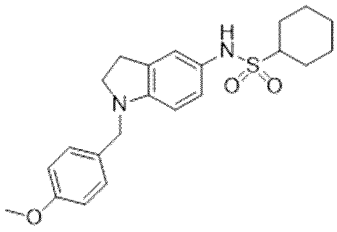

Synthesized starting from 5-nitroindoline, reacted with 4-methoxybenzaldehyde following method 1. After reduction of the nitro group, the corresponding amine was coupled with cyclohexylsulfonyl chloride. $^1H$ NMR ($CDCl_3$, 400 MHz): δ: 1.22-1.28 (m, 2H, C*H*$_2$); 1.57-1.72 (m, 4H, 2 C*H*$_2$); 1.89-1.92 (m, 2H, C*H*$_2$); 2.17-2.21 (m, 2H, C*H*$_2$); 2.92-2.98 (m, 1H, CH); 2.92-2.99 (m, 3H, H-3 and CH); 3.39 (t, 2H, H-2, *J* = 8.0 Hz); 3.83 (s, 3H, C*H*$_3$); 4.19 (s, 2H, C*H*$_2$); 5.93 (s, 1H, NH); 6.43 (d, 1H, H-7, *J* = 8.0 Hz); 6.90 (m, 3H, H-6 and aryl); 7.05 (s, 1H, H-4); 7.28 (d, 2H, aryl, *J* = 8.0 Hz). HR-MS *m/z:* calcd for $C_{22}H_{29}F_3N_2O_3S$, [(M+H)$^+$]: 401.1893; found 401.1902; [(M+Na)$^+$]: 423.1718; found 423.1724.

**N-(1-(4-fluorobenzyl)indolin-5-yl)cyclohexanesulfonamide**

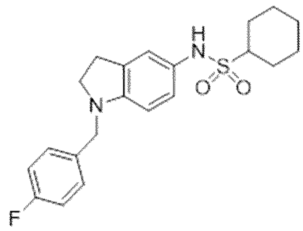

Synthesized starting from 5-nitroindoline, reacted with 4-fluorobenzaldehyde following method 1. After reduction of the nitro group, the corresponding amine was coupled with cyclohexylsulfonyl chloride. $^1H$ NMR ($CDCl_3$, 400 MHz): δ: 1.10-1.21 (m, 2H, C*H*$_2$); 1.47-1.62 (m, 4H, 2 C*H*$_2$); 1.80-1.82 (m, 2H, C*H*$_2$); 2.08-2.11 (m, 2H, C*H*$_2$); 2.83-2.91 (m, 3H, C*H*$_2$ and CH); 3.25 (t, 2H, C*H*$_2$, *J* = 8.0 Hz); 4.12 (s, 2H, C*H*$_2$); 5.96 (s, 1H, N*H*); 6.31 (d, 1H, aryl, *J* = 8.0 Hz); 6.80 (d, 1H, aryl, *J* = 8.0 Hz); 6.94-6.98 (m, 3H, aryl); 7.21-7.26 (m, 2H, aryl). HR-MS *m/z:* calcd for $C_{21}H_{26}FN_2O_2S$, [(M+H)$^+$]: 389.1694; found 389.1699; [(M+Na)$^+$]: 411.1513; found 411.1520.

**N-(1-(2,4-difluorobenzyl)indolin-5-yl)cyclohexanesulfonamide**

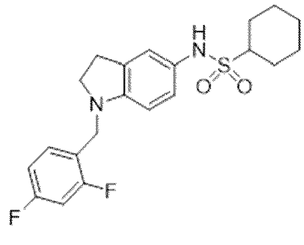

Synthesized starting from 5-nitroindoline, reacted with 2,4-difluorobenzyl bromide following method 2. After reduction of the nitro group, the corresponding amine was coupled with cyclohexylsulfonyl chloride. $^1H$ NMR ($CDCl_3$, 400 MHz): δ: 1.20-1.26 (m, 3H, C*H*$_2$); 1.50-1.71 (m, 2H, C*H*$_2$); 1.88-1.92 (m, 4H, 2 C*H*$_2$); 2.17-2.20 (m, 2H, C*H*$_2$); 2.91-3.02 (m, 3H, C*H*$_2$ and CH); 3.40 (t, 2H, C*H*$_2$, *J* = 8.0 Hz); 4.26 (s, 2H, C*H*$_2$); 6.21 (s, 1H, NH); 6.41 (d, 1H, aryl, *J* = 8.0 Hz); 6.85-6.93 (m, 3H, aryl); 7.07 (s, 1H, aryl); 7.34-7.40 (m, 1H, aryl). HR-MS *m/z:* calcd for $C_{21}H_{25}F_2N_2O_2S$, [(M+H)$^+$]: 407.1599; found 407.1611; [(M+Na)$^+$]: 429.1419; found 429.1427.

**N-(1-(4-fluorobenzyl)indolin-5-yl)benzenesulfonamide**

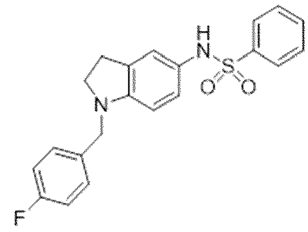

Synthesized starting from 5-nitroindoline, reacted with 4-fluorobenzaldehyde following method 1. After reduction of the nitro group, the corresponding amine was coupled with benzenesulfonyl chloride. $^1H$ NMR ($CDCl_3$, 400 MHz): δ: 2.91 (t, 2H, C*H*$_2$, *J* = 12.0 Hz); 3.31 (t, 2H, C*H*$_2$, *J* = 12.0 Hz); 4.18 (s, 2H, C*H*$_2$); 6.28-6.30 (m, 2H, aryl and NH); 6.61 (d, 1H, aryl, *J* = 8.0 Hz); 6.87 (s, 1H, aryl); 7.03 (t, 2H, aryl, *J* = 8.0 Hz); 7.28-7.30 (m, 2H, aryl); 7.46 (t, 2H, aryl, *J* = 8.0 Hz); 7.56 (t, 1H, aryl, *J* = 8.0 Hz); 7.74 (t, 2H, aryl, *J* = 8.0 Hz). HR-MS *m/z:* calcd for $C_{21}H_{20}FN_2O_2S$, [(M+H)$^+$]: 383.1224; found 383.1230.

**N-(1-(4-fluorobenzyl)indolin-5-yl)butane-1-sulfonamide**

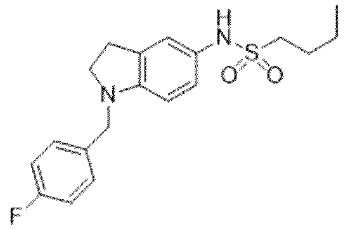

Synthesized starting from 5-nitroindoline, reacted with 4-fluorobenzaldehyde following method 1. After reduction of the nitro group, the corresponding amine was coupled with butanesulfonyl chloride. $^1H$ NMR ($CDCl_3$, 400 MHz): δ: 0.94 (t, 3H, C*H*$_3$, *J*= 8.0 Hz); 1.41-1.49 (m, 2H, C*H*$_2$); 1.80-1.88 (m, 2H, C*H*$_2$); 2.95-3.06 (m, 4H, 2 C*H*$_2$); 3.34 (t, 2H, C*H*$_2$, *J* = 8.0 Hz); 4.21 (s, 2H, C*H*$_2$); 6.28 (s, 1H, N*H*); 6.40 (d, 1H, aryl, J= 12.0 Hz); 6.91 (d, 1H, aryl, *J* = 12.0 Hz); 7.02-7.07 (m, 3H, aryl); 7.30-7.35 (m, 2H, aryl). HR-MS *m/z:* calcd for $C_{19}H_{24}FN_2O_2S$, [(M+H)$^+$]: 363.1537; found 363.1545.

**N-(1-(4-fluorobenzyl)indolin-5-yl)-2-methylpropane-1-sulfonamide**

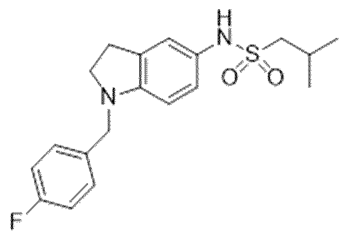

Synthesized starting from 5-nitroindoline, reacted with 4-fluorobenzaldehyde following method 1. After reduction of the nitro group, the corresponding amine was coupled with 2-methylpropane-1-sulfonyl chloride. $^1H$ NMR ($CDCl_3$, 400 MHz): δ: 1.08 (d, 6H, C*H*$_3$, *J* = 7.0 Hz); 2.25-2.29 (m, 1H, CH); 2.90 (d, 2H, C*H*$_2$ *J* = 4.0 Hz); 2.96 (t, 2H, C*H*$_2$, *J* = 8.0 Hz); 3.32 (t, 2H, C*H*$_2$, *J* = 8.0 Hz); 4.19 (s, 2H, C*H*$_2$); 6.17 (s, 1H, NH); 6.38 (d, 1H, aryl, *J* = 8.0 Hz); 6.88 (d, 1H, aryl, *J* = 8.0 Hz); 7.00-7.05 (m, 3H, aryl); 7.28-7.32 (m, 2H, aryl). HR-MS *m/z:* calcd for $C_{19}H_{24}FN_2O_2S$, [(M+H)$^+$]: 363.1537; found 363.1545.

**N-(1-(4-fluorobenzyl)indolin-5-yl)azepane-1-sulfonamide**

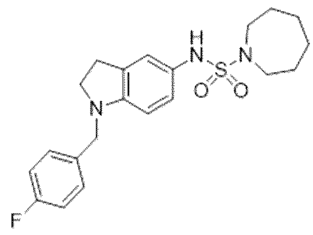

Synthesized starting from 5-nitroindoline, reacted with 4-fluorobenzaldehyde following method 1. After reduction of the nitro group, the corresponding amine was coupled with azepane-1-sulfonyl chloride. $^1$H NMR ($CDCl_3$, 400 MHz): δ: 1.54-1.59 (m, 4H, 2 C$H_2$); 1.68-1.70 (m, 4H, 2 C$H_2$); 2.97 (t, 2H, C$H_2$, $J$ = 12.0 Hz); 3.28-3.35 (m, 6H, 3 C$H_2$); 4.21 (s, 2H, C$H_2$); 5.99 (s, 1H, NH); 6.40 (d, 1H, aryl, J= 12.0 Hz); 6.88 (d, 1H, aryl, J= 8.0 Hz); 7.01-7.07 (m, 3H, aryl); 7.30-7.35 (m, 2H, aryl). HR-MS *m/z:* calcd for $C_{21}H_{27}FN_3O_2S$, [(M+H)$^+$]: 404.1803; found 404.1876.

**N-(1-(4-fluorobenzyl)indolin-5-yl)hexane-1-sulfonamide**

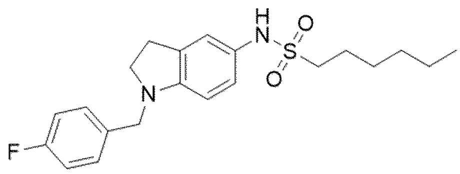

Synthesized starting from 5-nitroindoline, reacted with 4-fluorobenzaldehyde following method 1. After reduction of the nitro group, the corresponding amine was coupled with hexane-1-sulfonyl chloride. $^1$H NMR ($CD_3OD$, 400 MHz): δ: 0.91 (t, 3H, C$H_3$, $J$ = 8.0 Hz); 1.28-1.34 (m, 4H, C$H_2$); 1.36-1.46 (m, 2H, C$H_2$); 1.78-1.82 (m, 2H, C$H_2$); 3.13-3.18 (m, 4H, 2 C$H_2$); 3.91(t, 2H, C$H_2$, $J$= 8.0 Hz); 4.70 (s, 2H, C$H_2$); 7.18-7.27 (m, 3H, aryl); 7.33-7.35 (d, 2H, aryl, $J$ = 8.0 Hz); 7.51 (dd, 2H, aryl, $J_1$=4Hz, $J_2$=8 Hz). HR-MS *m/z:* calcd for $C_{21}H_{28}FN_2O_2S$, [(M+H)$^+$]: 391.1850; found 391.1862.

**N-(1-(4-fluorobenzyl)indolin-5-yl)-4-methylpiperazine-1-sulfonamide**

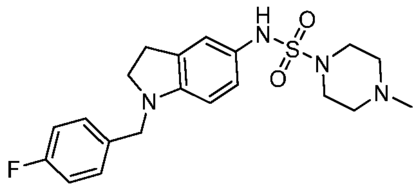

Synthesized starting from 5-nitroindoline, reacted with 4-fluorobenzaldehyde following method 1. After reduction of the nitro group, the corresponding amine was coupled with 4-methylpiperazine-1-sulfonyl chloride. $^1$H NMR ($CDCl_3$, 400 MHz): δ: 2.30 (s, 3H, C$H_3$); 2.43 (t, 4H, C$H_2$, $J$ = 4.0 Hz); 2.96 (t, 2H, C$H_2$, $J$ = 8.0 Hz); 3.29-3.34 (m, 6H, 3C$H_2$); 4.19 (s, 2H, C$H_2$); 6.38 (d, 1H, aryl, $J$ = 8.0 Hz); 6.93 (d, 1H, aryl, $J$ = 8.0 Hz); 7.01-7.07 (m, 3H, aryl); 7.30-7.34 (m, 2H, aryl). HR-MS *m/z:* calcd for $C_{20}H_{26}FN_4O_2S$, [(M+H)$^+$]: 405.1755; found 405.1762.

**1-((1S,4R)-7,7-dimethyl-2-oxobicyclo[2.2.1]heptan-1-yl)-N-(1-(4-fluorobenzyl)indolin-5-yl)methanesulfonamide**

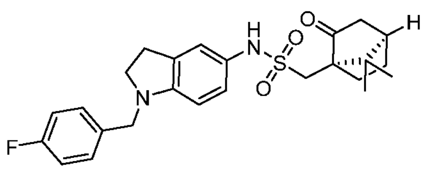

Synthesized starting from 5-nitroindoline, reacted with 4-fluorobenzaldehyde following method 1. After reduction of the nitro group, the corresponding amine was coupled with (1S)-(+)-10-camphorsulfonyl chloride. $[\alpha]^{25}_D$ : 37.50 ± 0.05 (c = 0.12, MeOH). $^1$H NMR ($CDCl_3$, 400 MHz): δ: 0.92 (s, 3H, C*H*$_3$); 0.99 (s, 3H, C*H*$_3$); 1.47-1.52 (m, 1H, CH); 1.98-2.20 (m, 5H, 2 C*H*$_2$ and C*H*$_{2a}$); 2.44-2.51 (m, 1H, C*H*$_{2b}$); 2.81 (d, 1H, C*H*$_{2a'}$, *J* = 16.0 Hz); 2.98 (t, 2H, C*H*$_2$, *J* = 12.0 Hz); 3.34 (t, 2H, C*H*$_2$, *J*= 12.0 Hz); 3.45 (d, 1H, C*H*$_{2b'}$, *J* = 16.0 Hz); 4.21 (s, 2H, C*H*$_2$); 6.41 (d, 1H, aryl, *J* = 12.0 Hz); 6.94 (d, 1H, aryl, *J* = 12.0 Hz); 7.02-7.06 (m, 2H, aryl); 7.10 (s, 1H, aryl); 7.31-7.33 (m, 2H, aryl); 7.45 (s, 1H, NH). HR-MS *m/z:* calcd for $C_{25}H_{30}FN_2O_3S$, [(M+H)$^+$]: 457.1986; found 457.2001.

**1-((1R,4S)-7,7-dimethyl-2-oxobicyclo[2.2.1]heptan-1-yl)-N-(1-(4-fluorobenzyl)indolin-5-yl)methanesulfonamide**

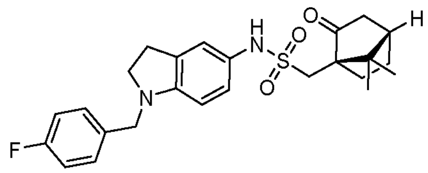

Synthesized starting from 5-nitroindoline, reacted with 4-fluorobenzaldehyde following method 1. After reduction of the nitro group, the corresponding amine was coupled with (1R)-(-)-10-camphorsulfonyl chloride. $[\alpha]^{25}_D$ : -38.10 ± 0.06 (c = 0.12, MeOH). $^1$H NMR ($CDCl_3$, 400 MHz): δ: 0.92 (s, 3H, C*H*$_3$); 0.98 (s, 3H, C*H*$_3$); 1.47-1.52 (m, 1H, CH); 1.97-2.22 (m, 5H, 2 C*H*$_2$ and C*H*$_{2a}$); 2.44-2.52 (m, 1H, C*H*$_{2b}$); 2.80 (d, 1H, C*H*$_{2a'}$, *J* = 16.0 Hz); 2.97 (t, 2H, *CH, J* = 12.0 Hz); 3.32 (t, 2H, *CH, J* = 12.0 Hz); 3.43 (d, 1H, C*H*$_{2b'}$, *J* = 16.0 Hz); 4.21 (s, 2H, C*H*$_2$); 6.41 (d, 1H, aryl, *J* = 12.0 Hz); 6.93 (d, 1H, aryl, *J* = 12.0 Hz); 7.01-7.10 (m, 3H, aryl); 7.30-7.35 (m, 2H, aryl); 7.47 (s, 1H, NH). HR-MS *m/z:* calcd for $C_{25}H_{30}FN_2O_3S$, [(M+H)$^+$]: 457.1986; found 457.1995.

**N-(1-(4-fluorobenzyl)indolin-5-yl)-3,5-dimethylpiperidine-1-sulfonamide**

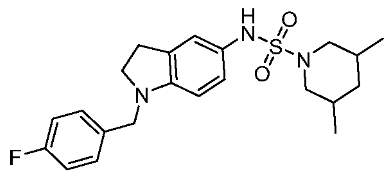

Synthesized starting from 5-nitroindoline, reacted with 4-fluorobenzaldehyde following method 1. After reduction of the nitro group, the corresponding amine was coupled with 3,5-dimethylpiperidine-1-sulfonyl chloride. $^1$H NMR ($CD_3OD$, 400 MHz): δ: 0.49-0.58 (m, 1H, CH); 0.78 (d, 6H, 2 $CH_3$, J=8.0 Hz); 1.43-1.50 (m, 2H, $CH_2$,); 1.64 (d, 2H, C*H*$_2$, J=16.0 Hz); 2.13 (t, 2H, C*H*$_2$, J=12.0 Hz); 3.02 (bs, 2H, C*H*$_2$); 3.57 (dd, 2H, C*H*$_2$, $J_1$= 4.0 Hz, $J_2$=9.0 Hz); 3.81 (bs, 2H, C*H*$_2$); 4.57 (s, 2H, C*H*$_2$); 7.06-7.16 (m, 5H, aryl); 7.35-7.38 (m, 2H, aryl). HR-MS *m/z:* calcd for $C_{22}H_{29}FN_3O_2S$, [(M+H)$^+$]: 418.1959; found 418.1964.

**N-(1-(4-fluorobenzoyl)indolin-5-yl)cyclohexanesulfonamide**

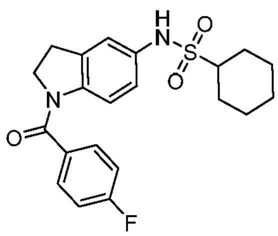

Synthesized starting from 5-nitroindoline, reacted with 4-fluorobenzoyl chloride following method 2. After

reduction of the nitro group, the corresponding amine was coupled with cyclohexylsulfonyl chloride.$^1$H NMR (DMSO-d6, 400 MHz): δ: 1.13-1.24 (m, 2H, C$H_2$); 1.36-1.42 (m, 4H, 2 C$H_2$); 1.58-1.60 (m, 1H, C$H_2$); 1.75-1.78 (m, 2H, C$H_2$); 2.00-2.03 (m, 2H, C$H_2$); 2.89-2.97 (m, 1H, CH); 3.07 (t, 2H, C$H_2$, $J$ = 8.0 Hz); 4.00 (t, 2H, C$H_2$, $J$ = 8.0 Hz); 7.04 (bs, 1H, aryl); 7.16 (s, 1H, aryl); 7.30-7.35 (m, 2H, aryl); 7.65-7.68 (m, 2H, aryl); 7.95 (bs, 1H, aryl); 9.65 (s, 1H, N*H*). HR-MS *m/z:* calcd for $C_{21}H_{24}FN_2O_3S$, [(M+H)$^+$]: 403.1486; found 403.1495; [(M+Na)$^+$]: 425.1306; found 425.1316.

**N-(1-(3,4-difluorobenzyl)indolin-5-yl)cyclohexanesulfonamide**

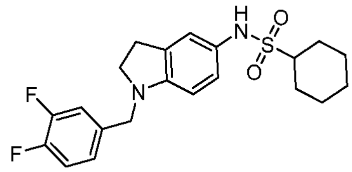

Synthesized starting from 5-nitroindoline, reacted with 3,4-difluorobenzyl bromide following method 2. After reduction of the nitro group, the corresponding amine was coupled with cyclohexylsulfonyl chloride.$^1$H NMR ($CDCl_3$, 400 MHz): δ: 1.20-1.31 (m, 2H, C$H_2$); 1.59-1.72 (m, 4H, 2 C$H_2$); 1.89-1.92 (m, 2H, C$H_2$); 2.18-2.21 (m, 2H, C$H_2$); 2.91-2.97 (m, 1H, CH); 3.01 (t, 2H, C$H_2$, $J$ = 8.0 Hz); 3.37 (t, 2H, C$H_2$, $J$ = 8.0 Hz); 4.19 (s, 2H, C$H_2$); 6.02 (s, 1H, NH); 6.36 (d, 1H, aryl, $J$ = 8.0 Hz); 6.90 (d, 1H, aryl, $J$ = 8.0 Hz); 7.08-7.22 (m, 4H, aryl). HR-MS *m/z:* calcd for $C_{21}H_{25}F_2N_2O_2S$, [(M+H)$^+$]: 407.1599; found 407.1612; [(M+Na)$^+$]: 429.1419; found 429.1426.

**N-(1-(2-(4-chlorophenyl)acetyl)indolin-5-yl)cyclohexanesulfonamide**

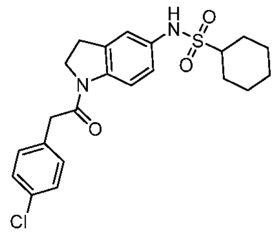

Synthesized starting from 5-nitroindoline, reacted with 2-(4-chlorophenyl)acetyl chloride following method 2. After reduction of the nitro group, the corresponding amine was coupled with cyclohexylsulfonyl chloride[1]H NMR ($CDCl_3$, 400 MHz): δ: 1.18-1.28 (m, 2H, C$H_2$); 1.54-1.71 (m, 4H, 2 C$H_2$); 1.89-1.92 (m, 2H, C$H_2$); 2.16 (d, 2H, C$H_2$, J=12.0 Hz); 2.90-2.97 (m, 1H, CH); 4.25 (s, 2H, C$H_2$); 6.11 (s, 1H, N*H*); 6.59 (t, 2H, aryl, J= 12.0 Hz); 6.84 (d, 1H, aryl, $J$ = 8.0 Hz); 7.03-7.09 (m, 4H, aryl);7.35 (dd, 2H, aryl, Ji=5.2 Hz, $J_2$=8.4 Hz);.HR-MS *m/z:* calcd for $C_{22}H_{26}ClNO_3S$, [(M+H)$^+$]: 433.1347 and [(M+H)$^+$+2]: 435.1318; found 433.1342 and 435.1323.

**N-(1-(2,4-bis(trifluoromethyl)benzyl)indolin-5-yl)cyclohexanesulfonamide**

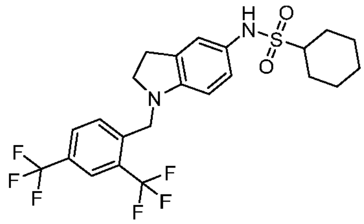

Synthesized starting from 5-nitroindoline, reacted with 1-(bromomethyl)-2,4-bis(trifluoromethyl)benzene following method 2. After reduction of the nitro group, the corresponding amine was coupled with cyclohexylsulfonyl chloride.$^1$H NMR ($CDCl_3$, 400 MHz): δ: 1.20-1.31 (m, 2H, C$H_2$); 1.56-1.72 (m, 4H, 2 C$H_2$); 1.90-1.92 (m, 2H, C$H_2$); 2.19-2.21 (m, 2H,

C*H*$_2$); 2.93-2.99 (m, 1H, CH); 3.09 (t, 2H, H-3, *J* = 8.0 Hz); 3.50 (t, 2H, H-2, *J* = 8.0 Hz); 4.49 (s, 2H, C*H*$_2$); 6.17 (s, 1H, N*H*); 6.24 (d, 1H, H-7, *J* = 12.0 Hz); 6.89 (d, 1H, H-6, *J* = 12.0 Hz); 7.13 (s, 1H, H-4); 7.80 (d, 1H, aryl, *J* = 12.0 Hz); 7.87 (d, 1H, aryl, *J* = 12.0 Hz);7.96 (s, 1H, aryl). HR-MS *m*/*z:* calcd for $C_{23}H_{25}F_6N_2O_2S$, [(M+H)$^+$]: 507.1535; found 507.1541; [(M+Na)$^+$]: 529.1355; found 529.1362.

**N-(1-(4-bromo-2-nitrobenzyl)indolin-5-yl)cyclohexanesulfonamide**

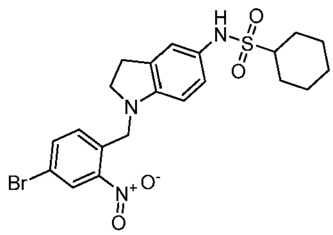

Synthesized starting from 5-nitroindoline, reacted with 4-bromo-1-(bromomethyl)-2-nitrobenzene following method 2. After reduction of the nitro group, the corresponding amine was coupled with cyclohexylsulfonyl chloride.$^1$H NMR ($CDCl_3$, 400 MHz): δ: 1.20-1.31 (m, 2H, C*H*$_2$); 1.56-1.72 (m, 4H, 2 C*H*$_2$); 1.89-1.92 (m, 2H, C*H*$_2$); 2.17-2.20 (m, 2H, C*H*$_2$); 2.91-2.99 (m, 1H, CH); 3.06 (t, 2H, H-3, *J* = 8.0 Hz); 3.48 (t, 2H, H-2, *J* = 8.0 Hz); 4.54 (s, 2H, C*H*$_2$); 5.99 (s, 1H, NH); 6.26 (d, 1H, H-7, *J* = 8.0 Hz); 6.87 (d, 1H, H-6, *J* = 8.0 Hz); 7.10 (s, 1H, H-4); 7.59 (d, 1H, aryl, *J* = 12.0 Hz); 7.74 (d, 1H, aryl, J = 12.0 Hz);8.21 (s, 1H, aryl). HR-MS *m*/*z:* calcd for $C_{21}H_{25}BrN_3O_4S$, [(M+H)$^+$]: 494.0744; found 494.0755; [(M+Na)$^+$]: 516.0563; found 516.0570.

**N-(1-(4-fluorophenethyl)indolin-5-yl)cyclohexanesulfonamide**

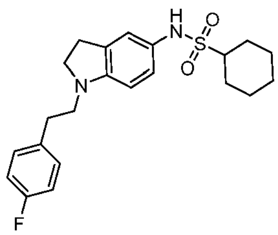

Synthesized starting from 5-nitroindoline, reacted with 1-(2-bromoethyl)-4-fluorobenzene following method 2. After reduction of the nitro group, the corresponding amine was coupled with cyclohexylsulfonyl chloride.$^1$H NMR ($CDCl_3$, 400 MHz): δ: 1.19-1.31 (m, 2H, C*H*$_2$); 1.62-1.71 (m, 4H, 2 C*H*$_2$); 1.89-1.92 (m, 2H, C*H*$_2$); 2.17-2.20 (m, 2H, C*H*$_2$); 2.85-3.00 (m, 5H, C*H, 2* C*H*$_2$); 3.30 (t, 2H, C*H*$_2$, *J* = 8.0 Hz); 3.42 (t, 2H, C*H*$_2$, *J* = 8.0 Hz); 5.99 (s, 1H, N*H*); 6.35 (d, 1H, aryl, J = 8.0 Hz); 6.90 (d, 1H, aryl, *J* = 8.0 Hz); 6.99-7.04 (m, 3H, aryl); 7.20-7.23 (m, 2H, aryl). HR-MS *m*/*z:* calcd for $C_{22}H_{28}FN_2O_2S$, [(M+H)$^+$]: 403.1850; found 403.1862; [(M+Na)$^+$]: 425.1669; found 425.1675.

**N-(1-(4-methoxybenzyl)-2-methylindolin-5-yl)cyclohexanesulfonamide**

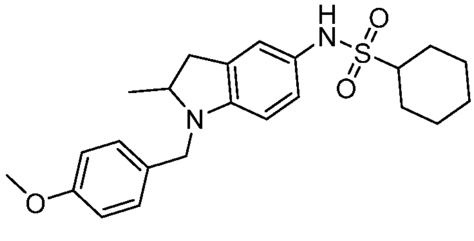

Synthesized starting from 2-methyl-5-nitroindoline, reacted with 4-methoxybenzaldheyde following method 1.

After reduction of the nitro group, the corresponding amine was coupled with cyclohexylsulfonyl chloride. $^1$H NMR ($CDCl_3$, 400 MHz): δ: 1.15-1.34 (m, 2H, C*H*$_2$); 1.33 (d, 3H, C*H*$_3$, J=8.0 Hz) 1.50-1.68 (m, 4H, 2C*H*$_2$); 1.84-1.86 (m, 2H, C*H*$_2$); 2.08-2.10 (m, 2H, C*H*$_2$); 2.60-2.66 (m, 1H, H-3a); 2.97-3.16 (m, 1H, CH); 3.02-3.11 (m, 1H, H-3b); 3.77-3.81 (m, 4H, H-2 and C*H*$_3$); 4.12 (d, 1H, C*H*$_2$a, J=20 Hz), 4.36 (d, 1H, C*H*$_2$b, J=20 Hz); 6.06 (s, $^1$H NH); 6.23 (s, 1H, H-4); 6.39 (d, 1H, H-6, J=8 Hz); 6.87 (d, 2H, aryl, J= 8 Hz) 6.94 (d, 1H, H-7, J=8 Hz); 7.27 (d, 2H, aryl, J=8 Hz); HR-MS *m/z:* calcd for $C_{23}H_{31}N_2O_3S$, [(M+H)$^+$]: 415.2050; found 415.2059; [(M+Na)$^+$]: 437.1869; found 437.1874.

**N-(1-(4-fluorobenzyl)indolin-5-yl)cyclohexanecarboxamide**

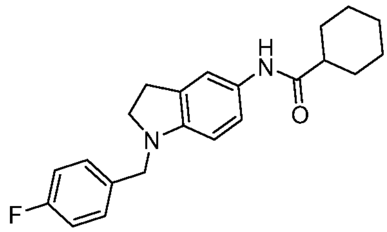

Synthesized starting from 5-nitroindoline, reacted with 4-fluorobenzaldheyde following method 1. After reduction of the nitro group, the corresponding amine was coupled with 2-cyclohexanecarbonyl bromide. $^1$H NMR ($CDCl_3$, 400 MHz): δ: 1.12-1.28 (m, 3H, C*H*$_2$); 1.41-1.54 (m, 3H, C*H*$_2$); 1.74-1.77 (m, 2H, C*H*$_2$); 1.86-1.89 (m, 2H, C*H*$_2$); 2.08-2.14 (m, 1H, CH); 2.86 (t, 2H, C*H*$_2$, *J* = 8.0 Hz); 3.19 (t, 2H, C*H*$_2$, *J* = 8.0 Hz); 4.10 (s, 2H, C*H*$_2$); 6.33 (d, 1H, aryl, *J* = 8.0 Hz); 6.92-6.96 (m, 4H, aryl); 7.22-7.25 (m, 2H, aryl); 7.29 (s, 1H, NH). HR-MS *m/z:* calcd for $C_{22}H_{26}FN_2O$, [(M+H)$^+$]: 353.2024; found 353.2031.

**N-(1-(4-fluorobenzyl)indolin-5-yl)acetamide**

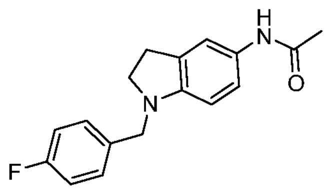

Synthesized starting from 5-nitroindoline, reacted with 4-fluorobenzaldheyde following method 1. After reduction of the nitro group, the corresponding amine was coupled with acetyl bromide. $^1$H NMR ($CDCl_3$, 400 MHz) δ: 2.06 (s, 3H, C*H*$_3$); 2.88 (t, 2H, C*H*$_2$, *J* = 8.0 Hz); 3.20 (t, 2H, C*H*$_2$, *J* = 8.0 Hz); 4.10 (s, 2H, C*H*$_2$); 6.33 (d, 1H, aryl, *J* = 8.0 Hz); 6.91-6.97 (m, 4H, aryl); 7.23-7.26 (m, 4H, aryl and N*H*). HR-MS *m/z:* calcd for $C_{17}H_{18}FN_2O$, [(M+H)$^+$]: 285.1398; found 285.1405.

**2-cyclohexyl-N-(1-(4-fluorobenzyl)indolin-5-yl)acetamide**

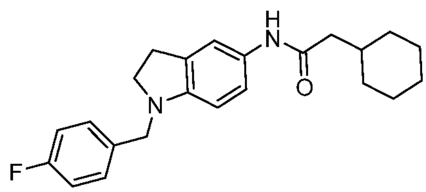

Synthesized starting from 5-nitroindoline, reacted with 4-fluorobenzaldheyde following method 1. After reduction of the nitro group, the corresponding amine was coupled with 2-cyclohexylacetyl bromide. $^1$H NMR (DMSO-d6, 400 MHz): δ: 0.90-1.00 (m, 2H, C*H*$_2$); 1.11-1.27 (m, 3H, C*H*$_2$); 1.26-1.77 (m, 5H, C*H*$_2$); 2.11 (d, 2H, C*H*$_2$, J = 8.0 Hz); 2.85 (t, 2H, C*H*$_2$, *J* = 8.0 Hz); 3.18 (t, 2H, C*H*$_2$, J = 8.0 Hz); 4.20 (s, 2H, C*H*$_2$); 6.51 (d, 1H, aryl, *J* = 8.0 Hz); 7.12-7.19 (m, 3H, aryl); 7.34-7.40 (m,

3H, aryl); 9.48 (s, 1H, NH). HR-MS *m/z:* calcd for $C_{23}H_{28}FN_2O$, [(M+H)+]: 367.2186; found 367.2195.

**ethyl (1-(4-fluorobenzyl)indolin-5-yl)carbamate**

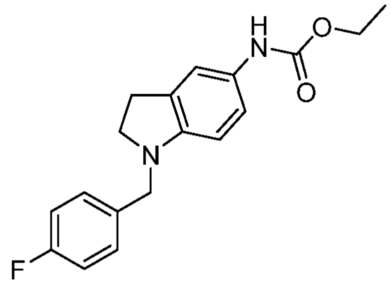

Synthesized starting from 5-nitroindoline, reacted with 4-fluorobenzaldheyde following method 1. After reduction of the nitro group, the corresponding amine was coupled with ethyl chloroformate. $^1H$ NMR ($CDCl_3$, 400 MHz): δ: 1.29 (t, 3H, $CH_3$, $J$ = 8.0 Hz); 2.94 (t, 2H, $CH_2$, $J$ = 8.0 Hz); 3.25 (t, 2H, $CH_2$, $J$ = 8.0 Hz); 4.16-4.22 (m, 4H, $CH_2$); 6.36 (bs, 1H, NH); 6.40 (d, 1H, aryl, $J$ = 8.0 Hz); 6.93 (d, 1H, aryl, $J$ = 8.0 Hz); 7.01-7.04 (m, 2H, aryl); 7.19 (s, 1H, aryl); 7.31-7.33 (m, 2H, aryl). HR-MS *m/z:* calcd for $C_{18}H_{20}FN_2O_2$, [(M+H)+]: 315.1503; found 315.1514.

**(1S,2R,5S)-2-isopropyl-5-methylcyclohexyl (1-(4-fluorobenzyl)indolin-5-yl)carbamate**

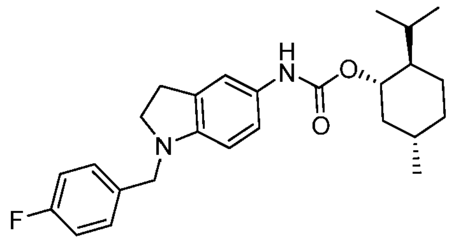

Synthesized starting from 5-nitroindoline, reacted with 4-fluorobenzaldheyde following method 1. After reduction of the nitro group, the corresponding amine was coupled with (1S)-(+)-menthyl chloroformate. $[\alpha]^{25}_D$ : 32.67 ± 0.04 (c = 0.15, MeOH). $^1H$ NMR ($CDCl_3$, 400 MHz): δ: 0.83 (d, 3H, $CH_3$, $J$ = 8.0 Hz); 0.93 (d, 6H, $CH_3$, $J$ = 8.0 Hz); 1.00-1.13 (m, 2H, $CH_2$); 1.28-1.37 (m, 1H, $CH_2$); 1.32-1.41 (m, 1H, $CH_2$); 1.47-1.56 (m, 1H, CH); 1.67-1.74 (m, 2H, $CH_2$); 1.94-2.04 (m, 1H, CH); 2.09-2.16 (m, 1H, $CH_2$); 2.96 (t, 2H, $CH_2$, $J$ = 12.0 Hz); 3.27 (t, 2H, $CH_2$, $J$ = 12.0 Hz); 4.18 (s, 2H, $CH_2$); 4.61-4.69 (m, 1H, CH); 6.34 (s, 1H, NH); 6.42 (d, 1H, aryl, $J$ = 12.0 Hz); 6.95 (d, 1H, aryl, $J$= 12.0 Hz); 7.01-7.06 (m, 2H, aryl); 7.25 (s, 1H, aryl); 7.31-7.36 (m, 2H, aryl). HR-MS *m/z:* calcd for $C_{26}H_{34}FN_2O_2$, [(M+H)+]: 425.2599; found 425.2608.

**Example 2** - **Synthesis of compounds of general formula 5**

The general synthetic procedure for compounds of general formula **5** is shown in Scheme 2.

Scheme 2:

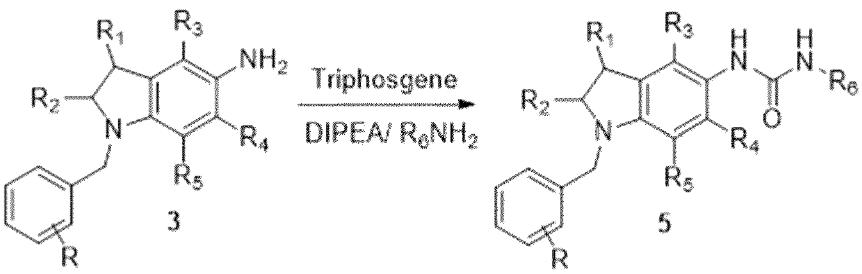

*Synthesis of final products of formula* ***5***

To a solution of triphosgene (1.0 equivalents) in 5 ml of anhydrous dichloromethane are added dropwise (during 10 minutes) 5 mL of a solution containing 1.25 equivalents of one of the intermediates with general formula **3** described in Example 1, and 2.6 equivalents of N,N'-diisopropylethylamine (DIPEA) in anhydrous dichloromethane. The reaction is magnetically stirred for 30-60 minutes at ambient temperature. Then 5 mL of a solution containing the proper amine of formula $R_6NH_2$ (2.5 equivalents) in anhydrous dichloromethane is added dropwise. The reaction is stirred for further 60 minutes and washed with citric acid (2N), saturated sodium bicarbonate solution and brine. The organic phase is then dried over sodium sulfate, filtered and concentrated in vacuo to obtain a compound of formula **5.** Crude products thus obtained are purified by flash column chromatography using n-hexane/ethyl acetate as mobile phases.

Below follow two examples of compounds prepared by using the above-described procedure for the synthesis of final products of formula **5:**

**1-cyclohexyl-3-(1-(4-fluorobenzyl)indolin-5-yl)urea**

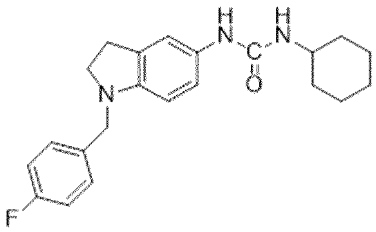

Synthesized starting from 1-(4-fluorobenzyl)indolin-5-amine, reacted with cyclohexylamine, following the general procedure. $^1$H NMR (DMSO-d6, 400 MHz): δ: 1.18-1.26 (m, 3H, C$H_2$ and C$H_{2a}$); 1.34-1.41 (m, 2H, C$H_2$); 1.59-1.64 (m, 1H, C$H_{2b}$); 1.71-1.76 (m, 2H, C$H_2$); 1.85-1.89 (m, 2H, C$H_2$); 2.91 (t, 2H, C$H_2$, *J* = 8.0 Hz); 3.23 (t, 2H, C$H_2$, *J* = 8.0 Hz); 3.48-3.53 (m, 1H, CH); 4.24 (s, 2H, C$H_2$); 5.93 (d, 1H, *NH, J = 8.0* Hz); 6.56 (d, 1H, aryl, *J = 4.0* Hz); 6.99 (d, 1H, aryl, *J= 8.0* Hz); 7.22-7.27 (m, 3H, aryl); 7.46-7.49 (m, 2H, aryl); 7.95 (s, 1H, NH). HR-MS *m/z:* calcd for $C_{22}H_{27}FN_3O$, [(M+H)$^+$]: 368.2133; found 368.2139.

**1-(1-(4-fluorobenzyl)indolin-5-yl)-3-neopentylurea**

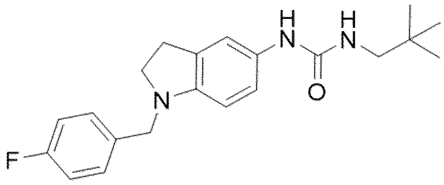

Synthesized starting from 1-(4-fluorobenzyl)indolin-5-amine, reacted with 2,2-dimethylpropan-1-amine, following the general procedure. $^1$H NMR ($CDCl_3$, 400 MHz): δ: 0.89 (s, 9H, C$H_3$); 2.99 (t, 2H, H-3, *J* = 8.0 Hz); 3.03 (d, 2H, C$H_2$, *J* = 4.0 Hz); 3.35 (t, 2H, H-2, *J* = 8.0 Hz); 4.23 (s, 2H, C$H_2$); 4.71 (t, 1H, *NH, J* = 4.0 Hz); 5.98 (s, 1H, NH); 6.45 (d, 1H, H-7, *J* = 8.0 Hz); 6.92 (d, 1H, H-6, *J*= 8.0 Hz); 6.91-7.07 (m, 3H, aryl and H-4); 7.32-7.35 (m, 2H, aryl). HR-MS *m/z:* calcd for $C_{21}H_{27}FN_3O$, [(M+H)$^+$]: 356.2133; found 356.2148.

**Example 3 - Synthesis of compounds of general formula 7 or 8**

The general procedure for the synthesis of compounds with general formula **7** or **8** is depicted in Scheme 3.

Scheme 3:

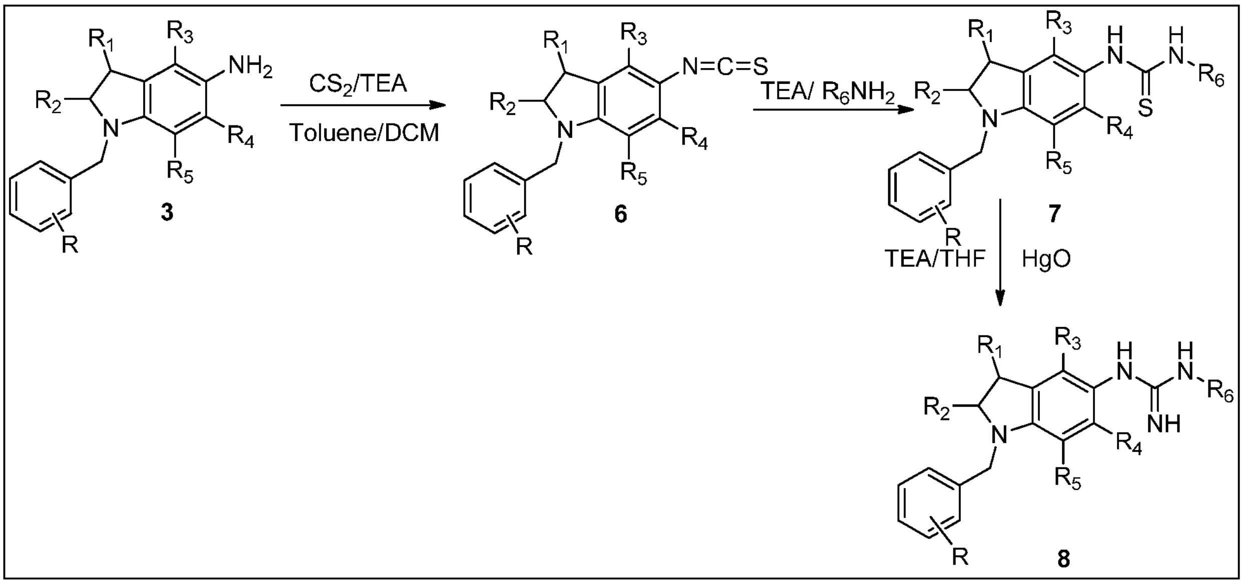


*Synthesis of final products of formula* ***7***

To a toluene solution (10 mL) containing one of the intermediates with general formula **3** described in Example 1 (1.0 eq) and 1.0 equivalents of triethylamine (TEA), are rapidly added 1.1 equivalents of carbon disulfide ($CS_2$) at the temperature of -15°C. The mixture is maintained under magnetic stirring for 2h. The reaction is then warmed to room temperature for 5 minutes and suddenly cooled to -20°C for 24h. The suspension thus obtained is warmed again to room temperature and added with 1.0 equivalents of triethylamine. After cooling to -5°C, 1.1 equivalents of ethylchloroformate are added. Reaction is allowed to reach room temperature and is maintained under stirring for 2h, before being quenched with HCl (2N). The organic phase is separated, washed with a saturated solution of sodium bicarbonate, dried over sodium sulfate, filtered and concentrated in vacuo to obtain a compound of formula **6.** Crude products are dissolved in 15 mL of anhydrous dichloromethane and added with 2.5 equivalents of TEA. To this solution 2.5 equivalents of the proper amine of formula $R_6NH_2$ are added dropwise, maintaining under stirring for 12h. The resulting solution is washed with citric acid (2N), dried over sodium sulfate, filtered and evaporated under vacuum to obtain a compound of formula 7. Crude products are purified by flash column chromatography using n-hexane/ethyl acetate as mobile phases.

Below follows the example of one derivative synthesized used the above-described procedure for the synthesis of final products of formula **7:**

**1-(1-(4-fluorobenzyl)indolin-5-yl)-3-neopentylthiourea**

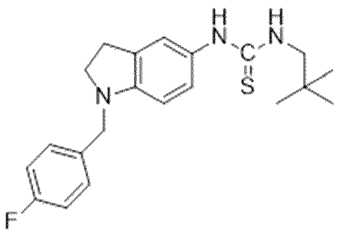

Synthesized starting from 1-(4-fluorobenzyl)indolin-5-amine, reacted with 2,2-dimethylpropan-1-amine, following the general procedure. $^1$H NMR ($CDCl_3$, 400 MHz): δ: 0.89 (s, 9H, C*H*$_3$); 2.99 (t, 2H, C*H*$_2$, *J= 8.0* Hz); 3.39 (t, 2H, C*H*$_2$, *J* = 8.0 Hz); 3.48 (d, 2H, C*H*$_2$, *J* = 8.0 Hz); 4.25 (s, 2H, C*H*$_2$); 5.93 (t, 1H, *NH, J* = 4.0 Hz); 6.46 (d, 1H, aryl, *J* = 8.0 Hz); 6.89-6.92 (m, 2H, aryl); 7.01-7.07 (m, 2H, aryl); 7.29-7.33 (m, 2H, aryl); 7.58 (s, 1H, NH). HR-MS *m/z:* calcd for $C_{21}H_{27}FN_3S$, [(M+H)$^+$]: 372.1904; found 372.1911.

*Synthesis of final products of formula **8***

Starting form 1.0 equivalents of one of the derivatives with general formula 7, dissolved in 10 mL of anhydrous THF, 76.5 equivalents of anhydrous sodium sulfate, 76.5 equivalents of anhydrous calcium chloride ($CaCl_2$) and 153 equivalents of triethylamine were added. The resulting suspension was stirred for 30-60 minutes. Then, 41 equivalents of HgO were added and the reaction was warmed to 55°C for 1-2h. The reaction is then filtered under vacuum in 1 mL solution of ammonium hydroxide and the filtrate is maintained under stirring for 4-5h at room temperature. The crude product is diluted in ethyl acetate and washed with sodium hydroxide (2N). The organic phase is then dried over sodium sulfate, filtered and concentrated in vacuo to obtain a compound of formula **8.** Crude products thus obtained are purified by flash column chromatography using dichloromethane/methanol as mobile phases.

Below follows the example of one derivative synthesized used the above-described procedure for the synthesis of final products of formula **8:**

**1-(1-(4-fluorobenzyl)indolin-5-yl)-3-neopentylguanidine**

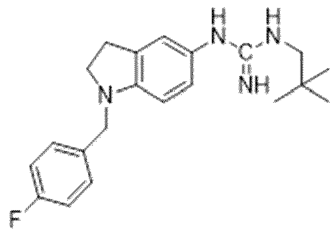

Synthesized starting from 1-(1-(4-fluorobenzyl)indolin-5-yl)-3-neopentylthiourea following the general procedure. $^1H$ NMR ($CDCl_3$, 400 MHz): δ: 0.97 (s, 9H, C$H_3$); 2.93 (t, 2H, H-3, $J$ = 8.0 Hz); 2.99 (s, 2H, C$H_2$); 3.25 (t, 2H, H-2, $J$= 8.0 Hz); 4.18 (s, 2H, C$H_2$); 6.47 (d, 1H, H-7, $J$ = 8.0 Hz); 6.67 (d, 1H, H-6, $J$ = 8.0 Hz); 6.77 (s, 1H, H-4); 7.02-7.06 (m, 2H, aryl); 7.34-7.37 (m, 2H, aryl). HR-MS *m/z:* calcd for $C_{21}H_{28}FN_4$, [(M+H)$^+$]: 355.2293; found 355.2300.

**Example 4 - Synthesis of compounds of general formula 12a or 12b**

The general synthetic procedure for compounds with general formula **12a** or **12b** is depicted in Scheme 4.

Scheme 4:

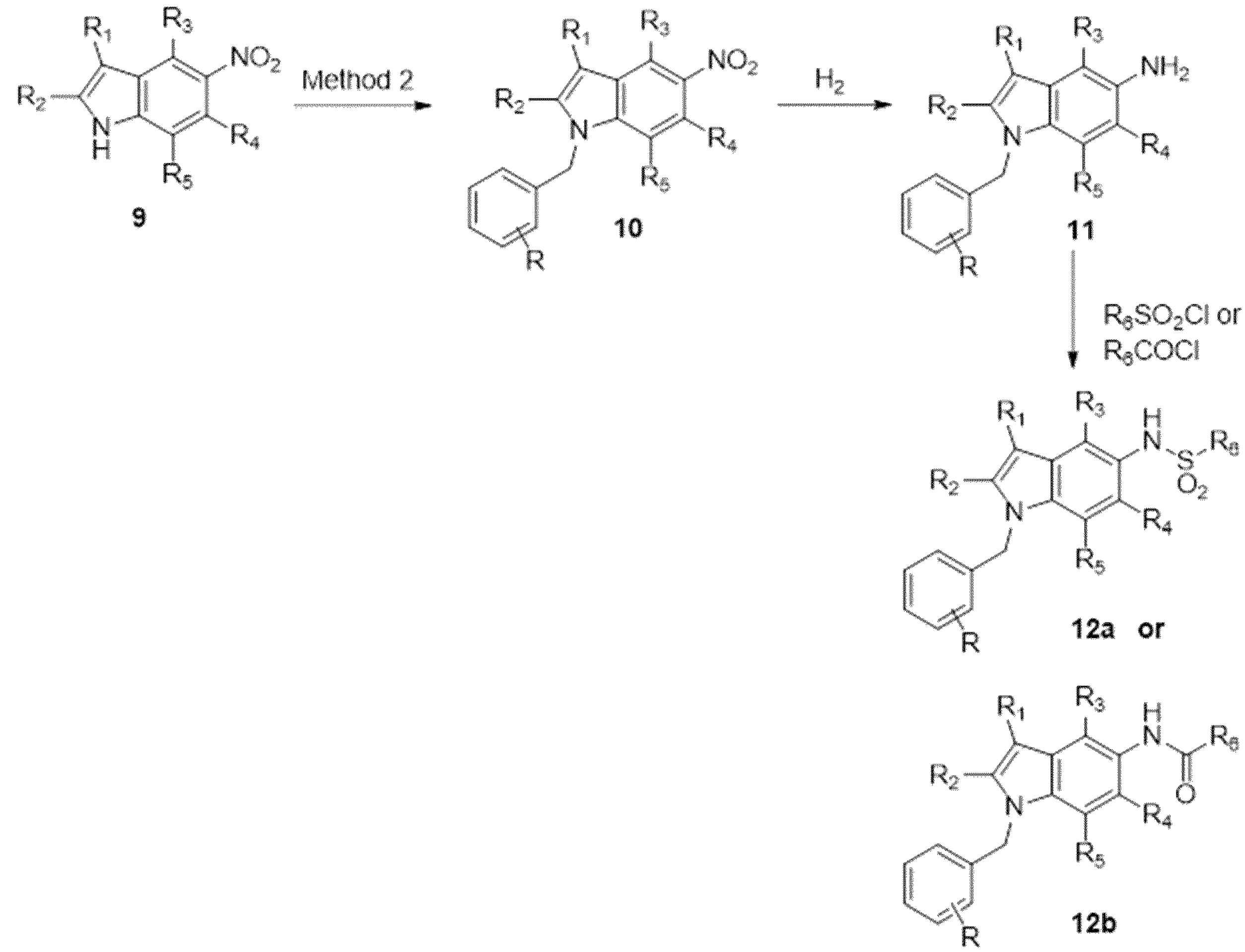


9 10 11 12a or 12b

*Synthesis of intermediates of formula **9***

The substitued 5-nitroindoles of formula **9** used as starting compounds in Scheme 4 can be synthesized using typical and well described synthetic methods that are known to skilled chemists. As an example, see WO2009/023677 A1.

*Synthesis of intermediates of formula **10***

The starting compounds of formula **9** can be then derivatized to compounds of formula **10** using the method 2 described in Example 1 for the synthesis of intermediates with general formula **2**.

*Synthesis of intermediates of formula **11***

Intermediates with the general formula **11** are synthesized following the continuous-flow hydrogenation procedures described in Example 1 for the synthesis of intermediates of formula **3.**

*Synthesis of final products of formula **12a** or **12b***

Derivatives with general formula **12a** can be synthesized according to the procedures previously described in Example 1 for the synthesis of final products of formula **4a,** while derivatives with general formula **12b** can be synthesized according to the procedures described for in Example 1 for the synthesis of final products of formula **4b.**

The list below depicts examples of some compounds prepared by using the above-described procedure for the synthesis of compounds of general formula **12a** or **12b:**

**N-(1-(4-fluorobenzyl)-1H-indol-5-yl)cyclohexanesulfonamide**

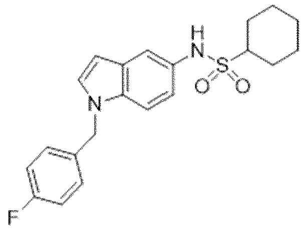

Synthesized starting from 5-nitro-1H-indole, reacted with 4-fluorobenzyl bromide following general procedure. After reduction of the nitro group, the corresponding amine was coupled with cyclohexanesulfonyl chloride. $^1$H NMR ($CDCl_3$, 400 MHz) δ: 1.19-1.29 (m, 2H, C*H*$_2$); 1.61-1.67 (m, 4H, 2 C*H*$_2$); 1.86-1.89 (m, 2H, C*H*$_2$); 2.19-2.22 (m, 2H, C*H*$_2$); 2.95-3.02 (m, 1H, C*H*); 5.29 (s, 2H, C*H*$_2$); 6.41 (s, 1H, NH); 6.55 (d, 1H, aryl, *J* = 3.2 Hz); 7.00-7.04 (m, 2H, aryl); 7.07-7.13 (m, 3H, aryl). 7.16 (d, 1H, H-2, *J* = 3.2 Hz); 7.22 (d, 1H, aryl, *J* = 8.0 Hz); 7.57 (s, 1H, aryl). HR-MS *m*/*z:* calcd for $C_{21}H_{24}FN_2O_2S$, [(M+H)$^+$]: 387.1537; found 387.1548; [(M+Na)$^+$]: 409.1356; found 409.1362.

**N-(1-(4-(trifluoromethyl)benzyl)-1H-indol-5-yl)cyclohexanesulfonamide**

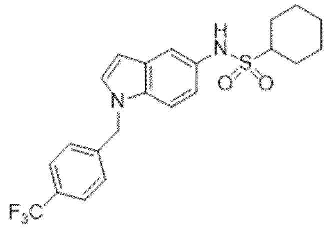

Synthesized starting from 5-nitro-1H-indole, reacted with 4-(trifluoromethyl)benzyl bromide following general procedure. After reduction of the nitro group, the corresponding amine was coupled with cyclohexanesulfonyl chloride. $^1$H NMR ($CDCl_3$, 400 MHz): δ: 1.20-1.25 (m, 2H, C*H*$_2$); 1.58-1.70 (m, 4H, 2 C*H*$_2$); 1.87-1.91 (m, 2H, C*H*$_2$); 2.20-2.23 (m, 2H, C*H*$_2$); 2.94-3.03 (m, 1H, C*H*); 5.39 (s, 2H, C*H*$_2$); 6.24 (s, 1H, N*H*); 6.58 (d, 1H, aryl, *J= 4.0* Hz); 7.08 (d, 1H, aryl, *J* = 8.0 Hz); 7.17-7.22 (m, 4H, aryl); 7.58-7.61 (m, 3H, aryl); HR-MS *m*/*z:* calcd for $C_{22}H_{24}F3N_2O_2S$, [(M+H)$^+$]: 437.1505; found 437.1513; [(M+Na)$^+$]: 459.1325; found 459.1333.

**N-(1-(4-(trifluoromethyl)benzyl)-1H-indol-5-yl)heptanamide**

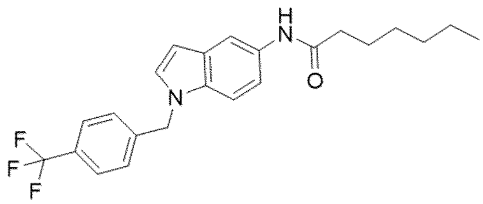

Synthesized starting from 5-nitro-1H-indole, reacted with 4-(trifluoromethyl)benzyl bromide following general procedure. After reduction of the nitro group, the corresponding amine was coupled with heptanoyl chloride.$^1$H NMR ($CDCl_3$, 400 MHz) δ: 0.92 (t, 3H, C*H*$_3$, *J* = 8.0 Hz); 1.33-1.44 (m, 6H, C*H*$_2$); 1.75-1.79 (m, 2H, C*H*$_2$); 1.94 (bs, 1H, NH); 2.39 (t, 2H, C*H*$_2$,*J* = 8.0 Hz); 5.38 (s, 2H, C*H*$_2$); 6.56 (d, 1H, aryl, *J* = 4.0 Hz); 7.14-7.23 (m, 6H, aryl); 7.56 (d, 2H, aryl, *J* = 8.0 Hz); 7.89 (s, 1H, NH);. HR-MS *m*/*z:* calcd for $C_{23}H_{26}F_3N_2O$, [(M+H)$^+$]: 403.1992; found 403.2003; [(M+Na)$^+$]: 425.1811; found 425.1820.

**N-(1-(4-methoxybenzyl)-1H-indol-5-yl)cyclohexanesulfonamide**

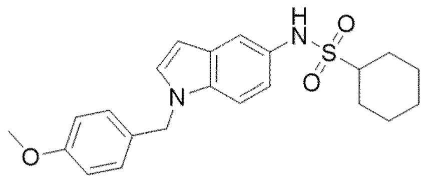

Synthesized starting from 5-nitro-1H-indole, reacted with 4-methoxybenzyl bromide following general procedure. After reduction of the nitro group, the corresponding amine was coupled with hexanesulfonyl chloride. $^1H$ NMR ($CDCl_3$, 400 MHz): δ: 1.20-1.25 (m, 2H, C*H*$_2$); 1.61-1.69 (m, 4H, 2 C*H*$_2$); 1.87-1.90 (m, 2H, C*H*$_2$); 2.20-2.22 (m, 2H, C*H*$_2$); 2.95-3.03 (m, 1H, CH); 3.80 (s, 3H, C*H*$_3$); 5.26 (s, 2H, C*H*$_2$); 6.21 (s, 1H, NH); 6.52 (d, 1H, H-3, *J* = 4.0 Hz); 6.86 (d, 2H, aryl, *J* = 12.0 Hz); 7.07-7.11 (m, 3H, H-6 and aryl); 7.16 (d, 1H, aryl, *J* = 4.0 Hz); 7.26 (d, 1H, aryl, *J* = 8.0 Hz); 7.55 (s, 1H, H-4). HR-MS *m/z:* calcd for $C_{22}H_{27}N_2O_3$, [(M+H)$^+$]: 399.1737; found 399.1745; [(M+Na)$^+$]: 421.1556; found 421.1566.

**2-cyclohexyl-N-(1-(4-fluorobenzyl)-1H-indol-5-yl)ethane-1-sulfonamide**

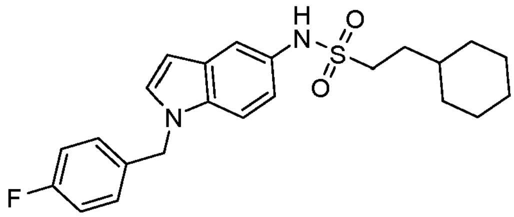

Synthesized starting from 5-nitro-1H-indole, reacted with 4-fluorobenzyl bromide following general procedure. After reduction of the nitro group, the corresponding amine was coupled with 2-cyclohexylethane-1-sulfonyl chloride. $^1H$ NMR ($CDCl_3$, 400 MHz): δ: 0.75-0.84 (m, 2H, C*H*$_2$); 1.01-1.24 (m, 4H, C*H*$_2$); 1.52-1.68 (m, 7H, C*H* and 3 C*H*$_2$); 1.62-1.68 (m, 2H, C*H*$_2$); 2.95-2.99 (m, 2H, C*H*$_2$), 5.20 (s, 2H, C*H*$_2$); 6.38 (s, 1H, N*H*); 6.45 (d, 1H, aryl, J= 3.4 Hz), 6.89-6.94 (m, 2H, aryl); 6.97-7.02 (m, 3H, Aryl); 7.07 (d, 1H, aryl, J= 4.0 Hz); 7.13 (d, 1H, aryl, J= 8.0 Hz); 7.46 (s, 1H, aryl). HR-MS *m/z:* calcd for $C_{24}H_{28}F_3N_2O_2S$, [(M+H)$^+$]: 414,1777; found 414.1783.

**3,3-dimethyl-N-(1-(4-(trifluoromethyl)benzyl)-1H-indol-5-yl)butanamide**

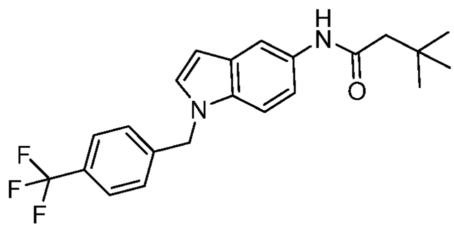

Synthesized starting from 5-nitro-1H-indole, reacted with 4-(trifluoromethyl)benzyl bromide following general procedure. After reduction of the nitro group, the corresponding amine was coupled with 3,3-dimethylbutanoyl chloride. $^1H$ NMR ($CDCl_3$, 400 MHz): δ: 1.14 (s, 9H, C*H*$_3$); 2.25 (s, 2H, C*H*$_2$); 5.36 (s, 2H, C*H*$_2$); 6.55 (s, 1H, aryl); 7.11-7.22 (m, 6H, aryl); 7.55 (d, 2H, aryl, J= 8.0 Hz); 7.90 (s, 1H, NH). HR-MS *m/z:* calcd for $C_{22}H_{24}F_3N_2O$, [(M+H)$^+$]: 389.1835; found 389.1901.

**Example 5** - **Synthesis of compounds of general formula 15a or 15b**

The general procedure for the synthesis of compounds with general formula **15a** or **15b** is shown in Scheme 5.

Scheme 5:

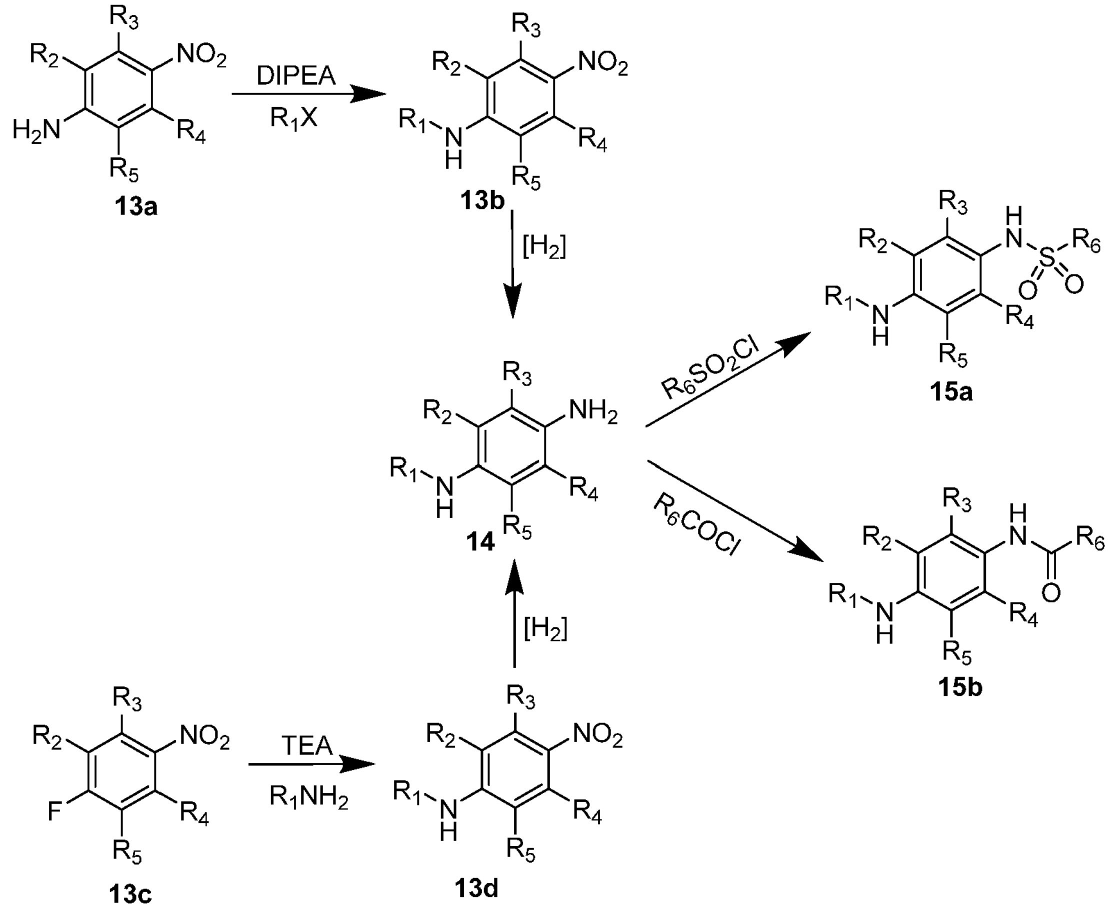


The differently substitued 4-nitroaniline of formula **13a** or the differently substituted 1-fluoro-4-nitrobenzene of formula **13c** used as starting compounds in Scheme 5 have been synthesized using typical and well described synthetic methods or, otherwise, are commercially available.

*Synthesis of intermediates of formula **13b***

For the synthesis of compounds with general formula **13b,** intermediates of formula **13a** (1.0 eq) are dissolved in 15 mL of dimethylformamide and added with 1.5 equivalents of triethylamine (TEA) or N,N-Diisopropylethylamine (DIPEA), a catalytic amount of KI (2.5% mol) and 1.1 equivalents of the proper halide with general formula RiX. The mixture is stirred at 60°C until completion. The resulting solution is diluted with water and extracted 3 times with ethyl acetate. Organic phases are dried over anhydrous $Na_2SO_4$, filtered and concentrated in vacuo. The crude products are purified by flash chromatography using mixtures of n-hexane/ethyl acetate as mobile phase.

*Synthesis of intermediates of formula **13d***

For the synthesis of compounds with general formula **13d,** intermediates of formula **13c** (1.0 eq) are dissolved in 15 mL of dimethylformamide and added with 1.2 equivalents of triethylamine (TEA), a catalytic amount of $I_2$ (5% mol) and 3.0 equivalents of the proper amine with general formula $R_1NH_2$. The mixture is refluxed at 120°C for 2.5-3.5h. The resulting solution is diluted with water and extracted 2 times with dichloromethane. Organic phases are dried over anhydrous $Na_2SO_4$, filtered and concentrated in vacuo. The crude products are purified by flash chromatography using mixtures of n-hexane/ethyl acetate as mobile phase.

*Synthesis of intermediates of formula **14***

Intermediates with general formula **14** can be synthesized starting from **13b or 13d** following the continuous-flow hydrogenation procedures described in Example 1 for the synthesis of intermediates with general formula **3.**

*Synthesis of final products of formula **15a** or **15b***

**15a** and **15b** are synthesized according to the procedures described in Example 1 for the synthesis of compounds of formula **4a** and **4b,** respectively. When optical isomers are obtained, separation is performed by HPLC using a teicoplanine-based chiral stationary phase previously described by Ismail et al. (J Chromatogr A. 2016, 4;1427:55-68).

The list below depicts examples of some compounds prepared by using the above-described procedure for the synthesis of compounds of general formula **15a** or **15b:**

**N-(2-amino-4-((4-(trifluoromethyl)benzyl)amino)phenyl)heptanamide**

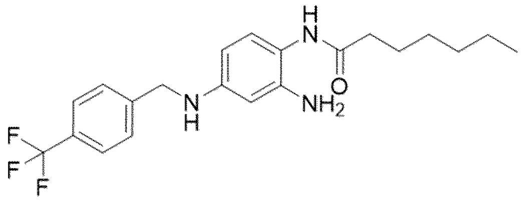

Synthesized starting from 4-nitrobenzene-1,3-diamine, reacted with 4-(trifluoromethyl)benzyl bromide following the general procedure for the synthesis of compounds with general formula **13b.** The obtained intermediate was reduced under continuous flow hydrogenation condition to give the corresponding $N^4$-(4-(trifluoromethyl)benzyl)benzene-1,2,4-triamine that was lately reacted with heptanoyl choride to give the final compound. $^1H$ NMR ($CD_3OD$, 400 MHz): δ: 0.81 (t, 3H, $CH_3$, J=4.2 Hz); 1.19-1.31 (m, 6H, 3 $CH_2$); 1.55-1.62 (m, 2H, $CH_2$); 2.25 (t, 2H, $CH_2$, J=7.8 Hz); 4.27 (s, 2H, $CH_2$); 5.95-6.00 (m, 2H, aryl); 6.65 (d, 1H, aryl, *J* = 12.2 Hz); 7.43 (d, 2H, aryl, *J* = 8.2 Hz); 7.48 (d, 2H, aryl, *J* = 8.4 Hz); HR-MS *m/z:* calcd for $C_{21}H_{27}F_3N_3O$, [(M+H)$^+$]: 394,2101; found 394.2109.

**N-(3-fluoro-4-((4-fluorobenzyl)amino)phenyl)heptanamide**

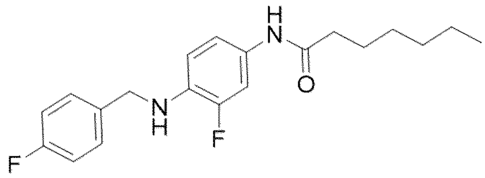

Synthesized starting from 2-fluoro-4-nitroaniline, reacted with 4-fluorobenzyl bromide following the general procedure for the synthesis of compounds with general formula **13b.** The obtained intermediate was reduced under continuous flow hydrogenation condition to give the corresponding 2-fluoro-$N^1$-(4-fluorobenzyl)benzene-1,4-diamine, that was lately reacted with heptanoyl choride to give the final compound. $^1H$ NMR ($CD_3OD$, 400 MHz): δ: 0.93 (t, 3H, $CH_3$, J=7.8 Hz); 1.31-1.42 (m, 6H, 3 $CH_2$); 1.66-1.73 (m, 2H, $CH_2$); 2.38 (t, 2H, $CH_2$, J=8.4 Hz); 4.52 (s, 2H, $CH_2$); 7.06-7.15 (m, 3H, aryl); 7.25 (d, 1H, aryl, *J* = 8.3 Hz); 7.40-7.43 (m, 2H, aryl); 7.71 (d, $^1H$ aryl, J= 12.5 Hz). HR-MS *m/z:* calcd for $C_{20}H_{25}F_2N_2O$, [(M+H)$^+$]: 347,1929; found 347,1936.

**N-(2-amino-3-fluoro-4-((4-(trifluoromethyl)benzyl)amino)phenyl)heptanamide**

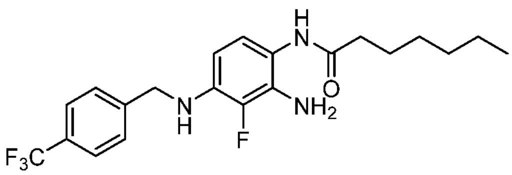

Synthesized starting from 2,3-difluoro-6-nitroaniline, reacted with (4-(trifluoromethyl)phenyl)methanamine following the general procedure for the synthesis of compounds with general formula **13d.** The obtained intermediate was reduced under continuous flow hydrogenation condition to give the corresponding 3-fluoro-$N^4$-(4-(trifluoromethyl)benzyl) benzene-1,2,4-triamine that was lately reacted with heptanoyl choride to give the final compound. $^1$H NMR (DMSO-d6, 400 MHz): δ: 0.87 (t, 3H, C*H*$_3$, J=8.3 Hz); 1.24-1.31 (m, 6H, 3 C*H*$_2$); 1.53-1.57 (m, 2H, C*H*$_2$); 2.24 (t, 2H, C*H*$_2$, J=8.1 Hz); 4.39 (d, 2H, C*H*$_2$, *J* = 7.7 Hz); 4.57 (bs, 2H, N*H*$_2$); 5.78 (t, 1H, aryl, *J* = 7.6 Hz); 6.03 (t, 1H, N*H*, *J* = 3.7 Hz); 6.58 (d, 1H, aryl, *J* = 8.4 Hz); 7.55 (d, 2H, aryl, *J* = 8.3 Hz); 7.66 (d, 2H, aryl, *J* = 8.4 Hz); 8.90 (s, 1H, CONH); HR-MS *m/z:* calcd for $C_{21}H_{26}F_4N_3O$, [(M+H)$^+$]: 412,2007; found 412.2011.

**N-(2-amino-4-((4-(trifluoromethoxy)benzyl)amino)phenyl)heptanamide**

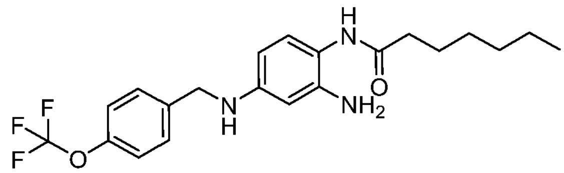

Synthesized starting from $N^1$-(4-methoxybenzyl)-3-nitrobenzene-1,4-diamine, reacted with 4-(trifluoromethyl) benzyl bromide following general procedure for the synthesis of compounds with general formula **13b.** After reduction of the nitro group, the corresponding amine was coupled with heptanoyl chloride. $^1$H NMR (DMSO-d6, 400 MHz): δ: 0.87 (t, 3H, C*H*$_3$, J=6.7 Hz); 1.20-1.31 (m, 6H, 3 C*H*$_2$); 1.53-1.57 (m, 2H, C*H*$_2$); 2.22 (t, 2H, C*H*$_2$, J=8.1 Hz); 4.22 (d, 2H, C*H*$_2$, J=7.8 Hz); 4.51 (bs, 2H N*H*$_2$); 5.84 (d, 1H, aryl, J= 8.4 Hz); 5.95-5.97 (m, 2H, aryl and NH); 6.70 (d, 1H, aryl, J= 8.2Hz); 7.30 (d, 2H, aryl, J= 11.6 Hz); 7.45 (d, 2H, aryl, J= 11.5 Hz); 8.80 (s, 1H, aryl, CONH); HR-MS *m*/*z:* calcd for $C_{21}H_{27}F_3N_3O_2$, [(M+H)$^+$]: 410,2050; found 410,2048.

**N-(2-amino-4-((4-(trifluoromethyl)benzyl)amino)phenyl)cyclohexanesulfonamide**

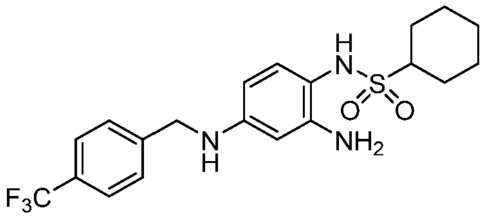

Synthesized starting from 4-nitrobenzene-1,3-diamine, reacted with 4-(trifluoromethyl)benzyl bromide following the general procedure for the synthesis of compounds with general formula **13b.** The obtained intermediate was reduced under continuous flow hydrogenation condition to give the corresponding $N^4$-(4-(trifluoromethyl)benzyl)benzene-1,2,4-triamine that was lately reacted with hexanesulfonyl chloride to give the final compound. $^1$H NMR ($CD_3OD$, 400 MHz): δ: 1.23-1.37 (m, 3H, C*H*$_2$ and C*H*); 2.21-2.24 (m, 2H, C*H*$_2$); 3.00 (t, 1H, C*H*, J=12.2 Hz); 5.13 (s, 2H, C*H*$_2$); 6.35 (d, 1H, aryl, J=8.1 Hz); 6.48 (s, 1H, aryl); 7.03 (d, 1H, aryl, J= 8.3 Hz); 7.62 (d, 2H, aryl J=8.2 Hz); 7.68 (d, 2H, aryl, J=8.3 Hz);. HR-MS *m*/*z:* calcd for $C_{20}H_{26}F_3N_3O_2S$, [(M+H)$^+$]: 429.1692; found 429.1701.

**Example 6 - Synthesis of compounds of general formula 17 or 18**

The general procedure for the synthesis of compounds with general formula **17** or **18** is shown in Scheme 6.

Scheme 6:

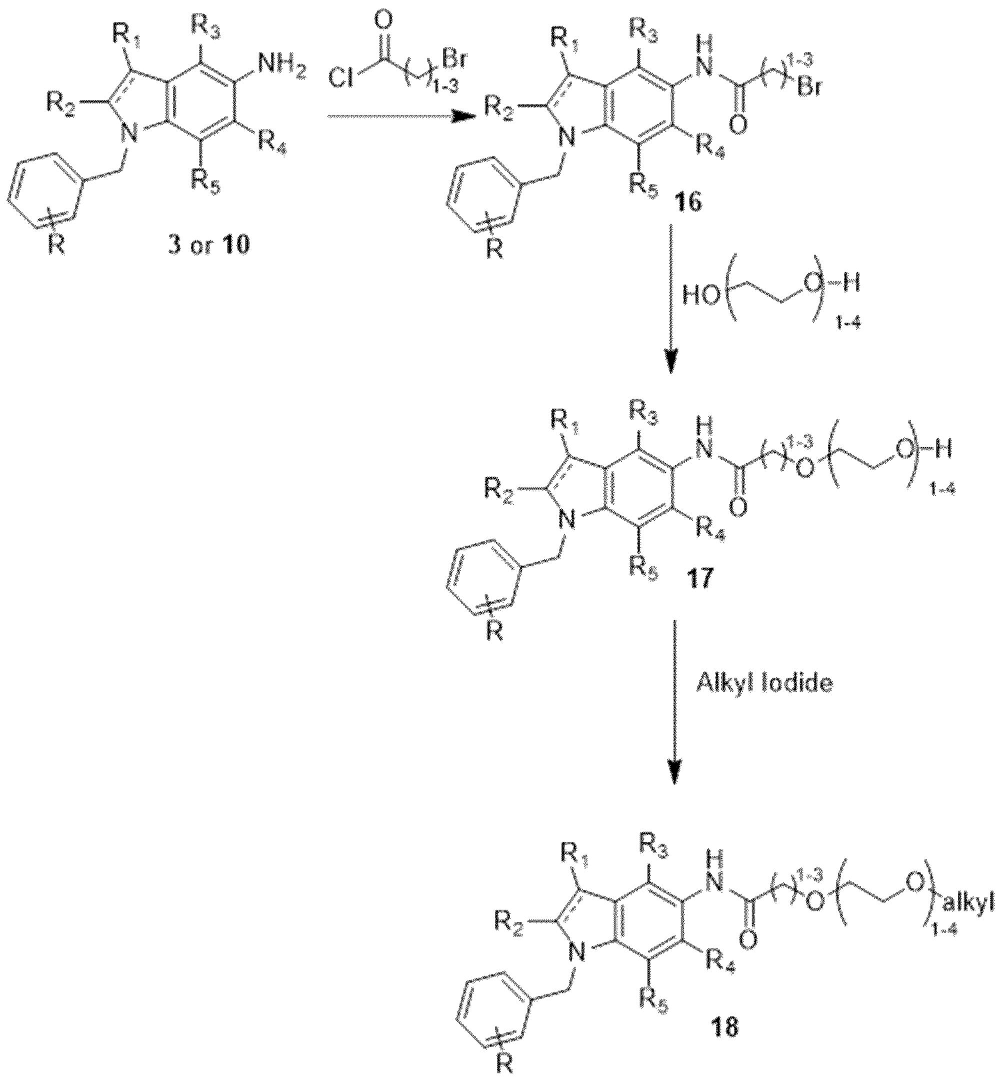


3 or 10

16

17

18

*Synthesis of final products of formula **17***

Intermediates with general formula **3** or **10** (1 eq), prepared as described in Examples 1 and 4 respectively, are dissolved in THF dry at a final concentration of 1 mM. Diisopropyl ethylamine (1.2 eq) is then added while the temperature is lowered to 0°C while stirring. To this solution is added dropwise a solution of bromoacetyl chloride or bromopropyl chloride or bromobutyl chloride in THF (1.2 eq, 1mM, 10 mL). The mixture is maintained at 0°C for 30 minutes to obtain an intermediate of formula **16,** then it is warmed to ambient temperature and 20 equivalents of the proper glycol (ethylene glycol, diethylene glycol, triethylene glycol or tetraethyleneglycol) are added together with 2.0 equivalents of DBU. The mixture is stirred for further 12 h at ambient temperature, then is diluted with water and ethyl acetate. The organic phase is isolated, washed twice with a saturated solution of sodium bicarbonate and brine, and dryed over anhydrous sodium sulfate. After filtration, the resulting solution is evaporated in vacuo and purified by flash chromatography using dichloromethane/methanol as mobile phase to afford a compound of formula **17.**

*Synthesis of final products of formula **18***

Derivatives of formula **17** (1.0 eq) are dissolved in 20 mL of anhydrous dichloromethane and added with 2.0 equivalents of DBU and 2.0 equivalents of the proper alkyl iodide of formula $CH_3(CH_2)_nI$, with n comprised between 0 and 10. The mixture is stirred at ambient temperature for 24h. Then the mixture is diluted with a saturated solution of sodium bicarbonate and further washed with brine. The organic phase is dried over anhydrous sodium sulfate, filtered and

concentrated to dryness before purification by flash column chromatography using dichloromethane/methanol as mobile phases to obtain a compound of formula **18.**

The following products represent an example of the molecules obtained by the above described synthetic protocol for the synthesis of compounds of general formula **17** or **18:**

**2-(2-methoxyethoxy)-N-(1-(4-(trifluoromethyl)benzyl)-1H-indol-5-yl)acetamide**

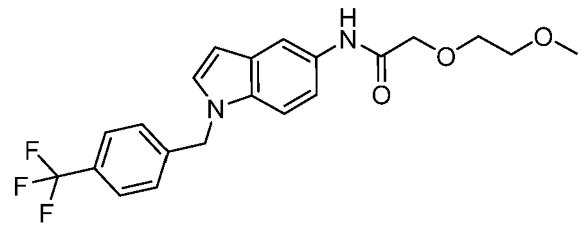

Synthesized starting from 1-(4-(trifluoromethyl)benzyl)-1H-indol-5-amine, reacted with chloroacetyl bromide following the general procedure for the synthesis of compounds with general formula **16.** The obtained intermediate of formula **16** was further reacted with ethylene glycol to give 2-(2-hydroxyethoxy)-N-(1-(4-(trifluoromethyl)benzyl)-1H-indol-5-yl)acetamide. Nucleophilic displacement of methyl iodide gave the final compound. $^1$H NMR ($CDCl_3$, 400 MHz): δ: 3.48 (s, 3H, C*H*$_3$); 3.64-3.66 (m, 2H, C*H*$_2$); 3.79-3.81 (m, 2H, C*H*$_2$); 4.16 (s, 2H, C*H*$_2$); 6.57 (d, 1H, aryl, J=3.3 Hz); 7.14-7.19 (m, 4H, aryl); 7.30 (d, 1H, aryl, J= 8.4 Hz); 7.56 (d, 2H, aryl, J=8.8 Hz); 8.01 (d, 1H, aryl J=2.4 Hz); 8.86 (s, 1H, CONH);. HR-MS *m/z:* calcd for $C_{21}H_{22}F_3N_2O_3$, [(M+H)$^+$]: 407,1577; found 407,1581.

**3-(2-methoxyethoxy)-N-(1-(4-(trifluoromethyl)benzyl)-1H-indol-5-yl)propanamide**

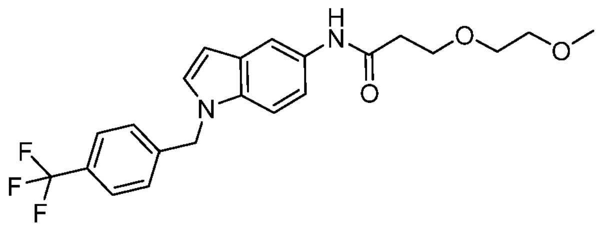

Synthesized starting from 1-(4-(trifluoromethyl)benzyl)-1H-indol-5-amine, reacted with 3-chloropropionyl chloride following the general procedure for the synthesis of compounds with general formula **16.** The obtained intermediate of formula **16** was further reacted with ethylene glycol to give 3-(2-hydroxyethoxy)-N-(1-(4-(trifluoromethyl)benzyl)-1H-indol-5-yl)propanamide. Nucleophilic displacement of methyl iodide gave the final compound. $^1$H NMR ($CDCl_3$, 400 MHz): δ: 2.60 (t, 2H C*H*$_2$, J= 7.8 Hz); 3.32 (s, 3H, C*H*$_3$); 3.42-3.54 (m, 2H, C*H*$_2$); 3.62-3.64 (m, 2H, C*H*$_2$); 3.76 (t, 2H, C*H*$_2$, J= 8.3 Hz); 5.27 (s, 2H, C*H*$_2$); 6.45 (d, 1H, aryl, J=3.4 Hz); 7.03-7.08 (m, 4H, aryl); 7.19 (d, 1H, aryl, J= 8.1 Hz); 7.45 (d, 2H, aryl, J=8.6 Hz); 7.85 (d, 1H, aryl J=2.1 Hz); 8.46 (s, 1H, CON*H*);. HR-MS *m/z:* calcd for $C_{22}H_{24}F_3N_2O_3$, [(M+H)$^+$]: 421,1734; found 421,1739.

*IN VITRO* PHARMACOLOGICAL ASSAYS

**Example 7 - Electrophysiological assays on mammalian cells transiently expressing Kv7 channel subunits**

*Cell Culture and Transient Transfection*

Channel subunits were expressed in Chinese Hamster Ovary (CHO) cells (ATCC, Catalogue Number CRL-9618) by transient transfection, using plasmids containing cDNAs encoding human Kv7.1, Kv7.2, Kv7.3, Kv7.4 and Kv7.2 W236L (a Kv7.2 mutant channel insensitive to retigabine as described in Results section) (Soldovieri et al. J Biol Chem. 2006;281(1):418-28; Iannotti et al. J Pharmacol Exp Ther. 2010;332(3):811-20; Landoulsi et al., Mol Pharmacol. 2013;84(5):763-73), all cloned in pcDNA3.1(+) vector (ThermoFisher Scientific, Catalog number: V79020). According to

the experimental protocol, these plasmids were expressed individually or in combination (in particular of Kv7.2 and Kv7.3), together with a plasmid-expressing enhanced green fluorescent protein (Clontech, Palo Alto, CA) used as a transfection marker. Total cDNA in the transfection mixture was kept at 4 μg. CHO cells were grown in 100-mm plastic Petri dishes in Dulbecco's modified Eagle's medium containing 10% fetal bovine serum, nonessential amino acids (0.1 mM), penicillin (50 U/ml), and streptomycin (50 mg/ml) in a humidified atmosphere at 37°C with 5% CO2. The cells were plated on glass coverslips coated with poly-L-lysine, and transfected one day after plating with the appropriate cDNA using Lipofectamine 2000 (Life Technologies), according to the manufacturer's protocol.

*Whole-cell electrophysiology*

Currents from CHO cells were recorded at room temperature (20-22°C) 24 hours after trasfection using the whole-cell configuration of the patch-clamp technique, with glass micropipettes of 3-5 MΩ resistance. During the recording, constant perfusion of extracellular solution was maintained. The extracellular solution contained (in mM): 138 NaCl, 2 $CaCl_2$, 5.4 KCl, 1 $MgCl_2$, 10 glucose, and 10 HEPES, at pH 7.4, adjusted with NaOH. The pipette (intracellular) solution contained (in mM): 140 KCl, 2 $MgCl_2$, 10 EGTA, 10 HEPES, 5 $Mg^{2+}$-ATP, at pH 7.3-7.4, adjusted with KOH. Currents were recorded using an Axopatch-200A amplifier, filtered at 5 kHz, and digitized using a DigiData 1440A (Molecular Devices). The pCLAMP software (version 10.2, Molecular Devices) was used for data acquisition and analysis. To evaluate the activity of exemplary compounds on Kv7.2 (see Table 1), as well as on Kv7.1, Kv7.2/7.3, Kv7.3 and Kv7.4 channels for selected compounds (see Table 2), the cells were clamped at -80 mV and currents were elicited by 3-s voltage ramps from -80 mV to +20 mV in the presence and absence of each compound. To calculate the $EC_{50}$, cells were perfused with increased concentration of the compund (0.01, 0.1, 1, 10 μM) until saturation was achieved. The effects of drugs were expressed as shift in the ramp-voltage (ΔV) calculated at 20% of the maximal current (Miceli et al, Proc Natl Acad Sci U S A. 2013, 110(11):4386-91). Curves were fitted using the following equation: y=max/(1+x/EC50)n, where x is the drug concentration, n is the Hill coefficient and max is the maximal value achieved, as previously described (Ambrosino et al, Br J Pharmacol. 2013, 168(6):1430-1444). Data analysis and fit were performed using Sigmaplot (version 12.3).

*Results*

Ramp-evoked Kv7.2 currents were analyzed by measuring the currents at -40 mV (a membrane potential value close to the activation threshold) and at 0 mV (a membrane potential value at which Kv7.2 conductance is largely saturated). In particular, -40 mV represents the threshold potential for spike initiation and 0 mV represents instead the membrane potential value at which maximal conductance of the channel is achieved. Using the measurements at -40 and 0 mV, the inventors have approximated drug effects at membrane potential values achieved by the neurons during low and high frequency firing activity, respectively. Results are reported as average values ± standard error from the mean (N ±SEM).

In Table 1, the effect of exemplary compounds of the invention is expressed as the ratio between current amplitude in the presence ($I_{drug}$) and absence ($I_{ctl}$) of the drug at these two membrane voltages. $I_{drug}/I_{ctl}$>1 represents activating activity; $I_{drug}/I_{ctl}$<1 represents inhibiting activity; $I_{drug}/I_{ctl}$=1 indicates no effect of the drug on this parameter.

**Table 1. Effect of exemplary compounds on Kv7.2 in comparison with retigabine**

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 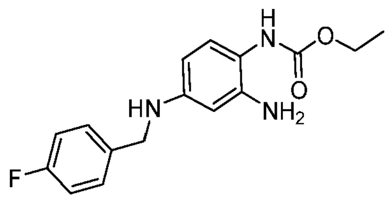 | **Retigabine** | 6.20±1.03 | 1.95±0.12 | 38 |
| 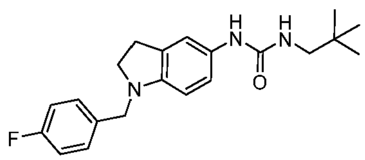 | **1-(1-(4-fluorobenzyl)indolin-5-yl)-3-neopentylurea** | 3.31±0.61 | 1.40±0.08 | 6 |

(continued)

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 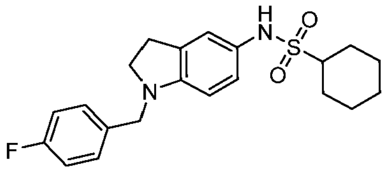 | **N-(1-(4-fluorobenzyl)indolin-5-yl) cyclohexanesulfonamide** | 6.41±0.93 | 2.81±0.26 | 9 |
| 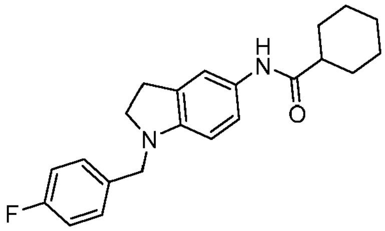 | **N-(1-(4-fluorobenzyl)indolin-5-yl) cyclohexanecarboxamide** | 1.32±0.21 | 0.66±0.07 | 3 |
| 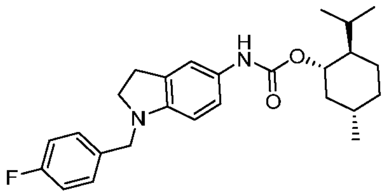 | **(1S,2R,5S)-2-isopropyl-5-methyl-cyclohexyl (1-(4-fluorobenzyl)in-dolin-5-yl)carbamate** | 0.80±0.14 | 0.48±0.07 | 5 |
| 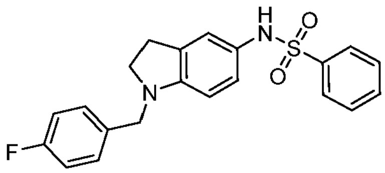 | **N-(1-(4-fluorobenzyl)indolin-5-yl) benzenesulfonamide** | 1.15±0.28 | 0.64±0.14 | 3 |
| 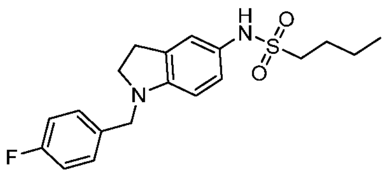 | **N-(1-(4-fluorobenzyl)indolin-5-yl) butane-1-sulfonamide** | 2.03±0.19 | 1.55±0.12 | 3 |
| 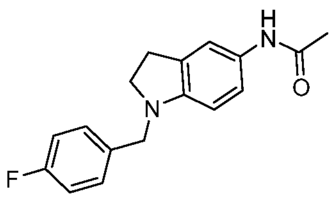 | **N-(1-(4-fluorobenzyl)indolin-5-yl) acetamide** | 1.88±0.14 | 1.64±0.20 | 5 |
| 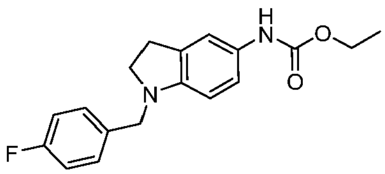 | **ethyl (1-(4-fluorobenzyl)indolin-5-yl)carbamate** | 2.01±0.58 | 1.26±0.13 | 6 |
| 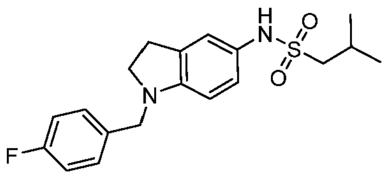 | **N-(1-(4-fluorobenzyl)indolin-5-yl)-2-methylpropane-1-sulfonamide** | 2.55±0.79 | 1.93±0.32 | 5 |

(continued)

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 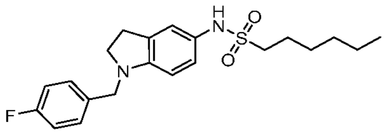 | **N-(1-(4-fluorobenzyl)indolin-5-yl) hexane-1-sulfonamide** | 2.48±0.20 | 2.18±0.09 | 3 |
| 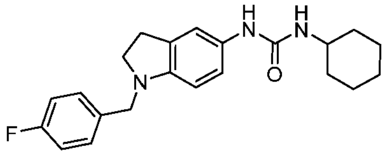 | **1-cyclohexyl-3-(1-(4-fluorobenzyl) indolin-5-yl)urea** | 0.95±0.16 | 0.85±0.12 | 3 |
| 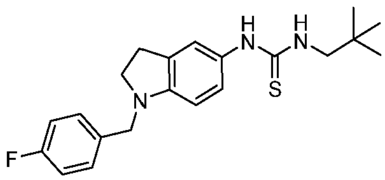 | **1-(1-(4-fluorobenzyl)indolin-5-yl)-3-neopentylthiourea** | 1.77±0.77 | 1.04±0.24 | 3 |
| 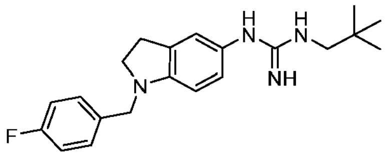 | **1-(1-(4-fluorobenzyl)indolin-5-yl)-3-neopentylguanidine** | 0.98±0.09 | 0.84±0.09 | 3 |
| 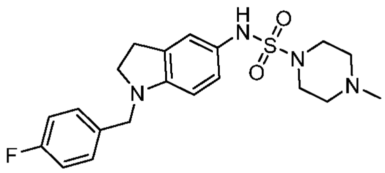 | **N-(1-(4-fluorobenzyl)indolin-5-yl)-4-methylpiperazine-1-sulfonamide** | 1.25±0.19 | 0.95±0.07 | 4 |
| 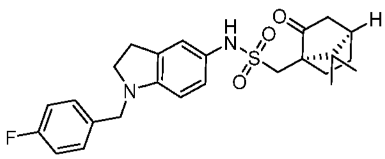 | **1-((1S,4R)-7,7-dimethyl-2-oxobicyclo[2.2.1]heptan-1-yl)-N-(1-(4-fluorobenzyl)indolin-5-yl)methanesulfonamide** | 1.12±0.05 | 0.99±0.06 | 3 |
| 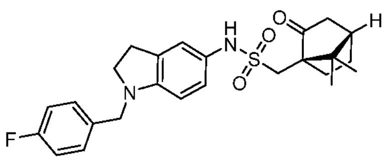 | **1-((1R,4S)-7,7-dimethyl-2-oxobicyclo[2.2.1]heptan-1-yl)-N-(1-(4-fluorobenzyl)indolin-5-yl)methanesulfonamide** | 1.49±0.28 | 1.16±0.07 | 5 |
| 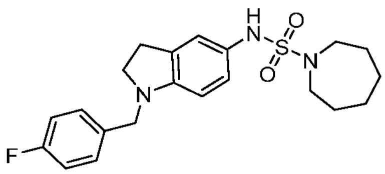 | **N-(1-(4-fluorobenzyl)indolin-5-yl) azepane-1-sulfonamide** | 0.89±0.12 | 0.98±0.11 | 5 |
| 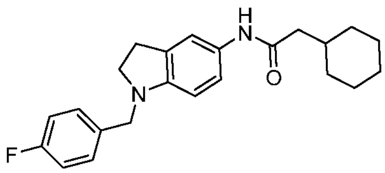 | **2-cyclohexyl-N-(1-(4-fluorobenzyl) indolin-5-yl)acetamide** | 2.37±0.40 | 1.71±0.12 | 4 |

(continued)

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 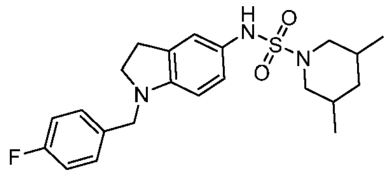 | N-(1-(4-fluorobenzyl)indolin-5-yl)-3,5-dimethylpiperidine-1-sulfonamide | 1.91±0.44 | 1.87±0.22 | 3 |
| 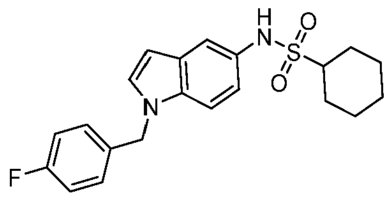 | N-(1-(4-fluorobenzyl)-1H-indol-5-yl)cyclohexanesulfonamide | 4.71±1.31 | 2.76±0.19 | 4 |
| 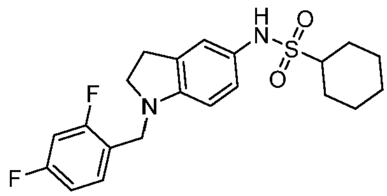 | N-(1-(2,4-difluorobenzyl)indolin-5-yl)cyclohexanesulfonamide | 5.82±1.38 | 3.79±1.09 | 3 |
| 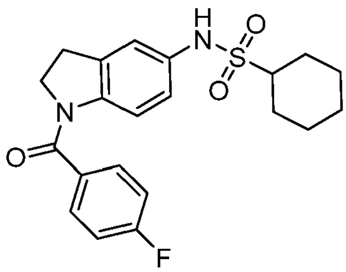 | N-(1-(4-fluorobenzoyl)indolin-5-yl) cyclohexanesulfonamide | 1.85±0.30 | 1.22±0.07 | 3 |
| 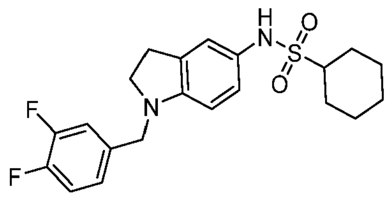 | N-(1-(3,4-difluorobenzyl)indolin-5-yl)cyclohexanesulfonamide | 4.56±1.11 | 2.11±0.18 | 4 |
| 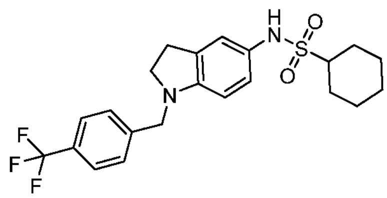 | N-(1-(4-(trifluoromethyl)benzyl)indolin-5-yl)cyclohexanesulfonamide | 3.2±0.20 | 2.37±0.58 | 4 |
| 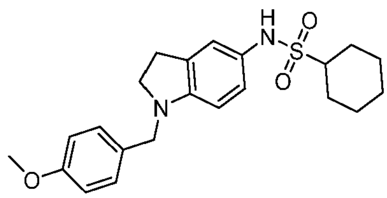 | N-(1-(4-methoxybenzyl)indolin-5-yl)cyclohexanesulfonamide | 6.43±1.51 | 4.24±0.83 | 3 |

(continued)

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 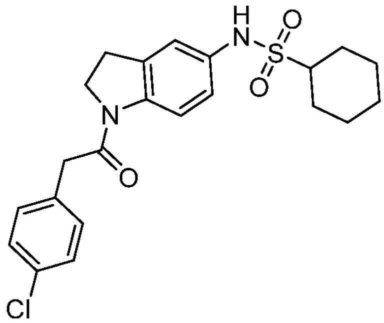 | **N-(1-(2-(4-chlorophenyl)acetyl)in-dolin-5-yl)cyclohexanesulfona-mide** | 1.45±0.08 | 1.18±0.08 | 3 |
| 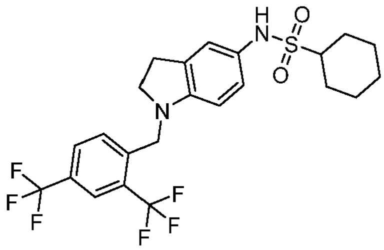 | **N-(1-(2,4-bis(trifluoromethyl)benzyl)i ndolin-5-yl)cyclohexanesulfonamide** | 1.53±0.39 | 1.22±0.11 | 5 |
| 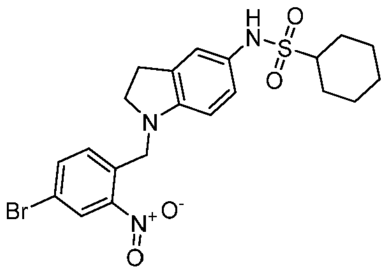 | **N-(1-(4-bromo-2-nitrobenzyl)indolin-5-yl)cyclohexanesulfonamide** | 7.27±1.32 | 3.09±0.52 | 4 |
| 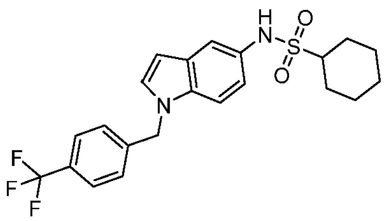 | **N-(1-(4-(trifluoromethyl)benzyl)-1H-indol-5-yl)cyclohexanesulfonamide** | 2.27±0.49 | 1.93±0.19 | 3 |
| 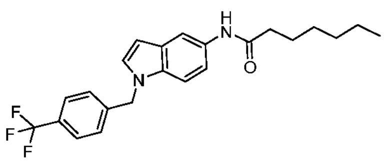 | **N-(1-(4-(trifluoromethyl)benzyl)-1H-indol-5-yl)heptanamide** | 9.34±0.82 | 3.24±0.18 | 7 |
| 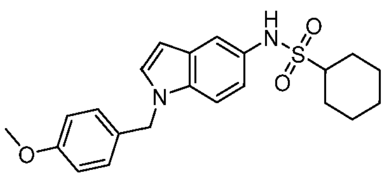 | **N-(1-(4-methoxybenzyl)-1H-indol-5-yl)cyclohexanesulfonamide** | 2.00±0.36 | 1.63±0.06 | 3 |

(continued)

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 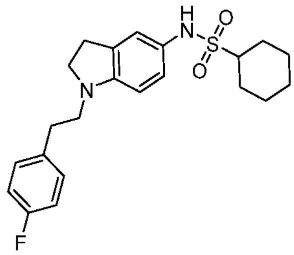 | N-(1-(4-fluorophenethyl)indolin-5-yl)cyclohexanesulfonamide | 3.24±1.52 | 1.37±0.17 | 4 |
| 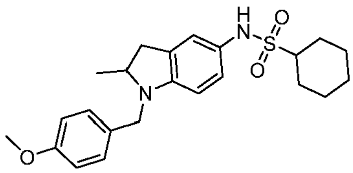 | N-(1-(4-methoxybenzyl)-2-methylindolin-5-yl)cyclohexanesulfonamide | 1.13±0.15 | 0.92±0.13 | 3 |
| 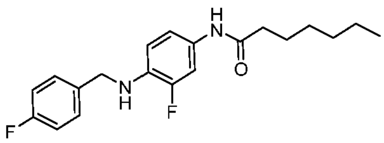 | N-(3-fluoro-4-((4-fluorobenzyl)amino)phenyl) heptanamide | 8.85±1.17 | 3.30±0.40 | 3 |
| 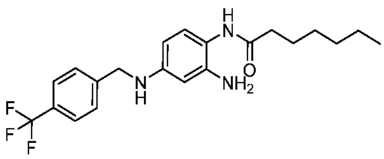 | N-(2-amino-4-((4-(trifluoromethyl) benzyl)ami no)phenyl)heptanamide | 16.90±3.33 | 2.55±0.56 | 5 |
| 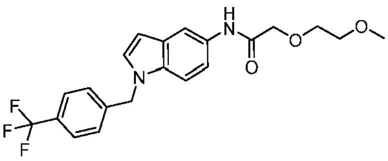 | 2-(2-methoxyethoxy)-N-(1-(4-(trifluoromethyl)benzyl)-1H-indol-5-yl)acetamide | 1.50±0.16 | 0.86±0.01 | 4 |
| 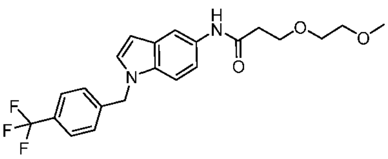 | 3-(2-methoxyethoxy)-N-(1-(4-(trifluoromethyl)benzyl)-1H-indol-5-yl)propanamide | 2.64±0.77 | 1.34±0.10 | 4 |
| 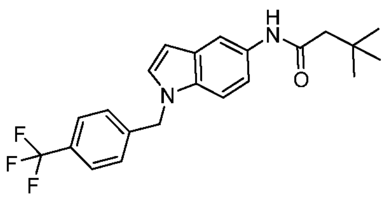 | 3,3-dimethyl-N-(1-(4-(trifluoromethyl)benzyl)-1H-indol-5-yl)butanamide | 8.23±1.36 | 3.54±0.88 | 3 |
| 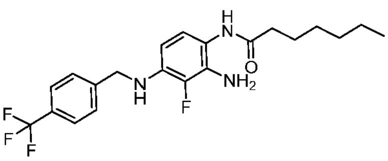 | N-(2-amino-3-fluoro-4-((4-(trifluoromethyl)benzyl)ami no)phenyl) heptanamide | 5.76±1.28 | 1.94±0.12 | 3 |
| 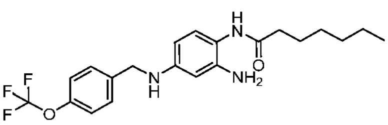 | N-(2-amino-4-((4-(trifluoromethoxy)benzyl)a mino)phenyl) heptanamide | 12.4±2.05 | 2.05±0.56 | 3 |

(continued)

| COMPOUND STRUCTURE | COMPOUND NAME | $I_{drug}/I_{ctl}$ at -40 mV | $I_{drug}/I_{ctl}$ at 0 mV | N |
|---|---|---|---|---|
| 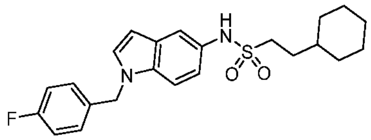 | **2-cyclohexyl-N-(1-(4-fluorobenzyl)-1H-indol-5-yl)ethane-1-sulfonamide** | 1.64±0.10 | 1.18±0.09 | 3 |
| 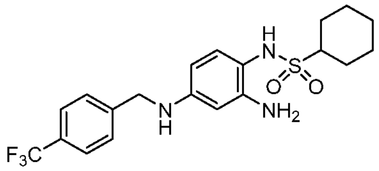 | **N-(2-amino-4-((4-(trifluoromethyl) benzyl)ami no)phenyl)cyclohexanesulfo namide** | 1.5±0.2 | 0.19±0.1 | 3 |
| 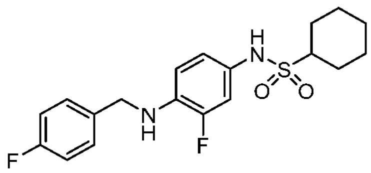 | **N-(3-fluoro-4-((4-fluorobenzyl)amino)phenyl) cyclohexanesulfonamide** | 0.78±0.15 | 0.83±0.10 | 5 |

As evidenced in Table 1, indole, indoline and aryl-1-4diamine are all suitable scaffolds for the design of Kv7.2 channel modulators. The activity of the derivatives is modulated in particular by the substituents present in position N1 of the indole/indoline core, as well in position N4 of the aryldiamino core. It was also found that the activity of the tested compounds is influenced by the substitution in position N5 of the indole/indoline core and N1 of the aryldiamine core, as well by the nature of the bond between these substituents and the nitrogen itself (i.e. substituent Q). Sulfonamide and amide derivatives act, preferentially, as agonists of Kv7.2 channel, depending also on the nature of the side chain. Flexible and highly lipophilic side chains (as for N-(2-amino-4-((4-(trifluoromethoxy)benzyl)amino)phenyl)heptanamide, N-(2-amino-4-((4-(trifluoromethyl)benzyl)amino)phenyl)heptanamide, N-(1-(4-fluorobenzyl)indolin-5-yl)cyclohexanesulfonamide and 3,3-dimethyl-N-(1-(4-(trifluoromethyl)benzyl)-1H-indol-5-yl)butanamide) tend to enhance the agonistic activity. Contrariwise, the use of rigid substituents is able to reduce currents, leading some of the tested molecules to behave as antagonist, as for example N-(1-(4-fluorobenzyl)indolin-5-yl)benzenesulfonamide, N-(1-(4-fluorobenzyl)indolin-5-yl)azepane-1-sulfonamide. The use of more rigid Q substituents such as urea (as for 1-cyclohexyl-3-(1-(4-fluorobenzyl) indolin-5-yl)urea) and guanidine (as for 1-(1-(4-fluorobenzyl)indolin-5-yl)-3-neopentylguanidine) tends to switch the activity to antagonism, as well.

The $EC_{50}$ of retigabine and of a compound of the invention [N-(1-(4-(trifluoromethyl)benzyl)-1H-indol-5-yl) heptanamide] for Kv.7.1, Kv7.2, Kv7.2/3, Kv7.3 and Kv7.4 currents was calculated (Table 2). For another compound of the invention (N-(2-amino-4-((4-(trifluoromethyl)benzyl)amino)phenyl)heptanamide), the $EC_{50}$ for Kv 7.1 and Kv7.2/7.3 was calculated (Table 2).

**Table 2. $EC_{50}$ values of retigabine, N-(1-(4-(trifluoromethyl)benzyl)-1H-indol-5-yl)heptanamide and N-(2-amino-4-((4-(trifluoromethyl)benzyl)amino)phenyl)heptanamide on different subtype of Kv7 channels**

| | **$EC_{50}$ (μM)** | | |
|---|---|---|---|
| | **RETIGABINE** | **N-(1-(4-(trifluoromethyl) benzyl)-1H-indol-5-yl) heptanamide** | **N-(2-amino-4-((4-(trifluoromethyl) benzyl) amino)phenyl)heptana mide** |
| **Kv7.1** | Inactive | Inactive | Inactive |
| **Kv7.2** | 0.93±0.43 | 0.08±0.04 | |
| **Kv7.2/7.3** | 1.72±0.41 | 0.21±0.14 | 0.20±0.10 |
| **Kv7.3** | 3.9±1.7 | 10.5±6 | |
| **Kv7.4** | 1.98±0.84 | 0.10±0.02 | |

The data shown in Table 2 suggest that the two novel compounds N-(1-(4-(trifluoromethyl)benzyl)-1H-indol-5-yl)heptanamide and N-(2-amino-4-((4-(trifluoromethyl)benzyl)amino)phenyl)heptanamide are eight times more potent than retigabine with respect to their ability to increase Kv7.2/3 currents. The data also suggest that the potency of N-(1-(4-(trifluoromethyl)benzyl)-1H-indol-5-yl)heptanamide is about 130-fold higher in activating Kv7.2 when compared to Kv7.3 homomers; by contrast, retigabine potency at Kv7.2 channels is only 4 times higher than that at Kv7.3 channels; the $EC_{50}$ of N-(1-(4-(trifluoromethyl)benzyl)-1H-indol-5-yl)heptanamide for Kv7.2 channels is similar to that for Kv7.4 channels. Due to their higher potency on Kv7.2/3 current, N-(1-(4-(trifluoromethyl)benzyl)-1H-indol-5-yl)heptanamide and N-(2-amino-4-((4-(trifluoromethyl)benzyl)amino)phenyl)heptanamide are expected to exert their desired pharmacodynamic effects at lower concentrations than retigabine.

Additionally, similar to retigabine, N-(1-(4-(trifluoromethyl)benzyl)-1H-indol-5-yl)heptanamide does not potentiate the currents carried by cardiac Kv7.1 channels, thus avoiding detrimental cardiovascular side effects. As a matter of fact, retigabine is selectively active on specific Kv7 channel subtypes, due to its ability to interact with specific channel residues; in particular, a major component of the retigabine binding site seems to be provided by a tryptophane residue at position 236 (W236) along the primary sequence of Kv7.2 subunit. The substitution of this residue with a leucine (the naturally-occurring residue in retigabine-insensitive Kv7.1 channels; W236L substitution) largely hampers the ability of retigabine to act as a Kv7.2 channel modulator (Wuttke et al., Mol. Pharmacol., 2005, 67(4), 1009-1017). Table 2 shows that the same W236L substitution also removes the activity of N-(1-(4-(trifluoromethyl)benzyl)-1H-indol-5-yl)heptanamide, one of the most effective compound herein described as Kv7.2 modulators.

In particular, application of 10 μM retigabine or 10 μM N-(1-(4-(trifluoromethyl)benzyl)-1H-indol-5-yl)heptanamide in cells expressing Kv7.2 W236L mutant channels, failed to affect current amplitude; indeed, the ratio between current amplitude in the presence and absence of the drug at -40 mV was $1.2\pm0.1$ and $1.3\pm0.3$ for retigabine and N-(1-(4-(trifluoromethyl)benzyl)-1H-indol-5-yl)heptanamide, respectively (see in vitro pharmacological assays). These results suggest that this compound displays a similar mode of action to retigabine.

**Example 8** - **Stability**

*Materials and methods*

In order to study the solution stability and photostability of the potassium channel modulators object of the present invention, a stock solution of the proper analyte (1 mg/mL) in dimethylsulfoxide (DMSO) has been prepared. This solution has been then diluted to a concentration of 100 ppm using extracellular solution containing (in mM): 138 NaCl, 2 $CaCl_2$, 5.4 KCl, 1 $MgCl_2$, 10 glucose, and 10 HEPES, at pH 7.4, adjusted with NaOH. To measure solution stability, the diluted solutions have been maintained at ambient temperature for 6 h. To measure photostability, the diluted solutions have been irradiated for 6 h using a Oriel Sol3A Solar Simulator (Newport corporation, Irvine, CA, USA) with a 12x12 inches output beam. Aliquots of solution have been withdrawn after 6h and analyzed by HPLC using a Kinetex C18, 150 mm × 4.6 mm × 2.6 μm (Phenomenex, Bologna, Italy) as stationary phase and a mobile phase constituted by A: $H_2O/CH_3CN$ (80:20) and B: $CH_3CN$. Gradient elution has been performed from 20% to 90% of B in 7.50 minutes and from 90% to 20% of B in 1 minute. The pump flux has been set to 1.5 mL/min. Column has been thermostatted at 40°C during analysis. Results are espressed in Table 3 as percentage of degradation of the product calculated as ratio between the peak area of the analyte at time 0 ($Pa_0$) and after 6h ($Pa_6$) using the following formula:

$$\% \text{ degradation} = (Pa_0\text{-}Pa_{6})/Pa_0 * 100.$$

*Results*

**Table 3: Stability in solution and photostability of exemplary compounds object of the present invention in comparison with retigabine**

| Compound structure | Compound name | Solution stability | Photostability |
|---|---|---|---|
| 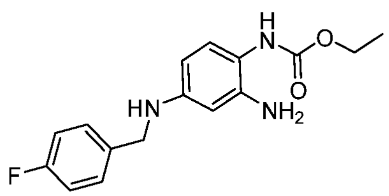 | **Retigabine** | A | C |

(continued)

| Compound structure | Compound name | Solution stability | Photostability |
|---|---|---|---|
| 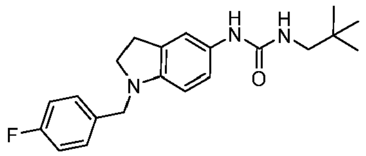 | 1-(1-(4-fluorobenzyl)indolin-5-yl)-3-neopentylurea | A | A |
| 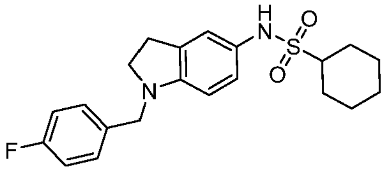 | N-(1-(4-fluorobenzyl)indolin-5-yl)cyclohexanesulfonamide | C | C |
| 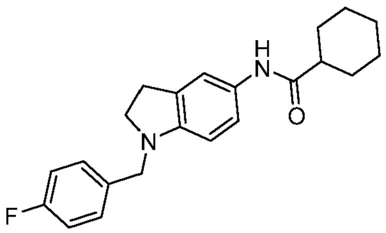 | N-(1-(4-fluorobenzyl)indolin-5-yl)cyclohexanecarboxamide | A | B |
| 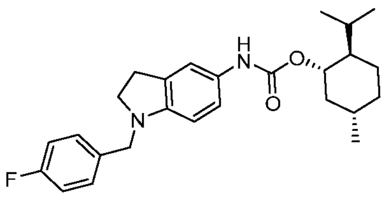 | (1S,2R,5S)-2-isopropyl-5-methylcyclohexyl (1-(4-fluorobenzyl)indolin-5-yl)carbamate | A | B |
| 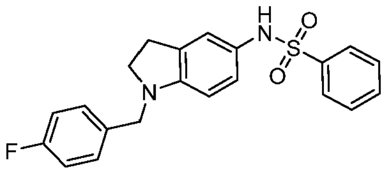 | N-(1-(4-fluorobenzyl)indolin-5-yl)benzenesulfonamide | A | B |
| 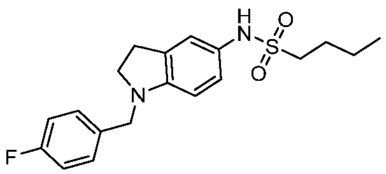 | N-(1-(4-fluorobenzyl)indolin-5-yl)butane-1-sulfonamide | C | C |
| 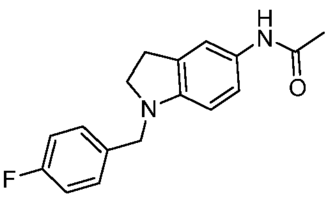 | N-(1-(4-fluorobenzyl)indolin-5-yl)acetamide | A | A |
| 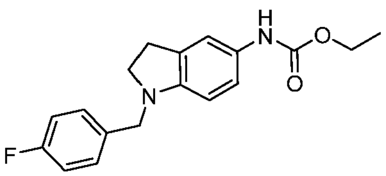 | ethyl (1-(4-fluorobenzyl)indolin-5-yl)carbamate | A | C |

(continued)

| Compound structure | Compound name | Solution stability | Photostability |
|---|---|---|---|
| 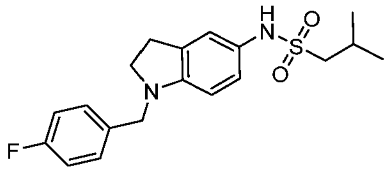 | N-(1-(4-fluorobenzyl)indolin-5-yl)-2-methylpropane-1-sulfonamide | C | C |
| 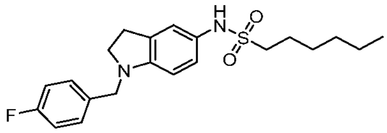 | N-(1-(4-fluorobenzyl)indolin-5-yl)hexane-1-sulfonamide | B | C |
| 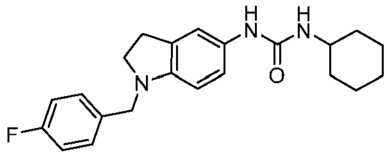 | 1-cyclohexyl-3-(1-(4-fluorobenzyl)indolin-5-yl)urea | A | B |
| 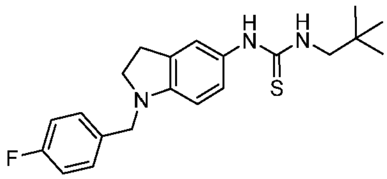 | 1-(1-(4-fluorobenzyl)indolin-5-yl)-3-neopentylthiourea | A | B |
| 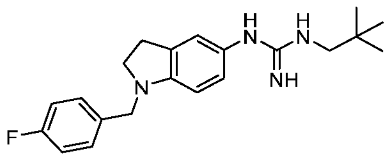 | 1-(1-(4-fluorobenzyl)indolin-5-yl)-3-neopentylguanidine | A | B |
| 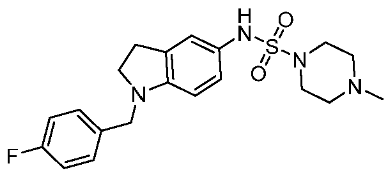 | N-(1-(4-fluorobenzyl)indolin-5-yl)-4-methylpiperazine-1-sulfonamide | B | B |
| 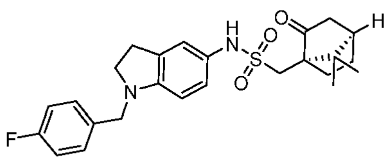 | 1-((1S,4R)-7,7-dimethyl-2-oxobicyclo [2.2.1]heptan-1-yl)-N-(1-(4-fluorobenzyl) indolin-5-yl)methanesulfonamide | C | C |
| 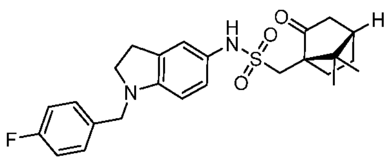 | 1-((1R,4S)-7,7-dimethyl-2-oxobicyclo [2.2.1]heptan-1-yl)-N-(1-(4-fluorobenzyl) indolin-5-yl)methanesulfonamide | C | C |
| 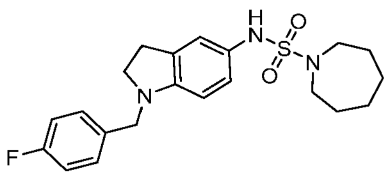 | N-(1-(4-fluorobenzyl)indolin-5-yl)azepane-1-sulfonamide | B | B |

(continued)

| Compound structure | Compound name | Solution stability | Photostability |
|---|---|---|---|
| 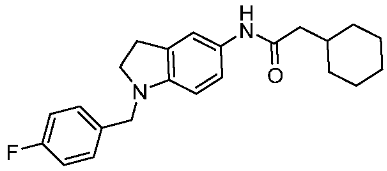 | **2-cyclohexyl-N-(1-(4-fluorobenzyl)indolin-5-yl)acetamide** | A | A |
| 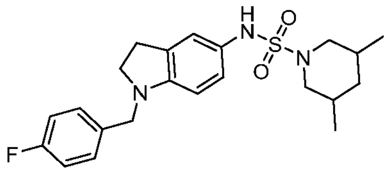 | **N-(1-(4-fluorobenzyl)indolin-5-yl)-3,5-dimethylpiperidine-1-sulfonamide** | B | B |
| 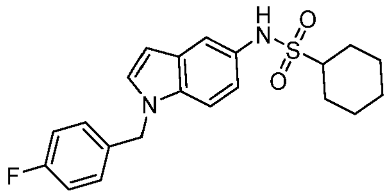 | **N-(1-(4-fluorobenzyl)-1H-indol-5-yl)cyclohexanesulfonamide** | A | A |
| 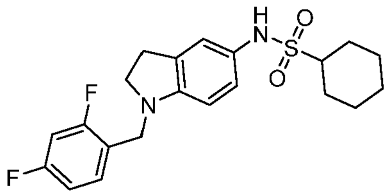 | **N-(1-(2,4-difluorobenzyl)indolin-5-yl)cyclohexanesulfonamide** | C | C |
| 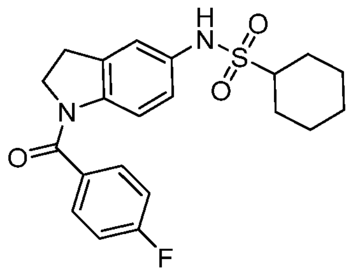 | **N-(1-(4-fluorobenzoyl)indolin-5-yl)cyclohexanesulfonamide** | A | B |
| 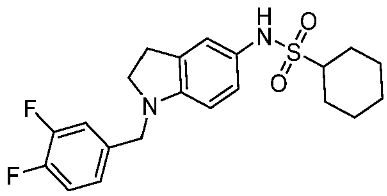 | **N-(1-(3,4-difluorobenzyl)indolin-5-yl)cyclohexanesulfonamide** | C | C |
| 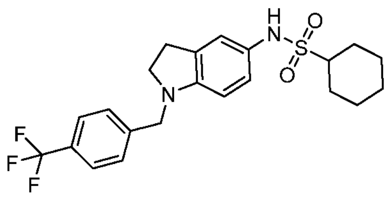 | **N-(1-(4-(trifluoromethyl)benzyl)indolin-5-yl)cyclohexanesulfonamide** | C | C |

(continued)

| Compound structure | Compound name | Solution stability | Photostability |
|---|---|---|---|
| 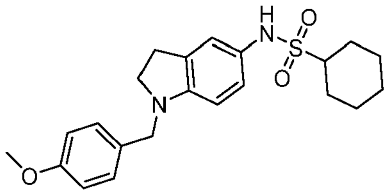 | N-(1-(4-methoxybenzyl)indolin-5-yl)cyclohexanesulfonamide | C | C |
| 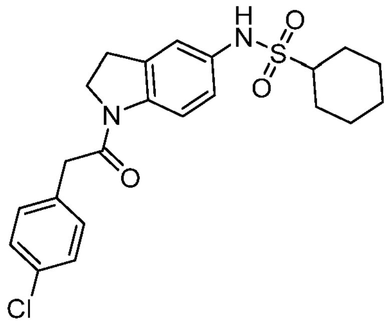 | N-(1-(2-(4-chlorophenyl)acetyl)indolin-5-yl)cyclohexanesulfonamide | A | B |
| 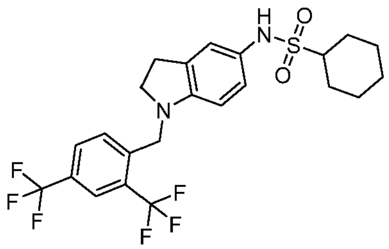 | N-(1-(2,4-bis(trifluoromethyl)benzyl)indolin-5-yl)cyclohexanesulfonamide | B | C |
| 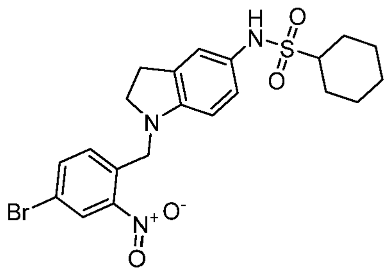 | N-(1-(4-bromo-2-nitrobenzyl)indolin-5-yl) cyclohexanesulfonamide | B | C |
| 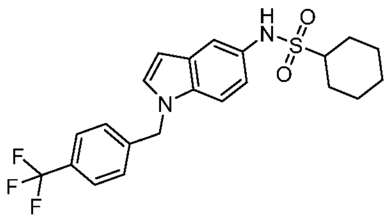 | N-(1-(4-(trifluoromethyl)benzyl)-1H-indol-5-yl)cyclohexanesulfonamide | A | A |
| 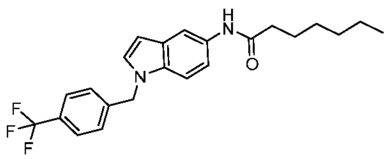 | N-(1-(4-(trifluoromethyl)benzyl)-1H-indol-5-yl)heptanamide | A | A |

(continued)

| Compound structure | Compound name | Solution stability | Photostability |
|---|---|---|---|
| 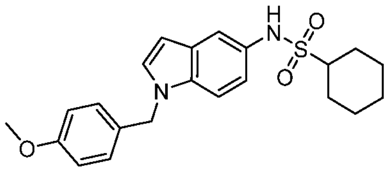 | N-(1-(4-methoxybenzyl)-1H-indol-5-yl)cyclohexanesulfonamide | A | A |
| 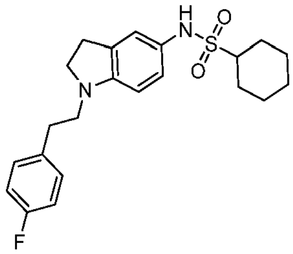 | N-(1-(4-fluorophenethyl)indolin-5-yl)cyclohexanesulfonamide | C | C |
| 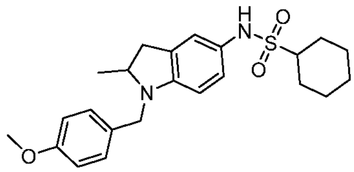 | N-(1-(4-methoxybenzyl)-2-methylindolin-5-yl)cyclohexanesulfonamide | B | C |
| 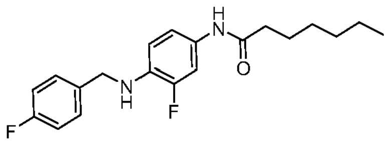 | N-(3-fluoro-4-((4-fluorobenzyl)amino) phenyl)heptana mide | A | A |
| 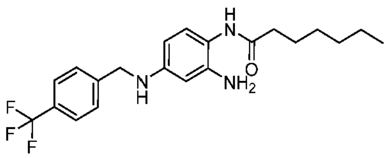 | N-(2-amino-4-((4-(trifluoromethyl)benzyl) amino)phen yl)heptanamide | A | B |
| 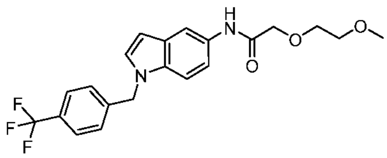 | 2-(2-methoxyethoxy)-N-(1-(4-(trifluoromethyl)benzyl)-1H-indol-5-yl)acetamide | A | A |
| 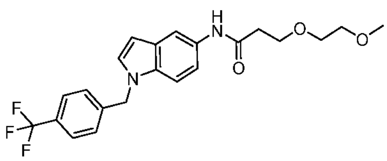 | 3-(2-methoxyethoxy)-N-(1-(4-(trifluoromethyl)benzyl)-1H-indol-5-yl)propanamide | A | A |
| 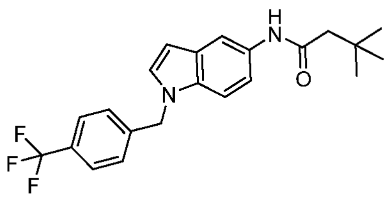 | 3,3-dimethyl-N-(1-(4-(trifluoromethyl) benzyl)-1H-indol-5-yl)butanamide | A | A |

(continued)

| Compound structure | Compound name | Solution stability | Photostability |
|---|---|---|---|
| 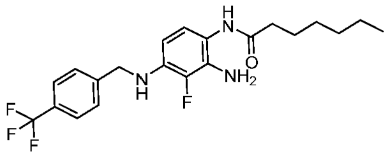 | N-(2-amino-3-fluoro-4-((4-(trifluoromethyl)benzyl)amino)phen yl)heptanamide | A | A |
| 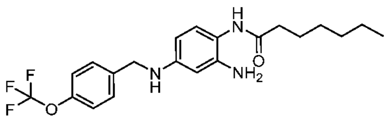 | N-(2-amino-4-((4-(trifluoromethoxy)benzyl)amino)ph enyl)heptanamide | A | B |
| 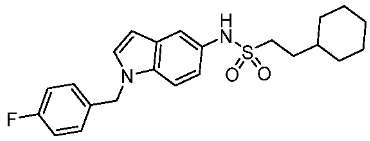 | 2-cyclohexyl-N-(1-(4-fluorobenzyl)-1H-indol-5-yl)ethane-1-sulfonamide | A | A |
| 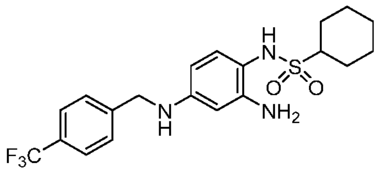 | N-(2-amino-4-((4-(trifluoromethyl)benzyl) amino)phen yl)cyclohexanesulfonamide | C | C |
| 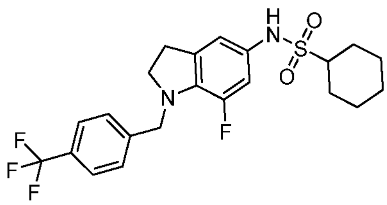 | N-(7-fluoro-1-(4-(trifluoromethyl)benzyl) indolin-5-yl) cyclohexanesulfonamide | B | B |
| 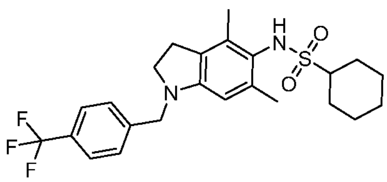 | N-(4,6-dimethyl-1-(4-(trifluoromethyl) benzyl)indolin-5-yl)cyclohexanesulfonamide | A | B |
| 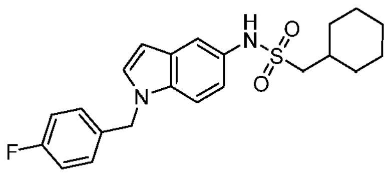 | 1-cyclohexyl-N-(1-(4-fluorobenzyl)-1H-indol-5-yl)methanesulfonamide | A | A |
| 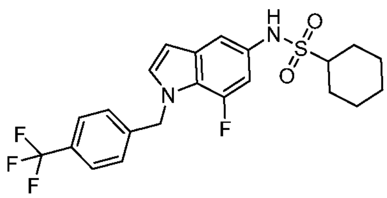 | N-(7-fluoro-1-(4-(trifluoromethyl)benzyl)-1H-indol-5-yl) cyclohexanesulfonamide | A | A |

(continued)

| Compound structure | Compound name | Solution stability | Photostability |
|---|---|---|---|
| 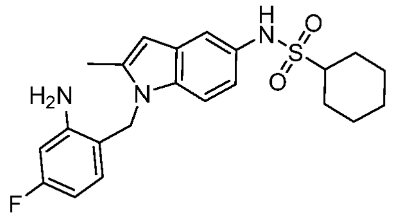 | N-(1-(2-amino-4-fluorobenzyl)-2-methyl-1H-indol-5-yl)cyclohexanesulfonamide | A | B |
| 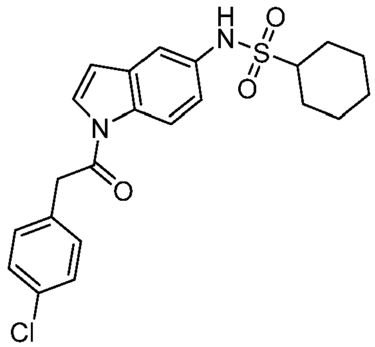 | N-(1-(2-(4-chlorophenyl)acetyl)-1H-indol-5-yl) cyclohexanesulfonamide | A | A |
| 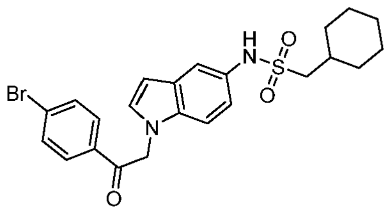 | N-(1-(2-(4-bromophenyl)-2-oxoethyl)-1H-indol-5-yl)-1-cyclohexylmethanesulfonamide | A | B |
| 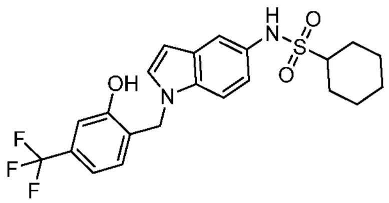 | N-(1-(2-hydroxy-4-(trifluoromethyl)benzyl)-1H-indol-5-yl)cyclohexanesulfonamide | A | A |
| 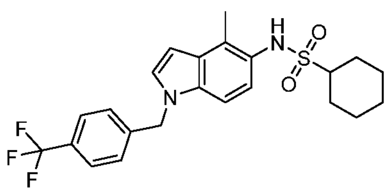 | N-(4-methyl-1-(4-(trifluoromethyl)benzyl)-1H-indol-5-yl)cyclohexanesulfonamide | A | B |
| 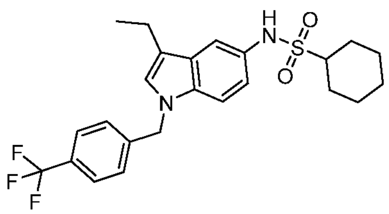 | N-(3-ethyl-1-(4-(trifluoromethyl)benzyl)-1H-indol-5-yl) cyclohexanesulfonamide | A | A |
| 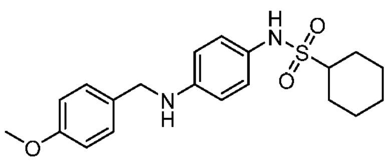 | N-(4-((4-methoxybenzyl)amino)phenyl) cyclohexanesulfonamide | B | C |

(continued)

| Compound structure | Compound name | Solution stability | Photostability |
|---|---|---|---|
| 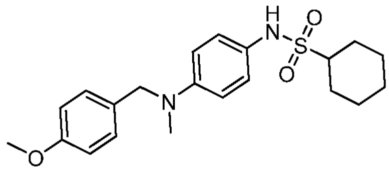 | **N-(4-((4-methoxybenzyl)(methyl)amino) phen yl) cyclohexanesulfonamide** | B | B |
| 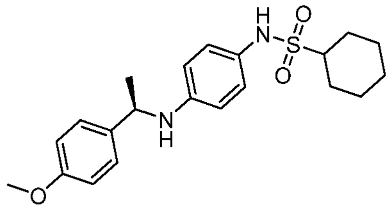 | **(R)-N-(4-((1-(4-methoxyphenyl)ethyl)amino)phenyl ) cyclohexanesulfonamide** | B | C |
| 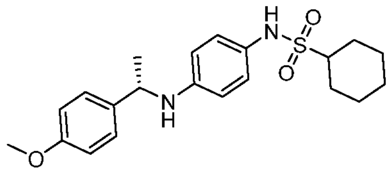 | **(S)-N-(4-((1-(4-methoxyphenyl)ethyl)amino)phenyl ) cyclohexanesulfonamide** | B | C |
| 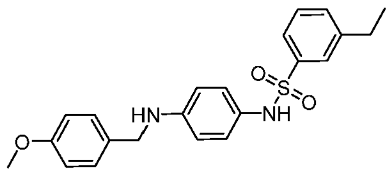 | **3-ethyl-N-(4-((4-methoxybenzyl)amino) phenyl)benze nesulfonamide** | B | C |
| A: degradation lower than 20%; B: 20%< degradation<40%; C: degradation > 40% | | | |

The data in Table 3 show that retigabine is chemically unstable when exposed to visible light; indeed, after 6 hours of exposure to light, more that 40% of the drug is degraded. By contrast, most of the indole, indoline and aryl-1-4diamine derivatives of the invention were degraded by less than 20% after light exposure for the same exposure time. In particular, the two compounds N-(1-(4-(trifluoromethyl)benzyl)-1H-indol-5-yl)heptanamide and N-(2-amino-4-((4-(trifluoromethyl) benzyl)amino)phenyl)heptanamide, in addition to being more potent than retigabine on Kv7.2/3 currents, are also chemically more stable than retigabine. The improved chemical stability shown by the compounds of the invention with respect to retigabine may translate, *in vivo,* in a lower tendency of these molecules to accumulate as blue-colored precipitates in the skin, mucasae, and in the retina of subjects taking these drugs.

## Claims

**1.** A compound of general formula I:

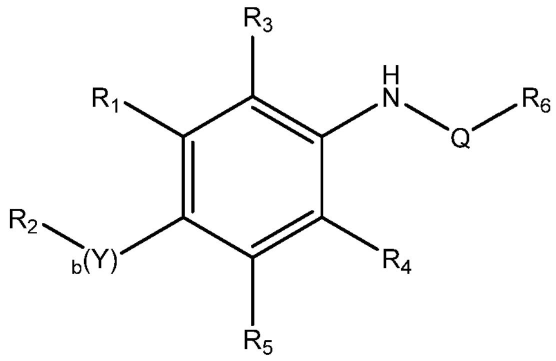

I

or a tautomer, salt, solvate, stereoisomer or isotope thereof, wherein:

Y is selected from: C1-C10 alkylamino, C1-C10 alkyl, C1-C10 alkoxy
$R_2$ is an aromatic ring optionally bearing in one or more positions at least one substituent independently selected from the group consisting of: OH, $NO_2$, halogen, $NH_2$, C1-C3 haloalkyl, C1-C3 haloalkoxy, C1-C6 alkylcarbonyl, acetamidyl, C1-C6 alkyloxy;
$R_1$, $R_3$, $R_4$ and $R_5$ are each independently selected from the group consisting of: H, $NH_2$, OH, halogen, C1-C6 alkyl, C3-C6 cycloalkyl, C1-C6 alkylcarbonyl, C1-C6 alkoxycarbonyl, C1-C6 alkylamino, C2-C6 dialkylamino, C1-C6 alkylaminocarbonyl, C1-C6 alkyloxy, C1-C6 alkylthio, C2-C8 alkenyl, C5-C7 cycloalkenyl, heterocycle, C2-C8 alkynyl and C5-C10 aryl, any of which being optionally substituted by one or more substituents independently selected from: hydroxyl, amine, thiol, carboxyl, carboxylic acid, and halogen;
Q is selected from the group consisting of: C=O, $SO_2$;
wherein:

when Q is C=O $R_6$ is selected from: linear or branched C3-C30 alkyl, linear or branched C5-C30 arylalkyl, C3-C8 cycloalkyl, linear or branched C2-C4 alkyl substituted by C3-C8 cycloalkyl, C2-C30 alkenyl, C2-C30 alkynyl and any of which being optionally substituted with one or more substituents independently selected from: halogen and cycloalkyl; and
when Q is $SO_2$, $R_6$ is C3-C8 cycloalkyl or aryl and is optionally substituted with one or more C1-C6 alkyl.

**2.** The compound according to claim 1 being selected from:

| | COMPOUND STRUCTURE | COMPOUND NAME |
|---|---|---|
| 1. | 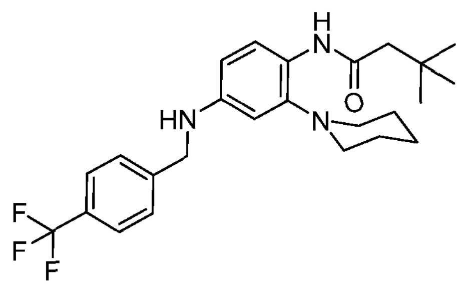 | N-(2-(piperidin-1-yl)-4-((4-(trifluoromethyl)benzyl)amino)p henyl)heptanamide |
| 2. | 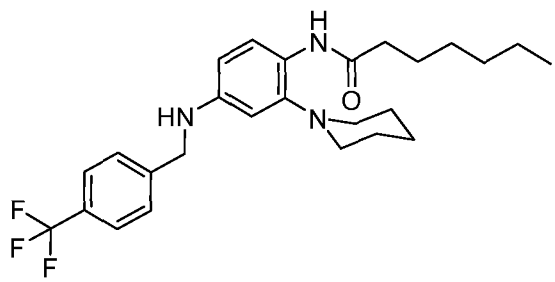 | N-(2-(piperidin-1-yl)-4-((4-(trifluoromethyl)benzyl)amino)p henyl)heptanamide |

(continued)

| | COMPOUND STRUCTURE | COMPOUND NAME |
|---|---|---|
| 3. | 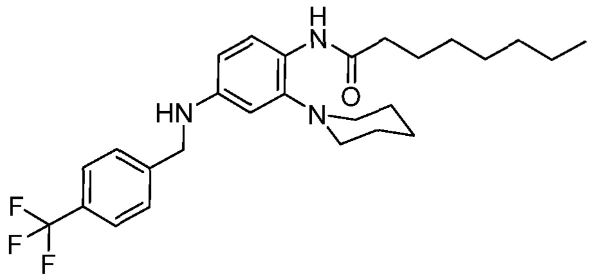 | N-(2-(piperidin-1-yl)-4-((4-(trifluoromethyl)benzyl)amino)p henyl)octanamide |
| 4. | 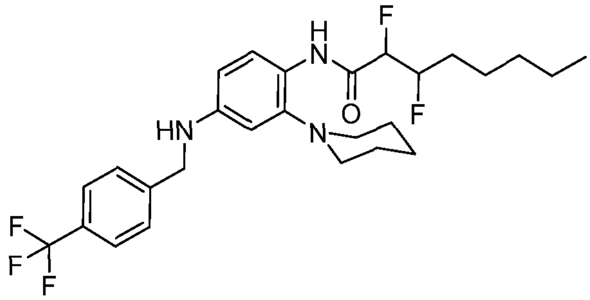 | 2,3-difluoro-N-(2-(piperidin-1-yl)-4-((4-(trifluoromethyl)benzyl)amino)p henyl)octanamide (2R,3 S)-2,3-difluoro-N-(2-(piperidin-1-yl)-4-((4-(trifluoromethyl)benzyl)amino)p henyl)octanamide (2S,3R)-2,3-difluoro-N-(2-(piperidin-1-yl)-4-((4-(trifluoromethyl)benzyl)amino)p henyl)octanamide (2R,3R)-2,3-difluoro-N-(2-(piperidin-1-yl)-4-((4-(trifluoromethyl)benzyl)amino)p henyl)octanamide (2S,3 S)-2,3-difluoro-N-(2-(piperidin-1-yl)-4-((4-(trifluoromethyl)benzyl)amino)p henyl)octanamide |
| 5. | 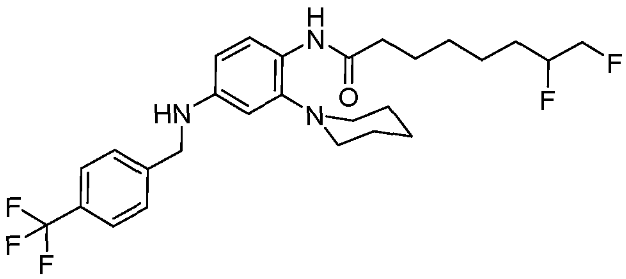 | 7,8-difluoro-N-(2-(piperidin-1-yl)-4-((4-(trifluoromethyl)benzyl)amino)p henyl)octanamide (7R)-7,8-difluoro-N-(2-(piperidin-1-yl)-4-((4-(trifluoromethyl)benzyl)amino)p henyl)octanamide (7S)-7,8-difluoro-N-(2-(piperidin-1-yl)-4-((4-(trifluoromethyl)benzyl)amino)p henyl)octanamide |
| 6. | 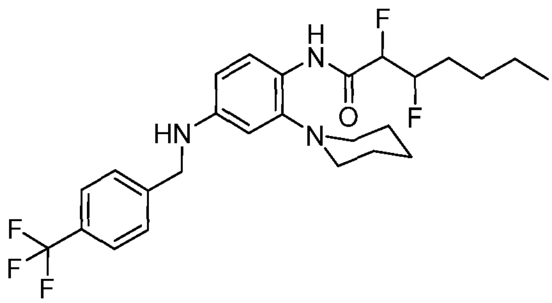 | 2,3-difluoro-N-(2-(piperidin-1-yl)-4-((4-(trifluoromethyl)benzyl)amino)p henyl)heptanamide (2R,3 S)-2,3-difluoro-N-(2-(piperidin-1-yl)-4-((4-(trifluoromethyl)benzyl)amino)p henyl)heptanamide (2S,3R)-2,3-difluoro-N-(2-(piperidin-1-yl)-4-((4-(trifluoromethyl)benzyl) amino)p henyl)heptanamide (2R,3R)-2,3-difluoro-N-(2-(piperidin-1-yl)-4-((4-(trifluoromethyl)benzyl)amino)p henyl)heptanamide (2S,3S)-2,3-difluoro-N-(2-(piperidin-1-yl)-4-((4-(trifluoromethyl)benzyl)amino)p henyl)heptanamide |
| 7. | 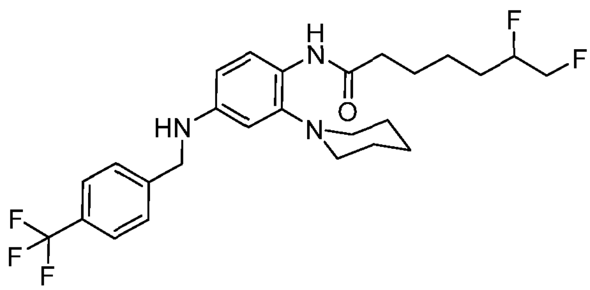 | 6,7-difluoro-N-(2-(piperidin-1-yl)-4-((4-(trifluoromethyl)benzyl)amino)p henyl)heptanamide (6R)-6,7-difluoro-N-(2-(piperidin-1-yl)-4-((4-(trifluoromethyl)benzyl)amino)p henyl)heptanamide (6S)-6,7-difluoro-N-(2-(piperidin-1-yl)-4-((4-(trifluoromethyl)benzyl)amino)p henyl)heptanamide |

(continued)

| | COMPOUND STRUCTURE | COMPOUND NAME |
|---|---|---|
| 8. | 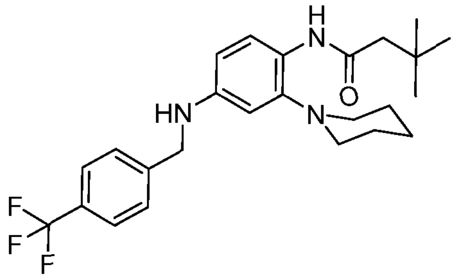 | 3,3-dimethyl-N-(2-(piperidin-1-yl)-4-((4-(trifluoromethyl)benzyl)amino)p henyl)butanamide |
| 9. | 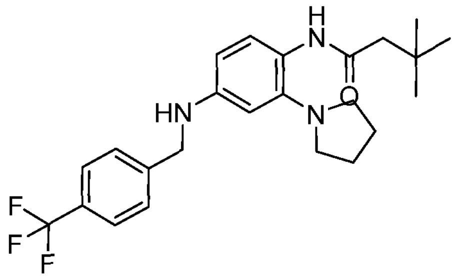 | 3,3-dimethyl-N-(2-(pyrrolidin-1-yl)-4-((4-(trifluoromethyl)benzyl)amino)p henyl)butanamide |
| 10. | 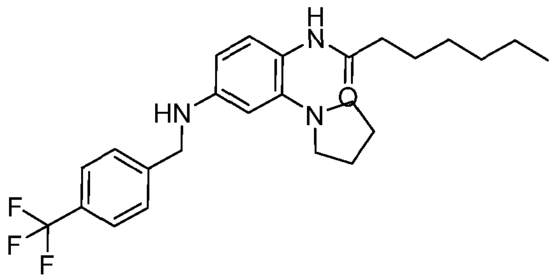 | N-(2-(pyrrolidin-1-yl)-4-((4-(trifluoromethyl)benzyl)amino)p henyl)heptanamide |
| 11. | 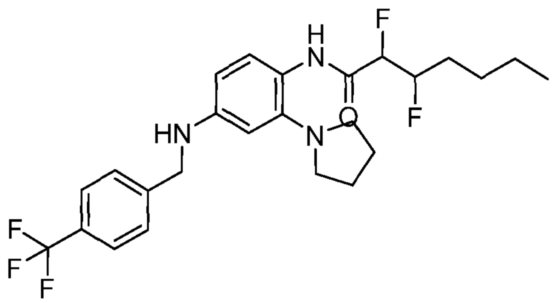 | (2R,3 S)-2,3-difluoro-N-(2-(pyrrolidin-1-yl)-4-((4-(trifluoromethyl)benzyl)amino)p henyl)heptanamide (2S,3R)- 2,3-difluoro-N-(2-(pyrrolidin-1-yl)-4-((4-(trifluoromethyl)benzyl)amino)p henyl)heptanamide (2R,3R)- 2,3-difluoro-N-(2-(pyrrolidin-1-yl)-4-((4-(trifluoromethyl)benzyl) amino)p henyl)heptanamide (2S,3S)- 2,3-difluoro-N-(2-(pyrrolidin-1-yl)-4-((4-(trifluoromethyl)benzyl)amino)p henyl)heptanamide |
| 12. | 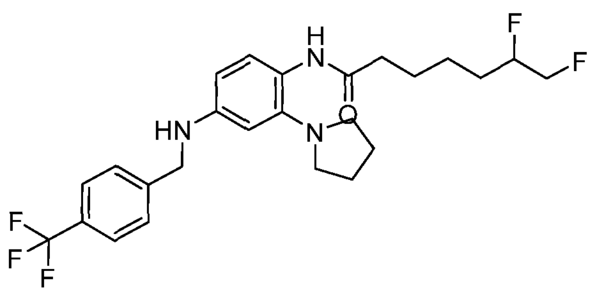 | 6,7-difluoro-N-(2-(pyrrolidin-1-yl)-4-((4-(trifluoromethyl)benzyl)amino)p henyl)heptanamide (6R)-6,7-difluoro-N-(2-(pyrrolidin-1-yl)-4-((4-(trifluoromethyl)benzyl)amino)p henyl)heptanamide (6S)-6,7-difluoro-N-(2-(pyrrolidin-1-yl)-4-((4-(trifluoromethyl)benzyl)amino)p henyl)heptanamide |

(continued)

| | COMPOUND STRUCTURE | COMPOUND NAME |
|---|---|---|
| 13. | 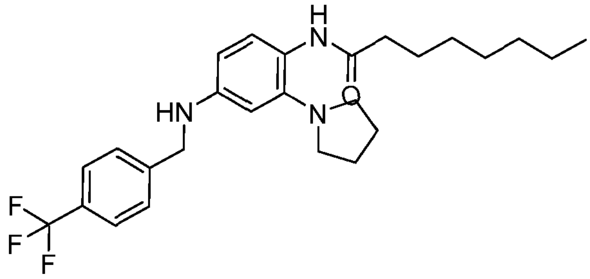 | N-(2-(pyrrolidin-1-yl)-4-((4-(trifluoromethyl)benzyl)amino)p henyl)octanamide |
| 14. | 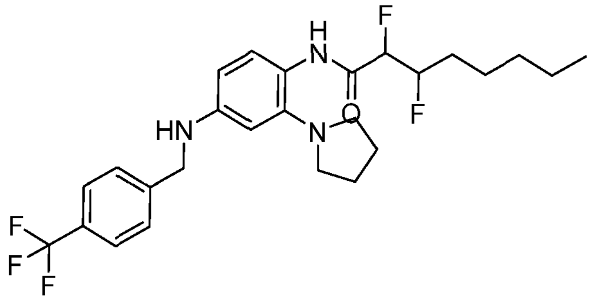 | 2,3-difluoro-N-(2-(pyrrolidin-1-yl)-4-((4-(trifluoromethyl)benzyl)amino)p henyl)octanamide (2R,3 S)-2,3-difluoro-N-(2-(pyrrolidin-1-yl)-4-((4-(trifluoromethyl)benzyl)amino)p henyl)octanamide (2S,3R)-2,3-difluoro-N-(2-(pyrrolidin-1-yl)-4-((4-(trifluoromethyl)benzyl)amino)p henyl)octanamide (2R,3R)-2,3-difluoro-N-(2-(pyrrolidin-1-yl)-4-((4-(trifluoromethyl)benzyl)amino)p henyl)octanamide (2S,3S)-2,3-difluoro-N-(2-(pyrrolidin-1-yl)-4-((4-(trifluoromethyl)benzyl)amino)p henyl)octanamide |
| 15. | 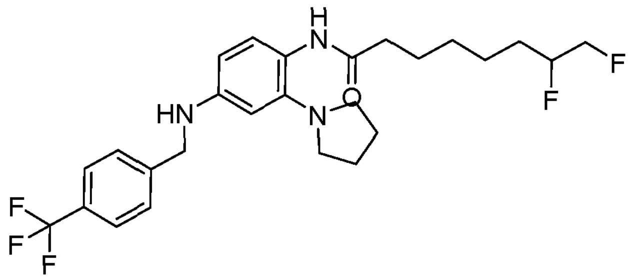 | 7,8-difluoro-N-(2-(pyrrolidin-1-yl)-4-((4-(trifluoromethyl)benzyl)amino)p henyl)octanamide (7R)-7,8-difluoro-N-(2-(pyrrolidin-1-yl)-4-((4-(trifluoromethyl)benzyl)amino)p henyl)octanamide (7S)-7,8-difluoro-N-(2-(pyrrolidin-1-yl)-4-((4-(trifluoromethyl)benzyl)amino)p henyl)octanamide |
| 16. | 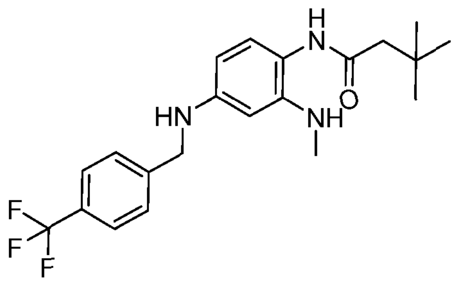 | 3,3-dimethyl-N-(2-(methylamino)-4-((4-(trifluoromethyl)benzyl)amino)p henyl)butanamide |
| 17. | 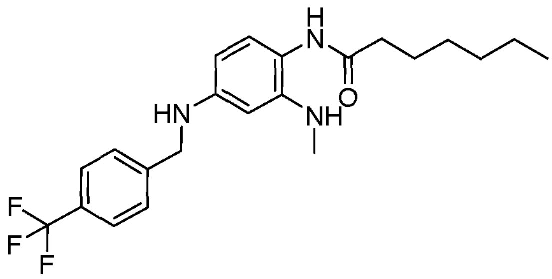 | N-(2-(methylamino)-4-((4-(trifluoromethyl)benzyl)amino)p henyl)heptanamide |

(continued)

| | COMPOUND STRUCTURE | COMPOUND NAME |
|---|---|---|
| 18. | 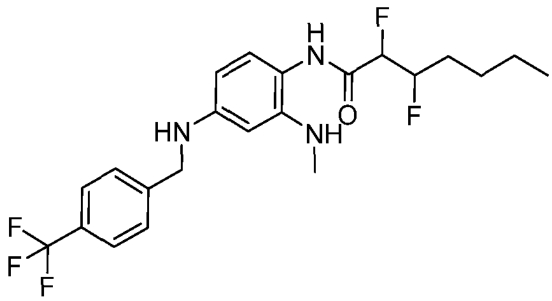 | 2,3-difluoro-N-(2-(methylamino)-4-((4-(trifluoromethyl)benzyl)amino)p henyl)heptanamide (2R, 3S)-2,3-difluoro-N-(2-(methylamino)-4-((4-(trifluoromethyl)benzyl)amino)p henyl)heptanamide (2S, 3R)-2,3-difluoro-N-(2-(methylamino)-4-((4-(trifluoromethyl)benzyl)amino)p henyl)heptanamide (2R, 3R)-2,3-difluoro-N-(2-(methylamino)-4-((4-(trifluoromethyl)benzyl)amino)p henyl)heptanamide (2S, 3S)-2,3-difluoro-N-(2-(methylamino)-4-((4-(trifluoromethyl)benzyl)amino)p henyl)heptanamide |
| 19. | 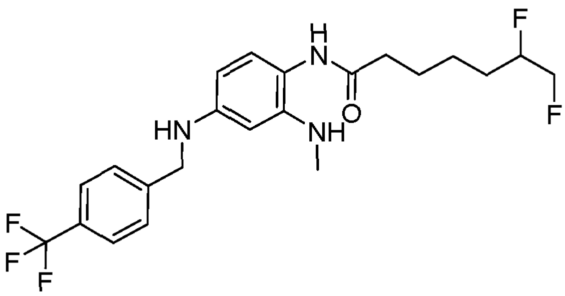 | 6,7-difluoro-N-(2-(methylamino)-4-((4-(trifluoromethyl)benzyl)amino)p henyl)heptanamide (6R)-6,7-difluoro-N-(2-(methylamino)-4-((4-(trifluoromethyl)benzyl)amino)p henyl)heptanamide (6S)-6,7-difluoro-N-(2-(methylamino)-4-((4-(trifluoromethyl)benzyl)amino)p henyl)heptanamide |
| 20. | 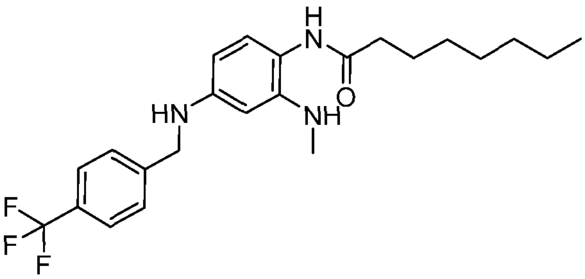 | N-(2-(methylamino)-4-((4-(trifluoromethyl)benzyl)amino)p henyl)octanamide |
| 21. | 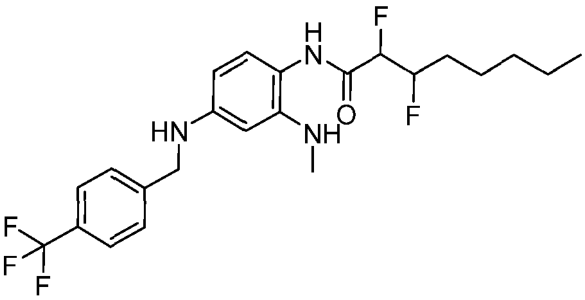 | 2,3-difluoro-N-(2-(methylamino)-4-((4-(trifluoromethyl)benzyl)amino)p henyl)octanamide (2R,3 S)-2,3-difluoro-N-(2-(methylamino)-4-((4-(trifluoromethyl)benzyl)amino)p henyl)octanamide (2S,3R)-2,3-difluoro-N-(2-(methylamino)-4-((4-(trifluoromethyl)benzyl)amino)p henyl)octanamide (2R,3R)-2,3-difluoro-N-(2-(methylamino)-4-((4-(trifluoromethyl)benzyl)amino)p henyl)octanamide (2S,3 S)-2,3-difluoro-N-(2-(methylamino)-4-((4-(trifluoromethyl)benzyl)amino)p henyl)octanamide |

(continued)

| | COMPOUND STRUCTURE | COMPOUND NAME |
|---|---|---|
| 22. | 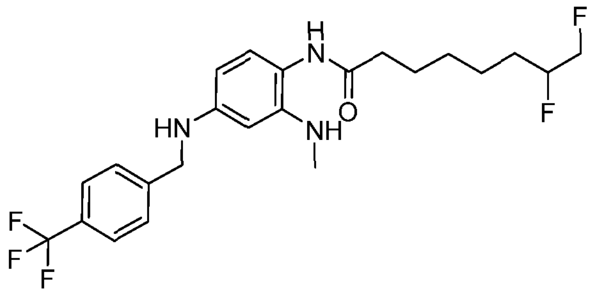 | 7,8-difluoro-N-(2-(methylamino)-4-((4-(trifluoromethyl)benzyl)amino)p henyl)octanamide (7R)-7,8-difluoro-N-(2-(methylamino)-4-((4-(trifluoromethyl)benzyl)amino)p henyl)octanamide (7S)-7,8-difluoro-N-(2-(methylamino)-4-((4-(trifluoromethyl)benzyl)amino)p henyl)octanamide |
| 23. | 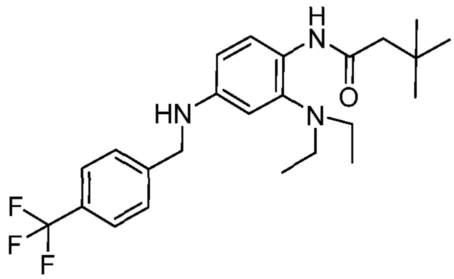 | N-(2-(diethylamino)-4-((4-(trifluoromethyl)benzyl)amino)p henyl)-3,3-dimethylbutanamide |
| 24. | 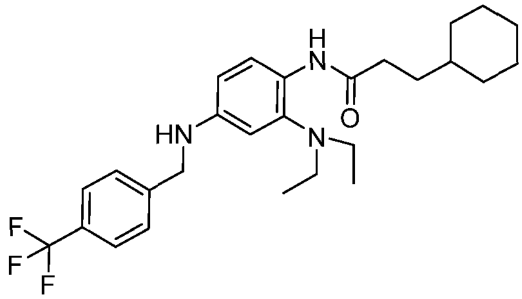 | 3-cyclohexyl-N-(2-(diethylamino)-4-((4-(trifluoromethyl)benzyl)amino)p henyl)propanamide |
| 25. | 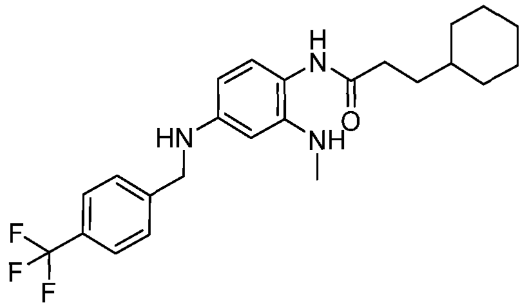 | 3-cyclohexyl-N-(2-(methylamino)-4-((4-(trifluoromethyl)benzyl)amino)p henyl)propanamide |
| 26. | 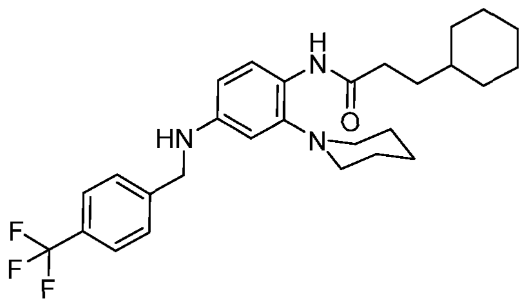 | 3-cyclohexyl-N-(2-(piperidin-1-yl)-4-((4-(trifluoromethyl)benzyl)amino)p henyl)propanamide |

(continued)

| | COMPOUND STRUCTURE | COMPOUND NAME |
|---|---|---|
| 27. | 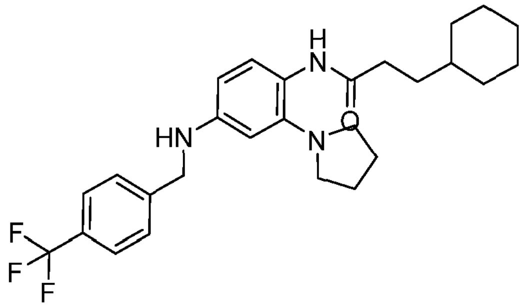 | 3-cyclohexyl-N-(2-(pyrrolidin-1-yl)-4-((4-(trifluoromethyl)benzyl)amino)p henyl)propanamide |
| 28. | 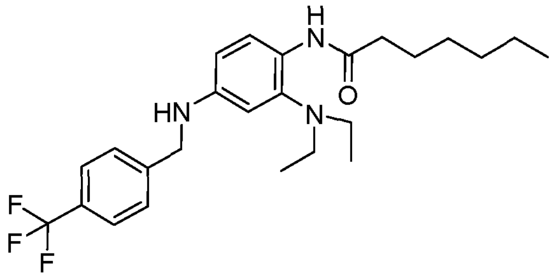 | N-(2-(diethylamino)-4-((4-(trifluoromethyl)benzyl)amino)p henyl)heptanamide |
| 29. | 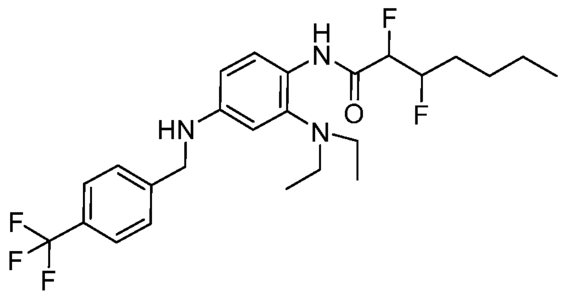 | N-(2-(diethylamino)-4-((4-(trifluoromethyl)benzyl)amino)p henyl)-2,3-difluoroheptanamide (2R,3S)- N-(2-(diethylamino)-4-((4-(trifluoromethyl)benzyl)amino)p henyl)-2,3-difluoroheptanamide (2S,3R)-N-(2-(diethylamino)-4-((4-(trifluoromethyl)benzyl)amino)p henyl)-2,3-difluoroheptanamide (2R,3R)- N-(2-(diethylamino)-4-((4-(trifluoromethyl)benzyl)amino)p henyl)-2,3-difluoroheptanamide (2S,3 S)-N-(2-(diethylamino)-4-((4-(trifluoromethyl)benzyl)amino)p henyl)-2,3-difluoroheptanamide. |
| 30. | 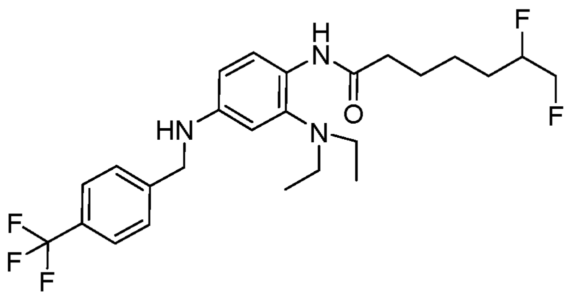 | N-(2-(diethylamino)-4-((4-(trifluoromethyl)benzyl)amino)p henyl)-6,7-difluoroheptanamide (6R)-N-(2-(diethylamino)-4-((4-(trifluoromethyl)benzyl)amino)p henyl)-6,7-difluoroheptanamide (6S)-N-(2-(diethylamino)-4-((4-(trifluoromethyl)benzyl)amino)p henyl)-6,7-difluoroheptanamide |
| 31. | 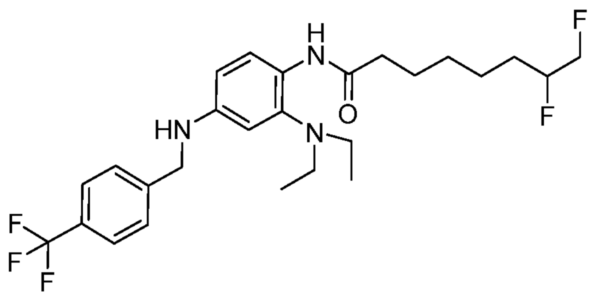 | N-(2-(diethylamino)-4-((4-(trifluoromethyl)benzyl)amino)p henyl)-7,8-difluorooctanamide (7R)-N-(2-(diethylamino)-4-((4-(trifluoromethyl)benzyl)amino)p henyl)-7,8-difluorooctanamide (7S)-N-(2-(diethylamino)-4-((4-(trifluoromethyl)benzyl)amino)p henyl)-7,8-difluorooctanamide |

(continued)

| | COMPOUND STRUCTURE | COMPOUND NAME |
|---|---|---|
| 32. | 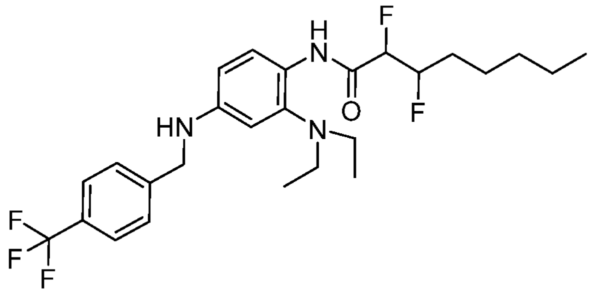 | N-(2-(diethylamino)-4-((4-(trifluoromethyl)benzyl)amino)p henyl)-2,3-difluorooctanamide (2R,3 S)-N-(2-(diethylamino)-4-((4-(trifluoromethyl)benzyl)amino)p henyl)-2,3-difluorooctanamide (2S,3R)-N-(2-(diethylamino)-4-((4-(trifluoromethyl)benzyl)amino)p henyl)-2,3-difluorooctanamide (2R,3R)-N-(2-(diethylamino)-4-((4-(trifluoromethyl)benzyl)amino)p henyl)-2,3-difluorooctanamide (2S,3S)-N-(2-(diethylamino)-4-((4-(trifluoromethyl)benzyl)amino)p henyl)-2,3-difluorooctanamide |
| 33. | 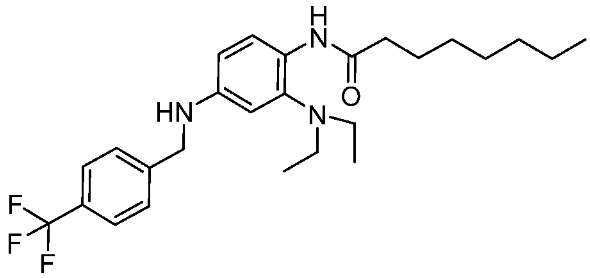 | N-(2-(diethylamino)-4-((4-(trifluoromethyl)benzyl)amino)p henyl)octanamide |
| 34. | 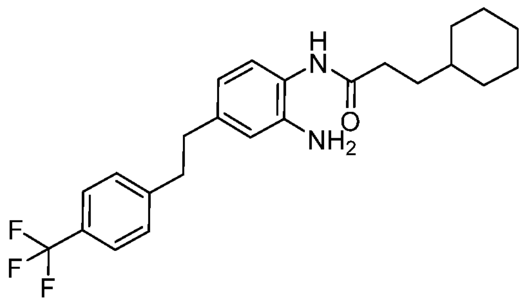 | N-(2-amino-4-(4-(trifluoromethyl)phenethyl)phen yl)-3-cyclohexylpropanamide |
| 35. | 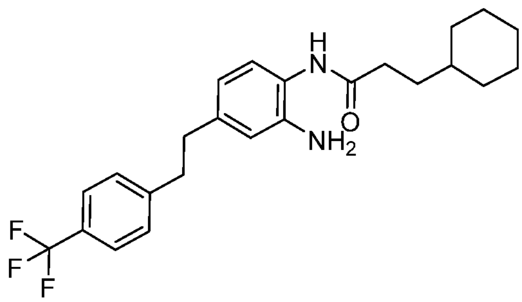 | N-(2-amino-4-(4-(trifluoromethyl)phenethyl)phen yl)-3-cyclohexylpropanamide |
| 36. | 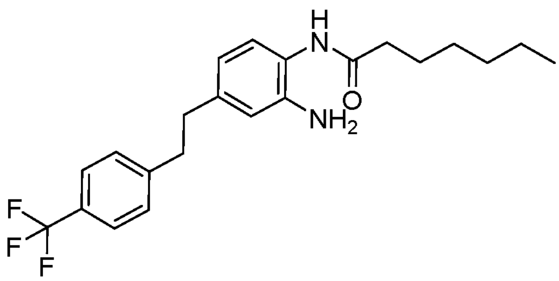 | N-(2-amino-4-(4-(trifluoromethyl)phenethyl)phen yl)heptanamide |

(continued)

| | COMPOUND STRUCTURE | COMPOUND NAME |
|---|---|---|
| 37. | 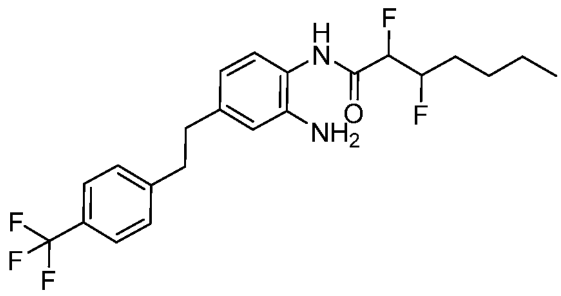 | N-(2-amino-4-(4-(trifluoromethyl)phenethyl)phen yl)-2,3-difluoroheptanamide (2R,3 S)-N-(2-amino-4-(4-(trifluoromethyl)phenethyl)phen yl)-2,3-difluoroheptanamide (2S,3R)-N-(2-amino-4-(4-(trifluoromethyl)phenethyl)phen yl)-2,3-difluoroheptanamide (2R,3R)-N-(2-amino-4-(4-(trifluoromethyl)phenethyl)phen yl)-2,3-difluoroheptanamide (2S,3 S)-N-(2-amino-4-(4-(trifluoromethyl)phenethyl)phen yl)-2,3-difluoroheptanamide |
| 38. | 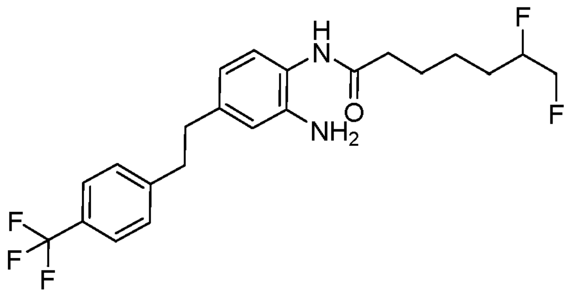 | N-(2-amino-4-(4-(trifluoromethyl)phenethyl)phen yl)-6,7-difluoroheptanamide (6R)-N-(2-amino-4-(4-(trifluoromethyl) phenethyl)phen yl)-6,7-difluoroheptanamide (6S)-N-(2-amino-4-(4-(trifluoromethyl)phenethyl)phen yl)-6,7-difluoroheptanamide |
| 39. | 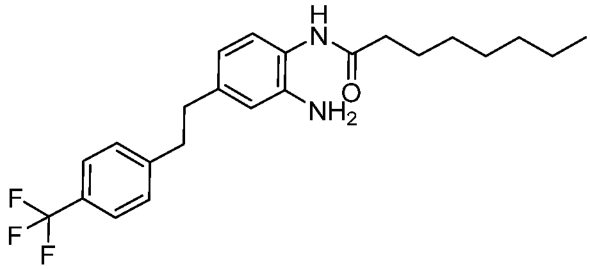 | N-(2-amino-4-(4-(trifluoromethyl)phenethyl)phen yl)octanamide |
| 40. | 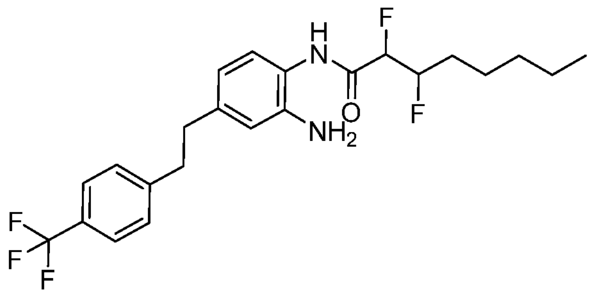 | N-(2-amino-4-(4-(trifluoromethyl)phenethyl)phen yl)-2,3-difluorooctanamide |
| 41. | 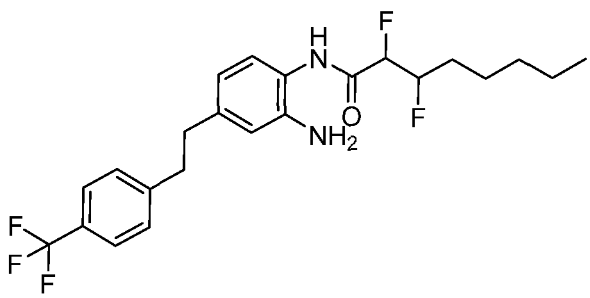 | N-(2-amino-4-(4-(trifluoromethyl)phenethyl)phen yl)-2,3-difluorooctanamide (2R,3 S)-N-(2-amino-4-(4-(trifluoromethyl)phenethyl)phen yl)-2,3-difluorooctanamide (2S,3R)-N-(2-amino-4-(4-(trifluoromethyl)phenethyl)phen yl)-2,3-difluorooctanamide (2R,3R)-N-(2-amino-4-(4-(trifluoromethyl)phenethyl) phen yl)-2,3-difluorooctanamide (2S,3 S)-N-(2-amino-4-(4-(trifluoromethyl)phenethyl)phen yl)-2,3-difluorooctanamide |

(continued)

| | COMPOUND STRUCTURE | COMPOUND NAME |
|---|---|---|
| 42. | 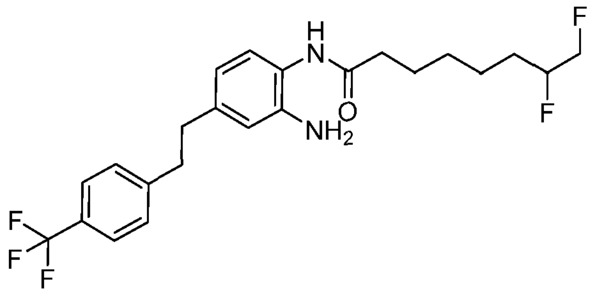 | N-(2-amino-4-(4-(trifluoromethyl)phenethyl)phen yl)-7,8-difluorooctanamide (7R)-N-(2-amino-4-(4-(trifluoromethyl) phenethyl)phen yl)-7,8-difluorooctanamide (7S)-N-(2-amino-4-(4-(trifluoromethyl)phenethyl)phen yl)-7,8-difluorooctanamide |
| 43. | 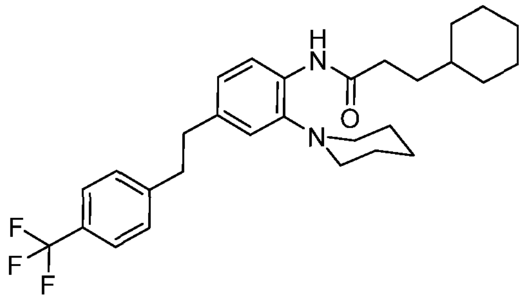 | 3-cyclohexyl-N-(2-(piperidin-1-yl)-4-(4-(trifluoromethyl)phenethyl)phen yl)propanamide |
| 44. | 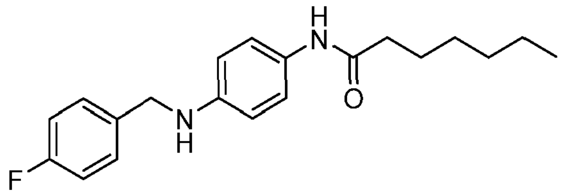 | N-(4-((4-fluorobenzyl)amino)phenyl)hept anamide |
| 45. | 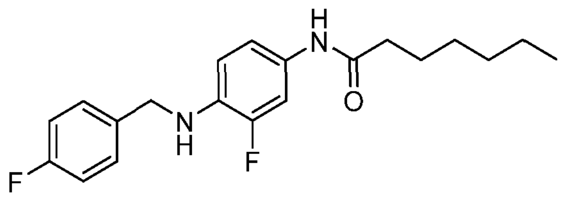 | N-(3-fluoro-4-((4-fluorobenzyl)amino) phenyl)hept anamide |
| 46. | 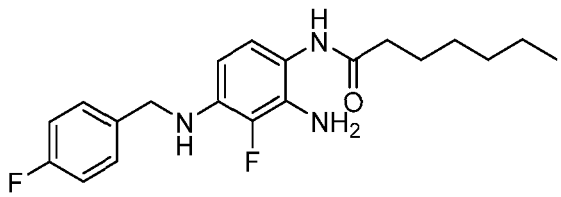 | N-(2-amino-3-fluoro-4-((4-fluorobenzyl) amino)phenyl)hept anamide |
| 47. | 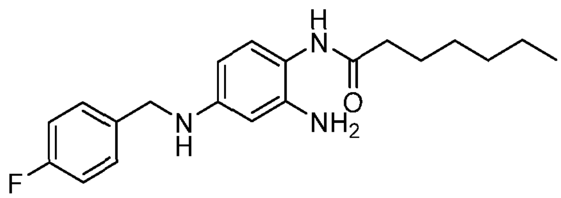 | N-(2-amino-4-((4-fluorobenzyl)amino) phenyl)hept anamide |
| 48. | 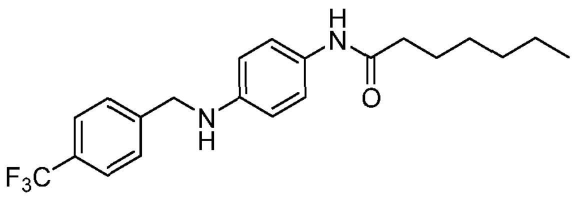 | N-(4-((4-(trifluoromethyl)benzyl)amino)p henyl)heptanamide |

(continued)

| | COMPOUND STRUCTURE | COMPOUND NAME |
|---|---|---|
| 49. | 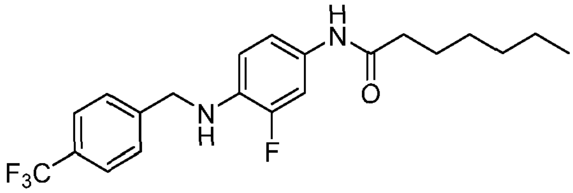 | N-(3-fluoro-4-((4-(trifluoromethyl)benzyl) amino)p henyl)heptanamide |
| 50. | 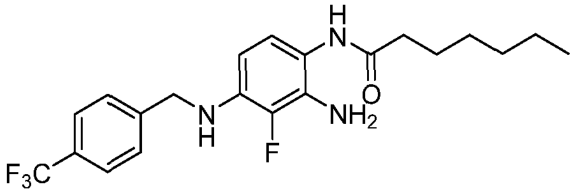 | N-(2-amino-3-fluoro-4-((4-(trifluoromethyl)benzyl)amino)p henyl)heptana-mide |
| 51. | 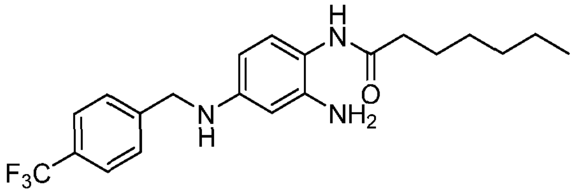 | N-(2-amino-4-((4-(trifluoromethyl)benzyl) amino)p henyl)heptanamide |
| 52. | 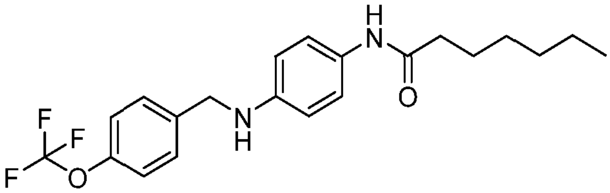 | N-(4-((4-(trifluoromethoxy)benzyl)amino ) phenyl)heptanamide |
| 53. | 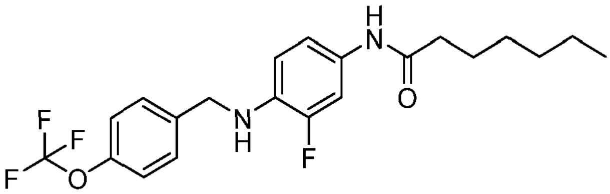 | N-(3-fluoro-4-((4-(trifluoromethoxy)benzyl)amino )phenyl)heptanamide |
| 54. | 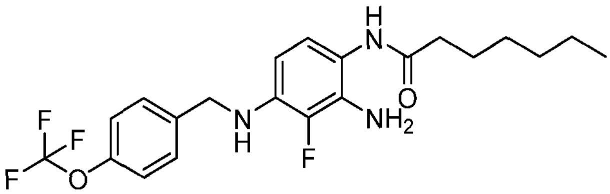 | N-(2-amino-3-fluoro-4-((4-(trifluoro-methoxy)benzyl)amino )phenyl)heptana-mide |
| 55. | 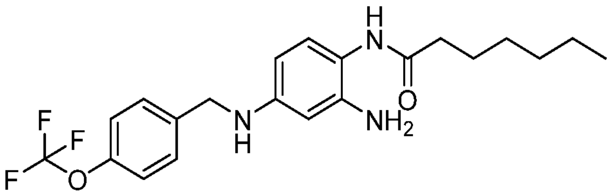 | N-(2-amino-4-((4-(trifluoromethoxy)benzyl)amino )phenyl)heptanamide |
| 56. | 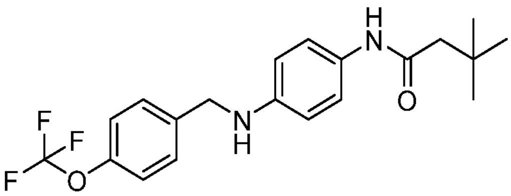 | 3,3-dimethyl-N-(4-((4-(trifluoromethoxy) benzyl)amino )phenyl)butanamide |

(continued)

| | COMPOUND STRUCTURE | COMPOUND NAME |
|---|---|---|
| 57. | 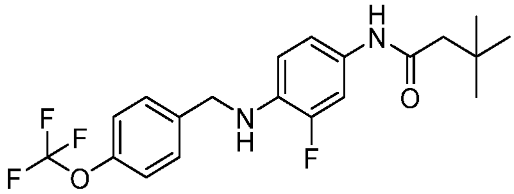 | N-(3-fluoro-4-((4-(trifluoromethoxy)benzyl)amino )phenyl)-3,3-dimethylbutanamide |
| 58. | 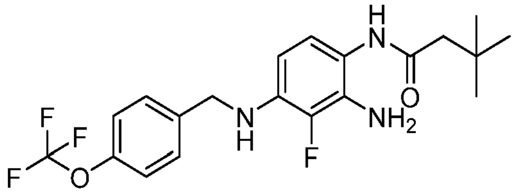 | N-(2-amino-3-fluoro-4-((4-(trifluoromethoxy)benzyl)amino )phenyl)-3,3-dimethylbutanamide |
| 59. | 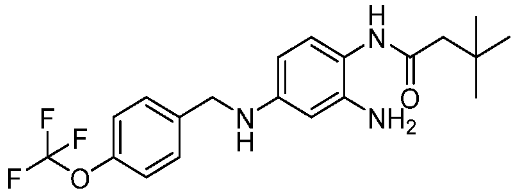 | N-(2-amino-4-((4-(trifluoromethoxy)benzyl)amino )phenyl)-3,3-dimethylbutanamide |
| 60. | 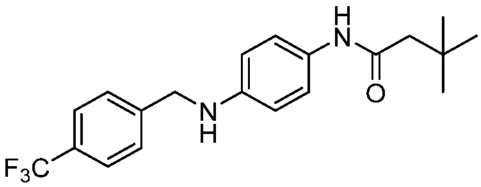 | 3,3-dimethyl-N-(4-((4-(trifluoromethyl) benzyl)amino)p henyl)butanamide |
| 61. | 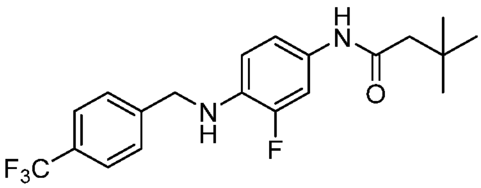 | N-(3-fluoro-4-((4-(trifluoromethyl)benzyl) amino)p henyl)-3,3-dimethylbutanamide |
| 62. | 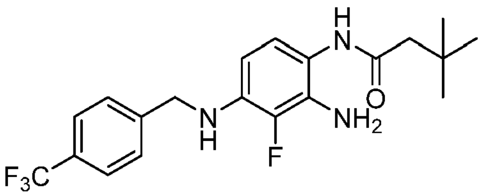 | N-(2-amino-3-fluoro-4-((4-(trifluoromethyl)benzyl)amino)p henyl)-3,3-dimethylbutanamide |
| 63. | 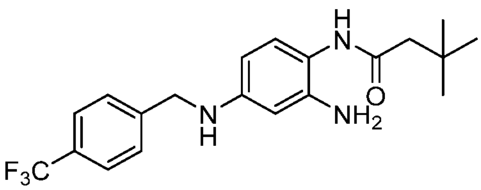 | N-(2-amino-4-((4-(trifluoromethyl)benzyl) amino)p henyl)-3,3-dimethylbutanamide |
| 64. | 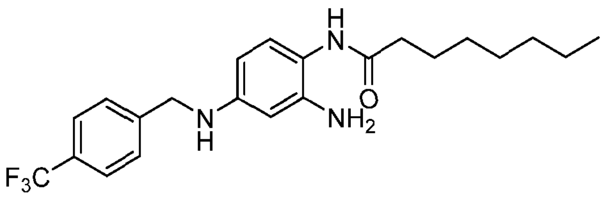 | N-(2-amino-4-((4-(trifluoromethyl)benzyl) amino)p henyl)octanamide |

(continued)

| | COMPOUND STRUCTURE | COMPOUND NAME |
|---|---|---|
| 65. | 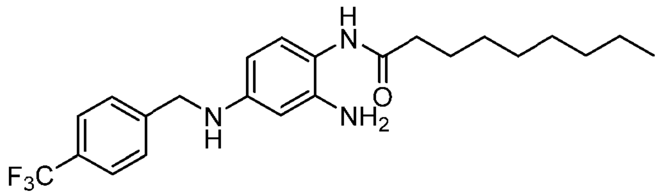 | N-(2-amino-4-((4-(trifluoromethyl)benzyl) amino)p henyl)nonanamide |
| 66. | 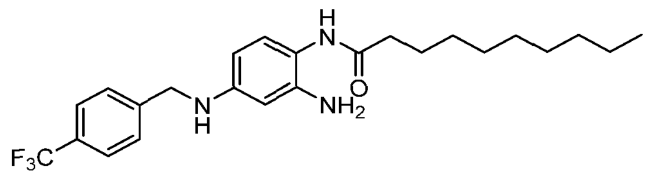 | N-(2-amino-4-((4-(trifluoromethyl)benzyl) amino)p henyl)decanamide |
| 67. | 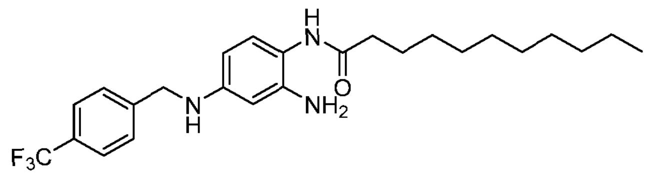 | N-(2-amino-4-((4-(trifluoromethyl)benzyl) amino)p henyl)undecanamide |
| 68. | 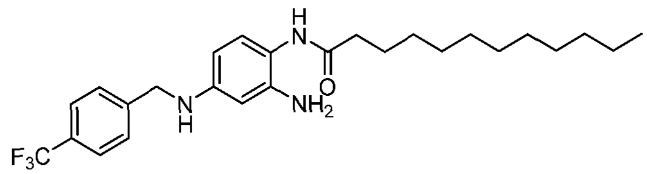 | N-(2-amino-4-((4-(trifluoromethyl)benzyl) amino)p henyl)dodecanamide |
| 69. | 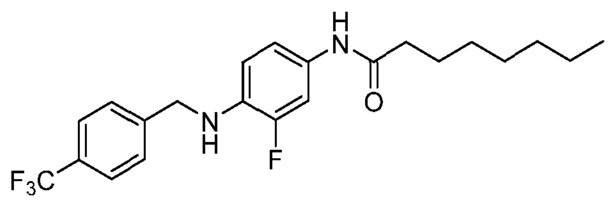 | N-(3-fluoro-4-((4-(trifluoromethyl)benzyl) amino)p henyl)octanamide |
| 70. | 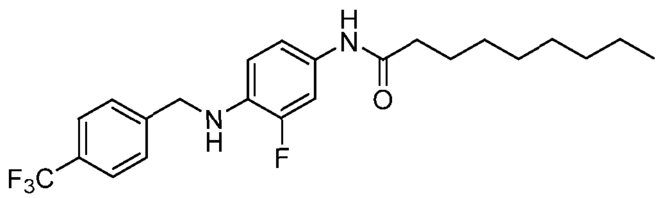 | N-(3-fluoro-4-((4-(trifluoromethyl)benzyl) amino)p henyl)nonanamide |
| 71. | 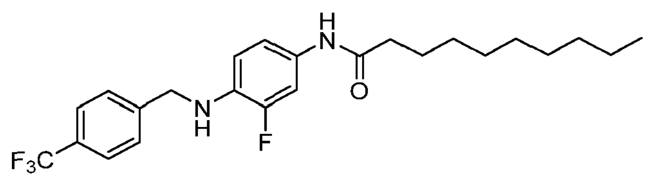 | N-(3-fluoro-4-((4-(trifluoromethyl)benzyl) amino)p henyl)decanamide |
| 72. | 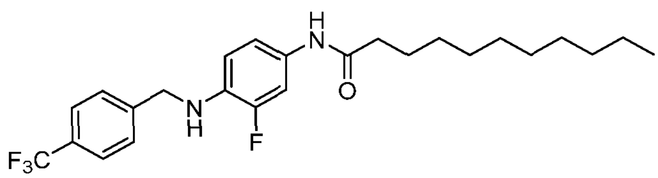 | N-(3-fluoro-4-((4-(trifluoromethyl)benzyl) amino)p henyl)undecanamide |

(continued)

| | COMPOUND STRUCTURE | COMPOUND NAME |
|---|---|---|
| 73. | 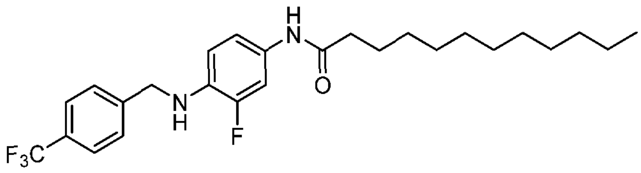 | N-(3-fluoro-4-((4-(trifluoromethyl)benzyl) amino)p henyl)dodecanamide |
| 74. | 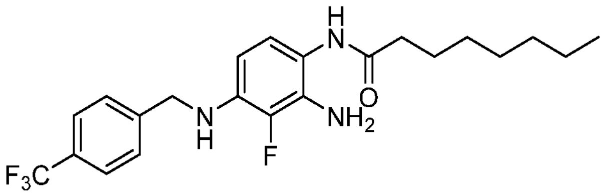 | N-(2-amino-3-fluoro-4-((4-(trifluoromethyl)benzyl)amino)p henyl)octanamide |
| 75. | 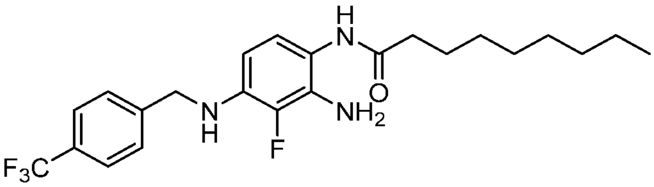 | N-(2-amino-3-fluoro-4-((4-(trifluoromethyl)benzyl)amino)p henyl)nonana-mide |
| 76. | 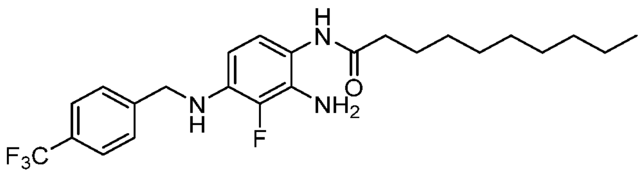 | N-(2-amino-3-fluoro-4-((4-(trifluoromethyl)benzyl)amino)p henyl)decana-mide |
| 77. | 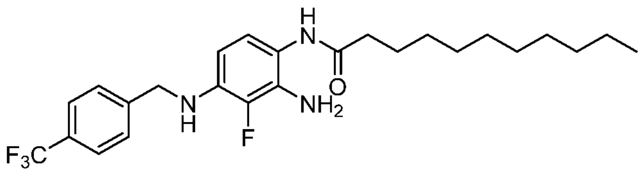 | N-(2-amino-3-fluoro-4-((4-(trifluoromethyl)benzyl)amino)p henyl)undecana-mide |
| 78. | 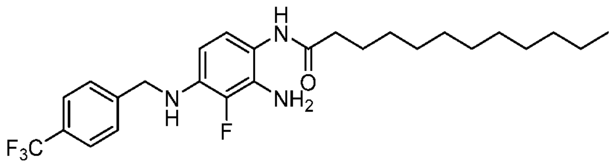 | N-(2-amino-3-fluoro-4-((4-(trifluoromethyl)benzyl)amino)p henyl)dodecana-mide |
| 79. | 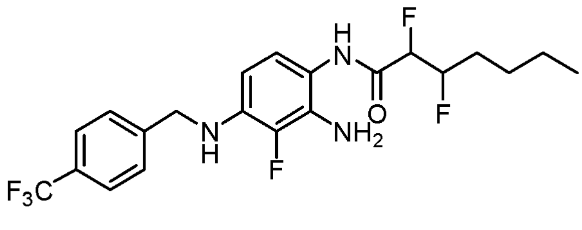 | N-(2-amino-3-fluoro-4-((4-(trifluoromethyl)benzyl)amino)p henyl)-2,3-difluoroheptanamide (2R,3 S)-N-(2-amino-3-fluoro-4-((4-(trifluoromethyl)benzyl)amino)p henyl)-2,3-difluoroheptanamide (2S,3R)-N-(2-amino-3-fluoro-4-((4-(trifluoromethyl)benzyl)amino)p henyl)-2,3-difluoroheptanamide (2R,3R)-N-(2-amino-3-fluoro-4-((4-(trifluoromethyl)benzyl) amino)p henyl)-2,3-difluoroheptanamide (2S,3S)-N-(2-amino-3-fluoro-4-((4-(trifluoromethyl)benzyl)amino)p henyl)-2,3-difluoroheptanamide racemic mixtures thereof |

(continued)

| | COMPOUND STRUCTURE | COMPOUND NAME |
|---|---|---|
| 80. | 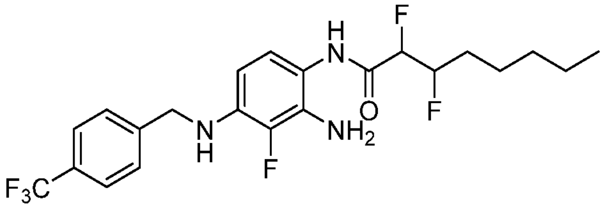 | N-(2-amino-3-fluoro-4-((4-(trifluoromethyl)benzyl)amino)p henyl)-2,3-difluorooctanamide (2R,3 S)-N-(2-amino-3-fluoro-4-((4-(trifluoromethyl)benzyl)amino)p henyl)-2,3-difluorooctanamide (2S,3R)-N-(2-amino-3-fluoro-4-((4-(trifluoromethyl)benzyl)amino)p henyl)-2,3-difluorooctanamide (2R,3R)-N-(2-amino-3-fluoro-4-((4-(trifluoromethyl)benzyl) amino)p henyl)-2,3-difluorooctanamide (2S,3S)-N-(2-amino-3-fluoro-4-((4-(trifluoromethyl)benzyl)amino)p henyl)-2,3-difluorooctanamide racemic mixtures thereof |
| 81. | 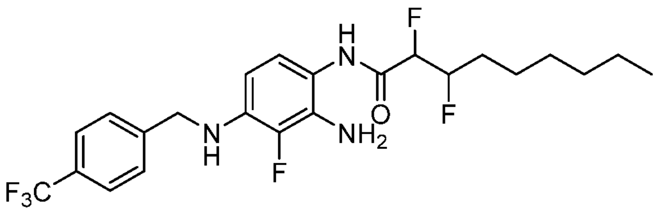 | N-(2-amino-3-fluoro-4-((4-(trifluoromethyl)benzyl)amino)p henyl)-2,3-difluorononanamide (2R,3S)-N-(2-amino-3-fluoro-4-((4-(trifluoromethyl)benzyl)amino)p henyl)-2,3-difluorononanamide (2S,3R)-N-(2-amino-3-fluoro-4-((4-(trifluoromethyl)benzyl)amino)p henyl)-2,3-difluorononanamide (2R,3R)-N-(2-amino-3-fluoro-4-((4-(trifluoromethyl)benzyl) amino)p henyl)-2,3-difluorononanamide (2S,3S)-N-(2-amino-3-fluoro-4-((4-(trifluoromethyl)benzyl)amino)p henyl)-2,3-difluorononanamide racemic mixtures thereof |
| 82. | 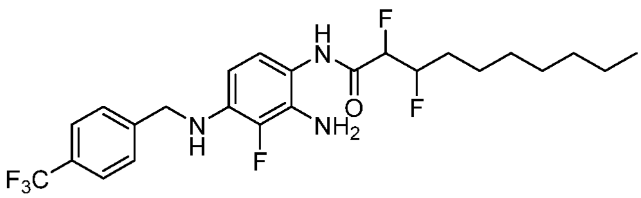 | N-(2-amino-3-fluoro-4-((4-(trifluoromethyl)benzyl)amino)p henyl)-2,3-difluorodecanamide (2R,3 S)-N-(2-amino-3-fluoro-4-((4-(trifluoromethyl)benzyl)amino)p henyl)-2,3-difluorodecanamide (2S,3R)-N-(2-amino-3-fluoro-4-((4-(trifluoromethyl)benzyl)amino)p henyl)-2,3-difluorodecanamide (2R,3R)-N-(2-amino-3-fluoro-4-((4-(trifluoromethyl)benzyl) amino)p henyl)-2,3-difluorodecanamide (2S,3S)-N-(2-amino-3-fluoro-4-((4-(trifluoromethyl)benzyl)amino)p henyl)-2,3-difluorodecanamide racemic mixtures thereof |

(continued)

| | COMPOUND STRUCTURE | COMPOUND NAME |
|---|---|---|
| 83. | 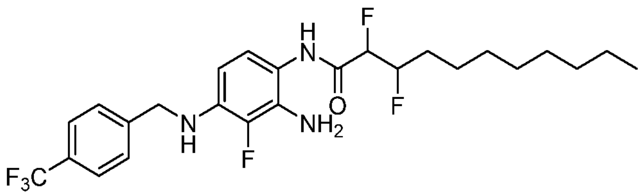 | N-(2-amino-3-fluoro-4-((4-(trifluoromethyl)benzyl)amino)p henyl)-2,3-difluoroundecanamide (2R,3 S)-N-(2-amino-3-fluoro-4-((4-(trifluoromethyl)benzyl)amino)p henyl)-2,3-difluoroundecanamide (2S,3R)-N-(2-amino-3-fluoro-4-((4-(trifluoromethyl)benzyl)amino)p henyl)-2,3-difluoroundecanamide (2R,3R)-N-(2-amino-3-fluoro-4-((4-(trifluoromethyl)benzyl) amino)p henyl)-2,3-difluoroundecanamide (2S,3S)-N-(2-amino-3-fluoro-4-((4-(trifluoromethyl)benzyl)amino)p henyl)-2,3-difluoroundecanamide racemic mixtures thereof |
| 84. | 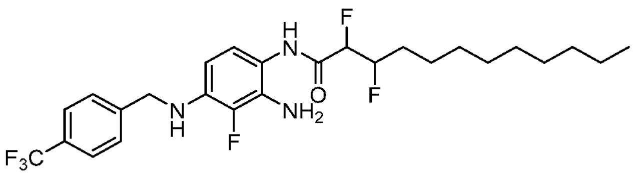 | N-(2-amino-3-fluoro-4-((4-(trifluoromethyl)benzyl)amino)p henyl)-2,3-difluorododecanamide (2R,3 S)-N-(2-amino-3-fluoro-4-((4-(trifluoromethyl)benzyl)amino)p henyl)-2,3-difluorododecanamide (2S,3R)-N-(2-amino-3-fluoro-4-((4-(trifluoromethyl)benzyl)amino)p henyl)-2,3-difluorododecanamide (2R,3R)-N-(2-amino-3-fluoro-4-((4-(trifluoromethyl)benzyl) amino)p henyl)-2,3-difluorododecanamide (2S,3S)-N-(2-amino-3-fluoro-4-((4-(trifluoromethyl)benzyl)amino)p henyl)-2,3-difluorododecanamide racemic mixtures thereof |
| 85. | 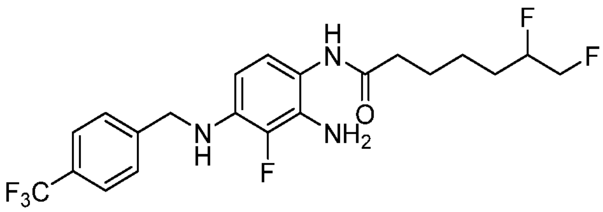 | N-(2-amino-3-fluoro-4-((4-(trifluoromethyl)benzyl)amino)p henyl)-6,7-difluoroheptanamide (6R)-N-(2-amino-3-fluoro-4-((4-(trifluoromethyl)benzyl)amino)p henyl)-6,7-difluoroheptanamide (6S)-N-(2-amino-3-fluoro-4-((4-(trifluoromethyl)benzyl)amino)p henyl)-6,7-difluoroheptanamide racemic mixtures thereof |
| 86. | 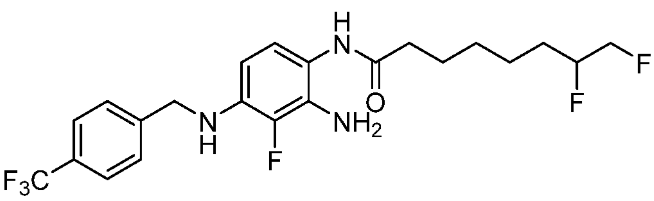 | N-(2-amino-3-fluoro-4-((4-(trifluoromethyl)benzyl)amino)p henyl)-6,7-difluorooctanamide (6R)-N-(2-amino-3-fluoro-4-((4-(trifluoromethyl)benzyl)amino)p henyl)-6,7-difluorooctanamide (6S)-N-(2-amino-3-fluoro-4-((4-(trifluoromethyl)benzyl)amino)p henyl)-6,7-difluorooctanamide racemic mixtures thereof |
| 87. | 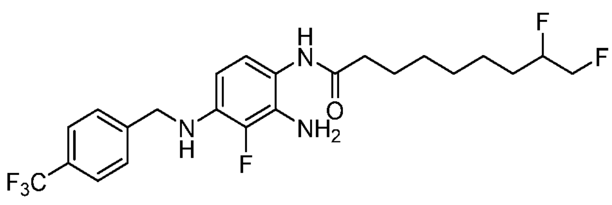 | N-(2-amino-3-fluoro-4-((4-(trifluoromethyl)benzyl)amino)p henyl)-8,9-difluorononanamide (8R)-N-(2-amino-3-fluoro-4-((4-(trifluoromethyl)benzyl)amino)p henyl)-8,9-difluorononanamide (8S)-N-(2-amino-3-fluoro-4-((4-(trifluoromethyl)benzyl)amino)p henyl)-8,9-difluorononanamide racemic mixtures thereof |

(continued)

| | COMPOUND STRUCTURE | COMPOUND NAME |
|---|---|---|
| 88. | 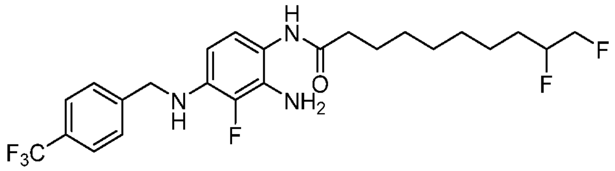 | N-(2-amino-3-fluoro-4-((4-(trifluoromethyl)benzyl)amino)p henyl)-9,10-difluorodecanamide (9R)-N-(2-amino-3-fluoro-4-((4-(trifluoromethyl)benzyl)amino)p henyl)-9,10-difluorodecanamide (9S)-N-(2-amino-3-fluoro-4-((4-(trifluoromethyl)benzyl)amino)p henyl)-9,10-difluorodecanamide racemic mixtures thereof |
| 89. | 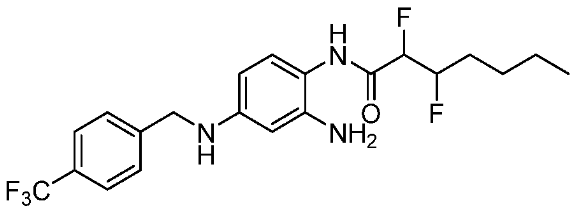 | N-(2-amino-4-((4-(trifluoromethyl)benzyl) amino)p henyl)-2,3-difluoroheptanamide (2R,3 S)-N-(2-amino-4-((4-(trifluoromethyl)benzyl)amino)p henyl)-2,3-difluoroheptanamide (2S,3R)-N-(2-amino-4-((4-(trifluoromethyl)benzyl)amino)p henyl)-2,3-difluoroheptanamide (2R,3R)-N-(2-amino-4-((4-(trifluoromethyl)benzyl)amino)p henyl)-2,3-difluoroheptanamide (2S,3 S)-N-(2-amino-4-((4-(trifluoromethyl)benzyl)amino)p henyl)-2,3-difluoroheptanamide racemic mixtures thereof |
| 90. | 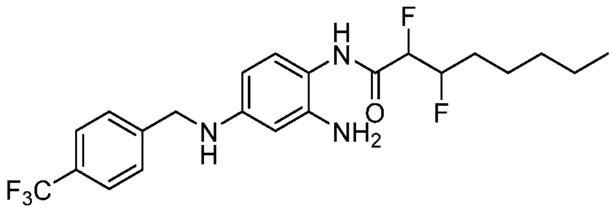 | N-(2-amino-4-((4-(trifluoromethyl)benzyl) amino)p henyl)-2,3-difluorooctanamide (2R,3 S)-N-(2-amino-4-((4-(trifluoromethyl)benzyl)amino)p henyl)-2,3-difluorooctanamide (2S,3R)-N-(2-amino-4-((4-(trifluoromethyl)benzyl)amino)p henyl)-2,3-difluorooctanamide (2R,3R)-N-(2-amino-4-((4-(trifluoromethyl)benzyl)amino)p henyl)-2,3-difluorooctanamide (2S,3 S)-N-(2-amino-4-((4-(trifluoromethyl)benzyl)amino)p henyl)-2,3-difluorooctanamide racemic mixtures thereof |
| 91. | 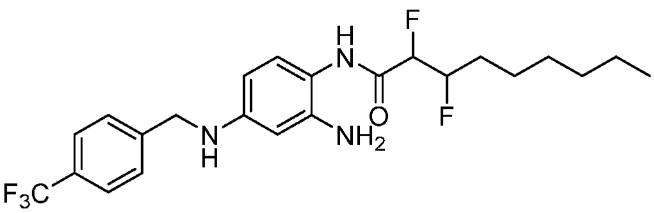 | N-(2-amino-4-((4-(trifluoromethyl)benzyl) amino)p henyl)-2,3-difluorononanamide (2R,3 S)-N-(2-amino-4-((4-(trifluoromethyl)benzyl)amino)p henyl)-2,3-difluorononanamide (2S,3R)-N-(2-amino-4-((4-(trifluoromethyl)benzyl)amino)p henyl)-2,3-difluorononanamide (2R,3R)-N-(2-amino-4-((4-(trifluoromethyl)benzyl)amino)p henyl)-2,3-difluorononanamide (2S,3 S)-N-(2-amino-4-((4-(trifluoromethyl)benzyl)amino)p henyl)-2,3-difluorononanamide racemic mixtures thereof |

(continued)

| | COMPOUND STRUCTURE | COMPOUND NAME |
|---|---|---|
| 92. | 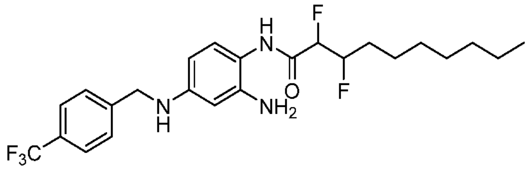 | N-(2-amino-4-((4-(trifluoromethyl)benzyl) amino)p henyl)-2,3-difluorodecanamide (2R,3 S)- N-(2-amino-4-((4-(trifluoromethyl)benzyl)amino)p henyl)-2,3-difluorodecanamide (2S,3R)- N-(2-amino-4-((4-(trifluoromethyl)benzyl)amino)p henyl)-2,3-difluorodecanamide (2R,3R)- N-(2-amino-4-((4-(trifluoromethyl)benzyl) amino)p henyl)-2,3-difluorodecanamide (2S,3 S)- N-(2-amino-4-((4-(trifluoromethyl)benzyl)amino)p henyl)-2,3-difluorodecanamide racemic mixtures thereof |
| 93. | 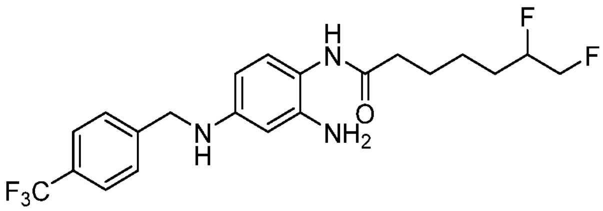 | N-(2-amino-4-((4-(trifluoromethyl)benzyl) amino)p henyl)-6,7-difluoroheptanamide (6R)-N-(2-amino-4-((4-(trifluoromethyl) benzyl)amino)p henyl)-6,7-difluoroheptanamide (6S)-N-(2-amino-4-((4-(trifluoromethyl)benzyl)amino)p henyl)-6,7-difluoroheptanamide racemic mixtures thereof |
| 94. | 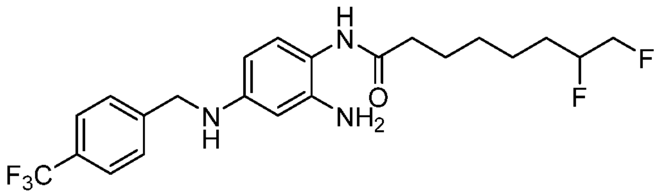 | N-(2-amino-4-((4-(trifluoromethyl)benzyl) amino)p henyl)-7,8-difluorooctanamide (7R)-N-(2-amino-4-((4-(trifluoromethyl) benzyl)amino)p henyl)-7,8-difluorooctanamide (7S)-N-(2-amino-4-((4-(trifluoromethyl)benzyl)amino)p henyl)-7,8-difluorooctanamide racemic mixtures thereof |
| 95. | 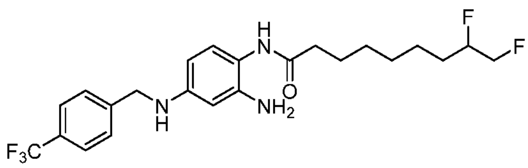 | N-(2-amino-3-fluoro-4-((4-(trifluoromethyl)benzyl)amino)p henyl)-6,7-difluorononanamide (6R)-N-(2-amino-3-fluoro-4-((4-(trifluoromethyl)benzyl)amino)p henyl)-6,7-difluorononanamide (6S)-N-(2-amino-3-fluoro-4-((4-(trifluoromethyl)benzyl)amino)p henyl)-6,7-difluorononanamide racemic mixtures thereof |
| 96. | 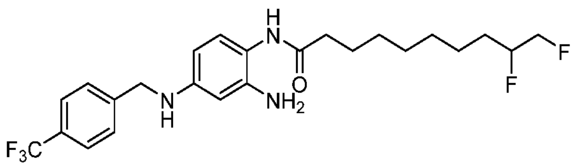 | N-(2-amino-3-fluoro-4-((4-(trifluoromethyl)benzyl)amino)p henyl)-6,7-difluorododecanamide (6R)-N-(2-amino-3-fluoro-4-((4-(trifluoromethyl)benzyl)amino)p henyl)-6,7-difluorododecanamide (6S)-N-(2-amino-3-fluoro-4-((4-(trifluoromethyl)benzyl)amino)p henyl)-6,7-difluorododecanamide racemic mixtures thereof |
| 97. | 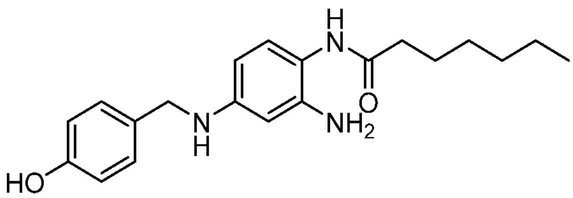 | N-(2-amino-4-((4-hydroxybenzyl)amino) phenyl)he ptanamide |

(continued)

| | COMPOUND STRUCTURE | COMPOUND NAME |
|---|---|---|
| 98. | 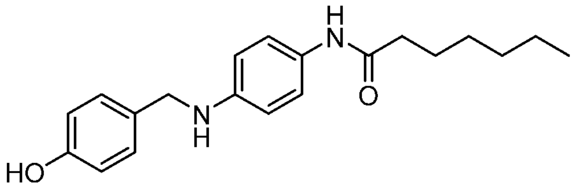 | N-(4-((4-hydroxybenzyl)amino)phenyl)he ptanamide |
| 99. | 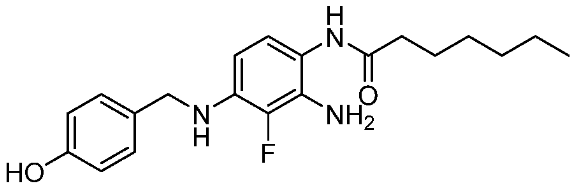 | N-(2-amino-3-fluoro-4-((4-hydroxybenzyl)amino)phenyl)he ptanamide |
| 100. | 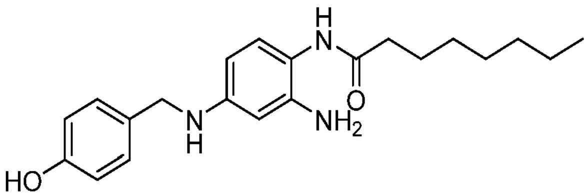 | N-(2-amino-4-((4-hydroxybenzyl)amino) phenyl)oc tanamide |
| 101. | 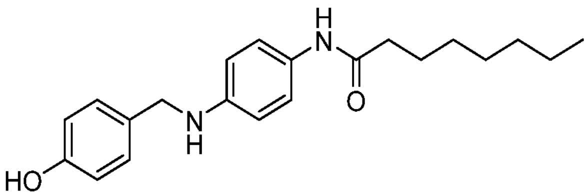 | N-(4-((4-hydroxybenzyl)amino)phenyl)oc tanamide |
| 102. | 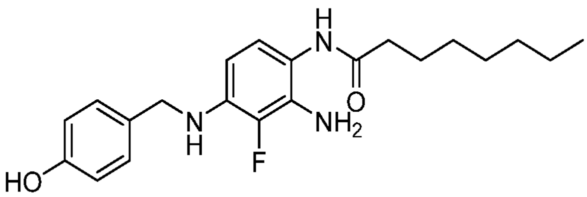 | N-(2-amino-3-fluoro-4-((4-hydroxybenzyl)amino)phenyl)oc tanamide |
| 103. | 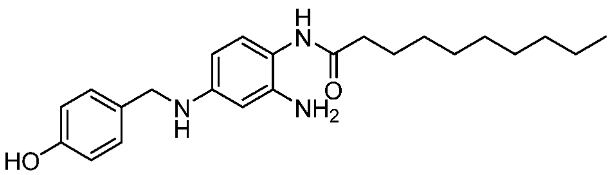 | N-(2-amino-4-((4-hydroxybenzyl)amino) phenyl)de canamide |
| 104. | 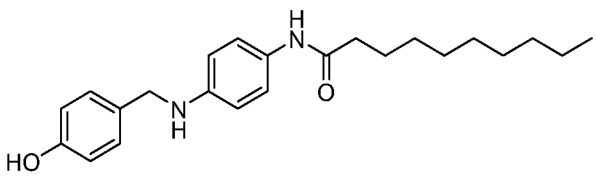 | N-(4-((4-hydroxybenzyl)amino)phenyl)de canamide |
| 105. | 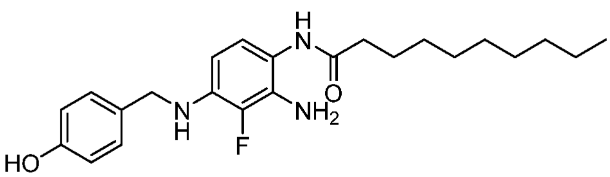 | N-(2-amino-3-fluoro-4-((4-hydroxybenzyl)amino)phenyl)de canamide |

(continued)

| | COMPOUND STRUCTURE | COMPOUND NAME |
|---|---|---|
| 106. | 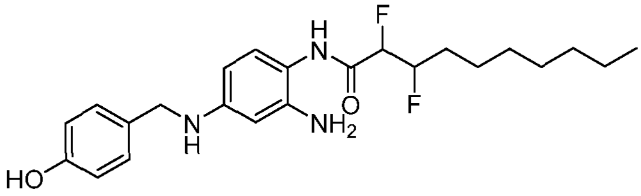 | N-(2-amino-4-((4-hydroxybenzyl)amino) phenyl)-2,3-difluorodecanamide (2R,3 S)-N-(2-amino-4-((4-hydroxybenzyl)amino)phenyl)-2,3-difluorodecanamide (2S,3R)-N-(2-amino-4-((4-hydroxybenzyl)amino)phenyl)-2,3-difluorodecanamide (2R,3R)-N-(2-amino-4-((4-hydroxybenzyl)amino)phenyl)-2,3-difluorodecanamide (2S,3 S)-N-(2-amino-4-((4-hydroxybenzyl)amino)phenyl)-2,3-difluorodecanamide racemic mixtures thereof |
| 107. | 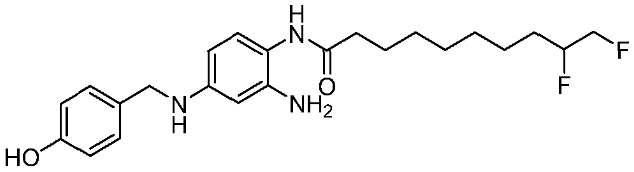 | N-(2-amino-4-((4-hydroxybenzyl)amino) phenyl)-9,10-difluorodecanamide (9R)-N-(2-amino-4-((4-hydroxybenzyl) amino)phenyl)-2,3-difluorodecanamide (9S)-N-(2-amino-4-((4-hydroxybenzyl) amino)phenyl)-2,3-difluorodecanamide racemic mixtures thereof |
| 108. | 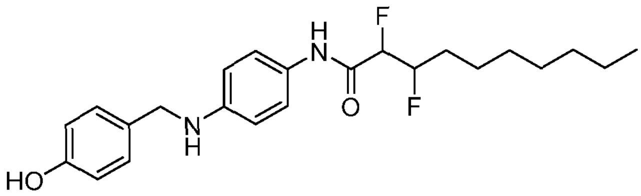 | 2,3-difluoro-N-(4-((4-hydroxybenzyl)amino)phenyl)de ca decanamide (2R,3S)-2,3-difluoro-N-(4-((4-hydroxybenzyl)amino)phenyl)de ca decanamide (2S,3R)-2,3-difluoro-N-(4-((4-hydroxybenzyl)amino)phenyl)de canamide (2R,3R)-2,3-difluoro-N-(4-((4-hydroxybenzyl)amino)phenyl)de ca decanamide (2S,3S)-2,3-difluoro-N-(4-((4-hydroxybenzyl)amino)phenyl)de canamide racemic mixtures thereof |
| 109. | 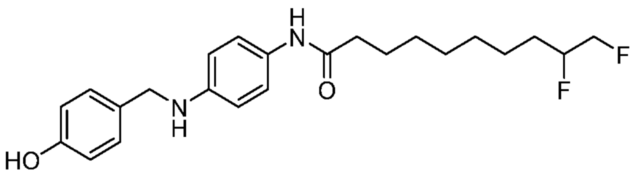 | 9,10-difluoro-N-(4-((4-hydroxybenzyl) amino)phenyl)de canamide (9R)-9,10-difluoro-N-(4-((4-hydroxybenzyl)amino) phenyl)de canamide (9S)-9,10-difluoro-N-(4-((4-hydroxybenzyl)amino)phenyl)de ca namide racemic mixtures thereof |
| 110. | 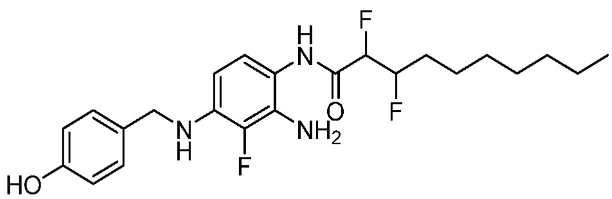 | N-(2-amino-3-fluoro-4-((4-hydroxybenzyl)amino)phenyl)-2,3-difluorodecanamide (2R,3 S)-N-(2-amino-3-fluoro-4-((4-hydroxybenzyl)amino)phenyl)-2,3-difluorodecanamide (2S,3R)-N-(2-amino-3-fluoro-4-((4-hydroxybenzyl)amino)phenyl)-2,3-difluorodecanamide (2R,3R)-N-(2-amino-3-fluoro-4-((4-hydroxybenzyl)amino)phenyl)-2,3-difluorodecanamide (2S,3S)-N-(2-amino-3-fluoro-4-((4-hydroxybenzyl)amino)phenyl)-2,3-difluorodecanamide racemic mixtures thereof |

(continued)

| | COMPOUND STRUCTURE | COMPOUND NAME |
|---|---|---|
| 111. | 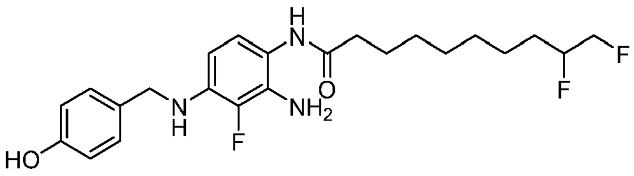 | N-(2-amino-3-fluoro-4-((4-hydroxybenzyl)amino)phenyl)-9,10-difluorodecanamide (9R)-N-(2-amino-3-fluoro-4-((4-hydroxybenzyl)amino)phenyl)-9,10-difluorodecanamide (9S)-N-(2-amino-3-fluoro-4-((4-hydroxybenzyl)amino)phenyl)-9,10-difluorodecanamide racemic mixtures thereof |
| 112. | 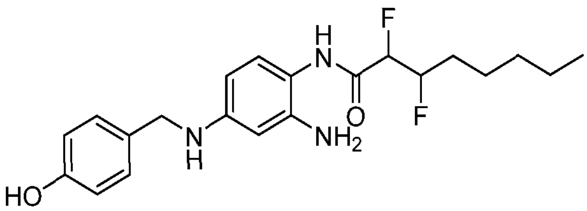 | N-(2-amino-4-((4-hydroxybenzyl)amino) phenyl)-2,3-difluorooctanamide (2R,3 S)-N-(2-amino-4-((4-hydroxybenzyl)amino)phenyl)-2,3-difluorooctanamide (2S,3R)-N-(2-amino-4-((4-hydroxybenzyl)amino)phenyl)-2,3-difluorooctanamide (2R,3R)-N-(2-amino-4-((4-hydroxybenzyl)amino)phenyl)-2,3-difluorooctanamide (2S,3 S)-N-(2-amino-4-((4-hydroxybenzyl)amino)phenyl)-2,3-difluorooctanamide racemic mixtures thereof |
| 113. | 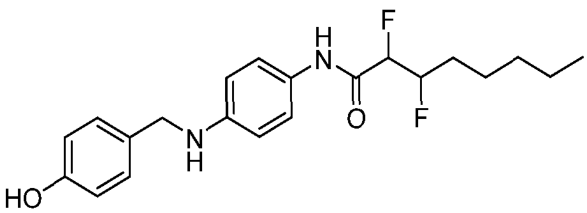 | 2,3-difluoro-N-(4-((4-hydroxybenzyl)amino)phenyl)oc tanamide (2R,3S)-2,3-difluoro-N-(4-((4-hydroxybenzyl)amino) phenyl)oc tanamide (2S,3R)-2,3-difluoro-N-(4-((4-hydroxybenzyl)amino)phenyl)oc (2R,3R)-2,3-difluoro-N-(4-((4-hydroxybenzyl)amino)phenyl)oc tanamide (2S,3S)-2,3-difluoro-N-(4-((4-hydroxybenzyl)amino)phenyl)oc tanamide racemic mixtures thereof |
| 114. | 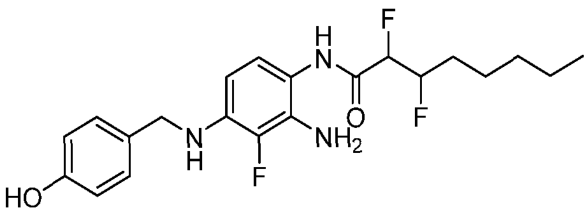 | N-(2-amino-3-fluoro-4-((4-hydroxybenzyl)amino)phenyl)-2,3-difluorooctanamide (2R,3S)-N-(2-amino-3-fluoro-4-((4-hydroxybenzyl)amino)phenyl)-2,3-difluorooctanamide (2S,3R)-N-(2-amino-3-fluoro-4-((4-hydroxybenzyl)amino)phenyl)-2,3-difluorooctanamide (2R,3R)-N-(2-amino-3-fluoro-4-((4-hydroxybenzyl)amino)phenyl)-2,3-difluorooctanamide (2S,3S)-N-(2-amino-3-fluoro-4-((4-hydroxybenzyl)amino)phenyl)-2,3-difluorooctanamide racemic mixtures thereof |
| 115. | 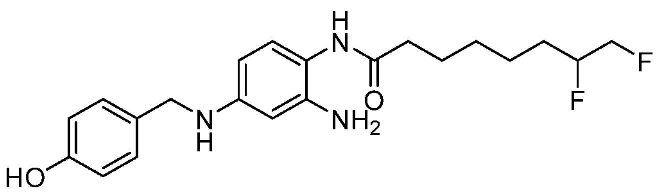 | N-(2-amino-4-((4-hydroxybenzyl)amino) phenyl)-7,8-difluorooctanamide (7S)-N-(2-amino-4-((4-hydroxybenzyl) amino)phenyl)-7,8-difluorooctanamide (7R)-N-(2-amino-4-((4-hydroxybenzyl) amino)phenyl)-7,8-difluorooctanamide racemic mixtures thereof |

(continued)

| | COMPOUND STRUCTURE | COMPOUND NAME |
|---|---|---|
| 116. | 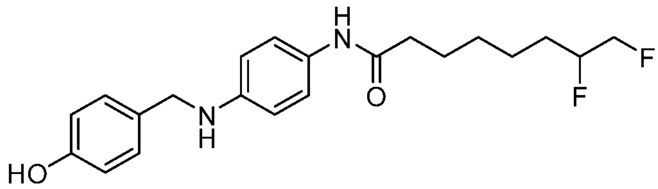 | 7,8-difluoro-N-(4-((4-hydroxybenzyl)amino)phenyl)oc tanamide (7R)-7,8-difluoro-N-(4-((4-hydroxybenzyl)amino)phenyl)oc tanamide (7S)-7,8-difluoro-N-(4-((4-hydroxybenzyl)amino)phenyl)oc tanamide racemic mixtures thereof |
| 117. | 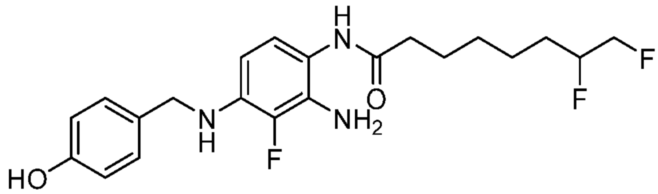 | N-(2-amino-3-fluoro-4-((4-hydroxybenzyl)amino)phenyl)-7,8-difluorooctanamide (7R)-N-(2-amino-3-fluoro-4-((4-hydroxybenzyl)amino)phenyl)-7,8-difluorooctanamide (7S)-N-(2-amino-3-fluoro-4-((4-hydroxybenzyl)amino)phenyl)-7,8-difluorooctanamide racemic mixtures thereof |
| 118. | 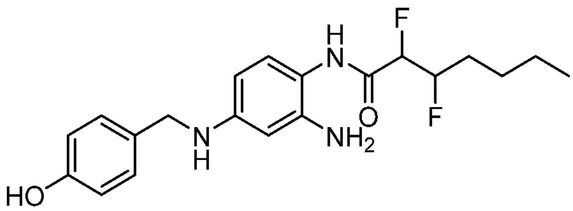 | N-(2-amino-4-((4-hydroxybenzyl)amino) phenyl)-2,3-difluoroheptanamide (2R,3 S)-N-(2-amino-4-((4-hydroxybenzyl)amino)phenyl)-2,3 -difluoroheptanamide (2S,3R)-N-(2-amino-4-((4-hydroxybenzyl)amino)phenyl)-2,3-difluoroheptanamide (2R,3R)-N-(2-amino-4-((4-hydroxybenzyl)amino)phenyl)-2,3 -difluoroheptanamide (2S,3 S)-N-(2-amino-4-((4-hydroxybenzyl)amino)phenyl)-2,3 -difluoroheptanamide racemic mixtures thereof |
| 119. | 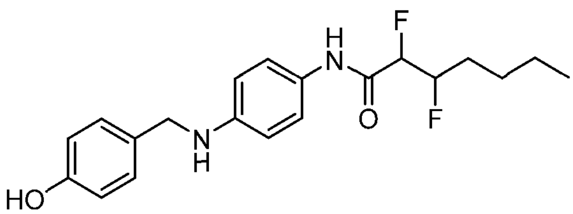 | 2,3-difluoro-N-(4-((4-hydroxybenzyl)amino)phenyl)he ptanamide (2R,3S)-2,3-difluoro-N-(4-((4-hydroxybenzyl)amino) phenyl)he ptanamide (2S,3R)-2,3-difluoro-N-(4-((4-hydroxybenzyl)amino) phenyl)he ptanamide (2R,3R)-2,3-difluoro-N-(4-((4-hydroxybenzyl)amino) phenyl)he ptanamide (2S,3S)-2,3-difluoro-N-(4-((4-hydroxybenzyl)amino) phenyl)he ptanamide racemic mixtures thereof |
| 120. | 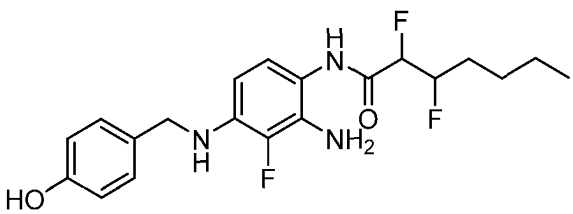 | N-(2-amino-3-fluoro-4-((4-hydroxybenzyl)amino)phenyl)-2,3 -difluoroheptanamide (2R,3 S)-N-(2-amino-3-fluoro-4-((4-hydroxybenzyl)amino)phenyl)-2,3 -difluoroheptanamide (2S,3R)-N-(2-amino-3-fluoro-4-((4-hydroxybenzyl)amino) phenyl)-2,3 -difluoroheptanamide (2R,3R)-N-(2-amino-3-fluoro-4-((4-hydroxybenzyl)amino)phenyl)-2,3 -difluoroheptanamide (2S,3S)-N-(2-amino-3-fluoro-4-((4-hydroxybenzyl)amino)phenyl)-2,3-difluoroheptanamide racemic mixtures thereof |

(continued)

| | COMPOUND STRUCTURE | COMPOUND NAME |
|---|---|---|
| 121. | 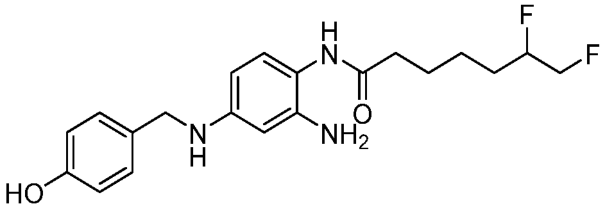 | N-(2-amino-4-((4-hydroxybenzyl)amino) phenyl)-6,7-difluoroheptanamide (6R)-N-(2-amino-4-((4-hydroxybenzyl) amino)phenyl)-6,7-difluoroheptanamide (6S)-N-(2-amino-4-((4-hydroxybenzyl) amino)phenyl)-6,7-difluoroheptanamide racemic mixtures thereof |
| 122. | 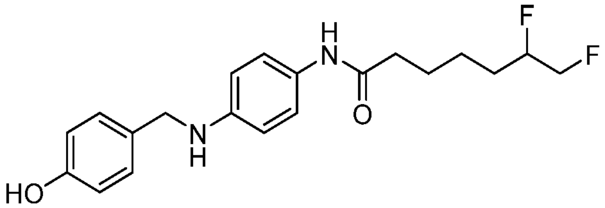 | 6,7-difluoro-N-(4-((4-hydroxybenzyl)amino)phenyl)he ptanamide (6R)-6,7-difluoro-N-(4-((4-hydroxybenzyl)amino) phenyl)he ptanamide (6S)-6,7-difluoro-N-(4-((4-hydroxybenzyl)amino)phenyl)he ptanamide racemic mixtures thereof |
| 123. | 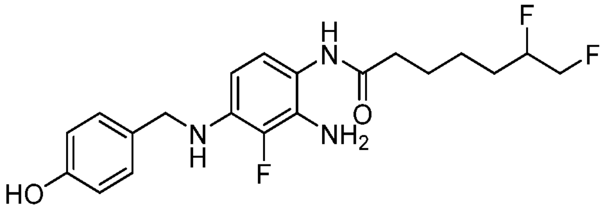 | N-(2-amino-3-fluoro-4-((4-hydroxybenzyl)amino)phenyl)-6,7-difluoroheptanamide (6R)-N-(2-amino-3-fluoro-4-((4-hydroxybenzyl)amino)phenyl)-6,7-difluoroheptanamide (6S)-N-(2-amino-3-fluoro-4-((4-hydroxybenzyl)amino)phenyl)-6,7-difluoroheptanamide racemic mixtures thereof |
| 124. | 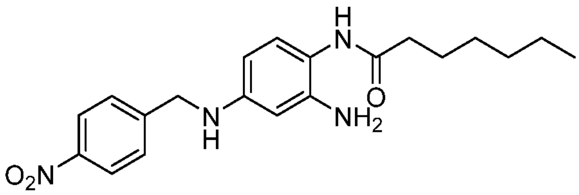 | N-(2-amino-4-((4-nitrobenzyl)amino)phenyl)hepta namide |
| 125. | 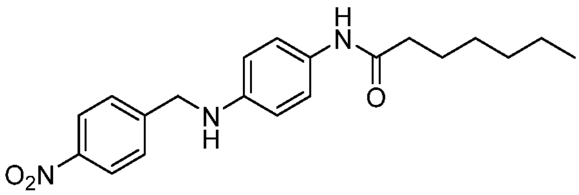 | N-(4-((4-nitrobenzyl)amino)phenyl)hepta namide |
| 126. | 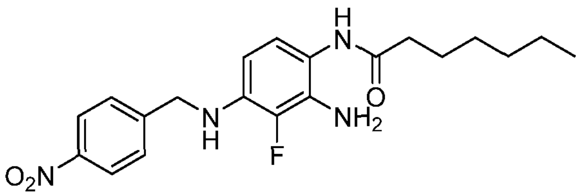 | N-(2-amino-3-fluoro-4-((4-nitrobenzyl) amino)phenyl)hepta namide |
| 127. | 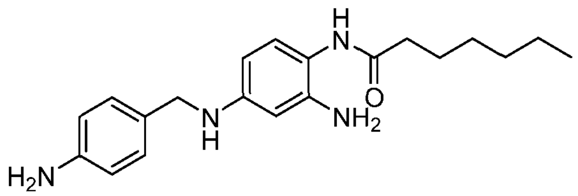 | N-(2-amino-4-((4-aminobenzyl)amino) phenyl)hept anamide |

(continued)

| | COMPOUND STRUCTURE | COMPOUND NAME |
|---|---|---|
| 128. | 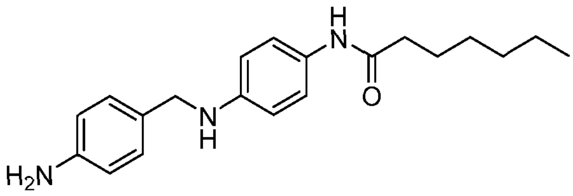 | N-(4-((4-aminobenzyl)amino)phenyl)hept anamide |
| 129. | 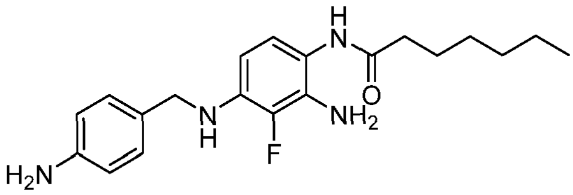 | N-(2-amino-3-fluoro-4-((4-aminobenzyl) amino)phenyl)hept anamide |
| 130. | 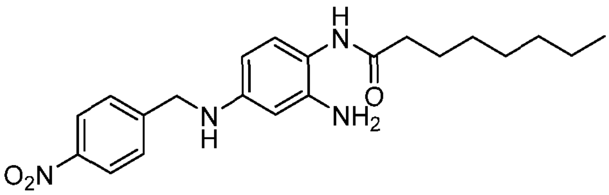 | N-(2-amino-4-((4-nitrobenzyl)amino)phe-nyl)octan amide |
| 131. | 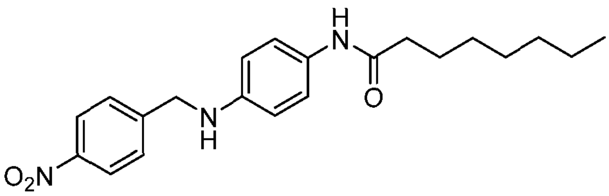 | N-(4-((4-nitrobenzyl)amino)phenyl)octan amide |
| 132. | 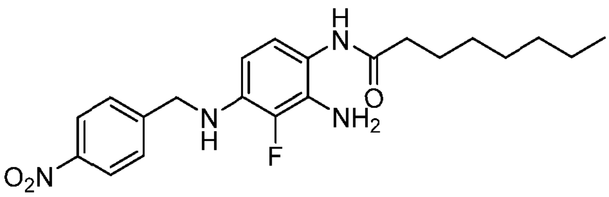 | N-(2-amino-3-fluoro-4-((4-nitrobenzyl) amino)phenyl)octan amide |
| 133. | 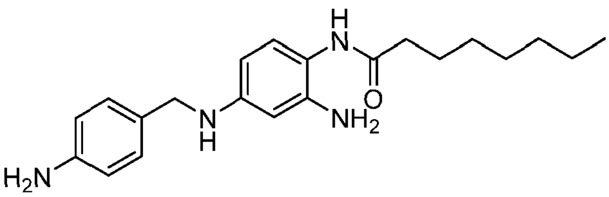 | N-(2-amino-4-((4-aminobenzyl)amino) phenyl)octa namide |
| 134. | 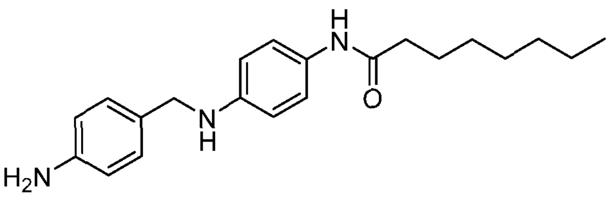 | N-(4-((4-aminobenzyl)amino)phenyl)octa namide |
| 135. | 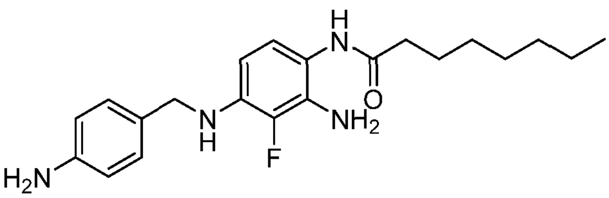 | N-(2-amino-3-fluoro-4-((4-aminobenzyl) amino)phenyl)octa namide |

(continued)

| | COMPOUND STRUCTURE | COMPOUND NAME |
|---|---|---|
| 136. | 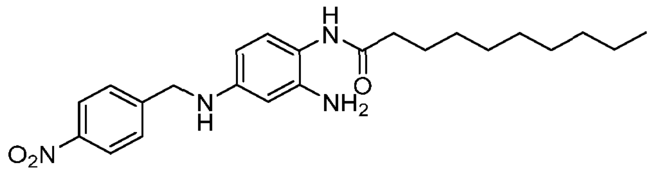 | N-(2-amino-4-((4-nitrobenzyl)amino)phenyl)decan amide |
| 137. | 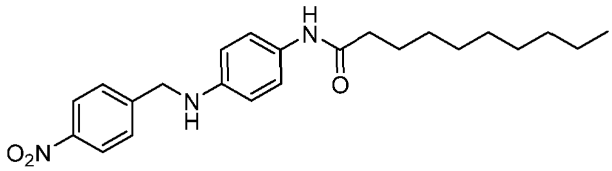 | N-(4-((4-nitrobenzyl)amino)phenyl)decan amide |
| 138. | 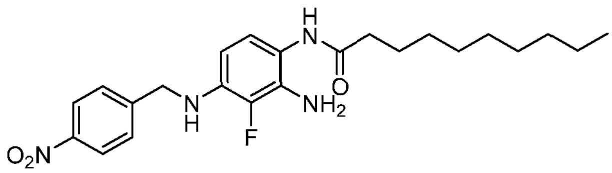 | N-(2-amino-3-fluoro-4-((4-nitrobenzyl) amino)phenyl)decan amide |
| 139. | 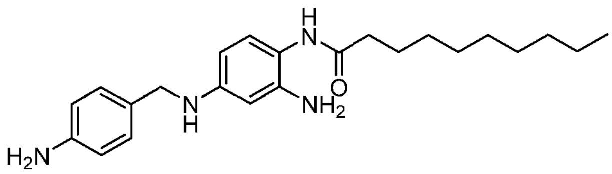 | N-(2-amino-4-((4-aminobenzyl)amino) phenyl)deca namide |
| 140. | 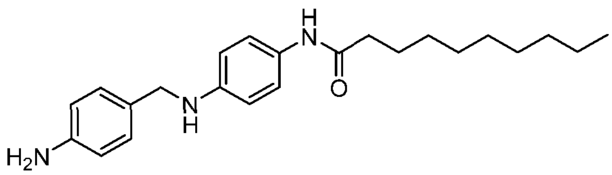 | N-(4-((4-aminobenzyl)amino)phenyl) deca namide |
| 141. | 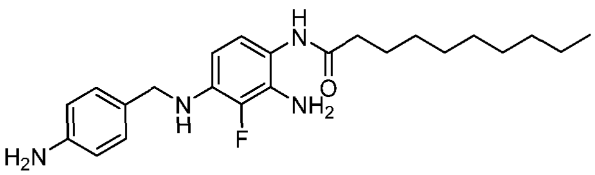 | N-(2-amino-3-fluoro-4-((4-aminobenzyl) amino)phenyl)deca namide |
| 142. | 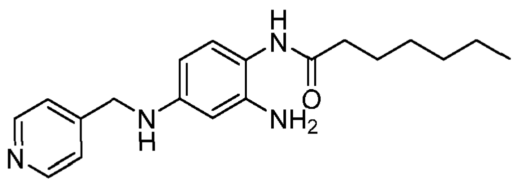 | N-(2-amino-4-((pyridin-4-ylmethyl)amino) phenyl)heptana mide |
| 143. | 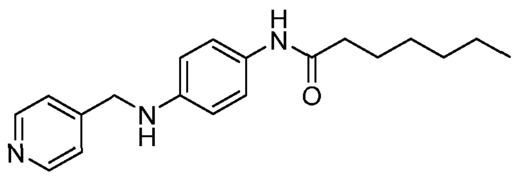 | N-(4-((pyridin-4-ylmethyl)amino)phenyl) heptana mide |
| 144. | 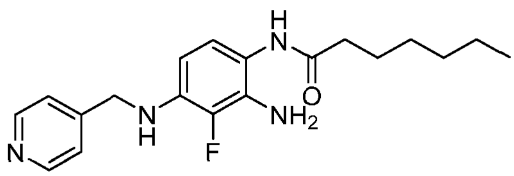 | N-(2-amino-3-fluoro-4-((pyridin-4-ylmethyl)amino)phenyl)heptana mide |

(continued)

| | COMPOUND STRUCTURE | COMPOUND NAME |
|---|---|---|
| 145. | 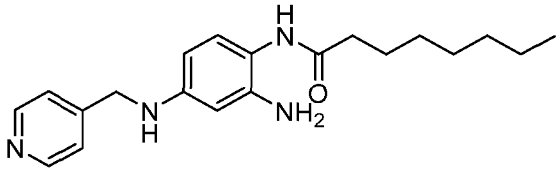 | N-(2-amino-4-((pyridin-4-ylmethyl)amino) phenyl)octanam ide |
| 146. | 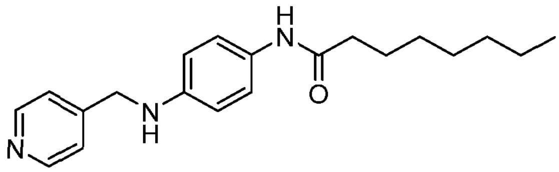 | N-(4-((pyridin-4-ylmethyl)amino)phenyl) octanam ide |
| 147. | 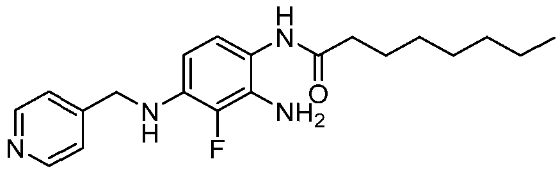 | N-(2-amino-3-fluoro-4-((pyridin-4-ylmethyl)amino)phenyl)octanam ide |
| 148. | 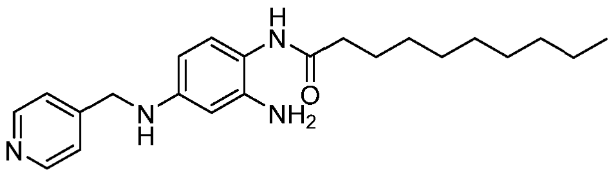 | N-(2-amino-4-((pyridin-4-ylmethyl)amino) phenyl)decana mide |
| 149. | 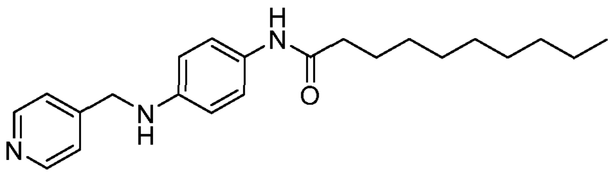 | N-(4-((pyridin-4-ylmethyl)amino)phenyl) decana mide |
| 150. | 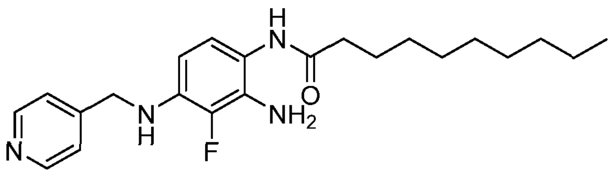 | N-(2-amino-3-fluoro-4-((pyridin-4-ylmethyl)amino)phenyl)decana mide |
| 151. | 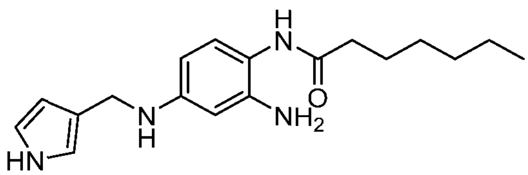 | N-(4-(((1H-pyrrol-3-yl)methyl)amino)-2-aminophenyl)heptanamide |
| 152. | 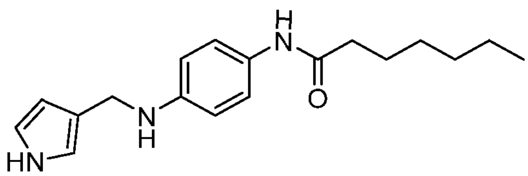 | N-(4-(((1H-pyrrol-3-yl)methyl)amino)phe-nyl)heptana mide |
| 153. | 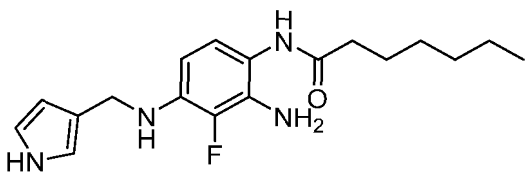 | N-(4-(((1H-pyrrol-3-yl)methyl)amino)-2-amino-3-fluorophenyl)heptanamide |

(continued)

| | COMPOUND STRUCTURE | COMPOUND NAME |
|---|---|---|
| 154. | 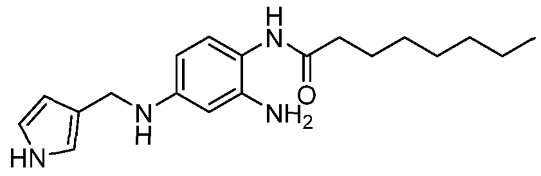 | N-(4-(((1H-pyrrol-3-yl)methyl)amino)-2-aminophenyl)octanamide |
| 155. | 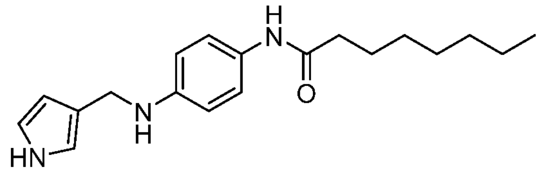 | N-(4-(((1H-pyrrol-3-yl)methyl)amino)phenyl)octana mide |
| 156. | 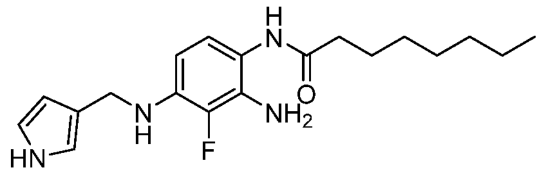 | N-(4-(((1H-pyrrol-3-yl)methyl)amino)-2-amino-3-fluorophenyl)octanamide |
| 157. | 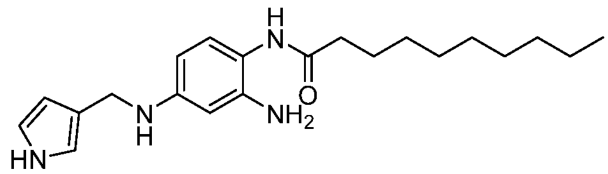 | N-(4-(((1H-pyrrol-3-yl)methyl)amino)-2-aminophenyl)decanamide |
| 158. | 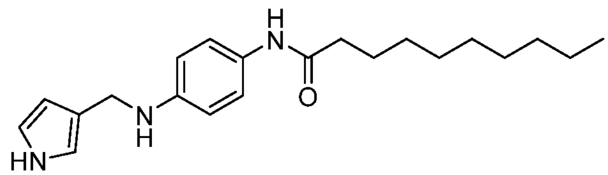 | N-(4-(((1H-pyrrol-3-yl)methyl)amino)phenyl)decana mide |
| 159. | 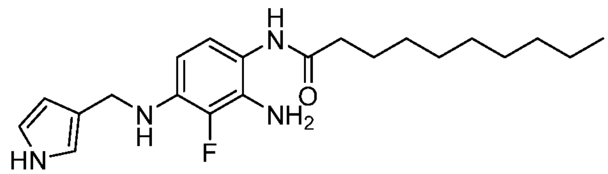 | N-(4-(((1H-pyrrol-3-yl)methyl)amino)-2-amino-3-fluorophenyl)decanamide |
| 160. | 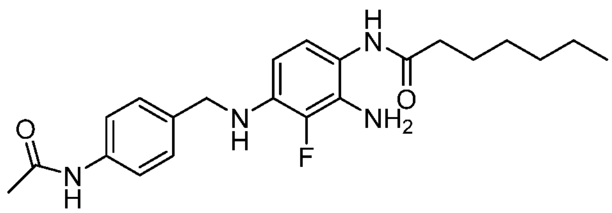 | N-(4-((4-acetamidobenzyl)amino)-2-amino-3-fluorophenyl)heptanamide |
| 161. | 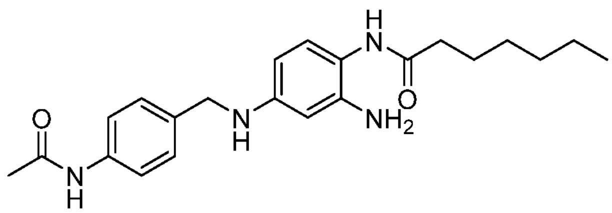 | N-(4-((4-acetamidobenzyl)amino)-2-aminophenyl)heptanamide |

(continued)

| | COMPOUND STRUCTURE | COMPOUND NAME |
|---|---|---|
| 162. | 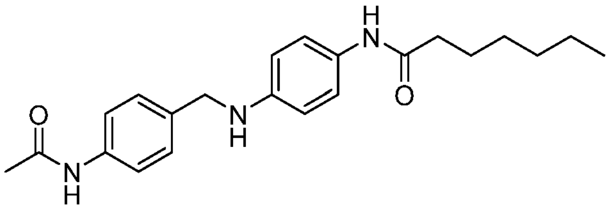 | N-(4-((4-acetamidobenzyl)amino)phenyl) heptanamide |
| 163. | 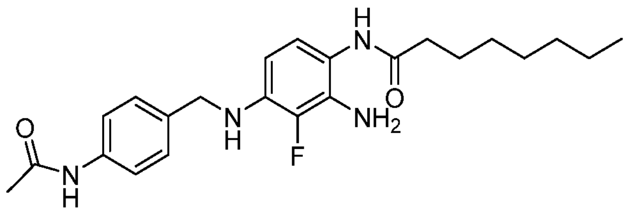 | N-(4-((4-acetamidobenzyl)amino)-2-amino-3-fluorophenyl)octanamide |
| 164. | 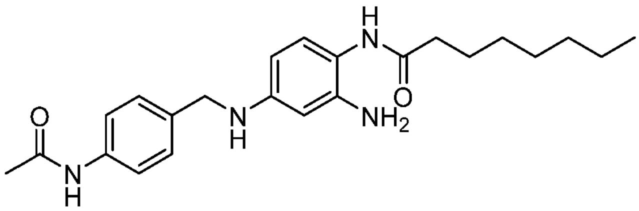 | N-(4-((4-acetamidobenzyl)amino)-2-aminophenyl)octanamide |
| 165. | 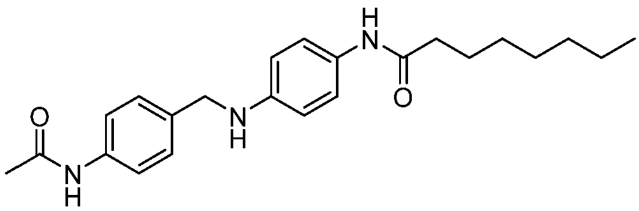 | N-(4-((4-acetamidobenzyl)amino)phenyl) octanamide |
| 166. | 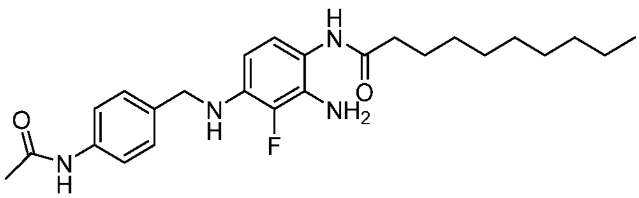 | N-(4-((4-acetamidobenzyl)amino)-2-amino-3-fluorophenyl)decanamide |
| 167. | 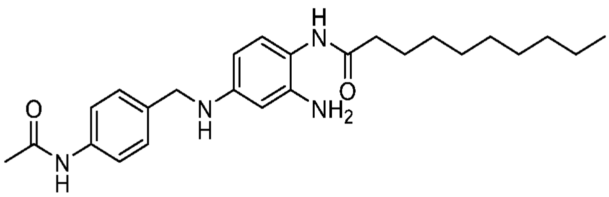 | N-(4-((4-acetamidobenzyl)amino)-2-aminophenyl)decanamide |
| 168. | 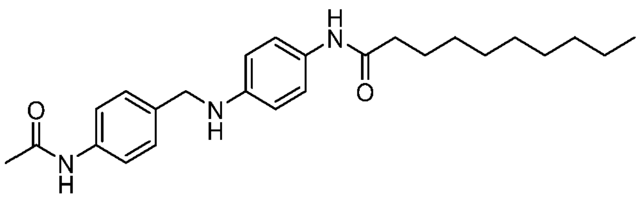 | N-(4-((4-acetamidobenzyl)amino)phenyl) decanamide |

(continued)

| | COMPOUND STRUCTURE | COMPOUND NAME |
|---|---|---|
| 169. | 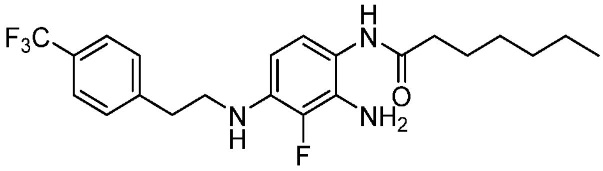 | N-(2-amino-3-fluoro-4-((4-(trifluoromethyl)phenethyl)amin o)phenyl)hepta-namide |
| 170. | 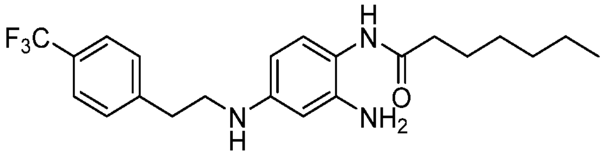 | N-(2-amino-4-((4-(trifluoromethyl)phenethyl)amin o)phenyl)heptanamide |
| 171. | 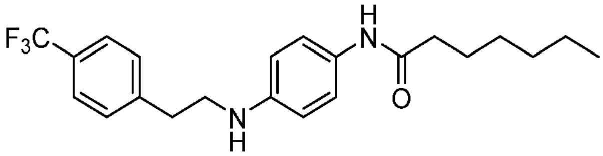 | N-(4-((4-(trifluoromethyl)phenethyl)amin o)phenyl)heptanamide |
| 172. | 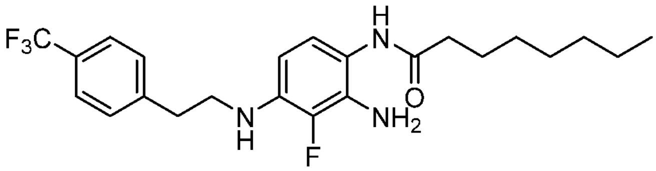 | N-(2-amino-3-fluoro-4-((4-(trifluoromethyl)phenethyl)amin o)phenyl)octana-mide |
| 173. | 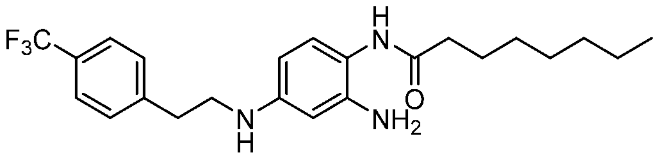 | N-(2-amino-4-((4-(trifluoromethyl)phenethyl)amin o)pheny)loctanamide |
| 174. | 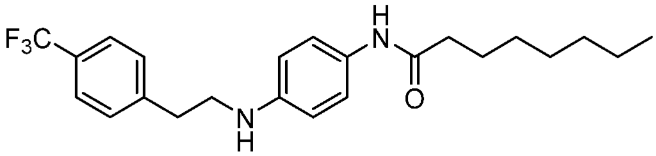 | N-(4-((4-(trifluoromethyl)phenethyl)amin o)phenyl)octanamide |
| 175. | 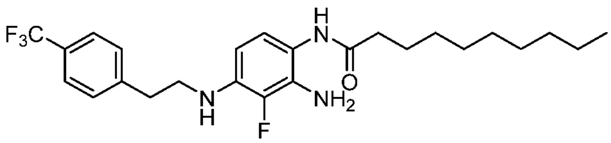 | N-(2-amino-3-fluoro-4-((4-(trifluoromethyl)phenethyl)amin o)phenyl)decana-mide |
| 176. | 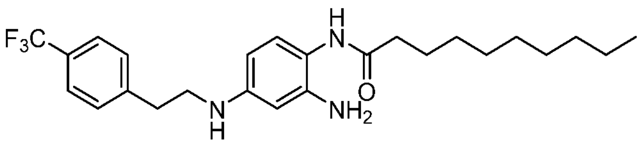 | N-(2-amino-4-((4-(trifluoromethyl)phenethyl)amin o)phenyl)decanamide |
| 177. | 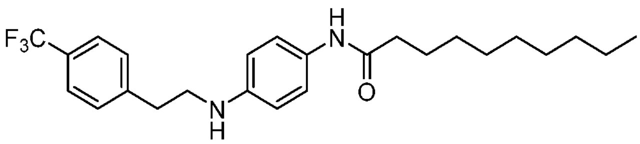 | N-(4-((4-(trifluoromethyl)phenethyl)amin o)phenyl)decanamide |

(continued)

| | COMPOUND STRUCTURE | COMPOUND NAME |
|---|---|---|
| 178. | 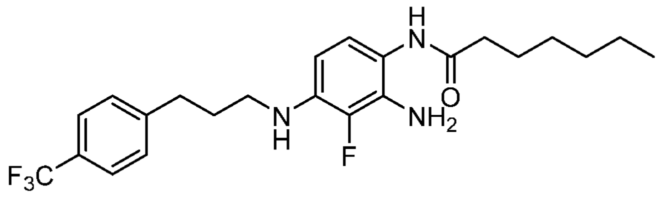 | N-(2-amino-3-fluoro-4-((3-(4-(trifluoromethyl)phenyl)propyl)a mino)phenyl)heptanamide |
| 179. | 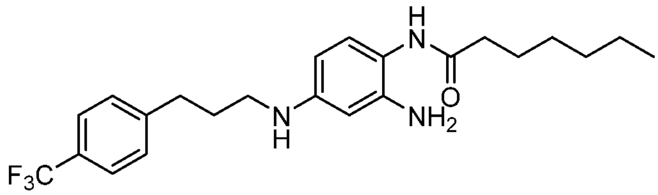 | N-(2-amino-4-((3-(4-(trifluoromethyl)phenyl)propyl)a mino)phenyl)heptanamide |
| 180. | 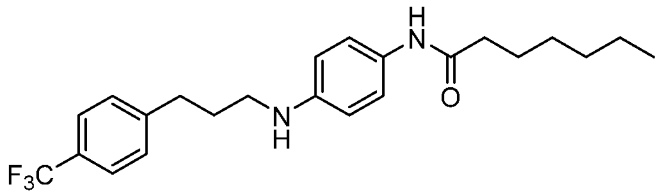 | N-(4-((3-(4-(trifluoromethyl)phenyl)propyl)a mino)phenyl)heptanamide |
| 181. | 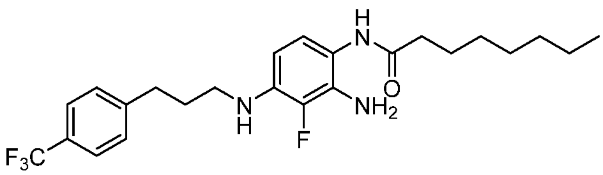 | N-(2-amino-3-fluoro-4-((3-(4-(trifluoromethyl)phenyl)propyl)a mino)phenyl)octanamide |
| 182. | 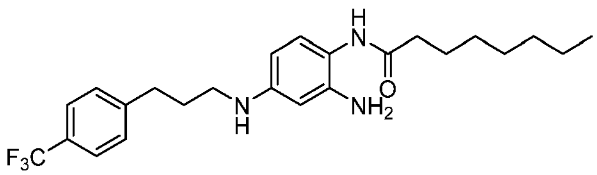 | N-(2-amino-4-((3-(4-(trifluoromethyl)phenyl)propyl)a mino)phenyl)octanamide |
| 183. | 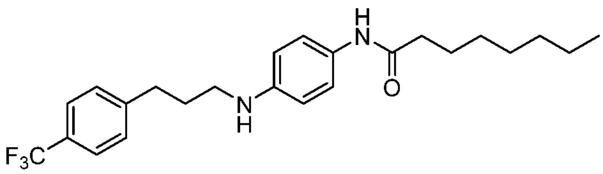 | N-(4-((3-(4-(trifluoromethyl)phenyl)propyl)a mino)phenyl)octanamide |
| 184. | 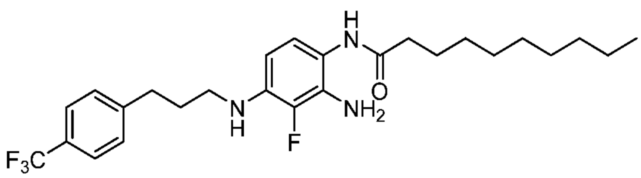 | N-(2-amino-3-fluoro-4-((3-(4-(trifluoromethyl)phenyl)propyl)a mino)phenyl)decanamide |
| 185. | 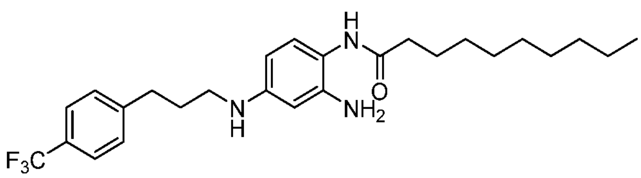 | N-(2-amino-4-((3-(4-(trifluoromethyl)phenyl)propyl)a mino)phenyl)decanamide |

(continued)

| | COMPOUND STRUCTURE | COMPOUND NAME |
|---|---|---|
| 186. | 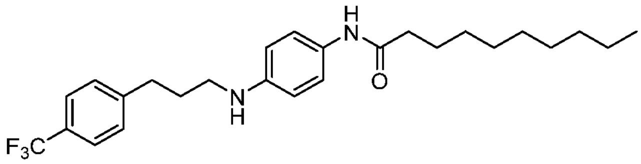 | N-(4-((3-(4-(trifluoromethyl)phenyl)propyl)a mino)phenyl)decanamide |
| 187. | 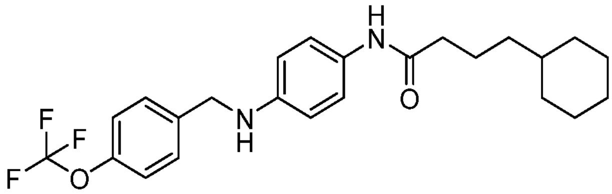 | 4-cyclohexyl-N-(4-((4-(trifluoromethoxy) benzyl)amino )phenyl)butanamide |
| 188. | 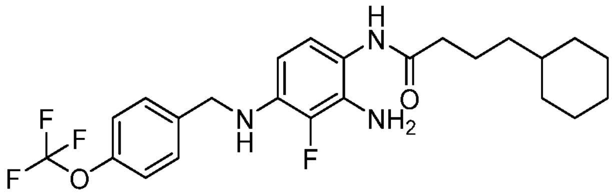 | N-(2-amino-3-fluoro-4-((4-(trifluoromethoxy)benzyl)amino )phenyl)-4-cyclohexylbutanamide |
| 189. | 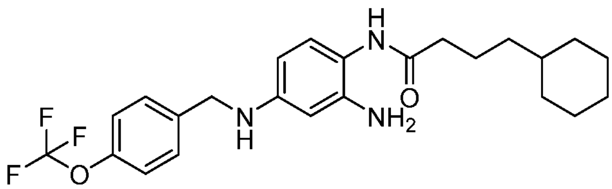 | N-(2-amino-4-((4-(trifluoromethoxy)benzyl)amino )phenyl)-4-cyclohexylbutanamide |
| 190. | 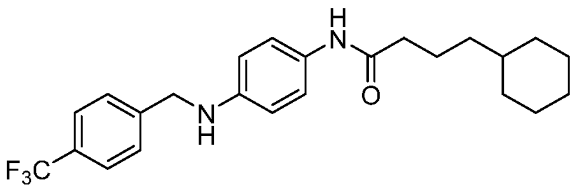 | 4-cyclohexyl-N-(4-((4-(trifluoromethyl) benzyl)amino)p henyl)butanamide |
| 191. | 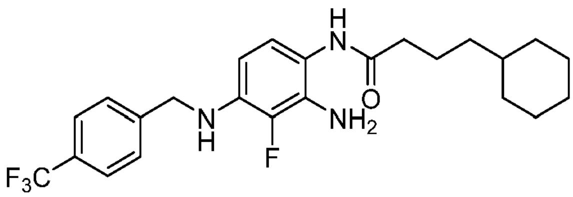 | N-(2-amino-3-fluoro-4-((4-(trifluoromethyl)benzyl)amino)p henyl)-4-cyclohexylbutanamide |
| 192. | 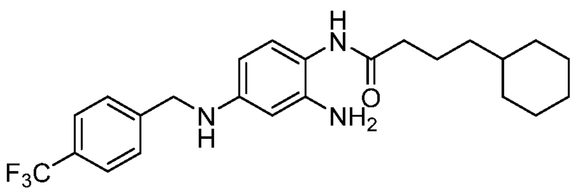 | N-(2-amino-4-((4-(trifluoromethyl)benzyl) amino)p henyl)-4-cyclohexylbutanamide |
| 193. | 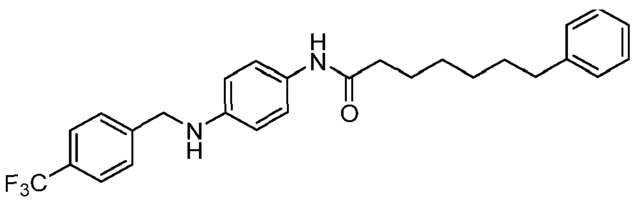 | 7-phenyl-N-(4-((4-(trifluoromethyl)benzyl) amino)p henyl)heptanamide |

(continued)

| | COMPOUND STRUCTURE | COMPOUND NAME |
|---|---|---|
| 194. | 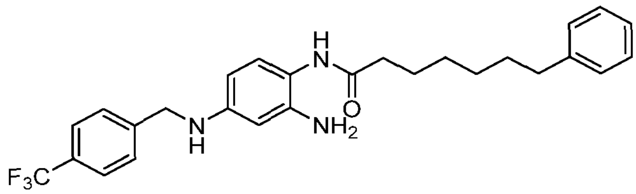 | N-(2-amino-4-((4-(trifluoromethyl)benzyl) amino)p henyl)-7-phenylheptanamide |
| 195. | 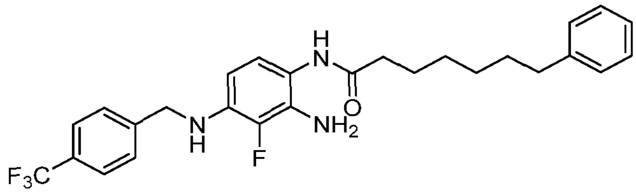 | N-(2-amino-3-fluoro-4-((4-(trifluoromethyl)benzyl)amino)p henyl)-7-phenyl-heptanamide |
| 196. | 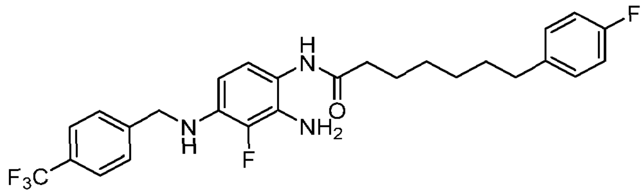 | N-(2-amino-3 -fluoro-4-((4-(trifluoromethyl)benzyl)amino)p henyl)-7-(4-fluor-ophenyl)heptanamide |
| 197. | 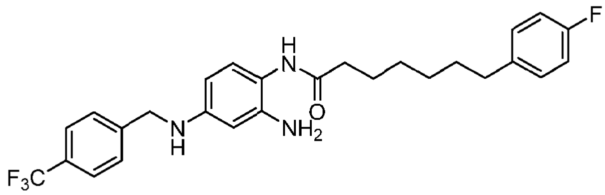 | N-(2-amino-4-((4-(trifluoromethyl)benzyl) amino)p henyl)-7-(4-fluorophenyl)hepta-namide |
| 198. | 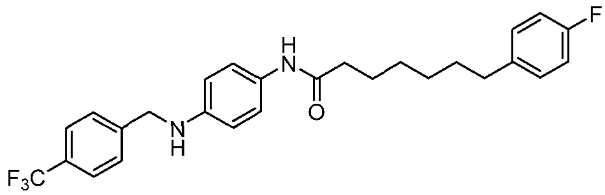 | 7-(4-fluorophenyl)-N-(4-((4-(trifluoromethyl)benzyl)amino)p henyl)heptana-mide |
| 199. | 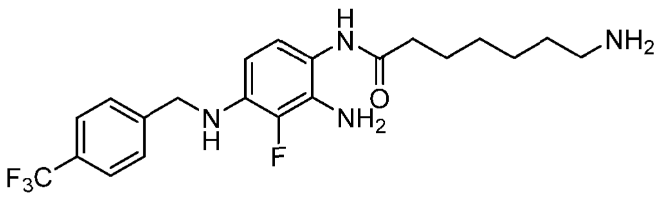 | 7-amino-N-(2-amino-3-fluoro-4-((4-(trifluoromethyl)benzyl)amino)p henyl)hep-tanamide |
| 200. | 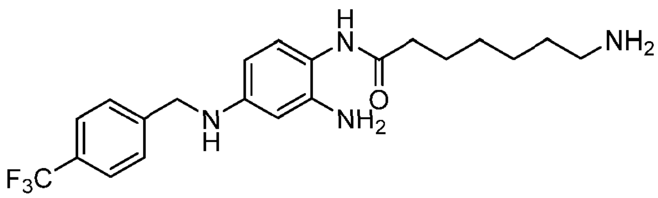 | 7-amino-N-(2-amino-4-((4-(trifluoromethyl)benzyl)amino)p henyl)heptana-mide |
| 201. | 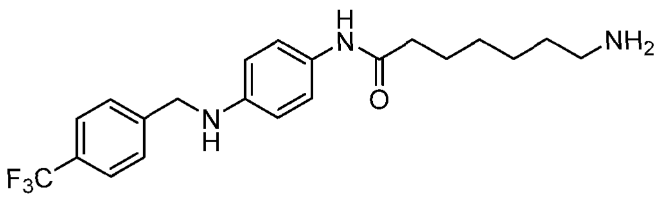 | 7-amino-N-(4-((4-(trifluoromethyl)benzyl) amino)p henyl)heptanamide |

(continued)

| | COMPOUND STRUCTURE | COMPOUND NAME |
|---|---|---|
| 202. | 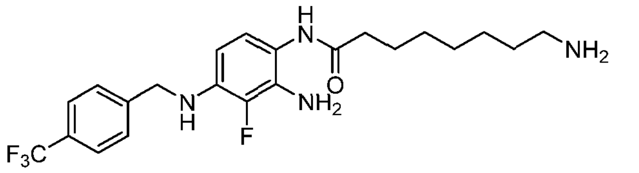 | 8-amino-N-(2-amino-3-fluoro-4-((4-(trifluoromethyl)benzyl)amino)p henyl)octanamide |
| 203. | 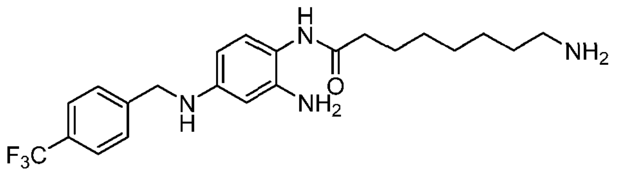 | 8-amino-N-(2-amino-4-((4-(trifluoromethyl)benzyl)amino)p henyl)octanamide |
| 204. | 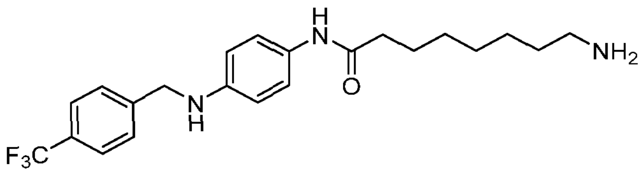 | 8-amino-N-(4-((4-(trifluoromethyl)benzyl) amino)p henyl)octanamide |
| 205. | 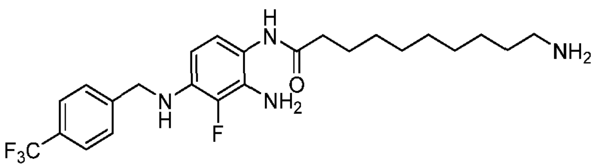 | 10-amino-N-(2-amino-3-fluoro-4-((4-(trifluoromethyl)benzyl)amino)p henyl)decanamide |
| 206. | 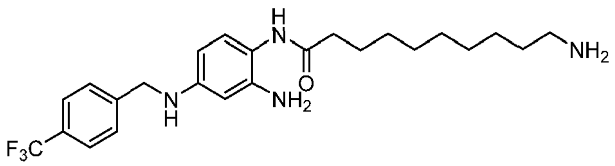 | 10-amino-N-(2-amino-4-((4-(trifluoromethyl)benzyl)amino)p henyl)decanamide |
| 207. | 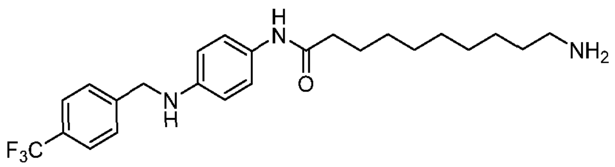 | 10-amino-N-(4-((4-(trifluoromethyl)benzyl)amino)p henyl)decanamide |
| 208. | 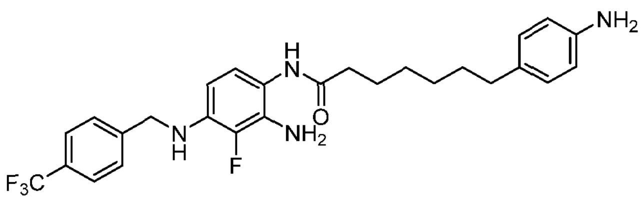 | N-(2-amino-3-fluoro-4-((4-(trifluoromethyl)benzyl)amino)p henyl)-7-(4-aminophenyl)heptanamide |
| 209. | 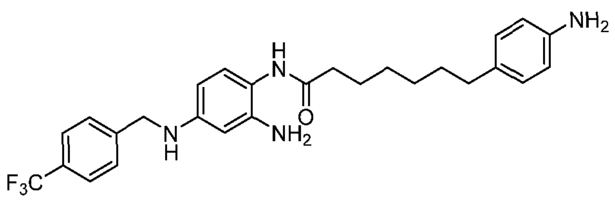 | N-(2-amino-4-((4-(trifluoromethyl)benzyl) amino)p henyl)-7-(4-aminophenyl)heptanamide |

(continued)

| | COMPOUND STRUCTURE | COMPOUND NAME |
|---|---|---|
| 210. | 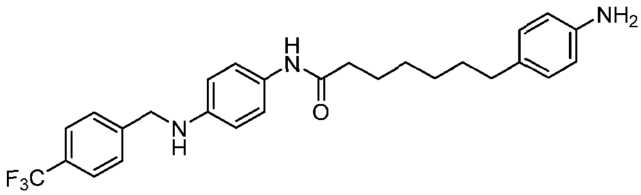 | 7-(4-aminophenyl)-N-(4-((4-(trifluoromethyl)benzyl)amino)p henyl)heptanamide |
| 211. | 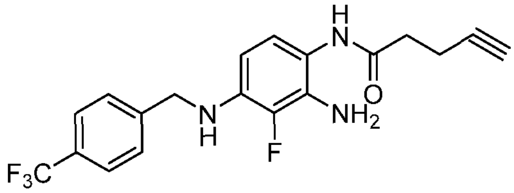 | N-(2-amino-3-fluoro-4-((4-(trifluoromethyl)benzyl)amino)p henyl)pent-4-ynamide |
| 212. | 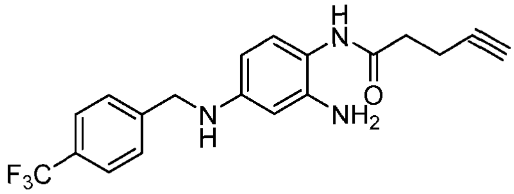 | N-(2-amino-4-((4-(trifluoromethyl)benzyl) amino)p henyl)pent-4-ynamide |
| 213. | 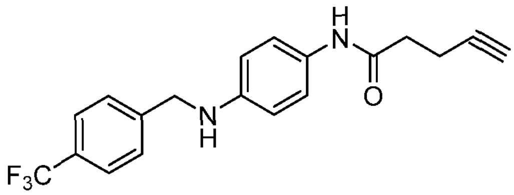 | N-(4-((4-(trifluoromethyl)benzyl)amino)p henyl)pent-4-ynamide |
| 214. | 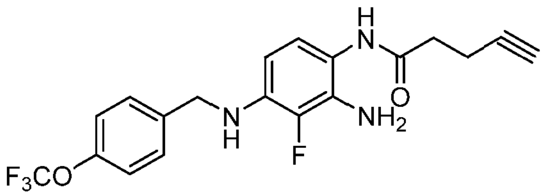 | N-(2-amino-3-fluoro-4-((4-(trifluoromethoxy)benzyl)amino )phenyl)pent-4-ynamide |
| 215. | 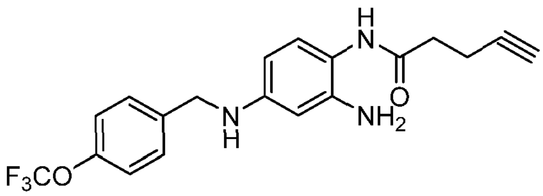 | N-(2-amino-4-((4-(trifluoromethoxy)benzyl)amino )phenyl)pent-4-ynamide |
| 216. | 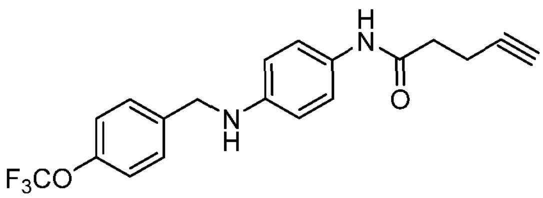 | N-(4-((4-(trifluoromethoxy)benzyl)amino ) phenyl)pent-4-ynamide |
| 217. | 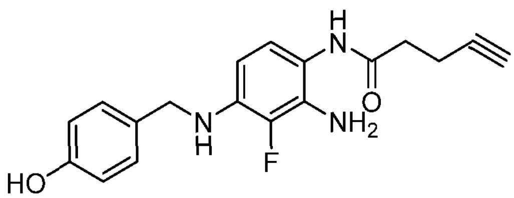 | N-(2-amino-3-fluoro-4-((4-hydroxybenzyl)amino)phenyl)pe nt-4-ynamide |

(continued)

| | COMPOUND STRUCTURE | COMPOUND NAME |
|---|---|---|
| 218. | 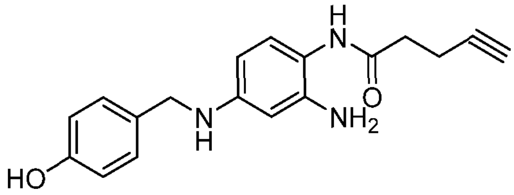 | N-(2-amino-4-((4-hydroxybenzyl)amino) phenyl)pe nt-4-ynamide |
| 219. | 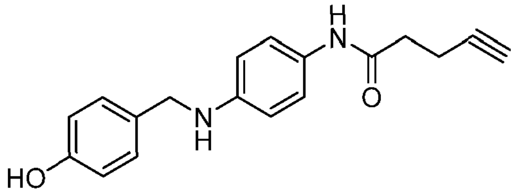 | N-(4-((4-hydroxybenzyl)amino)phenyl)pe nt-4-ynamide |
| 220. | 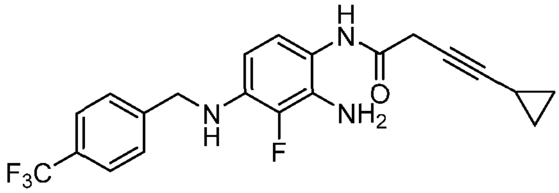 | N-(2-amino-3-fluoro-4-((4-(trifluoromethyl)benzyl)amino)p henyl)-4-cyclo-propylbut-3-ynamide |
| 221. | 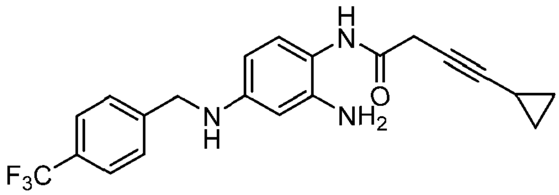 | N-(2-amino-4-((4-(trifluoromethyl)benzyl) amino)p henyl)-4-cyclopropylbut-3-yna-mide |
| 222. | 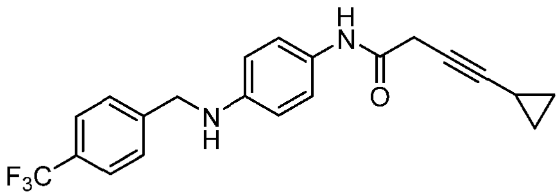 | 4-cyclopropyl-N-(4-((4-(trifluoromethyl) benzyl)amino)p henyl)but-3-ynamide |
| 223. | 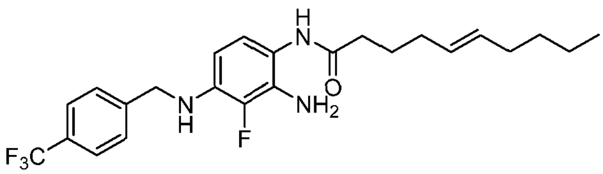 | (E)-N-(2-amino-3-fluoro-4-((4-(trifluoromethyl)benzyl)amino)p henyl)dec-5-en-amide |
| 224. | 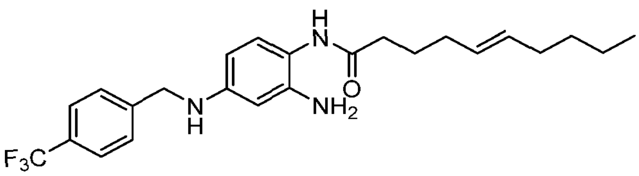 | (E)-N-(2-amino-4-((4-(trifluoromethyl) benzyl)amino)p henyl)dec-5-enamide |
| 225. | 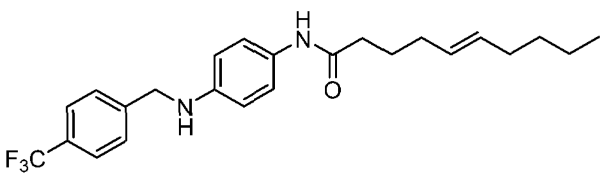 | (E)-N-(4-((4-(trifluoromethyl)benzyl)amino)p henyl)dec-5-enamide |

(continued)

| | COMPOUND STRUCTURE | COMPOUND NAME |
|---|---|---|
| 226. | 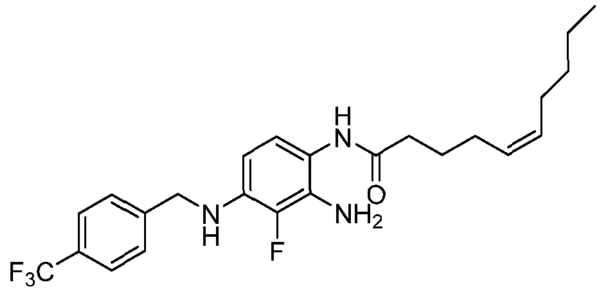 | (Z)-N-(2-amino-3-fluoro-4-((4-(trifluoromethyl)benzyl)amino)p henyl)dec-5-enamide |
| 227. | 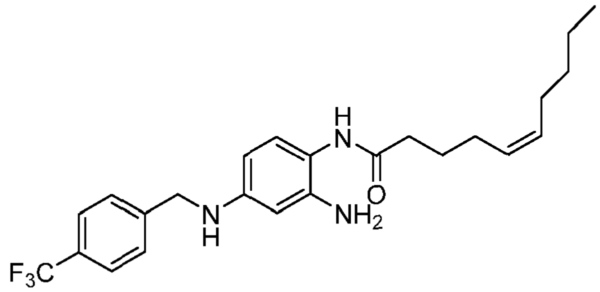 | (Z)-N-(2-amino-4-((4-(trifluoromethyl) benzyl)amino)p henyl)dec-5-enamide |
| 228. | 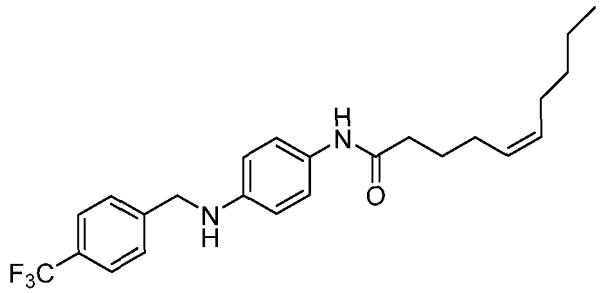 | (Z)-N-(4-((4-(trifluoromethyl)benzyl)amino)p henyl)dec-5-enamide |
| 229. | 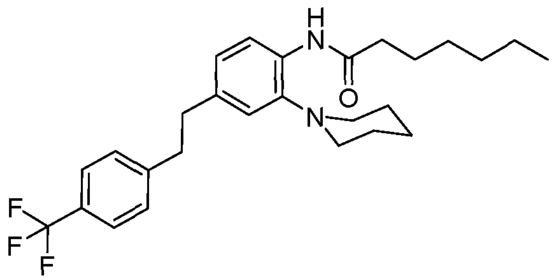 | N-(2-(piperidin-1-yl)-4-(4-(trifluoromethyl) phenethyl)phen yl)heptanamide |
| 230. | 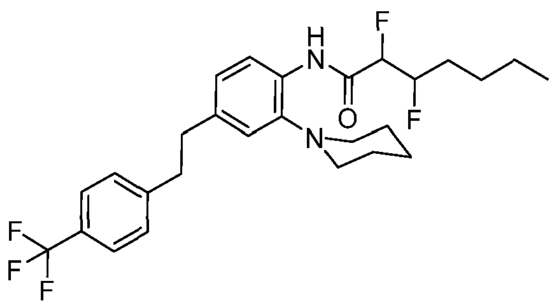 | 2,3-difluoro-N-(2-(piperidin-1-yl)-4-(4-(trifluoromethyl)phenethyl)phen yl)heptanamide<br>(2R,3 S)-2,3-difluoro-N-(2-(piperidin-1-yl)-4-(4-(trifluoromethyl)phenethyl)phen yl)heptanamide<br>(2S,3R)-2,3-difluoro-N-(2-(piperidin-1-yl)-4-(4-(trifluoromethyl)phenethyl)phen yl)heptanamide<br>(2R,3R)-2,3-difluoro-N-(2-(piperidin-1-yl)-4-(4-(trifluoromethyl)phenethyl)phen yl)heptanamide<br>(2S,3 S)-2,3-difluoro-N-(2-(piperidin-1-yl)-4-(4-(trifluoromethyl)phenethyl)phen yl)heptanamide |

(continued)

| | COMPOUND STRUCTURE | COMPOUND NAME |
|---|---|---|
| 231. | 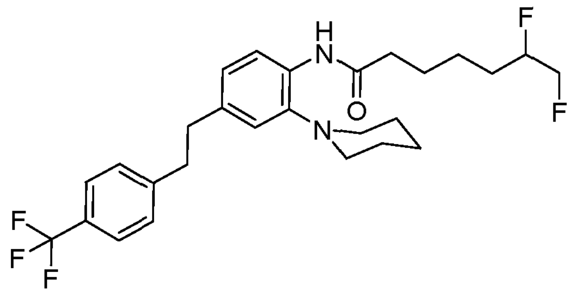 | 6,7-difluoro-N-(2-(piperidin-1-yl)-4-(4-(trifluoromethyl)phenethyl)phen yl)heptanamide<br>(6R)-6,7-difluoro-N-(2-(piperidin-1-yl)-4-(4-(trifluoromethyl)phenethyl)phen yl)heptanamide<br>(6S)-6,7-difluoro-N-(2-(piperidin-1-yl)-4-(4-(trifluoromethyl)phenethyl)phen yl)heptanamide |
| 232. | 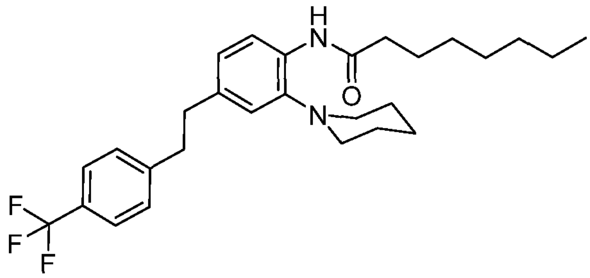 | N-(2-(piperidin-1-yl)-4-(4-(trifluoromethyl) phenethyl)phen yl)octanamide |
| 233. | 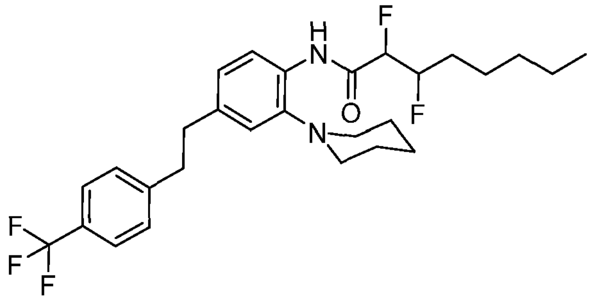 | 2,3-difluoro-N-(2-(piperidin-1-yl)-4-(4-(trifluoromethyl)phenethyl)phen yl)octanamide<br>(2R,3 S)-2,3-difluoro-N-(2-(piperidin-1-yl)-4-(4-(trifluoromethyl)phenethyl)phen yl)octanamide<br>(2S,3R)-2,3-difluoro-N-(2-(piperidin-1-yl)-4-(4-(trifluoromethyl)phenethyl)phen yl)octanamide<br>(2R,3R)-2,3-difluoro-N-(2-(piperidin-1-yl)-4-(4-(trifluoromethyl)phenethyl)phen yl)octanamide<br>(2S,3 S)-2,3-difluoro-N-(2-(piperidin-1-yl)-4-(4-(trifluoromethyl)phenethyl)phen yl)octanamide |
| 234. | 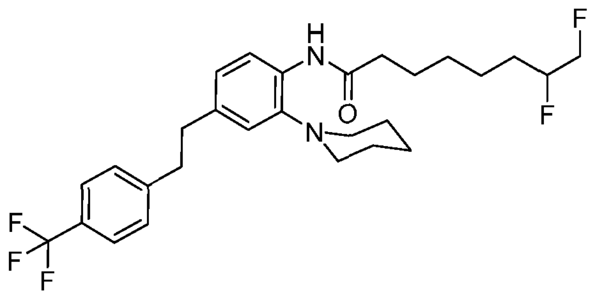 | 7,8-difluoro-N-(2-(piperidin-1-yl)-4-(4-(trifluoromethyl)phenethyl)phen yl)octanamide<br>(7R)-7,8-difluoro-N-(2-(piperidin-1-yl)-4-(4-(trifluoromethyl)phenethyl)phen yl)octanamide<br>(7S)-7,8-difluoro-N-(2-(piperidin-1-yl)-4-(4-(trifluoromethyl)phenethyl)phen yl)octanamide |
| 235. | 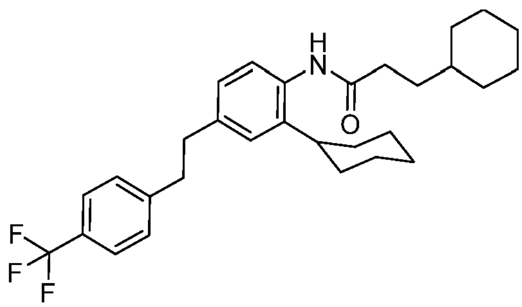 | 3-cyclohexyl-N-(2-cyclohexyl-4-(4-(trifluoromethyl)phenethyl)phen yl)propanamide |

(continued)

| | COMPOUND STRUCTURE | COMPOUND NAME |
|---|---|---|
| 236. | 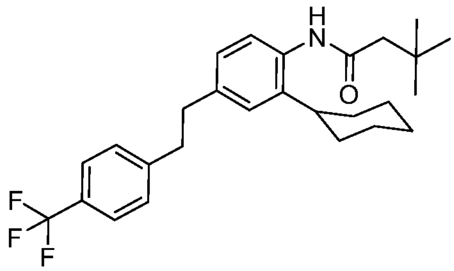 | N-(2-cyclohexyl-4-(4-(trifluoromethyl) phenethyl)phen yl)-3,3-dimethylbutanamide |
| 237. | 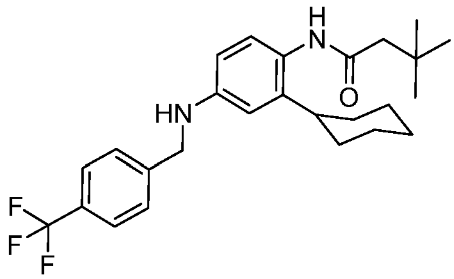 | N-(2-cyclohexyl-4-((4-(trifluoromethyl) benzyl)amino)p henyl)-3,3-dimethylbutanamide |
| 238. | 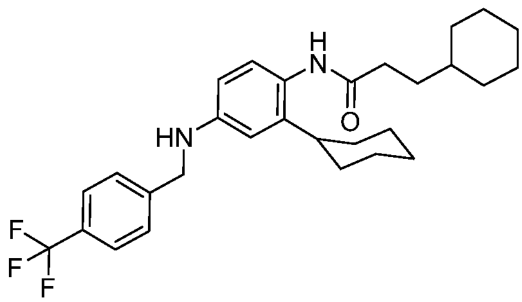 | 3-cyclohexyl-N-(2-cyclohexyl-4-((4-(trifluoromethyl)benzyl)amino)p henyl)propanamide |
| 239. | 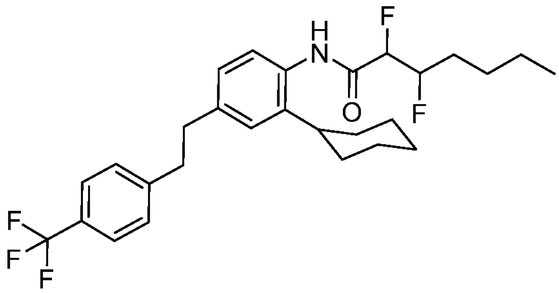 | N-(2-cyclohexyl-4-(4-(trifluoromethyl) phenethyl)phen yl)-2,3-difluoroheptanamide<br>(2R,3 S)-N-(2-cyclohexyl-4-(4-(trifluoromethyl)phenethyl)phen yl)-2,3-difluoroheptanamide<br>(2S,3R)-N-(2-cyclohexyl-4-(4-(trifluoromethyl)phenethyl)phen yl)-2,3-difluoroheptanamide<br>(2R,3R)-N-(2-cyclohexyl-4-(4-(trifluoromethyl)phenethyl)phen yl)-2,3-difluoroheptanamide<br>(2S,3 S)-N-(2-cyclohexyl-4-(4-(trifluoromethyl)phenethyl)phen yl)-2,3-difluoroheptanamide |
| 240. | 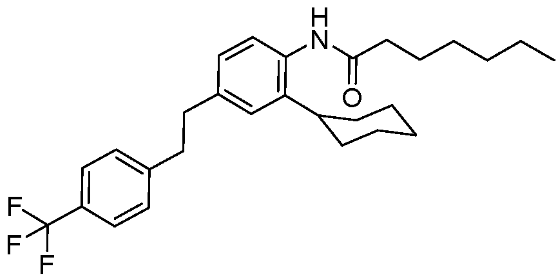 | N-(2-cyclohexyl-4-(4-(trifluoromethyl) phenethyl)phen yl)heptanamide |

(continued)

| | COMPOUND STRUCTURE | COMPOUND NAME |
|---|---|---|
| 241. | 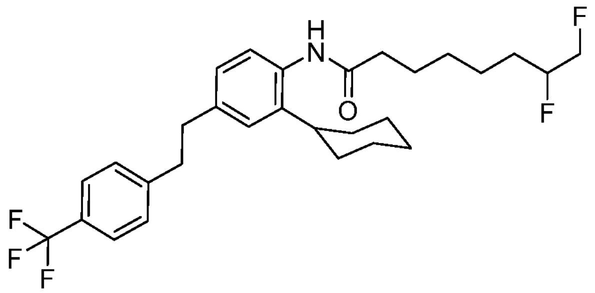 | N-(2-cyclohexyl-4-(4-(trifluoromethyl) phenethyl)phen yl)-7,8-difluorooctanamide (7R)-N-(2-cyclohexyl-4-(4-(trifluoromethyl)phenethyl)phen yl)-7,8-difluoroctanamide (7S)-N-(2-cyclohexyl-4-(4-(trifluoromethyl)phenethyl)phen yl)-7,8-difluorooctanamide |
| 242. | 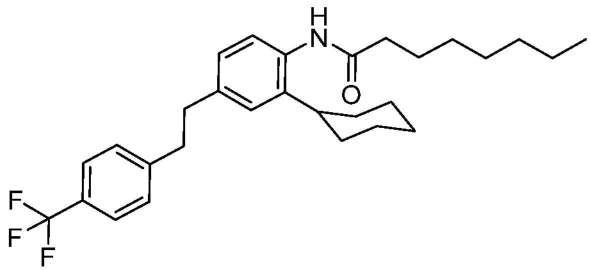 | N-(2-cyclohexyl-4-(4-(trifluoromethyl) phenethyl)phen yl)octanamide |
| 243. | 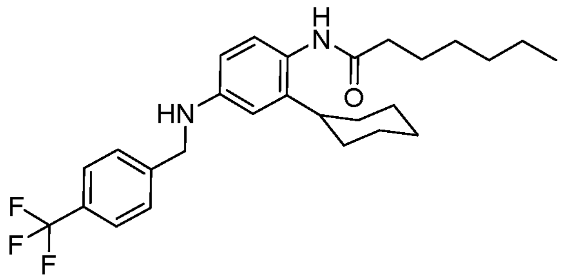 | N-(2-cyclohexyl-4-((4-(trifluoromethyl) benzyl)amino)p henyl)heptanamide |
| 244. | 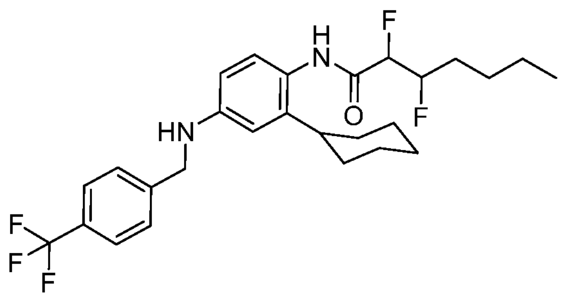 | N-(2-cyclohexyl-4-((4-(trifluoromethyl) benzyl)amino)p henyl)-2,3-difluoroheptanamide<br>(2R,3 S)-N-(2-cyclohexyl-4-((4-(trifluoromethyl)benzyl)amino)p henyl)-2,3-difluoroheptanamide<br>(2S,3R)-N-(2-cyclohexyl-4-((4-(trifluoromethyl)benzyl)amino)p henyl)-2,3-difluoroheptanamide<br>(2R,3R)-N-(2-cyclohexyl-4-((4-(trifluoromethyl)benzyl)amino)p henyl)-2,3-difluoroheptanamide<br>(2S,3 S)-N-(2-cyclohexyl-4-((4-(trifluoromethyl)benzyl)amino)p henyl)-2,3-difluoroheptanamide |
| 245. | 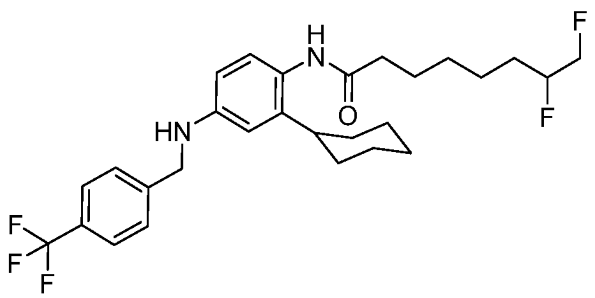 | N-(2-cyclohexyl-4-((4-(trifluoromethyl) benzyl)amino)p henyl)-7,8-difluorooctanamide<br>(7R)-N-(2-cyclohexyl-4-((4-(trifluoromethyl)benzyl)amino)p henyl)-7,8-difluorooctanamide<br>(7S)- N-(2-cyclohexyl-4-((4-(trifluoromethyl)benzyl)amino)p henyl)-7,8-difluorooctanamide |

(continued)

| | COMPOUND STRUCTURE | COMPOUND NAME |
|---|---|---|
| 246. | 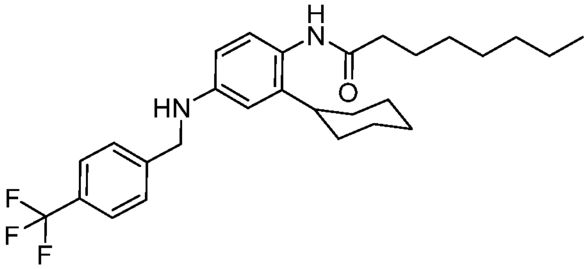 | N-(2-cyclohexyl-4-((4-(trifluoromethyl) benzyl)amino)p henyl)octanamide |
| 247. | 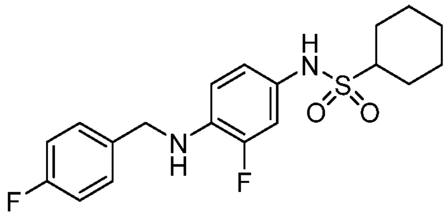 | N-(3-fluoro-4-((4-fluorobenzyl)amino) phenyl)cycl ohexanesulfonamide |
| 248. | 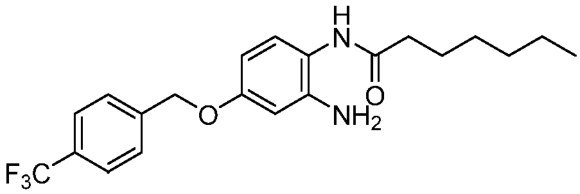 | N-(2-amino-4-((4-(trifluoromethyl)benzyl) oxy)phe nyl)heptanamide |
| 249. | 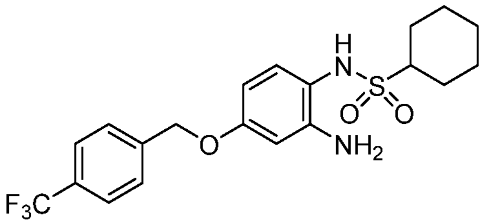 | N-(2-amino-4-((4-(trifluoromethyl)benzyl) oxy)phe nyl)cyclohexanesulfonamide |
| 250. | 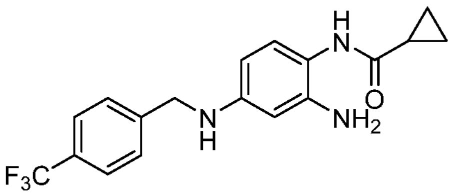 | N-(2-amino-4-((4-(trifluoromethyl)benzyl) amino)p henyl)cyclopropanecarboxamide |
| 251. | 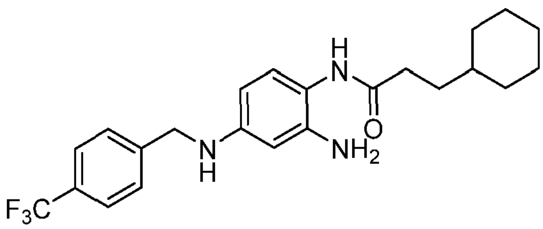 | N-(2-amino-4-((4-(trifluoromethyl)benzyl) amino)p henyl)-3-cyclohexylpropanamide |
| 252. | 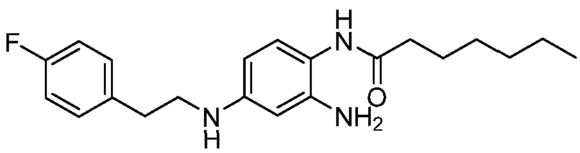 | N-(2-amino-4-((4-fluorophenethyl)amino) phenyl)h eptanamide |

(continued)

| | COMPOUND STRUCTURE | COMPOUND NAME |
|---|---|---|
| 253. | 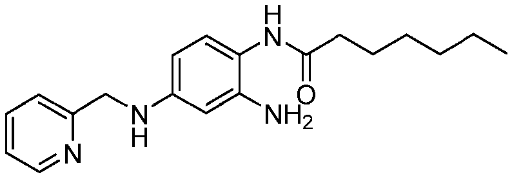 | N-(2-amino-4-((pyridin-2-ylmethyl)amino) phenyl)heptana mide |
| 254. | 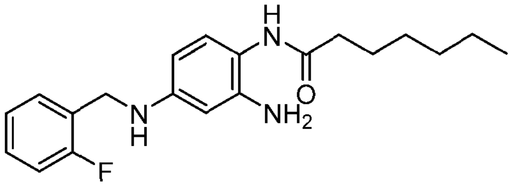 | N-(2-amino-4-((2-fluorobenzyl)amino) phenyl)hept anamide |
| 255. | 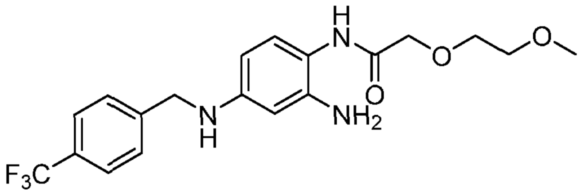 | N-(2-amino-4-((4-(trifluoromethyl)benzyl) amino)p henyl)-2-(2-methoxyethoxy) acetamide |
| 256. | 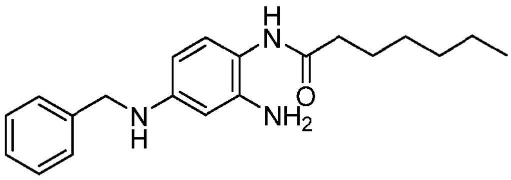 | N-(2-amino-4-(benzylamino)phenyl)hep-tanami de |
| 257. | 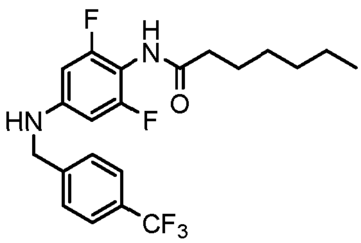 | N-(2,6-difluoro-4-((4-(trifluoromethyl)benzyl)amino)p henyl)heptanamide |
| 258. | 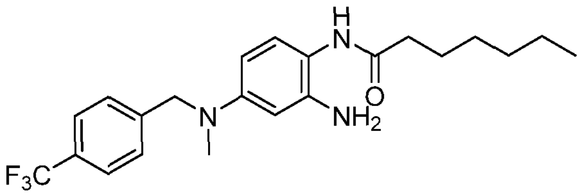 | N-(2-amino-4-(methyl(4-(trifluoromethyl) benzyl)amino)p henyl)heptanamide |
| 259. | 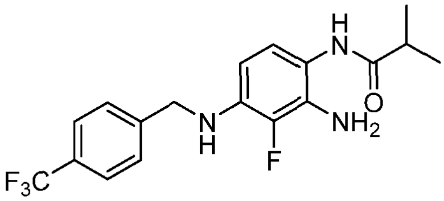 | N-(2-amino-3-fluoro-4-((4-(trifluoromethyl)benzyl)amino)p henyl)isobutyra-mide |
| 260. | 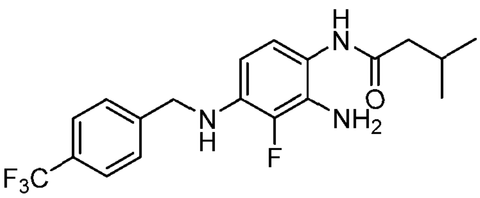 | N-(2-amino-3-fluoro-4-((4-(trifluoromethyl)benzyl)amino)p henyl)-3-methyl-butanamide |

(continued)

| | COMPOUND STRUCTURE | COMPOUND NAME |
|---|---|---|
| 261. | 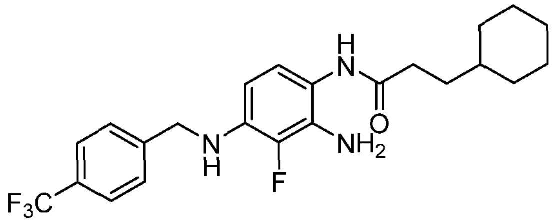 | N-(2-amino-3-fluoro-4-((4-(trifluoromethyl)benzyl)amino)p henyl)-3-cyclo-hexylpropanamide |
| 262. | 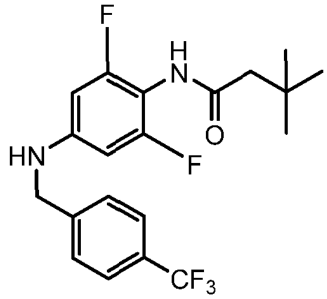 | N-(2,6-difluoro-4-((4-(trifluoromethyl)benzyl)amino)p henyl)-3,3-dimethylbutana-mide |
| 263. | 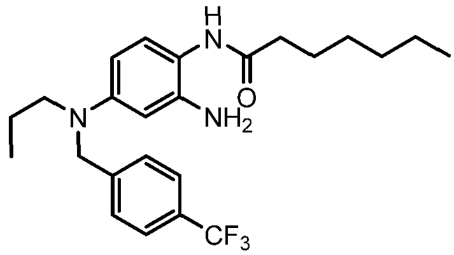 | N-(2-amino-4-(propyl(4-(trifluoromethyl) benzyl)amino)p henyl)heptanamide |
| 264. | 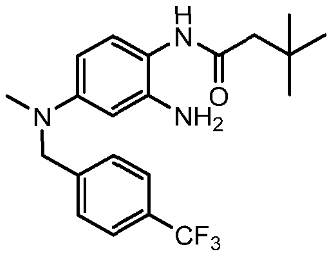 | N-(2-amino-4-(methyl(4-(trifluoromethyl) benzyl)amino)p henyl)-3,3-dimethylbuta-namide |
| 265. | 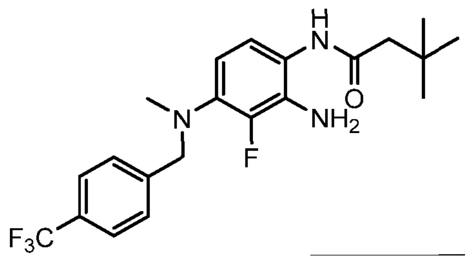 | N-(2-amino-3-fluoro-4-(methyl(4-(trifluoromethyl)benzyl)amino)p henyl)-3,3-di-methylbutanamide |
| 266. | 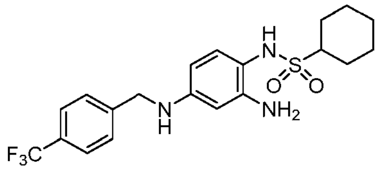 | N-(2-amino-4-((4-(trifluoromethyl)benzyl) amino)p henyl)cyclohexanesulfonamide |

(continued)

| | COMPOUND STRUCTURE | COMPOUND NAME |
|---|---|---|
| 267. | 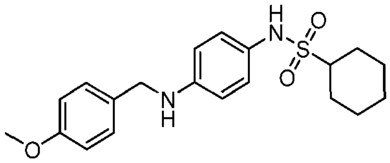 | N-(4-((4-methoxybenzyl)amino)phenyl) cyclohexanesulfonamide |
| 268. | 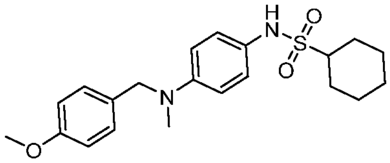 | N-(4-((4-methoxybenzyl)(methyl)amino)p henyl) cyclohexanesulfonamide |
| 269. | 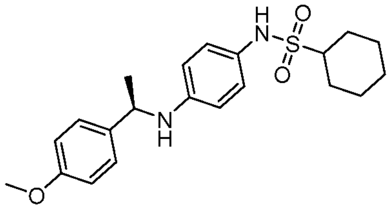 | (R)-N-(4-((1-(4-methoxyphenyl)ethyl)amino)phe nyl) cyclohexanesulfonamide |
| 270. | 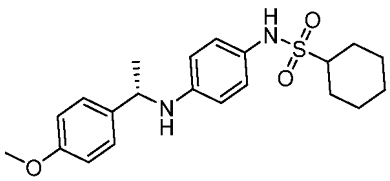 | (S)-N-(4-((1-(4-methoxyphenyl)ethyl)amino)phe nyl) cyclohexanesulfonamide |
| 271. | 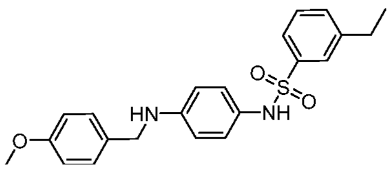 | 3-ethyl-N-(4-((4-methoxybenzyl)amino) phenyl)b enzenesulfonamide |

or a tautomer, salt, solvate, stereoisomer or isotope thereof.

**3.** The compound or tautomer, salt, solvate, stereoisomer or isotope thereof according to any one of the previous claims, wherein said compound is a voltage-gated potassium channel modulator, preferably a Kv7.1, Kv7.2, Kv7.3, Kv7.4 and/or Kv7.5 modulator.

**4.** The compound or tautomer, salt, solvate, stereoisomer or isotope thereof as defined in any one of the previous claims for use as a medicament; preferably

for use in the treatment and/or prevention of a condition **characterised by** a defect in the structure and/or function of at least one voltage-gated potassium channel, preferably at least one of Kv7. 1, Kv7.2, Kv7.3, Kv7.4 and/or Kv7.5; preferably

wherein said condition is selected from the group consisting of: a central nervous system disease, a peripheral nervous system disease, a sensory system disease, a respiratory system disease, a genitourinary system disease, a gastrointestinal system disease, a cardiovascular disease, a skeletal muscle disease and a channelopathy; preferably

wherein said condition is selected from the group consisting of: epilepsy, a convulsive disorder, a seizure, a seizure disorder, a disorder **characterised by** hyperexcitability of the nervous system, a neonatal epilepsy, spontaneous contractions, an early-onset epileptic disorder, Ohtahara syndrome, early infantile epileptic encelopathy with suppression-burst, benign familiar neonatal seizures, epileptic encelopathy, severe sporadic neonatal epileptic encelopathy, an intellectual disability, a psychiatric or cognitive comorbidity, a cognitive defect, anxiety, depression, schizophrenia, sudden death in epilepsy, drug-resistant epilepsy, neuropathic pain, an

ischemic stroke, angina, migraine, a neurodegenerative disorder, a neurologic disorder, amyotrophic lateral sclerosis, Alzheimer's disease, Parkinson's disease, tremors, paroxysmal choreoathetosis, tinnitus, pain, asthma, bladder hyper-reactivity, urinary incontinence, constipation, irritable bowel syndrome, diarrhea, Krohn's disease, hypertension, vascular hypertension, muscular dystrophy, a skeletal muscle hyperexcitability disease, myokymia, myotonia, a genetic channelopathy, a transcriptional channelopathy, a Kv7.2- and/or Kv7.3-related disorder, a Kv 7.2 encelopathy, autosomal dominant type 2 deafness (DFNA2), ataxia, episodic ataxia type 1, episodic ataxia with myokymia syndrome, neuromyotonia, hypokalaemic periodic paralysis, an arrhythmia, a congenital arrhythmia, a long QT syndrome, Romano-Ward syndrome, Jervell and Lange-Nielsen syndrome, Bartter syndrome, hyperinsulinemic hypoglycaemia of infancy (HHI), attenuate vasoconstrictor response, type 2 diabetes and multiple sclerosis.

**5.** The compound or tautomer, salt, solvate, stereoisomer or isotope thereof as defined in any one of claims 1-3 for use as an anticonvulsant, analgesic, anti-ischemic, anti-arrhythmic, neuroprotective, anti-tinnitus, antiasthmatic, gastro-intestinal motility modulator, antihypertensive, antimyotonic and/or anti-skeletal muscle dystrophia medicament.

**6.** A pharmaceutical composition comprising the compound or tautomer, salt, solvate, stereoisomer or isotope thereof as defined in any one of claims 1-3 and a pharmaceutically acceptable carrier.

**7.** The pharmaceutical composition according to claim 6 comprising a further therapeutic agent selected from: an anticonvulsivant, an analgesic and a nonsteroidal anti-inflammatory drug (NSAID), preferably said anticonvulsant is selected from the group consisting of: Acetazolamide, Brivaracetam, Carbamazepine, Clobazam, Clonazepam, Diazepam, Eslicarbazepine, Ethosuximide, Fosphenytoin, Gabapentin, Lacosamide, Lamotrigine, Levetiracetam, Lorazepam, Methosuximide, Nitrazepam, Oxcarbazepine, Perampanel, Phenobarbital, Phenytoin, Pregabalin, Primidone, Rufinamide, Stiripentol, Topiramate, Valproic Acid, Vigabatrin, Felbamate, Tiagabine and Zonisamide, preferably said analgesic is acetaminophen or an opioid, preferably said nonsteroidal anti-inflammatory drug (NSAID) is selected from the group consisting of: aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, meloxicam, nabumetone, naproxen, nimesulide, nitroflurbiprofen, olsalazine, oxaprozin, phenylbutazone, piroxicam, sulfasalazine, sulindac, tolmetin and zomepirac, preferably said opioid is morphine.

**8.** The pharmaceutical composition as defined in claim 6 or 7 for use in the treatment and/or prevention of a condition **characterised by** a defect in the structure and/or function of at least one voltage-gated potassium channel, preferably at least one of Kv7.1, Kv7.2, Kv7.3, Kv7.4 and/or Kv7.5.

**9.** The compound for use according to any one of claims 4-5 or the pharmaceutical composition for use according to claim 8 being administered to an animal, preferably a companion animal or livestock.

**10.** The compound or tautomer, salt, solvate, stereoisomer or isotope thereof as defined in any one of claims 1-3 for use in a method of modulating a voltage-gated potassium channel, preferably said voltage-gated potassium channel is Kv7.1, Kv7.2, Kv7.3, Kv7.4 and/or Kv7.5.

**11.** An *in-vitro* method of modulating a voltage-gated potassium channel by using the compound or tautomer, salt, solvate, stereoisomer or isotope thereof as defined in any one of claims 1-3, said voltage-gated potassium channel being preferably Kv7.1, Kv7.2, Kv7.3, Kv7.4 and/or Kv7.5.

## Patentansprüche

**1.** Verbindung der allgemeinen Formel I: 2.

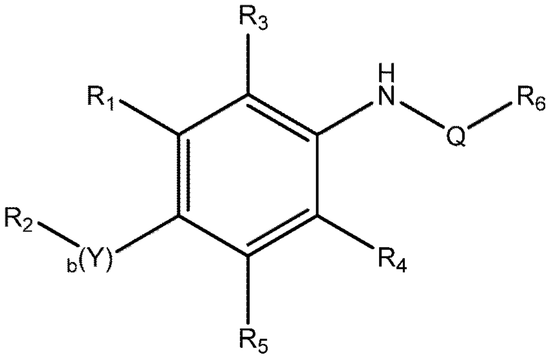

I

oder ein Tautomer, Salz, Solvat, Stereoisomer oder Isotop davon, wobei:

Y ausgewählt ist aus: C1-C10-Alkylamino, C1-C10-Alkyl, C1-C10-Alkoxy,
$R_2$ ein aromatischer Ring ist, der gegebenenfalls in einer oder mehreren Positionen mindestens einen Substituenten trägt, unabhängig ausgewählt aus der Gruppe bestehend aus: OH, $NO_2$, Halogen, $NH_2$, C1-C3-Haloalkyl, C1-C3-Haloalkoxy, C1-C6-Alkylcarbonyl, Acetamidyl, C1-C6-Alkyloxy;
$R_1$, $R_3$, $R_4$ und $R_5$ jeweils unabhängig voneinander ausgewählt sind aus der Gruppe bestehend aus: H, $NH_2$, OH, Halogen, C1-C6-Alkyl, C3-C6-Cycloalkyl, C1-C6-Alkylcarbonyl, C1-C6-Alkoxycarbonyl, C1-C6-Alkylamino, C2-C6-Dialkylamino, C1-C6-Alkylaminocarbonyl, C1-C6-Alkyloxy, C1-C6-Alkylthio, C2-C8-Alkenyl, C5-C7-Cycloalkenyl, Heterocyclus, C2-C8-Alkinyl und C5-C10-Aryl, wobei jedes davon gegebenenfalls durch einen oder mehrere Substituenten substituiert ist, die unabhängig voneinander ausgewählt sind aus: Hydroxyl, Amin, Thiol, Carboxyl, Carbonsäure und Halogen; Q ausgewählt ist aus der Gruppe bestehend aus: C=O, $SO_2$; wobei:

wenn Q für C=O steht, $R_6$ ausgewählt ist aus: linearem oder verzweigtem C3-C30-Alkyl, linearem oder verzweigtem C5-C30-Arylalkyl, C3-C8-Cycloalkyl, linearem oder verzweigtem C2-C4-Alkyl, das mit C3-C8-Cycloalkyl substituiert ist, C2-C30-Alkenyl, C2-C30-Alkinyl, und wobei jedes davon gegebenenfalls durch einen oder mehrere Substituenten substituiert ist, die unabhängig voneinander ausgewählt sind aus: Halogen und Cycloalkyl; und
wenn Q für $SO_2$ steht, $R_6$ für C3-C8-Cycloalkyl oder - Aryl steht und gegebenenfalls mit einem oder mehreren C1-C6-Alkyl substituiert ist.

**2.** Verbindung nach Anspruch 1, ausgewählt aus:

| | STRUKTUR DER VERBINDUNG | NAME DER VERBINDUNG |
|---|---|---|
| 1. | 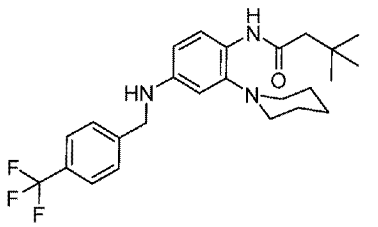 | N-(2-(Piperidin-1-yl)-4-((4-(trifluormethyl)benzyl)ami no)phenyl)heptanamid |
| 2. | 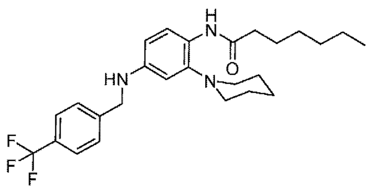 | N-(2-(Piperidin-1-yl)-4-((4-(trifluormethyl)benzyl)ami no)phenyl)heptanamid |

(continued)

| | STRUKTUR DER VERBINDUNG | NAME DER VERBINDUNG |
|---|---|---|
| 3. | 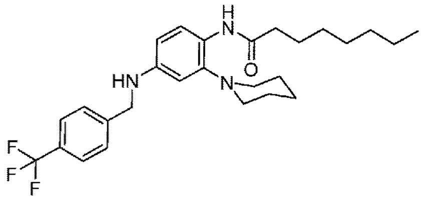 | N-(2-(Piperidin-1-yl)-4-((4-(trifluormethyl)benzyl)ami no)phenyl)octanamid |
| 4. | 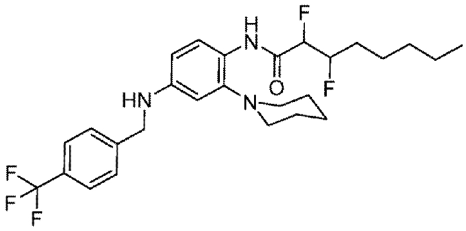 | 2,3-Difluor-N-(2-(piperidin-1-yl)-4-((4-(trifluormethyl) benzyl)ami no)phenyl)octanamid<br>(2R,3S)-2,3-Difluor-N-(2-(piperidin-1-yl)-4-((4-(trifluormethyl)benzyl)ami no)phenyl)octanamid<br>(2S,3R)-2,3-Difluor-N-(2-(piperidin-1-yl)-4-((4-(trifluormethyl)benzyl)ami no)phenyl)octanamid<br>(2R,3R)-2,3-Difluor-N-(2-(piperidin-1-yl)-4-((4-(trifluormethyl)benzyl)ami no)phenyl)octanamid<br>(2S,3S)-2,3-Difluor-N-(2-(piperidin-1-yl)-4-((4-(trifluormethyl)benzyl)ami no)phenyl)octanamid |
| 5. | 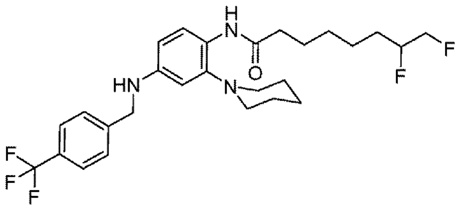 | 7,8-Difluor-N-(2-(piperidin-1-yl)-4-((4-(trifluormethyl) benzyl)ami no)phenyl)octanamid<br>(7R)-7,8-Difluor-N-(2-(piperidin-1-yl)-4-((4-(trifluormethyl)benzyl)ami no)phenyl)octanamid<br>(7S)-7,8-Difluor-N-(2-(piperidin-1-yl)-4-((4-(trifluormethyl)benzyl)ami no)phenyl)octanamid |
| 6. | 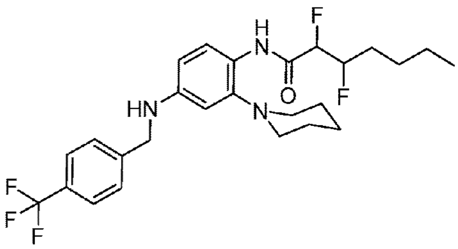 | 2,3-Difluor-N-(2-(piperidin-1-yl)-4-((4-(trifluormethyl) benzyl)ami no)phenyl)heptanamid<br>(2R,3S)-2,3-Difluor-N-(2-(piperidin-1-yl)-4-((4-(trifluormethyl)benzyl)ami no)phenyl)heptanamid<br>(2S,3R)-2,3-Difluor-N-(2-(piperidin-1-yl)-4-((4-(trifluormethyl)benzyl)ami no)phenyl)heptanamid<br>(2R,3R)-2,3-Difluor-N-(2-(piperidin-1-yl)-4-((4-(trifluormethyl)benzyl)ami no)phenyl)heptanamid<br>(2S,3S)-2,3-Difluor-N-(2-(piperidin-1-yl)-4-((4-(trifluormethyl)benzyl)ami no)phenyl)heptanamid |
| 7. | 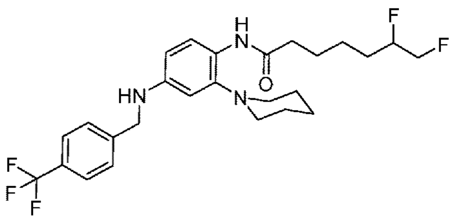 | 6,7-Difluor-N-(2-(piperidin-1-yl)-4-((4-(trifluormethyl) benzyl)ami no)phenyl)heptanamid<br>(6R)-6,7-Difluor-N-(2-(piperidin-1-yl)-4-((4-(trifluormethyl)benzyl)ami no)phenyl)heptanamid<br>(6S)-6,7-Difluor-N-(2-(piperidin-1-yl)-4-((4-(trifluormethyl)benzyl)ami no)phenyl)heptanamid |
| 8. | 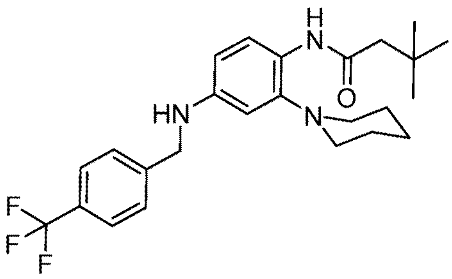 | 3,3-Dimethyl-N-(2-(piperidin-1-yl-)-4-((4-(trifluormethyl) benzyl)ami no)phenyl)butanamid |

(continued)

| | STRUKTUR DER VERBINDUNG | NAME DER VERBINDUNG |
|---|---|---|
| 9. | 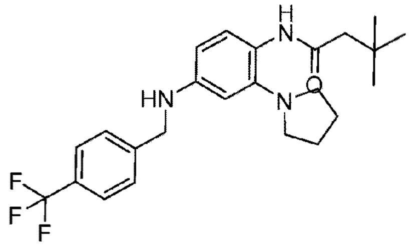 | 3,3-Dimethyl-N-(2-(pyrrolidin-1-yl)-4-((4-(trifluormethyl) benzyl)ami no)phenyl)butanamid |
| 10. | 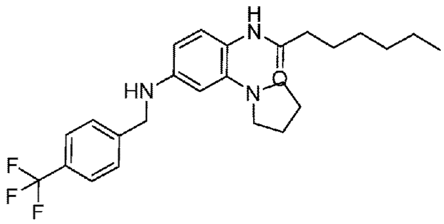 | N-(2-(Pyrrolidin-1-yl)-4-((4-(trifluormethyl)benzyl)ami no)phenyl)heptanamid |
| 11. | 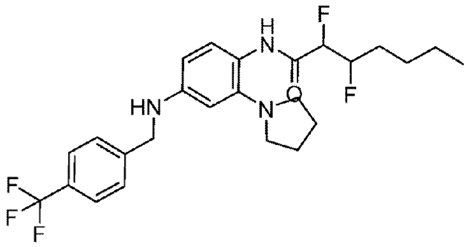 | (2R,3S)-2,3-Difluor-N-(2-pyrrolidin-1-yl)-4-((4-(trifluormethyl)benzyl)ami no)phenyl)heptanamid<br>(2S,3R)-2,3-Difluor-N-(2-pyrrolidin-1-yl)-4-((4-(trifluormethyl)benzyl)ami no)phenyl)heptanamid<br>(2R,3R)-2,3-Difluor-N-(2-pyrrolidin-1-yl)-4-((4-(trifluormethyl)benzyl)ami no)phenyl)heptanamid<br>(2S,3S)-2,3-Difluor-N-(2-pyrrolidin-1-yl)-4-((4-(trifluormethyl)benzyl)ami no)phenyl)heptanamid |
| 12. | 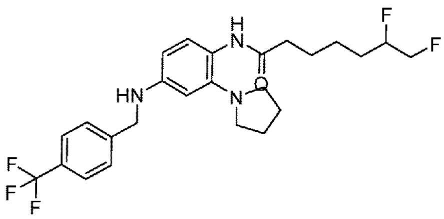 | 6,7-Difluor-N-(2-(pyrrolidin-1-yl)-((4-(trifluormethyl)benzyl)ami no)phenyl)heptanamid<br>(6R)-6,7-Difluor-N-(2-(pyrrolidin-1-yl)-((4-(trifluormethyl)benzyl)ami no)phenyl)heptanamid<br>(6S)-6,7-Difluor-N-(2-(pyrrolidin-1-yl)-((4-(trifluormethyl)benzyl)ami no)phenyl)heptanamid |
| 13. | 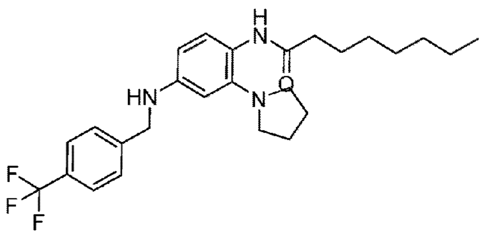 | N-(2-(Pyrrolidin-1-yl)-4-((4-(trifluormethyl)benzyl)ami no)phenyl)octanamid |
| 14. | 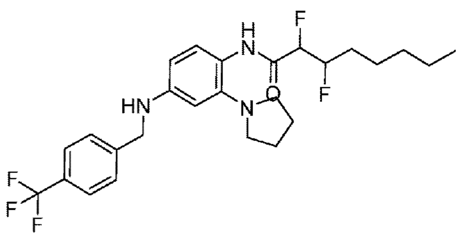 | 2,3-Difluor-N-(2-(pyrrolidin-1-yl)-4-((4-(trifluormethyl) benzyl)ami no)phenyl)octanamid<br>(2R,3S)-2,3-Difluor-N-(2-(pyrrolidin-1-yl)-4-((4-(trifluormethyl)benzyl)ami no)phenyl)octanamid<br>(2S,3R)-2,3-Difluor-N-(2-(pyrrolidin-1-yl)-4-((4-(trifluormethyl)benzyl)ami no)phenyl)octanamid<br>(2R,3R)-2,3-Difluor-N-(2-(pyrrolidin-1-yl)-4-((4-(trifluormethyl)benzyl)ami no)phenyl)octanamid<br>(2S,3S)-2,3-Difluor-N-(2-(pyrrolidin-1-yl)-4-((4-(trifluormethyl)benzyl)ami no)phenyl)octanamid |

(continued)

| | STRUKTUR DER VERBINDUNG | NAME DER VERBINDUNG |
|---|---|---|
| 15. | 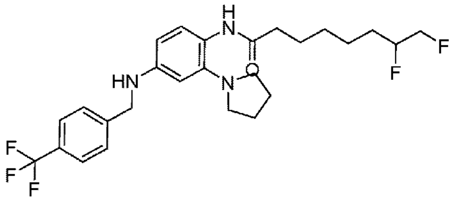 | 7,8-Difluor-N-(2-(pyrrolidin-1-yl)-4-((4-(trifluormethyl) benzyl)ami no)phenyl)octanamid<br>(7R)-7,8-Difluor-N-(2-(pyrrolidin-1-yl)-4-((4-(trifluormethyl)benzyl)ami no)phenyl)octanamid<br>(7S)-7,8-Difluor-N-(2-(pyrrolidin-1-yl)-4-((4-(trifluormethyl)benzyl)ami no)phenyl)octanamid |
| 16. | 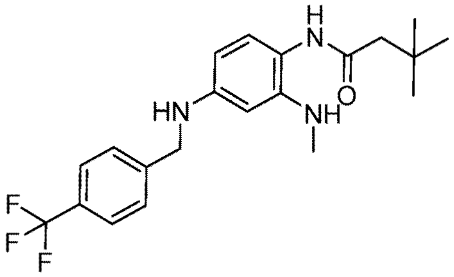 | 3,3-Dimethyl-N-(2(methylamino) -4-((4-(trifluormethyl) benzyl)ami no)phenyl)butanamid |
| 17. | 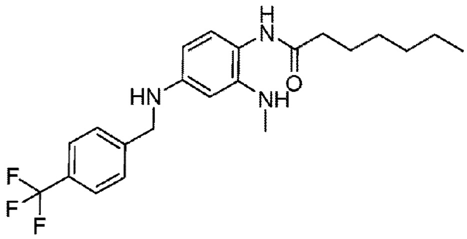 | N-(2-(Methylamino)-4-((4-(trifluormethyl)benzyl)ami no) phenyl)heptanamid |
| 18. | 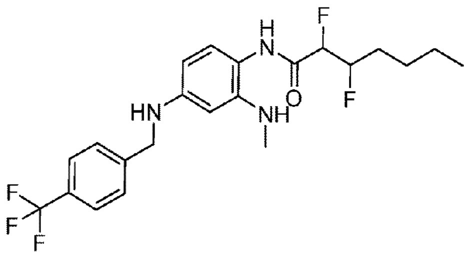 | 2,3-Difluor-N-(2-(methylamino) -4-((4-(trifluormethyl) benzyl)ami no)phenyl)heptanamid<br>(2R,3S)-2,3-Difluor-N-(2-(methylamino) -4-((4-(trifluormethyl)benzyl)ami no)phenyl)heptanamid<br>(2S,3R)-2,3-Difluor-N-(2-(methylamino) -4-((4-(trifluormethyl)benzyl)ami no)phenyl)heptanamid<br>(2R,3R)-2,3-Difluor-N-(2-(methylamino) -4-((4-(trifluormethyl)benzyl)ami no)phenyl)heptanamid<br>(2S,3S)-2,3-Difluor-N-(2-(methylamino) -4-((4-(trifluormethyl)benzyl)ami no)phenyl)heptanamid |
| 19. | 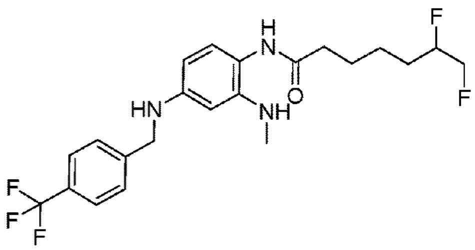 | 6,7-Difluor-N-(2-(methylamino) -4-((4-(trifluormethyl) benzyl)ami no)phenyl)heptanamid<br>(6R)-6,7-Difluor-N-(2-(methylamino) -4-((4-(trifluormethyl)benzyl)ami no)phenyl)heptanamid<br>(6S)-6,7-Difluor-N-(2-(methylamino) -4-((4-(trifluormethyl)benzyl)ami no)phenyl)heptanamid |
| 20. | 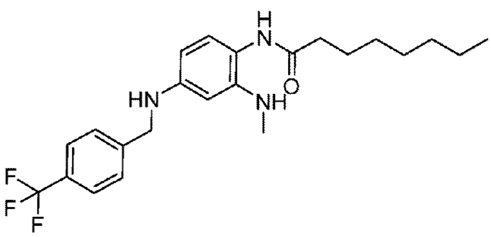 | N-(2-(Methylamino)-4-((4-(trifluormethyl)benzyl)ami no) phenyl)octanamid |

(continued)

| | STRUKTUR DER VERBINDUNG | NAME DER VERBINDUNG |
|---|---|---|
| 21. | 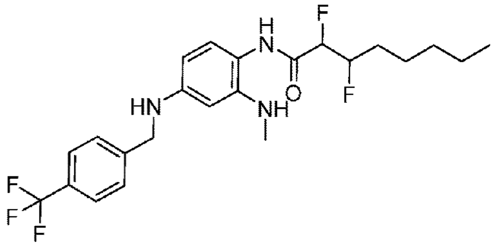 | 2,3-Difluor-N-(2(methylamino) -4-((4-(trifluormethyl) benzyl)ami no)phenyl)octanamid (2R,3S)-2,3-Difluor-N-(2-(methylamino) -4-((4-(trifluormethyl)benzyl)ami no)phenyl)octanamid (2S,3R)-2,3-Difluor-N-(2-(methylamino) -4-((4-(trifluormethyl)benzyl)ami no)phenyl)octanamid (2R,3R)-2,3-Difluor-N-(2-(methylamino) -4-((4-(trifluormethyl)benzyl)ami no)phenyl)octanamid (2S,3S)-2,3-Difluor-N-(2-(methylamino) -4-((4-(trifluormethyl)benzyl)ami no)phenyl)octanamid |
| 22. | 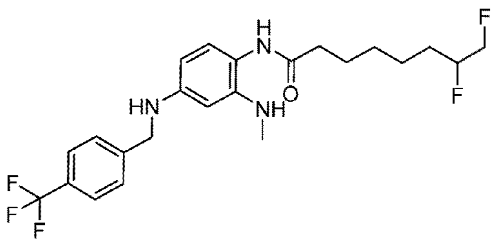 | 7,8-Difluor-N-(2(methylamino) -4-((4-(trifluormethyl) benzyl)ami no)phenyl)octanamid (7R)-7,8-Difluor-N-(2(methylamino) -4-((4-(trifluormethyl)benzyl)ami no)phenyl)octanamid (7S)-7,8-Difluor-N-(2(methylamino) -4-((4-(trifluormethyl)benzyl)ami no)phenyl)octanamid |
| 23. | 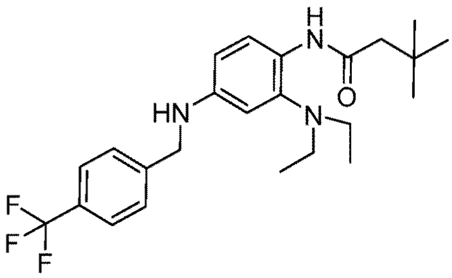 | N-(2-(Diethylamino)-4-((4-(trifluormethyl)benzyl)ami no)phenyl)-3,3-dimethylbutanamid |
| 24. | 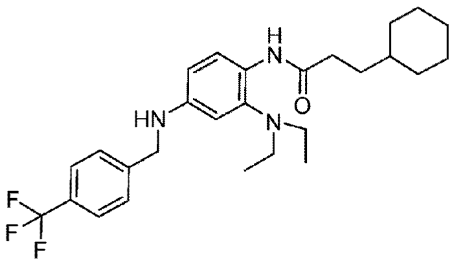 | 3-Cyclohexyl-N-(2(diethylamino)-4-((4-(trifluormethyl) benzyl)ami no)phenyl)propanamid |
| 25. | 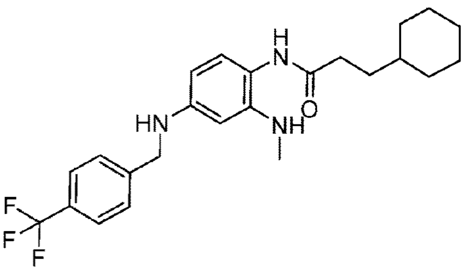 | 3-Cyclohexyl-N-(2-(methylamino) -4-((4-(trifluormethyl) benzyl)ami no)phenyl)propanamid |

(continued)

| | STRUKTUR DER VERBINDUNG | NAME DER VERBINDUNG |
|---|---|---|
| 26. | 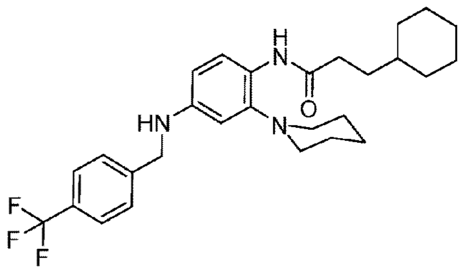 | 3-Cyclohexyl-N-(2-(piperidin-1-yl)-4-((4-(trifluormethyl) benzyl)ami no)phenyl)propanamid |
| 27. | 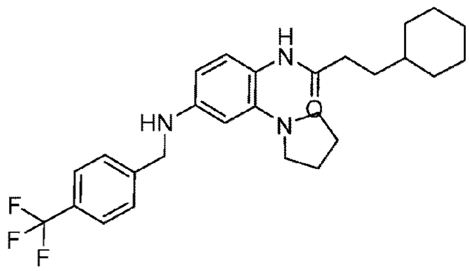 | 3-Cyclohexyl-N-(2-(piperidin-1-yl)-4-((4-(trifluormethyl) benzyl)ami no)phenyl)propanamid |
| 28. | 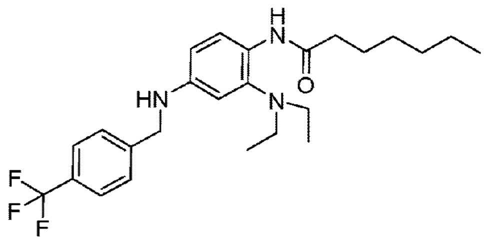 | N-(2-(Diethylamino)-4-((4-(trifluormethyl)benzyl)ami no)phenyl)heptanamid |
| 29. | 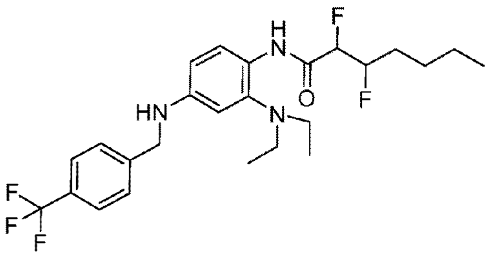 | N-(2-(Diethylamino)-4-((4-(trifluormethyl)benzyl)ami no)phenyl)-2,3-difluorheptanamid<br>(2R, 3S) -N- (2-(Diethylamino) -4-((4-(trifluormethyl) benzyl)ami no)phenyl)-2,3-difluorheptanamid<br>(2S, 3R) -N- (2-(Diethylamino) -4-((4-(trifluormethyl) benzyl)ami no)phenyl)-2,3-difluorheptanamid<br>(2R,3R)-N-(2-(Diethylamino)-4-((4-(trifluormethyl)benzyl)ami no)phenyl)-2,3-difluorheptanamid<br>(2S,3S)-N-(2-(Diethylamino)-4-((4-(trifluormethyl)benzyl)ami no)phenyl)-2,3-difluorheptanamid. |
| 30. | 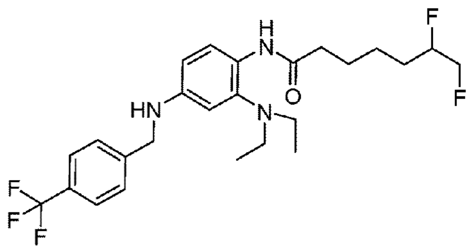 | N-(2-(Diethylamino)-4-((4-(trifluormethyl)benzyl)ami no)phenyl)-6,7-difluorheptanamid<br>(6R)-N-(2-(Diethylamino)-4-((4-(trifluormethyl)benzyl) ami no)phenyl)-6,7-difluorheptanamid<br>(6S)-N-(2-(Diethylamino)-4-((4-(trifluormethyl)benzyl) ami no)phenyl)-6,7-difluorheptanamid |
| 31. | 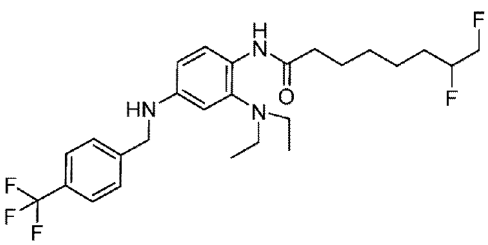 | N-(2-(Diethylamino)-4-((4-(trifluormethyl)benzyl)ami no)phenyl)-7,8-difluoroctanamid<br>(7R)-N-(2-(Diethylamino)-4-((4-(trifluormethyl)benzyl) ami no)phenyl)-7,8-difluoroctanamid<br>(7S)-N-(2-(Diethylamino)-4-((4-(trifluormethyl)benzyl) amino)phenyl)-7,8-difluoroctanamid |

(continued)

| | STRUKTUR DER VERBINDUNG | NAME DER VERBINDUNG |
|---|---|---|
| 32. | 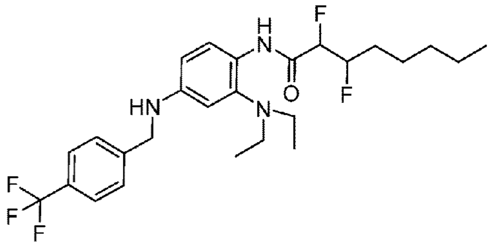 | N-(2-(Diethylamino)-4-((4-(trifluormethyl)benzyl)ami no)phenyl)-2,3-difluoroctanamid (2R, 3S) -N- (2-(Diethylamino) -4-((4-(trifluormethyl) benzyl)ami no)phenyl)-2,3-difluoroctanamid (2S, 3R) -N- (2-(Diethylamino) -4-((4-(trifluormethyl) benzyl)ami no)phenyl)-2,3-difluoroctanamid (2R, 3R) -N- (2-(Diethylamino) -4-((4-(trifluormethyl) benzyl)ami no)phenyl)-2,3-difluoroctanamid (2S, 3S) -N- (2-(Diethylamino) -4-((4-(trifluormethyl) benzyl)ami no)phenyl)-2,3-difluoroctanamid |
| 33. | 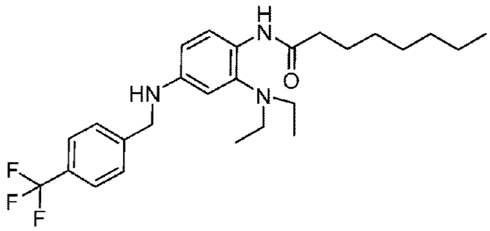 | N-(2-(Diethylamino)-4-((4-(trifluormethyl)benzyl)ami no)phenyl)octanamid |
| 34. | 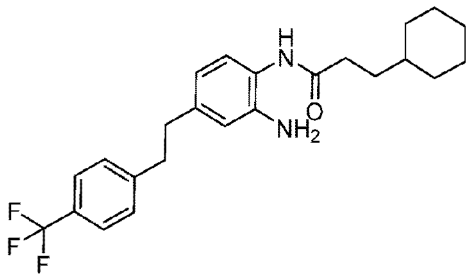 | N-(2-Amino-4-(4-(trifluormethyl)phenethyl) phenyl)-3-cyclohexylpropanamid |
| 35. | 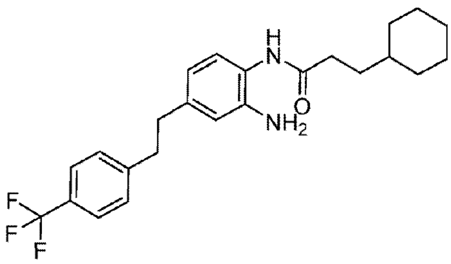 | N-(2-Amino-4-(4-(trifluormethyl)phenethyl) phenyl)-3-cyclohexylpropanamid |
| 36. | 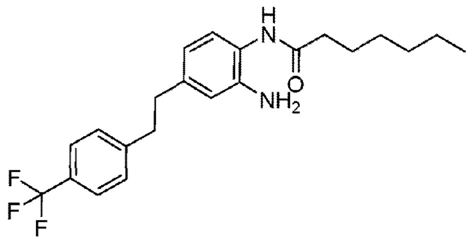 | N-(2-Amino-4-(4-(trifluormethyl)phenethyl) phenyl)heptanamid |

(continued)

| | STRUKTUR DER VERBINDUNG | NAME DER VERBINDUNG |
|---|---|---|
| 37. | 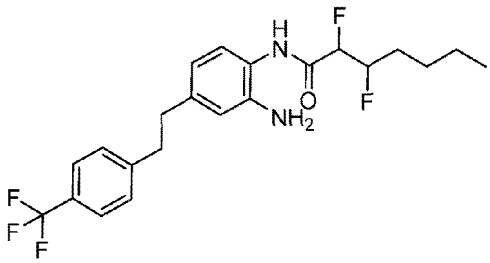 | N-(2-Amino-4-(4-(trifluormethyl)phenethyl) phenyl)-2,3-difluorheptanamid (2R,3S)-N-(2-Amino-4-(4-(trifluormethyl)phenethyl) phenyl)-2,3-difluorheptanamid (2S,3R)-N-(2-Amino-4-(4-(trifluormethyl)phenethyl) phenyl)-2,3-difluorheptanamid (2R,3R)-N-(2-Amino-4-(4-(trifluormethyl)phenethyl) phenyl)-2,3-difluorheptanamid (2S,3S)-N-(2-Amino-4-(4-(trifluormethyl)phenethyl) phenyl)-2,3-difluorheptanamid |
| 38. | 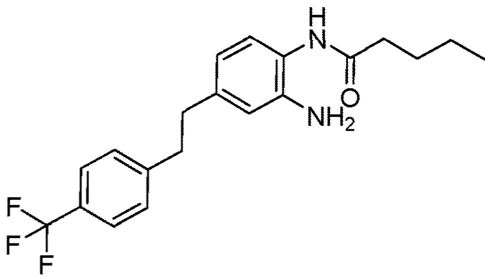 | N-(2-Amino-4-(4-(trifluormethyl)phenethyl) phenyl)-6,7-difluorheptanamid (6R)-N-(2-Amino-4-(4-(trifluormethyl)phenethyl)phenyl)-6,7-difluorheptanamid (6S)-N-(2-Amino-4-(4-(trifluormethyl)phenethyl) phenyl)-6,7-difluorheptanamid |
| 39. | 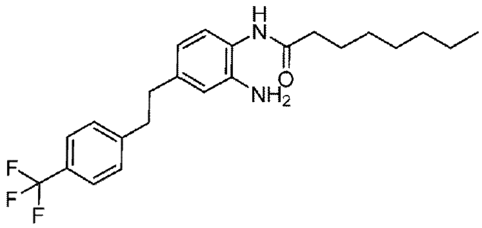 | N-(2-Amino-4-(4-(trifluormethyl)phenethyl) phenyl)octanamid |
| 40. | 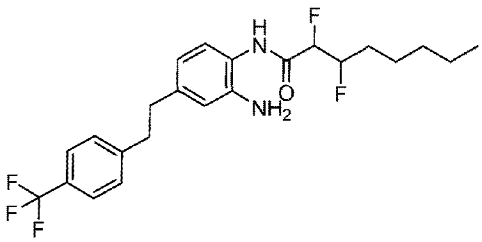 | N-(2-Amino-4-(4-(trifluormethyl)phenethyl) phenyl)-2,3-difluoroctanamid |
| 41. | 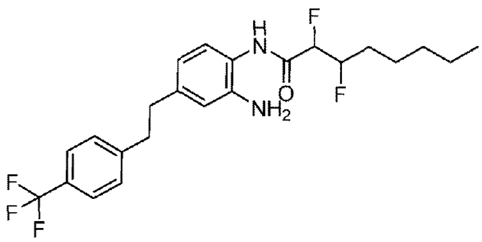 | N-(2-Amino-4-(4-(trifluormethyl)phenethyl) phenyl)-2,3-difluoroctanamid (2R,3S)-N-(2-Amino-4-(4-(trifluormethyl)phenethyl) phenyl)-2,3-difluoroctanamid (2S,3R)-N-(2-Amino-4-(4-(trifluormethyl)phenethyl) phenyl)-2,3-difluoroctanamid (2R,3R)-N-(2-Amino-4-(4-(trifluormethyl)phenethyl) phenyl)-2,3-difluoroctanamid (2S,3S)-N-(2-Amino-4-(4-(trifluormethyl)phenethyl) phenyl)-2,3-difluoroctanamid |
| 42. | 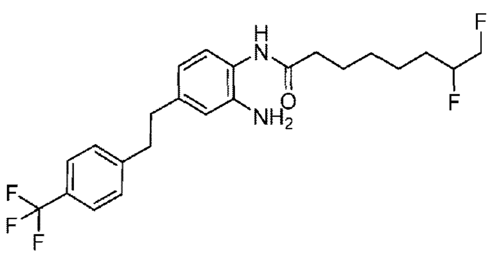 | N-(2-Amino-4-(4-(trifluormethyl)phenethyl) phenyl)-7,8-difluoroctanamid (7R)-N-(2-Amino-4-(4-(trifluormethyl)phenethyl) phenyl)-7,8-difluoroctanamid (7S)-N-(2-Amino-4-(4-(trifluormethyl)phenethyl) phenyl)-7,8-difluoroctanamid |

(continued)

| | STRUKTUR DER VERBINDUNG | NAME DER VERBINDUNG |
|---|---|---|
| 43. | 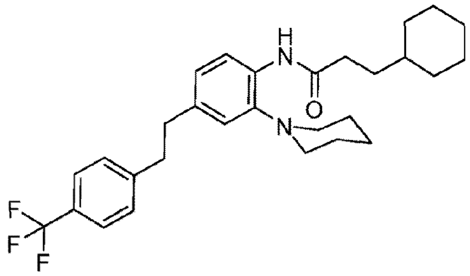 | 3-Cyclohexyl-N-(2-piperidin-1-yl)-4(4-(trifluormethyl) phenethyl) phenyl)propanamid |
| 44. | 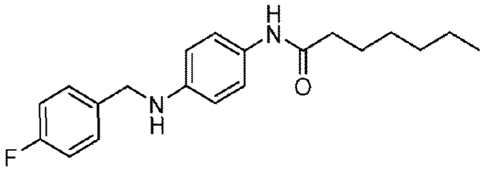 | N-(4-((4-Fluorbenzyl)amino)phenyl)h eptanamid |
| 45. | 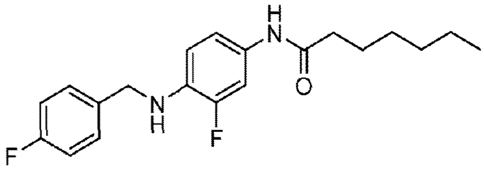 | N-(3-Fluor-4-((4-fluorbenzyl)amino)phenyl)h eptanamid |
| 46. | 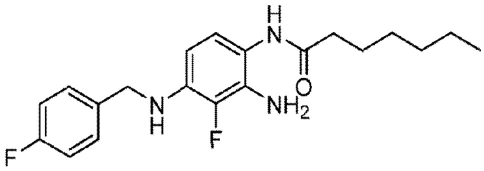 | N-(2-Amino-3-fluor-4-((4-fluorbenzyl)amino)phenyl)h eptanamid |
| 47. | 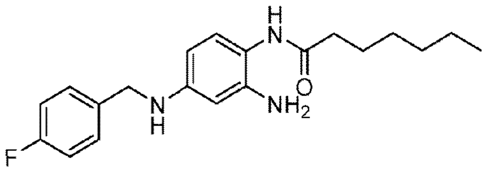 | N-(2-Amino-4-((4-fluorbenzyl)amino)phenyl)h eptan-amid |
| 48. | 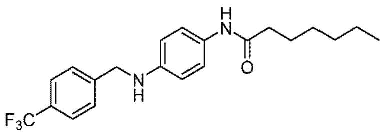 | N-(4-((4-(Trifluormethyl)benzyl)ami no)phenyl)heptan-amid |
| 49. | 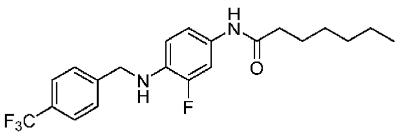 | N-(3-Fluor-4-((4-(trifluormethyl)benzyl)ami no)phenyl) heptanamid |
| 50. | 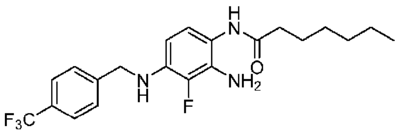 | N-(2-Amino-3-fluor-4-((4-(trifluormethyl)benzyl)ami no) phenyl)heptanamid |
| 51. | 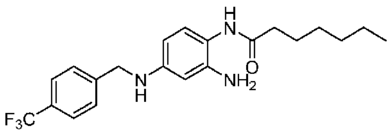 | N-(2-Amino-4-((4-(trifluormethyl)benzyl)ami no)phenyl) heptanamid |

(continued)

| | STRUKTUR DER VERBINDUNG | NAME DER VERBINDUNG |
|---|---|---|
| 52. | 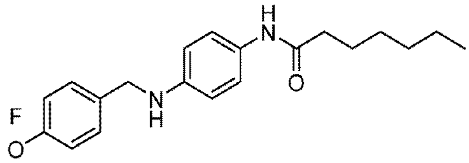 | N-(4-((4-(Trifluormethoxy)benzyl)am ino)phenyl)heptanamid |
| 53. | 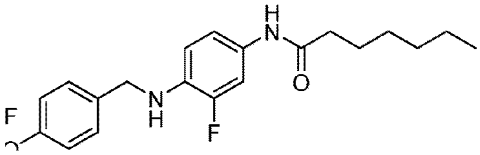 | N-(3-Fluor-4-((4-(trifluormethoxy)benzyl)am ino)phenyl) heptanamid |
| 54. | 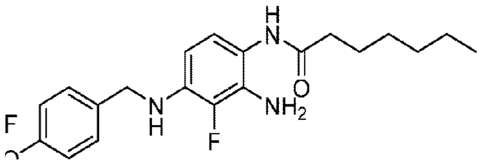 | N-(2-Amino-3-fluor-4-((4-(trifluormethoxy)benzyl)am ino)phenyl)heptanamid |
| 55. | 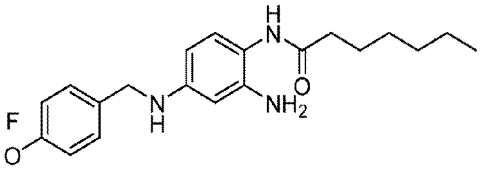 | N-(2-Amino-4-((4-(trifluormethoxy)benzyl)am ino)phenyl)heptanamid |
| 56. | 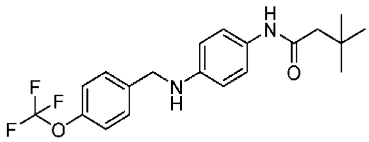 | 3,3-Dimethyl-N-(4-((4-(trifluormethoxy)benzyl)am ino) phenyl)butanamid |
| 57. | 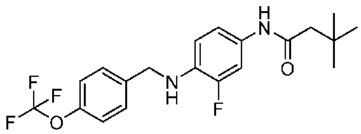 | N-(3-Fluor-4-((4-(trifluormethoxy)benzyl)am ino)phenyl)-3,3-dimethylbutanamid |
| 58. | 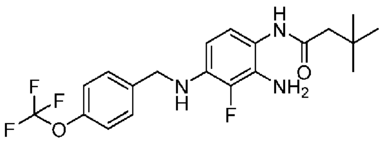 | N-(2-Amino-3-fluor-4-((4-(trifluormethoxy)benzyl)am ino)phenyl)-3,3-dimethylbutanamid |
| 59. | 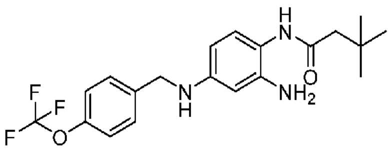 | N-(2-Amino-4-((4-(trifluormethoxy)benzyl)am ino)phenyl)-3,3-dimethylbutanamid |
| 60. | 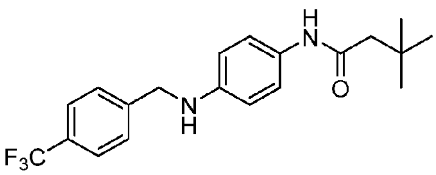 | 3,3-Dimethyl-N-(4-((4-(trifluormethyl)benzyl)ami no) phenyl)butanamid |

(continued)

| | STRUKTUR DER VERBINDUNG | NAME DER VERBINDUNG |
|---|---|---|
| 61. | 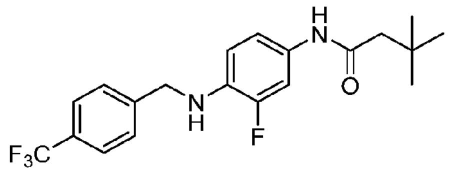 | N-(3-Fluor-4-((4-(trifluormethyl)benzyl)ami no)phenyl)-3,3-dimethylbutanamid |
| 62. | 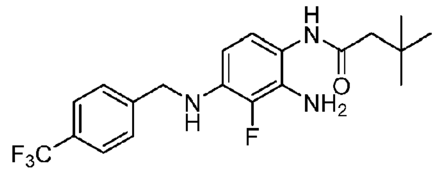 | N-(2-Amino-3-fluor-4-((4-(trifluormethyl)benzyl)ami no) phenyl)-3,3-dimethylbutanamid |
| 63. | 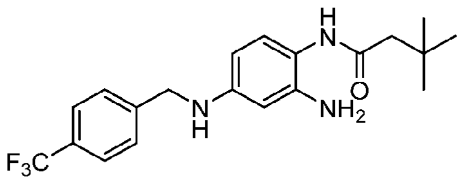 | N-(2-Amino-4-((4-(trifluormethyl)benzyl)ami no)phenyl)-3,3-dimethylbutanamid |
| 64. | 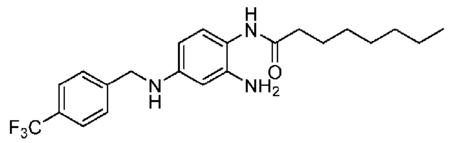 | N-(2-Amino-4-((4-(trifluormethyl)benzyl)ami no)phenyl) octanamid |
| 65. | 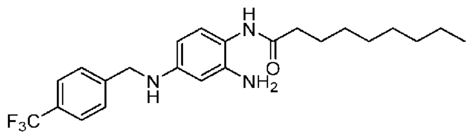 | N-(2-Amino-4-((4-(trifluormethyl)benzyl)ami no)phenyl) nonanamid |
| 66. | 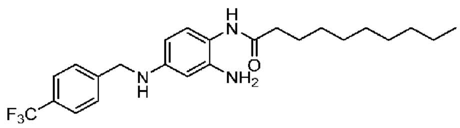 | N-(2-Amino-4-((4-(trifluormethyl)benzyl)ami no)phenyl) decanamid |
| 67. | 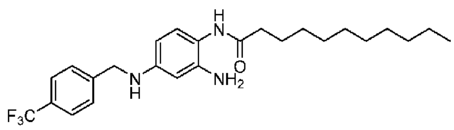 | N-(2-Amino-4-((4-(trifluormethyl)benzyl)ami no)phenyl) undecanamid |
| 68. | 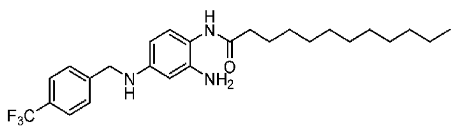 | N-(2-Amino-4-((4-(trifluormethyl)benzyl)amino)phenyl) dodecanamid |
| 69. | 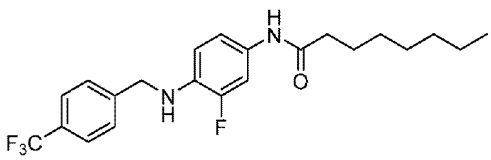 | N-(3-Fluor-4-((4-(trifluormethyl)benzyl)ami no)phenyl) octanamid |
| 70. | 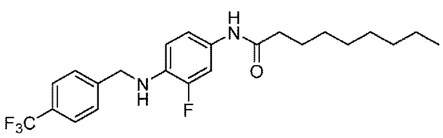 | N-(3-Fluor-4-((4-(trifluormethyl)benzyl)ami no)phenyl) nonanamid |

(continued)

| | STRUKTUR DER VERBINDUNG | NAME DER VERBINDUNG |
|---|---|---|
| 71. | 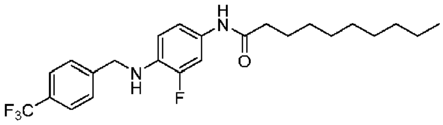 | N-(3-Fluor-4-((4-(trifluormethyl)benzyl)ami no)phenyl) decanamid |
| 72. | 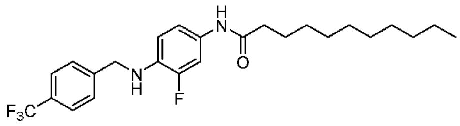 | N-(3-Fluor-4-((4-(trifluormethyl)benzyl)ami no)phenyl) undecanamid |
| 73. | 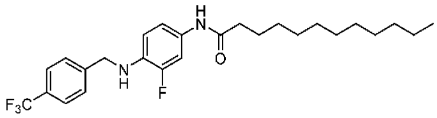 | N-(3-Fluor-4-((4-(trifluormethyl)benzyl)ami no)phenyl) dodecanamid |
| 74. | 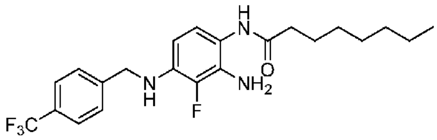 | N-(2-Amino-3-fluor-4-((4-(trifluormethyl)benzyl)ami no) phenyl)octanamid |
| 75. | 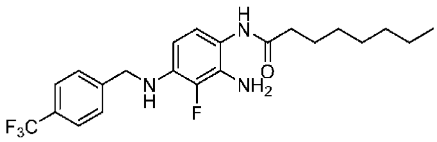 | N-(2-Amino-3-fluor-4-((4-(trifluormethyl)benzyl)ami no) phenyl)nonanamid |
| 76. | 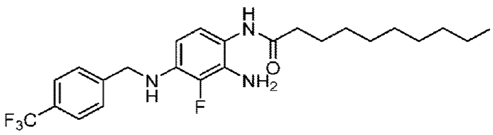 | N-(2-Amino-3-fluor-4-((4-(trifluormethyl)benzyl)ami no) phenyl)decanamid |
| 77. | 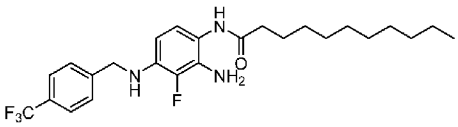 | N-(2-Amino-3-fluor-4-((4-(trifluormethyl)benzyl)ami no) phenyl)undecanamid |
| 78. | 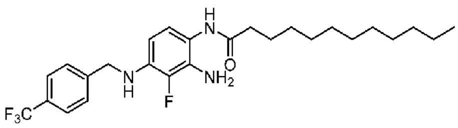 | N-(2-Amino-3-fluor-4-((4-(trifluormethyl)benzyl)ami no) phenyl)dodecanamid |
| 79. | 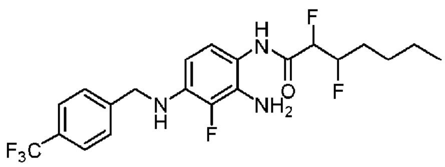 | N-(2-Amino-3-fluor-4-((4-(trifluormethyl)benzyl)ami no) phenyl)-2,3-difluorheptanamid<br>(2R,3S)-N-(2-Amino-3-fluor-4-((4-(trifluormethyl)benzyl)ami no)phenyl)-2,3-difluorheptanamid<br>(2S,3R)-N-(2-Amino-3-fluor-4-((4-(trifluormethyl)benzyl)ami no)phenyl)-2,3-difluorheptanamid<br>(2R,3R)-N-(2-Amino-3-fluor-4-((4-(trifluormethyl)benzyl)ami no)phenyl)-2,3-difluorheptanamid<br>(2S,3S)-N-(2-Amino-3-fluor-4-((4-(trifluormethyl)benzyl)ami no)phenyl)-2,3-difluorheptanamid razemische Gemische davon |

(continued)

| | STRUKTUR DER VERBINDUNG | NAME DER VERBINDUNG |
|---|---|---|
| 80. | 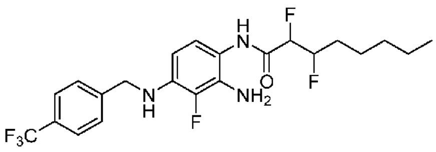 | N-(2-Amino-3-fluor-4-((4-(trifluormethyl)benzyl)ami no) phenyl)-2,3-difluoroctanamid (2R,3S)-N-(2-Amino-3-fluor-4-((4-(trifluormethyl)benzyl)ami no)phenyl)-2,3-difluoroctanamid (2S,3R)-N-(2-Amino-3-fluor-4-((4-(trifluormethyl)benzyl)ami no)phenyl)-2,3-difluoroctanamid (2R,3R)-N-(2-Amino-3-fluor-4-((4-(trifluormethyl)benzyl)ami no)phenyl)-2,3-difluoroctanamid (2S,3S)-N-(2-Amino-3-fluor-4-((4-(trifluormethyl)benzyl)ami no)phenyl)-2,3-difluoroctanamid razemische Gemische davon |
| 81. | 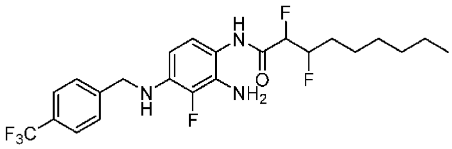 | N-(2-Amino-3-fluor-4-((4-(trifluormethyl)benzyl)ami no) phenyl)-2,3-difluornonanamid (2R,3S)-N-(2-Amino-3-fluor-4-((4-(trifluormethyl)benzyl)ami no)phenyl)-2,3-difluornonanamid (2S,3R)-N-(2-Amino-3-fluor-4-((4-(trifluormethyl)benzyl)ami no)phenyl)-2,3-difluornonanamid (2R,3R) -N-(2-Amino-3-fluor-4-((4-(trifluormethyl)benzyl)ami no)phenyl)-2,3-difluornonanamid (2S,3R)-N-(2-Amino-3-fluor-4-((4-(trifluormethyl)benzyl)amino)phenyl)-2,3-difluornonanamid razemische Gemische davon |
| 82. | 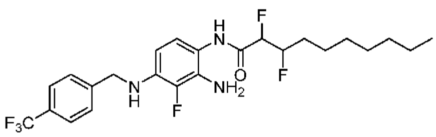 | N-(2-Amino-3-fluor-4-((4-(trifluormethyl)benzyl)ami no) phenyl)-2,3-difluordecanamid (2R,3S)-N-(2-Amino-3-fluor-4-((4-(trifluormethyl)benzyl)ami no)phenyl)-2,3-difluordecanamid (2S,3R)-N-(2-Amino-3-fluor-4-((4-(trifluormethyl)benzyl)ami no)phenyl)-2,3-difluordecanamid (2R,3R)-N-(2-Amino-3-fluor-4-((4-(trifluormethyl)benzyl)ami no)phenyl)-2,3-difluordecanamid (2S,3S)-N-(2-Amino-3-fluor-4-((4-(trifluormethyl)benzyl)ami no)phenyl)-2,3-difluordecanamid razemische Gemische davon |
| 83. | 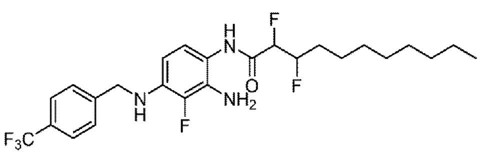 | N-(2-Amino-3-fluor-4-((4-(trifluormethyl)benzyl)ami no) phenyl)-2,3-difluorundecanamid (2R,3S)-N-(2-Amino-3-fluor-4-((4-(trifluormethyl)benzyl)ami no)phenyl)-2,3-difluorundecanamid (2S,3R)-N-(2-Amino-3-fluor-4-((4-(trifluormethyl)benzyl)ami no)phenyl)-2,3-difluorundecanamid (2R,3R)-N-(2-Amino-3-fluor-4-((4-(trifluormethyl)benzyl)ami no)phenyl)-2,3-difluorundecanamid (2S,3S)-N-(2-Amino-3-fluor-4-((4-(trifluormethyl)benzyl)ami no)phenyl)-2,3-difluorundecanamid razemische Gemische davon |

(continued)

| | STRUKTUR DER VERBINDUNG | NAME DER VERBINDUNG |
|---|---|---|
| 84. | 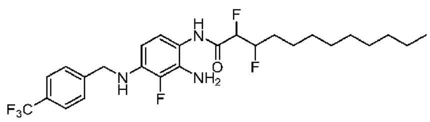 | N-(2-Amino-3-fluor-4-((4-(trifluormethyl)benzyl)ami no) phenyl)-2,3-difluordodecanamid (2R,3S)-N-(2-Amino-3-fluor-4-((4-(trifluormethyl)benzyl)ami no)phenyl)-2,3-difluordodecanamid (2S,3R)-N-(2-Amino-3-fluor-4-((4-(trifluormethyl)benzyl)ami no)phenyl)-2,3-difluordodecanamid (2R,3R)-N-(2-Amino-3-fluor-4-((4-(trifluormethyl)benzyl)ami no)phenyl)-2,3-difluordodecanamid (2S,3S)-N-(2-Amino-3-fluor-4-((4-(trifluormethyl)benzyl)ami no)phenyl)-2,3-difluordodecanamid razemische Gemische davon |
| 85. | 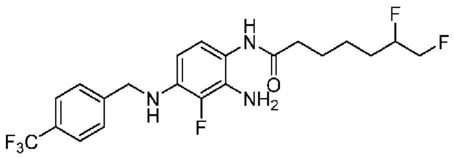 | N-(2-Amino-3-fluor-4-((4-(trifluormethyl)benzyl)ami no) phenyl)-6,7-difluorheptanamid (6R)-N-(2-Amino-3-fluor-4-((4-(trifluormethyl)benzyl) ami no)phenyl)-6,7-difluorheptanamid (6S)-N-(2-Amino-3-fluor-4-((4-(trifluormethyl)benzyl) ami no)phenyl)-6,7-difluorheptanamid razemische Gemische davon |
| 86. | 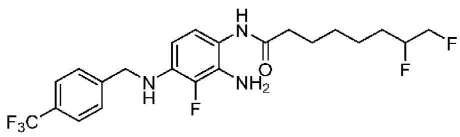 | N-(2-Amino-3-fluor-4-((4-(trifluormethyl)benzyl)ami no) phenyl)-6,7-difluoroctanamid (6R)-N-(2-Amino-3-fluor-4-((4-(trifluormethyl)benzyl) ami no)phenyl)-6,7-difluoroctanamid (6S)-N-(2-Amino-3-fluor-4-((4-(trifluormethyl)benzyl) ami no)phenyl)-6,7-difluoroctanamid razemische Gemische davon |
| 87. | 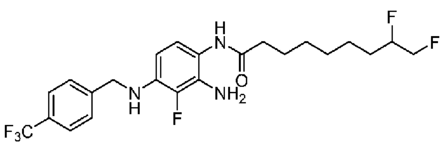 | N-(2-Amino-3-fluor-4-((4-(trifluormethyl)benzyl)ami no) phenyl)-8,9-difluornonanamid (8R) -N-(2-Amino-3-fluor-4-((4-(trifluormethyl)benzyl) ami no)phenyl)-8,9-difluornonanamid (8S)-N-(2-Amino-3-fluor-4-((4-(trifluormethyl)benzyl) ami no)phenyl)-8,9-difluornonanamid razemische Gemische davon |
| 88. | 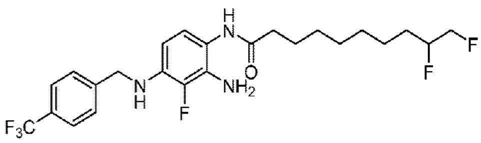 | N-(2-Amino-3-fluor-4-((4-(trifluormethyl)benzyl)ami no) phenyl)-9,10-difluordecanamid (9R)-N-(2-Amino-3-fluor-4-((4-(trifluormethyl)benzyl) ami no)phenyl)-9,10-difluordecanamid (9S)-N-(2-Amino-3-fluor-4-((4-(trifluormethyl)benzyl) ami no)phenyl)-9,10-difluordecanamid razemische Gemische davon |
| 89. | 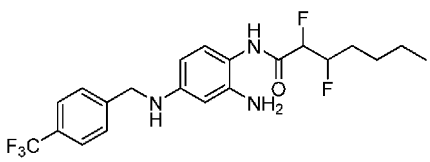 | N-(2-Amino-4-((4-(trifluormethyl)benzyl)ami no)phenyl)-2,3-difluorheptanamid (2R,3S)-N-(2-Amino-4-((4-(trifluormethyl)benzyl)amino) phenyl)-2,3-difluorheptanamid (2S,3R)-N-(2-Amino-4-((4-(trifluormethyl)benzyl)ami no)phenyl)-2,3-difluorheptanamid (2R,3R)-N-(2-Amino-4-((4-(trifluormethyl)benzyl)ami no)phenyl)-2,3-difluorheptanamid (2S,3S)-N-(2-Amino-4-((4-(trifluormethyl)benzyl)ami no)phenyl)-2,3-difluorheptanamid razemische Gemische davon |

(continued)

| | STRUKTUR DER VERBINDUNG | NAME DER VERBINDUNG |
|---|---|---|
| 90. | 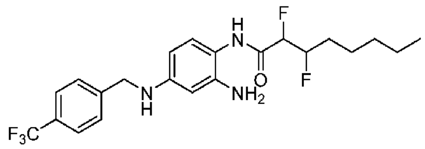 | N-(2-Amino-4-((4-(trifluormethyl)benzyl)ami no)phenyl)-2,3-difluoroctanamid (2R,3S)-N-(2-Amino-4-((4-(trifluormethyl)benzyl)ami no)phenyl)-2,3-difluoroctanamid (2S,3R)-N-(2-Amino-4-((4-(trifluormethyl)benzyl)ami no)phenyl)-2,3-difluoroctanamid (2R,3R)-N-(2-Amino-4-((4-(trifluormethyl)benzyl)ami no)phenyl)-2,3-difluoroctanamid (2S,3S)-N-(2-Amino-4-((4-(trifluormethyl)benzyl)ami no)phenyl)-2,3-difluoroctanamid razemische Gemische davon |
| 91. | 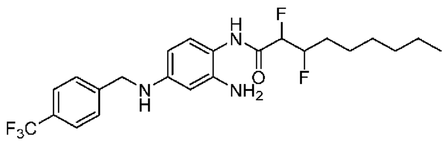 | N-(2-Amino-4-((4-(trifluormethyl)benzyl)ami no)phenyl)-2,3-difluornonanamid (2R,3S)-N-2-Amino-4-((4-(trifluormethyl)benzyl)ami no) phenyl)-2,3-difluornonanamid (2S,3R)-N-(2-Amino-4-((4-(trifluormethyl)benzyl)ami no)phenyl)-2,3-difluornonanamid (2R,3R)-N-(2-Amino-4-((4-(trifluormethyl)benzyl)ami no)phenyl)-2,3-difluornonanamid (2S,3S)-N-(2-Amino-4-((4-(trifluormethyl)benzyl)ami no)phenyl)-2,3-difluornonanamid razemische Gemische davon |
| 92. | 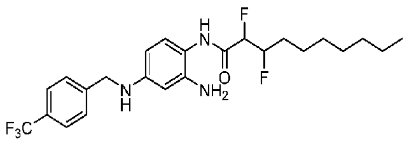 | N-(2-Amino-4-((4-(trifluoromethyl)benzyl)am ino)phenyl)-2,3-difluordecanamid (2R,3S)-N-(2-Amino-4((4-(trifluormethyl)benzyl)ami no) phenyl)-2,3-difluordecanamid (2S,3R)-N-(2-Amino-4((4-(trifluormethyl)benzyl)ami no) phenyl)-2,3-difluordecanamid (2R,3R)-N-(2-Amino-4((4-(trifluormethyl)benzyl)ami no) phenyl)-2,3-difluordecanamid (2S,3S)-N-(2-Amino-4((4-(trifluormethyl)benzyl)ami no) phenyl)-2,3-difluordecanamid razemische Gemische davon |
| 93. | 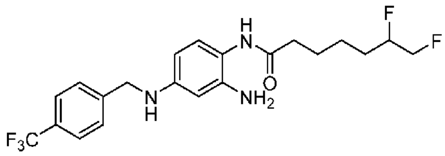 | N-(2-Amino-4-((4-(trifluormethyl)benzyl)ami no)phenyl)-6,7-difluorheptanamid (6R)-N-(2-Amino-4-((4-(trifluormethyl)benzyl)ami no) phenyl)-6,7-difluorheptanamid (6S)-N-(2-Amino-4-((4-(trifluormethyl)benzyl)ami no) phenyl)-6,7-difluorheptanamid razemische Gemische davon |
| 94. | 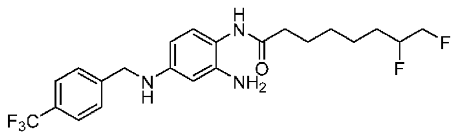 | N-(2-Amino-4-((4-(trifluormethyl)benzyl)ami no)phenyl)-7,8-difluoroctanamid (7R)-N-(2-Amino-4-((4-(trifluormethyl)benzyl)ami no) phenyl)-7,8-difluoroctanamid (7S)-N-(2-Amino-4-((4-(trifluormethyl)benzyl)ami no) phenyl)-7,8-difluoroctanamid razemische Gemische davon |

(continued)

| | STRUKTUR DER VERBINDUNG | NAME DER VERBINDUNG |
|---|---|---|
| 95. | 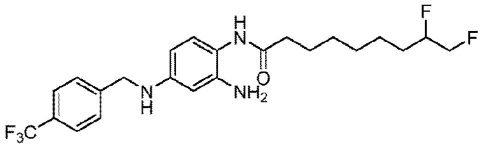 | N-(2-Amino-3-fluor-4-((4-(trifluormethyl)benzyl)ami no) phenyl)-6,7-difluornonanamid (6R)-N-(2-Amino-3-fluor-4-((4-(trifluormethyl)benzyl) ami no)phenyl)-6,7-difluornonanamid (6S)-N-(2-Amino-3-fluor-4-((4-(trifluormethyl)benzyl) ami no)phenyl)-6,7-difluornonanamid razemische Gemische davon |
| 96. | 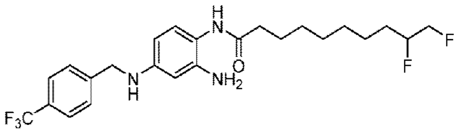 | N-(2-Amino-3-fluor-4-((4-(trifluormethyl)benzyl)ami no) phenyl)-6,7-difluordodecanamid (6R)-N-(2-Amino-3-fluor-4-((4-(trifluormethyl)benzyl) ami no)phenyl)-6,7-difluordodecanamid (6S)-N-(2-Amino-3-fluor-4-((4-(trifluormethyl)benzyl) ami no)phenyl)-6,7-difluordodecanamid razemische Gemische davon |
| 97. | 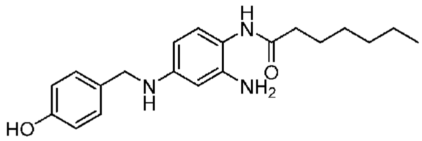 | N-(2-Amino-4-((4-hydroxybenzyl)amino)phenyl )heptanamid |
| 98. | 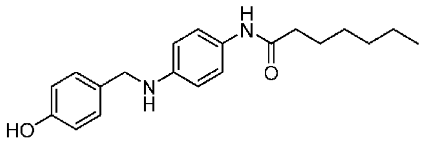 | N-(4-((4-Hydroxybenzyl)amino)phenyl )heptanamid |
| 99. | 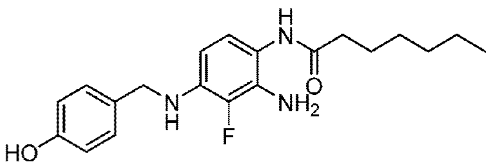 | N-(2-Amino-3-fluor-4-((4-hydroxybenzyl)amino)phenyl ) heptanamid |
| 100. | 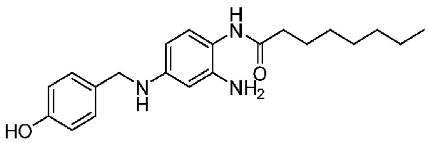 | N-(2-Amino-4-((4-hydroxybenzyl)amino)phenyl )octanamid |
| 101. | 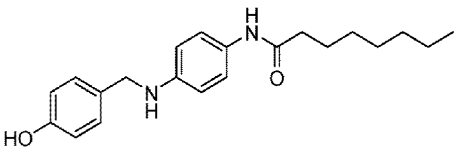 | N-(4-((4-Hydroxybenzyl)amino)phenyl )octanamid |
| 102. | 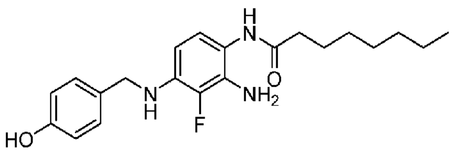 | N-(2-Amino-3-fluor-4-((4-hydroxybenzyl)amino)phenyl ) octanamid |
| 103. | 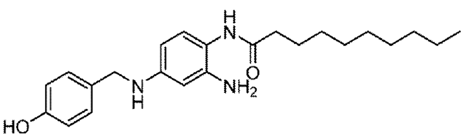 | N-(2-Amino-4-((4-hydroxybenzyl)amino)phenyl )decanamid |

(continued)

| | STRUKTUR DER VERBINDUNG | NAME DER VERBINDUNG |
|---|---|---|
| 104. | 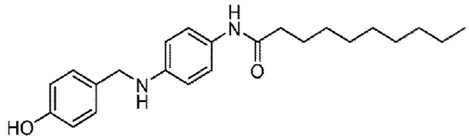 | N-(4-((4-Hydroxybenzyl)amino)phenyl )decanamid |
| 105. | 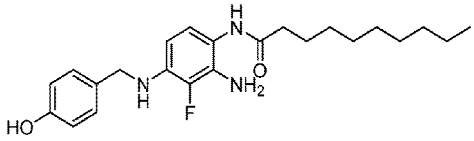 | N-(2-Amino-3-fluor-4-((4-hydroxybenzyl)amino)phenyl ) decanamid |
| 106. | 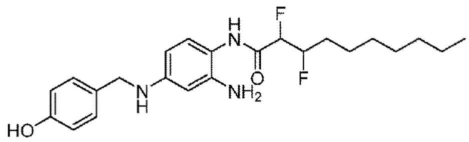 | N-(2-Amino-4-((4-hydroxybenzyl)amino)phenyl )-2,3-difluordecanamid<br>(2R,3S)-N-(2-Amino-4-((4-hydroxybenzyl)amino)phenyl )-2,3-difluordecanamid<br>(2S,3R)-N-(2-Amino-4-((4-hydroxybenzyl)amino)phenyl )-2,3-difluordecanamid<br>(2R,3R)-N-(2-Amino-4-((4-hydroxybenzyl)amino)phenyl )-2,3-difluordecanamid<br>(2S,3S)-N-(2-Amino-4-((4-hydroxybenzyl)amino)phenyl )-2,3-difluordecanamid razemische Gemische davon |
| 107. | 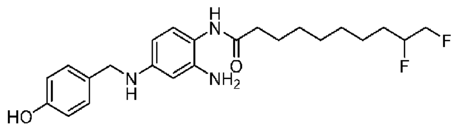 | N-(2-Amino-4-((4-hydroxybenzyl)amino)phenyl )-9,10-difluordecanamid<br>(9R)-N-(2-Amino-4-((4-hydroxybenzyl)amino)phenyl )-2,3-difluordecanamid<br>(9S)-N-(2-Amino-4-((4-hydroxybenzyl)amino)phenyl )-2,3-difluordecanamid razemische Gemische davon |
| 108. | 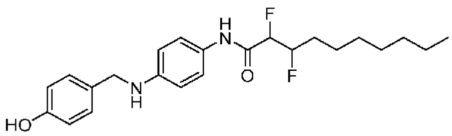 | 2,3-Difluor-N-(4-((4-hydroxybenzyl)amino)phenyl )decadecanamid<br>(2R,3S)-2,3-Difluor-N-(4-((4-hydroxybenzyl)amino)phenyl )decadecanamid<br>(2S,3R)-2,3-Difluor-N-(4-((4-hydroxybenzyl)amino)phenyl )decanamid<br>(2R,3R)-2,3-Difluor-N-(4-((4-hydroxybenzyl)amino) phenyl )decadecanamid<br>(2S,3S)-2,3-Difluor-N-(4-((4-hydroxybenzyl)amino)phenyl )decanamid razemische Gemische davon |
| 109. | 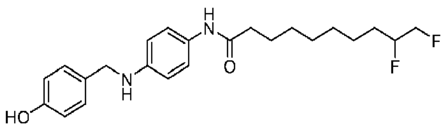 | 9,10-Difluor-N-(4-((4-hydroxybenzyl)amino)phenyl )decanamid<br>(9R)-9,10-Difluor-N-(4-((4-hydroxybenzyl)amino)phenyl)decanamid<br>(9S)-9,10-Difluor-N-(4-((4-hydroxybenzyl)amino)phenyl )decanamid razemische Gemische davon |

(continued)

| | STRUKTUR DER VERBINDUNG | NAME DER VERBINDUNG |
|---|---|---|
| 110. | 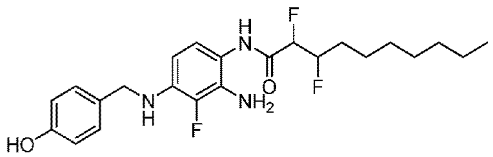 | N-(2-Amino-3-fluor-4-((4-hydroxybenzyl)amino)phenyl )-2,3-difluordecanamid (2R,3S)-N-(2-Amino-3-fluor-4-((4-hydroxybenzyl)amino)phenyl )-2,3-difluordecanamid (2S,3R)-N-(2-Amino-3-fluor-4-((4-hydroxybenzyl)amino)phenyl )-2,3-difluordecanamid (2R,3R)-N-(2-Amino-3-fluor-4-((4-hydroxybenzyl)amino)phenyl )-2,3-difluordecanamid (2S,S3)-N-(2-Amino-3-fluor-4-((4-hydroxybenzyl)amino)phenyl )-2,3-difluordecanamid razemische Gemische davon |
| 111. | 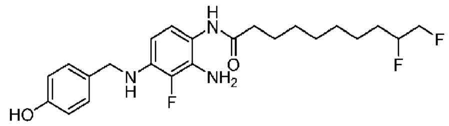 | N-(2-Amino-3-fluor-4-((4-hydroxybenzyl)amino)phenyl )-9,10-difluordecanamid (9R)-N-(2-Amino-3-fluor-4-((4-hydroxybenzyl)amino) phenyl )-9,10-difluordecanamid (9S)-N-(2-Amino-3-fluor-4-((4-hydroxybenzyl)amino) phenyl)-9,10-difluordecanamid razemische Gemische davon |
| 112. | 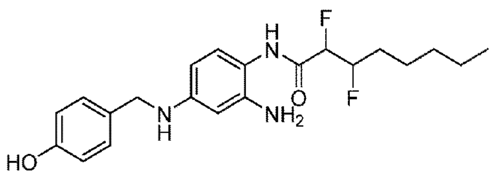 | N-(2-Amino-4-((4-hydroxybenzyl)amino)phenyl )-2,3-difluoroctanamid (2R,3S)-N-(2-Amino-4-((4-hydroxybenzyl)amino)phenyl )-2,3-difluoroctanamid (2S,3R)-N-(2-Amino-4-((4-hydroxybenzyl)amino)phenyl )-2,3-difluoroctanamid (2R,3R)-N-(2-Amino-4-((4-hydroxybenzyl)amino)phenyl )-2,3-difluoroctanamid (SR,3S)-N-(2-Amino-4-((4-hydroxybenzyl)amino)phenyl )-2,3-difluoroctanamid razemische Gemische davon |
| 113. | 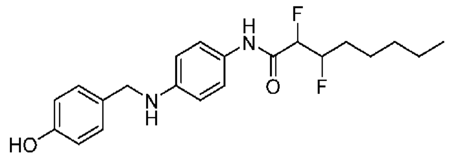 | 2,3-Difluor-N-(4-((4-hydroxybenzyl)amino)phenyl )octanamid (2R,3S)-2,3-Difluor-N-(4-((4-hydroxybenzyl)amino)phenyl )octanamid (2S,3R)-2,3-Difluor-N-(4-((4-hydroxybenzyl)amino)phenyl )octanamid (2R,3R)-2,3-Difluor-N-(4-((4-hydroxybenzyl)amino) phenyl )octanamid (2S,3S)-2,3-Difluor-N-(4-((4-hydroxybenzyl)amino)phenyl)octanamid razemische Gemische davon |
| 114. | 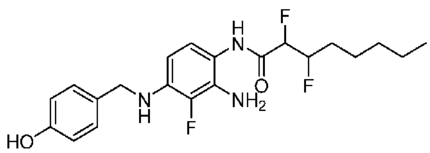 | N-(2-Amino-3-fluor-4-((4-hydroxybenzyl)amino)phenyl )-2,3-difluoroctanamid (2R,3S)-N-(2-Amino-3-fluor-4-((4-hydroxybenzyl)amino)phenyl )-2,3-difluoroctanamid (2S,3R)-N-(2-Amino-3-fluor-4-((4-hydroxybenzyl)amino)phenyl )-2,3-difluoroctanamid (2R,3R)-N-(2-Amino-3-fluor-4-((4-hydroxybenzyl)amino)phenyl )-2,3-difluoroctanamid (2S,3S)-N-(2-Amino-3-fluor-4-((4-hydroxybenzyl)amino)phenyl )-2,3-difluoroctanamid razemische Gemische davon |

(continued)

| | STRUKTUR DER VERBINDUNG | NAME DER VERBINDUNG |
|---|---|---|
| 115. | 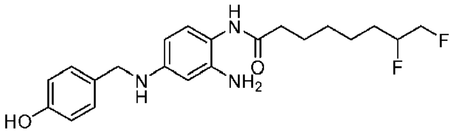 | N-(2-Amino-4-((4-hydroxybenzyl)amino)phenyl )-7,8-difluoroctanamid (7S)-N-(2-Amino-4-((4-hydroxybenzyl)amino)phenyl )-7,8-difluoroctanamid (7R)-N-(2-Amino-4-((4-hydroxybenzyl)amino)phenyl )-7,8-difluoroctanamid razemische Gemische davon |
| 116. | 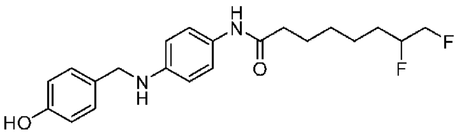 | 7,8-Difluor-N-(4-((4-hydroxybenzyl)amino)phenyl )octanamid (7R)-7,8-Difluor-N-(4-((4-hydroxybenzyl)amino)phenyl ) octanamid (7S)-7,8-Difluor-N-(4-((4-hydroxybenzyl)amino)phenyl ) octanamid razemische Gemische davon |
| 117. | 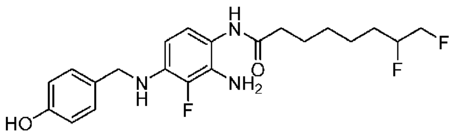 | N-(2-Amino-3-fluor-4-((4-hydroxybenzyl)amino)phenyl ) 7,8-difluoroctanamid (7R)-N-(2-Amino-3-fluor-4-((4-hydroxybenzyl)amino) phenyl )-7,8-difluoroctanamid (7S)-N-(2-Amino-3-fluor-4-((4-hydroxybenzyl)amino) phenyl )-7,8-difluoroctanamid razemische Gemische davon |
| 118. | 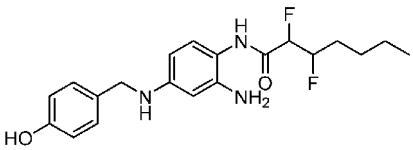 | N-(2-Amino-4-((4-hydroxybenzyl)amino)phenyl )-2,3-difluorheptanamid (2R,3S)-N-(2-Amino-4-((4-hydroxybenzyl)amino)phenyl )-2,3-difluorheptanamid (2S,3R)-N-(2-Amino-4-((4-hydroxybenzyl)amino)phenyl )-2,3-difluorheptanamid (2R,3R)-N-(2-Amino-4-((4-hydroxybenzyl)amino)phenyl )-2,3-difluorheptanamid (2S,3S)-N-(2-Amino-4-((4-hydroxybenzyl)amino)phenyl )-2,3-difluorheptanamid razemische Gemische davon |
| 119. | 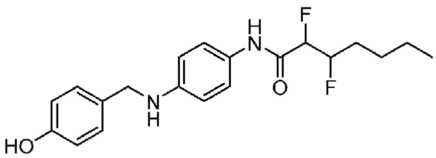 | 2,3-Difluor-N-(4-((4-hydroxybenzyl)amino)phenyl )heptanamid (2R,3S)-2,3-Difluor-N-(4-((4-hydroxybenzyl)amino)phenyl )heptanamid (2S,3R)-2,3-Difluor-N-(4-((4-hydroxybenzyl)amino)phenyl )heptanamid (2R,3R)-2,3-Difluor-N-(4-((4-hydroxybenzyl)amino) phenyl )heptanamid (2S,3S)-N-(2-Amino-4-((4-hydroxybenzyl)amino)phenyl )heptanamid razemische Gemische davon |
| 120. | 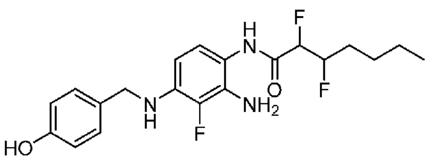 | N-(2-Amino-3-fluor-4-((4-hydroxybenzyl)amino)phenyl )-2,3-difluorheptanamid (2R,3S)-N-(2-Amino-3-fluor-4-((4-hydroxybenzyl)amino)phenyl )-2,3-difluorheptanamid (2S,3R)-N-(2-Amino-3-fluor-4-((4-hydroxybenzyl)amino)phenyl )-2,3-difluorheptanamid (2R,3R)-N-(2-Amino-3-fluor-4-((4-hydroxybenzyl)amino)phenyl )-2,3-difluorheptanamid (2S,3S)-N-(2-Amino-3-fluor-4-((4-hydroxybenzyl)amino)phenyl )-2,3-difluorheptanamid razemische Gemische davon |

(continued)

| | STRUKTUR DER VERBINDUNG | NAME DER VERBINDUNG |
|---|---|---|
| 121. | 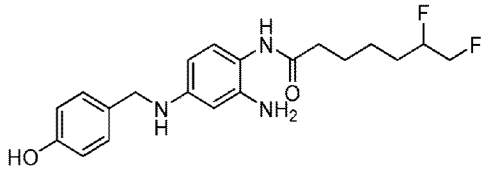 | N-(2-Amino-4-((4-hydroxybenzyl)amino)phenyl )-6,7-difluorheptanamid<br>(6R)-N-(2-Amino-4-((4-hydroxybenzyl)amino)phenyl )-6,7-difluorheptanamid<br>(6S)-N-(2-Amino-4-((4-hydroxybenzyl)amino)phenyl )-6,7-difluorheptanamid razemische Gemische davon |
| 122. | 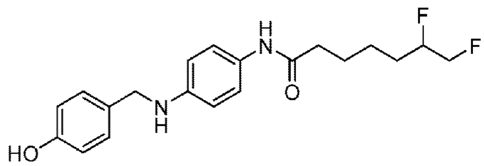 | 6,7-Difluor-N-(4-((4-hydroxybenzyl)amino)phenyl )heptanamid<br>(6R)-6,7-Difluor-N-(4-((4-hydroxybenzyl)amino)phenyl ) heptanamid<br>(6S)-6,7-Difluor-N-(4-((4-hydroxybenzyl)amino)phenyl ) heptanamid razemische Gemische davon |
| 123. | 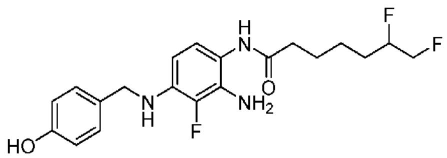 | N-(2-Amino-3-fluor-4-((4-hydroxybenzyl)amino)phenyl )-6,7-difluorheptanamid<br>(6R)-N-(2-Amino-3-fluor-4-((4-hydroxybenzyl)amino) phenyl )-6,7-difluorheptanamid<br>(6S)-N-(2-Amino-3-fluor-4-((4-hydroxybenzyl)amino) phenyl )-6,7-difluorheptanamid razemische Gemische davon |
| 124. | 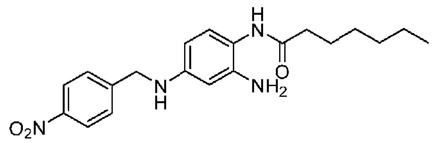 | N-(2-Amino-4-((4-nitrobenzyl)amino)phenyl)h eptanamid |
| 125. | 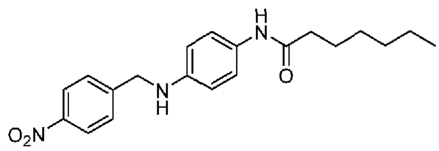 | N-(4-((4-Nitrobenzyl)amino)phenyl)h eptanamid |
| 126. | 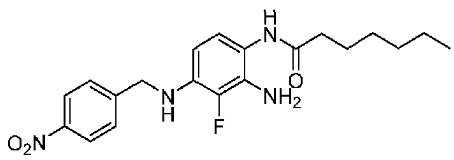 | N-(2-Amino-3-fluor-4-((4-nitrobenzyl)amino)phenyl)h eptanamid |
| 127. | 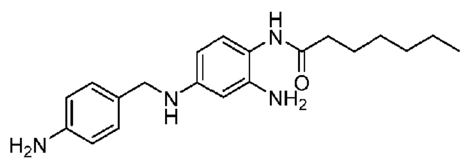 | N-(2-Amino-4-((4-aminobenzyl)amino)phenyl)h eptanamid |
| 128. | 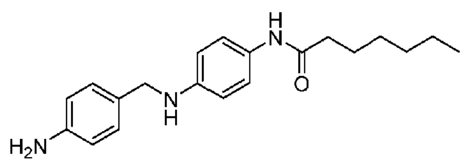 | N-(4-((4-Aminobenzyl)amino)phenyl)h eptanamid |
| 129. | 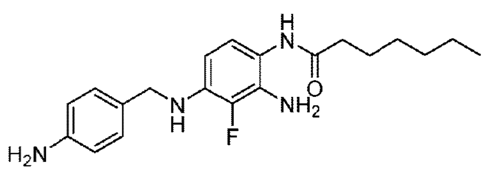 | N-(2-Amino-3-fluor-4-((4-aminobenzyl)amino)phenyl)h eptanamid |

(continued)

| | STRUKTUR DER VERBINDUNG | NAME DER VERBINDUNG |
|---|---|---|
| 130. | 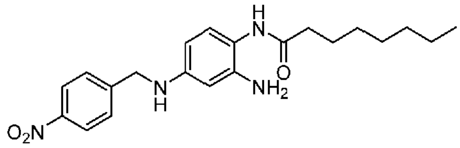 | N-(2-Amino-4-((4-nitrobenzyl)amino)phenyl)o ctanamid |
| 131. | 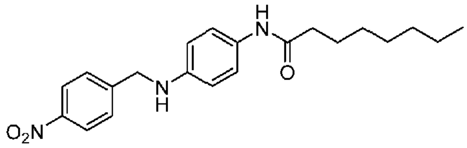 | N-(4-((4-nitrobenzyl)amino)phenyl)o ctanamid |
| 132. | 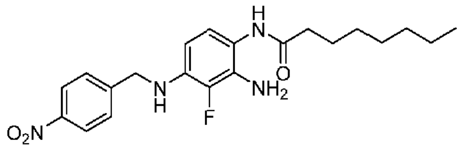 | N-(2-Amino-3-fluor-4-((4-nitrobenzyl)amino)phenyl)o ctanamid |
| 133. | 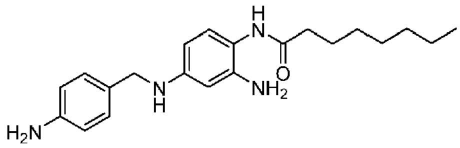 | N-(2-Amino-4-((4-aminobenzyl)amino)phenyl)o ctan-amid |
| 134. | 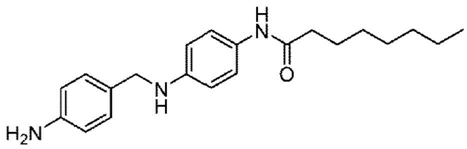 | N-(4-((4-Aminobenzyl)amino)phenyl)o ctanamid |
| 135. | 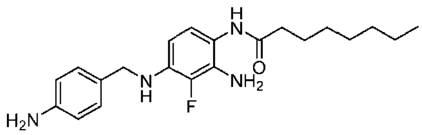 | N-(2-Amino-3-fluor-4-((4-aminobenzyl)amino)phenyl)o ctanamid |
| 136. | 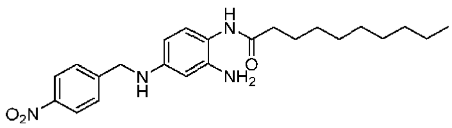 | N-(2-Amino-4-((4-nitrobenzyl) amino) phenyl)d ecan-amid |
| 137. | 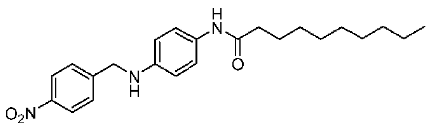 | N-(4-((4-Nitrobenzyl) amino) phenyl)d ecanamid |
| 138. | 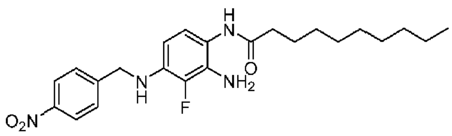 | N-(2-Amino-3-fluor-4-((4-nitrobenzyl)amino)phenyl)d ecanamid |
| 139. | 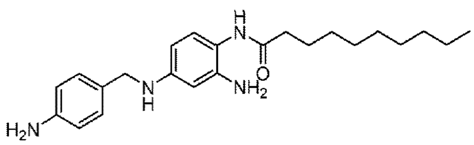 | N-(2-Amino-4-((4-aminobenzyl)amino)phenyl)d ecan-amid |

(continued)

| | STRUKTUR DER VERBINDUNG | NAME DER VERBINDUNG |
|---|---|---|
| 140. | 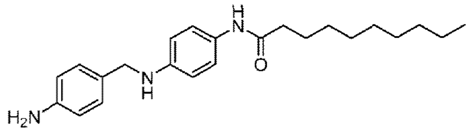 | N-(4-((4-Aminobenzyl)amino)phenyl)d ecanamid |
| 141. | 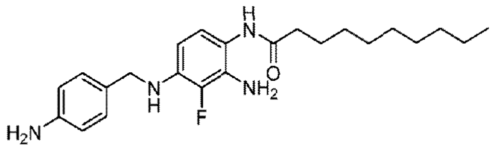 | N-(2-Amino-3-fluor-4-((4-aminobenzyl)amino)phenyl)d ecanamid |
| 142. | 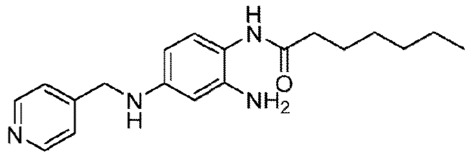 | N-(2-Amino-4-((pyridin-4-ylmethyl)amino)phenyl)hept anamid |
| 143. | 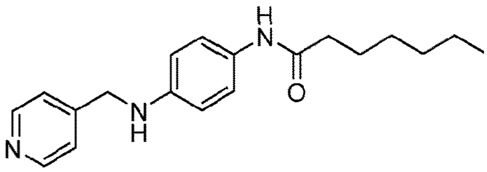 | N-(4-((Pyridin-4-ylmethyl)amino)phenyl)hept anamid |
| 144. | 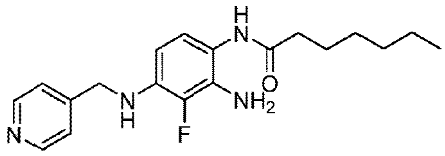 | N-(2-Amino-3-fluor-4-((pyridin-4-ylmethyl)amino)phe-nyl)hept anamid |
| 145. | 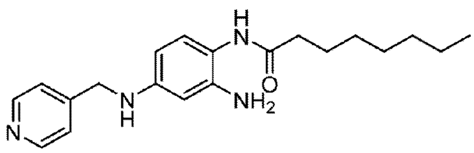 | N-(2-Amino-4-((pyridin-4-ylmethyl)amino)phenyl)octa namid |
| 146. | 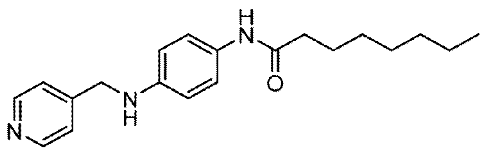 | N-(4-((Pyridin-4-ylmethyl)amino)phenyl)octa namid |
| 147. | 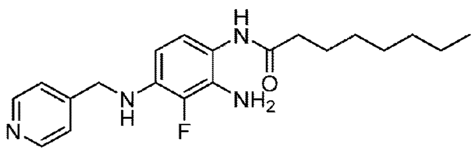 | N-(2-Amino-3-fluor-4-((pyridin-4-ylmethyl)amino)phe-nyl)octa namid |
| 148. | 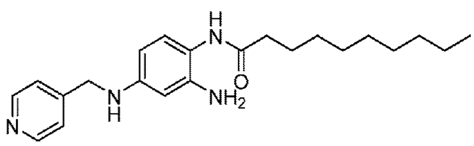 | N-(2-Amino-4-((pyridin-4-ylmethyl)amino)phenyl)deca namid |
| 149. | 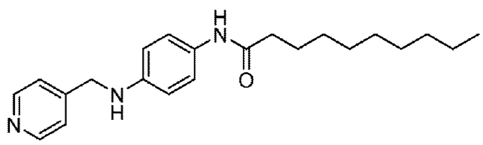 | N-(4-((Pyridin-4-ylmethyl)amino)phenyl)deca namid |

(continued)

| | STRUKTUR DER VERBINDUNG | NAME DER VERBINDUNG |
|---|---|---|
| 150. | 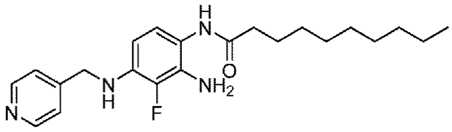 | N-(2-Amino-3-fluor-4-((pyridin-4-ylmethyl)amino)phenyl)deca namid |
| 151. | 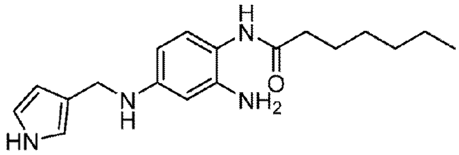 | N-(4-(((1H-Pyrrol-3-yl)methyl)amino)-2-aminophenyl) heptanamid |
| 152. | 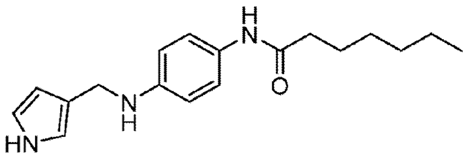 | N-(4-(((1H-Pyrrol-3-yl)methyl)amino)phenyl)hep tanamid |
| 153. | 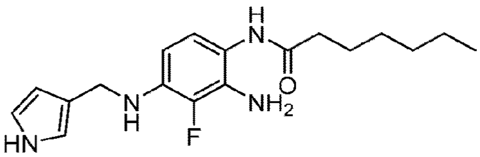 | N-(4-(((1H-Pyrrol-3-yl)methyl)amino)-2-amino-3-fluorphenyl)heptanamid |
| 154. | 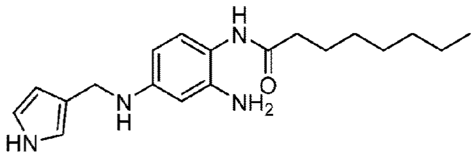 | N-(4-(((1H-Pyrrol-3-yl)methyl)amino)-2-aminophenyl) octanamid |
| 155. | 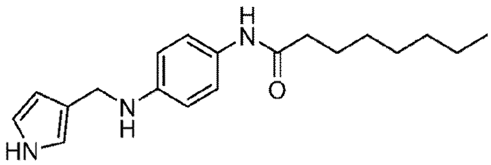 | N-(4-(((1H-Pyrrol-3-yl)methyl)amino)phenyl)oct anamid |
| 156. | 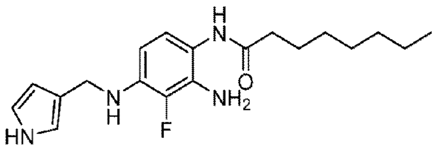 | N-(4-(((1H-Pyrrol-3-yl)methyl)amino)-2-amino-3-fluorphenyl)octanamid |
| 157. | 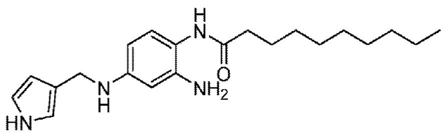 | N-(4-(((1H-Pyrrol-3-yl)methyl)amino)-2-aminophenyl) decanamid |
| 158. | 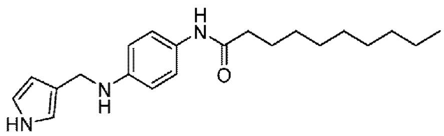 | N-(4-(((1H-Pyrrol-3-yl)methyl)amino)phenyl)dec anamid |
| 159. | 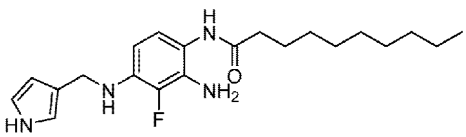 | N-(4-(((1H-Pyrrol-3-yl)methyl)amino)-2-amino-3-fluorphenyl)decanamid |

(continued)

| | STRUKTUR DER VERBINDUNG | NAME DER VERBINDUNG |
|---|---|---|
| 160. | 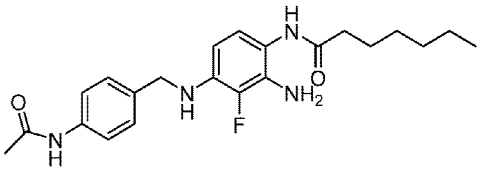 | N-(4-((4-Acetamidobenzyl)amino)-2-amino-3-fluorphenyl)heptanamid |
| 161. | 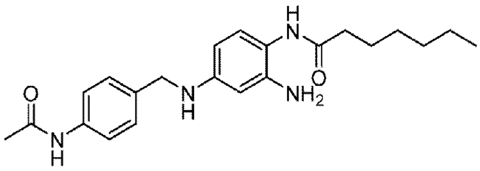 | N-(4-((4-Acetamidobenzyl)amino)-2-aminophenyl)heptanamid |
| 162. | 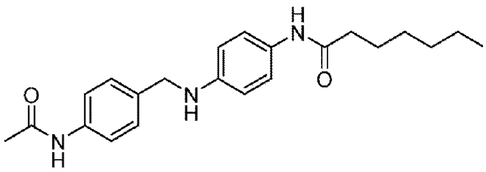 | N-(4-((4-Acetamidobenzyl)amino)phen yl)heptanamid |
| 163. | 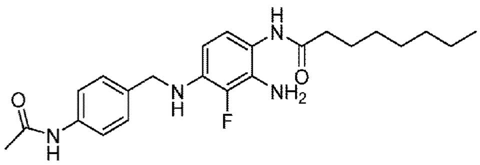 | N-(4-((4-Acetamidobenzyl)amino)-2-amino-3-fluorphenyl)octanamid |
| 164. | 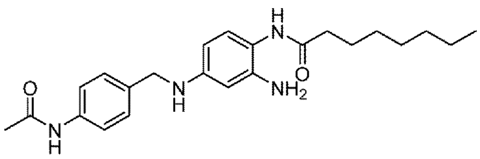 | N-(4-((4-Acetamidobenzyl)amino)-2-aminophenyl)octanamid |
| 165. | 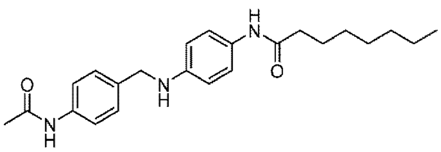 | N-(4-((4-Acetamidobenzyl)amino)phen yl)octanamid |
| 166. | 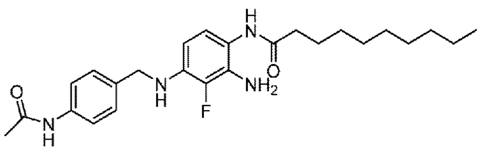 | N-(4-((4-Acetamidobenzyl)amino)-2-amino-3-fluorphenyl)decanamid |
| 167. | 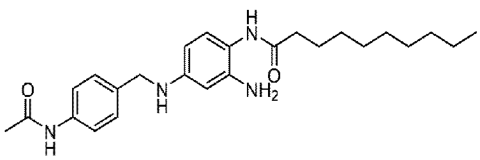 | N-(4-((4-Acetamidobenzyl)amino)-2-aminophenyl)decanamid |
| 168. | 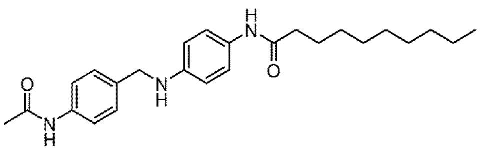 | N-(4-((4-Acetamidobenzyl)amino)phen yl)decanamid |

(continued)

| | STRUKTUR DER VERBINDUNG | NAME DER VERBINDUNG |
|---|---|---|
| 169. | 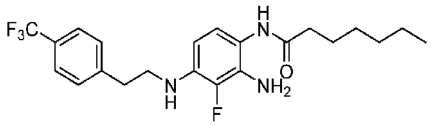 | N-(2-Amino-3-fluor-4-((4-(trifluormethyl)phenethyl) amino)phenyl)heptanamid |
| 170. | 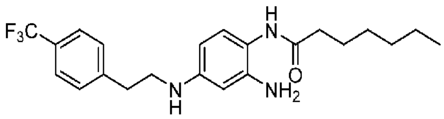 | N-(2-Amino-4-((4-(trifluormethyl)phenethyl) amino)phenyl)heptanamid |
| 171. | 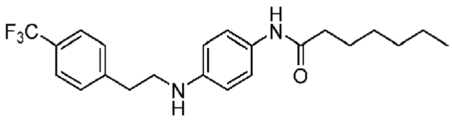 | N-(4-((4-(Trifluormethyl)phenethyl) amino)phenyl)heptanamid |
| 172. | 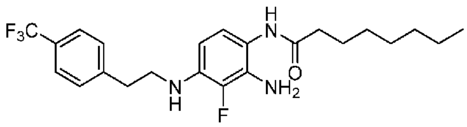 | N-(2-Amino-3-fluor-4-((4-(trifluormethyl)phenethyl) amino)phenyl)octanamid |
| 173. | 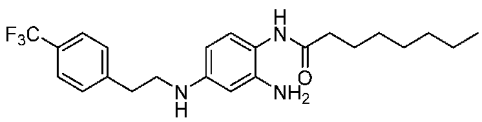 | N-(2-Amino-4-((4-(trifluormethyl)phenethyl) amino)phenyl)octanamid |
| 174. | 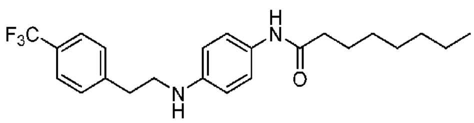 | N-(4-((4-(Trifluormethyl)phenethyl) amino)phenyl)octanamid |
| 175. | 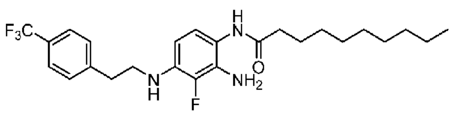 | N-(2-Amino-3-fluor-4-((4-(trifluormethyl)phenethyl)amino)phenyl)decanamid |
| 176. | 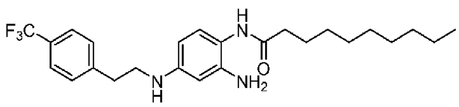 | N-(2-Amino-4-((4-(trifluormethyl)phenethyl) amino)phenyl)decanamid |
| 177. | 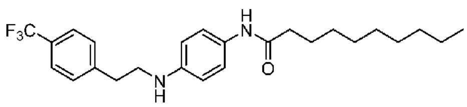 | N-(4-((4-(Trifluormethyl)phenethyl) amino)phenyl)decanamid |
| 178. | 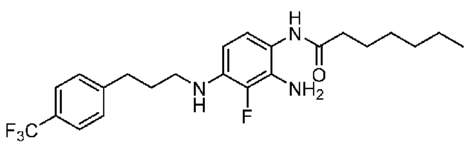 | N-(2-Amino-3-fluor-4-((3-(4-(trifluormethyl)phenyl)propyl)amino)phenyl)heptanami d |
| 179. | 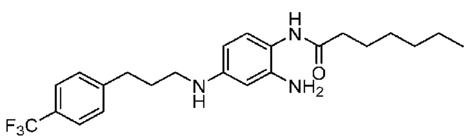 | N-(2-Amino-4-((3-(4-(trifluormethyl)phenyl)pro pyl)amino)phenyl)heptanami d |
| 180. | 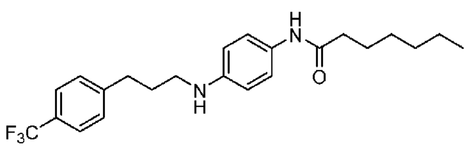 | N-(4-((3-(4-(Trifluormethyl)phenyl)pro pyl)amino)phenyl)heptanami d |

(continued)

| | STRUKTUR DER VERBINDUNG | NAME DER VERBINDUNG |
|---|---|---|
| 181. | 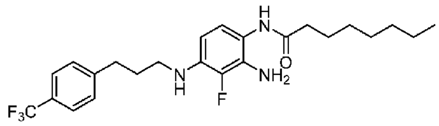 | N-(2-Amino-3-fluor-4-((3-(4-(trifluormethyl)phenyl)pro pyl)amino)phenyl)octanamid |
| 182. | 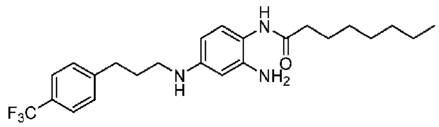 | N-(2-Amino-4-((3-(4-(trifluormethyl)phenyl)pro pyl)amino)phenyl)octanamid |
| 183. | 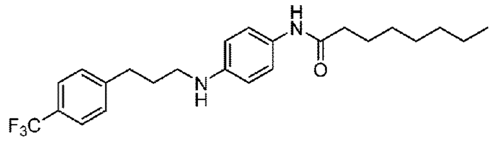 | N-(4-((3-(4-(Trifluormethyl)phenyl)pro pyl)amino)phenyl)octanamid |
| 184. | 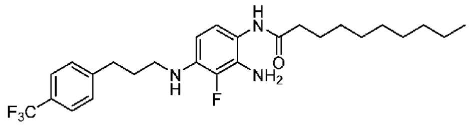 | N-(2-Amino-3-fluor-4-((3-(4(trifluormethyl)phenyl)p ropyl)amino) phenyl) decanam id |
| 185. | 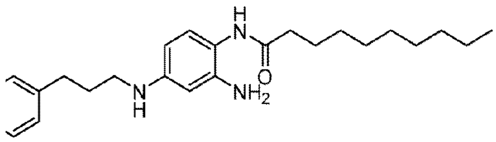 | N-(2-Amino-4-((3-(4-(trifluormethyl)phenyl)pro pyl)amino)phenyl)decanamid |
| 186. | 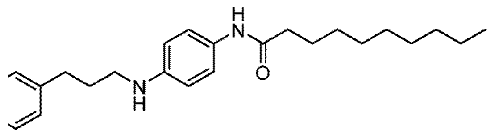 | N-(4-((3-(4-(Trifluormethyl)phenyl)pro pyl)amino)phenyl)decanamid |
| 187. | 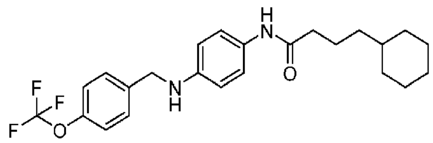 | 4-Cyclohexyl-N-(4-((4-(trifluormethoxy)benzyl)am ino) phenyl)butanamid |
| 188. | 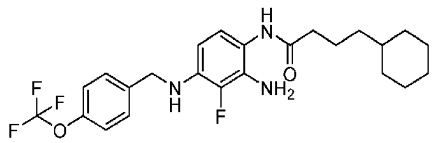 | N-(2-Amino-3-fluor-4-((4-(trifluormethoxy)benzyl)am ino)phenyl)-4-cyclohexylbutanamid |
| 189. | 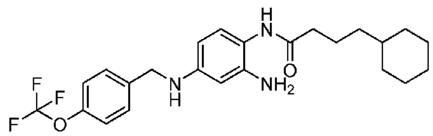 | N-(2-Amino-4-((4-(trifluormethoxy)benzyl)am ino)phenyl)-4-cyclohexylbutanamid |
| 190. | 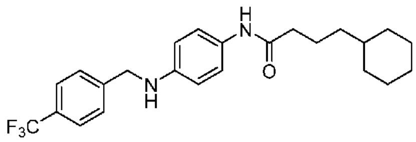 | 4-Cyclohexyl-N-(4-((4-(trifluormethyl)benzyl)ami no) phenyl)butanamid |
| 191. | 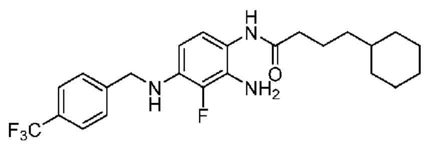 | N-(2-Amino-3-fluor-4-((4-(trifluormethyl)benzyl)ami no) phenyl)-4-cyclohexylbutanamid |

(continued)

| | STRUKTUR DER VERBINDUNG | NAME DER VERBINDUNG |
|---|---|---|
| 192. | 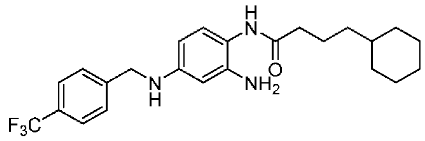 | N-(2-Amino-4-((4-(trifluormethyl)benzyl)ami no)phenyl)-4-cyclohexylbutanamid |
| 193. | 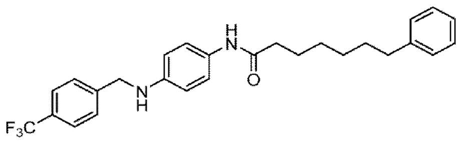 | 7-Phenyl-N-(4-((4-(trifluormethyl)benzyl)ami no)phenyl) heptanamid |
| 194. | 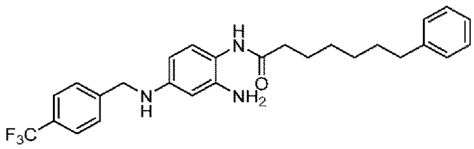 | N-(2-Amino-4-((4-(trifluormethyl)benzyl)ami no)phenyl)-7-phenylheptanamid |
| 195. | 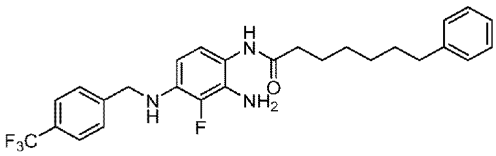 | N-(2-Amino-3-fluor-4-((4-(trifluormethyl)benzyl)ami no) phenyl)-7-phenylheptanamid |
| 196. | 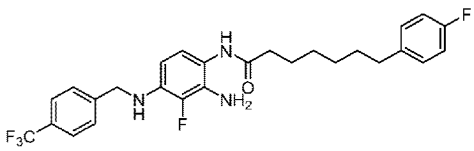 | N-(2-Amino-3-fluor-4-((4-(trifluormethyl)benzyl)ami no) phenyl-7-(4-fluorphenyl)heptanamid |
| 197. | 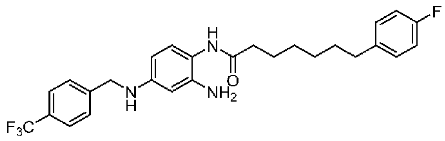 | N-(2-Amino-4-((4-(trifluormethyl)benzyl)ami no)phenyl)-7-(4-fluorphenyl)heptanamid |
| 198. | 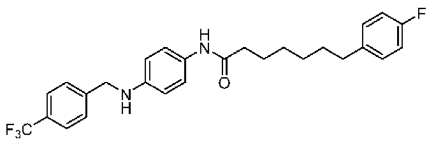 | 7-(4-Fluorphenyl)-N-(4-((4-(trifluormethyl)benzyl)ami no)phenyl)heptanamid |
| 199. | 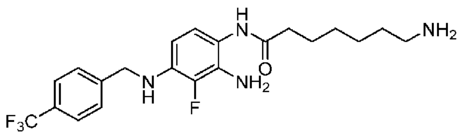 | 7-Amino-N-(2-amino-3-fluor-4-((4-(trifluormethyl)benzyl)ami no)phenyl)heptanamid |
| 200. | 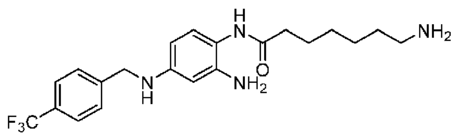 | 7-Amino-N-(2-amino-4-((4-(trifluormethyl)benzyl)ami no)phenyl)heptanamid |
| 201. | 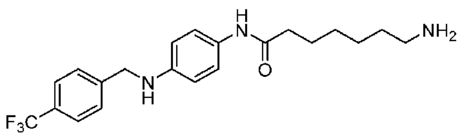 | 7-Amino-N-(4-((4-(trifluormethyl)benzyl)ami no)phenyl) heptanamid |

(continued)

| | STRUKTUR DER VERBINDUNG | NAME DER VERBINDUNG |
|---|---|---|
| 202. | 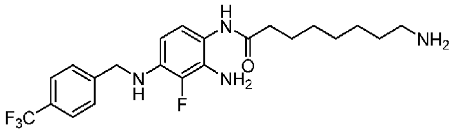 | 8-Amino-N-(2-amino-3-fluor-4-((4-(trifluormethyl)benzyl)ami no)phenyl)octanamid |
| 203. | 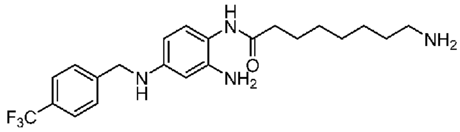 | 8-Amino-N-(2-amino-4-((4-(trifluormethyl)benzyl)ami no)phenyl)octanamid |
| 204. | 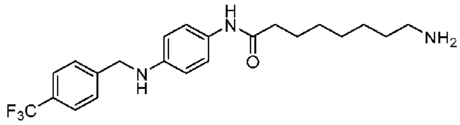 | 8-Amino-N-(4-((4-(trifluormethyl)benzyl)ami no)phenyl) octanamid |
| 205. | 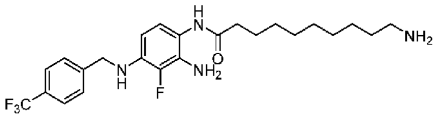 | 10-Amino-N-(2-amino-3-fluor-4-((4-(trifluormethyl)benzyl)ami no)phenyl)decanamid |
| 206. | 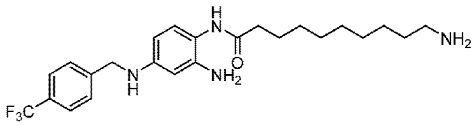 | 10-Amino-N-(2-amino-4-((4-(trifluormethyl)benzyl)ami no)phenyl)decanamid |
| 207. | 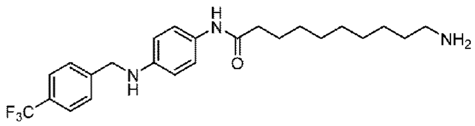 | 10-Amino-N-(4-((4-(trifluormethyl)benzyl)ami no)phenyl)decanamid |
| 208. | 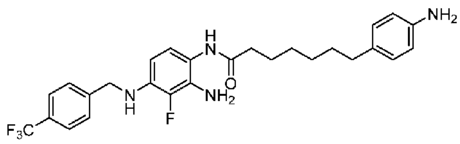 | N-(2-Amino-3-fluor-4-((4-(trifluormethyl)benzyl)ami no) phenyl-7-(4-aminophenyl)heptanamid |
| 209. | 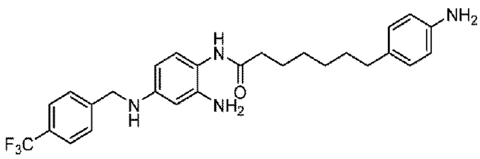 | N-(2-Amino-4-((4-(trifluormethyl)benzyl)ami no)phenyl)-7-(4-aminophenyl)heptanamid |
| 210. | 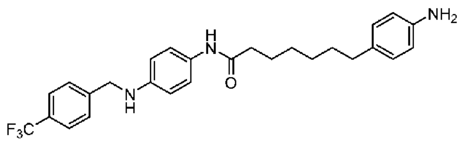 | 7-(4-Aminophenyl)-N-(4-((4-(trifluormethyl)benzyl)ami no)phenyl)heptanamid |
| 211. | 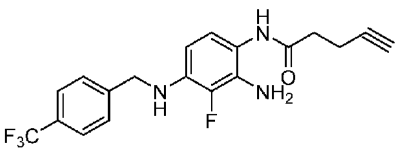 | N-(2-Amino-3-fluor-4-((4-(trifluormethyl)benzyl)ami no) phenyl)pent-4-ynamid |

(continued)

| | STRUKTUR DER VERBINDUNG | NAME DER VERBINDUNG |
|---|---|---|
| 212. | 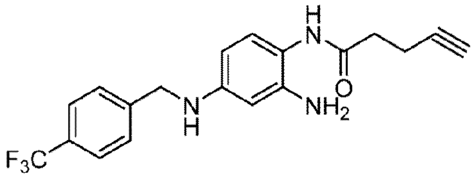 | N-(2-Amino-4-((4-(trifluormethyl)benzyl)ami no)phenyl) pent-4-ynamid |
| 213. | 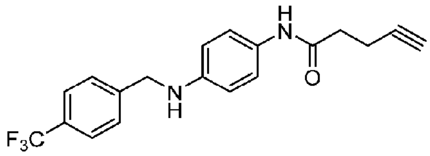 | N-(4-((4-(Trifluormethyl)benzyl)ami no)phenyl)pent-4-ynamid |
| 214. | 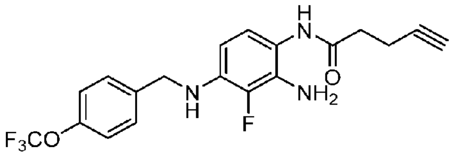 | N-(2-Amino-3-fluor-4-((4-(trifluormethoxy)benzyl)am ino)phenyl)pent-4-ynamid |
| 215. | 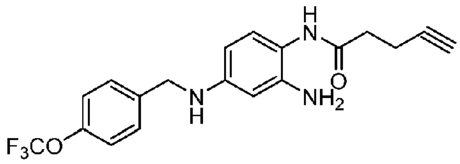 | N-(2-Amino-4-((4-(trifluormethoxy)benzyl)am ino)phenyl)pent-4-ynamid |
| 216. | 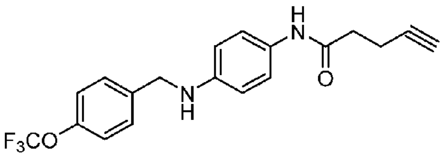 | N-(4-((4-(Trifluormethoxy)benzyl)am ino)phenyl)pent-4-ynamid |
| 217. | 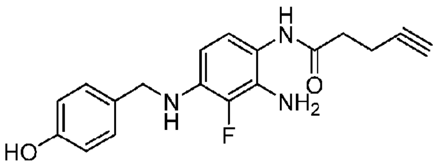 | N-(2-Amino-3-fluor-4-((4-hydroxybenzyl)amino)phenyl ) pent-4-ynamid |
| 218. | 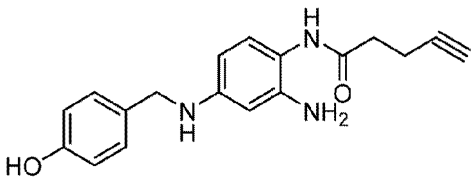 | N-(2-Amino-4-((4-hydroxybenzyl)amino)phenyl )pent-4-ynamid |
| 219. | 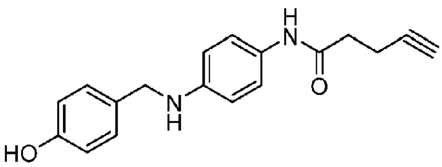 | N-(4-((4-Hydroxybenzyl)amino)phenyl )pent-4-ynamid |
| 220. | 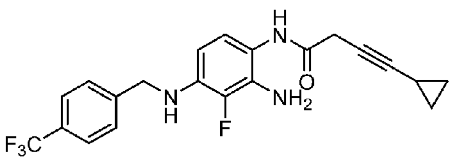 | N-(2-Amino-3-fluor-4-((4-(trifluormethyl)benzyl)ami no) phenyl)-4-cyclopropylbut-3-ynamid |

(continued)

| | STRUKTUR DER VERBINDUNG | NAME DER VERBINDUNG |
|---|---|---|
| 221. | 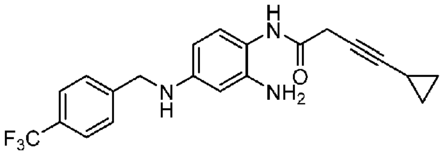 | N-(2-Amino-4-((4-(trifluormethyl)benzyl)ami no)phenyl)-4-cyclopropylbut-3-ynamid |
| 222. | 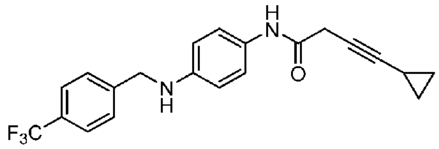 | 4-Cyclopropyl-N-(4-((4-(trifluormethyl)benzyl)ami no)phenyl)but-3-ynamid |
| 223. | 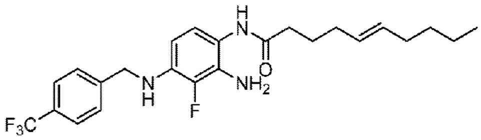 | (E)-N-(2-Amino-3-fluor-4-((4-(trifluormethyl)benzyl)ami no)phenyl)dec-5-enamid |
| 224. | 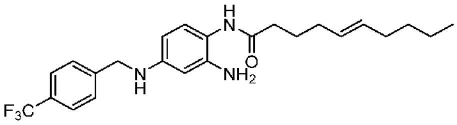 | (E)-N-(2-Amino-4-((4-(trifluormethyl)benzyl)ami no)phenyl)dec-5-enamid |
| 225. | 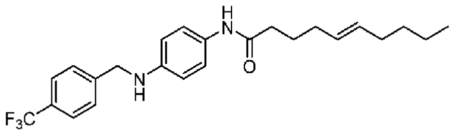 | (E)-N-(4-((4-(Trifluormethyl)benzyl)ami no)phenyl)dec-5-enamid |
| 226. | 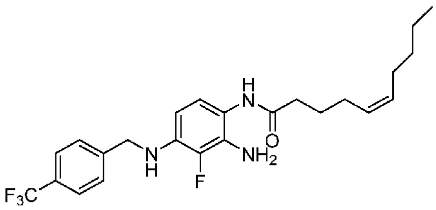 | (Z)-N-(2-Amino-3-fluor-4-((4-(trifluormethyl)benzyl)ami no)phenyl)dec-5-enamid |
| 227. | 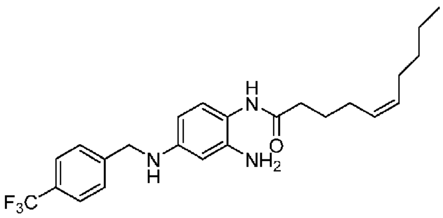 | (Z)-N-(2-Amino-4-((4-(trifluormethyl)benzyl)ami no)phenyl)dec-5-enamid |
| 228. | 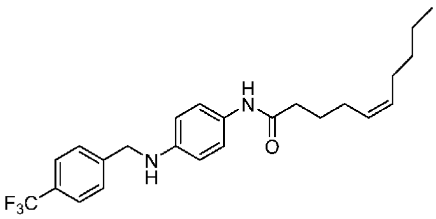 | (Z)-N-(4-((4-(Trifluormethyl)benzyl)ami no)phenyl)dec-5-enamid |

(continued)

| | STRUKTUR DER VERBINDUNG | NAME DER VERBINDUNG |
|---|---|---|
| 229. | 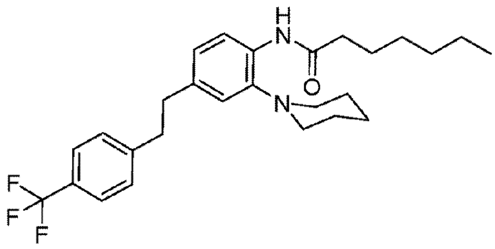 | N-(2-(Piperidin-1-yl)-4-(4-(trifluormethyl)phenethyl) phenyl)heptanamid |
| 230. | 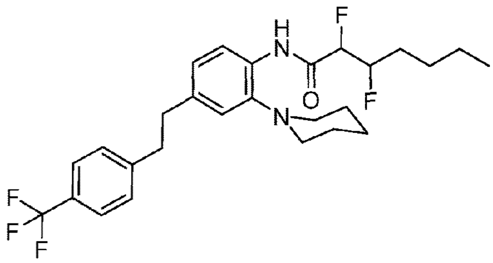 | 2,3-Difluor-N-(2-(piperidin-1-yl)-4-(4-(trifluormethyl) phenethyl) phenyl)heptanamid<br>(2R,3S)-2,3-Difluor-N-(2-(piperidin-1-yl)-4-(4-(trifluor-methyl)phenethyl) phenyl)heptanamid<br>(2S,3R)-2,3-Difluor-N-(2-(piperidin-1-yl)-4-(4-(trifluor-methyl)phenethyl) phenyl)heptanamid<br>(2R,3R)-2,3-Difluor-N-(2-(piperidin-1-yl)-4-(4-(trifluor-methyl)phenethyl) phenyl)heptanamid<br>(2S,3S)-2,3-Difluor-N-(2-(piperidin-1-yl)-4-(4-(trifluormethyl)phenethyl) phenyl)heptanamid |
| 231. | 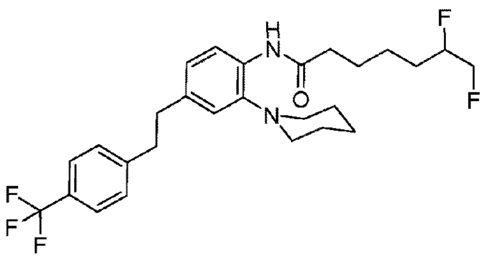 | 6,7-Difluor-N-(2-(piperidin-1-yl)-4-(4-(trifluormethyl) phenethyl) phenyl)heptanamid<br>(6R)-6,7-Difluor-N-(2-(piperidin-1-yl)-4-(4-(trifluorme-thyl)phenethyl) phenyl)heptanamid<br>(6S)-6,7-Difluor-N-(2-(piperidin-1-yl)-4-(4-(trifluormethyl)phenethyl) phenyl)heptanamid |
| 232. | 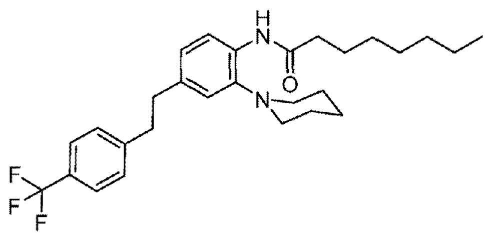 | N-(2-(Piperidin-1-yl)-4-(4-(trifluormethyl)phenethyl) phenyl)octanamid |
| 233. | 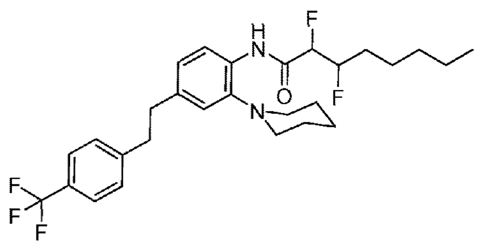 | 2,3-Difluor-N-(2-(piperidin-1-yl)-4-(4-(trifluormethyl) phenethyl) phenyl)octanamid<br>(2R,3S)-2,3-Difluor-N-(2-(piperidin-1-yl)-4-(4-(trifluor-methyl)phenethyl) phenyl)octanamid<br>(2S,3R)-2,3-Difluor-N-(2-(piperidin-1-yl)-4-(4-(trifluor-methyl)phenethyl) phenyl)octanamid<br>(2R,3R)-2,3-Difluor-N-(2-(piperidin-1-yl)-4-(4-(trifluor-methyl)phenethyl) phenyl)octanamid<br>(2S,3S)-2,3-Difluor-N-(2-(piperidin-1-yl)-4-(4-(trifluormethyl)phenethyl) phenyl)octanamid |
| 234. | 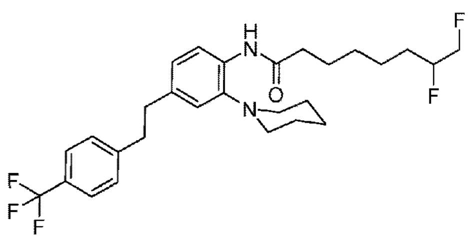 | 7,8-Difluor-N-(2-(piperidin-1-yl)-4-(4-(trifluormethyl) phenethyl) phenyl)octanamid<br>(7R)-7,8-Difluor-N-(2-(piperidin-1-yl)-4-(4-(trifluorme-thyl)phenethyl) phenyl)octanamid<br>(7S)-7,8-Difluor-N-(2-(piperidin-1-yl)-4-(4-(trifluorme-thyl)phenethyl)phenyl)octanamid |

(continued)

| | STRUKTUR DER VERBINDUNG | NAME DER VERBINDUNG |
|---|---|---|
| 235. | 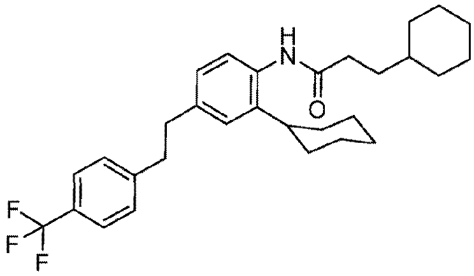 | 3-Cyclohexyl-N-(2-cyclohexyl-4-(4-(trifluormethyl)phenethyl) phenyl)propanamid |
| 236. | 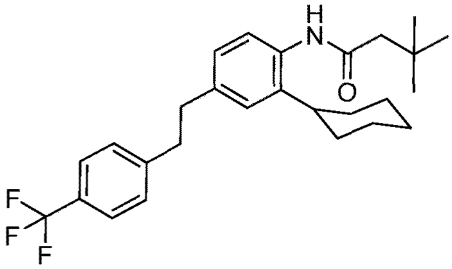 | N-(2-Cyclohexyl-4-(4-(trifluormethyl)phenethyl) phenyl)-3,3-dimethylbutanamid |
| 237. | 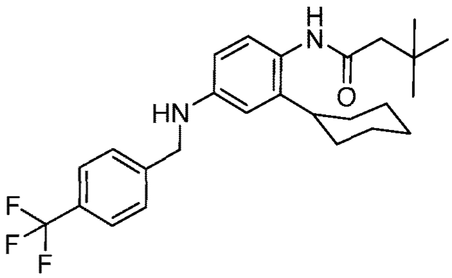 | N-(2-Cyclohexyl-4-((4-(trifluormethyl)benzyl)ami no) phenyl)-3,3-dimethylbutanamid |
| 238. | 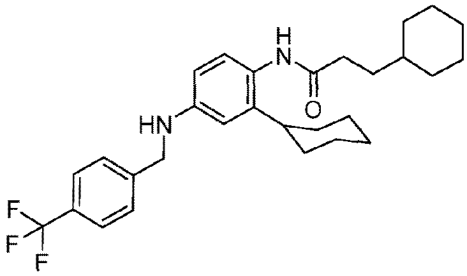 | 3-Cyclohexyl-N-(2-cyclohexyl-4-((4-(trifluormethyl)benzyl)ami no)phenyl)propanamid |
| 239. | 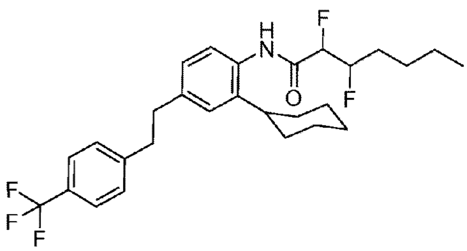 | N-(2-Cyclohexyl-4-(4-(trifluormethyl)phenethyl) phenyl)-2,3-difluorheptanamid (2R,3S)-N-(2-Cyclohexyl-4-(4-(trifluormethyl)phenethyl) phenyl)-2,3-difluorheptanamid (2S,3R)-N-(2-Cyclohexyl-4-(4-(trifluormethyl)phenethyl)phenyl)-2,3-difluorheptanamid (2R,3R)-N-(2-Cyclohexyl-4-(4-(trifluormethyl)phenethyl) phenyl)-2,3-difluorheptanamid (2S,3S)-N-(2-Cyclohexyl-4-(4-(trifluormethyl)phenethyl) phenyl)-2,3-difluorheptanamid |

(continued)

| | STRUKTUR DER VERBINDUNG | NAME DER VERBINDUNG |
|---|---|---|
| 240. | 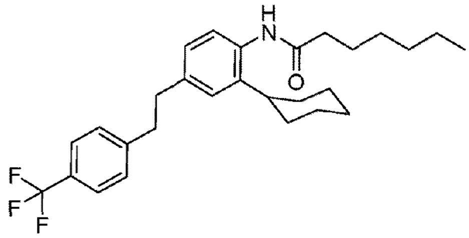 | N-(2-Cyclohexyl-4-(4-(trifluormethyl)phenethyl) phenyl) heptanamid |
| 241. | 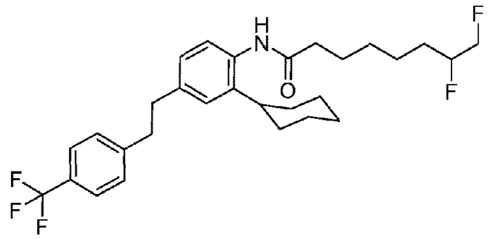 | N-(2-Cyclohexyl-4-(4-(trifluormethyl)phenethyl) phenyl)-7,8-difluoroctanamid<br>(7R)-N-(2-Cyclohexyl-4-(4-(trifluormethyl)phenethyl) phenyl)-7,8-difluoroctanamid<br>(7S)-N-(2-Cclohexyl-4-(4-(trifluormethyl)phenethyl) phenyl)-7,8-difluoroctanamid |
| 242. | 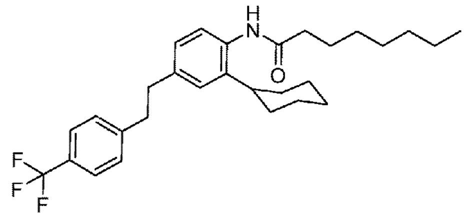 | N-(2-Cyclohexyl-4-(4-(trifluormethyl)phenethyl) phenyl) octanamid |
| 243. | 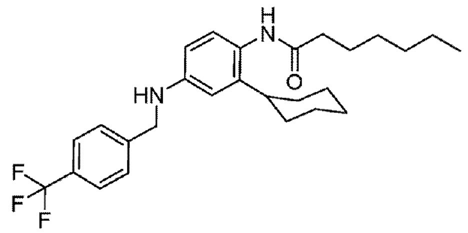 | N-(2-Cyclohexyl-4-((4-(trifluormethyl)benzyl)ami no) phenyl)heptanamid |
| 244. | 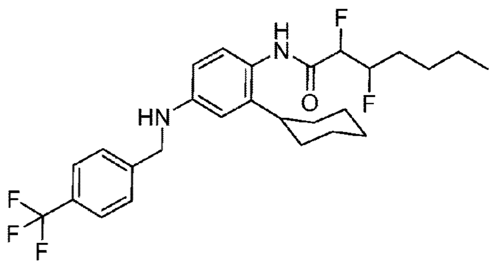 | N-(2-Cyclohexyl-4-((4-(trifluormethyl)benzyl)ami no) phenyl)-2,3-difluorheptanamid<br>(2R,3S)-N-(2-Cyclohexyl-4-(4-(trifluormethyl)benzyl) ami no)phenyl)-2,3-difluorheptanamid<br>(2S,3R)-N-(2-Cyclohexyl-4-(4-(trifluormethyl)benzyl) ami no)phenyl)-2,3-difluorheptanamid<br>(2R,3R)-N-(2-Cyclohexyl-4-(4-(trifluormethyl)benzyl) ami no)phenyl)-2,3-difluorheptanamid<br>(2S,3S)-N-(2-Cyclohexyl-4-((4-(trifluormethyl)benzyl) ami no)phenyl)-2,3-difluorheptanamid |
| 245. | 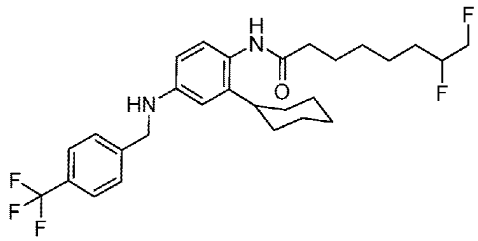 | N-(2-Cyclohexyl-4-((4-(trifluormethyl)benzyl)ami no) phenyl)-7,8-difluoroctanamid<br>(7R)-N-(2-Cyclohexyl-4-((4-(trifluormethyl)benzyl)amino)phenyl)-7,8-difluoroctanamid<br>(7S)-N-(2-Cyclohexyl-4-((4-(trifluormethyl)benzyl)ami no)phenyl)-7,8-difluoroctanamid |

(continued)

| | STRUKTUR DER VERBINDUNG | NAME DER VERBINDUNG |
|---|---|---|
| 246. | 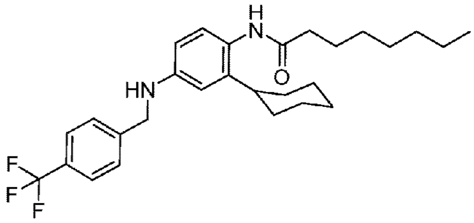 | N-(2-Cyclohexyl-4-((4-(trifluormethyl)benzyl)ami no)phenyl)octanamid |
| 247. | 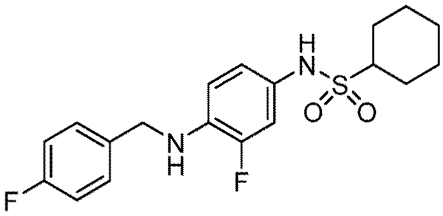 | N-(3-Fluor-4-((4fluorbenzyl)amino)pheny l)cyclohexan-sulfonamid |
| 248. | 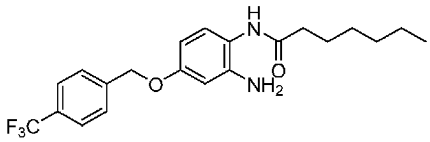 | N-(2-Amino-4-((4-(trifluormethyl)benzyl)oxy )phenyl)heptanamid |
| 249. | 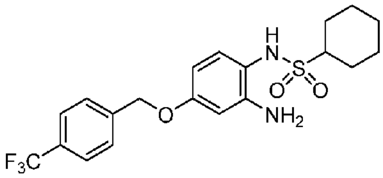 | N-(2-Amino-4-((4-(trifluormethyl)benzyl)oxy )phenyl)cyclohexansulfonam id |
| 250. | 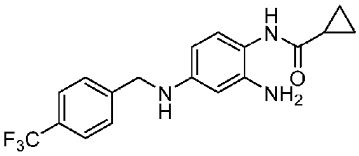 | N-(2-Amino-4-((4-(trifluormethyl)benzyl)ami no)phenyl)cyclopropancarbo xamid |
| 251. | 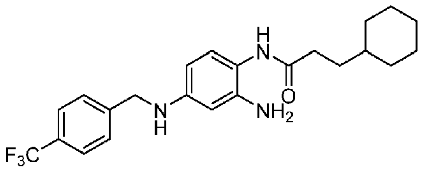 | N-(2-Amino-4-((4-(trifluormethyl)benzyl)ami no)phenyl)-3-cyclohexylpropanamid |
| 252. | 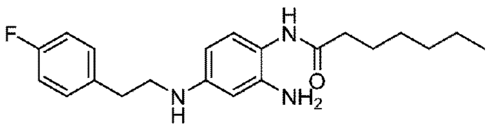 | N-(2-Amino-4-((4-fluorphenethyl)amino)pheny l)heptanamid |
| 253. | 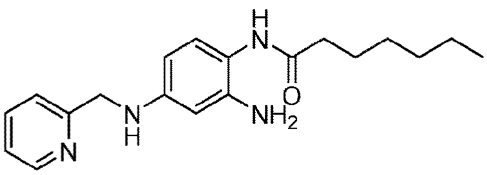 | N-(2-Amino-4-((pyridin-2-ylmethyl)amino)phenyl)heptanamid |

(continued)

| | STRUKTUR DER VERBINDUNG | NAME DER VERBINDUNG |
|---|---|---|
| 254. | 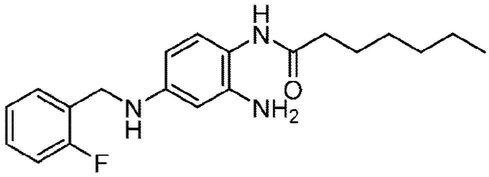 | N-(2-Amino-4-((-2-fluorbenzyl)amino)phenyl)h eptan-amid |
| 255. | 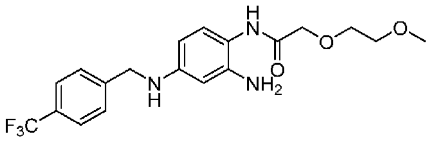 | N-(2-Amino-4-((4-(trifluormethyl)benzyl)ami no)phenyl)-2-(2-methoxyethoxy)acetamid |
| 256. | 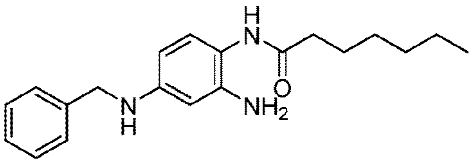 | N-(2-Amino-4-(benzylamino)phenyl)heptan amid |
| 257. | 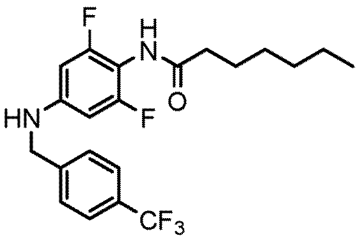 | N-(2,6-Difluor-4-((4-(trifluormethyl)benzyl)ami no)phe-nyl)heptanamid |
| 258. | 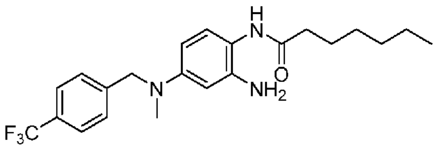 | N-(2-Amino-4-(methyl(4-(trifluormethyl)benzyl)ami no) phenyl)heptanamid |
| 259. | 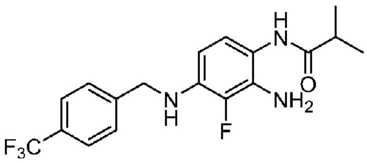 | N-(2-Amino-3-fluor-4-((4-(trifluormethyl)benzyl)ami no) phenyl)isobutyramid |
| 260. | 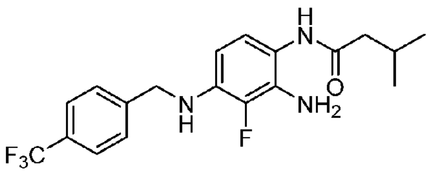 | N-(2-Amino-3-fluor-4-((4-(trifluormethyl)benzyl)ami no) phenyl)-3-methylbutanamid |
| 261. | 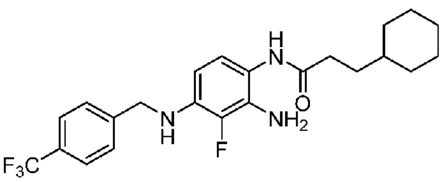 | N-(2-Amino-3-fluor-4-((4-(trifluormethyl)benzyl)ami no) phenyl)-3-cyclohexylpropanamid |

(continued)

| | STRUKTUR DER VERBINDUNG | NAME DER VERBINDUNG |
|---|---|---|
| 262. | 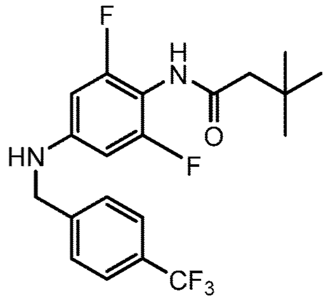 | N-(2,6-Difluor-4-((4-(trifluormethyl)benzyl)ami no)phenyl)-3,3-dimethylbutanamid |
| 263. | 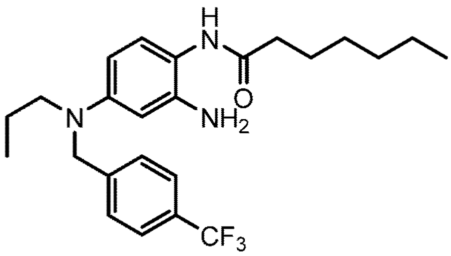 | N-(2-Amino-4-(propyl(4-(trifluormethyl)benzyl)ami no) phenyl)heptanamid |
| 264. | 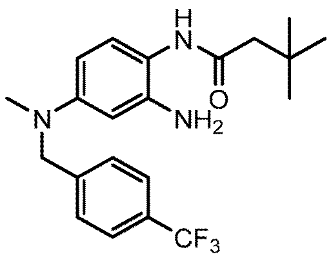 | N-(2-Amino-4-((4-(trifluormethyl)benzyl)ami no)phenyl)-3,3-dimethylbutanamid |
| 265. | 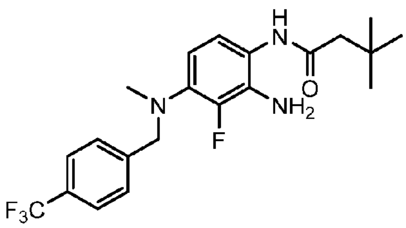 | N-(2-Amino-3-fluor-4-(methyl(4-(trifluormethyl)benzyl) ami no)phenyl)-3,3-dimethylbutanamid |
| 266. | 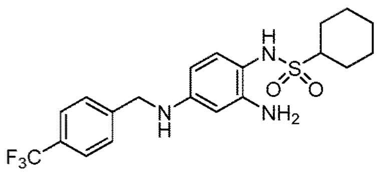 | N-(2-Amino-4-((4-(trifluormethyl)benzyl)ami no)phenyl) cyclohexansulfon amid |
| 267. | 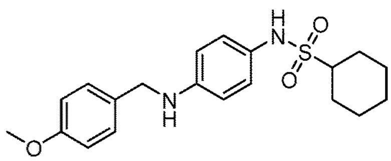 | N-(4-((4-Methoxybenzyl)amino)phenyl )cyclohexansul-fonamid |
| 268. | 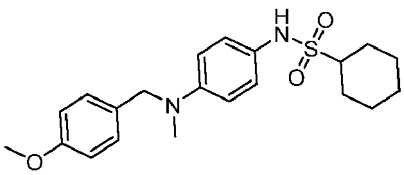 | N-(4-((4-Methoxybenzyl) (methyl)amin o)phenyl)cyclo-hexansulfona mid |

(continued)

| | STRUKTUR DER VERBINDUNG | NAME DER VERBINDUNG |
|---|---|---|
| 269. | 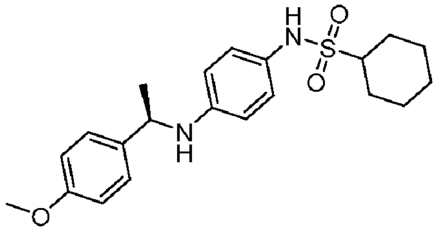 | (R)-N-(4-((1-(4-Methoxyphenyl) ethyl) amino) phenyl) cyclohexansulfonami d |
| 270. | 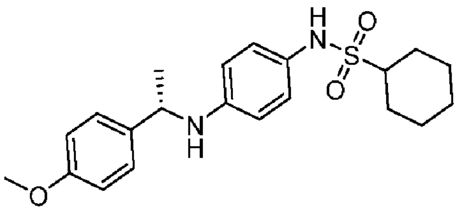 | (S)-N-(4-((1-(4-Methoxyphenyl)ethyl)amino) phenyl) cyclohexansulfonami d |
| 271. | 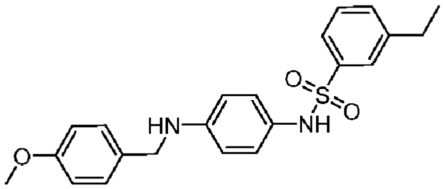 | 3-Ethyl-N-(4-((4-methoxybenzyl)amino)phenyl )benzensulfonamid |

oder ein Tautomer, Salz, Solvat, Stereoisomer oder Isotop davon.

3. Verbindung oder Tautomer, Salz, Solvat, Stereoisomer oder Isotop davon nach einem der vorhergehenden Ansprüche, wobei die Verbindung ein spannungsgesteuerter Kaliumkanal-Modulator ist, vorzugsweise ein Kv7.1-, Kv7.2-, Kv7.3-, Kv7.4- und/oder Kv7.5-Modulator.

4. Verbindung oder Tautomer, Salz, Solvat, Stereoisomer oder Isotop davon, wie in einem der vorhergehenden Ansprüche definiert, zur Verwendung als Medikament; vorzugsweise

zur Verwendung bei der Behandlung und/oder Vorbeugung eines Zustands, der **gekennzeichnet ist durch** einen Defekt in der Struktur und/oder Funktion mindestens eines spannungsgesteuerten Kaliumkanals, vorzugsweise mindestens eines von Kv7.1, Kv7.2, Kv7.3, Kv7.4 und/oder Kv7.5; wobei der Zustand vorzugsweise ausgewählt ist aus der Gruppe bestehend aus: einer Erkrankung des zentralen Nervensystems, einer Erkrankung des peripheren Nervensystems, einer Erkrankung des sensorischen Systems, einer Erkrankung des Atmungssystems, einer Erkrankung des Urogenitalsystems, einer Erkrankung des Magen-Darm-Systems, einer Herz-Kreislauf-Erkrankung, einer Skelettmuskelerkrankung und einer Kanalopathie; wobei der Zustand vorzugsweise ausgewählt ist aus der Gruppe bestehend aus: Epilepsie, einer Krampfstörung, einem Anfall, einer Anfallsstörung, einer durch Übererregbarkeit des Nervensystems gekennzeichneten Störung, einer neonatalen Epilepsie, spontanen Kontraktionen, einer früh einsetzenden epileptischen Störung, dem Ohtahara-Syndrom, einer frühinfantile epileptischen Enzephalopathie mit Suppressions-Burst, gutartigen familiären neonatalen Anfällen, einer epileptischen Enzephalopathie, einer schweren sporadischen neonatalen epileptischen Enzephalopathie, einer geistigen Behinderung, einer psychiatrischen oder kognitiven Komorbidität, einem kognitiven Defekt, Angstzuständen, Depressionen, Schizophrenie, plötzlichem Tod durch Epilepsie, medikamentenresistenter Epilepsie, neuropathischen Schmerzen, einem ischämischen Schlaganfall, Angina pectoris, Migräne, einer neurodegenerativen Erkrankung, einer neurologischen Erkrankung, amyotropher Lateralsklerose, Alzheimer-Krankheit, Parkinson-Krankheit, Tremor, paroxysmaler Choreoathetose, Tinnitus, Schmerzen, Asthma, Blasenhyperreaktivität, Harninkontinenz, Verstopfung, Reizdarmsyndrom, Durchfall, Morbus Crohn, Hypertonie, vaskulärer Hypertonie, Muskeldystrophie, einer Skelettmuskelübererregbarkeitserkrankung, Myokymie, Myotonie, einer genetischen Kanalopathie, einer transkriptionellen Kanalopathie, einer Kv7.2- und/oder Kv7.3-bedingten Störung, einer Kv7.2-Enzephalopathie, autosomal-dominanter Taubheit Typ 2 (DFNA2), Ataxie, episodischer Ataxie Typ 1, episodischer Ataxie mit Myokymie-Syndrom, Neuromyotonie, hypokaliämischer periodischer Paralyse, einer Arrhythmie, einer angeborenen Arrhythmie, einem Long-QT-Syndrom, Romano-Ward-Syndrom, Jervell- und Lange-Nielsen-Syndrom, Bartter-Syndrom, hyperinsulinämischer Hypoglykämie im Kindesalter (HHI), schwächerer vasokonstriktorischer Reaktion, Typ-2-Diabetes und Multipler Sklerose.

**5.** Verbindung oder Tautomer, Salz, Solvat, Stereoisomer oder Isotop davon, wie in einem der Ansprüche 1-3 definiert, zur Verwendung als Antikonvulsivum, Analgetikum, Antiischämikum, Antiarrhythmikum, neuroprotektives Medikament, Medikament gegen Tinnitus, Antiasthmatikum, Medikament zur Modulation der gastrointestinalen Motilität, blutdrucksenkendes Medikament, Medikament gegen Myotonie und/oder gegen Skelettmuskeldystrophie.

**6.** Pharmazeutische Zusammensetzung, umfassend die Verbindung oder das Tautomer, Salz, Solvat, Stereoisomer oder Isotop davon, wie in einem der Ansprüche 1-3 definiert, und einen pharmazeutisch verträglichen Träger.

**7.** Pharmazeutische Zusammensetzung nach Anspruch 6, umfassend ein weiteres therapeutisches Mittel, ausgewählt aus: einem Antikonvulsivum, einem Analgetikum und einem nichtsteroidalen Antiphlogistikum (NSAID), wobei das Antikonvulsivum vorzugsweise ausgewählt ist aus der Gruppe bestehend aus: Acetazolamid, Brivaracetam, Carbamazepin, Clobazam, Clonazepam, Diazepam, Eslicarbazepin, Ethosuximid, Fosphenytoin, Gabapentin, Lacosamid, Lamotrigin, Levetiracetam, Lorazepam, Methosuximid, Nitrazepam, Oxcarbazepin, Perampanel, Phenobarbital, Phenytoin, Pregabalin, Primidon, Rufinamid, Stiripentol, Topiramat, Valproinsäure, Vigabatrin, Felbamat, Tiagabin und Zonisamid, wobei das Analgetikum vorzugsweise Acetaminophen oder ein Opioid ist, wobei das nichtsteroidale Antiphlogistikum (NSAID) vorzugsweise ausgewählt ist aus der Gruppe, bestehend aus: Aspirin, Diclofenac, Diflusinal, Etodolac, Fenbufen, Fenoprofen, Flufenisal, Flurbiprofen, Ibuprofen, Indomethacin, Ketoprofen, Ketorolac, Meclofenaminsäure, Mefenaminsäure, Meloxicam, Nabumeton, Naproxen, Nimesulid, Nitroflurbiprofen, Olsalazin, Oxaprozin, Phenylbutazon, Piroxicam, Sulfasalazin, Sulindac, Tolmetin und Zomepirac, wobei das Opioid vorzugsweise Morphin ist.

**8.** Pharmazeutische Zusammensetzung, wie in Anspruch 6 oder 7 definiert, zur Verwendung bei der Behandlung und/oder Vorbeugung eines Zustands, der **gekennzeichnet ist durch** einen Defekt in der Struktur und/oder Funktion mindestens eines spannungsgesteuerten Kaliumkanals, vorzugsweise mindestens eines von Kv7.1, Kv7.2, Kv7.3, Kv7.4 und/oder Kv7.5.

**9.** Verbindung zur Verwendung nach einem der Ansprüche 4-5 oder pharmazeutische Zusammensetzung zur Verwendung nach Anspruch 8, die einem Tier, vorzugsweise einem Haustier oder Nutztier, verabreicht wird.

**10.** Verbindung oder Tautomer, Salz, Solvat, Stereoisomer oder Isotop davon, wie in einem der Ansprüche 1-3 definiert, zur Verwendung bei einem Verfahren zu Modulation eines spannungsgesteuerten Kaliumkanals, wobei der spannungsgesteuerte Kaliumkanal vorzugsweise Kv7.1, Kv7.2, Kv7.3, Kv7.4 und/oder Kv7.5 ist.

**11.** In-vitro-Verfahren zur Modulation eines spannungsgesteuerten Kaliumkanals unter Verwendung der Verbindung oder des Tautomers, Salzes, Solvats, Stereoisomers oder Isotops davon, wie in einem der Ansprüche 1-3 definiert, wobei der spannungsgesteuerte Kaliumkanal vorzugsweise Kv7.1, Kv7.2, Kv7.3, Kv7.4 und/oder Kv7.5 ist.

## Revendications

**1.** Composé de formule générale I :

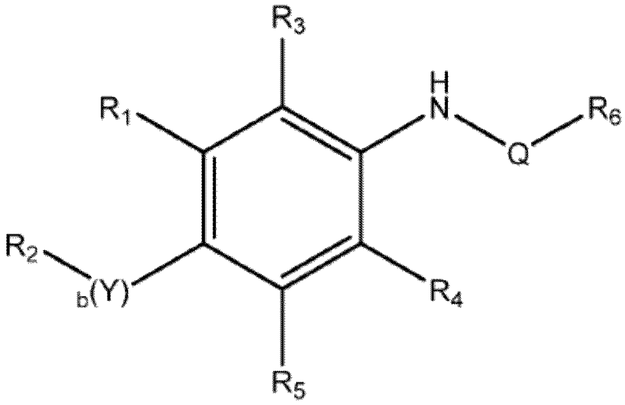

I

ou un tautomère, un sel, un solvate, un stéréoisomère ou un isotope de celui-ci, dans lequel :

Y est choisi parmi : un groupe alkylamino en C1 à C10, un groupe alkyle en C1 à C10, un groupe alcoxy en C1 à C10,
$R_2$ est un cycle aromatique portant éventuellement sur une ou plusieurs positions au moins un substituant indépendamment choisi dans le groupe constitué par : OH, $NO_2$, un atome d'halogène, $NH_2$, un groupe halogénoalkyle en C1 à C3, un groupe halogénoalcoxy en C1 à C3, un groupe alkylcarbonyle en C1 à C6, un groupe acétamidyle, un groupe alkyloxy en C1 à C6 ;
$R_1$, $R_3$, $R_4$ et $R_5$ sont chacun indépendamment choisis dans le groupe constitué par : H, $NH_2$, OH, un atome d'halogène, un groupe alkyle en C1 à C6, un groupe cycloalkyle en C3 à C6, un groupe alkylcarbonyle en C1 à C6, un groupe alcoxycarbonyle en C1 à C6, un groupe alkylamino en C1 à C6, un groupe dialkylamino en C2 à C6, un groupe alkylaminocarbonyle en C1 à C6, un groupe alkyloxy en C1 à C6, un groupe alkylthio en C1 à C6, un groupe alcényle en C2 à C8, un groupe cycloalcényle en C5 à C7, un hétérocycle, un groupe alcynyle en C2 à C8 et un groupe aryle en C5 à C10, l'un quelconque d'entre eux étant éventuellement substitué par un ou plusieurs substituants indépendamment choisis parmi : un groupe hydroxyle, un groupe amine, un groupe thiol, un groupe carboxyle, un acide carboxylique et un atome d'halogène ;
Q est choisi dans le groupe constitué par : C=O, $SO_2$ ;
dans lequel :

lorsque Q est C=O, $R_6$ est choisi parmi : un groupe alkyle en C3 à C30 linéaire ou ramifié, un groupe arylalkyle en C5 à C30 linéaire ou ramifié, un groupe cycloalkyle en C3 à C8, un groupe alkyle en C2 à C4 linéaire ou ramifié substitué par un groupe cycloalkyle en C3 à C8, un groupe alcényle en C2 à C30, un groupe alcynyle en C2 à C30 et l'un quelconque d'entre eux étant éventuellement substitué par un ou plusieurs substituants choisis indépendamment parmi : un atome d'halogène et un groupe cycloalkyle ; et
lorsque Q est $SO_2$, $R_6$ est un groupe cycloalkyle en C3 à C8 ou un groupe aryle et est éventuellement substitué par un ou plusieurs groupes alkyle en C1 à C6.

**2.** Composé selon la revendication 1 qui est choisi parmi :

| | STRUCTURE DU COMPOSÉ | NOM DU COMPOSÉ |
|---|---|---|
| 1. | 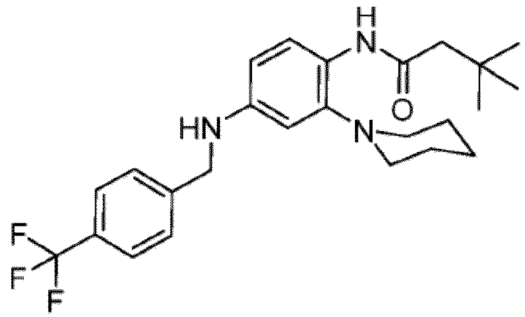 | N-(2-(pipéridin-1-yl)-4-((4-(trifluorométhyl)benzyl) amino)phényl)heptanamide |
| 2. | 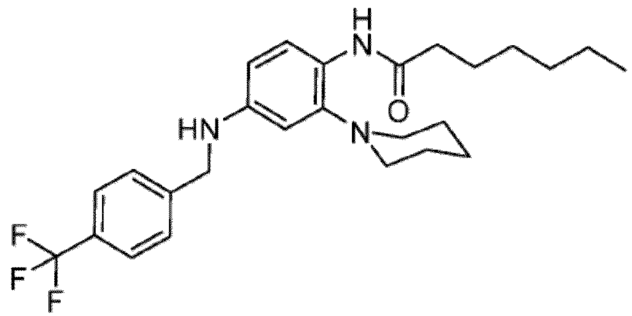 | N-(2-(pipéridin-1-yl)-4-((4-(trifluorométhyl)benzyl) amino)phényl)heptanamide |
| 3. | 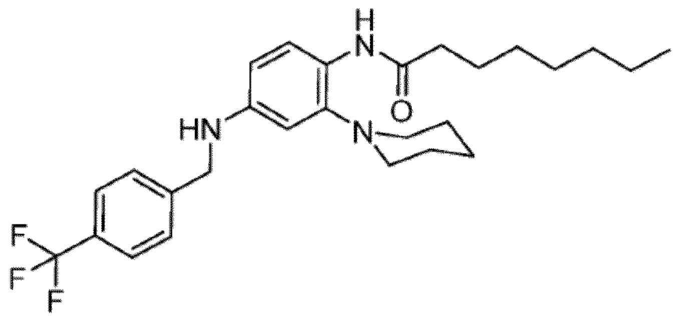 | N-(2-(pipéridin-1-yl)-4-((4-(trifluorométhyl)benzyl) amino)phényl)octanamide |

(continued)

| | STRUCTURE DU COMPOSÉ | NOM DU COMPOSÉ |
|---|---|---|
| 4. | 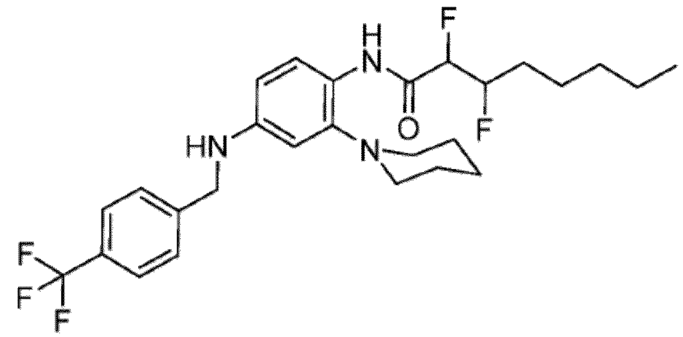 | 2,3-difluoro-N-(2-(pipéridin-1-yl)-4-((4-(trifluorométhyl)benzyl) amino)phényl)octanamide (2R,3S)-2,3-difluoro-N-(2-(pipéridin-1-yl)-4-((4-(trifluorométhyl)benzyl) amino)phényl)octanamide (2S,3R)-2,3-difluoro-N-(2-(pipéridin-1-yl)-4-((4-(trifluorométhyl)benzyl) amino)phényl)octanamide (2R,3R)-2,3-difluoro-N-(2-(pipéridin-1-yl)-4-((4-(trifluorométhyl)benzyl) amino)phényl)octanamide (2S,3S)-2,3-difluoro-N-(2-(pipéridin-1-yl)-4-((4-(trifluorométhyl)benzyl) amino)phényl)octanamide |
| 5. | 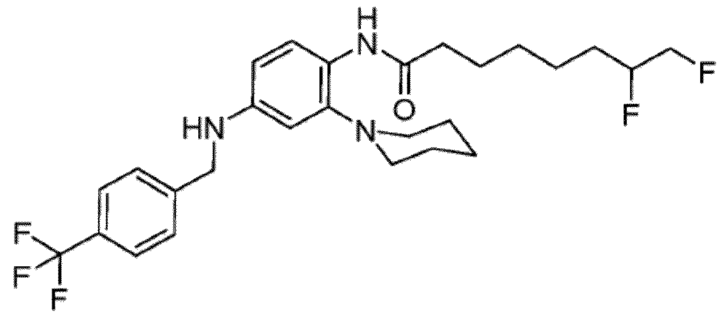 | 7,8-difluoro-N-(2-(pipéridin-1-yl)-4-((4-(trifluorométhyl)benzyl) amino)phényl)octanamide (7R)-7,8-difluoro-N-(2-(pipéridin-1-yl)-4-((4-(trifluorométhyl)benzyl) amino)phényl)octanamide (7S)-7,8-difluoro-N-(2-(pipéridin-1-yl)-4-((4-(trifluorométhyl)benzyl) amino)phényl)octanamide |
| 6. | 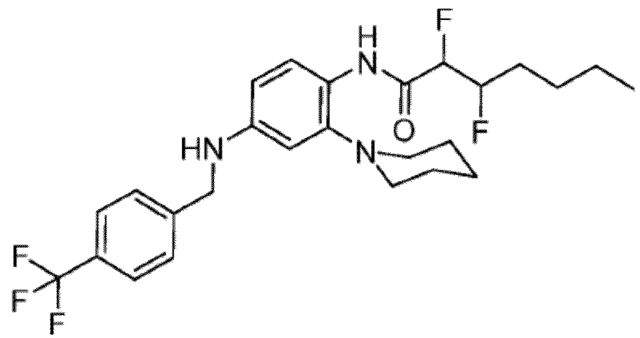 | 2,3-difluoro-N-(2-(pipéridin-1-yl)-4-((4-(trifluorométhyl)benzyl) amino)phényl)heptanamide (2R,3S)-2,3-difluoro-N-(2-(pipéridin-1-yl)-4-((4-(trifluorométhyl)benzyl) amino)phényl)heptanamide (2S,3R)-2,3-difluoro-N-(2-(pipéridin-1-yl)-4-((4-(trifluorométhyl)benzyl) amino)phényl)heptanamide (2R,3R)-2,3-difluoro-N-(2-(pipéridin-1-yl)-4-((4-(trifluorométhyl)benzyl) amino)phényl)heptanamide (2S,3S)-2,3-difluoro-N-(2-(pipéridin-1-yl)-4-((4-(trifluorométhyl)benzyl) amino)phényl)heptanamide |

(continued)

| | STRUCTURE DU COMPOSÉ | NOM DU COMPOSÉ |
|---|---|---|
| 7. | 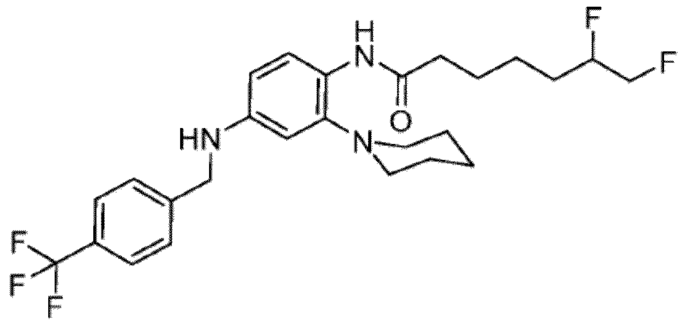 | 6,7-difluoro-N-(2-(pipéridin-1-yl)-4-((4-(trifluorométhyl)benzyl) amino)phényl)heptanamide (6R)-6,7-difluoro-N-(2-(pipéridin-1-yl)-4-((4-(trifluorométhyl)benzyl) amino)phényl) heptanamide (6S)-6,7-difluoro-N-(2-(pipéridin-1-yl)-4-((4-(trifluorométhyl)benzyl) amino) phényl)heptanamide |
| 8. | 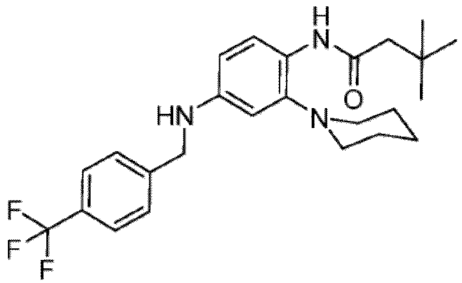 | 3,3-diméthyl-N-(2-(pipéridin-1-yl)-4-((4-(trifluorométhyl)benzyl) amino)phényl)butanamide |
| 9. | 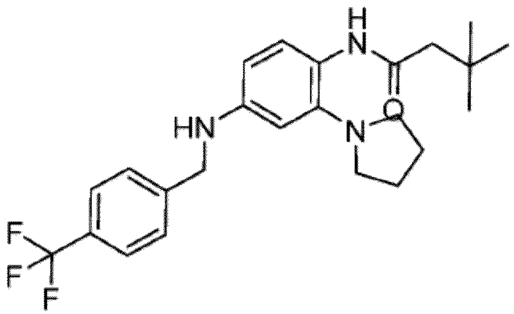 | 3,3-diméthyl-N-(2-(pyrrolidin-1-yl)-4-((4-(trifluorométhyl)benzyl) amino)phényl)butanamide |

(continued)

| | STRUCTURE DU COMPOSÉ | NOM DU COMPOSÉ |
|---|---|---|
| 10 | 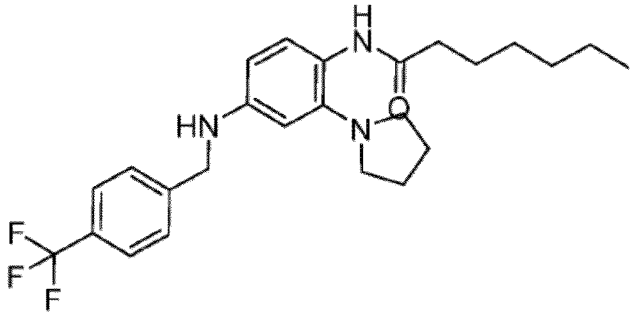 | N-(2-(pyrrolidin-1-yl)-4-((4-(trifluorométhyl)benzyl) amino)phényl)heptanamide |
| 11 | 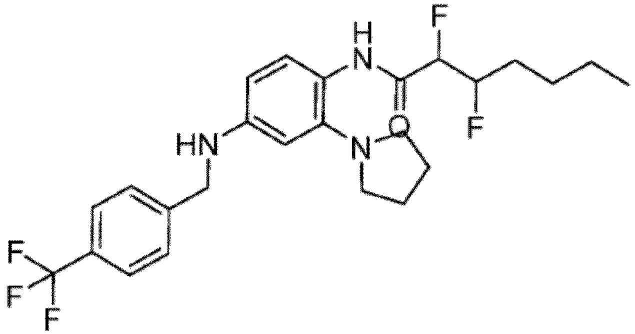 | (2R,3S)-2,3-difluoro-N-(2-(pyrrolidin-1-yl)-4-((4-(trifluorométhyl)benzyl) amino)phényl) heptanamide (2S,3R)-2,3-difluoro-N-(2-(pyrrolidin-1-yl)-4-((4-(trifluorométhyl)benzyl) amino)phényl)heptanamide (2R,3R)-2,3-difluoro-N-(2-(pyrrolidin-1-yl)-4-((4-(trifluorométhyl)benzyl) amino)phényl)heptanamide (2S,3S)-2,3-difluoro-N-(2-(pyrrolidin-1-yl)-4-((4-(trifluorométhyl)benzyl) amino)phényl)heptanamide |
| 12 | 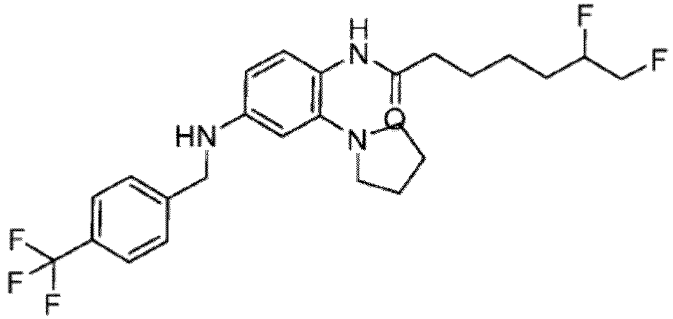 | 6,7-difluoro-N-(2-(pyrrolidin-1-yl)-4-((4-(trifluorométhyl)benzyl) amino)phényl)heptanamide (6R)-6,7-difluoro-N-(2-(pyrrolidin-1-yl)-4-((4-(trifluorométhyl)benzyl) amino)phényl) heptanamide (6S)-6,7-difluoro-N-(2-(pyrrolidin-1-yl)-4-((4-(trifluorométhyl)benzyl) amino)phényl)heptanamide |

(continued)

| | STRUCTURE DU COMPOSÉ | NOM DU COMPOSÉ |
|---|---|---|
| 13 | 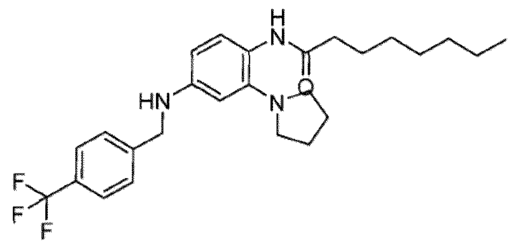 | N-(2-(pyrrolidin-1-yl)-4-((4-(trifluorométhyl)benzyl) amino)phényl)octanamide |
| 14 | 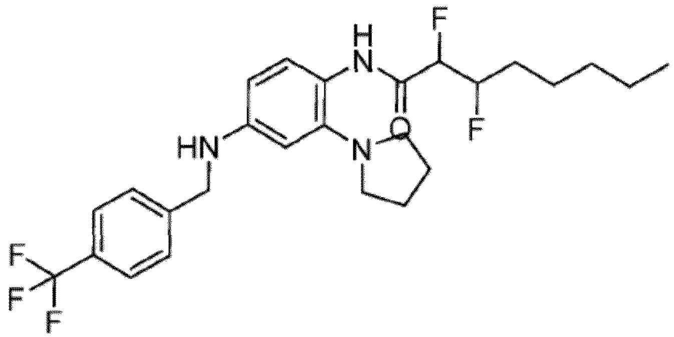 | 2,3-difluoro-N-(2-(pyrrolidin-1-yl)-4-((4-(trifluorométhyl)benzyl) amino)phényl)octanamide (2R,3S)-2,3-difluoro-N-(2-(pyrrolidin-1-yl)-4-((4-(trifluorométhyl)benzyl) amino) phényl)octanamide (2S,3R)-2,3-difluoro-N-(2-(pyrrolidin-1-yl)-4-((4-(trifluorométhyl)benzyl) amino)phényl)octanamide (2R,3R)-2,3-difluoro-N-(2-(pyrrolidin-1-yl)-4-((4-(trifluorométhyl)benzyl) amino)phényl)octanamide (2S,3S)-2,3-difluoro-N-(2-(pyrrolidin-1-yl)-4-((4-(trifluorométhyl)benzyl) amino)phényl)octanamide |
| 15 | 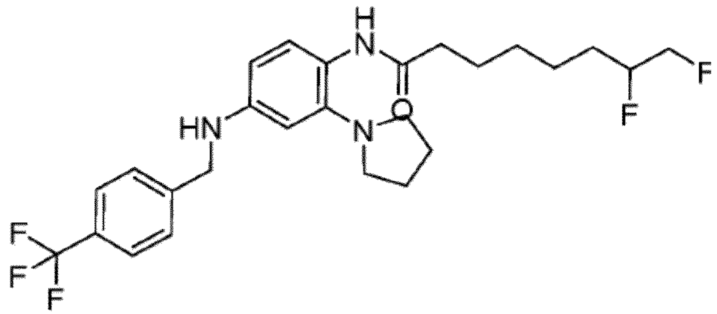 | 7,8-difluoro-N-(2-(pyrrolidin-1-yl)-4-((4-(trifluorométhyl)benzyl) amino)phényl)octanamide (7R)-7,8-difluoro-N-(2-(pyrrolidin-1-yl)-4-((4-(trifluorométhyl)benzyl) amino)phényl) octanamide (7S)-7,8-difluoro-N-(2-(pyrrolidin-1-yl)-4-((4-(trifluorométhyl)benzyl) amino) phényl)octanamide |

(continued)

| | STRUCTURE DU COMPOSÉ | NOM DU COMPOSÉ |
|---|---|---|
| 16 | 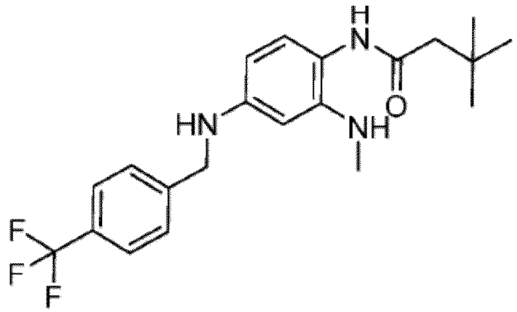 | 3,3-diméthyl-N-(2-(méthylamino)-4-((4-(trifluorométhyl)benzyl) amino)phényl)butanamide |
| 17 | 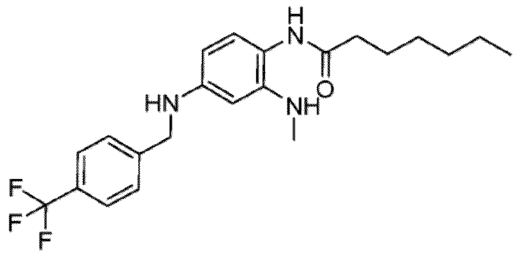 | N-(2-(méthylamino)-4-((4-(trifluorométhyl)benzyl) amino)phényl)heptanamide |
| 18 | 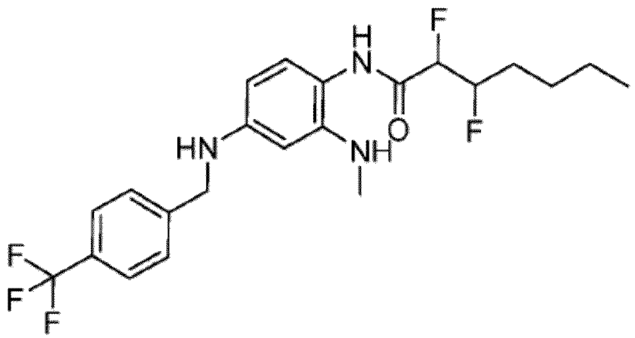 | 2,3-difluoro-N-(2-(méthylamino)-4-((4-(trifluorométhyl)benzyl) amino)phényl)heptanamide (2R,3S)-2,3-difluoro-N-(2-(méthylamino)-4-((4-(trifluorométhyl)benzyl) amino)phényl)heptanamide (2S,3R)-2,3-difluoro-N-(2-(méthylamino)-4-((4-(trifluorométhyl)benzyl) amino)phényl)heptanamide (2R,3R)-2,3-difluoro-N-(2-(méthylamino)-4-((4-(trifluorométhyl)benzyl) amino)phényl)heptanamide (2S,3S)-2,3-difluoro-N-(2-(méthylamino)-4-((4-(trifluorométhyl)benzyl) amino)phényl)heptanamide |

(continued)

| | STRUCTURE DU COMPOSÉ | NOM DU COMPOSÉ |
|---|---|---|
| 19 | 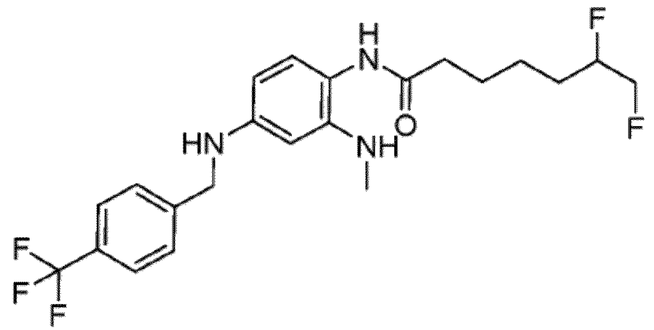 | 6,7-difluoro-N-(2-(méthylamino)-4-((4-(trifluorométhyl)benzyl) amino)phényl)heptanamide (6R)-6,7-difluoro-N-(2-(méthylamino)-4-((4-(trifluorométhyl)benzyl) amino)phényl) heptanamide (6S)-6,7-difluoro-N-(2-(méthylamino)-4-((4-(trifluorométhyl)benzyl) amino) phényl)heptanamide |
| 20 | 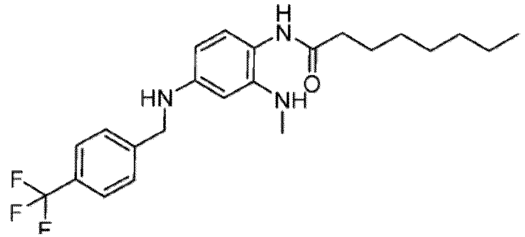 | N-(2-(méthylamino)-4-((4-(trifluorométhyl)benzyl) amino)phényl)octanamide |
| 21 | 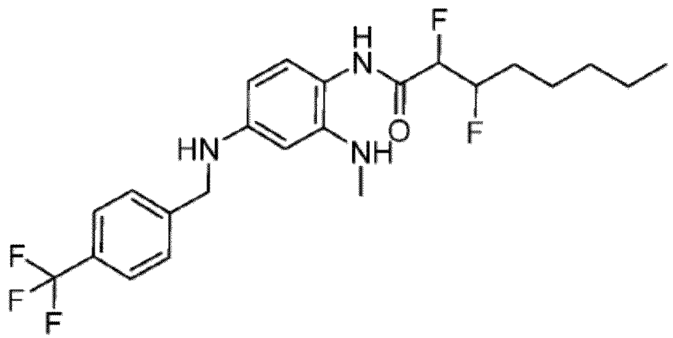 | 2,3-difluoro-N-(2-(méthylamino)-4-((4-(trifluorométhyl)benzyl) amino)phényl)octanamide (2R,3S)-2,3-difluoro-N-(2-(méthylamino)-4-((4-(trifluorométhyl)benzyl) amino)phényl)octanamide (2S,3R)-2,3-difluoro-N-(2-(méthylamino)-4-((4-(trifluorométhyl)benzyl) amino) phényl)octanamide (2R,3R)-2,3-difluoro-N-(2-(méthylamino)-4-((4-(trifluorométhyl)benzyl) amino)phényl)octanamide (2S,3S)-2,3-difluoro-N-(2-(méthylamino)-4-((4-(trifluorométhyl)benzyl) amino)phényl)octanamide |

(continued)

| | STRUCTURE DU COMPOSÉ | NOM DU COMPOSÉ |
|---|---|---|
| 22 | 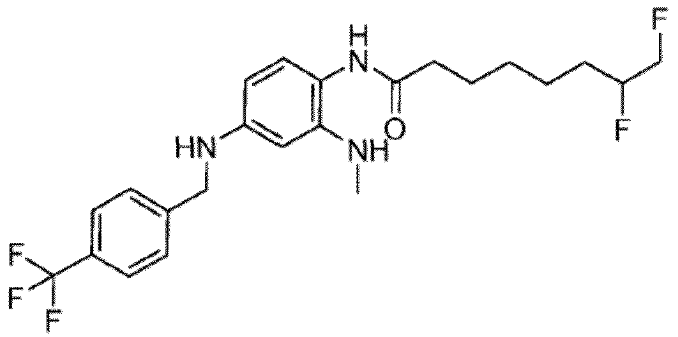 | 7,8-difluoro-N-(2-(méthylamino)-4-((4-(trifluorométhyl)benzyl) amino)phényl)octanamide (7R)-7,8-difluoro-N-(2-(méthylamino)-4-((4-(trifluorométhyl)benzyl) amino)phényl)octanamide (7S)-7,8-difluoro-N-(2-(méthylamino)-4-((4-(trifluorométhyl)benzyl) amino)phényl)octanamide |
| 23 | 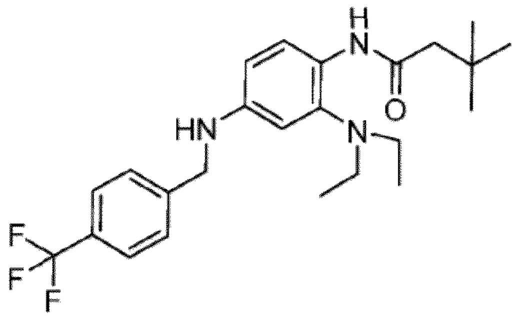 | N-(2-(diéthylamino)-4-((4-(trifluorométhyl)benzyl) amino)phényl)-3,3-diméthylbutanamide |
| 24 | 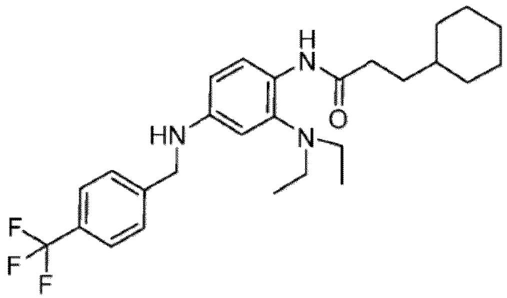 | 3-cyclohexyl-N-(2-(diéthylamino)-4-((4-(trifluorométhyl)benzyl) amino)phényl)propanamide |

(continued)

| | STRUCTURE DU COMPOSÉ | NOM DU COMPOSÉ |
|---|---|---|
| 25 | 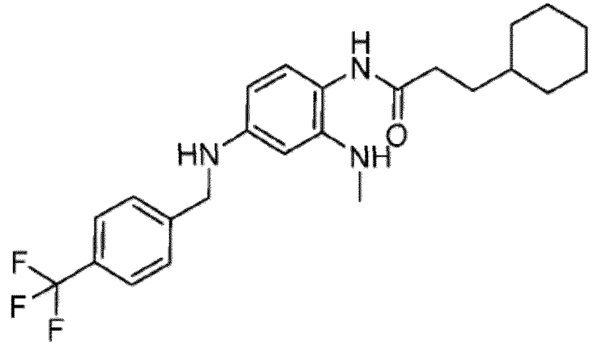 | 3-cyclohexyl-N-(2-(méthylamino)-4-((4-(trifluorométhyl)benzyl) amino)phényl)propanamide |
| 26 | 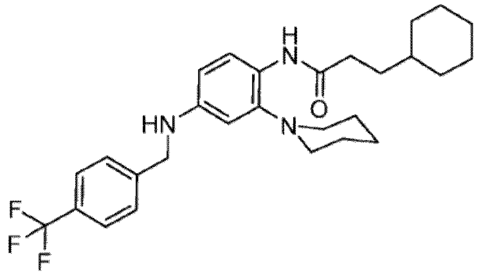 | 3-cyclohexyl-N-(2-(pipéridin-1-yl)-4-((4-(trifluorométhyl)benzyl) amino)phényl)propanamide |
| 27 | 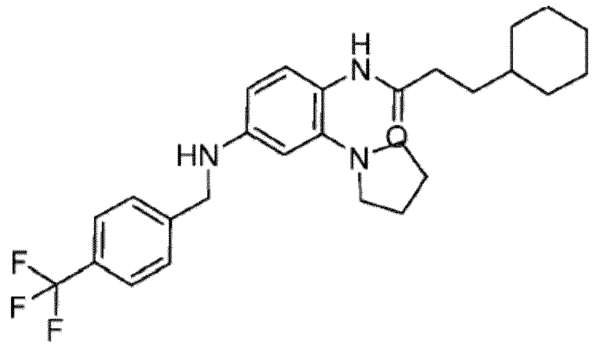 | 3-cyclohexyl-N-(2-(pyrrolidin-1-yl)-4-((4-(trifluorométhyl)benzyl) amino)phényl)propanamide |

(continued)

| | STRUCTURE DU COMPOSÉ | NOM DU COMPOSÉ |
|---|---|---|
| 28 | 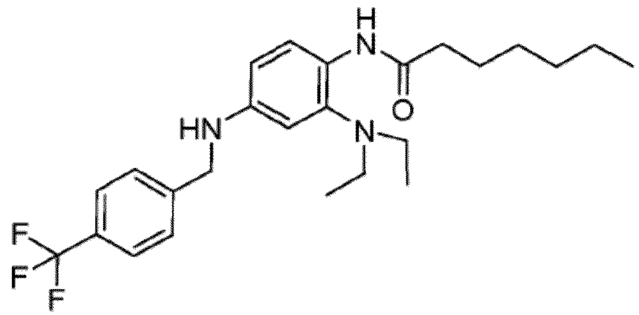 | N-(2-(diéthylamino)-4-((4-(trifluorométhyl)benzyl) amino)phényl)heptanamide |
| 29 | 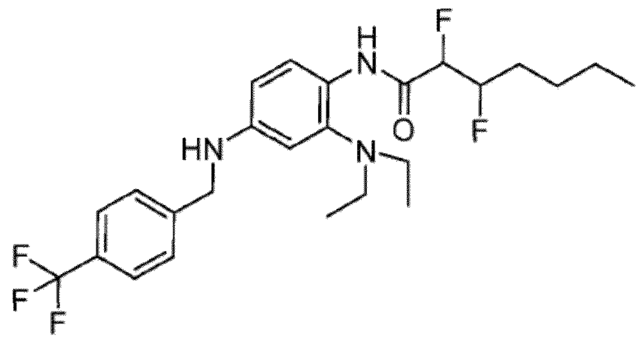 | N-(2-(diéthylamino)-4-((4-(trifluorométhyl)benzyl) amino)phényl)-2,3-difluoroheptanamide (2R, 3S) -N- (2-(diéthylamino)-4-((4-(trifluorométhyl)benzyl) amino)phényl)-2,3-difluoroheptanamide (2S,3R) -N-(2-(diéthylamino)-4-((4-(trifluorométhyl)benzyl) amino) phényl)-2,3-difluoroheptanamide (2R, 3R) -N- (2-(diéthylamino)-4-((4-(trifluorométhyl) benzyl) amino)phényl)-2,3-difluoroheptanamide (2S,3S)-N-(2-(diéthylamino)-4-((4-(trifluorométhyl)benzyl) amino)phényl)-2,3-difluoroheptanamide |
| 30 | 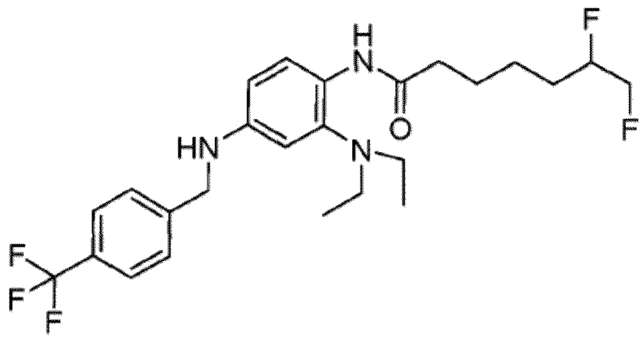 | N-(2-(diéthylamino)-4-((4-(trifluorométhyl)benzyl) amino)phényl)-6,7-difluoroheptanamide (6R)-N-(2-(diéthylamino)-4-((4-(trifluorométhyl)benzyl) amino)phényl)-6,7-difluoroheptanamide (6S)-N-(2-(diéthylamino)-4-((4-(trifluorométhyl)benzyl) amino)phényl)-6,7-difluoroheptanamide |

(continued)

| | STRUCTURE DU COMPOSÉ | NOM DU COMPOSÉ |
|---|---|---|
| 31 | 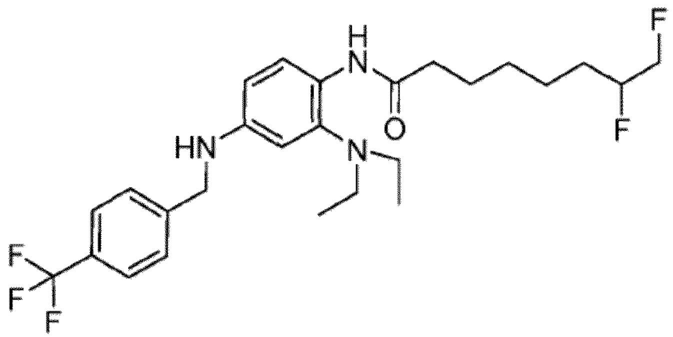 | N-(2-(diéthylamino)-4-((4-(trifluorométhyl)benzyl) amino)phényl)-7,8-difluorooctanamide (7R)-N-(2-(diéthylamino)-4-((4-(trifluorométhyl)benzyl) amino)phényl)-7,8-difluorooctanamide (7S)-N-(2-(diéthylamino)-4-((4-(trifluorométhyl)benzyl) amino)phényl)-7,8-difluorooctanamide |
| 32 | 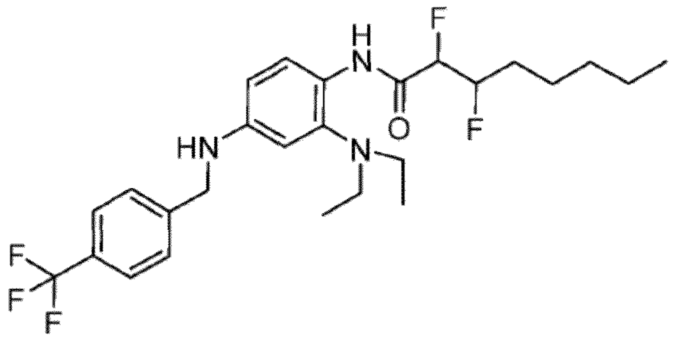 | N-(2-(diéthylamino)-4-((4-(trifluorométhyl)benzyl) amino)phényl)-2,3-difluorooctanamide (2R,3S)-N-(2-(diéthylamino)-4-((4-(trifluorométhyl)benzyl) amino)phényl)-2,3-difluorooctanamide (2S,3R)-N-(2-(diéthylamino)-4-((4-(trifluorométhyl)benzyl) amino)phényl)-2,3-difluorooctanamide (2R, 3R) -N- (2-(diéthylamino)-4-((4-(trifluorométhyl)benzyl) amino) phényl)-2,3-difluorooctanamide (2S,3S)-N-(2-(diéthylamino)-4-((4-(trifluorométhyl)benzyl) amino)phényl)-2,3-difluorooctanamide |
| 33 | 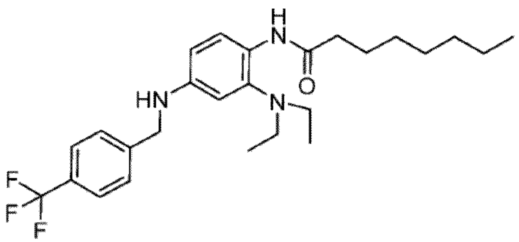 | N-(2-(diéthylamino)-4-((4-(trifluorométhyl)benzyl) amino)phényl)octanamide |

(continued)

| | STRUCTURE DU COMPOSÉ | NOM DU COMPOSÉ |
|---|---|---|
| 34 | 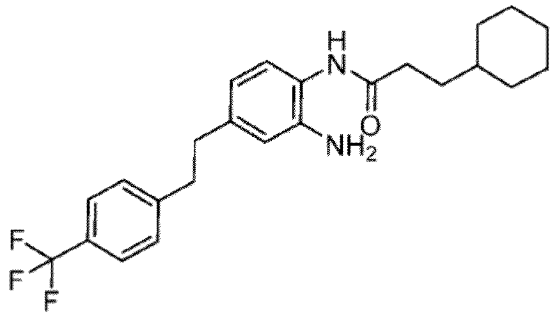 | N-(2-amino-4-(4-(trifluorométhyl)phénéth yl)phényl)-3-cyclohexylpropanamide |
| 35 | 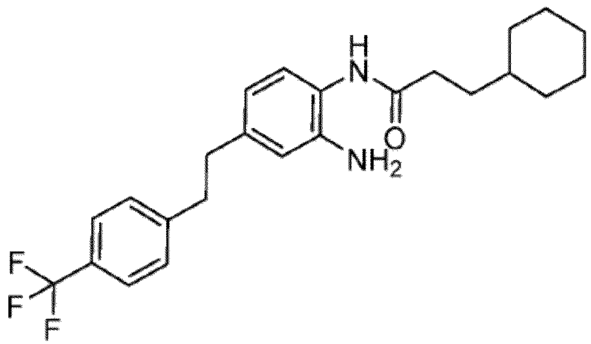 | N-(2-amino-4-(4-(trifluorométhyl)phénéth yl)phényl)-3-cyclohexylpropanamide |
| 36 | 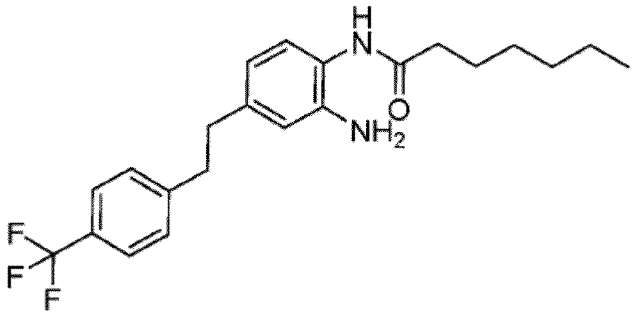 | N-(2-amino-4-(4-(trifluorométhyl)phénéth yl)phényl)heptanamide |

(continued)

| | STRUCTURE DU COMPOSÉ | NOM DU COMPOSÉ |
|---|---|---|
| 37 | 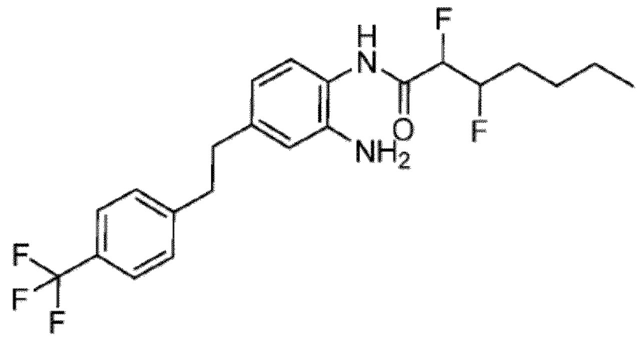 | N-(2-amino-4-(4-(trifluorométhyl)phénéth yl)phényl)-2,3-difluoroheptanamide (2R,3S)-N-(2-amino-4-(4-(trifluorométhyl)phénéth yl)phényl)-2,3-difluoroheptanamide (2S,3R)-N-(2-amino-4-(4-(trifluorométhyl)phénéth yl)phényl)-2,3-difluoroheptanamide (2R,3R)-N-(2-amino-4-(4-(trifluorométhyl)phénéth yl)phényl)-2,3-difluoroheptanamide (2S,3S)-N-(2-amino-4-(4-(trifluorométhyl)phénéth yl)phényl)-2,3-difluoroheptanamide |
| 38 | 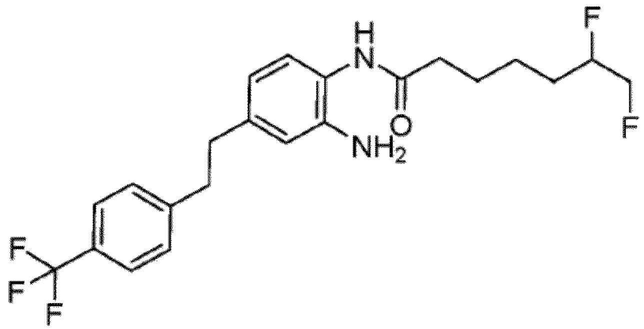 | N-(2-amino-4-(4-(trifluorométhyl)phénéth yl)phényl)-6,7-difluoroheptanamide (6R)-N-(2-amino-4-(4-(trifluorométhyl)phénéth yl)phényl)-6,7-difluoroheptanamide (6S)-N-(2-amino-4-(4-(trifluorométhyl)phénéth yl)phényl)-6,7-difluoroheptanamide |
| 39 | 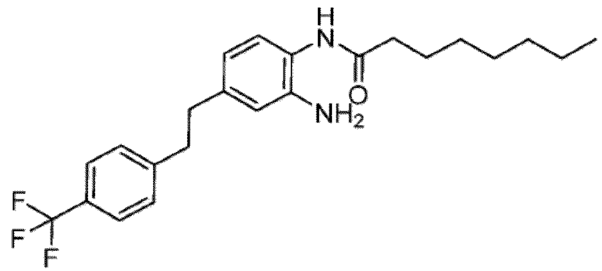 | N-(2-amino-4-(4-(trifluorométhyl)phénéth yl)phényl)octanamide |

(continued)

| | STRUCTURE DU COMPOSÉ | NOM DU COMPOSÉ |
|---|---|---|
| 40 | 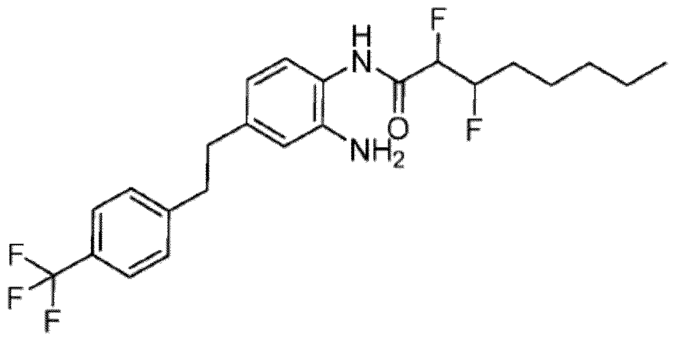 | N-(2-amino-4-(4-(trifluorométhyl)phénéth yl)phényl)-2,3-difluorooctanamide |
| 41 | 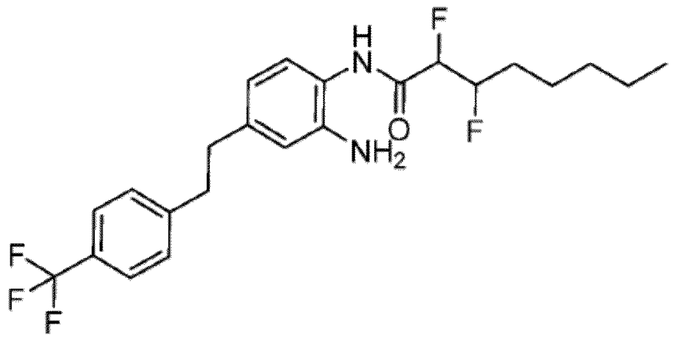 | N-(2-amino-4-(4-(trifluorométhyl)phénéth yl)phényl)-2,3-difluorooctanamide (2R,3S)-N-(2-amino-4-(4-(trifluorométhyl)phénéth yl)phényl)-2,3-difluorooctanamide (2S,3R)-N-(2-amino-4-(4-(trifluorométhyl)phénéth yl)phényl)-2,3-difluorooctanamide (2R,3R)-N-(2-amino-4-(4-(trifluorométhyl)phénéth yl)phényl)-2,3-difluorooctanamide (2S,3S)-N-(2-amino-4-(4-(trifluorométhyl)phénéth yl)phényl)-2,3-difluorooctanamide |
| 42 | 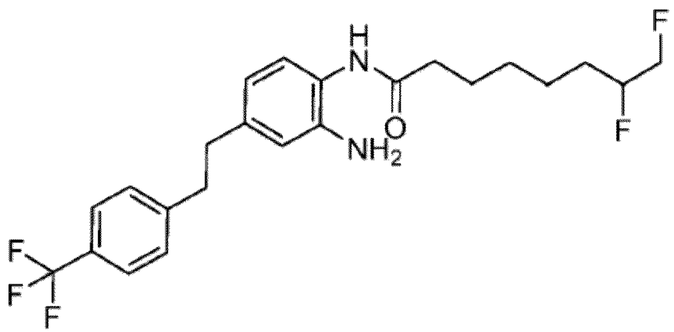 | N-(2-amino-4-(4-(trifluorométhyl)phénéth yl)phényl)-7,8-difluorooctanamide (7R)-N-(2-amino-4-(4-(trifluorométhyl)phénéth yl)phényl)-7,8-difluorooctanamide (7S)-N-(2-amino-4-(4-(trifluorométhyl)phénéth yl)phényl)-7,8-difluorooctanamide |

(continued)

| | STRUCTURE DU COMPOSÉ | NOM DU COMPOSÉ |
|---|---|---|
| 43 | 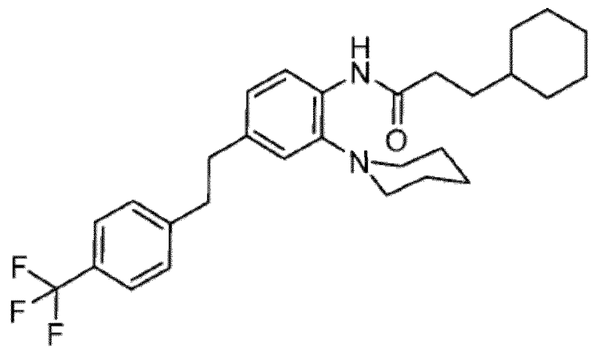 | 3-cyclohexyl-N-(2-pipéridin-1-yl)-4-(4-(trifluorométhyl)phénéth yl)phényl)propanamide |
| 44 | 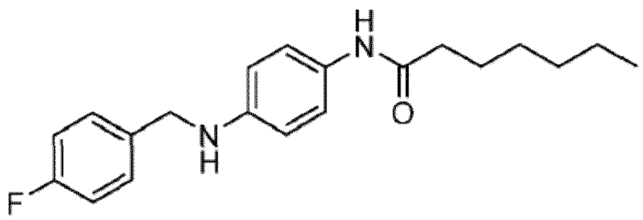 | N-(4-((4-fluorobenzyl)amino)phény l)heptanamide |
| 45 | 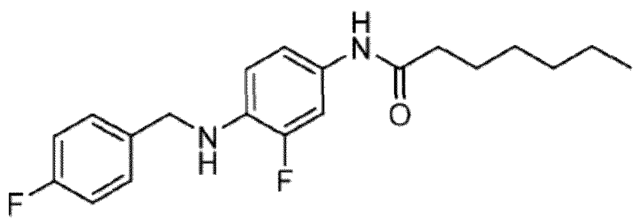 | N-(3-fluoro-4-((4-fluorobenzyl)amino)phény l)heptanamide |
| 46 | 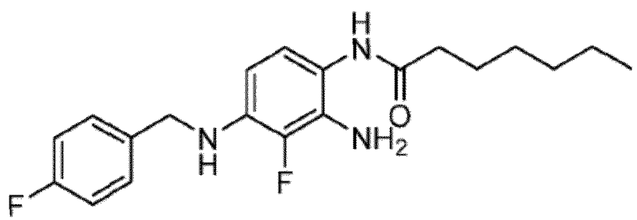 | N-(2-amino-3-fluoro-4-((4-fluorobenzyl)amino)phény l)heptanamide |

(continued)

| | STRUCTURE DU COMPOSÉ | NOM DU COMPOSÉ |
|---|---|---|
| 47 | 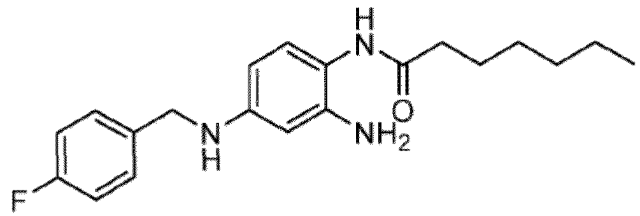 | N-(2-amino-4-((4-fluorobenzyl)amino)phény l)heptanamide |
| 48 | 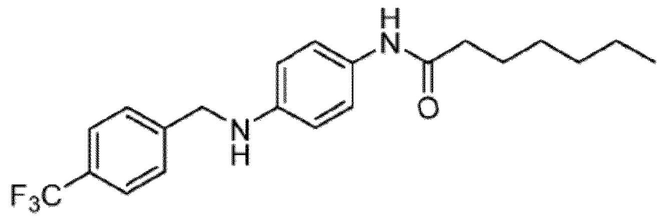 | N-(4-((4-(trifluorométhyl)benzyl) amino)phényl)heptanamide |
| 49 | 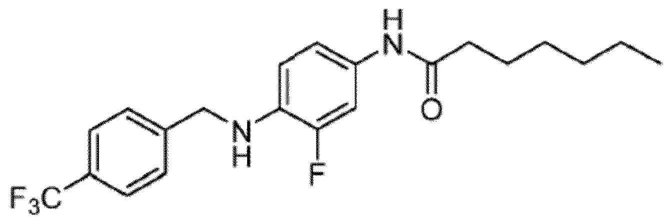 | N-(3-fluoro-4-((4-(trifluorométhyl)benzyl) amino)phényl)heptanamide |
| 50 | 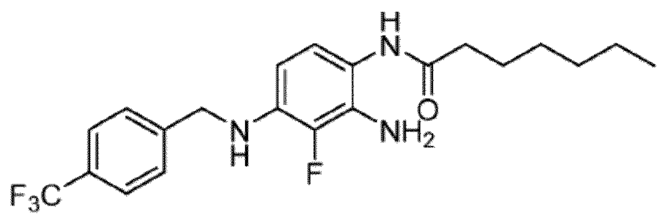 | N-(2-amino-3-fluoro-4-((4-(trifluorométhyl)benzyl) amino)phényl)heptanamide |

(continued)

| | STRUCTURE DU COMPOSÉ | NOM DU COMPOSÉ |
|---|---|---|
| 51 | 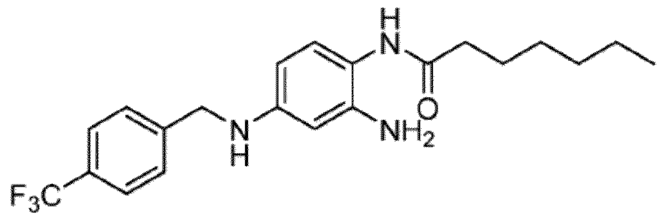 | N-(2-amino-4-((4-(trifluorométhyl)benzyl) amino)phényl)heptanamide |
| 52 | 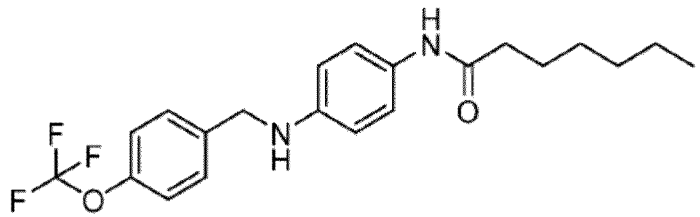 | N-(4-((4-(trifluorométhoxy)benzyl )amino)phényl)heptanamid e |
| 53 | 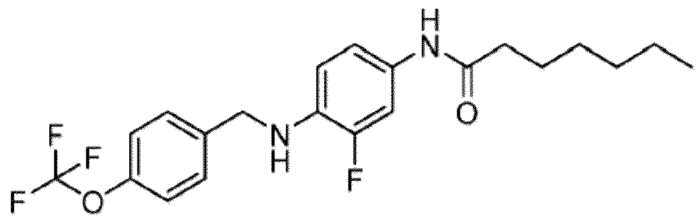 | N-(3-fluoro-4-((4-(trifluorométhoxy)benzyl )amino)phényl)heptanamid e |
| 54 | 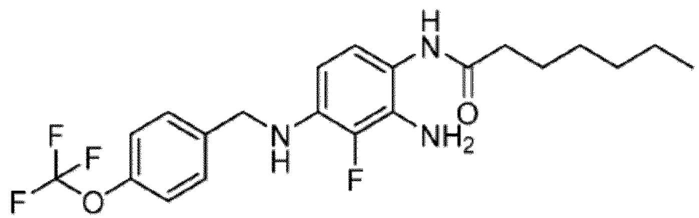 | N-(2-amino-3-fluoro-4-((4-(trifluorométhoxy)benzyl )amino)phényl)heptanamid e |

(continued)

| | STRUCTURE DU COMPOSÉ | NOM DU COMPOSÉ |
|---|---|---|
| 55 | 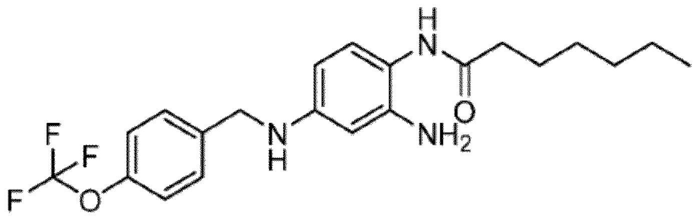 | N-(2-amino-4-((4-(trifluorométhoxy)benzyl )amino)phényl)heptanamid e |
| 56 | 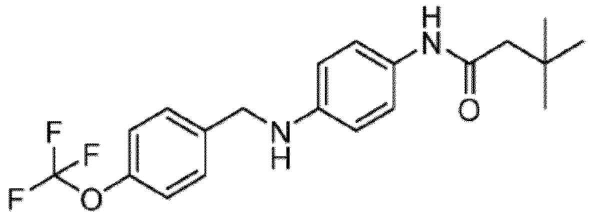 | 3,3-diméthyl-N-(4-((4-(trifluorométhoxy)benzyl )amino)phényl)butanamide |
| 57 | 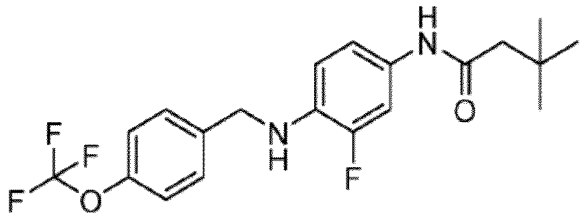 | N-(3-fluoro-4-((4-(trifluorométhoxy)benzyl )amino)phényl)-3,3-diméthylbutanamide |
| 58 | 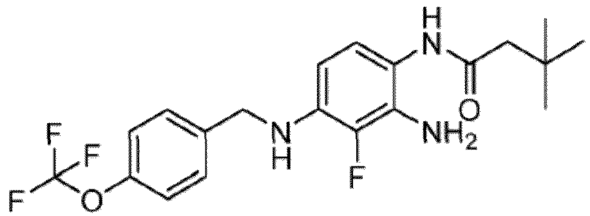 | N-(2-amino-3-fluoro-4-((4-(trifluorométhoxy)benzyl )amino)phényl)-3,3-diméthylbutana-mide |

(continued)

| | STRUCTURE DU COMPOSÉ | NOM DU COMPOSÉ |
|---|---|---|
| 59 | 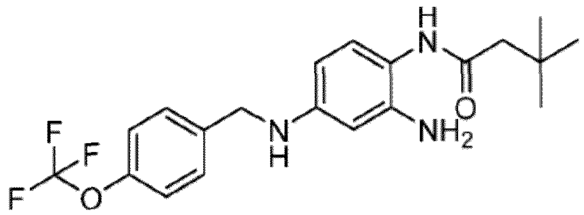 | N-(2-amino-4-((4-(trifluorométhoxy)benzyl )amino)phényl)-3,3-diméthylbutanamide |
| 60 | 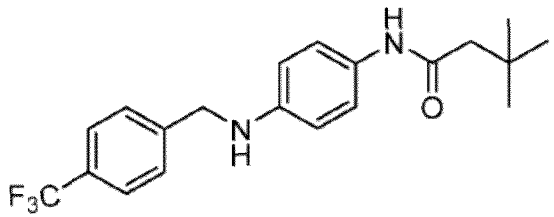 | 3,3-diméthyl-N-(4-((4-(trifluorométhyl)benzyl) amino)phényl)butanamide |
| 61 | 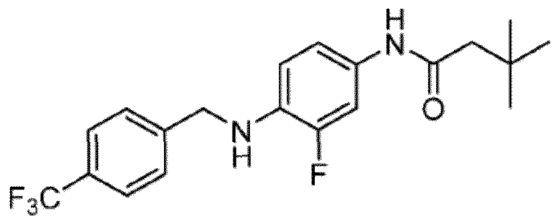 | N-(3-fluoro-4-((4-(trifluorométhyl)benzyl) amino)phényl)-3,3-diméthylbutanamide |
| 62 | 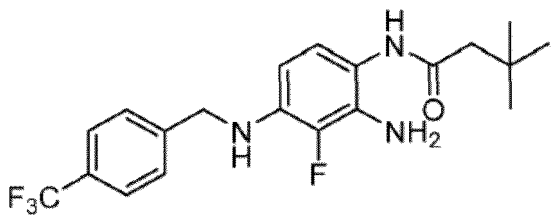 | N-(2-amino-3-fluoro-4-((4-(trifluorométhyl)benzyl) amino)phényl)-3,3-diméthylbutana-mide |

(continued)

| | STRUCTURE DU COMPOSÉ | NOM DU COMPOSÉ |
|---|---|---|
| 63 | 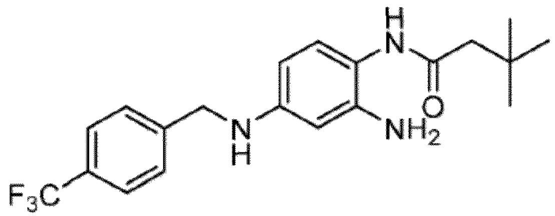 | N-(2-amino-4-((4-(trifluorométhyl)benzyl) amino)phényl)-3,3-diméthylbutanamide |
| 64 | 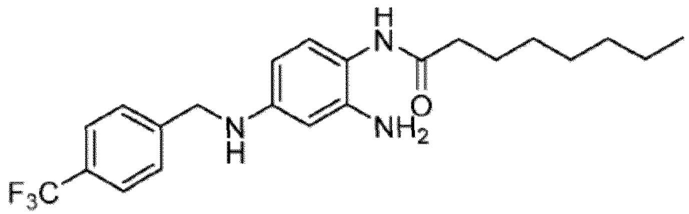 | N-(2-amino-4-((4-(trifluorométhyl)benzyl) amino)phényl)octanamide |
| 65 | 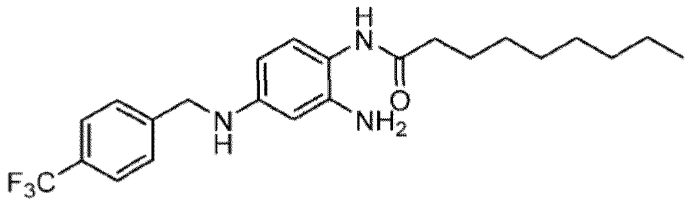 | N-(2-amino-4-((4-(trifluorométhyl)benzyl) amino)phényl)nonanamide |
| 66 | 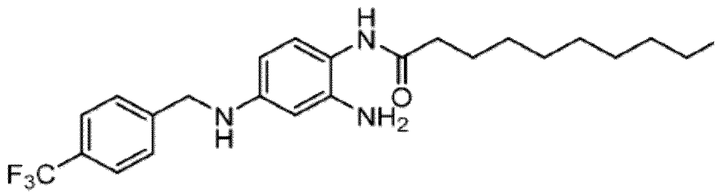 | N-(2-amino-4-((4-(trifluorométhyl)benzyl) amino)phényl)décanamide |
| 67 | 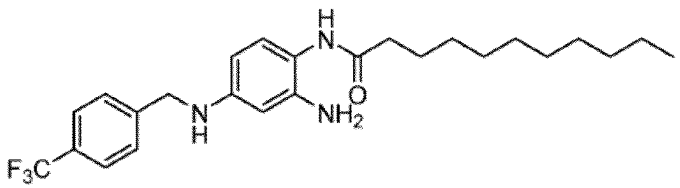 | N-(2-amino-4-((4-(trifluorométhyl)benzyl) amino)phényl)undécanamid e |

(continued)

| | STRUCTURE DU COMPOSÉ | NOM DU COMPOSÉ |
|---|---|---|
| 68 | 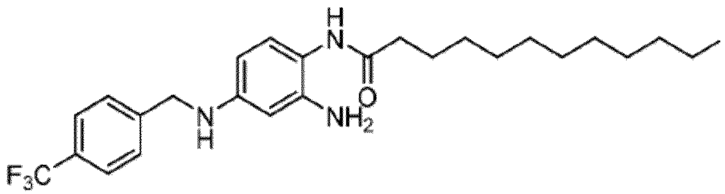 | N-(2-amino-4-((4-(trifluorométhyl)benzyl) amino)phényl)dodécanamid e |
| 69 | 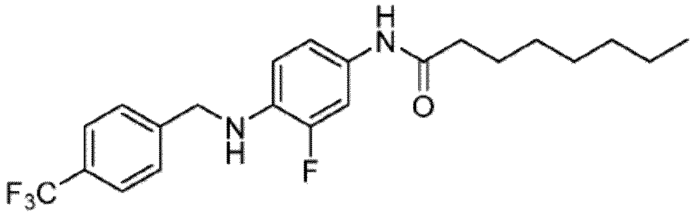 | N-(3-fluoro-4-((4-(trifluorométhyl)benzyl) amino)phényl)octanamide |
| 70 | 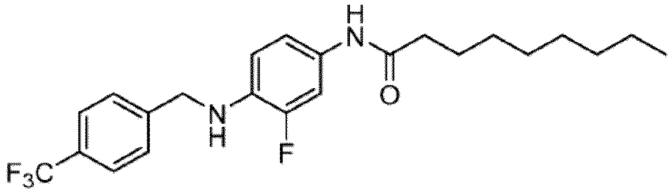 | N-(3-fluoro-4-((4-(trifluorométhyl)benzyl) amino)phényl)nonanamide |
| 71 | 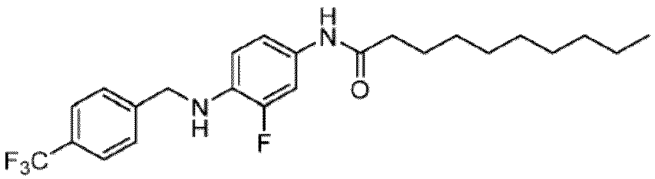 | N-(3-fluoro-4-((4-(trifluorométhyl)benzyl) amino)phényl)décanamide |
| 72 | 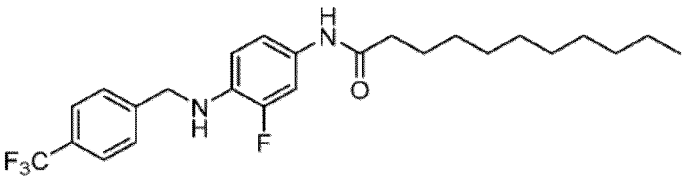 | N-(3-fluoro-4-((4-(trifluorométhyl)benzyl) amino)phényl)undécanamid e |

(continued)

| | STRUCTURE DU COMPOSÉ | NOM DU COMPOSÉ |
|---|---|---|
| 73 | 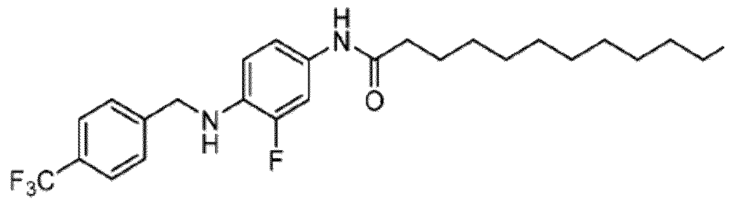 | N-(3-fluoro-4-((4-(trifluorométhyl)benzyl) amino)phényl)dodécanamid e |
| 74 | 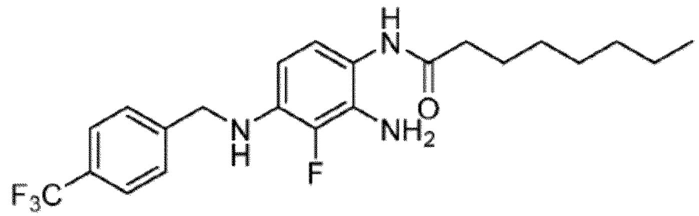 | N-(2-amino-3-fluoro-4-((4-(trifluorométhyl)benzyl) amino)phényl)octanamide |
| 75 | 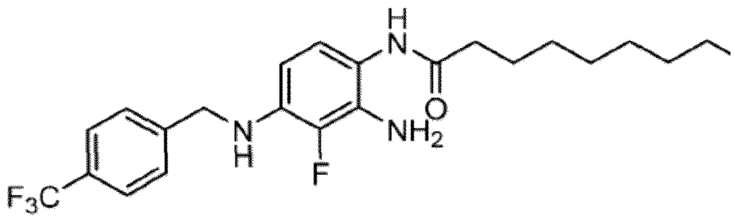 | N-(2-amino-3-fluoro-4-((4-(trifluorométhyl)benzyl) amino)phényl)nonanamide |
| 76 | 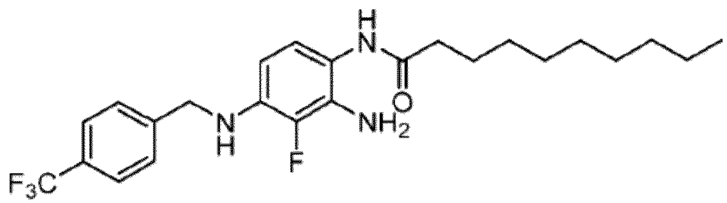 | N-(2-amino-3-fluoro-4-((4-(trifluorométhyl)benzyl) amino)phényl)décanamide |
| 77 | 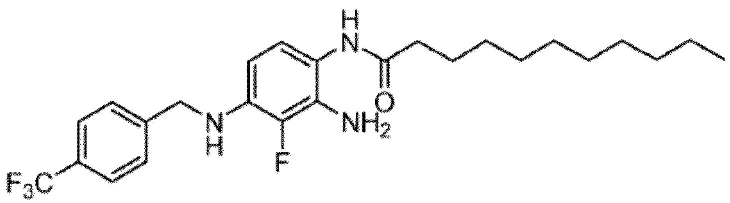 | N-(2-amino-3-fluoro-4-((4-(trifluorométhyl)benzyl) amino)phényl)undécanamid e |

(continued)

| | STRUCTURE DU COMPOSÉ | NOM DU COMPOSÉ |
|---|---|---|
| 78 | 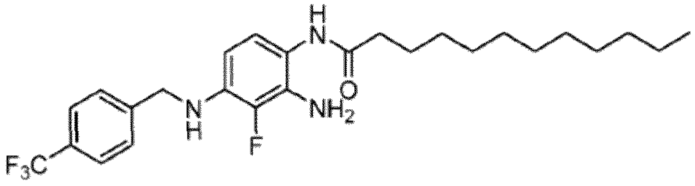 | N-(2-amino-3-fluoro-4-((4-(trifluorométhyl)benzyl) amino)phényl)dodécanamid e |
| 79 | 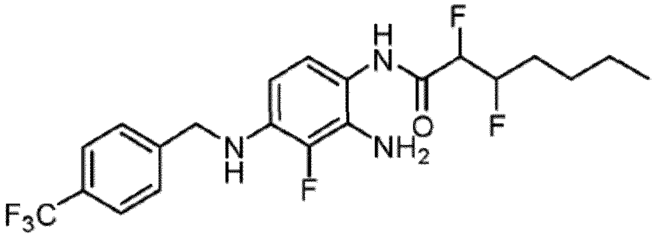 | N-(2-amino-3-fluoro-4-((4-(trifluorométhyl)benzyl) amino)phényl)-2,3-difluoroheptanamide (2R,3S)-N-(2-amino-3-fluoro-4-((4-(trifluorométhyl)benzyl) amino)phényl)-2,3-difluoroheptanamide (2S,3R)-N-(2-amino-3-fluoro-4-((4-(trifluorométhyl)benzyl) amino) phényl)-2,3-difluoroheptanamide (2R,3R)-N-(2-amino-3-fluoro-4-((4-(trifluorométhyl) benzyl) amino)phényl)-2,3-difluoroheptanamide (2S,3S)-N-(2-amino-3-fluoro-4-((4-(trifluorométhyl)benzyl) amino)phényl)-2,3-difluoroheptanamide mélanges racémiques de ceux-ci |
| 80 | 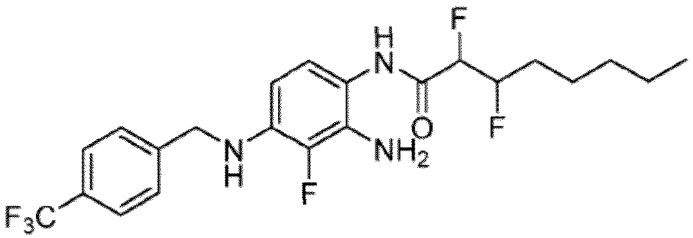 | N-(2-amino-3-fluoro-4-((4-(trifluorométhyl)benzyl) amino)phényl)-2,3-difluorooctanamide (2R,3S)-N-(2-amino-3-fluoro-4-((4-(trifluorométhyl)benzyl) amino)phényl)-2,3-difluorooctanamide (2S,3R)-N-(2-amino-3-fluoro-4-((4-(trifluorométhyl)benzyl) amino)phényl)-2,3-difluorooctanamide (2R,3R)-N-(2-amino-3-fluoro-4-((4-(trifluorométhyl)benzyl) amino)phényl)-2,3-difluorooctanamide (2S,3S)-N-(2-amino-3-fluoro-4-((4-(trifluorométhyl)benzyl) amino)phényl)-2,3-difluorooctanamide mélanges racémiques de ceux-ci |
| 81 | 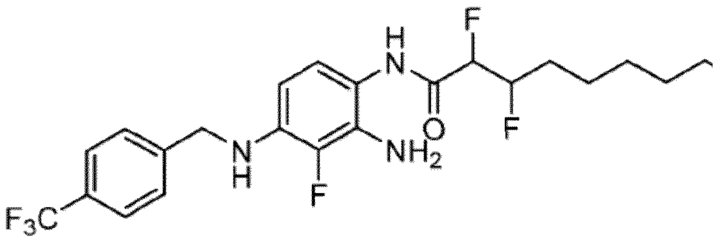 | N-(2-amino-3-fluoro-4-((4-(trifluorométhyl)benzyl) amino)phényl)-2,3-difluorononanamide (2R,3S)-N-(2-amino-3-fluoro-4-((4-(trifluorométhyl)benzyl) amino)phényl)-2,3-difluorononanamide (2S,3R)-N-(2-amino-3-fluoro-4-((4-(trifluorométhyl)benzyl) amino) phényl)-2,3-difluorononanamide (2R,3R)-N-(2-amino-3-fluoro-4-((4-(trifluorométhyl)benzyl) amino)phényl)-2,3-difluoroonoanamide (2S,3S)-N-(2-amino-3-fluoro-4-((4-(trifluorométhyl)benzyl) amino)phényl)-2,3-difluorononanamide mélanges racémiques de ceux-ci |

(continued)

| | STRUCTURE DU COMPOSÉ | NOM DU COMPOSÉ |
|---|---|---|
| 82 | 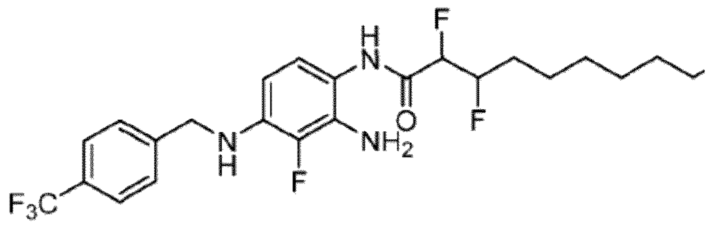 | N-(2-amino-3-fluoro-4-((4-(trifluorométhyl)benzyl) amino)phényl)-2,3-difluorodécanamide (2R,3S)-N-(2-amino-3-fluoro-4-((4-(trifluorométhyl)benzyl) amino)phényl)-2,3-difluorodécanamide (2S,3R)-N-(2-amino-3-fluoro-4-((4-(trifluorométhyl)benzyl) amino)phényl)-2,3-difluorodécanamide (2R,3R)-N-(2-amino-3-fluoro-4-((4-(trifluorométhyl)benzyl) amino)phényl)-2,3-difluorodécanamide (2S,3S)-N-(2-amino-3-fluoro-4-((4-(trifluorométhyl)benzyl) amino)phényl)-2,3-difluorodécanamide mélanges racémiques de ceux-ci |
| 83 | 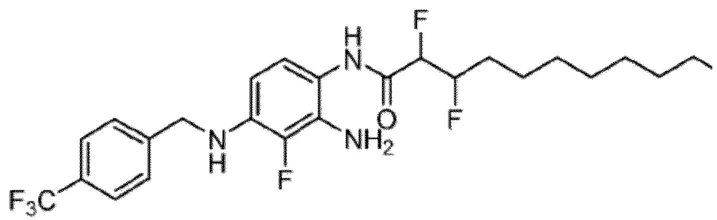 | N-(2-amino-3-fluoro-4-((4-(trifluorométhyl)benzyl) amino)phényl)-2,3-difluoroundécanamide (2R,3S)-N-(2-amino-3-fluoro-4-((4-(trifluorométhyl)benzyl) amino)phényl)-2,3-difluoroundécanamide (2S,3R)-N-(2-amino-3-fluoro-4-((4-(trifluorométhyl)benzyl) amino)phényl)-2,3-difluoroundécanamide (2R,3R)-N-(2-amino-3-fluoro-4-((4-(trifluorométhyl)benzyl) amino)phényl)-2,3-difluoroundécanamide (2S,3S)-N-(2-amino-3-fluoro-4-((4-(trifluorométhyl)benzyl) amino)phényl)-2,3-difluoroundécanamide mélanges racémiques de ceux-ci |
| 84 | 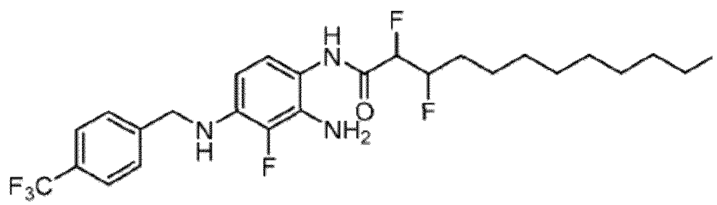 | N-(2-amino-3-fluoro-4-((4-(trifluorométhyl)benzyl) amino)phényl)-2,3-difluorododécanamide (2R,3S)-N-(2-amino-3-fluoro-4-((4-(trifluorométhyl)benzyl) amino)phényl)-2,3-difluorododécanamide (2S,3R)-N-(2-amino-3-fluoro-4-((4-(trifluorométhyl)benzyl) amino)phényl)-2,3-difluorododécanamide (2R,3R)-N-(2-amino-3-fluoro-4-((4-(trifluorométhyl)benzyl) amino)phényl)-2,3-difluorododécanamide (2S,3S)-N-(2-amino-3-fluoro-4-((4-(trifluorométhyl)benzyl) amino)phényl)-2,3-difluorododécanamide mélanges racémiques de ceux-ci |
| 85 | 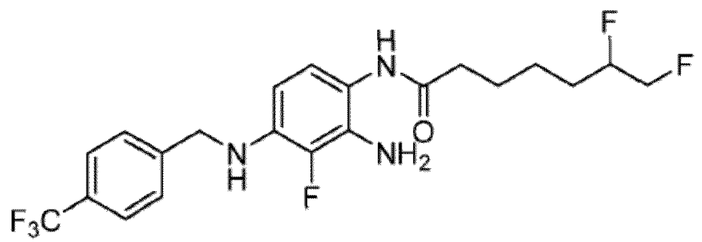 | N-(2-amino-3-fluoro-4-((4-(trifluorométhyl)benzyl) amino)phényl)-6,7-difluoroheptanamide (6R)-N-(2-amino-3-fluoro-4-((4-(trifluorométhyl)benzyl) amino)phényl)-6,7-difluoroheptanamide (6S)-N-(2-amino-3-fluoro-4-((4-(trifluorométhyl)benzyl) amino)phényl)-6,7-difluoroheptanamide mélanges racémiques de ceux-ci |

(continued)

| | STRUCTURE DU COMPOSÉ | NOM DU COMPOSÉ |
|---|---|---|
| 86 | 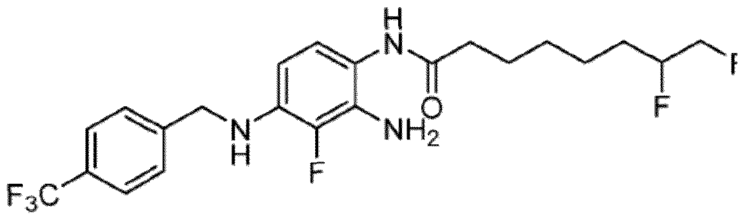 | N-(2-amino-3-fluoro-4-((4-(trifluorométhyl)benzyl) amino)phényl)-6,7-difluorooctanamide (6R)-N-(2-amino-3-fluoro-4-((4-(trifluorométhyl)benzyl) amino)phényl)-6,7-difluorooctanamide (6S)-N-(2-amino-3-fluoro-4-((4-(trifluorométhyl)benzyl) amino)phényl)-6,7-difluorooctanamide mélanges racémiques de ceux-ci |
| 87 | 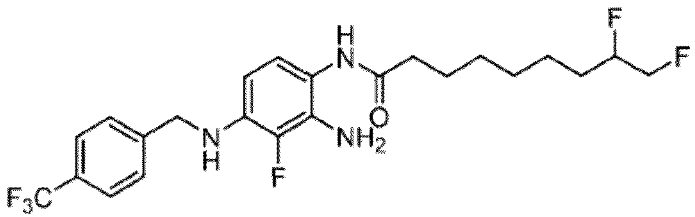 | N-(2-amino-3-fluoro-4-((4-(trifluorométhyl)benzyl) amino)phényl)-8,9-difluorononanamide (8R)-N-(2-amino-3-fluoro-4-((4-(trifluorométhyl)b,enzyl )amino)phényl)-8,9-difluorononanamide (8S)-N-(2-amino-3-fluoro-4-((4-(trifluorométhyl)benzyl) amino)phényl)-8,9-difluorononanamide mélanges racémiques de ceux-ci |
| 88 | 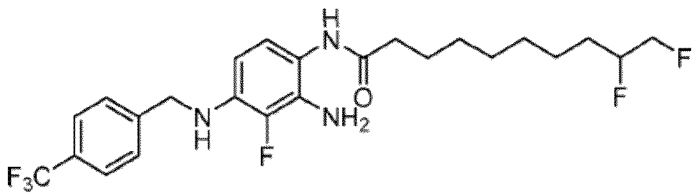 | N-(2-amino-3-fluoro-4-((4-(trifluorométhyl)benzyl) amino)phényl)-9,10-difluorodécanamide (9R)-N-(2-amino-3-fluoro-4-((4-(trifluorométhyl)benzyl) amino)phényl)-9,10-difluorodécanamide (9S)-N-(2-amino-3-fluoro-4-((4-(trifluorométhyl)benzyl) amino)phényl)-9,10-difluorodécanamide mélanges racémiques de ceux-ci |
| 89 | 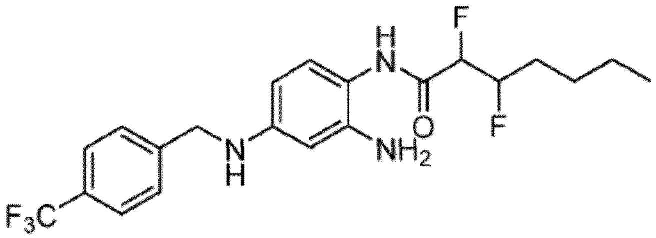 | N-(2-amino-4-((4-(trifluorométhyl)benzyl) amino)phényl)-2,3-difluoroheptanamide (2R,3S)-N-(2-amino-4-((4-(trifluorométhyl)benzyl) amino)phényl)-2,3-difluoroheptanamide (2S,3R)-N-(2-amino-4-((4-(trifluorométhyl)benzyl) amino)phényl)-2,3-difluoroheptanamide (2R,3R)-N-(2-amino-4-((4-(trifluorométhyl)benzyl) amino)phényl)-2,3-difluoroheptanamide (2S,3S)-N-(2-amino-4-((4-(trifluorométhyl)benzyl) amino)phényl)-2,3-difluoroheptanamide mélanges racémiques de ceux-ci |

(continued)

| | STRUCTURE DU COMPOSÉ | NOM DU COMPOSÉ |
|---|---|---|
| 90 | 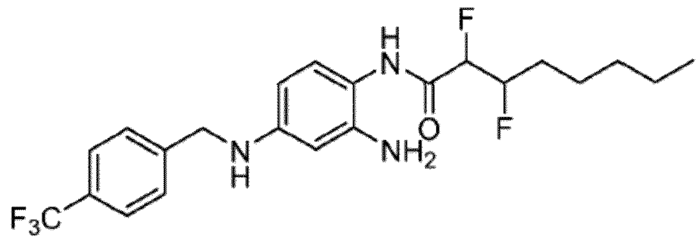 | N-(2-amino-4-((4-(trifluorométhyl)benzyl) amino)phényl)-2,3-difluorooctanamide (2R,3S)-N-(2-amino-4-((4-(trifluorométhyl)benzyl) amino)phényl)-2,3-difluorooctanamide (2S,3R)-N-(2-amino-4-((4-(trifluorométhyl)benzyl) amino)phényl)-2,3-difluorooctanamide (2R,3R)-N-(2-amino-4-((4-(trifluorométhyl)benzyl) amino)phényl)-2,3-difluorooctanamide (2S,3S)-N-(2-amino-4-((4-(trifluorométhyl)benzyl) amino)phényl)-2,3-difluorooctanamide mélanges racémiques de ceux-ci |
| 91 | 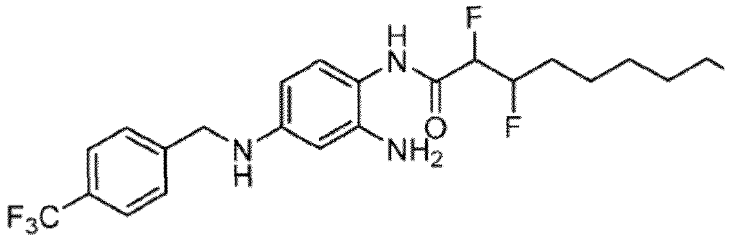 | N-(2-amino-4-((4-(trifluorométhyl)benzyl) amino)phényl)-2,3-difluorononanamide (2R,3S)-N-(2-amino-4-((4-(trifluorométhyl)benzyl) amino)phényl)-2,3-difluorononanamide (2S,3R)-N-(2-amino-4-((4-(trifluorométhyl)benzyl) amino)phényl)-2,3-difluorononanamide (2R,3R)-N-(2-amino-4-((4-(trifluorométhyl)benzyl) amino)phényl)-2,3-difluorononanamide (2S,3S)-N-(2-amino-4-((4-(trifluorométhyl)benzyl) amino)phényl)-2,3-difluorononanamide mélanges racémiques de ceux-ci |
| 92 | 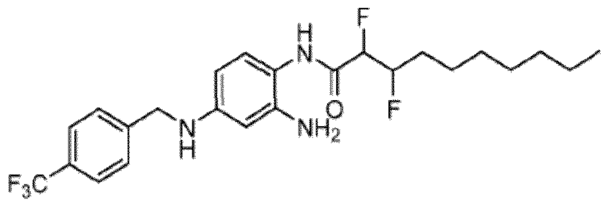 | N-(2-amino-4-((4-(trifluorométhyl)benzyl) amino)phényl)-2,3-difluorodécanamide (2R,3S)-N-(2-amino-4-((4-(trifluorométhyl)benzyl) amino)phényl)-2,3-difluorodécanamide (2S,3R)-N-(2-amino-4-((4-(trifluorométhyl)benzyl) amino)phényl)-2,3-difluorodécanamide (2R,3R)-N-(2-amino-4-((4-(trifluorométhyl)benzyl) amino)phényl)-2,3-difluorodécanamide (2S,3S)-N-(2-amino-4-((4-(trifluorométhyl)benzyl) amino)phényl)-2,3-difluorodécanamide mélanges racémiques de ceux-ci |
| 93 | 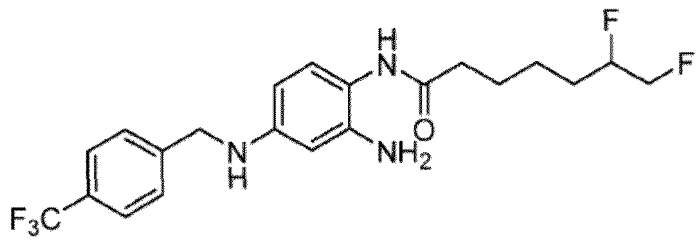 | N-(2-amino-4-((4-(trifluorométhyl)benzyl) amino)phényl)-6,7-difluoroheptanamide (6R)-N-(2-amino-4-((4-(trifluorométhyl)benzyl) amino)phényl)-6,7-difluoroheptanamide (6S)-N-(2-amino-4-((4-(trifluorométhyl)benzyl) amino)phényl)-6,7-difluoroheptanamide mélanges racémiques de ceux-ci |

(continued)

| | STRUCTURE DU COMPOSÉ | NOM DU COMPOSÉ |
|---|---|---|
| 94 | 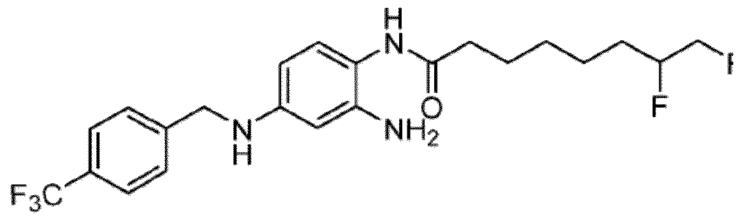 | N-(2-amino-4-((4-(trifluorométhyl)benzyl) amino)phényl)-7,8-difluorooctanamide (7R)-N-(2-amino-4-((4-(trifluorométhyl)benzyl) amino)phényl)-7,8-difluorooctanamide (7S)-N-(2-amino-4-((4-(trifluorométhyl)benzyl) amino)phényl)-7,8-difluorooctanamide mélanges racémiques de ceux-ci |
| 95 | 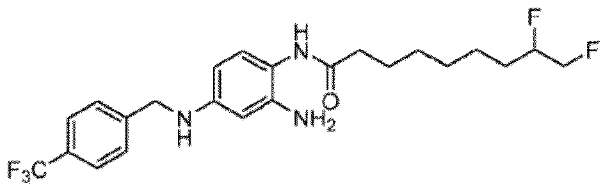 | N-(2-amino-3-fluoro-4-((4-(trifluorométhyl)benzyl) amino)phényl)-6,7-difluorononanamide (6R)-N-(2-amino-4-((4-(trifluorométhyl)benzyl) amino)phényl)-6,7-difluorononanamide (6S)-N-(2-amino-4-((4-(trifluorométhyl)benzyl) amino)phényl)-6,7-difluorononanamide mélanges racémiques de ceux-ci |
| 96 | 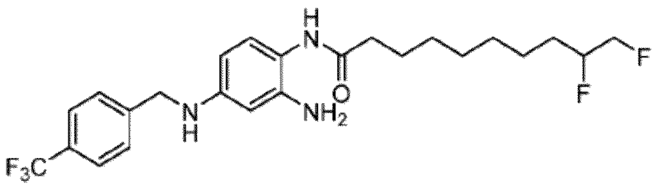 | N-(2-amino-3-fluoro-4-((4-(trifluorométhyl)benzyl) amino)phényl)-6,7-difluorodécanamide (6R)-N-(2-amino-3-fluoro-4-((4-(trifluorométhyl)benzyl) amino)phényl)-6,7-difluorodécanamide (6S)-N-(2-amino-3-fluoro-4-((4-(trifluorométhyl)benzyl) amino)phényl)-6,7-difluorodécanamide mélanges racémiques de ceux-ci |
| 97 | 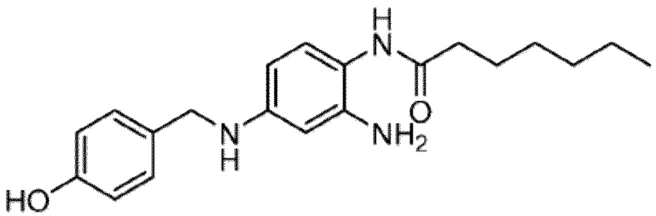 | N-(2-amino-4-((4-hydroxybenzyl)amino)phén yl)heptanamide |
| 98 | 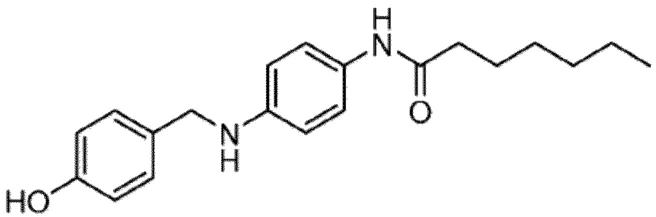 | N-(4-((4-hydroxybenzyl)amino)phén yl)heptanamide |

(continued)

| | STRUCTURE DU COMPOSÉ | NOM DU COMPOSÉ |
|---|---|---|
| 99 | 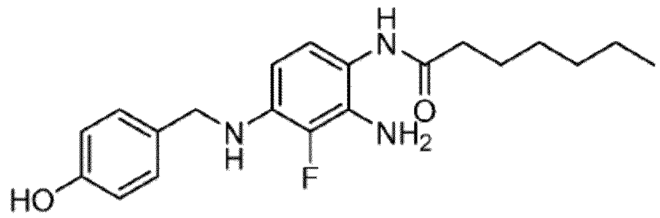 | N-(2-amino-3-fluoro-4-((4-hydroxybenzyl)amino)phén yl)heptanamide |
| 10 | 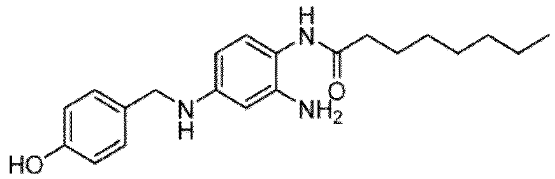 | N-(2-amino-4-((4-hydroxybenzyl)amino)phén yl)octanamide |
| 10 | 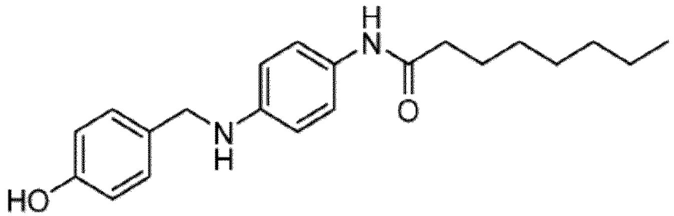 | N-(4-((4-hydroxybenzyl)amino)phén yl)octanamide |
| 10 | 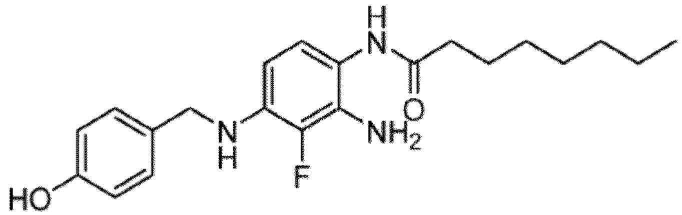 | N-(2-amino-3-fluoro-4-((4-hydroxybenzyl)amino)phén yl)octanamide |
| 10 | 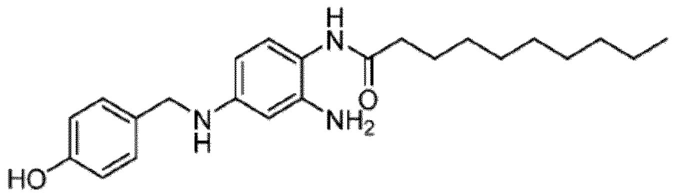 | N-(2-amino-4-((4-hydroxybenzyl)amino)phén yl)décanamide |

(continued)

| | STRUCTURE DU COMPOSÉ | NOM DU COMPOSÉ |
|---|---|---|
| 10 | 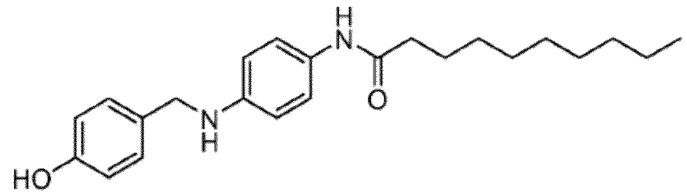 | N-(4-((4-hydroxybenzyl)amino)phén yl)décanamide |
| 10 | 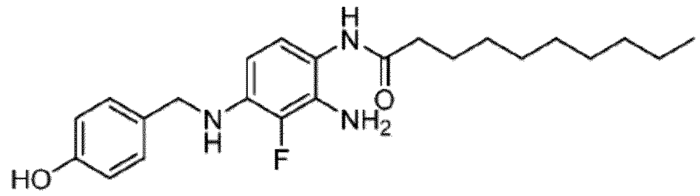 | N-(2-amino-3-fluoro-4-((4-hydroxybenzyl)amino)phén yl)décanamide |
| 10 | 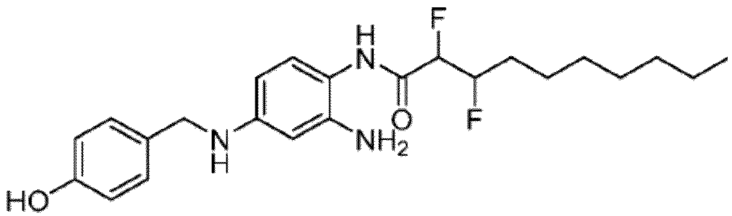 | N-(2-amino-4-((4-hydroxybenzyl)amino)phén yl)-2,3-difluorodécanamide (2R,3S)-N-(2-amino-4-((4-hydroxybenzyl)amino)phén yl)-2,3-difluorodécanamide (2S,3R)-N-(2-amino-4-((4-hydroxybenzyl)amino)phén yl)-2,3-difluorodécanamide (2R,3R)-N-(2-amino-4-((4-hydroxybenzyl)amino)phén yl)-2,3-difluorodécanamide (2S,3S)-N-(2-amino-4-((4-hydroxybenzyl)amino)phén yl)-2,3-difluorodécanamide mélanges racémiques de ceux-ci |
| 10 | 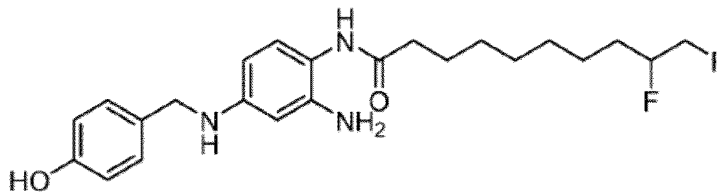 | N-(2-amino-4-((4-hydroxybenzyl)amino)phén yl)-9,10-difluorodécanamide (9R)-N-(2-amino-4-((4-hydroxybenzyl)amino)phén yl)-2,3-difluorodécanamide (9S)-N-(2-amino-4-((4-hydroxybenzyl)amino)phén yl)-2,3-difluorodécanamide mélanges racémiques de ceux-ci |
| 10 | 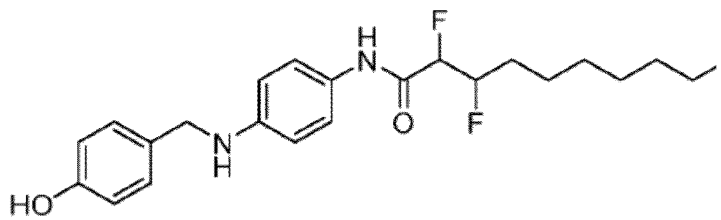 | 2,3-difluoro-N-(4-((4-hydroxybenzyl)amino)phén yl)déca décanamide (2R,3S)-2,3-difluoro-N-(4-((4-hydroxybenzyl)amino)phén yl)déca décanamide (2S,3R)-2,3-difluoro-N-(4-((4-hydroxybenzyl)amino)phén yl)déca décanamide (2R,3R)-2,3-difluoro-N-(4-((4-hydroxybenzyl)amino)phén yl)déca décanamide (2S,3S)-2,3-difluoro-N-(4-((4-hydroxybenzyl)amino)phén yl)déca décanamide mélanges racémiques de ceux-ci |

(continued)

| | STRUCTURE DU COMPOSÉ | NOM DU COMPOSÉ |
|---|---|---|
| 10 | 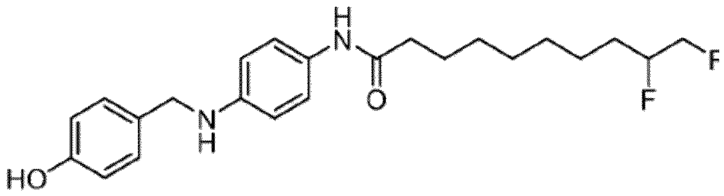 | 9,10-difluoro-N-(4-((4-hydroxybenzyl)amino)phén yl)décanamide (9R)-9,10-difluoro-N-(4-((4-hydroxybenzyl)amino)phén yl)décanamide (9S)-9,10-difluoro-N-(4-((4-hydroxybenzyl)amino)phén yl)décanamide mélanges racémiques de ceux-ci |
| 11 | 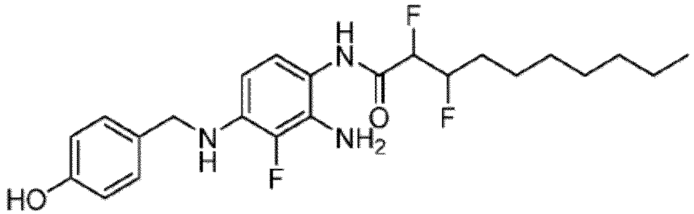 | N-(2-amino-3-fluoro-4-((4-hydroxybenzyl)amino)phén yl)-2,3-difluorodécanamide (2R,3S)-N-(2-amino-3-fluoro-4-((4-hydroxybenzyl)amino)phén yl)-2,3-difluorodécanamide (2S,3R)-N-(2-amino-3-fluoro-4-((4-hydroxybenzyl)amino)phén yl)-2,3-difluorodécanamide (2R,3R)-N-(2-amino-3-fluoro-4-((4-hydroxybenzyl)amino)phén yl)-2,3-difluorodécanamide (2S,3S)-N-(2-amino-3-fluoro-4-((4-hydroxybenzyl)amino)phén yl)-2,3-difluorodécanamide mélanges racémiques de ceux-ci |
| 11 | 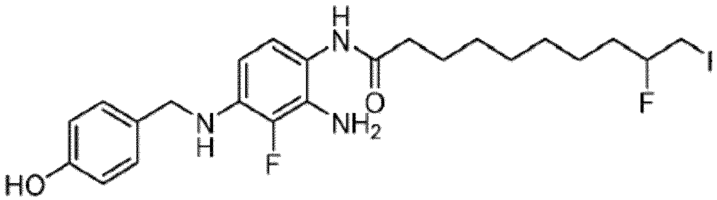 | N-(2-amino-3-fluoro-4-((4-hydroxybenzyl)amino)phén yl)-9,10-difluorodécanamide (9R)-N-(2-amino-3-fluoro-4-((4-hydroxybenzyl)amino)phén yl)-9,10-difluorodécanamide (9S)-N-(2-amino-3-fluoro-4-((4-hydroxybenzyl)amino)phén yl)-9,10-difluorodécanamide mélanges racémiques de ceux-ci |
| 11 | 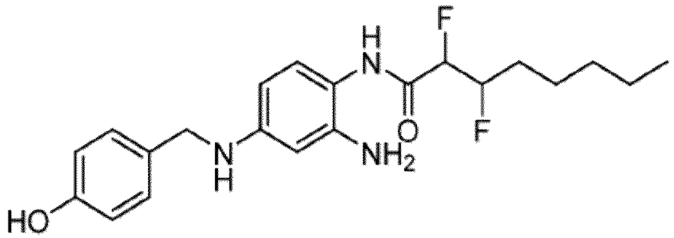 | N-(2-amino-4-((4-hydroxybenzyl)amino)phén yl)-2,3-difluorooctanamide (2R,3S)-N-(2-amino-4-((4-hydroxybenzyl)amino)phényl)-2,3-difluorooctanamide (2S,3R)-N-(2-amino-4-((4-hydroxybenzyl)amino)phén yl)-2,3-difluorooctanamide (2R,3R)-N-(2-amino-4-((4-hydroxybenzyl)amino)phén yl)-2,3-difluorooctanamide (2S,3S)-N-(2-amino-4-((4-hydroxybenzyl)amino)phén yl)-2,3-difluorooctnamide mélanges racémiques de ceux-ci |

(continued)

| | STRUCTURE DU COMPOSÉ | NOM DU COMPOSÉ |
|---|---|---|
| 11 | 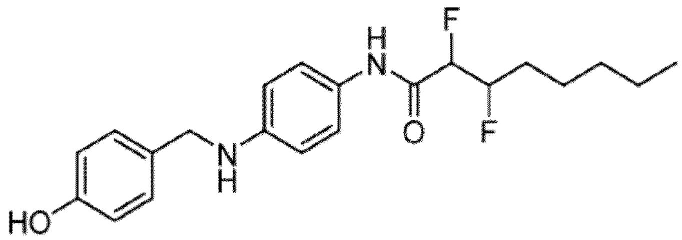 | 2,3-difluoro-N-(4-((4-hydroxybenzyl)amino)phén yl)octanamide (2R,3S)-2,3-difluoro-N-(4-((4-hydroxybenzyl)amino)phén yl)octanamide (2S,3R)-2,3-difluoro-N-(4-((4-hydroxybenzyl)amino)phén yl)octanamide (2R,3R)-2,3-difluoro-N-(4-((4-hydroxybenzyl)amino)phén yl)octanamide (2S,3S)-2,3-difluoro-N-(4-((4-hydroxybenzyl)amino)phén yl)octanamide mélanges racémiques de ceux-ci |
| 11 | 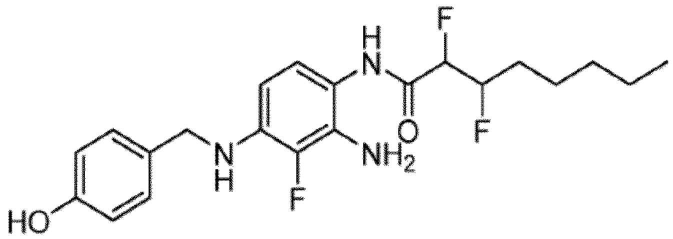 | N-(2-amino-3-fluoro-4-((4-hydroxybenzyl)amino)phén yl)-2,3-difluorooctanamide (2R,3S)-N-(2-amino-3-fluoro-4-((4-hydroxybenzyl)amino)phén yl)-2,3-difluorooctanamide (2S,3R)-N-(2-amino-3-fluoro-4-((4-hydroxybenzyl)amino)phén yl)-2,3-difluorooctanamide (2R,3R)-N-(2-amino-3-fluoro-4-((4-hydroxybenzyl)amino)phén yl)-2,3-difluorooctanamide (2S,3S)-N-(2-amino-3-fluoro-4-((4-hydroxybenzyl)amino)phén yl)-2,3-difluorooctanamide mélanges racémiques de ceux-ci |
| 11 | 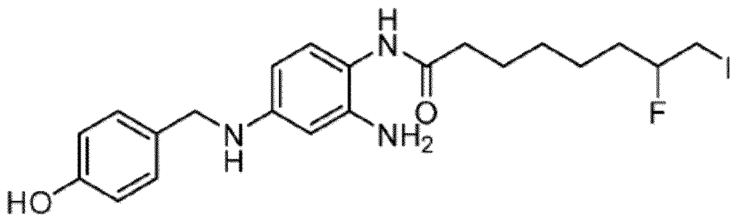 | N-(2-amino-4-((4-hydroxybenzyl)amino)phén yl)-7,8-difluorooctanamide (7S)-N-(2-amino-4-((4-hydroxybenzyl)amino)phényl)-7,8-difluorooctanamide (7R)-N-(2-amino-4-((4-hydroxybenzyl)amino)phén yl)-7,8-difluorooctanamide mélanges racémiques de ceux-ci |
| 11 | 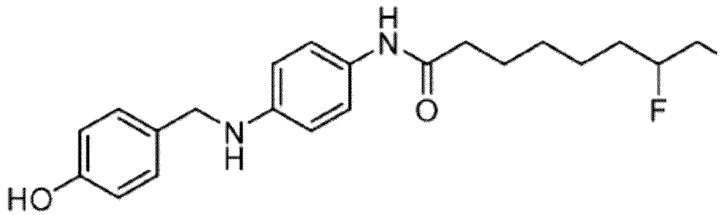 | 7,8-difluoro-N-(4-((4-hydroxybenzyl)amino)phén yl)-octanamide (7R)-7,8-difluoro-N-(4-((4-hydroxybenzyl)amino)phén yl)-octanamide (7S)-7,8-difluoro-N-(4-((4-hydroxybenzyl)amino)phén yl)-octanamide mélanges racémiques de ceux-ci |

(continued)

| | STRUCTURE DU COMPOSÉ | NOM DU COMPOSÉ |
|---|---|---|
| 11 | 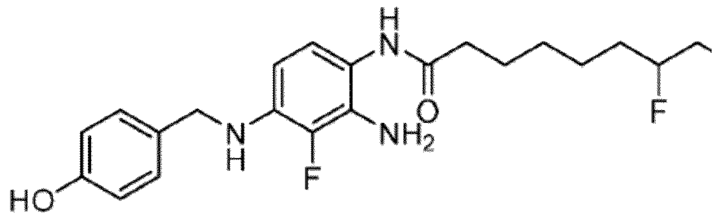 | N-(2-amino-3-fluoro-4-((4-hydroxybenzyl)amino)phén yl)-7,8-difluorooctanamide (7R)-N-(2-amino-3-fluoro-4-((4-hydroxybenzyl)amino)phén yl)-7,8-difluorooctanamide (7S)-N-(2-amino-3-fluoro-4-((4-hydroxybenzyl)amino)phén yl)-7,8-difluorooctanamide mélanges racémiques de ceux-ci |
| 11 | 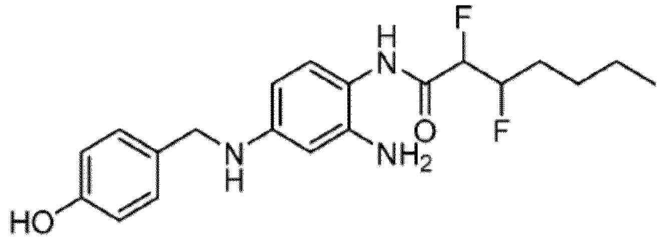 | N-(2-amino-4-((4-hydroxybenzyl)amino)phén yl)-2,3-difluoroheptanamide (2R,3S)-N-(2-amino-4-((4-hydroxybenzyl)amino)phén yl)-2,3-difluoroheptanamide (2S,3R)-N-(2-amino-4-((4-hydroxybenzyl)amino)phén yl)-2,3-difluoroheptanamide (2R,3R)-N-(2-amino-4-((4-hydroxybenzyl)amino)phén yl)-2,3-difluoroheptanamide (2S,3S)-N-(2-amino-4-((4-hydroxybenzyl)amino)phén yl)-2,3-difluoroheptanamide mélanges racémiques de ceux-ci |
| 11 | 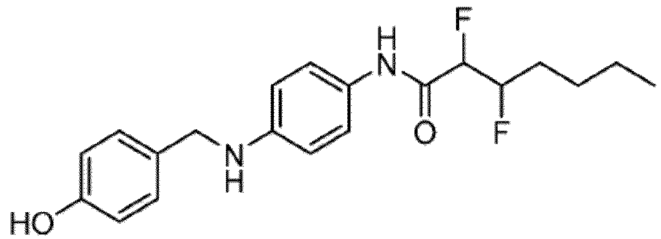 | 2,3-difluoro-N-(4-((4-hydroxybenzyl)amino)phén yl)heptanamide (2R,3S)-2,3-difluoro-N-(4-((4-hydroxybenzyl)amino)phén yl)heptanamide (2S,3R)-2,3-difluoro-N-(4-((4-hydroxybenzyl)amino)phén yl)heptanamide (2R,3R)-2,3-difluoro-N-(4-((4-hydroxybenzyl) amino)phén yl)heptanamide (2S,3S)-2,3-difluoro-N-(4-((4-hydroxybenzyl)amino)phén yl)heptanamide mélanges racémiques de ceux-ci |
| 12 | 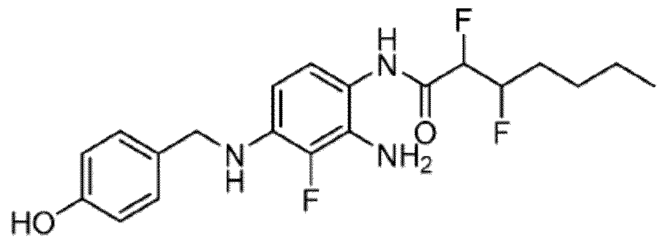 | N-(2-amino-3-fluoro-4-((4-hydroxybenzyl)amino)phén yl)-2,3-difluoroheptanamide (2R,3S)-N-(2-amino-3-fluoro-4-((4-hydroxybenzyl)amino)phén yl)-2,3-difluoroheptanamide (2S,3R)-N-(2-amino-3-fluoro-4-((4-hydroxybenzyl)amino)phén yl)-2,3-difluoroheptanamide (2R,3R)-N-(2-amino-3-fluoro-4-((4-hydroxybenzyl)amino)phén yl)-2,3-difluoroheptanamide (2S,3S)-N-(2-amino-3-fluoro-4-((4-hydroxybenzyl)amino)phén yl)-2,3-difluoroheptanamide mélanges racémiques de ceux-ci |

(continued)

| | STRUCTURE DU COMPOSÉ | NOM DU COMPOSÉ |
|---|---|---|
| 12 | 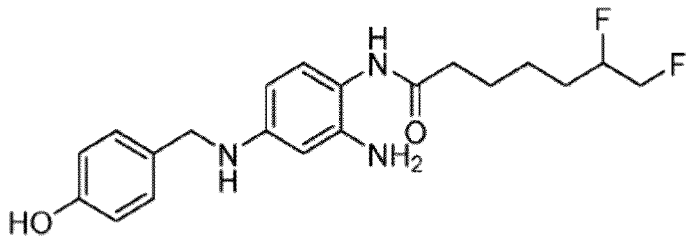 | N-(2-amino-4-((4-hydroxybenzyl)amino)phén yl)-6,7-difluoroheptanamide (6R)-N-(2-amino-4-((4-hydroxybenzyl)amino)phén yl)-6,7-difluoroheptanamide (6S)-N-(2-amino-4-((4-hydroxybenzyl)amino)phén yl)-6,7-difluoroheptanamide mélanges racémiques de ceux-ci |
| 12 | 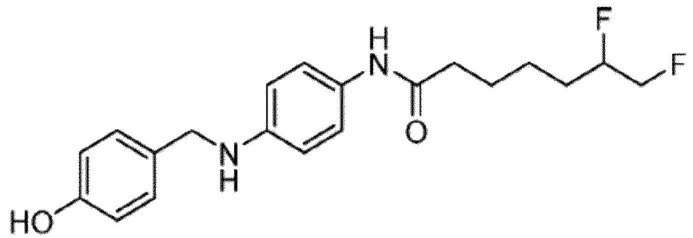 | 6,7-difluoro-N-(4-((4-hydroxybenzyl)amino)phén yl)-heptanamide (6R)-6,7-difluoro-N-(4-((4-hydroxybenzyl)amino)phén yl)-heptanamide (6S)-6,7-difluoro-N-(4-((4-hydroxybenzyl)amino)phén yl)-heptanamide mélanges racémiques de ceux-ci |
| 12 | 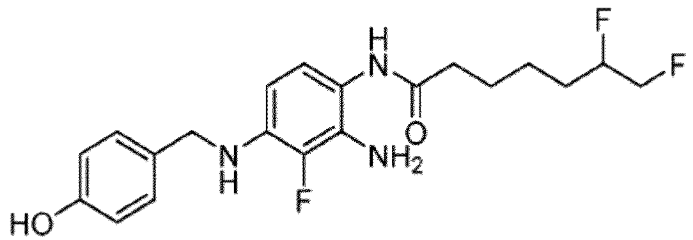 | N-(2-amino-3-fluoro-4-((4-hydroxybenzyl)amino)phén yl)-6,7-difluoroheptanamide (6R)-N-(2-amino-3-fluoro-4-((4-hydroxybenzyl)amino)phényl)-6,7-difluoroheptanamide (6S)-N-(2-amino-3-fluoro-4-((4-hydroxybenzyl)amino)phén yl)-6,7-difluoroheptanamide mélanges racémiques de ceux-ci |
| 12 | 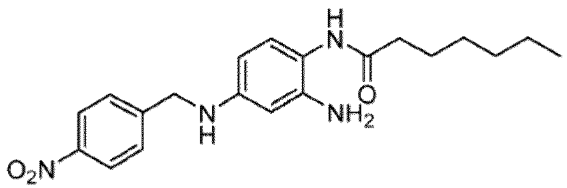 | N-(2-amino-4-((4-nitrobenzyl)amino)phényl )heptanamide |

(continued)

| | STRUCTURE DU COMPOSÉ | NOM DU COMPOSÉ |
|---|---|---|
| 12 | 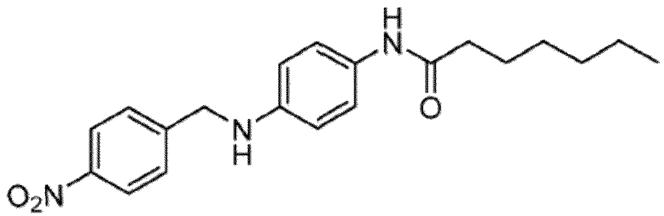 | N-(4-((4-nitrobenzyl)amino)phényl )heptanamide |
| 12 | 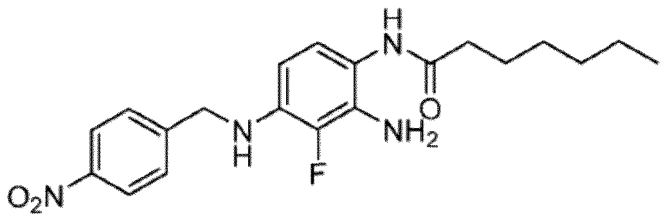 | N-(2-amino-3-fluoro-4-((4-nitrobenzyl)amino)phényl )heptanamide |
| 12 | 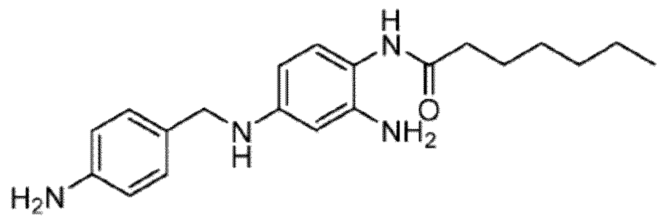 | N-(2-amino-4-((4-aminobenzyl)amino)phényl )heptanamide |
| 12 | 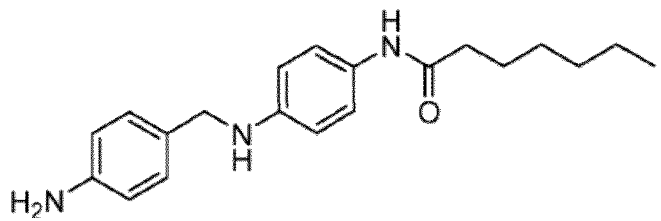 | N-(4-((4-aminobenzyl)amino)phényl )heptanamide |

(continued)

| | STRUCTURE DU COMPOSÉ | NOM DU COMPOSÉ |
|---|---|---|
| 12 | 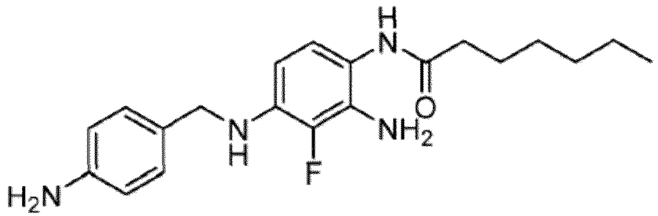 | N-(2-amino-3-fluoro-4-((4-aminobenzyl)amino)phényl )heptanamide |
| 13 | 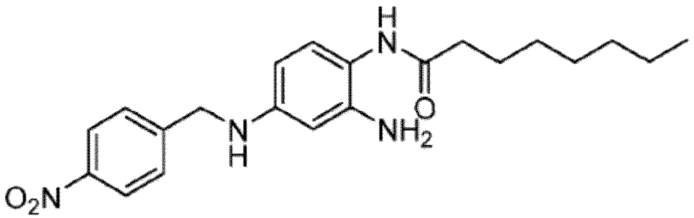 | N-(2-amino-4-((4-nitrobenzyl)amino)phényl )octanamide |
| 13 | 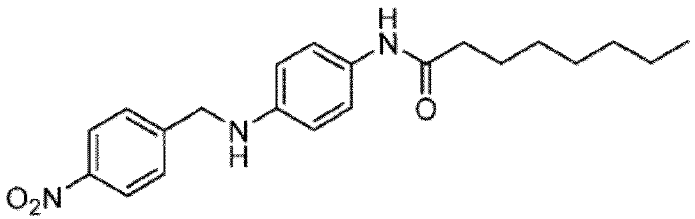 | N-(4-((4-nitrobenzyl)amino)phényl )octanamide |
| 13 | 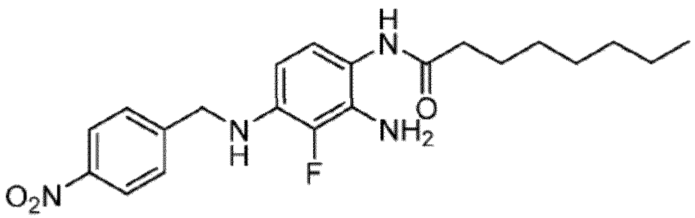 | N-(2-amino-3-fluoro-4-((4-nitrobenzyl)amino)phényl )octanamide |

(continued)

| | STRUCTURE DU COMPOSÉ | NOM DU COMPOSÉ |
|---|---|---|
| 13 | 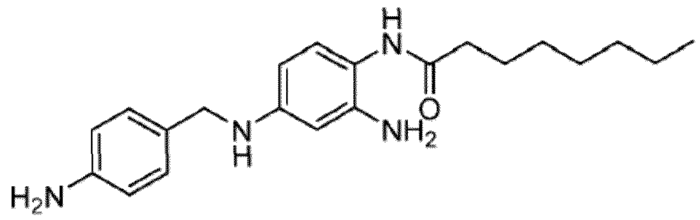 | N-(2-amino-4-((4-aminobenzyl)amino)phényl )octanamide |
| 13 | 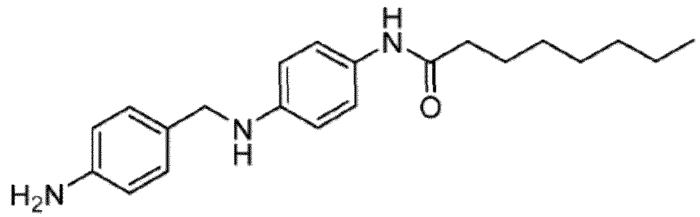 | N-(4-((4-aminobenzyl)amino)phényl )octanamide |
| 13 | 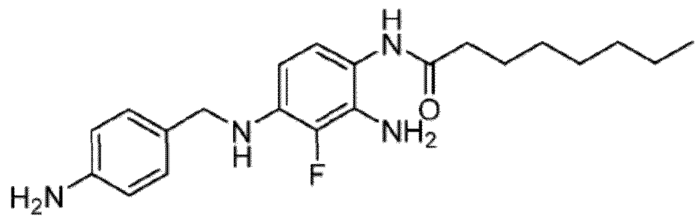 | N-(2-amino-3-fluoro-4-((4-aminobenzyl)amino)phényl )octanamide |
| 13 | 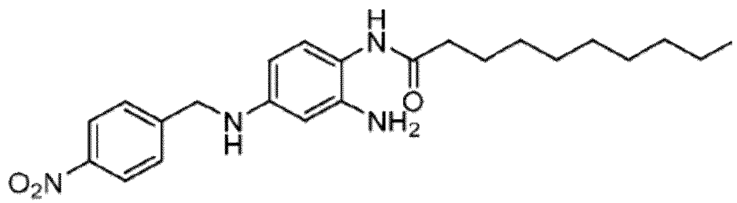 | N-(2-amino-4-((4-nitrobenzyl)amino)phényl )décanamide |
| 13 | 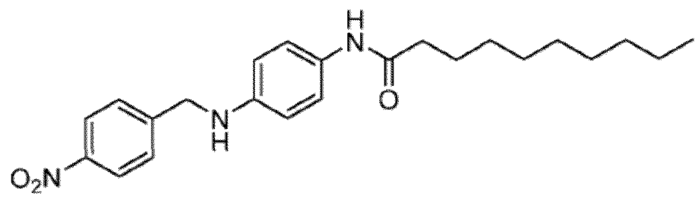 | N-(4-((4-nitrobenzyl)amino)phényl )décanamide |

(continued)

| | STRUCTURE DU COMPOSÉ | NOM DU COMPOSÉ |
|---|---|---|
| 13 | 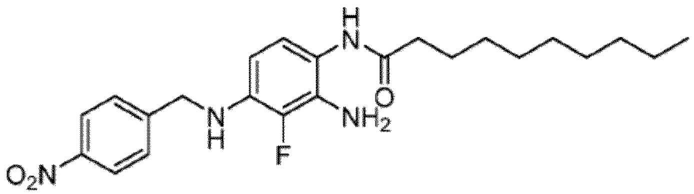 | N-(2-amino-3-fluoro-4-((4-nitrobenzyl)amino)phényl )décanamide |
| 13 | 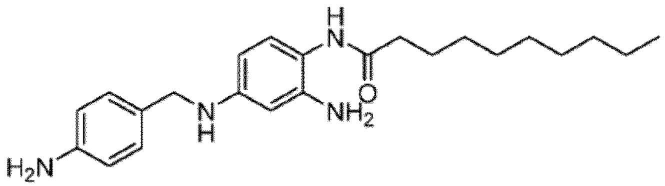 | N-(2-amino-4-((4-aminobenzyl)amino)phényl )décanamide |
| 14 | 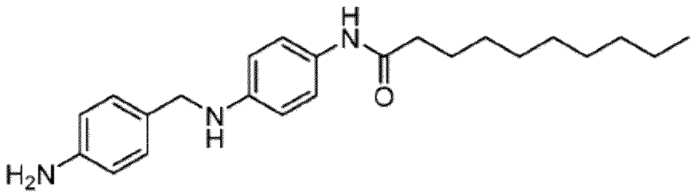 | N-(4-((4-aminobenzyl)amino)phényl )décanamide |
| 14 | 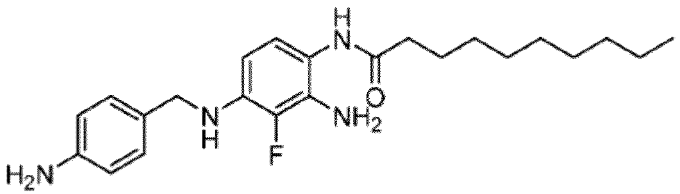 | N-(2-amino-3-fluoro-4-((4-aminobenzyl)amino)phényl )décanamide |
| 14 | 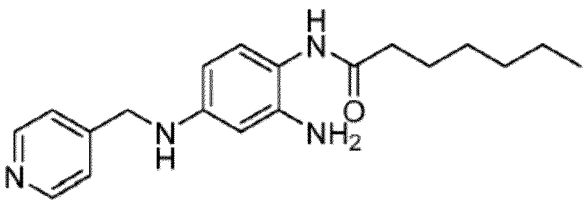 | N-(2-amino-4-((pyridin-4-ylméthyl)amino)phényl)he ptanamide |

(continued)

| | STRUCTURE DU COMPOSÉ | NOM DU COMPOSÉ |
|---|---|---|
| 14 | 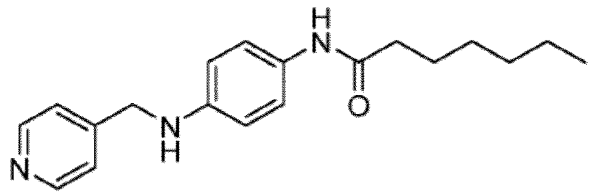 | N-(4-((pyridin-4-ylméthyl)amino)phényl)he ptanamide |
| 14 | 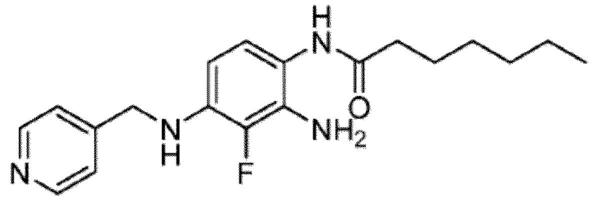 | N-(2-amino-3-fluoro-4-((pyridin-4-ylméthyl)amino)phényl)he ptanamide |
| 14 | 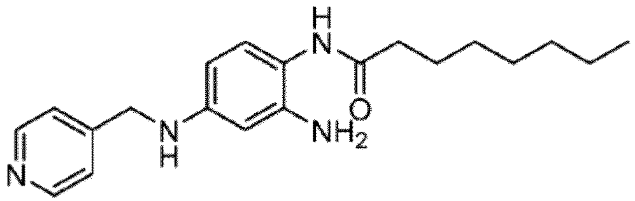 | N-(2-amino-4-((pyridin-4-ylméthyl)amino)phényl)oc tanamide |
| 14 | 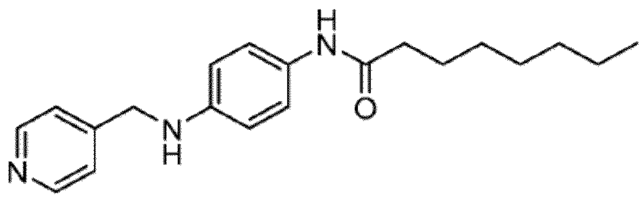 | N-(4-((pyridin-4-ylméthyl)amino)phényl)oc tanamide |
| 14 | 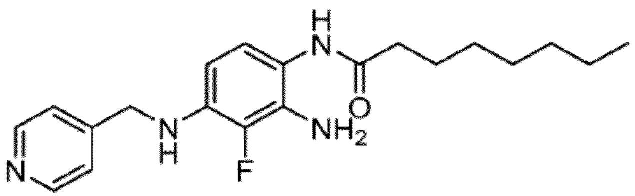 | N-(2-amino-3-fluoro-4-((pyridin-4-ylméthyl)amino)phényl)oc tanamide |

(continued)

| | STRUCTURE DU COMPOSÉ | NOM DU COMPOSÉ |
|---|---|---|
| 14 | 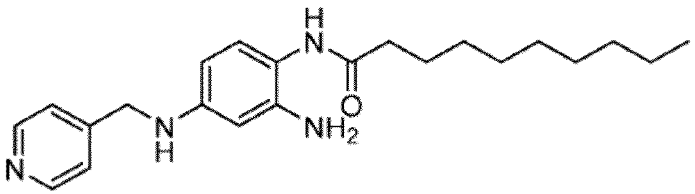 | N-(2-amino-4-((pyridin-4-ylméthyl)amino)phényl)dé canamide |
| 14 | 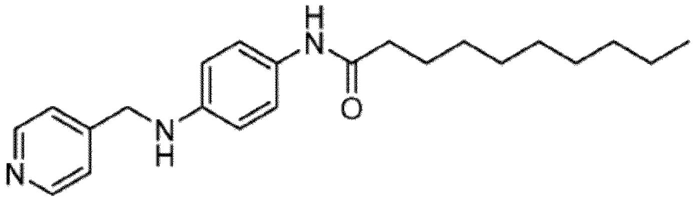 | N-(4-((pyridin-4-ylméthyl)amino)phényl)dé canamide |
| 15 | 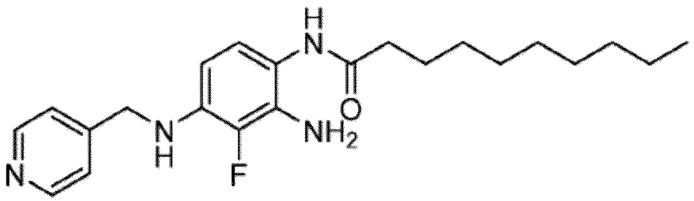 | N-(2-amino-3-fluoro-4-((pyridin-4-ylméthyl)amino)phényl)dé canamide |
| 15 | 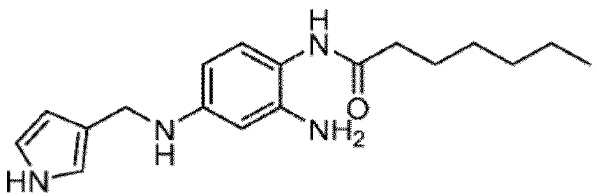 | N-(4-(((1H-pyrrol-3-yl)méthyl)amino)-2-aminophényl)heptanamide |
| 15 | 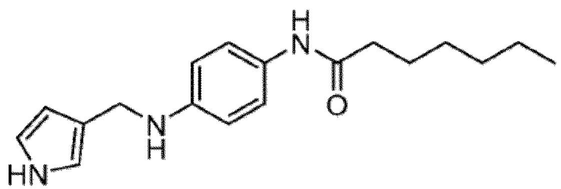 | N-(4-(((1H-pyrrol-3-yl)méthyl)amino)phényl)h eptanamide |

(continued)

| | STRUCTURE DU COMPOSÉ | NOM DU COMPOSÉ |
|---|---|---|
| 15 | 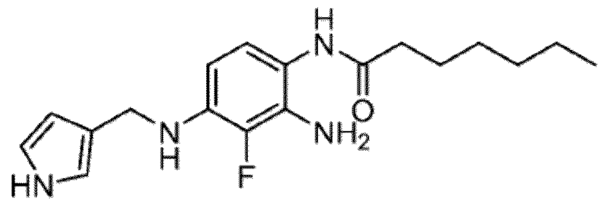 | N-(4-(((1H-pyrrol-3-yl)méthyl)amino)-2-amino-3-fluorophényl)heptanamide |
| 15 | 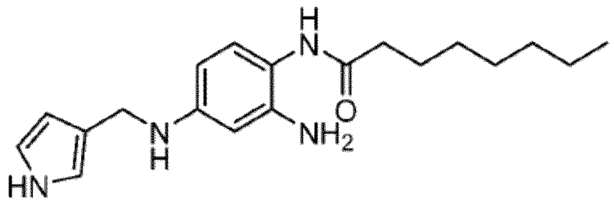 | N-(4-(((1H-pyrrol-3-yl)méthyl)amino)-2-aminophényl)octanamide |
| 15 | 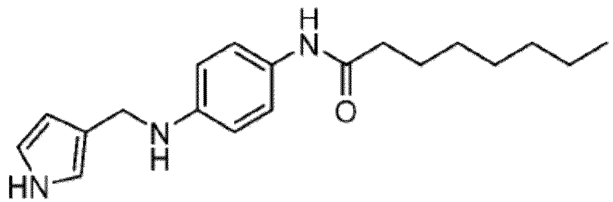 | N-(4-(((1H-pyrrol-3-yl)méthyl)amino)phényl)o ctanamide |
| 15 | 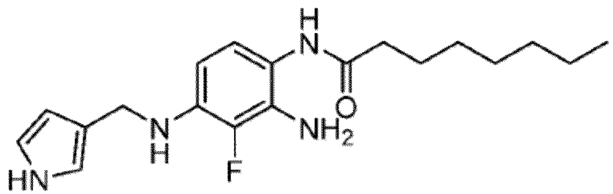 | N-(4-(((1H-pyrrol-3-yl)méthyl)amino)-2-amino-3-fluorophényl)octanamide |
| 15 | 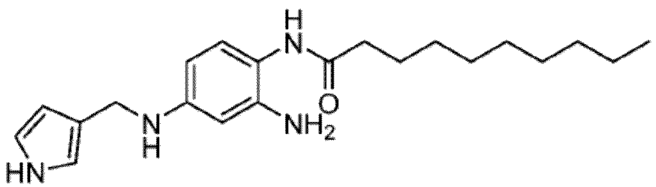 | N-(4-(((1H-pyrrol-3-yl)méthyl)amino)-2-aminophényl)décanamide |

(continued)

| | STRUCTURE DU COMPOSÉ | NOM DU COMPOSÉ |
|---|---|---|
| 15 | 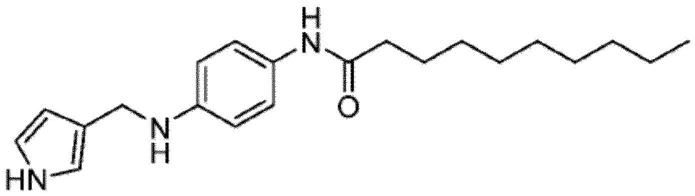 | N-(4-(((1H-pyrrol-3-yl)méthyl)amino)phényl)d écanamide |
| 15 | 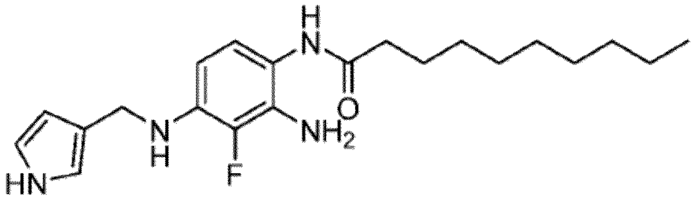 | N-(4-(((1H-pyrrol-3-yl)méthyl)amino)-2-amino-3-fluorophényl)décanamide |
| 16 | 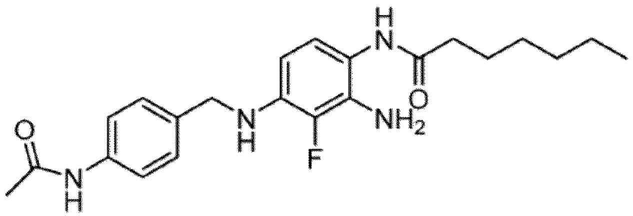 | N-(4-((4-acétamidobenzyl)amino)-2-amino-3-fluorophényl)heptanamide |
| 16 | 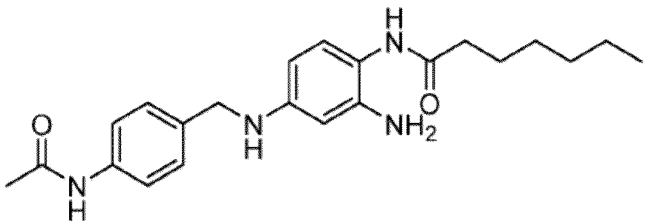 | N-(4-((4-acétamidobenzyl)amino)-2-aminophényl)heptanamide |

(continued)

| | STRUCTURE DU COMPOSÉ | NOM DU COMPOSÉ |
|---|---|---|
| 16 | 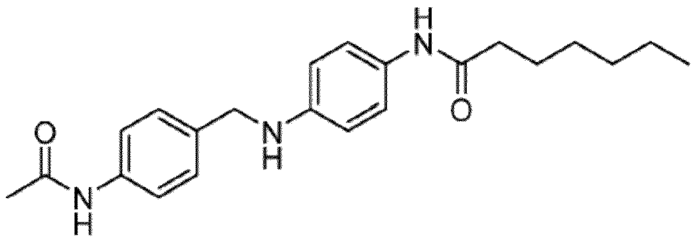 | N-(4-((4-acétamidobenzyl)amino)ph ényl)heptanamide |
| 16 | 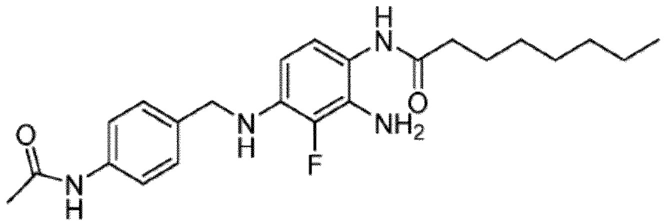 | N-(4-((4-acétamidobenzyl)amino)-2-amino-3-fluorophényl)octanamide |
| 16 | 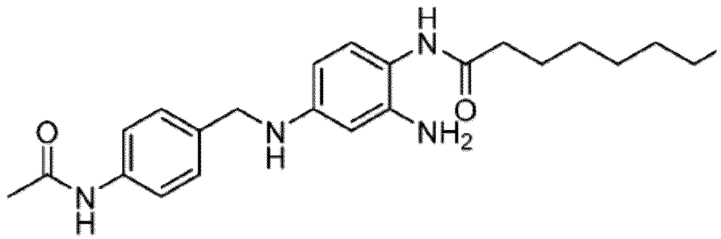 | N-(4-((4-acétamidobenzyl)amino)-2-aminophényl)octanamide |
| 16 | 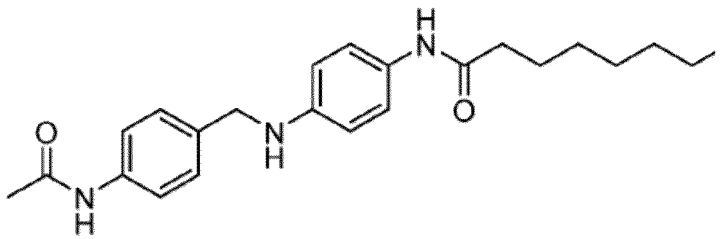 | N-(4-((4-acétamidobenzyl)amino)ph ényl)octanamide |

(continued)

| | STRUCTURE DU COMPOSÉ | NOM DU COMPOSÉ |
|---|---|---|
| 16 | 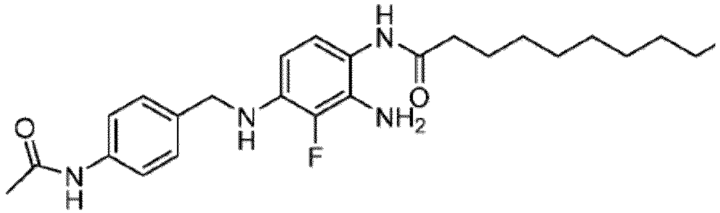 | N-(4-((4-acétamidobenzyl)amino)-2-amino-3-fluorophényl)décanamide |
| 16 | 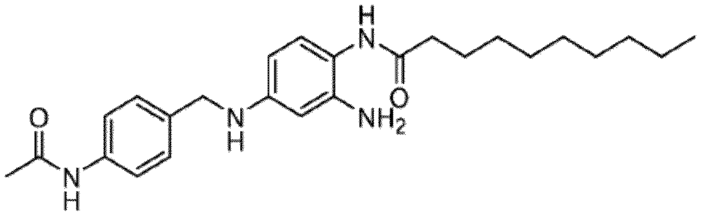 | N-(4-((4-acétamidobenzyl)amino)-2-aminophényl)décanamide |
| 16 | 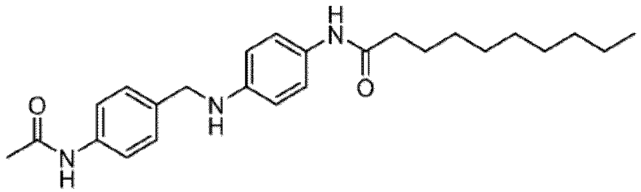 | N-(4-((4-acétamidobenzyl)amino)ph ényl)décanamide |
| 16 | 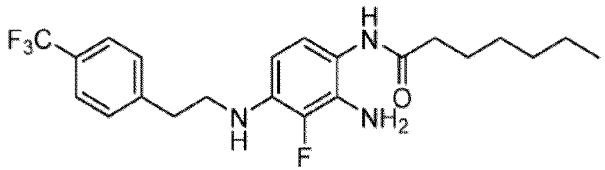 | N-(2-amino-3-fluoro-4-((4-(trifluorométhyl)phénéthyl)amino)phényl)heptanam ide |
| 17 | 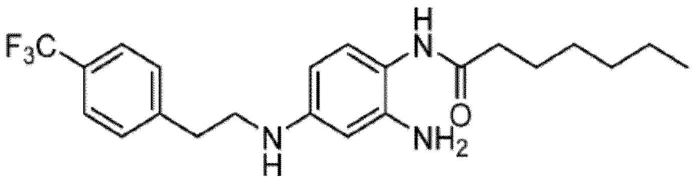 | N-(2-amino-4-((4-(trifluorométhyl)phénéth yl)amino)phényl)heptanam ide |

(continued)

| | STRUCTURE DU COMPOSÉ | NOM DU COMPOSÉ |
|---|---|---|
| 17 | 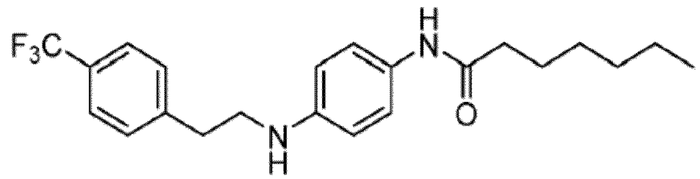 | N-(4-((4-(trifluorométhyl)phénéth yl)amino)phényl)heptanam ide |
| 17 | 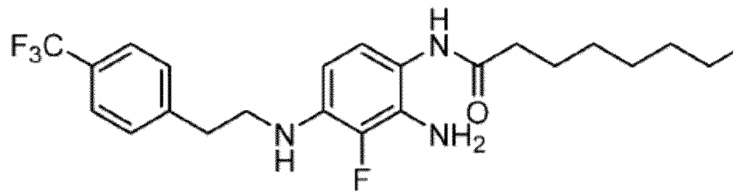 | N-(2-amino-3-fluoro-4-((4-(trifluorométhyl)phénéth yl)amino)phényl)octanami de |
| 17 | 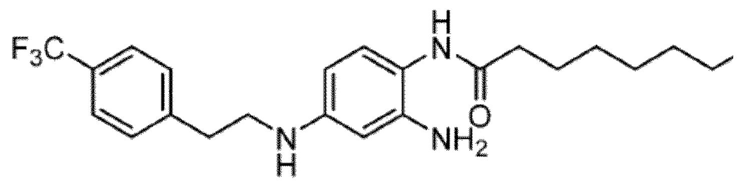 | N-(2-amino-4-((4-(trifluorométhyl)phénéth yl)amino)phényl)octanami de |
| 17 | 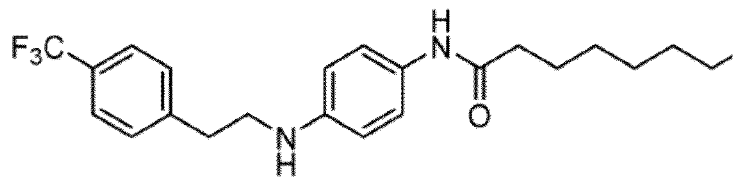 | N-(4-((4-(trifluorométhyl)phénéth yl)amino)phényl)octanami de |
| 17 | 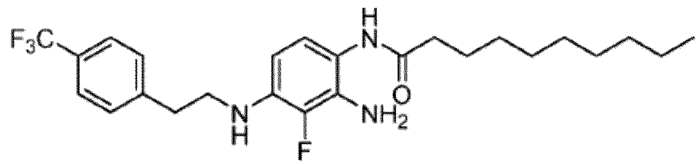 | N-(2-amino-3-fluoro-4-((4-(trifluorométhyl)phénéth yl)amino)phényl)décanami de |

(continued)

| | STRUCTURE DU COMPOSÉ | NOM DU COMPOSÉ |
|---|---|---|
| 17 | 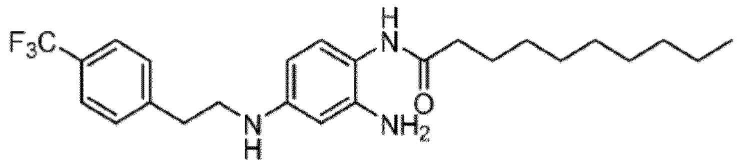 | N-(2-amino-4-((4-(trifluorométhyl)phénéth yl)amino)phényl)décanami de |
| 17 | 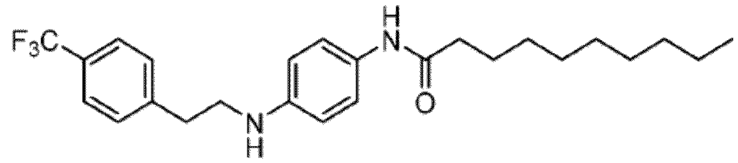 | N-(4-((4-(trifluorométhyl)phénéth yl)amino)phényl)décanami de |
| 17 | 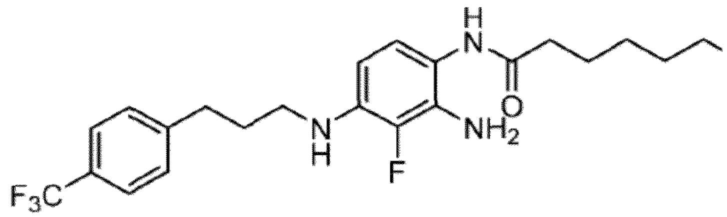 | N-(2-amino-3-fluoro-4-((3-(4-(trifluorométhyl)phényl) propyl)amino)phényl)hept anamide |
| 17 | 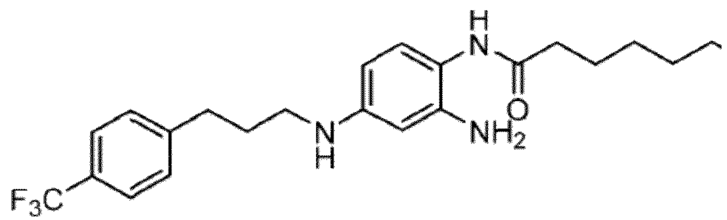 | N-(2-amino-4-((3-(4-(trifluorométhyl)phényl) propyl)amino)phényl)hept anamide |
| 18 | 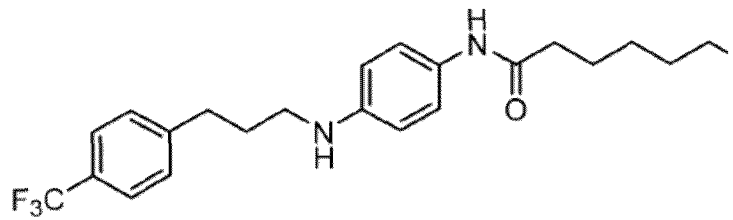 | N- (4-((3-(4-(trifluorométhyl)phényl) propyl)amino)phényl)hept anamide |

(continued)

| | STRUCTURE DU COMPOSÉ | NOM DU COMPOSÉ |
|---|---|---|
| 18 | 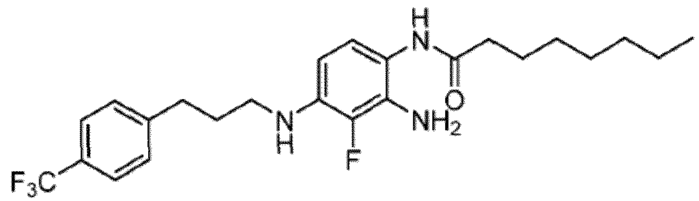 | N-(2-amino-3-fluoro-4-((3-(4-(trifluorométhyl)phényl) propyl)amino)phényl)octa namide |
| 18 | 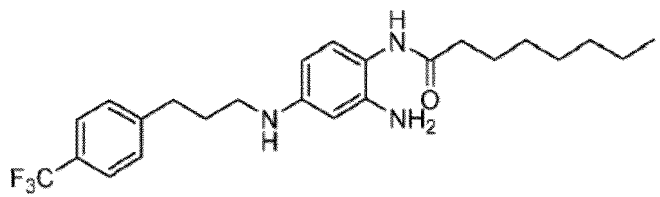 | N-(2-amino-4-((3-(4-(trifluorométhyl)phényl) propyl)amino)phényl)octa namide |
| 18 | 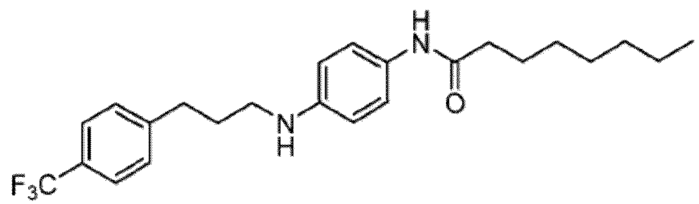 | N-(4-((3-(4-(trifluorométhyl)phényl) propyl)amino)phényl)octa namide |
| 18 | 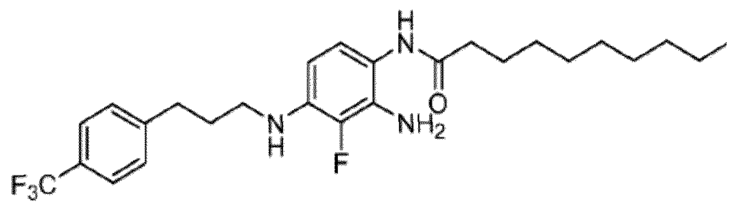 | N-(2-amino-3-fluoro-4-((3-(4-(trifluorométhyl)phényl) propyl)amino)phényl)déca namide |
| 18 | 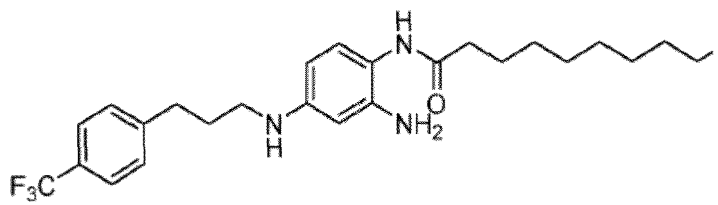 | N-(2-amino-4-((3-(4-(trifluorométhyl)phényl) propyl)amino)phényl)déca namide |

(continued)

| | STRUCTURE DU COMPOSÉ | NOM DU COMPOSÉ |
|---|---|---|
| 18 | 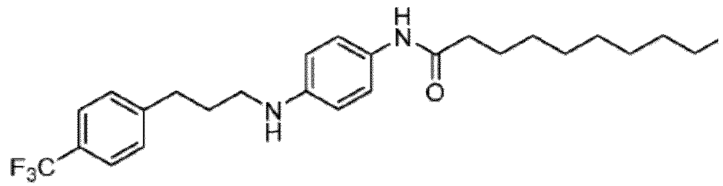 | N- (4- ((3- (4-(trifluorométhyl)phényl) propyl)amino)phényl)déca namide |
| 18 | 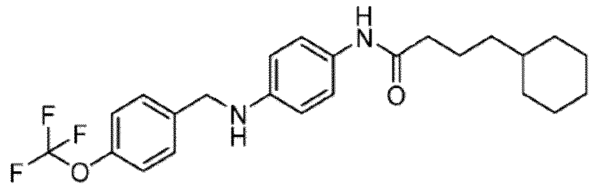 | 4-cyclohexyl-N-(4-((4-(trifluorométhoxy)benzyl )amino)phényl)butanamide |
| 18 | 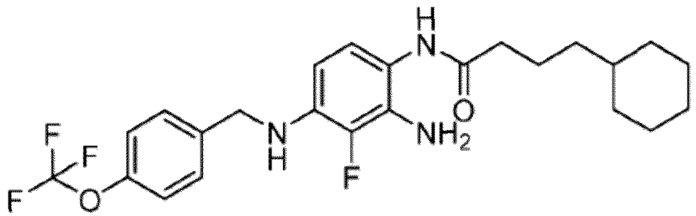 | N-(2-amino-3-fluoro-4-((4-(trifluorométhoxy)benzyl )amino)phényl)-4-cyclohexylbutanamide |
| 18 | 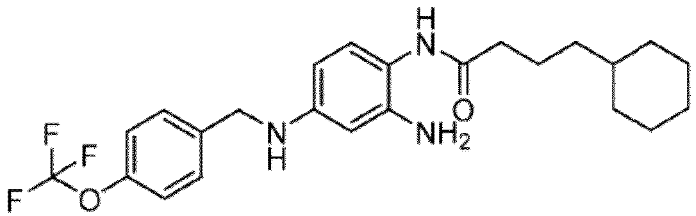 | N-(2-amino-4-((4-(trifluorométhoxy)benzyl )amino)phényl)-4-cyclohexylbutanamide |
| 19 | 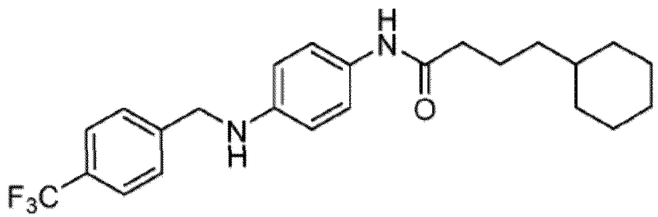 | 4-cyclohexyl-N-(4-((4-(trifluorométhyl)benzyl) amino)phényl)butanamide |

(continued)

| | STRUCTURE DU COMPOSÉ | NOM DU COMPOSÉ |
|---|---|---|
| 19 | 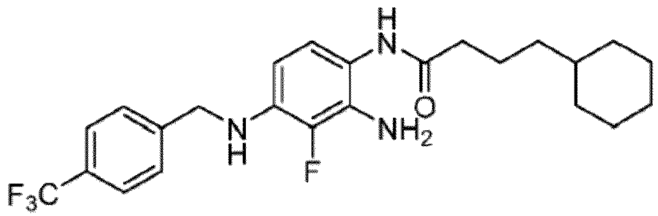 | N-(2-amino-3-fluoro-4-((4-(trifluorométhyl)benzyl) amino)phényl)-4-cyclohexylbutanamide |
| 19 | 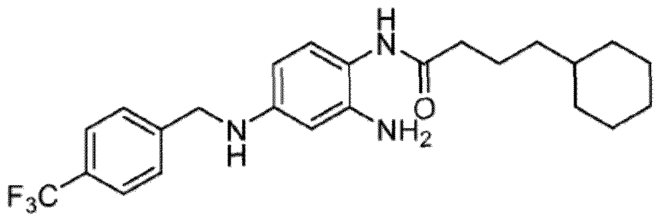 | N-(2-amino-4-((4-(trifluorométhyl)benzyl) amino)phényl)-4-cyclohexylbutanamide |
| 19 | 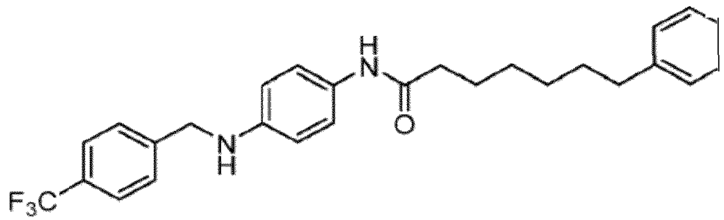 | 7-phényl-N-(4-((4-(trifluorométhyl)benzyl) amino)phényl)heptanamide |
| 19 | 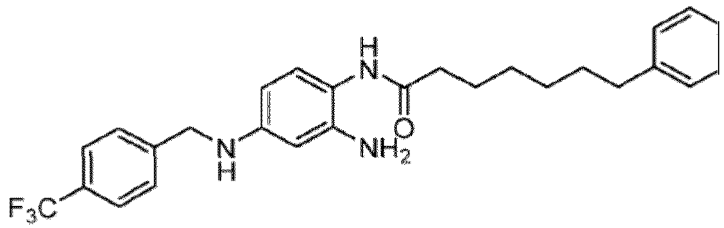 | N-(2-amino-4-((4-(trifluorométhyl)benzyl) amino)phényl)-7-phénylheptanamide |

(continued)

| | STRUCTURE DU COMPOSÉ | NOM DU COMPOSÉ |
|---|---|---|
| 19 | 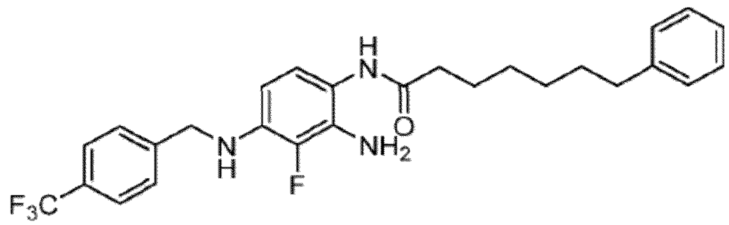 | N-(2-amino-3-fluoro-4-((4-(trifluorométhyl)benzyl) amino)phényl)-7-phénylheptanamide |
| 19 | 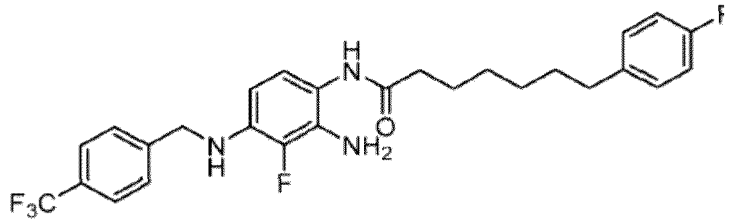 | N-(2-amino-3-fluoro-4-((4-(trifluorométhyl)benzyl) amino)phényl)-7-(4-fluorophényl)heptanamide |
| 19 | 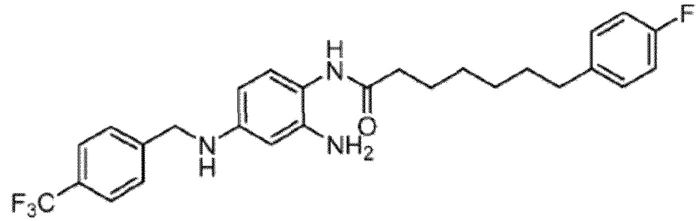 | N-(2-amino-4-((4-(trifluorométhyl)benzyl) amino)phényl)-7-(4-fluorophényl)heptanamide |
| 19 | 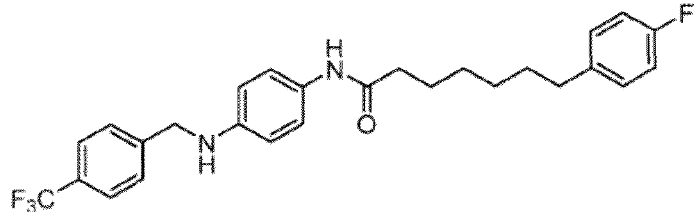 | 7-(4-fluorophényl)-N-(4-((4-(trifluorométhyl)benzyl) amino)phényl)heptanamide |

(continued)

| | STRUCTURE DU COMPOSÉ | NOM DU COMPOSÉ |
|---|---|---|
| 19 | 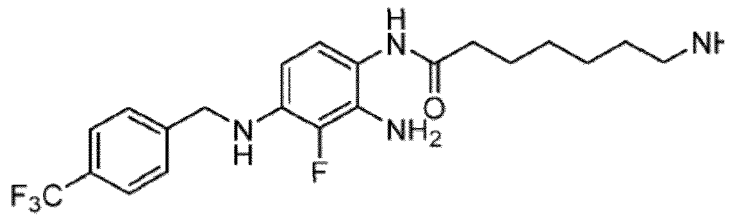 | 7-amino-N-(2-amino-3-fluoro-4-((4-(trifluorométhyl)benzyl) amino)phényl)heptanamide |
| 20 | 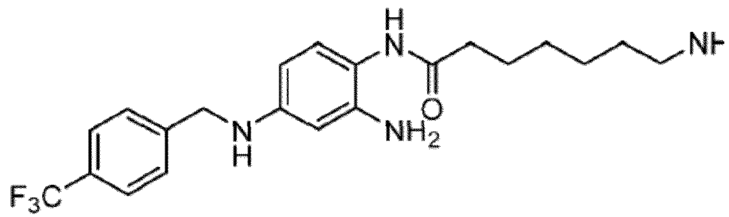 | 7-amino-N-(2-amino-4-((4-(trifluorométhyl)benzyl) amino)phényl)heptanamide |
| 20 | 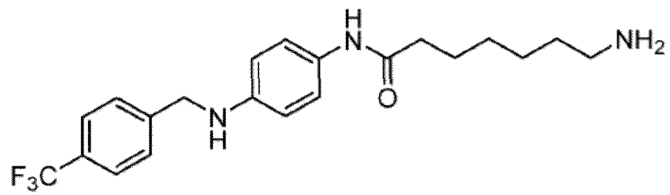 | 7-amino-N-(4-((4-(trifluorométhyl)benzyl) amino)phényl)heptanamide |
| 20 | 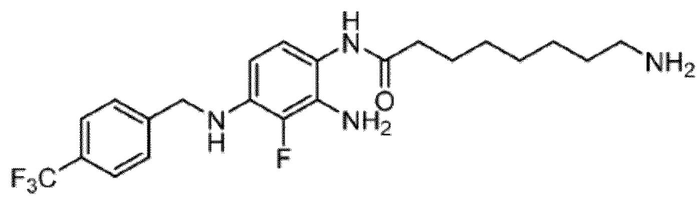 | 8-amino-N-(2-amino-3-fluoro-4-((4-(trifluorométhyl)benzyl) amino)phényl)octanamide |
| 20 | 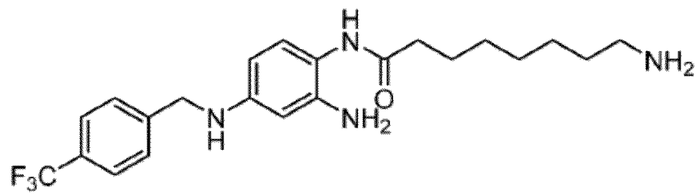 | 8-amino-N-(2-amino-4-((4-(trifluorométhyl)benzyl) amino)phényl)octanamide |

(continued)

| | STRUCTURE DU COMPOSÉ | NOM DU COMPOSÉ |
|---|---|---|
| 20 | 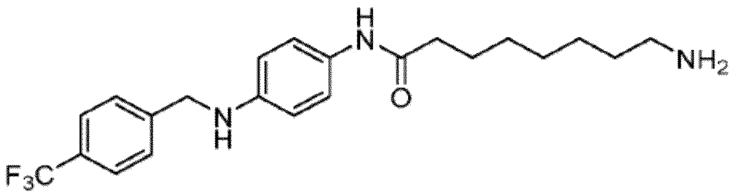 | 8-amino-N-(4-((4-(trifluorométhyl)benzyl) amino)phényl)octanamide |
| 20 | 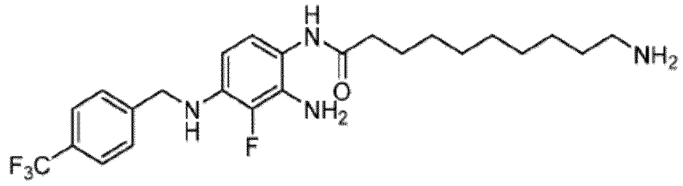 | 10-amino-N-(2-amino-3-fluoro-4-((4-(trifluorométhyl)benzyl) amino)phényl)décanamide |
| 20 | 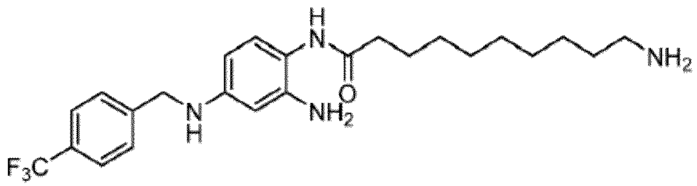 | 10-amino-N-(2-amino-4-((4-(trifluorométhyl)benzyl) amino)phényl)décanamide |
| 20 | 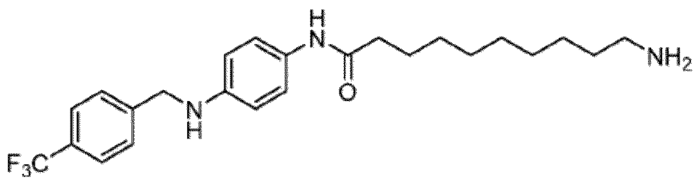 | 10-amino-N-(4-((4-(trifluorométhyl)benzyl) amino)phényl)décanamide |
| 20 | 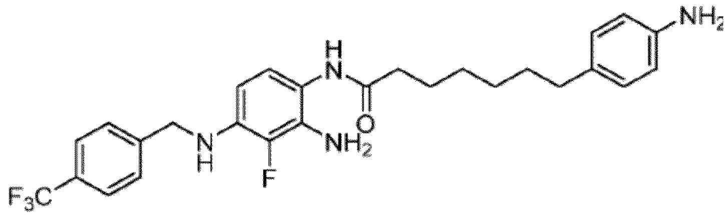 | N-(2-amino-3-fluoro-4-((4-(trifluorométhyl)benzyl) amino)phényl)-7-(4-aminophényl)heptanamide |

(continued)

| | STRUCTURE DU COMPOSÉ | NOM DU COMPOSÉ |
|---|---|---|
| 20 | 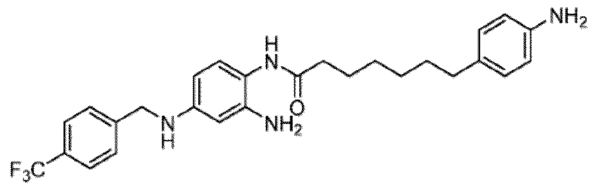 | N-(2-amino-4-((4-(trifluorométhyl)benzyl) amino)phényl)-7-(4-aminophényl)heptanamide |
| 21 | 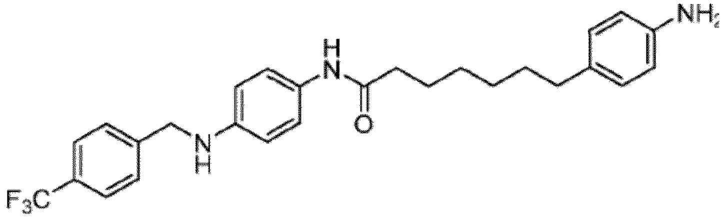 | 7-(4-aminophényl)-N-(4-((4-(trifluorométhyl)benzyl) amino)phényl)heptanamide |
| 21 | 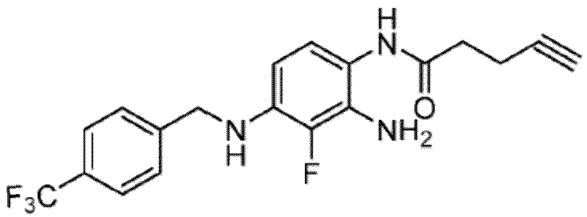 | N-(2-amino-3-fluoro-4-((4-(trifluorométhyl)benzyl) amino)phényl)pent-4-ynamide |
| 21 | 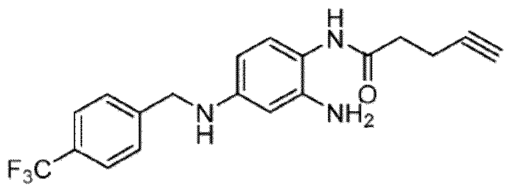 | N-(2-amino-4-((4-(trifluorométhyl)benzyl) amino)phényl)pent-4-ynamide |
| 21 | 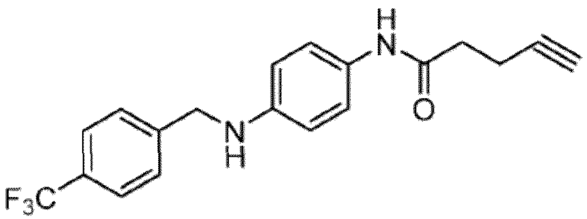 | N-(4-((4-(trifluorométhyl)benzyl) amino)phényl)pent-4-ynamide |

(continued)

| | STRUCTURE DU COMPOSÉ | NOM DU COMPOSÉ |
|---|---|---|
| 21 | 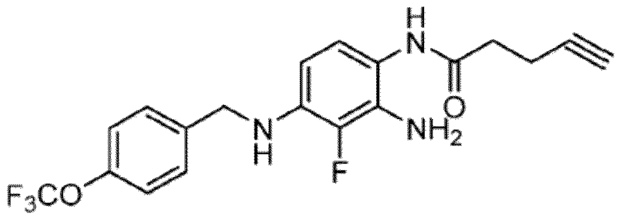 | N-(2-amino-3-fluoro-4-((4-(trifluorométhoxy)benzyl )amino)phényl)pent-4-ynamide |
| 21 | 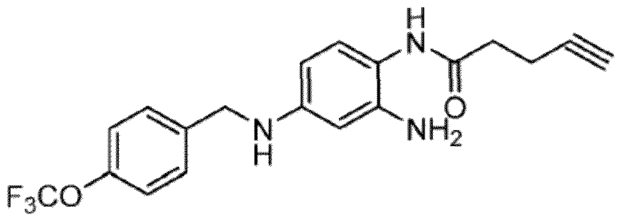 | N-(2-amino-4-((4-(trifluorométhoxy)benzyl )amino)phényl)pent-4-ynamide |
| 21 | 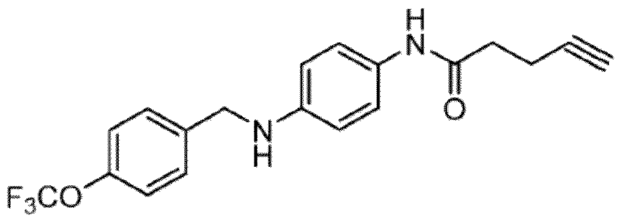 | N-(4-((4-(trifluorométhoxy)benzyl )amino)phényl)pent-4-ynamide |
| 21 | 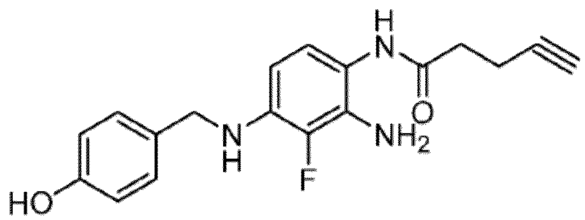 | N-(2-amino-3-fluoro-4-((4-hydroxybenzyl)amino)phén yl)pent-4-ynamide |

(continued)

| | STRUCTURE DU COMPOSÉ | NOM DU COMPOSÉ |
|---|---|---|
| 21 | 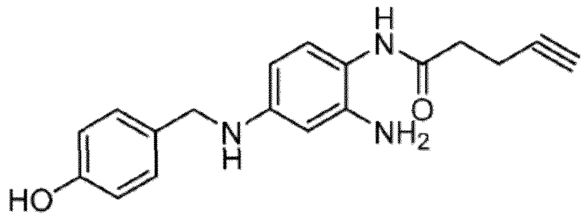 | N-(2-amino-4-((4-hydroxybenzyl)amino)phén yl)pent-4-ynamide |
| 21 | 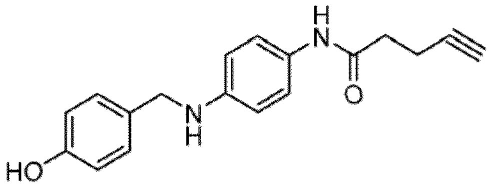 | N-(4-((4-hydroxybenzyl)amino)phén yl)pent-4-ynamide |
| 22 | 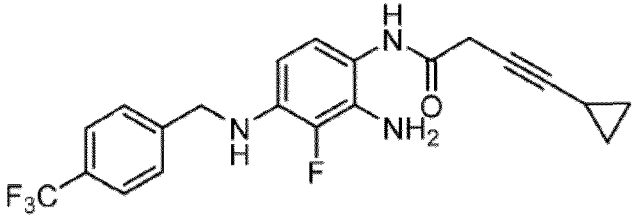 | N-(2-amino-3-fluoro-4-((4-(trifluorométhyl)benzyl) amino)phényl)-4-cyclopropylbut-3-ynamide |
| 22 | 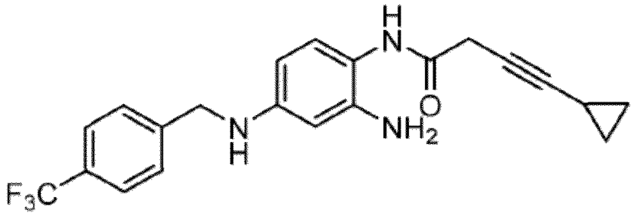 | N-(2-amino-4-((4-(trifluorométhyl)benzyl) amino)phényl)-4-cyclopropylbut-3-ynamide |

(continued)

| | STRUCTURE DU COMPOSÉ | NOM DU COMPOSÉ |
|---|---|---|
| 22 | 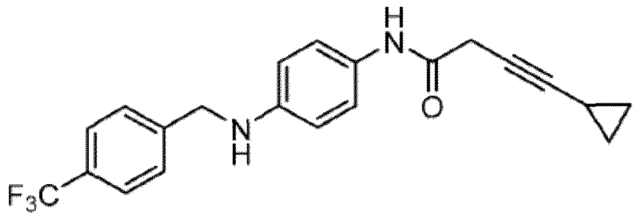 | 4-cyclopropyl-N-(4-((4-(trifluorométhyl)benzyl) amino)phényl)but-3-ynamide |
| 22 | 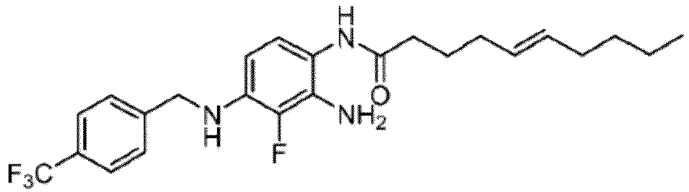 | (E)-N-(2-amino-3-fluoro-4-((4-(trifluorométhyl)benzyl) amino)phényl)déc-5-énamide |
| 22 | 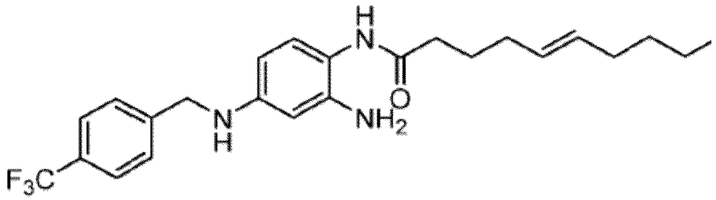 | (E)-N-(2-amino-4-((4-(trifluorométhyl)benzyl) amino)phényl)déc-5-énamide |
| 22 | 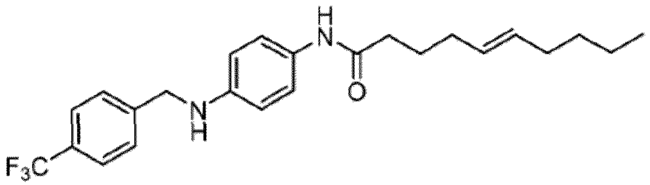 | (E)-N- (4- ((4-(trifluorométhyl)benzyl) amino)phényl)déc-5-énamide |

(continued)

| | STRUCTURE DU COMPOSÉ | NOM DU COMPOSÉ |
|---|---|---|
| 22 | 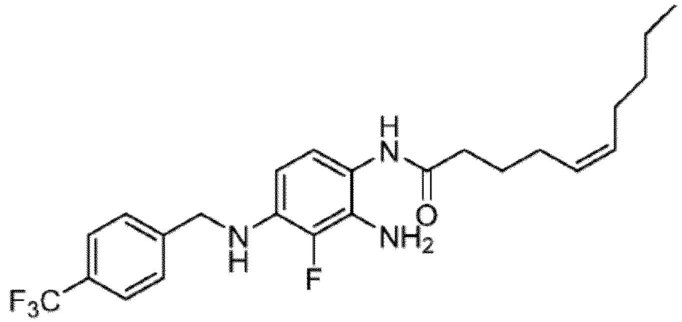 | (Z)-N-(2-amino-3-fluoro-4-((4-(trifluorométhyl)benzyl) amino)phényl)déc-5-énamide |
| 22 | 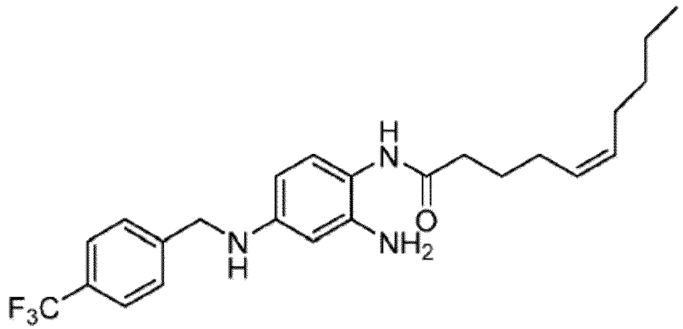 | (Z)-N-(2-amino-4-((4-(trifluorométhyl)benzyl) amino)phényl)déc-5-énamide |
| 22 | 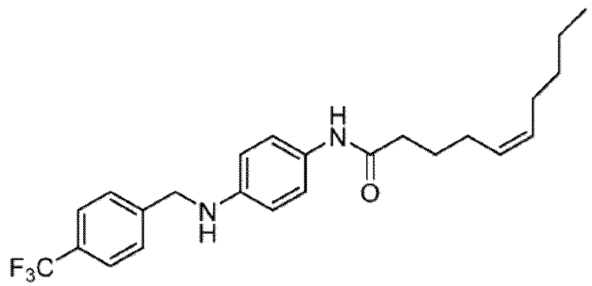 | (Z)-N-(4-((4-(trifluorométhyl)benzyl) amino)phényl)déc-5-énamide |

(continued)

| | STRUCTURE DU COMPOSÉ | NOM DU COMPOSÉ |
|---|---|---|
| 22 | 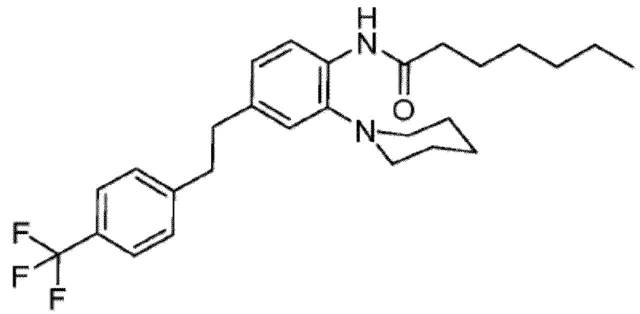 | N-(2-(pipéridin-1-yl)-4-(4-(trifluorométhyl)phénéth yl)phényl)heptanamide |
| 23 | 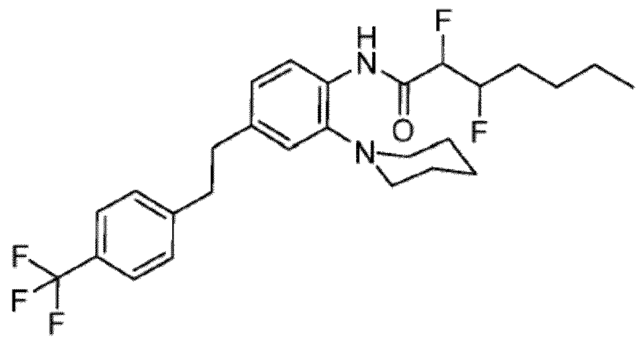 | 2,3-difluoro-N-(2-(pipéridin-1-yl)-4-(4-(trifluorométhyl)phénéth yl)phényl)heptanamide (2R,3S)-2,3-difluoro-N-(2-(pipéridin-1-yl)-4-(4-(trifluorométhyl)phénéth yl)phényl)heptanamide (2S,3R)-2,3-difluoro-N-(2-(pipéridin-1-yl)-4-(4-(trifluorométhyl)phénéth yl)phényl)heptanamide (2R,3R)-2,3-difluoro-N-(2-(pipéridin-1-yl)-4-(4-(trifluorométhyl)phénéth yl)phényl)heptanamide (2S,3S)-2,3-difluoro-N-(2-(pipéridin-1-yl)-4-(4-(trifluorométhyl) phénéth yl)phényl)heptanamide |
| 23 | 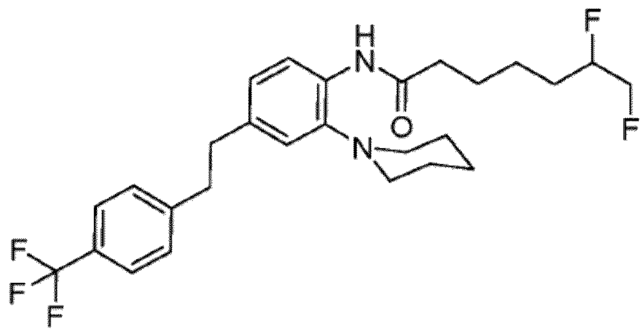 | 6,7-difluoro-N-(2-(pipéridin-1-yl)-4-(4-(trifluorométhyl)phénéth yl)phényl)heptanamide (6R)-6,7-difluoro-N-(2-(pipéridin-1-yl)-4-(4-(trifluorométhyl)phénéth yl)phényl)heptanamide (6S)-6,7-difluoro-N-(2-(pipéridin-1-yl)-4-(4-(trifluorométhyl)phénéth yl)phényl)heptanamide |

(continued)

| | STRUCTURE DU COMPOSÉ | NOM DU COMPOSÉ |
|---|---|---|
| 23 | 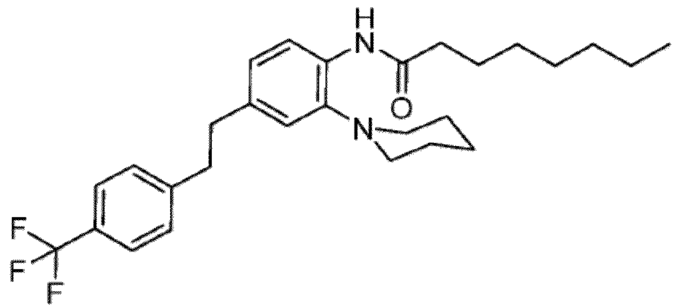 | N-(2-(pipéridin-1-yl)-4-(4-(trifluorométhyl)phénéth yl)phényl)octanamide |
| 23 | 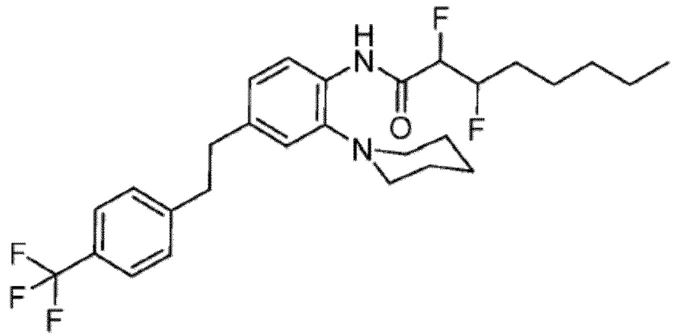 | 2,3-difluoro-N-(2-(pipéridin-1-yl)-4-(4-(trifluorométhyl)phénéth yl)phényl)octanamide (2R,3S)-2,3-difluoro-N-(2-(pipéridin-1-yl)-4-(4-(trifluorométhyl)phénéth yl)phényl)octanamide (2S,3R)-2,3-difluoro-N-(2-(pipéridin-1-yl)-4-(4-(trifluorométhyl)phénéth yl)phényl) octanamide (2R,3R)-2,3-difluoro-N-(2-(pipéridin-1-yl)-4-(4-(trifluorométhyl)phénéth yl) phényl)octanamide (2S,3S)-2,3-difluoro-N-(2-(pipéridin-1-yl)-4-(4-(trifluorométhyl)phénéth yl)phényl)octanamide |
| 23 | 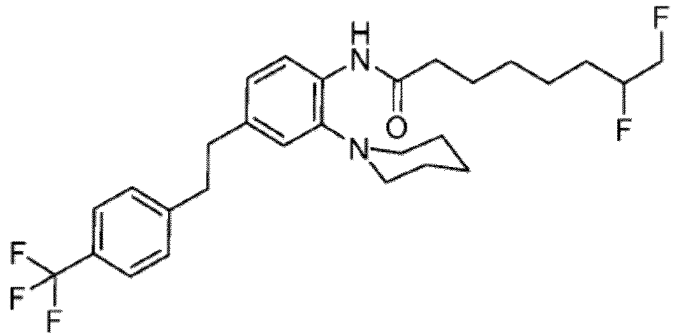 | 7,8-difluoro-N-(2-(pipéridin-1-yl)-4-(4-(trifluorométhyl)phénéth yl)phényl)octanamide (7R)-7,8-difluoro-N-(2-(pipéridin-1-yl)-4-(4-(trifluorométhyl)phénéth yl)phényl)octanamide (7S)-7,8-difluoro-N-(2-(pipéridin-1-yl)-4-(4-(trifluorométhyl)phénéth yl)phényl)octanamide |

(continued)

| | STRUCTURE DU COMPOSÉ | NOM DU COMPOSÉ |
|---|---|---|
| 23 | 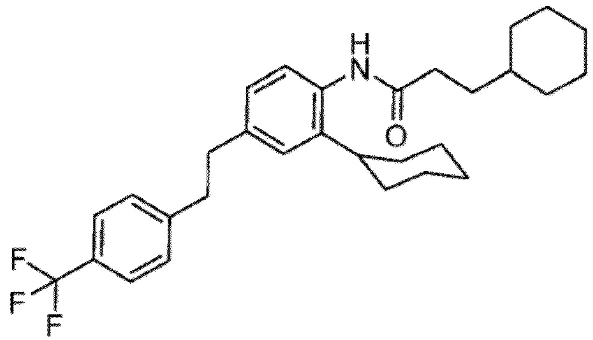 | 3-cyclohexyl-N-(2-cyclohexyl-4-(4-(trifluorométhyl)phénéth yl)phényl)propanamide |
| 23 | 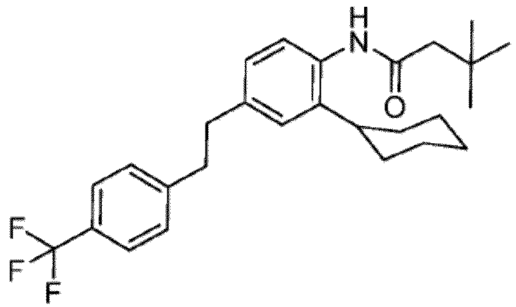 | N-(2-cyclohexyl-4-(4-(trifluorométhyl)phénéth yl)phényl)-3,3-diméthylbutanamide |
| 23 | 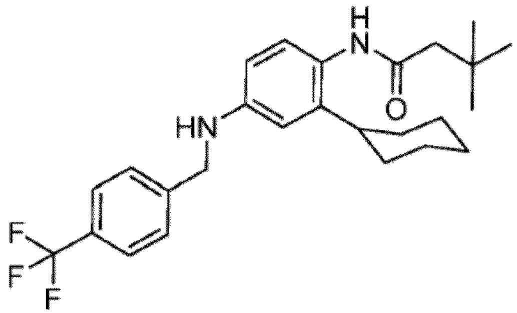 | N-(2-cyclohexyl-4-((4-(trifluorométhyl)benzyl) amino)phényl)-3,3-diméthylbutanamide |

(continued)

| | STRUCTURE DU COMPOSÉ | NOM DU COMPOSÉ |
|---|---|---|
| 23 | 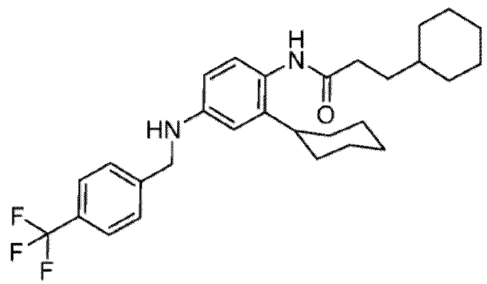 | 3-cyclohexyl-N-(2-cyclohexyl-4-((4-(trifluorométhyl)benzyl) amino)phényl)propanamide |
| 23 | 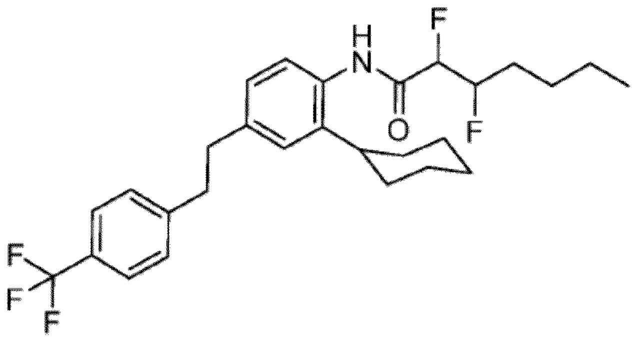 | N-(2-cyclohexyl-4-(4-(trifluorométhyl)phénéth yl)phényl)-2,3-difluoroheptanamide (2R,3S) -N-(2-cyclohexyl-4-(4-(trifluorométhyl)phénéth yl)phényl)-2,3-difluoroheptanamide (2S,3R)-N-(2-cyclohexyl-4-(4-(trifluorométhyl)phénéth yl)phényl)-2,3-difluoroheptanamide (2R,3R)-N-(2-cyclohexyl-4-(4-(trifluorométhyl)phénéth yl)phényl)-2,3-difluoroheptanamide (2S,3S)-N-(2-cyclohexyl-4-(4-(trifluorométhyl)phénéth yl)phényl)-2,3-difluoroheptanamide |
| 24 | 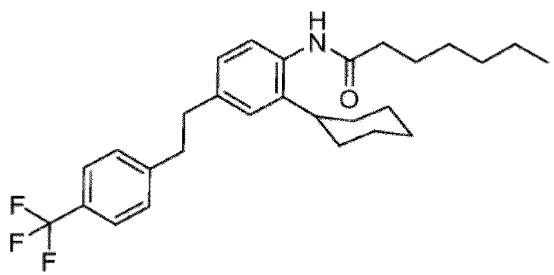 | N-(2-cyclohexyl-4-(4-(trifluorométhyl)phénéth yl)phényl)heptanamide |

(continued)

| | STRUCTURE DU COMPOSÉ | NOM DU COMPOSÉ |
|---|---|---|
| 24 | 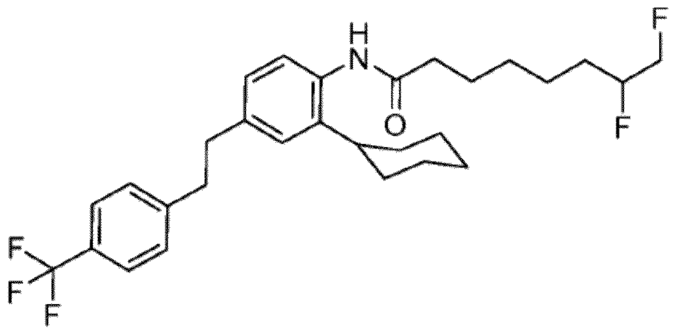 | N-(2-cyclohexyl-4-(4-(trifluorométhyl)phénéth yl)phényl)-7,8-difluorooctanamide<br>(7R)-N-(2-cyclohexyl-4-(4-(trifluorométhyl)phénéth yl)phényl)-7,8-difluorooctanamide<br>(7S)-N-(2-cyclohexyl-4-(4-(trifluorométhyl)phénéth yl)phényl)-7,8-difluorooctanamide |
| 24 | 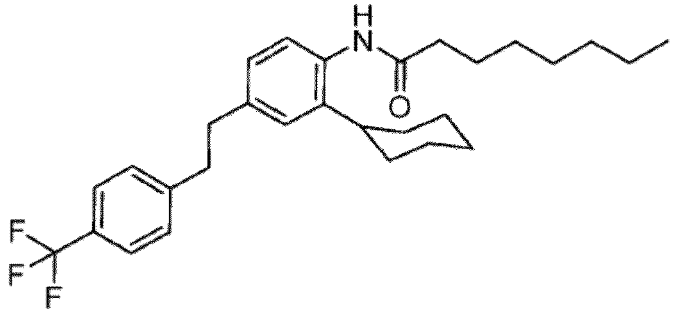 | N-(2-cyclohexyl-4-(4-(trifluorométhyl)phénéth yl)phényl)octanamide |
| 24 | 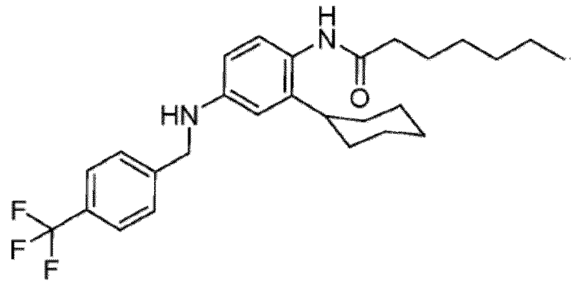 | N-(2-cyclohexyl-4-((4-(trifluorométhyl)benzyl) amino)phényl)heptanamide |

(continued)

| | STRUCTURE DU COMPOSÉ | NOM DU COMPOSÉ |
|---|---|---|
| 24 | 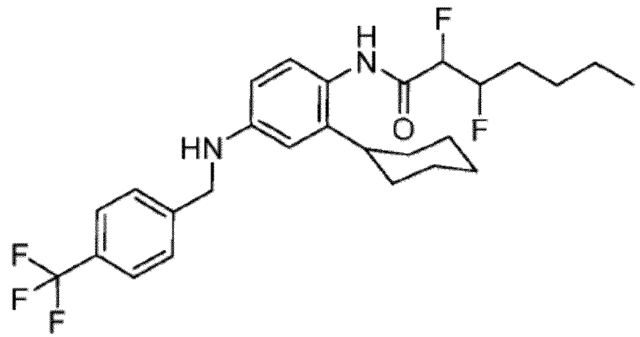 | N-(2-cyclohexyl-4-((4-(trifluorométhyl)benzyl) amino)phényl)-2,3-difluoroheptanamide (2R,3S)-N-(2-cyclohexyl-4-((4-(trifluorométhyl)benzyl) amino)phényl)-2,3-difluoroheptanamide (2S,3R)-N-(2-cyclohexyl-4-((4-(trifluorométhyl)benzyl) amino)phényl)-2,3-difluoroheptanamide (2R,3R)-N-(2-cyclohexyl-4-((4-(trifluorométhyl)benzyl) amino)phényl)-2,3-difluoroheptanamide (2S,3S)-N-(2-cyclohexyl-4-((4-(trifluorométhyl)benzyl) amino)phényl)-2,3-difluoroheptanamide |
| 24 | 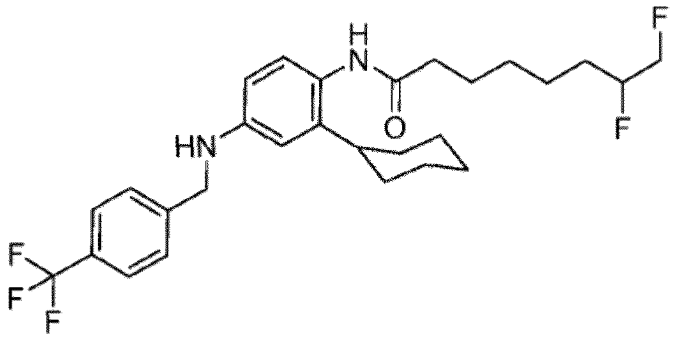 | N-(2-cyclohexyl-4-((4-(trifluorométhyl)benzyl) amino)phényl)-7,8-difluorooctanamide (7R)-N-(2-cyclohexyl-4-((4-(trifluorométhyl)benzyl) amino)phényl)-7,8-difluorooctanamide (7S)-N-(2-cyclohexyl-4-((4-(trifluorométhyl)benzyl) amino)phényl)-7,8-difluorooctanamide |
| 24 | 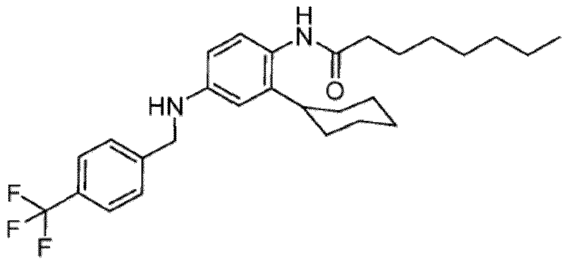 | N-(2-cyclohexyl-4-((4-(trifluorométhyl)benzyl) amino)phényl)octanamide |

(continued)

| | STRUCTURE DU COMPOSÉ | NOM DU COMPOSÉ |
|---|---|---|
| 24 | 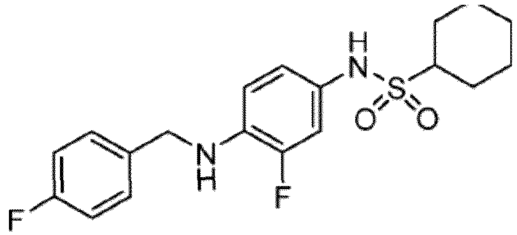 | N-(3-fluoro-4-((4-fluorobenzyl)amino)phény l)cyclohexanesulfonamide |
| 24 | 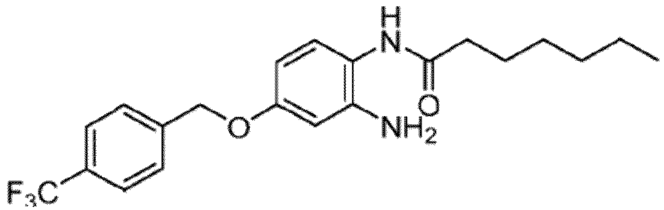 | N-(2-amino-4-((4-(trifluorométhyl)benzyl) oxy)phényl)heptanamide |
| 24 | 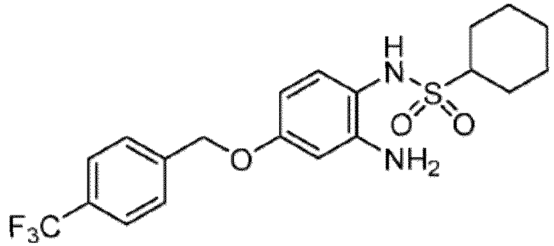 | N-(2-amino-4-((4-(trifluorométhyl)benzyl) oxy)phényl)cyclohexanesu lfonamide |
| 25 | 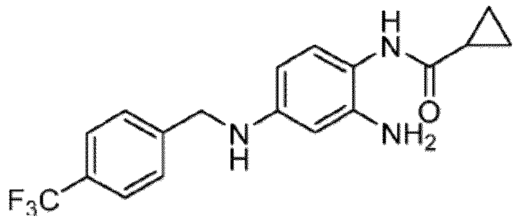 | N-(2-amino-4-((4-(trifluorométhyl)benzyl) amino)phényl)cyclopropan ecarboxamide |

(continued)

| | STRUCTURE DU COMPOSÉ | NOM DU COMPOSÉ |
|---|---|---|
| 25 | 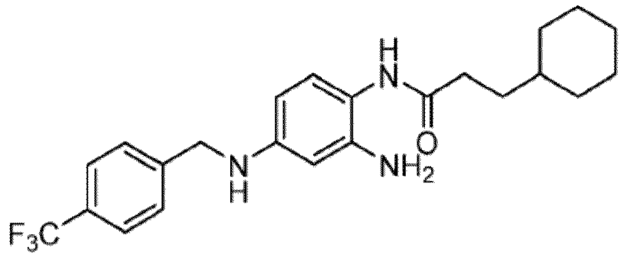 | N-(2-amino-4-((4-(trifluorométhyl)benzyl) amino)phényl)-3-cyclohexylpropanamide |
| 25 | 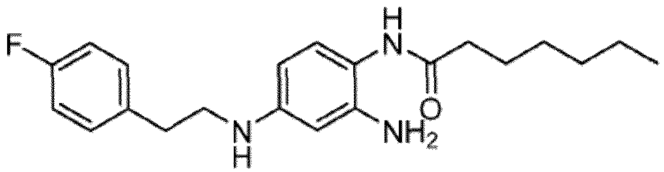 | N-(2-amino-4-((4-fluorophénéthyl)amino)ph ényl)heptanamide |
| 25 | 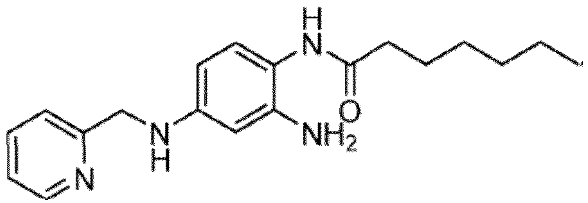 | N-(2-amino-4-((pyridin-2-ylméthyl)amino)phényl)he ptanamide |
| 25 | 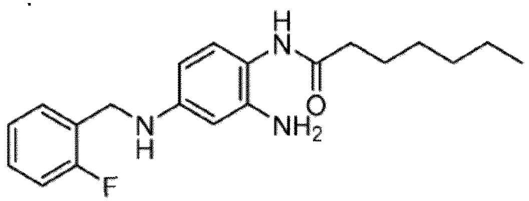 | N-(2-amino-4-((2-fluorobenzyl)amino)phény l)heptanamide |
| 25 | 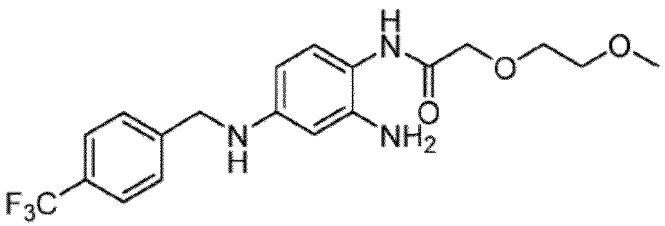 | N-(2-amino-4-((4-(trifluorométhyl)benzyl) amino)phényl)-2-(2-méthoxyéthoxy)acétamide |

(continued)

| | STRUCTURE DU COMPOSÉ | NOM DU COMPOSÉ |
|---|---|---|
| 25 | 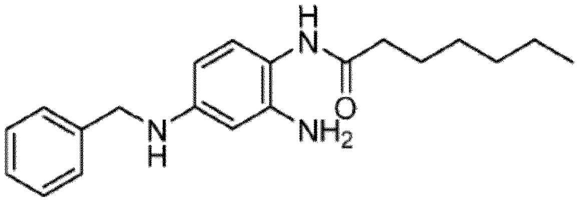 | N-(2-amino-4-(benzylamino)phényl)hept anamide |
| 25 | 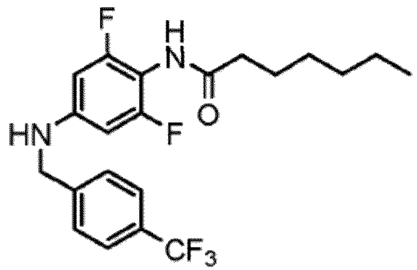 | N-(2,6-difluoro-4-((4-(trifluorométhyl)benzyl) amino)phényl)heptanamide |
| 25 | 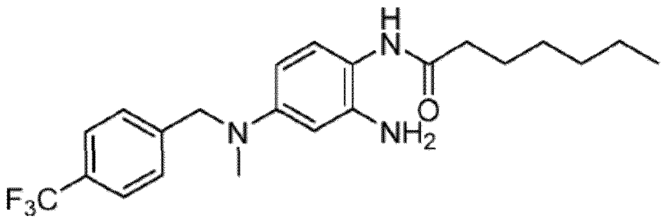 | N-(2-amino-4-(méthyl(4-(trifluorométhyl)benzyl) amino)phényl)heptanamide |
| 25 | 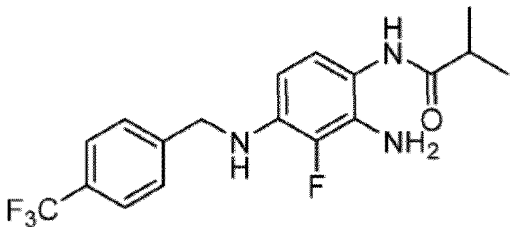 | N-(2-amino-3-fluoro-4-((4-(trifluorométhyl)benzyl) amino)phényl)isobutyrami de |

(continued)

| | STRUCTURE DU COMPOSÉ | NOM DU COMPOSÉ |
|---|---|---|
| 26 | 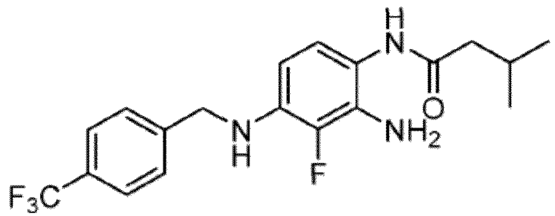 | N-(2-amino-3-fluoro-4-((4-(trifluorométhyl)benzyl) amino)phényl)-3-méthylbutanamide |
| 26 | 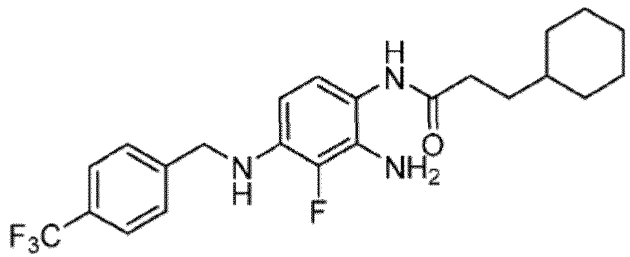 | N-(2-amino-3-fluoro-4-((4-(trifluorométhyl)benzyl) amino)phényl)-3-cyclohexylpropanamide |
| 26 | 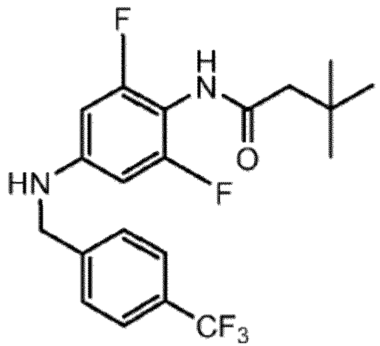 | N-(2,6-difluoro-4-((4-(trifluorométhyl)benzyl) amino)phényl)-3,3-diméthylbutanamide |

(continued)

| | STRUCTURE DU COMPOSÉ | NOM DU COMPOSÉ |
|---|---|---|
| 26 | 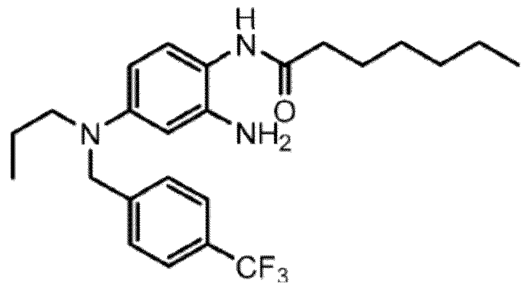 | N-(2-amino-4-(propyl(4-(trifluorométhyl)benzyl) amino)phényl)heptanamide |
| 26 | 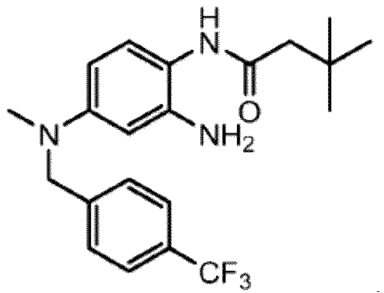 | N-(2-amino-4-(méthyl(4-(trifluorométhyl)benzyl) amino)phényl)-3,3-diméthylbutanamide |
| 26 | 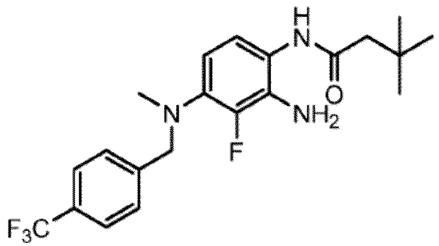 | N-(2-amino-3-fluoro-4-(méthyl(4-(trifluorométhyl)benzyl) amino)phényl)-3,3-diméthylbutanamide |
| 26 | 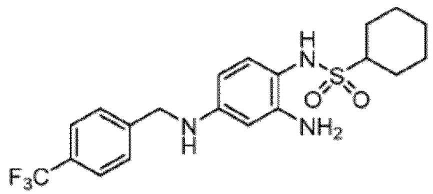 | N-(2-amino-4-((4-(trifluorométhyl)benzyl) amino)phényl)cyclohexane sulfonamide |

(continued)

| | STRUCTURE DU COMPOSÉ | NOM DU COMPOSÉ |
|---|---|---|
| 26 | 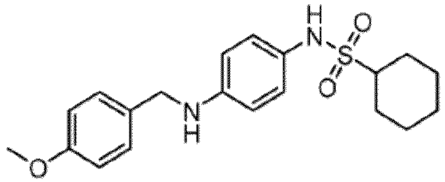 | N-(4-((4-méthoxybenzyl)amino)phén yl)cyclohexanesulfonamid e |
| 26 | 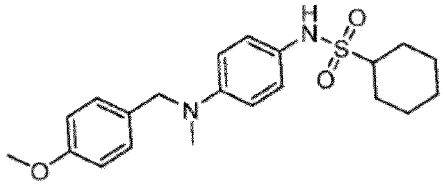 | N-(4-((4-méthoxybenzyl) (méthyl)am ino)phényl)cyclohexanesu lfonamide |
| 26 | 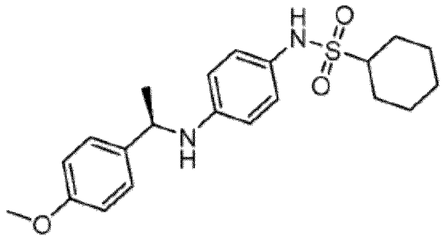 | (R)-N- (4- ((1-(4-méthoxyphényl)éthyl)amin o)phényl)cyclohexanesulf onamide |
| 27 | 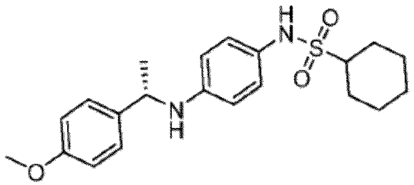 | (S)-N- (4- ((1-(4-méthoxyphényl)éthyl)amin o)phényl)cyclohexanesulf onamide |
| 27 | 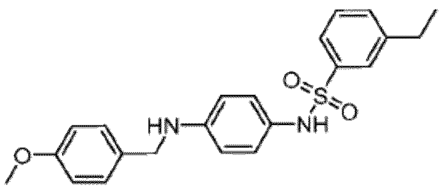 | 3-éthyl-N-(4-((4-méthoxybenzyl)amino)phén yl)benzènesulfonamide |

ou un tautomère, un sel, un solvate, un stéréoisomère ou un isotope de ceux-ci.

**3.** Composé ou un tautomère, un sel, un solvate, un stéréoisomère ou un isotope de celui-ci selon l'une quelconque des revendications précédentes, dans lequel ledit composé est un modulateur des canaux potassiques voltage-dépendants, de préférence un modulateur Kv7.1, Kv7.2, Kv7.3, Kv7.4 et/ou Kv7.5.

**4.** Composé ou un tautomère, un sel, un solvate, un stéréoisomère ou un isotope de celui-ci tel que défini dans l'une quelconque des revendications précédentes, pour une utilisation comme médicament ; de préférence

pour une utilisation dans le traitement et/ou la prévention d'une affection **caractérisée par** un défaut dans la structure et/ou la fonction d'au moins un canal potassique voltage-dépendant, de préférence d'au moins un parmi Kv7.1, Kv7.2, Kv7.3, Kv7.4 et/ou Kv7.5 ; de préférence

dans lequel ladite affection est choisie dans le groupe constitué par : une maladie du système nerveux central, une maladie du système nerveux périphérique, une maladie du système sensoriel, une maladie du système respiratoire, une maladie du système génito-urinaire, une maladie du système gastro-intestinal, une maladie cardiovasculaire, une maladie du muscle squelettique et une canalopathie ; de préférence

dans lequel ladite affection est choisie dans le groupe constitué par : l'épilepsie, un trouble convulsif, une convulsion, un trouble convulsif, un trouble **caractérisé par** une hyperexcitabilité du système nerveux, une épilepsie néonatale, des contractions spontanées, un trouble épileptique précoce, le syndrome d'Ohtahara, l'encéphalopathie épileptique infantile précoce avec suppression-burst, les convulsions néonatales familières bénignes, l'encéphalopathie épileptique, l'encéphalopathie épileptique néonatale sporadique sévère, une déficience intellectuelle, une comorbidité psychiatrique ou cognitive, un déficit cognitif, l'anxiété, la dépression, la schizophrénie, la mort subite liée à l'épilepsie, l'épilepsie pharmaco-résistante, la douleur neuropathique, un accident vasculaire cérébral ischémique, l'angine de poitrine, la migraine, un trouble neurodégénératif, un trouble neurologique, la sclérose latérale amyotrophique, la maladie d'Alzheimer, la maladie de Parkinson, les tremblements, la choréoathétose paroxystique, les acouphènes, la douleur, l'asthme, l'hyperréactivité vésicale, l'incontinence urinaire, la constipation, le syndrome du côlon irritable, la diarrhée, la maladie de Crohn, l'hypertension, l'hypertension vasculaire, la dystrophie musculaire, une maladie d'hyperexcitabilité des muscles squelettiques, la myokymie, la myotonie, une canalopathie génétique, une canalopathie transcriptionnelle, un trouble lié à Kv7.2 et/ou Kv7.3, une encéphalopathie liée à Kv 7.2, la surdité autosomique dominante de type 2 (DFNA2), l'ataxie, l'ataxie épisodique de type 1, le syndrome d'ataxie épisodique avec myokymie, la neuromyotonie, la paralysie périodique hypokaliémique, une arythmie, une arythmie congénitale, le syndrome du QT long, le syndrome de Romano-Ward, le syndrome de Jervell et Lange-Nielsen, le syndrome de Bartter, l'hypoglycémie hyperinsulinémique infantile (HHI), l'atténuation de la réponse vasoconstrictrice, le diabète de type 2 et la sclérose en plaques.

**5.** Composé ou un tautomère, un sel, un solvate, un stéréoisomère ou un isotope de celui-ci, tel que défini dans l'une quelconque des revendications 1 à 3 pour une utilisation comme médicament anticonvulsivant, analgésique, anti-ischémique, antiarythmique, neuroprotecteur, antiacouphène, antiasthmatique, modulateur de la motilité gastro-intestinale, antihypertenseur, antimyotonique et/ou contre la dystrophie musculaire squelettique.

**6.** Composition pharmaceutique comprenant le composé ou un tautomère, un sel, un solvate, un stéréoisomère ou un isotope de celui-ci tel que défini dans l'une quelconque des revendications 1 à 3 et un véhicule pharmaceutiquement acceptable.

**7.** Composition pharmaceutique selon la revendication 6 comprenant un autre agent thérapeutique choisi parmi : un anticonvulsivant, un analgésique et un médicament anti-inflammatoire non stéroïdien (AINS), de préférence ledit anticonvulsivant est choisi dans le groupe constitué par : l'acétazolamide, le brivaracétam, la carbamazépine, le clobazam, le clonazépam, le diazépam, l'eslicarbazépine, l'éthosuximide, la fosphénytoïne, la gabapentine, le lacosamide, la lamotrigine, le lévétiracétam, le lorazépam, le méthosuximide, le nitrazépam, l'oxcarbazépine, le pérampanel, le phénobarbital, la phénytoïne, la prégabaline, la primidone, le rufmamide, le stiripentol, le topiramate, l'acide valproïque, la vigabatrine, le felbamate, la tiagabine et le zonisamide, de préférence, ledit analgésique est l'acétaminophène ou un opioïde, de préférence ledit médicament anti-inflammatoire non stéroïdien (AINS) est choisi dans le groupe constitué par : l'aspirine, le diclofénac, le diflusinal, l'étodolac, le fenbufène, le fénoprofène, le flufénisal, le flurbiprofène, l'ibuprofène, l'indométhacine, le kétoprofène, le kétorolac, l'acide méclofénamique, l'acide méfénamique, le méloxicam, la nabumétone, le naproxène, le nimésulide, le nitroflurbiprofène, l'olsalazine, l'oxaprozine, la phénylbutazone, le piroxicam, la sulfasalazine, le sulindac, la tolmétine et le zomépirac, de préférence ledit opioïde est la morphine.

**8.** Composition pharmaceutique telle que définie dans la revendication 6 ou 7 pour une utilisation dans le traitement et/ou la prévention d'une affection **caractérisée par** un défaut dans la structure et/ou la fonction d'au moins un canal potassique voltage-dépendant, de préférence d'au moins un parmi Kv7.1, Kv7.2, Kv7.3, Kv7.4 et/ou Kv7.5.

**9.** Composé pour une utilisation selon l'une quelconque des revendications 4 à 5 ou composition pharmaceutique pour une utilisation selon la revendication 8 qui est administré(e) à un animal, de préférence un animal de compagnie ou du bétail.

**10.** Composé ou un tautomère, un sel, un solvate, un stéréoisomère ou un isotope de celui-ci tel que défini dans l'une quelconque des revendications 1 à 3 pour une utilisation dans une méthode de modulation d'un canal potassique voltage-dépendant, de préférence ledit canal potassique voltage-dépendant est Kv7.1, Kv7.2, Kv7.3, Kv7.4 et/ou Kv7.5.

**11.** Méthode in vitro de modulation d'un canal potassique voltage-dépendant en utilisant le composé ou un tautomère, un sel, un solvate, un stéréoisomère ou un isotope de celui-ci tel que défini dans l'une quelconque des revendications 1 à 3, ledit canal potassique voltage-dépendant étant de préférence Kv7.1, Kv7.2, Kv7.3, Kv7.4 et/ou Kv7.5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5384330 A
- WO 2005087754 A
- WO 2008024398 A
- WO 2009023677 A
- US 20140336252 A
- WO 2009023677 A1


### Non-patent literature cited in the description


- **ALDRICH R. et al.** Potassium channels.. *IUPHAR/BPS Guide to PHARMACOLOGY*
- **SOLDOVIERI MV et al.** *Physiology*, 2011, vol. 26 (5), 365-376
- **MICELI et al.** *GeneReviews*, 2016
- **PORTER RJ et al.** *Neurology*, 2007, vol. 68 (15), 1197-1204
- **BORLAK et al.** *Metabolism: Clinical and Experimental*, 2006, vol. 55 (6), 711-21
- **KUMAR et al.** *Mol. Pharmacol.*, 2016, vol. 89 (6), 667-77
- **ZHANG H et al.** *Neuron*, 2003, vol. 37, 963-975
- **YUS-NAJERA E et al.** *JBC*, 2002, vol. 277, 28545-28553
- **WEN H et al.** *J Neurosci*, 2002, vol. 22, 7991-8001
- **HOSHI N et al.** *Nat Neurosci*, 2003, vol. 6, 564-571
- **PAN Z et al.** *J Neurosci*, 2006, vol. 26, 2599-2613
- **EKBERG J et al.** *JBC*, 2007, vol. 282, 12135-12142
- **SM BERGE et al.** *J. Pharm. Sci*, 1977, vol. 66, 1-19
- **PL GOULD**. *Int. J. Pharm.*, 1986, vol. 33, 201-217
- **LD BIGHLEY et al.** Encyclopedia of Pharmaceutical Technology. Marcel Dekker Inc, 1996, vol. 13, 453-497
- **KIBBE**. Handbook of Pharmaceutical Excipients. Pharmaceutical Press, 2000
- **REMINGTON**. The Science and Practice of Pharmacy. Lippincott Williams & Wilkins, 2000
- **ISMAIL et al.** *J Chromatogr A.*, 2016, vol. 4 (1427), 55-68
- **SOLDOVIERI et al.** *J Biol Chem.*, 2006, vol. 281 (1), 418-28
- **IANNOTTI et al.** *J Pharmacol Exp Ther.*, 2010, vol. 332 (3), 811-20
- **LANDOULSI et al.** *Mol Pharmacol.*, 2013, vol. 84 (5), 763-73
- **MICELI et al.** *Proc Natl Acad Sci U S A.*, 2013, vol. 110 (11), 4386-91
- **AMBROSINO et al.** *Br J Pharmacol.*, 2013, vol. 168 (6), 1430-1444
- **WUTTKE et al.** *Mol. Pharmacol.*, 2005, vol. 67 (4), 1009-1017